# EUROPEAN PATENT APPLICATION

(11) **EP 4 556 026 A1**
(43) Date of publication of application: **21.05.2025**
(21) Application number: 23839014.0
(22) Date of filing: 13.07.2023
(51) Int. Cl.: A61K 47/68, C07K 16/30, A61K 39/00, A61P 35/00, C07D 491/22, A61K 45/00

(54) **ANTIBODY-DRUG CONJUGATE, AND PREPARATION METHOD THEREFOR AND USE THEREOF**

(30) Priority: 14.07.2022 CN 202210838614
(71) Applicant: Medilink Therapeutics (Suzhou) Co., Ltd., Suzhou, Jiangsu 215000 (CN)
(72) Inventor: XIAO, Liang, Suzhou, Jiangsu 215000 (CN); CAI, Jiaqiang, Suzhou, Jiangsu 215000 (CN); XUE, Tongtong, Suzhou, Jiangsu 215000 (CN)
(74) Representative: Vossius & Partner Patentanwälte Rechtsanwälte mbB
(86) International application number: PCT/CN2023/107220
(87) International publication number: WO 2024/012524

(57) **Abstract**

The present disclosure relates to a bioactive conjugate, and a preparation method therefor and the use thereof. Specifically, disclosed are an antibody-drug conjugate as represented by formula XV, and a preparation method therefor and the use thereof in the prevention and/or treatment of diseases related to an abnormal cell activity, including but not limited to tumor diseases.

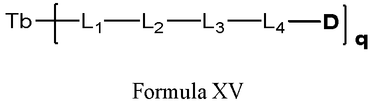

## Description

### TECHNICAL FIELD

The present disclosure belongs to the field of pharmaceutical technology, and relates to various antibodies, an antibody-drug conjugate, a preparation method therefor, and use thereof in the prevention and/or treatment of a disease associated with abnormal cell activity, including but not limited to use thereof in the prevention and/or treatment of a tumor disease.

### BACKGROUND

Chemotherapy using cytotoxin was once the standard treatment for a cancer, but highly lethal cytotoxic molecules kill normal cells, causing severe toxic side effects. Targeted anti-tumor drugs have both targeting property and anti-tumor activity, and thus have become a hotspot in the current tumor research field. However, the targeted drugs often cause significant toxic side effects due to the problem of target selectivity, such that the therapeutic effect of the targeted drugs is limited. Biomacromolecule drugs, such as antibodies or antibody fragments, although highly targeted, have limited or no therapeutic effect on solid tumors. ADC is a conjugate of an antibody and a small molecule drug, which combines the targeting effect of the antibody with the activity of the bioactive molecule, acting as a biological missile with promising advantages of efficacy and safety. The antibody guides the ADC to bind to a target cell, which is subsequently internalized by the cell. The small molecule drug is then released inside the cell through enzymatic hydrolysis by a specific enzyme to treat a disease.

ADC drugs have developed very rapidly in recent years, with 14 types of ADCs already on the market. Among many targeted ADCs, antibody/ADC drugs targeting NaPi2b are all in the clinical research stage. For many targets, developing monoclonal antibodies or ADC drugs with differentiation, higher quality, and better safety can provide tumor patients with broader and better medication options, and also has a broad market prospect.

### CONTENT OF THE PRESENT INVENTION

To improve the therapeutic effecacy of an antibody-drug conjugate (ADC), reduce toxic side effects of the drug, and expand the therapeutic window, the present disclosure provides an antibody-drug conjugate of formula (XV) or a pharmaceutically acceptable salt or solvate thereof, which includes a bioactive molecule (drug molecule), a linker, and a targeting moiety, wherein the targeting moiety is attached to the linker via an active group (e.g., a sulfhydryl group) to form the antibody-drug conjugate. The present disclosure also develops an antibody comprising a variable light chain domain (VL) and/or a variable heavy chain domain (VH) against NaPi2b, wherein the antibody comprising the variable light chain domain and/or the variable heavy chain domain is derived from a human antibody.

For this purpose, in a first aspect of the present disclosure, the present disclosure provides an antibody-drug conjugate of formula XV, 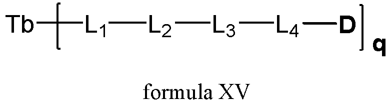
or a stereoisomer of the antibody-drug conjugate, a prodrug thereof, a pharmaceutically acceptable salt thereof, or a pharmaceutically acceptable solvate thereof,
wherein:
   Tb is an anti-NaPi2b antibody or an antigen-binding fragment thereof;
   q is a drug-to-antibody coupling ratio;
   D is a bioactive molecular fragment;
   L₁ is an extension unit;
   L₂ is absent or is a connector unit;
   L₃ is selected from an amino acid residue and a short peptide consisting of 2-10 amino acid residues;
   L₄ is absent or present, and when L₄ is present, L₄ is selected from 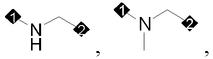 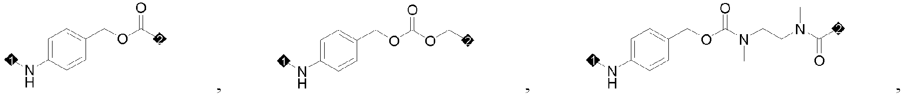 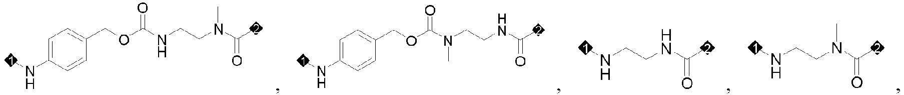 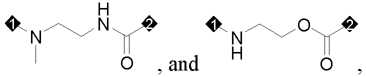 wherein position 1 is attached to L₃, and position 2 is attached to D.

In addition, it should also be noted that when referring to "position 1 of L₁ being attached to Tb via a sulfur (S) atom", it will be understood by those skilled in the art that position 1 of L₁ is attached to a sulfhydryl group contained in Tb (e.g., an antibody) itself after the disulfide bond is opened (e.g., the disulfide bond may be opened by reducing the disulfide bond using the reducing agent TCEP to generate the sulfhydryl group -SH), that is, -S- between L₁ and Tb is not an additional extraneous sulfur atom. For example, in 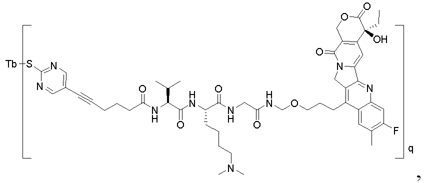 -S- is not an additional extraneous sulfur atom, but is -S- formed by attaching the sulfhydryl group contained in Tb itself after the disulfide bond is opened to position 1 of L₁ such

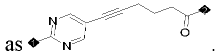

The extension unit is a component of an antibody-drug conjugate or a drug-linker conjugate or a linker, which functions to attach Tb bound to a target to the remainder of the antibody-drug conjugate or the remainder of the linker. The extension unit is capable of attaching the Tb unit to L₂ (if present) or L₃, and specific examples include, but are not limited to: 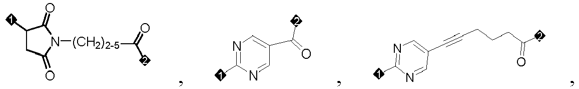 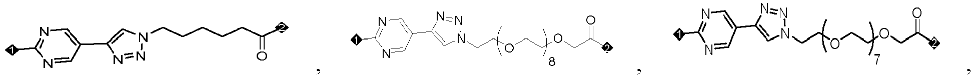 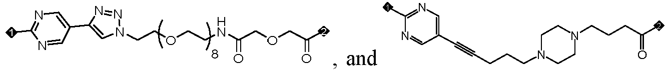 (wherein position 1 is attached to the antibody moiety bound to a target, and position 2 is attached to L₂ or L₃).

In some embodiments, L₁ is selected from: 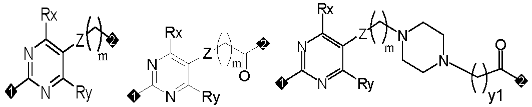 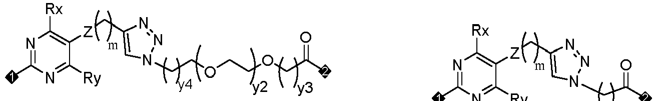 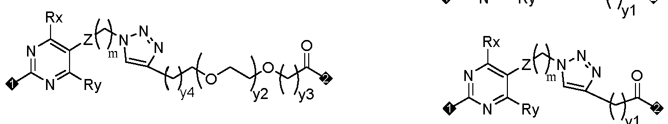 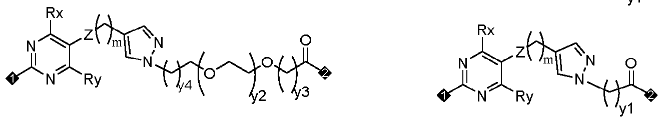 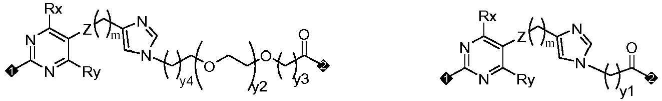 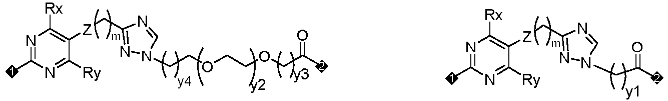 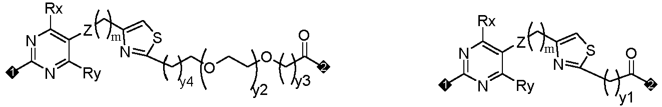 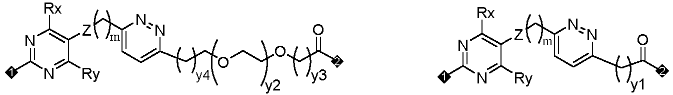 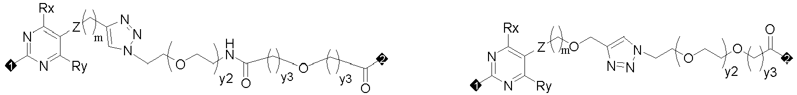 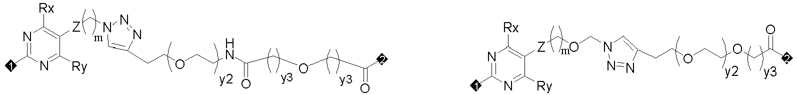 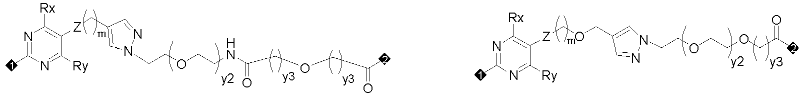 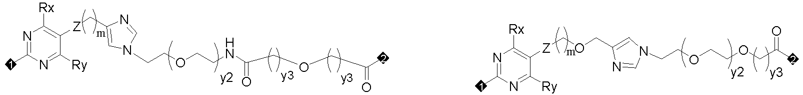 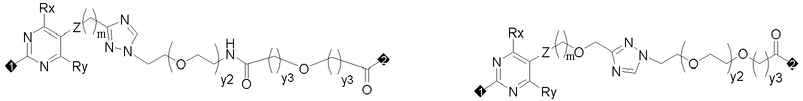 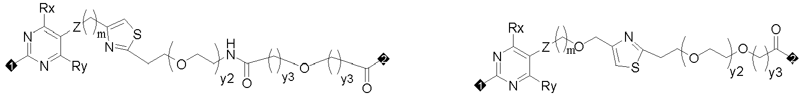 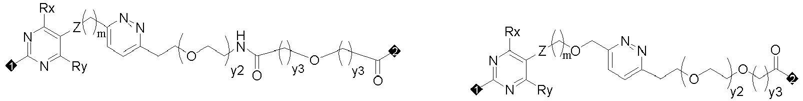 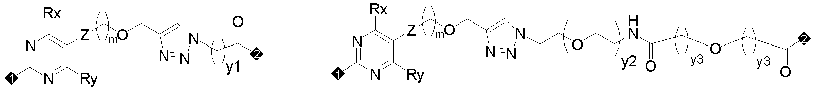 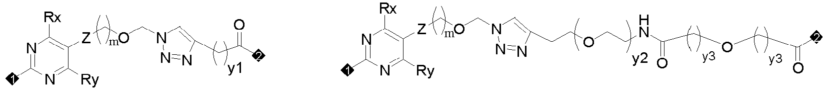 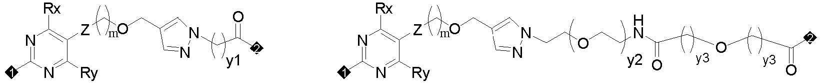 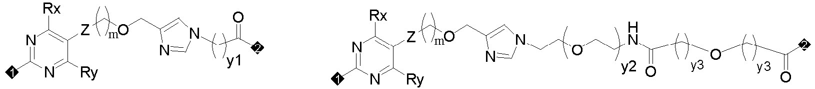 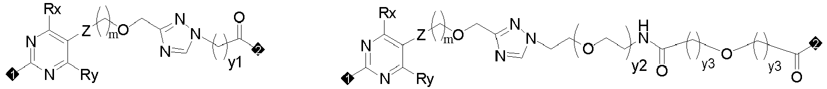 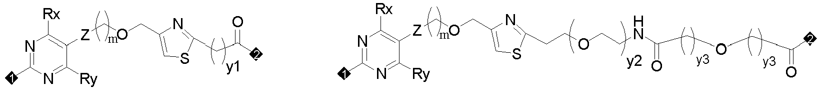 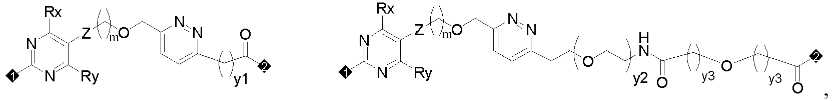
wherein each Z is independently selected from a direct bond, a carbon-carbon triple bond, a carbon-carbon double bond, C6-10 aryl, 5-10 membered heteroaryl, acylamino, sulfonamido, imino, and CF₂;
Rx and Ry are independently selected from H and C1-4 alkyl;
each m is independently selected from 0, 1, 2, 3, 4, 5, and 6;
y1, y2, y3, and y4 are independently selected from any integer between 0 and 20;
position 1 is attached to Tb via a sulfur (S) atom, and position 2 is attached to L₂ or L₃.

The connector unit is a component of an antibody-drug conjugate or a drug-linker conjugate or a linker, which functions to bind the extension unit to an amino acid residue or a fragment or short peptide consisting of 1-10 amino acid residues. The connector unit, when present, is capable of attaching L₁ to L₃. Specific examples include, but are not limited to: 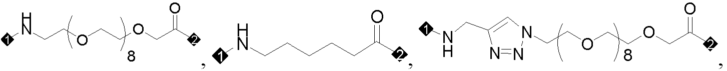 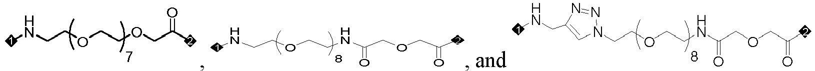 (wherein position 1 is attached to the extension unit, and position 2 is attached to L₃).

In some embodiments, L₂ is absent or present, and when L₂ is present, L₂ is selected from: 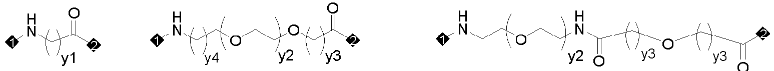 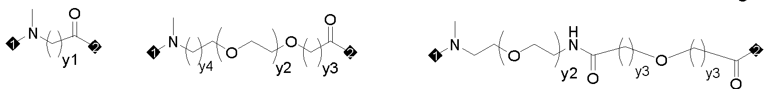 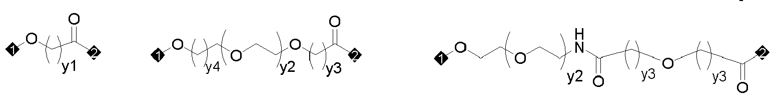 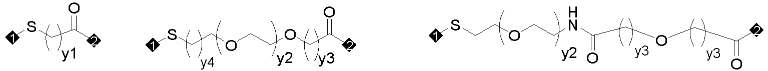 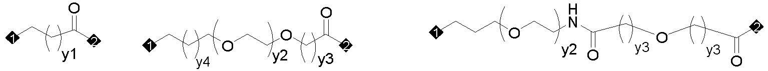 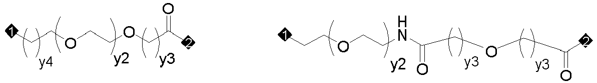 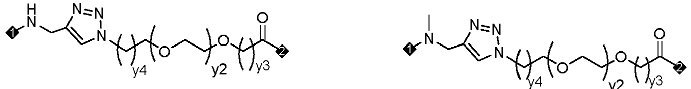 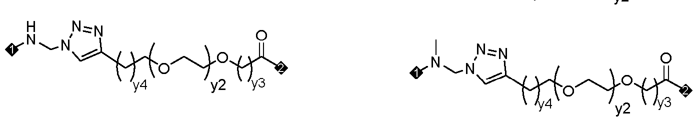 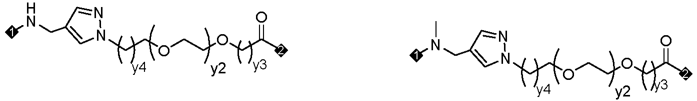 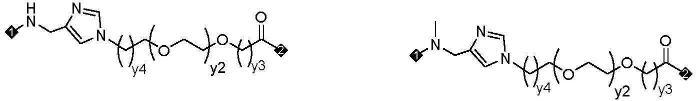 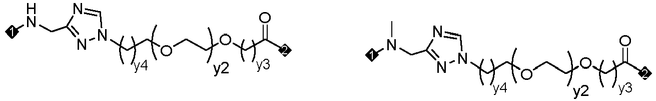 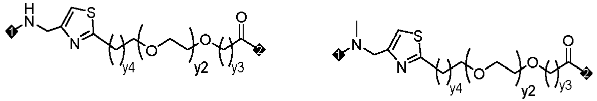 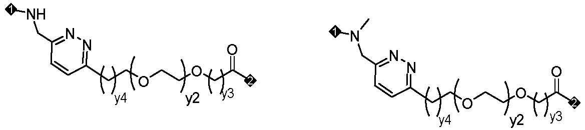 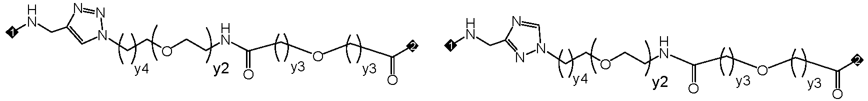 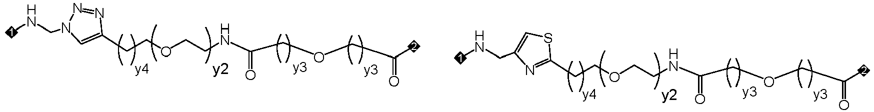 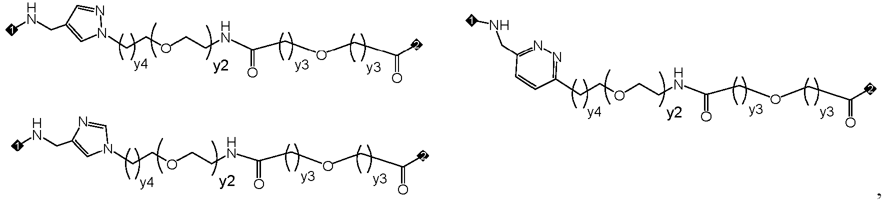 wherein y1, y2, y3, and y4 are independently selected from any integer between 0 and 20, position 1 is attached to L₁, and position 2 is attached to L₃.

In some embodiments, L₁ is

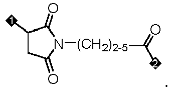

In some embodiments, L₁ is selected from 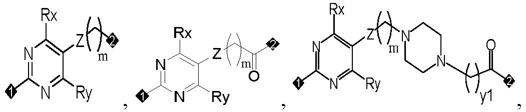 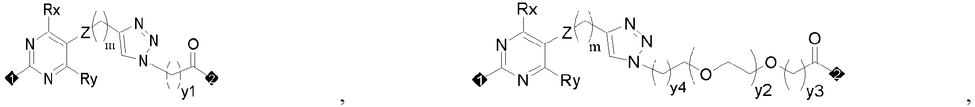 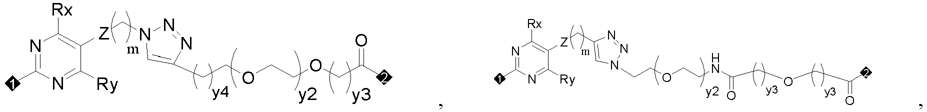 and 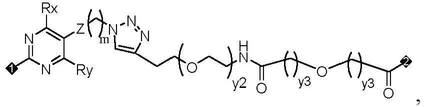 wherein each Z is independently selected from a direct bond, a carbon-carbon triple bond, a carbon-carbon double bond, C6-10 aryl, 5-10 membered heteroaryl, and acylamino (preferably selected from a direct bond, a carbon-carbon triple bond, and a carbon-carbon double bond); Rx and Ry are independently selected from H and C1-4 alkyl; each m is independently selected from 0, 1, 2, 3, 4, 5, and 6; y1 is selected from any integer between 1 and 6 (e.g., 4, 5, 6); each y2 is independently selected from any integer between 0 and 15 (e.g., between 6 and 15); each y3 is independently selected from 1, 2, and 3; each y4 is independently selected from 0 and 1; position 1 is attached to Tb via a sulfur (S) atom, and position 2 is attached to L₂ or L₃.

In some embodiments, L₁ is selected from 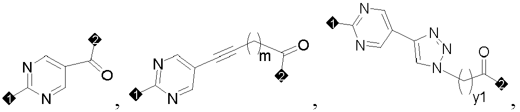 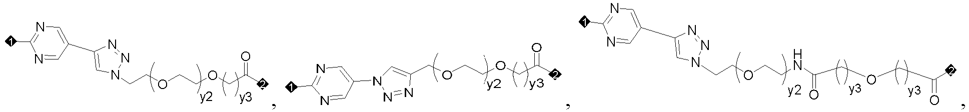 and 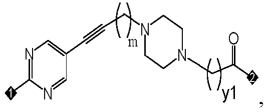 wherein m is selected from 2, 3, and 4, y1 is selected from any integer between 1 and 6 (e.g., 4, 5, 6), each y2 is independently selected from any integer between 0 and 10 (e.g., between 6 and 10), each y3 is independently selected from 1 and 2, position 1 is attached to Tb via a sulfur (S) atom, and position 2 is attached to L₂ or L₃.

In some embodiments, L₁ is selected from

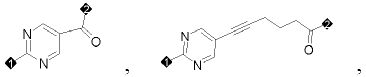

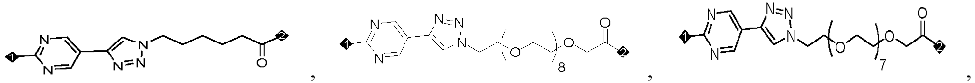

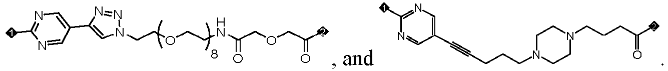

In some embodiments, L₁ is selected from 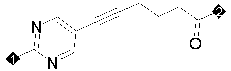 and 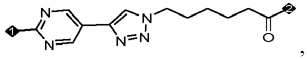 wherein position 1 is attached to Tb via a sulfur (S) atom, and position 2 is attached to L₂ or L₃.

In some embodiments, L₂ is absent or present, and when L₂ is present, L₂ is selected from 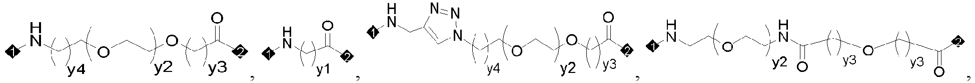 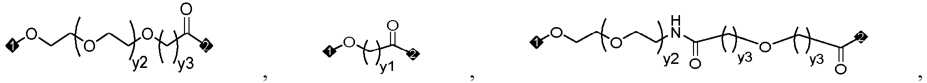 and 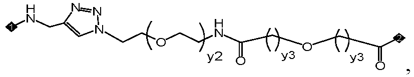 wherein y1 is selected from any integer between 1 and 6 (e.g., 4, 5, 6), each y2 is independently selected from any integer between 0 and 10 (e.g., between 6 and 10), each y3 is independently selected from 1 and 2, each y4 is independently selected from 0 and 1, position 1 is attached to L₁, and position 2 is attached to L₃.

In some embodiments, L₂ is absent or present, and when L₂ is present, L₂ is selected from 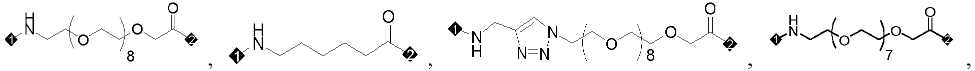 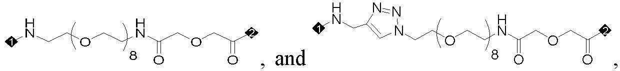 wherein position 1 is attached to L₁, and position 2 is attached to L₃.

In some embodiments, L₂ is absent.

In some embodiments, L₂ is selected from

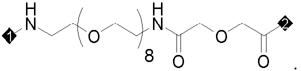

In some embodiments, L₃ is selected from an amino acid residue and a short peptide consisting of 2-10 amino acid residues, wherein the amino acid residue is selected from a natural amino acid residue and a non-natural amino acid residue, or is selected from an amino acid residue represented by AA₁ and a stereoisomer thereof.

In some embodiments, L₃ is selected from amino acid residues Val, D-Val, Cit, Phe, Lys, Lys(Ac), Leu, Gly, Ala, Asn, Asp, Arg, AA¹, and a short peptide consisting of 2-10 amino acid residues selected from Val, Cit, Phe, Lys, D-Val, Leu, Gly, Ala, Asn, Asp, and AA¹.

In some embodiments, L₃ is selected from Val, Cit, Phe, Lys, D-Val, Leu, Gly, Ala, Asn, AA¹, Val-Cit, Cit-Val, Cit-Ala, Val-Ala, Lys-Val, Val-Lys(Ac), Phe-Lys, Phe-Lys(Ac), Ala-Ala, Val-AA¹, Ala-AA¹, Gly-AA¹, AA'-Gly, Ala-Ala-Ala, Ala-Ala-Asn, Ala-Ala-Asp, Val-AA'-Gly, Ala-AA'-Gly, Gly-AA¹-Gly, Lys-Ala-Ala-Asn, Lys-Ala-Ala-Asp, Gly-Phe-Gly, Gly-Gly-Phe-Gly, D-Val-Leu-Lys, Gly-Gly-Arg, Ala-Ala-Asn, Gly-Gly-Phe, Val-Lys-Gly, Val-Lys-Gly-Gly, Val-Lys, and Lys-Ala-Asn.

In some embodiments, L₃ is selected from AA¹, AA¹-Gly, Val-Cit, Val-AA¹-Gly, AA'-Ala-Asn, and Gly-Gly-Phe-Gly.

In some embodiments, L₃ is selected from AA¹ and Val-AA'-Gly.

In some embodiments, L₃ is selected from Val-AA¹-Gly.

In some embodiments, L₃ is selected from 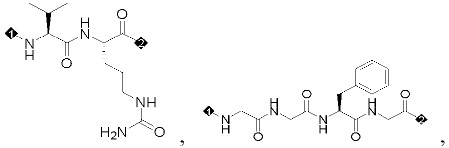 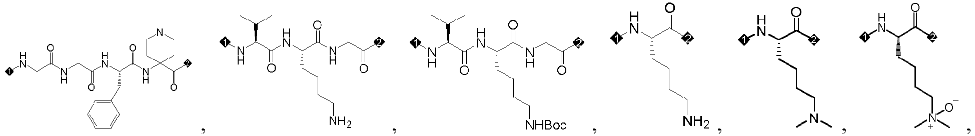 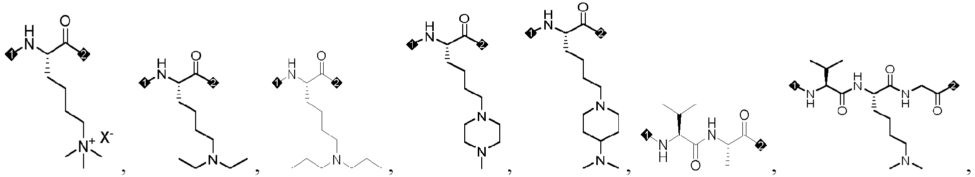 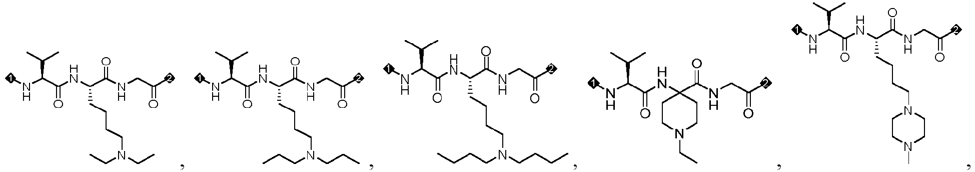 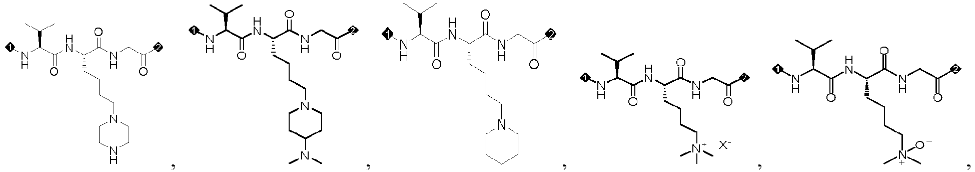 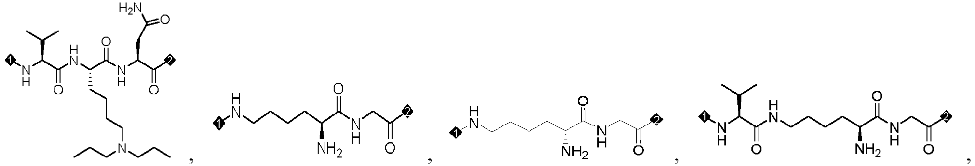 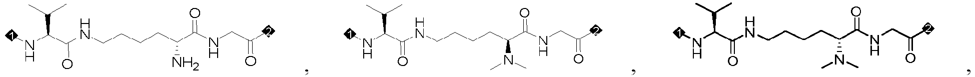 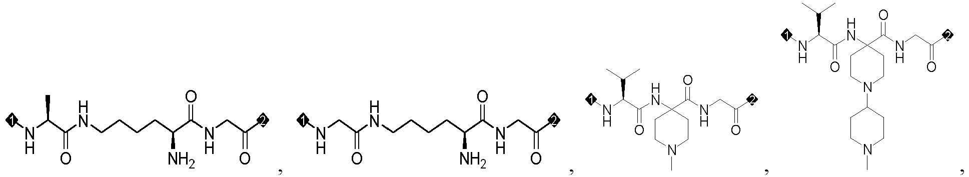 and 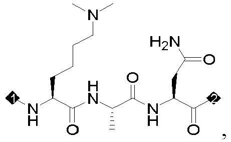 wherein X⁻ is selected from a halide ion, a carboxylate ion, a sulfate ion, a hydrogen sulfate ion, and OH⁻, position 1 is attached to L₁ or L₂, and position 2 is attached to L₄ or D.

In some embodiments, L₃ is selected from 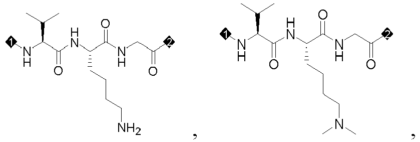 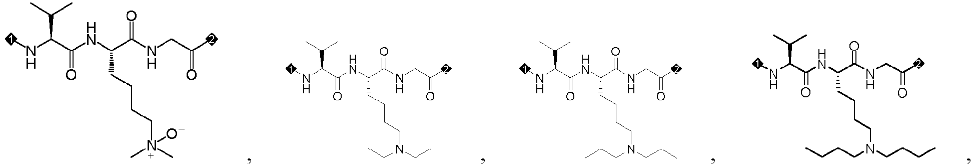 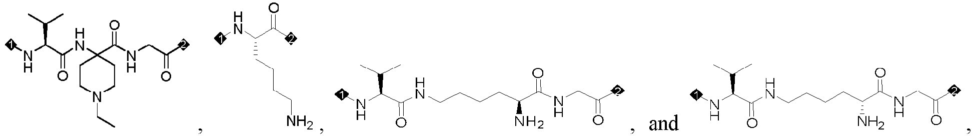 wherein position 1 is attached to L₁ or L₂, and position 2 is attached to L₄ or D.

In some embodiments, L₃ is selected from 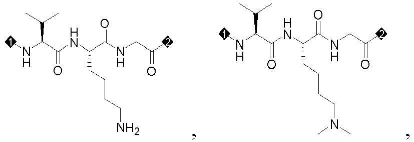 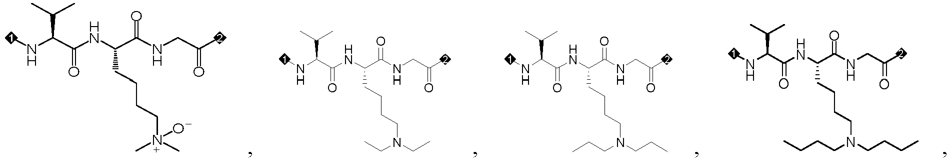 and 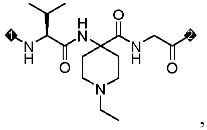 wherein position 1 is attached to L₁ or L₂, and position 2 is attached to L₄ or D.

In some embodiments, the structure of the amino acid residue represented by AA¹ is shown as follows: 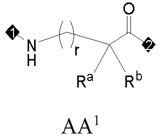 wherein:
R^{a} and R^{b} are each independently selected from H, 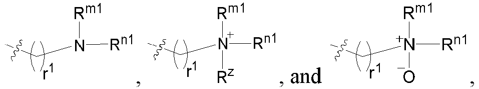 and R^{a} and R^{b} are not both H; or
R^{a} and R^{b}, together with the carbon atom to which they are both attached, form a 4-10 membered heterocyclic ring, the 4-10 membered heterocyclic ring being optionally substituted with one or more R⁰;
r and r¹ are each independently selected from any integer from 0 to 20;
R^{m1} and Rⁿ¹ are each independently selected from H, C1-6 alkyl, C3-6 cycloalkyl, and -COOR^{x1};
R^{x1} is selected from C1-6 alkyl; or
R^{m1} and Rⁿ¹, together with the nitrogen atom to which they are both attached, form a 4-10 membered heterocyclic ring, the 4-10 membered heterocyclic ring being optionally substituted with one or more R^{0'};
R^{z} is selected from C1-6 alkyl;
R⁰ and R^{0'} are each independently selected from C1-6 alkyl, C3-6 cycloalkyl, -NR^{m2}Rⁿ², and 4-10 membered heterocyclyl optionally substituted with C1-6 alkyl;
R^{m2} and Rⁿ² are each independently selected from H and C1-6 alkyl.

In some embodiments, either R^{a} or R^{b} is H, and the other is selected from 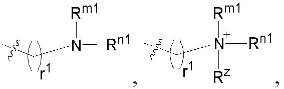 and

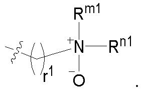

In some embodiments, either R^{a} or R^{b} is H, and the other is selected from 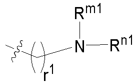 and

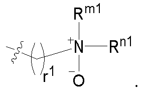

In some embodiments, R^{a} and R^{b}, together with the carbon atom to which they are both attached, form a 5-6 membered heterocyclic ring substituted with R⁰.

In some embodiments, R^{a} and R^{b}, together with the carbon atom to which they are both attached, form a piperidine ring or piperazine ring substituted with R⁰.

In some embodiments, R^{a} and R^{b}, together with the carbon atom to which they are both attached, form a piperidine ring substituted with R⁰.

In some embodiments, R^{a} and R^{b}, together with the carbon atom to which they are both attached, form 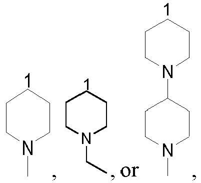 wherein the carbon atom at position 1 is the carbon atom to which R^{a} and R^{b} are both attached.

In some embodiments, R^{a} and R^{b}, together with the carbon atom to which they are both attached, form 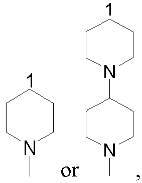 wherein the carbon atom at position 1 is the carbon atom to which R^{a} and R^{b} are both attached.

In some embodiments, r and r¹ are each independently selected from 0, 1, 2, 3, 4, and 5.

In some embodiments, r and r¹ are each independently selected from 0 and 4.

In some embodiments, either r or r¹ is 0, and the other is 4.

In some embodiments, R^{m1} and Rⁿ¹ are each independently selected from H, methyl, ethyl, n-propyl, *n-*butyl, -COOCH3, -COOCH2CH3, -COOCH2CH2CH3, -COOCH(CH3)2, -COOC(CH3)3, and - COOCH2CH2CH2CH3.

In some embodiments, R^{m1} and Rⁿ¹ are each independently selected from H, C1-6 alkyl, C3-6 cycloalkyl, and tert-butoxycarbonyl.

In some embodiments, R^{m1} and Rⁿ¹ are each independently selected from H and C1-6 alkyl.

In some embodiments, R^{m1} and Rⁿ¹ are each independently selected from H, methyl, ethyl, and n-propyl.

In some embodiments, in r and r¹, when r is 4, and r¹ is 0, R^{m1} and Rⁿ¹ are each independently selected from H and C1-6 alkyl (e.g., H and methyl); when r is 0, and r¹ is 4, R^{m1} and Rⁿ¹ are each independently selected from C1-6 alkyl (e.g., methyl, ethyl, and n-propyl), preferably selected from C2-6 alkyl (e.g., ethyl and n-propyl).

In some embodiments, R^{m1} and Rⁿ¹, together with the nitrogen atom to which they are both attached, form a 5-6 membered heterocyclic ring optionally substituted with R^{0'}.

In some embodiments, R^{m1} and Rⁿ¹, together with the nitrogen atom to which they are both attached, form a piperidine ring or piperazine ring optionally substituted with R^{0'}.

In some embodiments, R^{m1} and Rⁿ¹, together with the nitrogen atom to which they are both attached, form 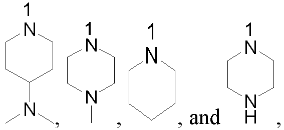 wherein the nitrogen atom at position 1 is the nitrogen atom to which R^{m1} and Rⁿ¹ are both attached.

In some embodiments, R^{z} is methyl.

In some embodiments, R⁰ and R^{0'} are each independently selected from C1-6 alkyl, -NR^{m2}Rⁿ², and 5-6 membered heterocyclyl optionally substituted with C1-6 alkyl.

In some embodiments, R⁰ is selected from C1-6 alkyl and 5-6 membered heterocyclyl substituted with C1-6 alkyl, the 5-6 membered heterocyclyl being selected from piperidinyl and piperazinyl.

In some embodiments, R⁰ is selected from methyl, ethyl, and 5-6 membered heterocyclyl substituted with methyl, the 5-6 membered heterocyclyl being piperidinyl.

In some embodiments, R⁰ is selected from methyl and 5-6 membered heterocyclyl substituted with methyl, the 5-6 membered heterocyclyl being piperidinyl.

In some embodiments, R⁰ is selected from methyl, ethyl, and

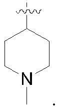

In some embodiments, R⁰ is selected from methyl and

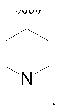

In some embodiments, R^{0'} is selected from C1-6 alkyl and -NR^{m2}Rⁿ².

In some embodiments, R^{0'} is selected from methyl and -NR^{m2}Rⁿ².

In some embodiments, R^{m2} and Rⁿ² are methyl.

In some embodiments, the amino acid residue represented by AA¹ is selected from

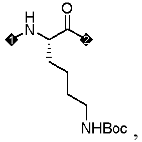

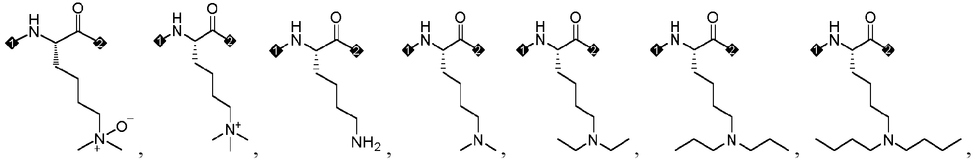

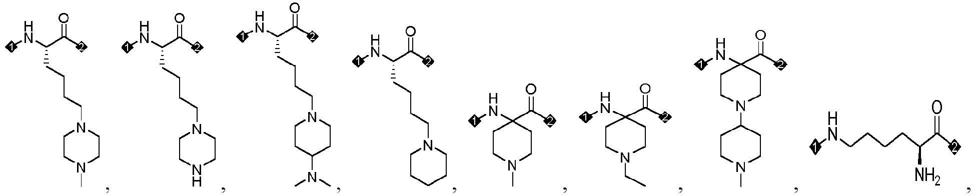

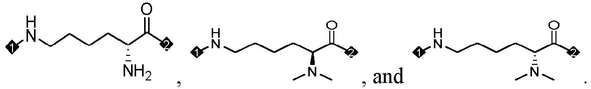

In some embodiments, the amino acid residue represented by AA¹ is selected from

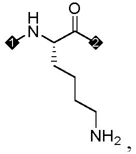

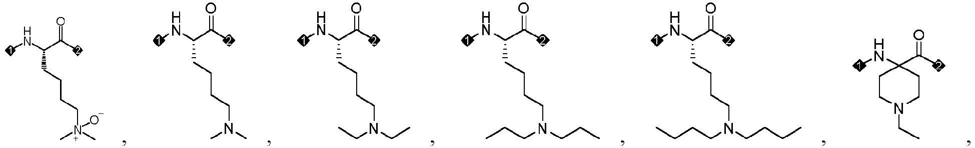

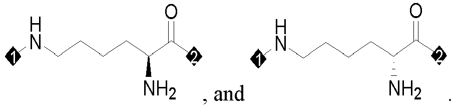

In some embodiments, the amino acid residue represented by AA¹ is selected from

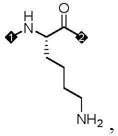

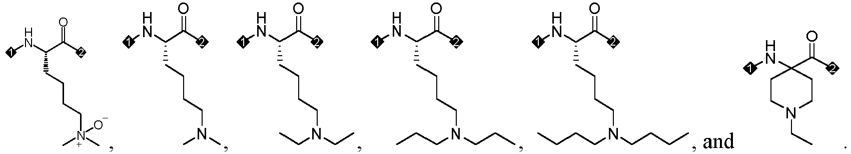

In some embodiments, L₄ is absent or present, and when L₄ is present, L₄ is 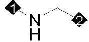 or 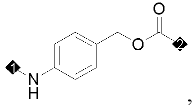 wherein position 1 is attached to L₃, and position 2 is attached to D.

In some embodiments, L₄ is absent.

In some embodiments, L₄ is selected from 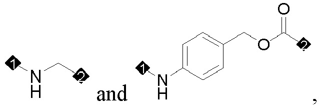 wherein position 1 is attached to L₃, and position 2 is attached to D.

In some embodiments, L₄ is selected from 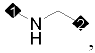 wherein position 1 is attached to L₃, and position 2 is attached to D.

In some embodiments, the structure of 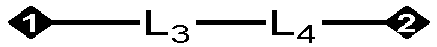 is selected from the following structural fragments:

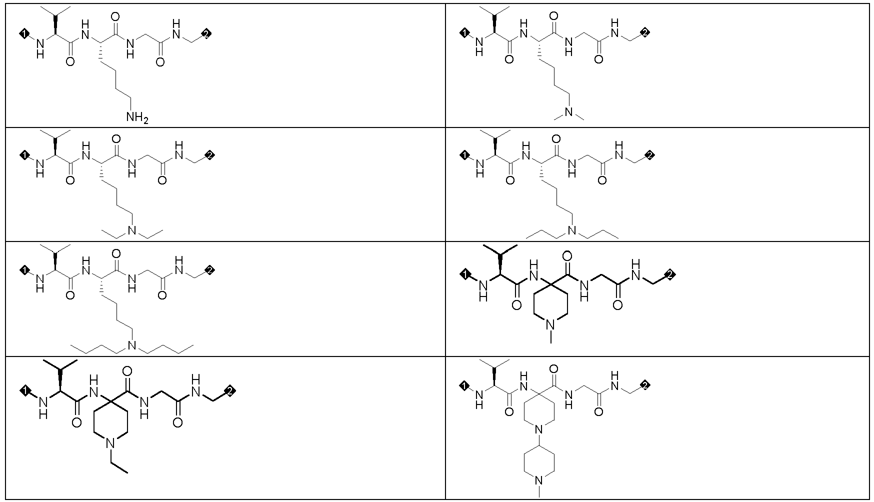

In some embodiments, q is selected from any numerical value between 0.1 and 16.0; in a preferred embodiment, q is selected from any integer between 0.1 and 16.0.

In some embodiments, q is selected from any numerical value between 0.1 and 8.0; in a preferred embodiment, q is selected from any integer between 0.1 and 8.0.

In some embodiments, q is selected from any numerical value between 2 and 8.

In some embodiments, q is selected from any numerical value between 3 and 8.

In some embodiments, q is selected from any numerical value between 4 and 8.

In some embodiments, q is selected from any numerical value between 6 and 8.

In some embodiments, q is selected from any integer between 2 and 8.

In some embodiments, q is selected from any integer between 3 and 8.

In some embodiments, q is selected from any integer between 4 and 8.

In some embodiments, q is selected from any integer between 6 and 8.

In some embodiments, q is selected from 1, 2, 3, 4, 5, 6, 7, 8, 9, 10, 11, and 12.

In some embodiments, q is selected from 2, 4, 6, and 8.

In the present disclosure, the bioactive molecular fragment refers to a moiety (fragment or group) of an antibody-drug conjugate (or referred to as ADC) well known in the art, which is capable of forming a bioactive drug (e.g., a small molecule cytotoxic drug, which comprises the group that results from the loss of an atom or an atomic group) or a derivative thereof (e.g., a precursor thereof) after cleavage/degradation/enzyme digestion of a linker among tumor tissues or inside tumor cells. To avoid ambiguity, "drug" does not refer to only "medicines" that have been approved by the pharmaceutical regulatory authorities, but also includes any molecule with potential therapeutic bioactivity in clinical practice or in research and development as well as in academic studies.

In some embodiments, D is a molecular fragment having anti-tumor bioactivity.

In some embodiments, D is a molecular fragment having anti-tumor bioactivity, wherein the bioactive molecule is selected from a cytotoxic agent and a derivative thereof, such as a DNA topoisomerase inhibitor (e.g., camptothecin bioactive molecule, such as camptothecin, DXD, camptothecin with a modified substituent, or DXD with a modified substituent) or a tubulin inhibitor (e.g., an MMAF tubulin inhibitor or an MMAE tubulin inhibitor).

In some embodiments, the antibody-drug conjugate has a structure of formula I: 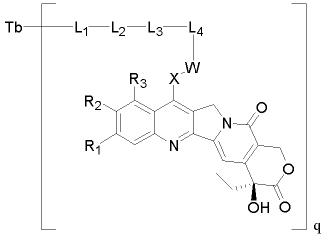 wherein
R₁ and R₂ are each independently selected from H, halogen, -OH, optionally substituted C1-6 alkyl, and optionally substituted C1-6 alkoxy, or
R₁ and R₂, together with the carbon atoms to which they are attached, form a 5-7 membered carbocyclic ring, or a 5-7 membered heterocyclic ring comprising one or more O, S, N, carbonyl, sulfoxide group or sulfone group or any combination thereof;
R₃ is selected from H, halogen, -OH, -NH₂, optionally substituted C1-6 alkyl, and optionally substituted C1-6 alkoxy, or
R₃ and X, together with the carbon atoms to which they are attached, form a 5-7 membered carbocyclic ring, or a 5-7 membered heterocyclic ring comprising one or more O, S, N, carbonyl, sulfoxide group or sulfone group or any combination thereof, or
R₃ and R₂, together with the carbon atoms to which they are attached, form a 5-7 membered carbocyclic ring, or a 5-7 membered heterocyclic ring comprising one or more O, S, N, carbonyl, sulfoxide group or sulfone group or any combination thereof;
W is absent or present, and when W is present, W is selected from -O-, -S-, -NR₄-, 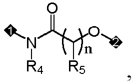 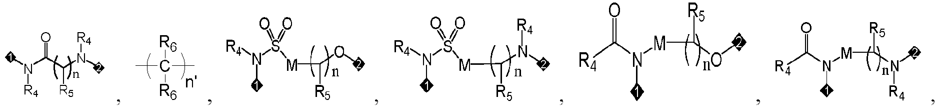 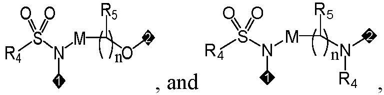 wherein position 1 is attached to X, and position 2 is attached to L₄ or L₃;
X is selected from a direct bond, optionally substituted -O-(CH₂)ₙ₃-, -N(R₄)-(CH₂)ₙ₃-, -S-(CH₂)ₙ₃-, carbonyl-(CH₂)ₙ₃, -SO₂-(CH₂)ₙ₃-, 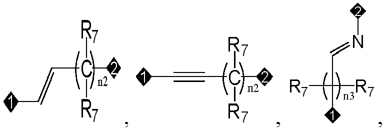 -(CH₂)ₙ₁-, C3-6 cycloalkyl, C6-10 aryl, 5-10 membered heteroaryl, and 4-10 membered heterocyclyl, wherein position 1 is attached to the parent ring, and position 2 is attached to W or L₄; the substituent is selected from one or more C1-4 alkyl, C3-6 cycloalkyl, and C3-6 cycloalkyl formed by multiple C1-4 alkyl together with the carbon atom to which they are simultaneously attached;
each M is independently selected from a direct bond and -CR^{5a}R^{5b}-;
R₄, R₅, R^{5a}, R^{5b}*,* R₆, and R₇ are each independently selected from H, optionally substituted C1-4 alkyl, optionally substituted C1-4 alkoxy, and optionally substituted C3-6 cycloalkyl;
n, n', n1, n2, and n3 are each independently selected from any integer between 0 and 6;
L₄ is absent or present, and when L₄ is present, L₄ is selected from 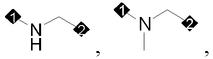 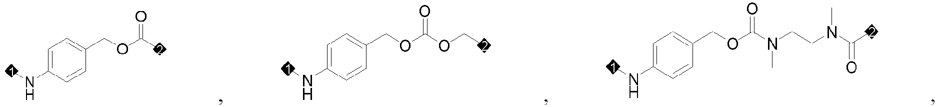 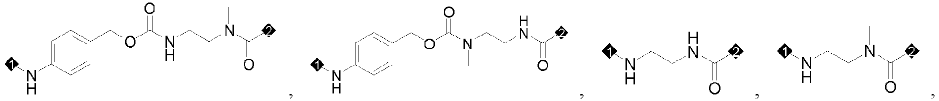 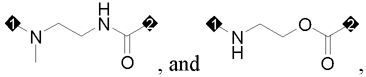 wherein position 1 is attached to L₃, and position 2 is attached to W or X.
Tb, L₁, L₂, L₃, and q are as defined in any embodiment specifically described above and herein.

In some embodiments, R₁ and R₂ are each independently selected from H, halogen, and C1-4 alkyl.

In some embodiments, R₁ and R₂, together with the carbon atoms to which they are attached, form a 5-6 membered heterocyclic ring comprising 1, 2, or 3 O, S, or N, or any combination thereof.

In some embodiments, R₁ is selected from H and halogen, and R₂ is selected from H and C1-4 alkyl.

In some embodiments, R₁ and R₂, together with the carbon atoms to which they are attached, form 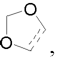 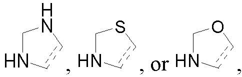 wherein the dotted line indicates the position at which the heterocyclic ring is fused to the phenyl ring.

In some embodiments, R₁ is H or F, and R₂ is H or methyl.

In some embodiments, R₁ is F, and R₂ is methyl, or R₁ and R₂, together with the carbon atoms to which they are attached, form

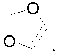

In some embodiments, R₁ is F, and R₂ is methyl.

In some embodiments, R₁ and R₂, together with the carbon atoms to which they are attached, form

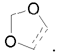

In some embodiments, R₃ is selected from H and C1-4 alkyl.

In some embodiments, R₃ and X, together with the carbon atoms to which they are attached, form a 5-6 membered carbocyclic ring.

In some embodiments, R₃ is H, or R₃ and X, together with the carbon atoms to which they are attached, form 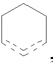 , wherein the dotted line indicates the position at which the carbocyclic ring is fused to the phenyl ring and the pyridine ring.

In some embodiments, R₃ is H.

In some embodiments, W is absent or present, and when W is present, W is selected from -O-, -S-, -NR₄-, 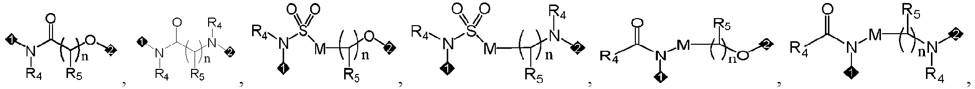 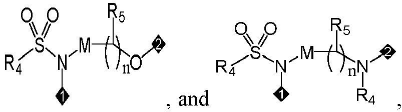 wherein position 1 is attached to X, and position 2 is attached to L₄ or L₃.

In some embodiments, W is absent or present, and when W is present, W is selected from -O-, -S-, -NR₄-, 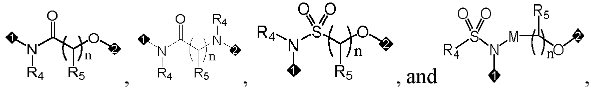 wherein position 1 is attached to X, and position 2 is attached to L₄ or L₃.

In some embodiments, W is selected from -O-, -NR₄-, and 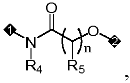 wherein position 1 is attached to X, and position 2 is attached to L₄ or L₃.

In some embodiments, W is selected from -O- and -NR₄-, wherein position 1 is attached to X, and position 2 is attached to L₄ or L₃.

In some embodiments, X is selected from optionally substituted -(CH₂)ₙ₁-, 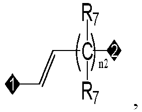 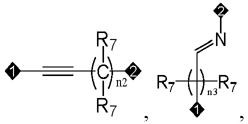 C6-10 aryl, 5-10 membered heteroaryl, and 4-10 membered heterocyclyl, wherein position 1 is attached to the parent ring, and position 2 is attached to W or L₄; the substituent is selected from 1 or 2 C1-4 alkyl, and C3-6 cycloalkyl formed by 2 C1-4 alkyl together with the carbon atom to which they are both attached.

In some embodiments, X is selected from optionally substituted 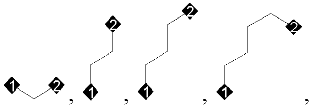 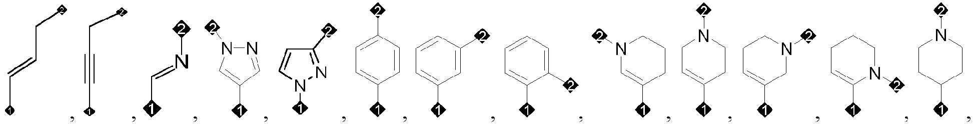 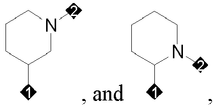 wherein position 1 is attached to the parent ring, and position 2 is attached to W or L₄; the substituent is selected from 1 or 2 C1-4 alkyl (e.g., methyl), and C3-6 cycloalkyl (e.g., cyclopropyl) formed by 2 C1-4 alkyl (e.g., methyl) together with the carbon atom to which they are both attached.

In some embodiments, X is selected from 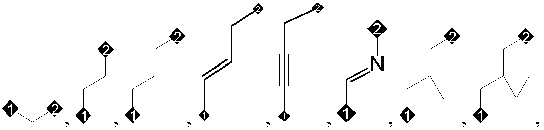 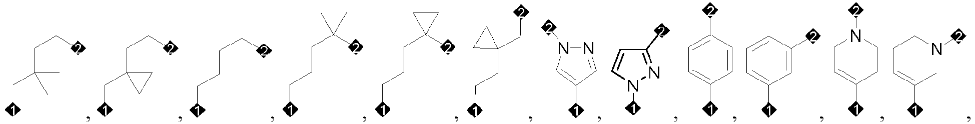 and 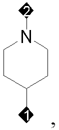 wherein position 1 is attached to the parent ring, and position 2 is attached to W or L₄.

In some embodiments, X is selected from 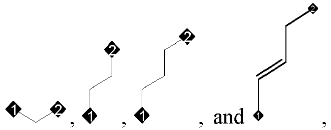 wherein position 1 is attached to the parent ring, and position 2 is attached to W.

In some embodiments, when W is absent, X is selected from 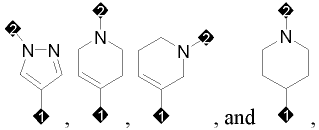 wherein position 1 is attached to the parent ring, and position 2 is attached to L₄; when W is present, X is selected from 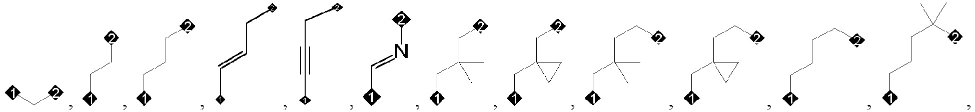 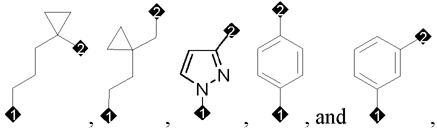 wherein position 1 is attached to the parent ring, and position 2 is attached to W.

In some embodiments, W is selected from -O-, -NR₄-, and 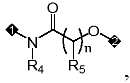 wherein position 1 is attached to X, and position 2 is attached to L₄ or L₃; X is selected from 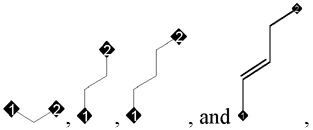 wherein position 1 is attached to the parent ring, and position 2 is attached to W.

In some embodiments, R₄ and R₅ are each independently selected from H, C1-4 alkyl, and C3-6 cycloalkyl.

In some embodiments, each R₄ is independently selected from H, C1-4 alkyl, and C3-6 cycloalkyl, and R₅ is H.

In some embodiments, each R₄ is independently selected from H, methyl, ethyl, n-propyl, isopropyl, *tert-*butyl, and cyclopropyl, and R₅ is H.

In some embodiments, R^{5a} and R^{5b} are each independently selected from H and C1-4 alkyl.

In some embodiments, R^{5a} and R^{5b} are each independently selected from H and methyl.

In some embodiments, each R₇ is independently selected from H and C1-4 alkyl.

In some embodiments, R₇ is H.

In some embodiments, n is selected from 1, 2, and 3.

In some embodiments, n is 1.

In some embodiments, n1 is selected from 1, 2, 3, and 4.

In some embodiments, n2 is 1.

In some embodiments, n3 is 0.

In some embodiments, L₃ is selected from 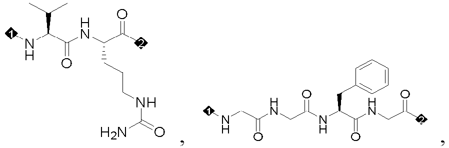 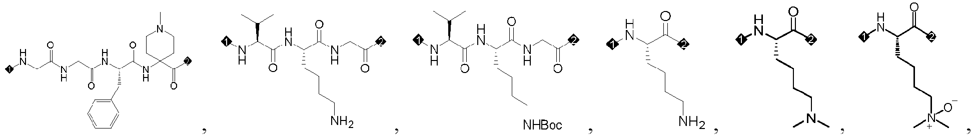 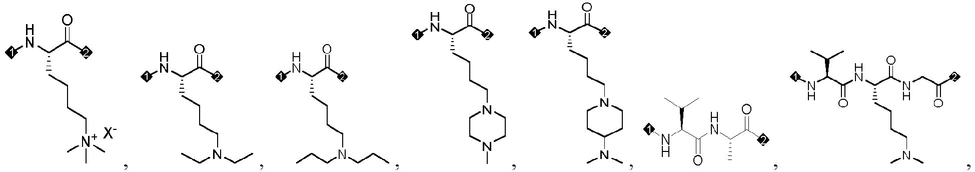 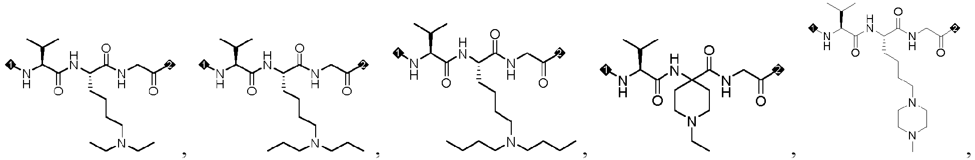 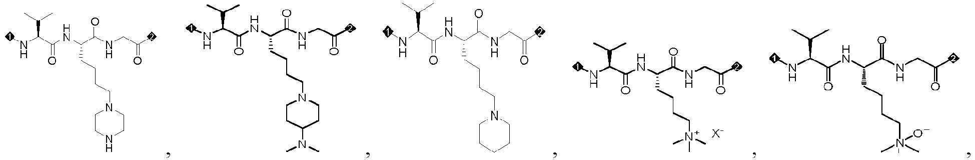 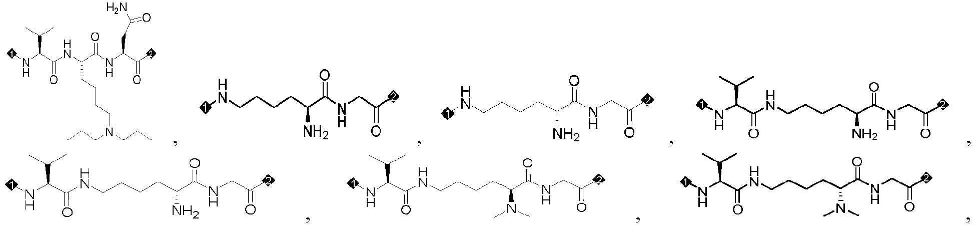 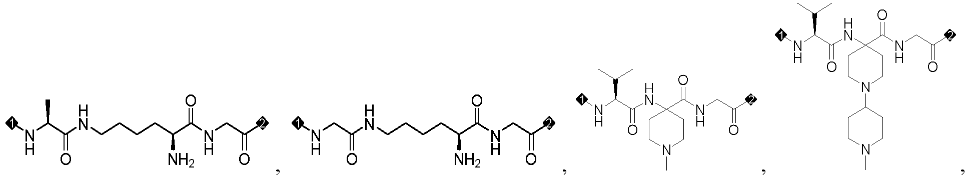 and 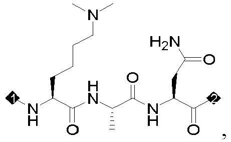 wherein X⁻ is selected from a halide ion, a carboxylate ion, a sulfate ion, a hydrogen sulfate ion, and OH⁻, position 1 is attached to L₁ or L₂, and position 2 is attached to L₄ or W.

In some embodiments, L₃ is selected from 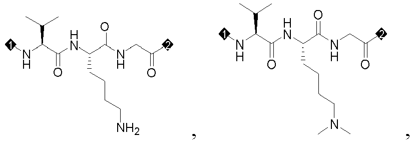 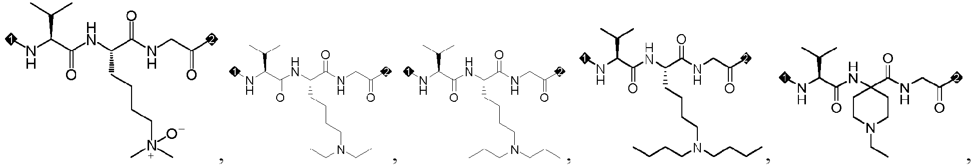 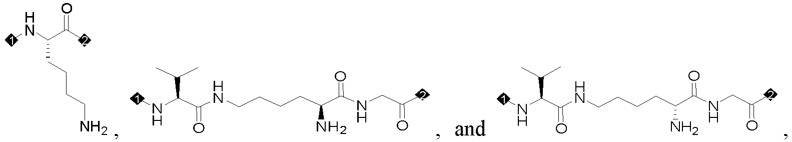 wherein position 1 is attached to L₁ or L₂, and position 2 is attached to L₄ or W.

In some embodiments, L₃ is selected from 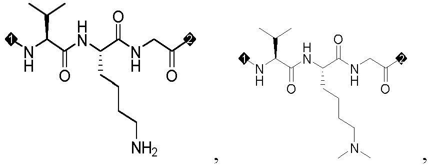 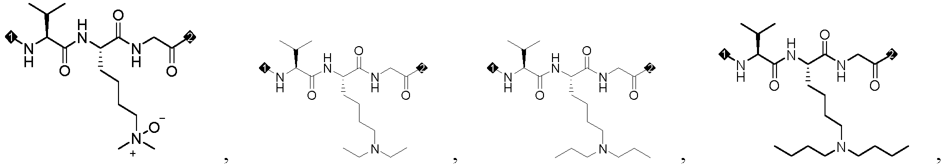 and 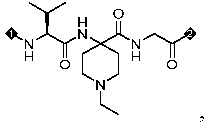 wherein position 1 is attached to L₁ or L₂, and position 2 is attached to L₄ or W.

In some embodiments, L₄ is absent or present, and when L₄ is present, L₄ is 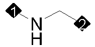 or 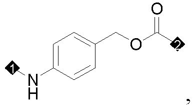 wherein position 1 is attached to L₃, and position 2 is attached to W or X.

In some embodiments, L₄ is absent.

In some embodiments, L₄ is selected from 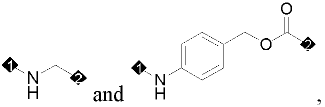 wherein position 1 is attached to L₃, and position 2 is attached to W or X.

In some embodiments, L₄ is selected from 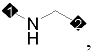 wherein position 1 is attached to L₃, and position 2 is attached to W or X.

It should be noted that, as previously mentioned, W is absent or present, and thus, when W is absent, position 1 of L₄ is attached to L₃, and position 2 of L₄ is attached to X; when W is present, position 1 of L₄ is attached to L₃, and position 2 of L₄ is attached to W. The following attachment relationships of L₄ can be understood with reference to the aforementioned content.

In some embodiments, the structure of 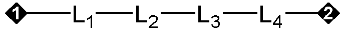 is selected from the following structural fragments:

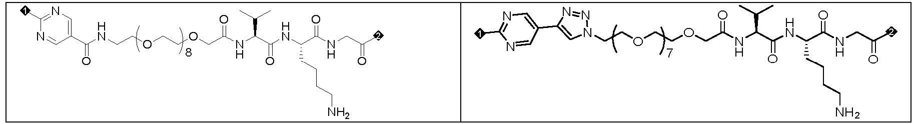

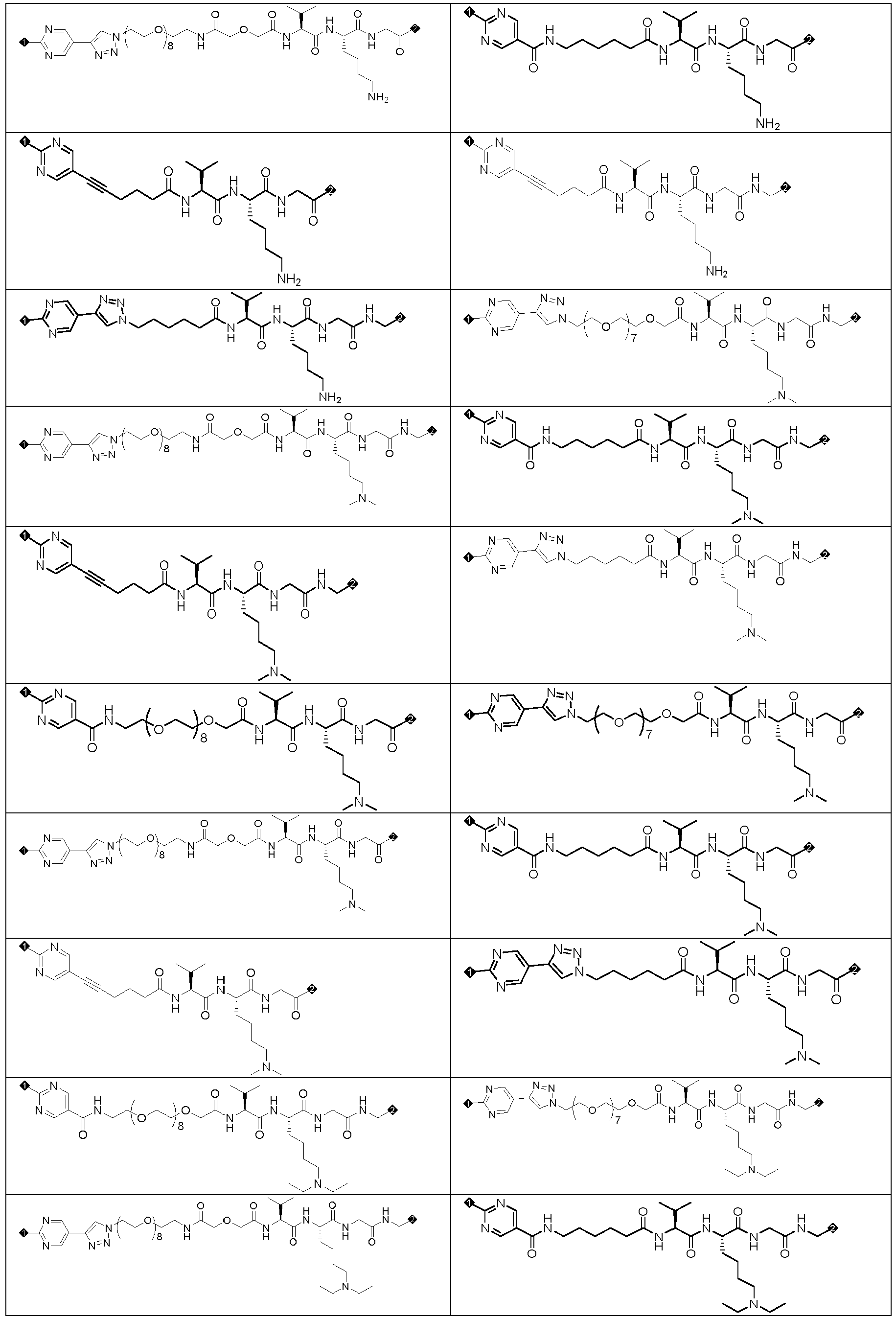

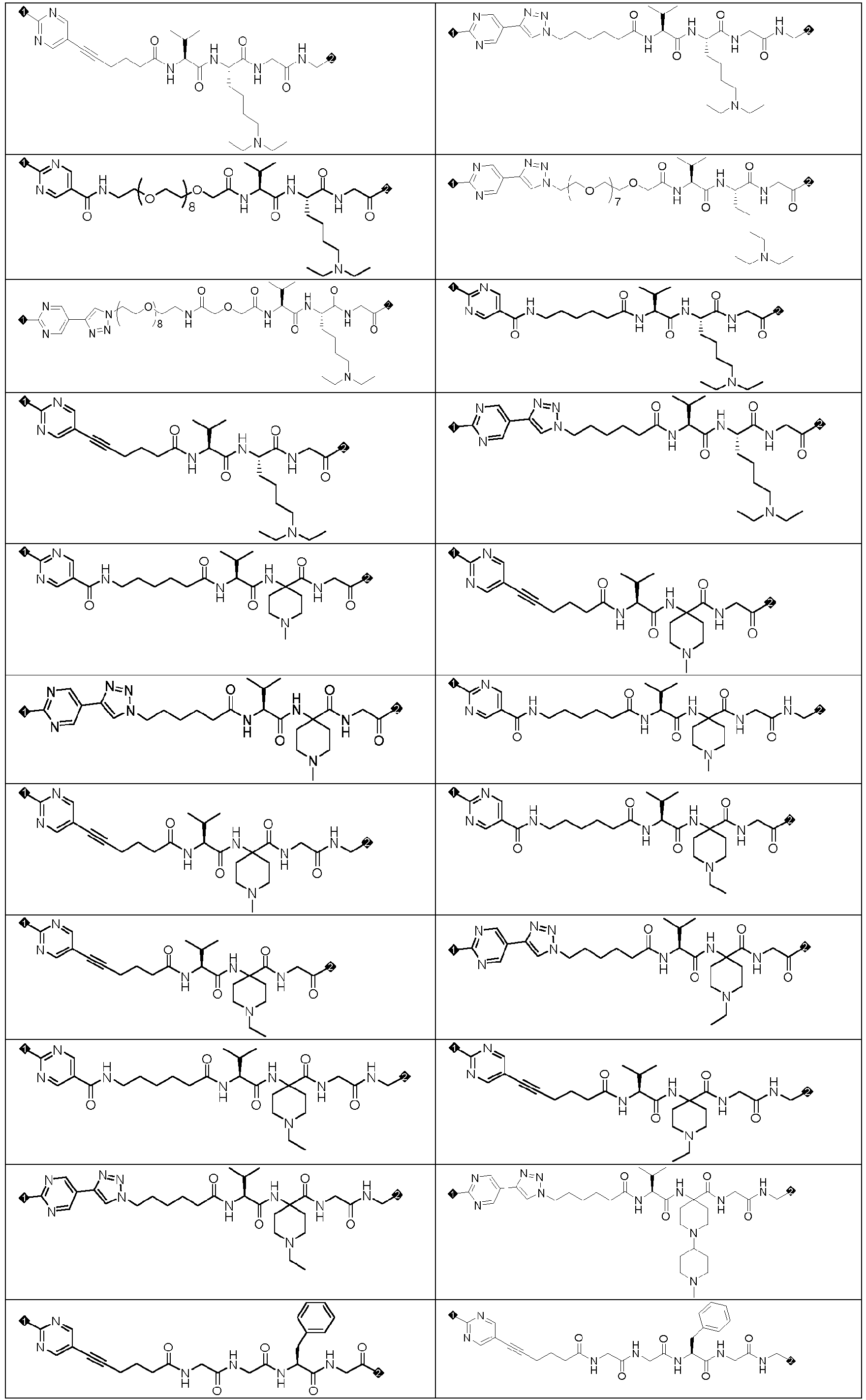

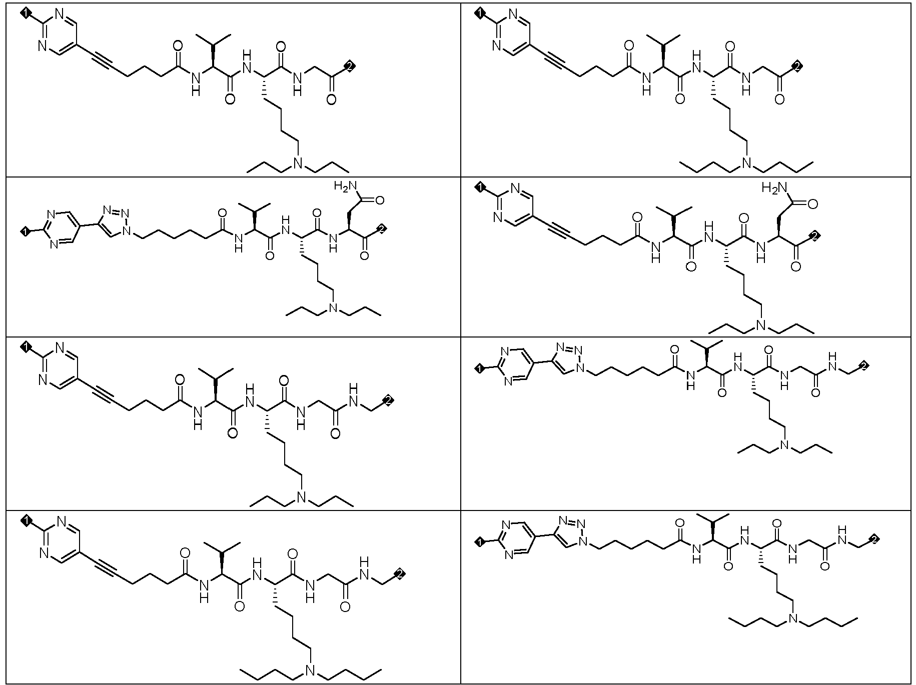

wherein position 1 is attached to Tb, and position 2 is attached to W.

In some embodiments, D is a structural fragment of 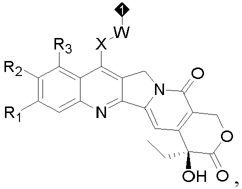 wherein position 1 is attached to L₃ or L₄; for example, D is 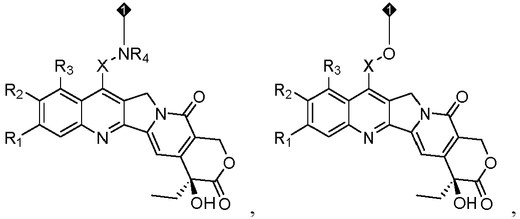 or

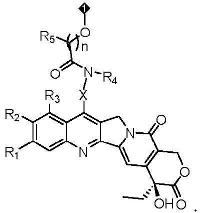

In some embodiments, the structure of 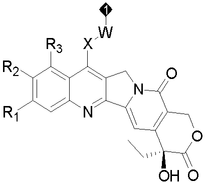 is selected from the following structural fragments: 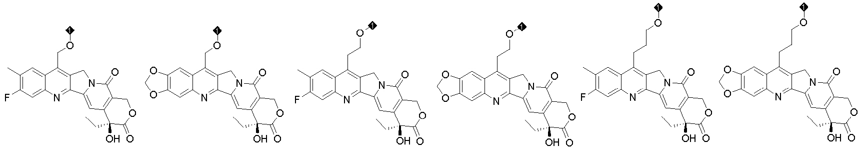 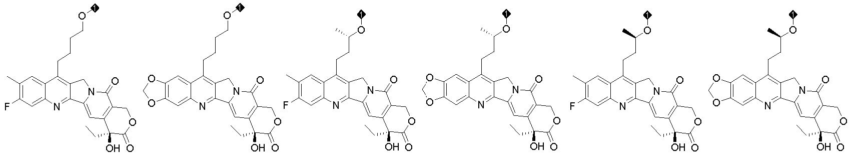 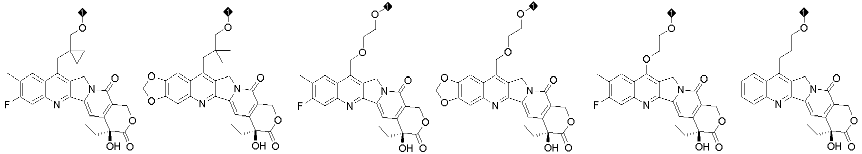 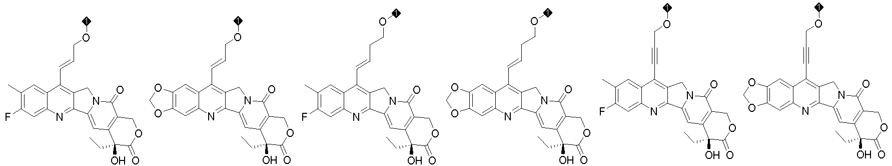 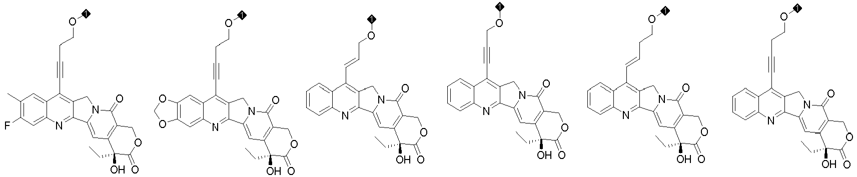 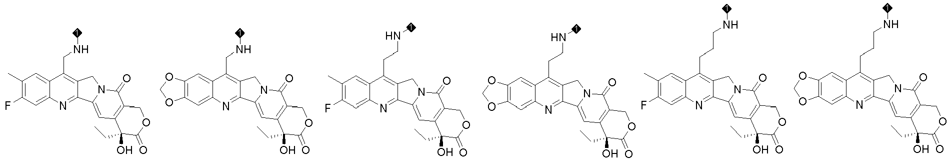 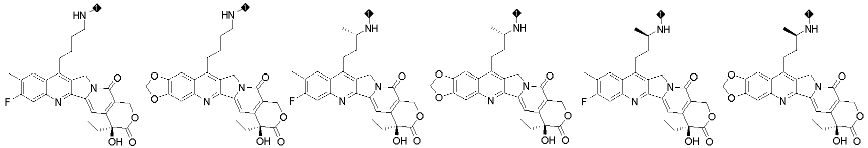 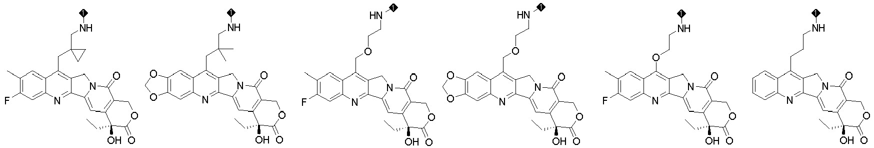 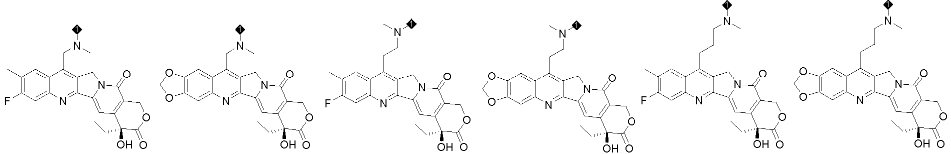 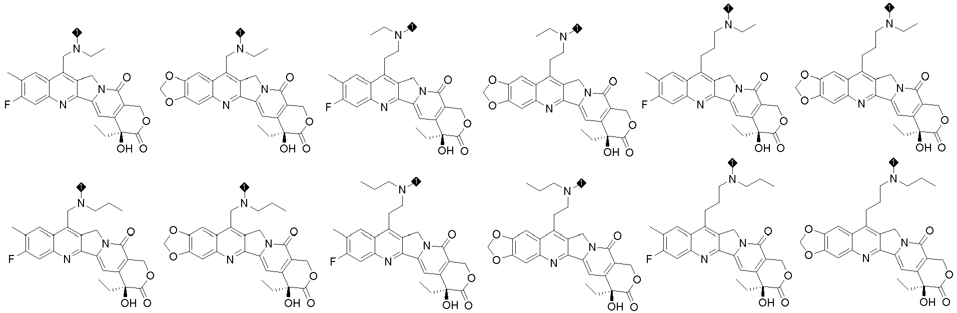 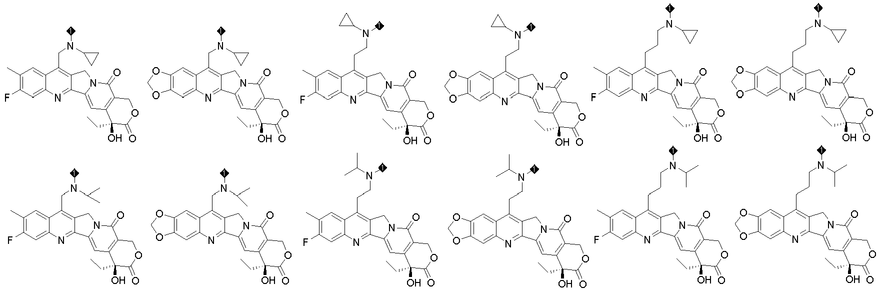 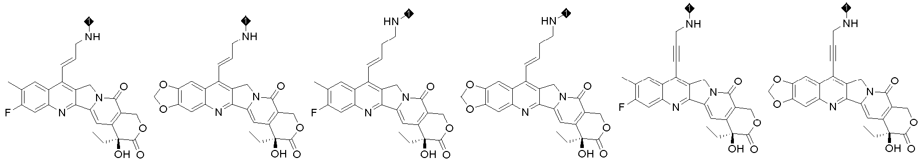 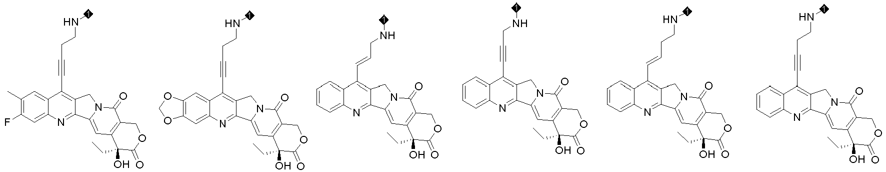 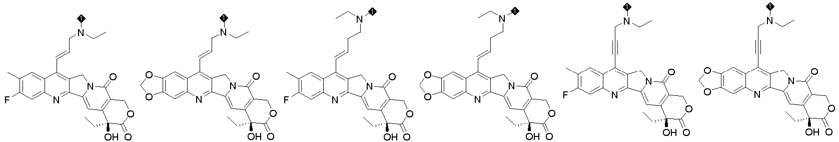 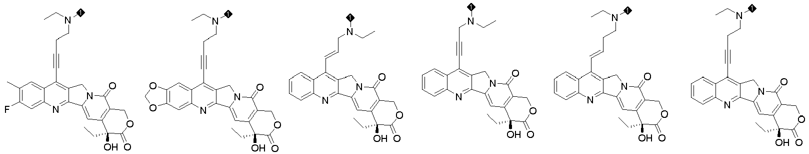 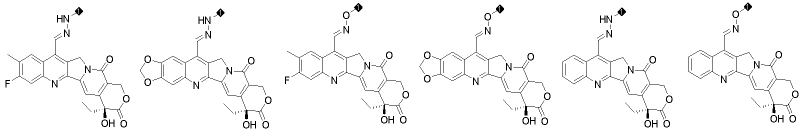 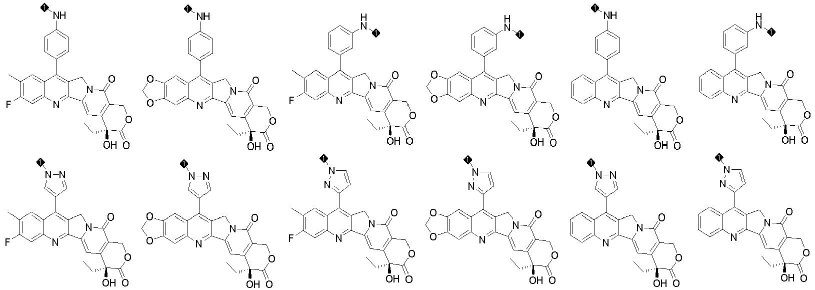 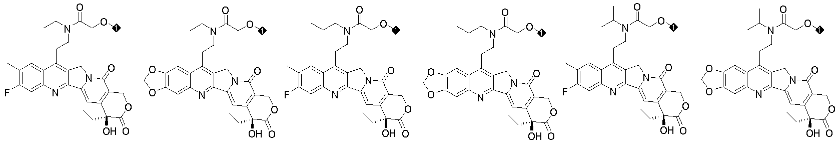 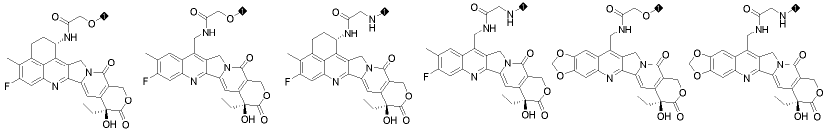 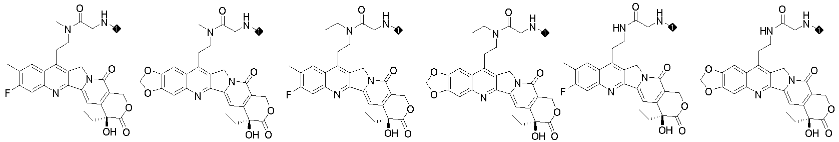 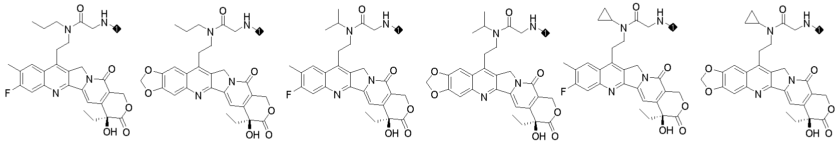 wherein position 1 is attached to L₄; when L₄ is absent, position 1 is attached to L₃.

In some embodiments,
W is absent or present, and when W is present, W is selected from -O-, -S-, -NR₄-, 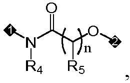 and 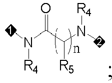 for example, W is absent, or is -O-, -NR₄-, or 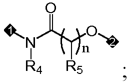 R₄ and R₅ are each independently selected from H and C1-4 alkyl; n is independently selected from 0, 1, 2, 3, and 4;
X is selected from 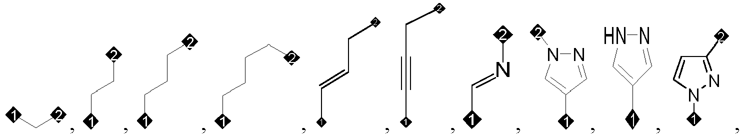 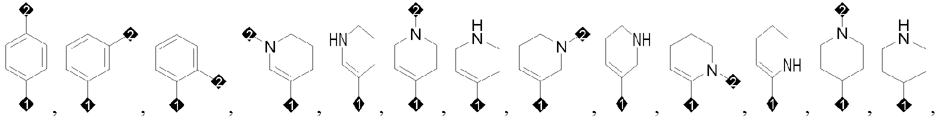 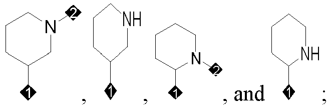 R₁ is selected from H and halogen, and R₂ is selected from H and C1-4 alkyl, or R₁ and R₂, together with the carbon atoms to which they are attached, form 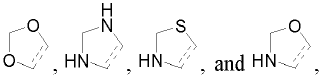 wherein the dotted line indicates the position at which the heterocyclic ring is fused to the phenyl ring;
R₃ is selected from H and C1-4 alkyl, or R₃ and X, together with the carbon atoms to which they are attached, form a 5-6 membered carbocyclic ring;
preferably, W is absent or present, and when W is present, W is selected from -O-, -NR₄- (e.g., -NH-, - N(CH₃)-, and -N(C₂H₅)-), 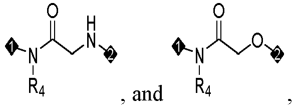 wherein R₄ is independently selected from H, methyl, ethyl, isopropyl, n-propyl, *tert-*butyl*,* and cyclopropyl;
when W is absent, X is selected from 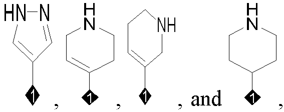 wherein position 1 is attached to the parent ring; when W is present, X is selected from 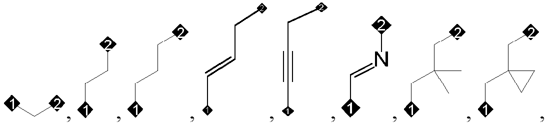 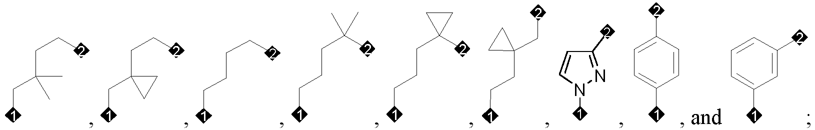 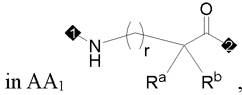
r is selected from 0, 1, 2, 3, 4, and 5;
either R^{a} or R^{b} is H, and the other is selected from 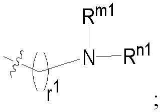 or R^{a} and R^{b}, together with the carbon atom to which they are both attached, form a 5-6 membered heterocyclic ring substituted with R⁰;
R₁ is selected from H and halogen, and R₂ is selected from H and C1-4 alkyl, or R₁ and R₂, together with the carbon atoms to which they are attached, form 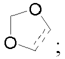
R₃ is selected from H and C1-4 alkyl, or R₃ and X, together with the carbon atoms to which they are attached, form R₃ and X, together with the carbon atoms to which they are attached, form 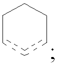
more preferably, W is selected from -O- and -NR₄-, wherein position 1 is attached to X, and position 2 is attached to L₄ or L₃,
X is selected from 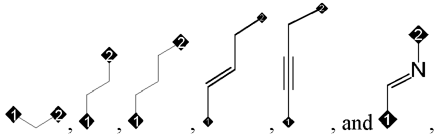 wherein position 1 is attached to the parent ring, and position 2 is attached to W; 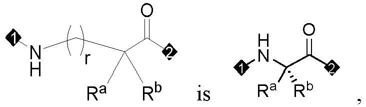
such as 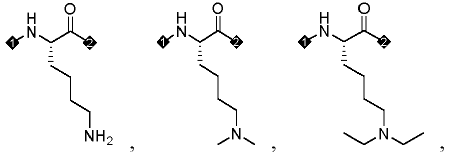 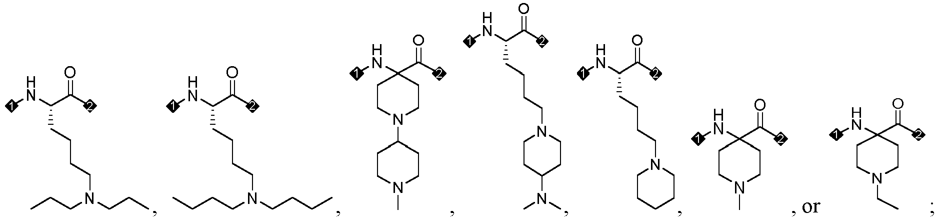
R₁ is F, and
R₂ is methyl, or R₁ and R₂, together with the carbon atoms to which they are attached, form 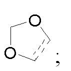 ;
R₃ is H.

In some embodiments, the antibody-drug conjugate has a structure of formula I-1: 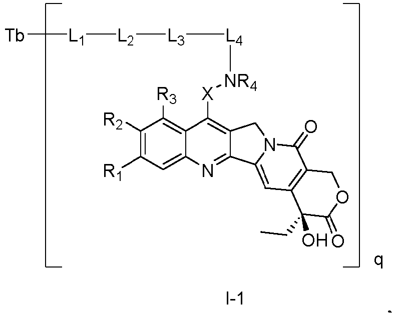 wherein Tb, L₁, L₂, L₃, L₄, X, R₁, R₂, R₃, R₄, and q are as defined in any embodiment specifically described above and herein.

In some embodiments, the antibody-drug conjugate has a structure of formula I-1A or I-1B: 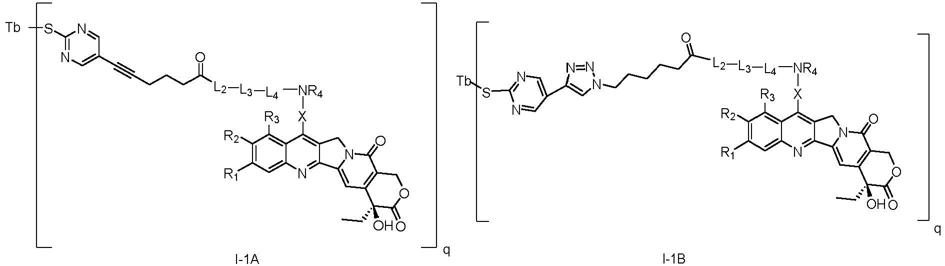 wherein Tb, L₂, L₃, L₄, X, R₁, R₂, R₃, R₄, and q are as defined in any embodiment specifically described above and herein.

In some embodiments, the antibody-drug conjugate has a structure of formula I-2: 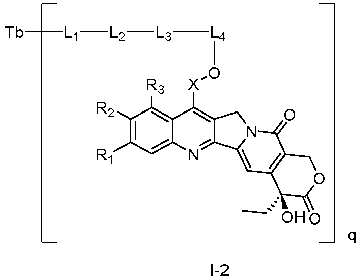 wherein Tb, L₁, L₂, L₃, L₄, X, R₁, R₂, R₃, and q are as defined in any embodiment specifically described above and herein.

In some embodiments, the antibody-drug conjugate has a structure of formula I-2A or I-2B: 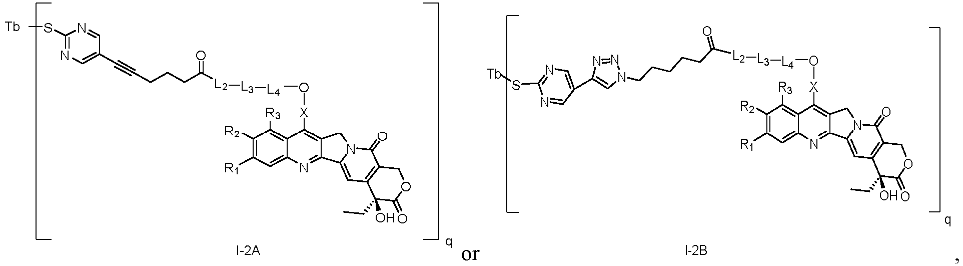 wherein Tb, L₂, L₃, L₄, X, R₁, R₂, R₃, and q are as defined in any embodiment specifically described above and herein.

In some embodiments, the antibody-drug conjugate has a structure of formula I-3: 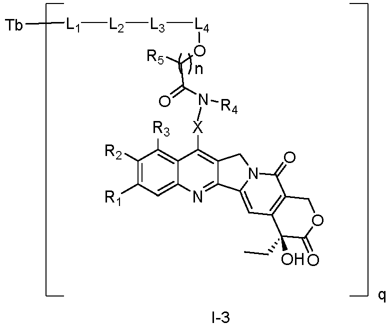 wherein Tb, L₁, L₂, L₃, L₄, X, R₁, R₂, R₃, R₄, R₅, n, and q are as defined in any embodiment specifically described above and herein.

In some embodiments, the antibody-drug conjugate has a structure of formula I-3A or I-3B: 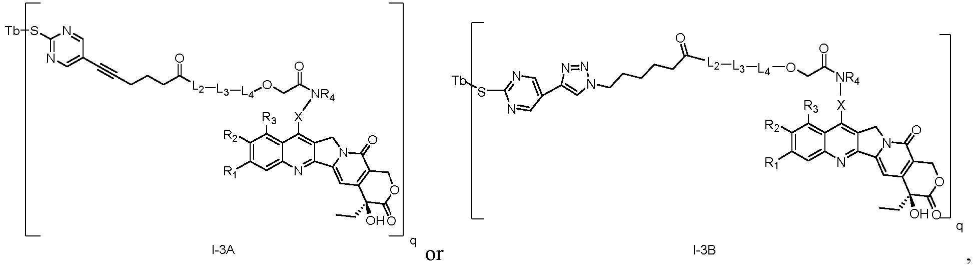 wherein Tb, L₂, L₃, L₄, X, R₁, R₂, R₃, R₄, and q are as defined in any embodiment specifically described above and herein.

In some embodiments, the antibody-drug conjugate has a structure of formula I-A: 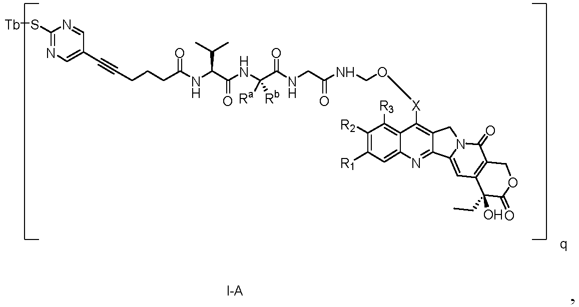 wherein Tb, X, R₁, R₂, R₃, R^{a}, R^{b}, and q are as defined in any embodiment specifically described above and herein.

In some embodiments, the antibody-drug conjugate has a structure of formula I-B: 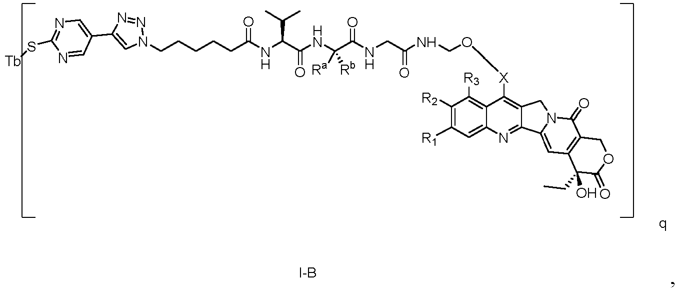 wherein Tb, X, R₁, R₂, R₃, R^{a}, R^{b}, and q are as defined in any embodiment specifically described above and herein.

In some embodiments, the antibody-drug conjugate is selected from the following:

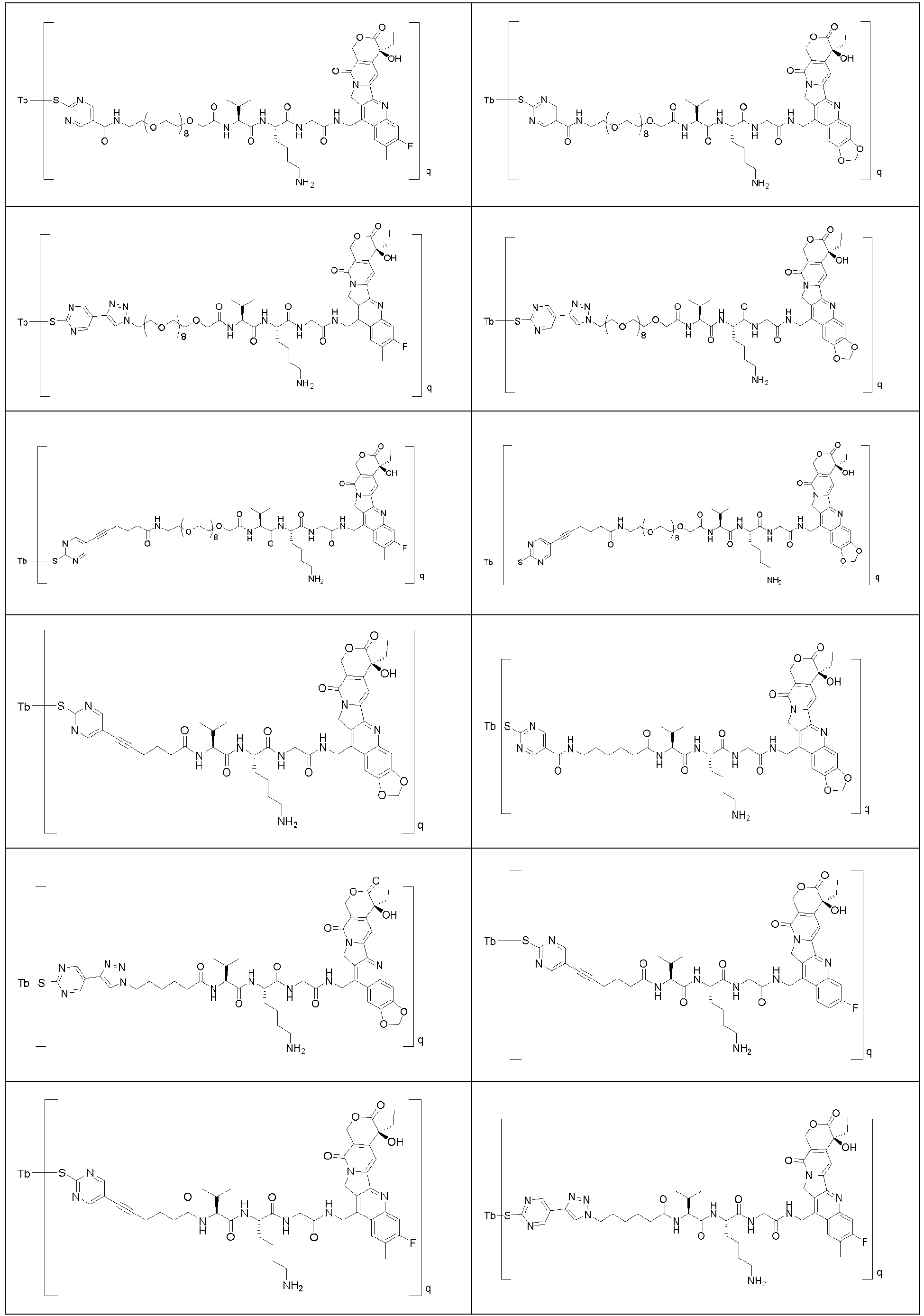

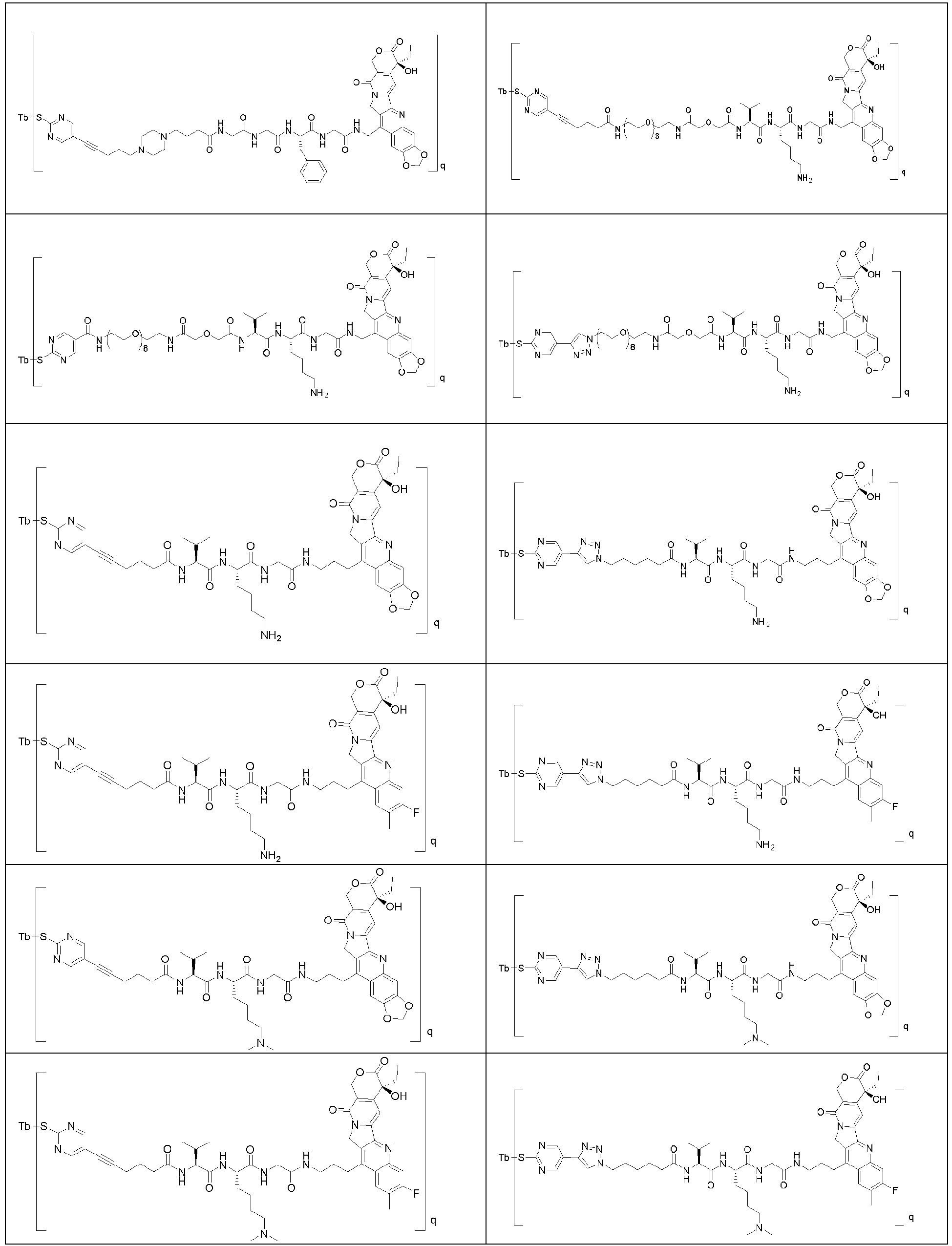

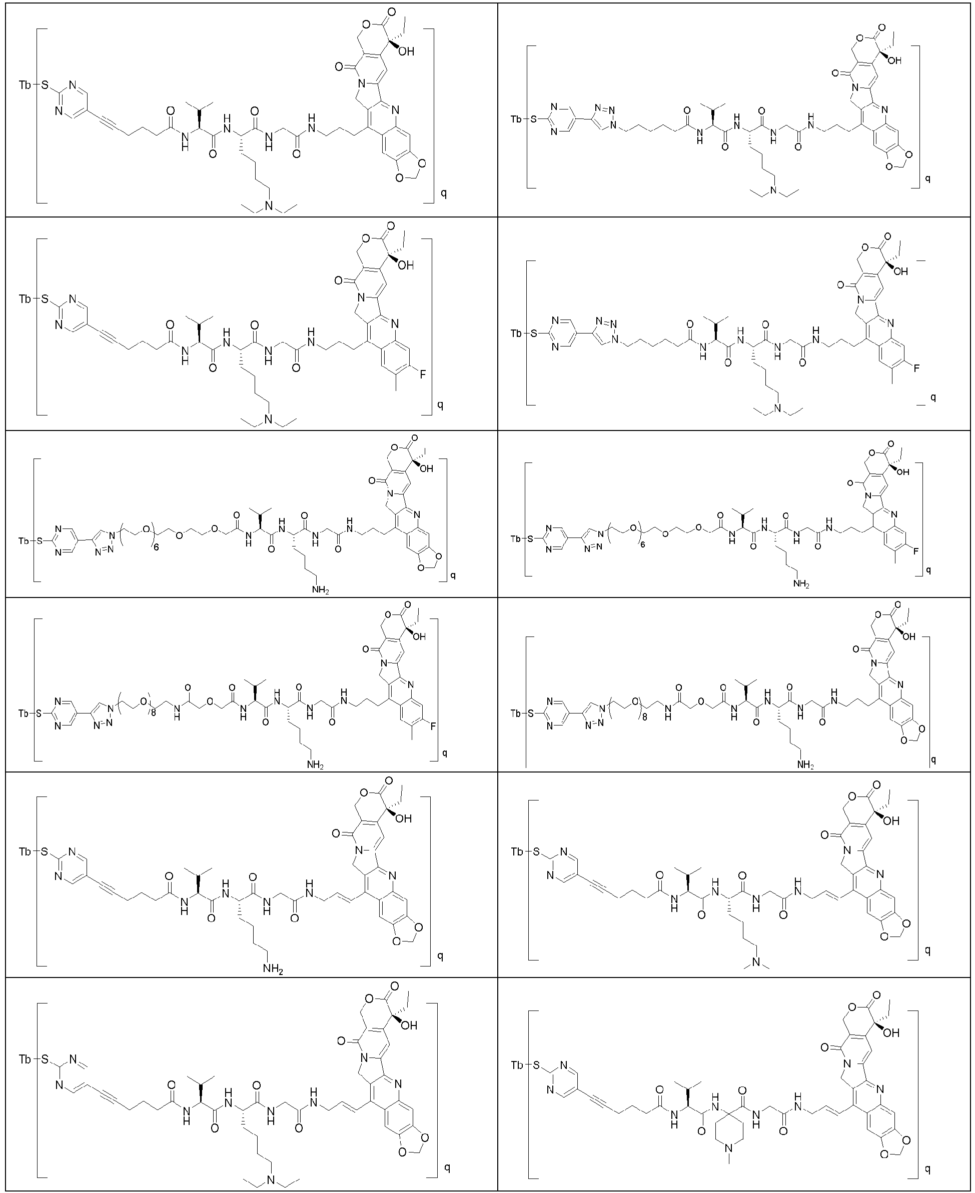

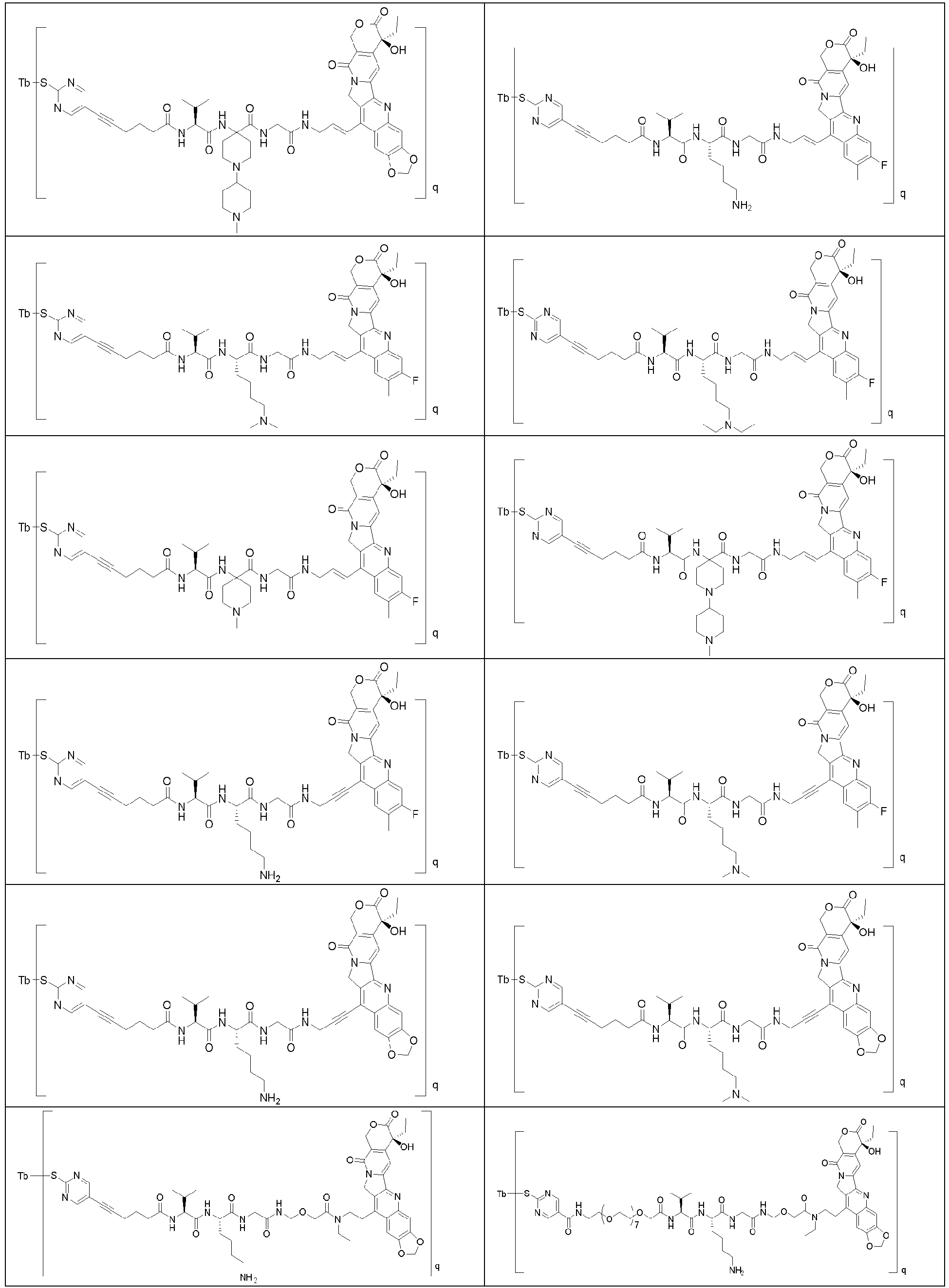

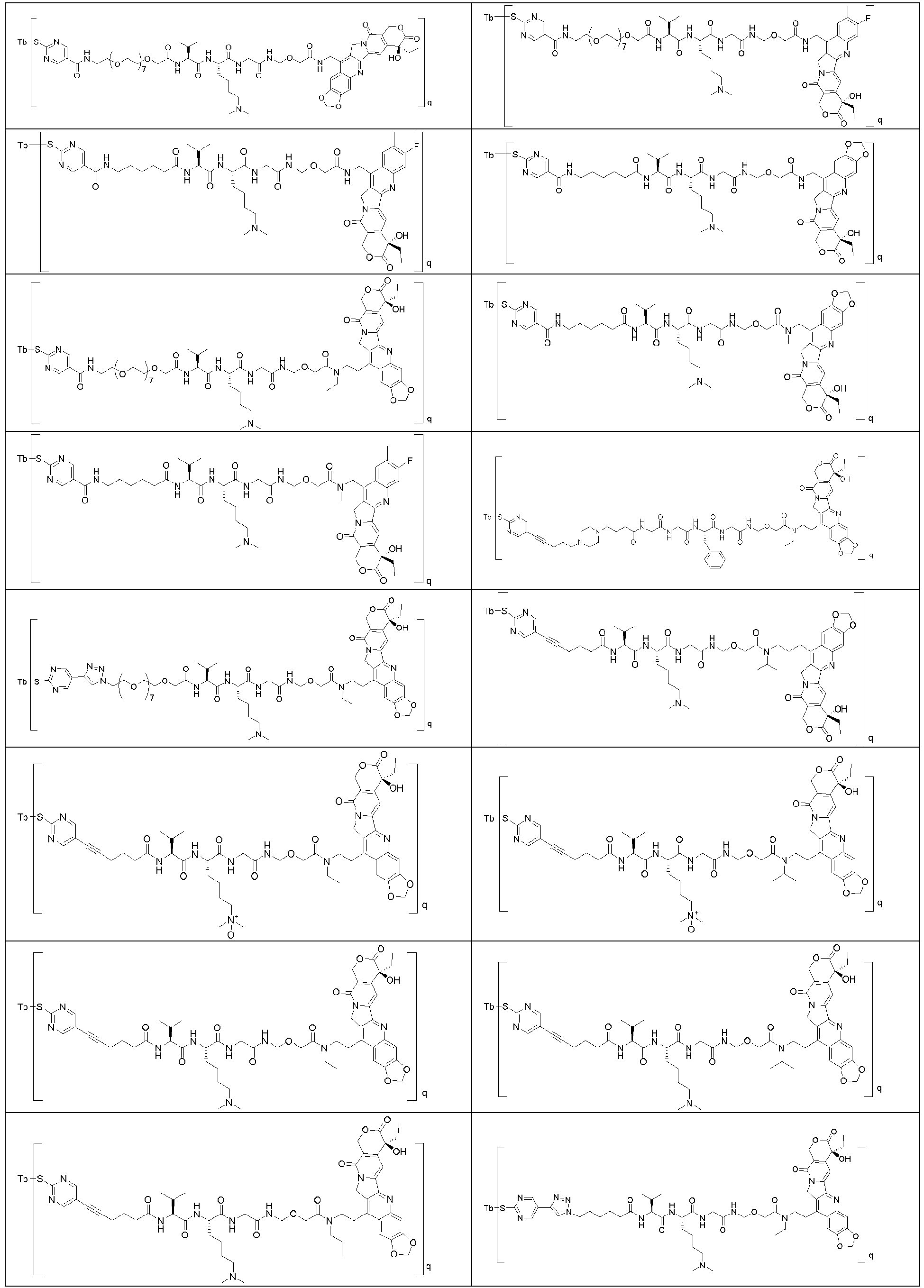

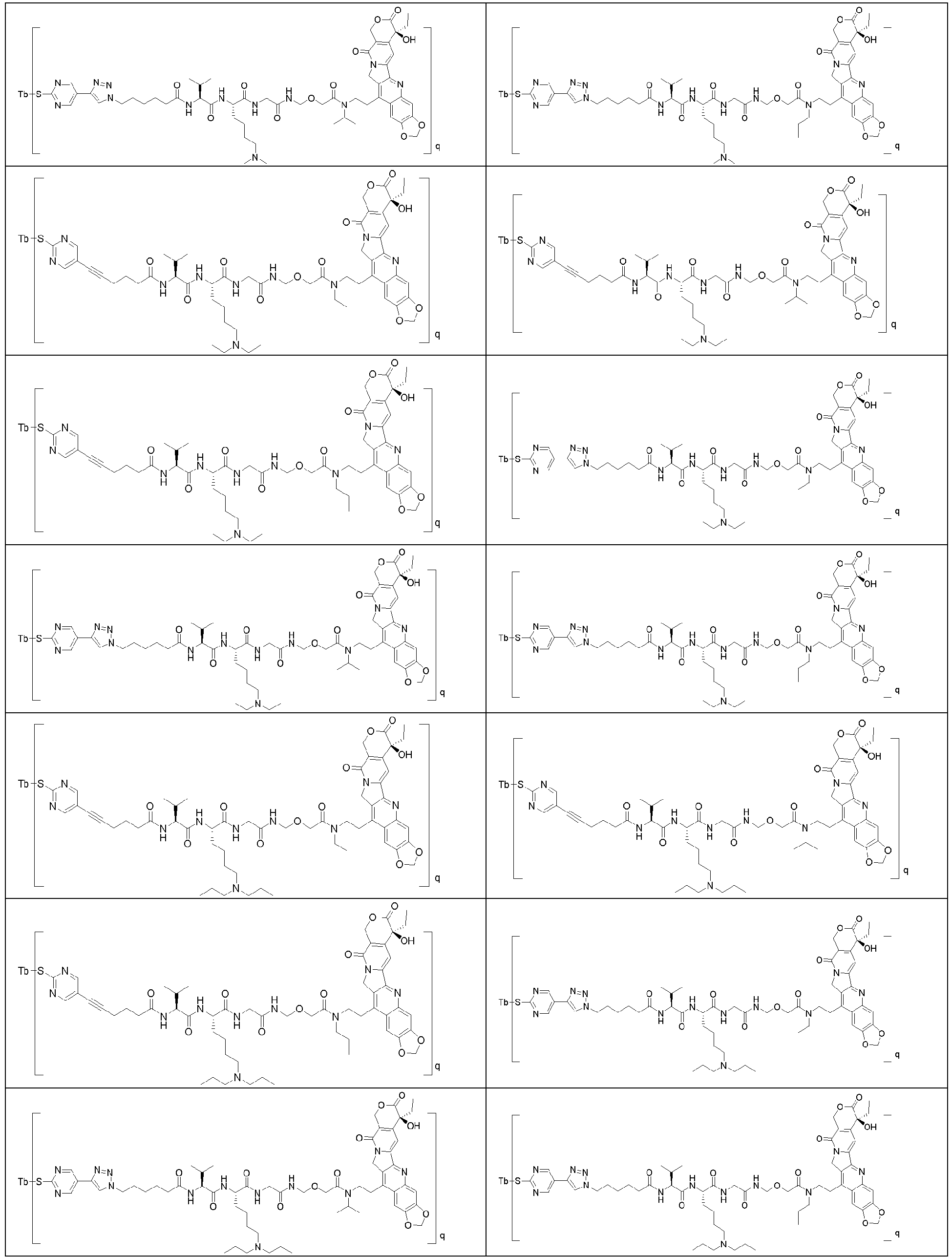

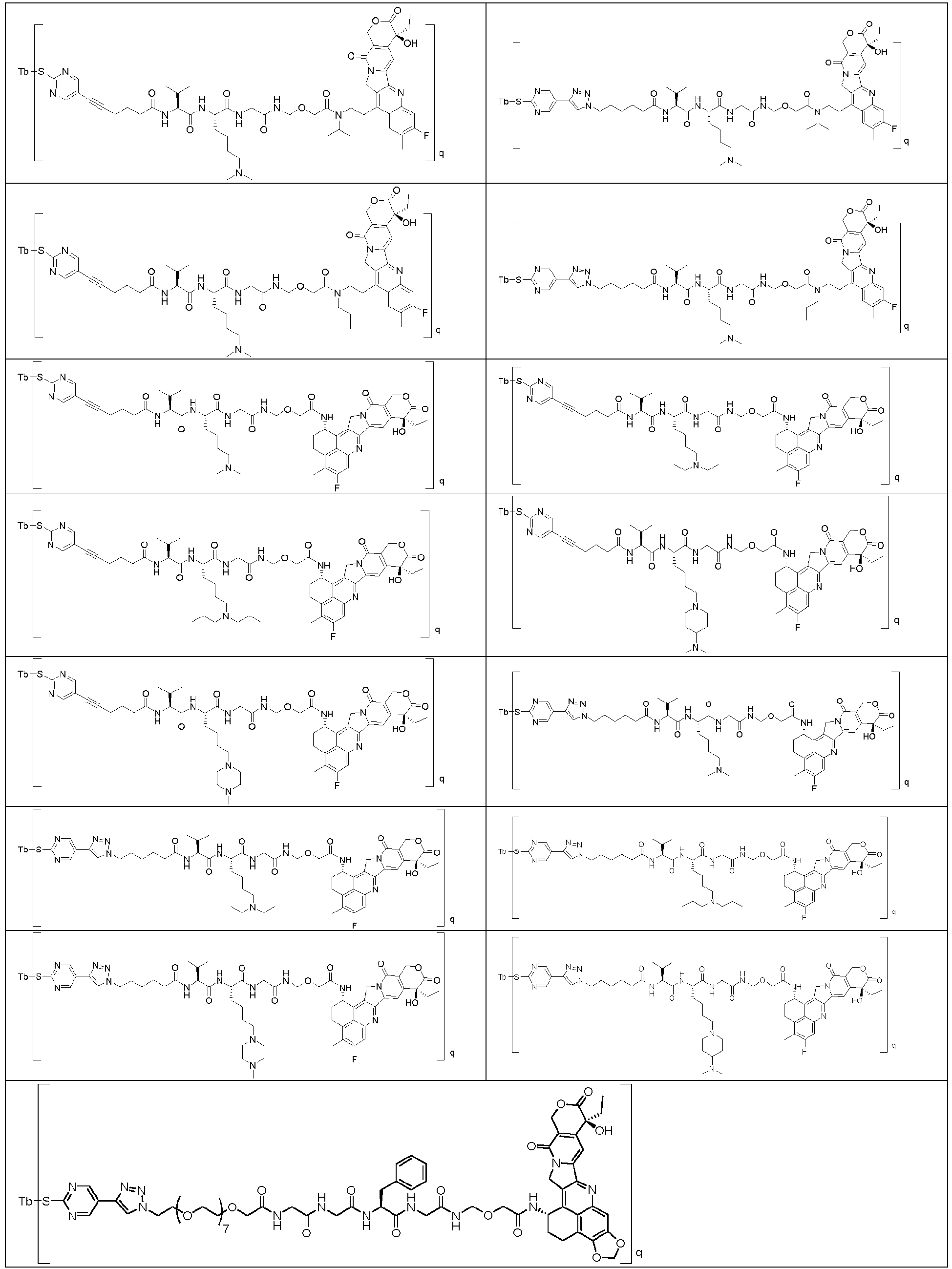

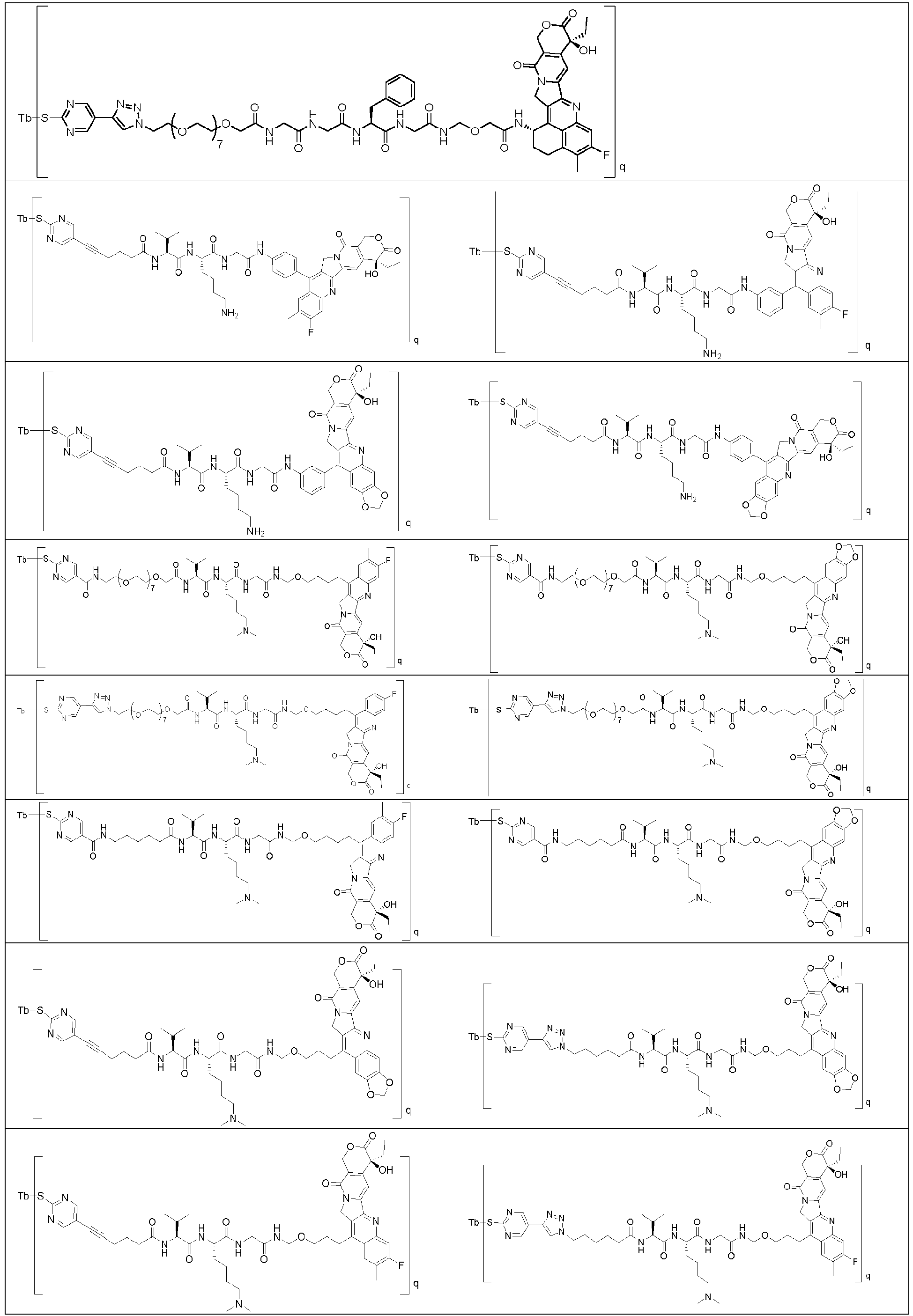

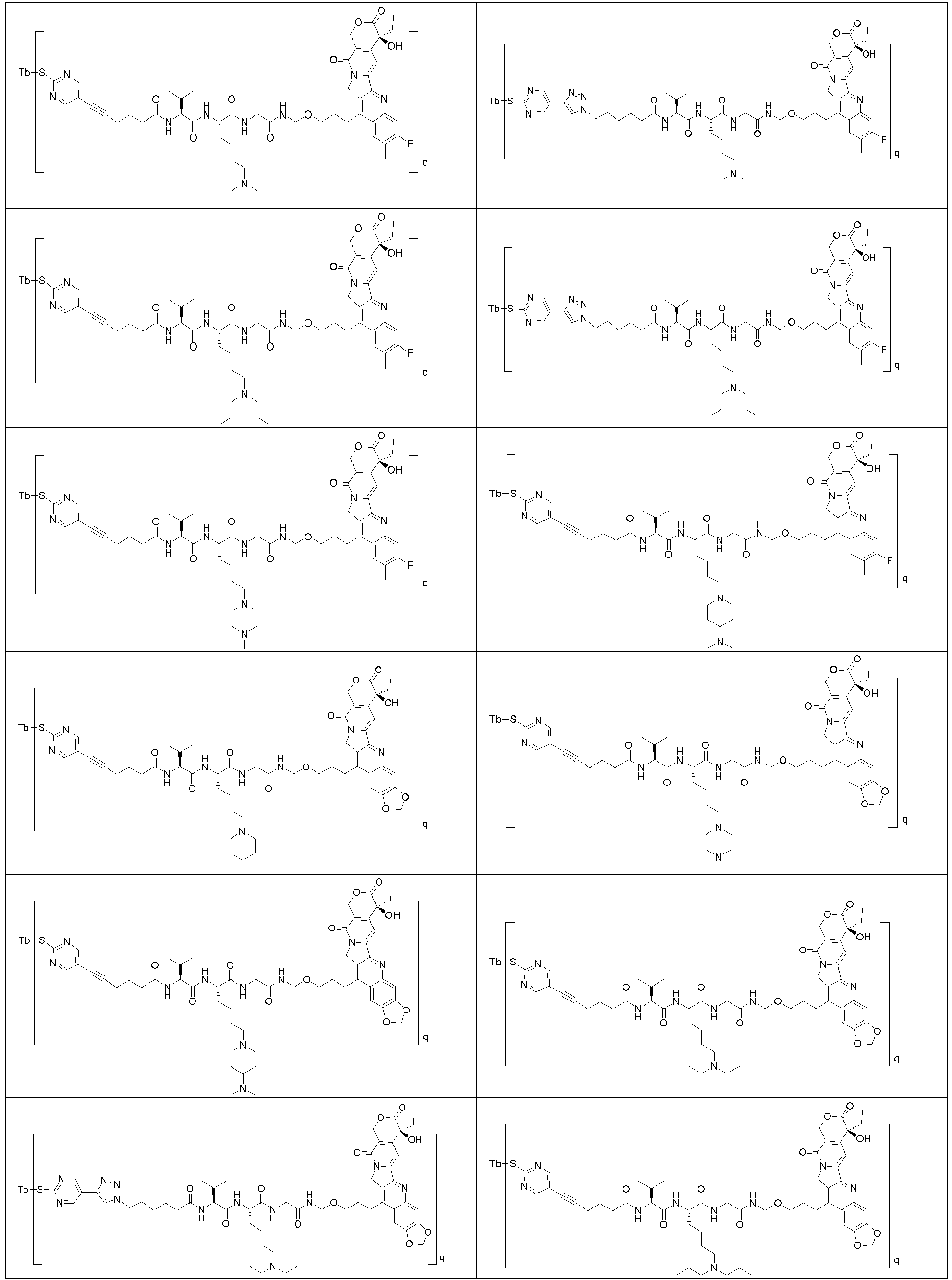

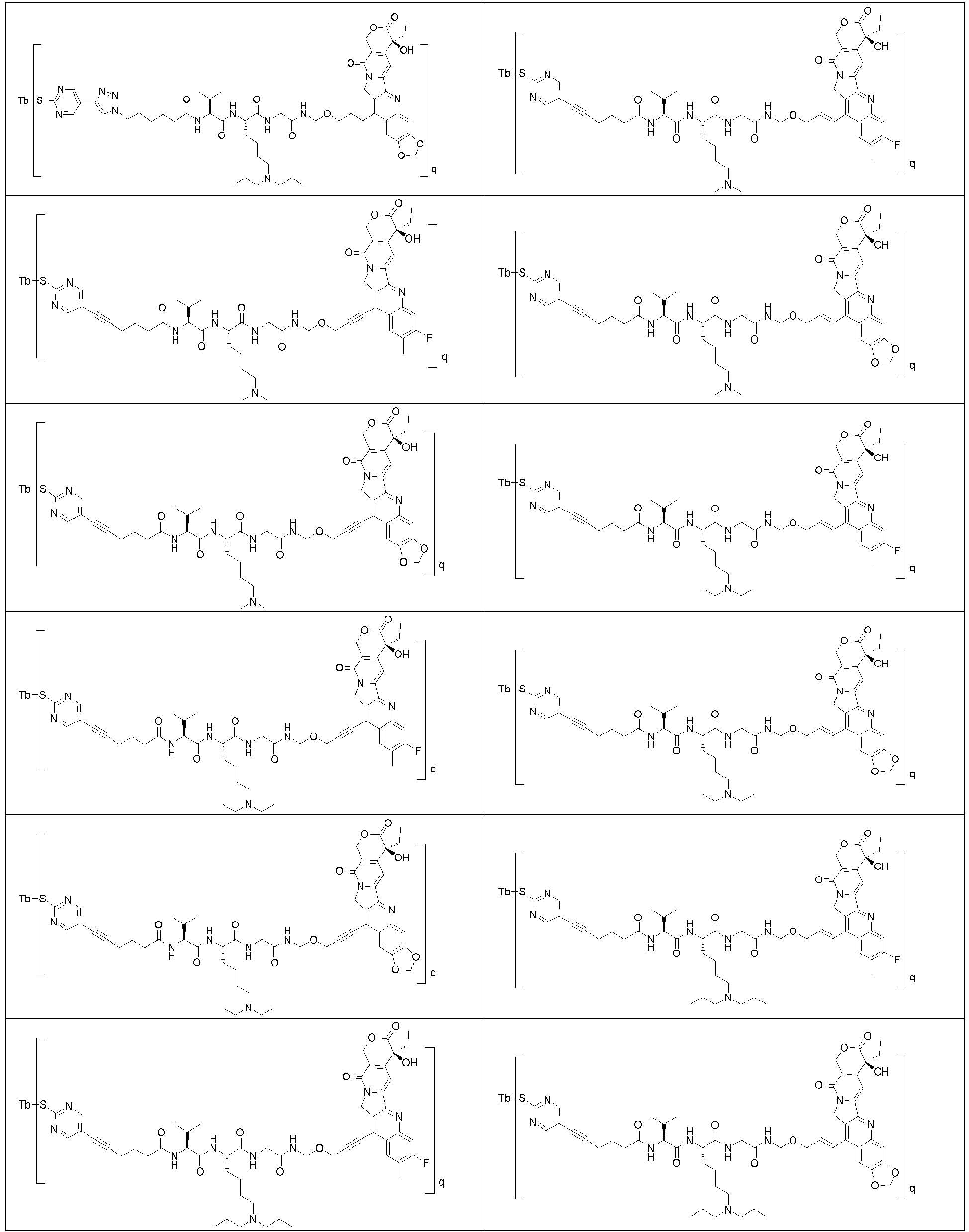

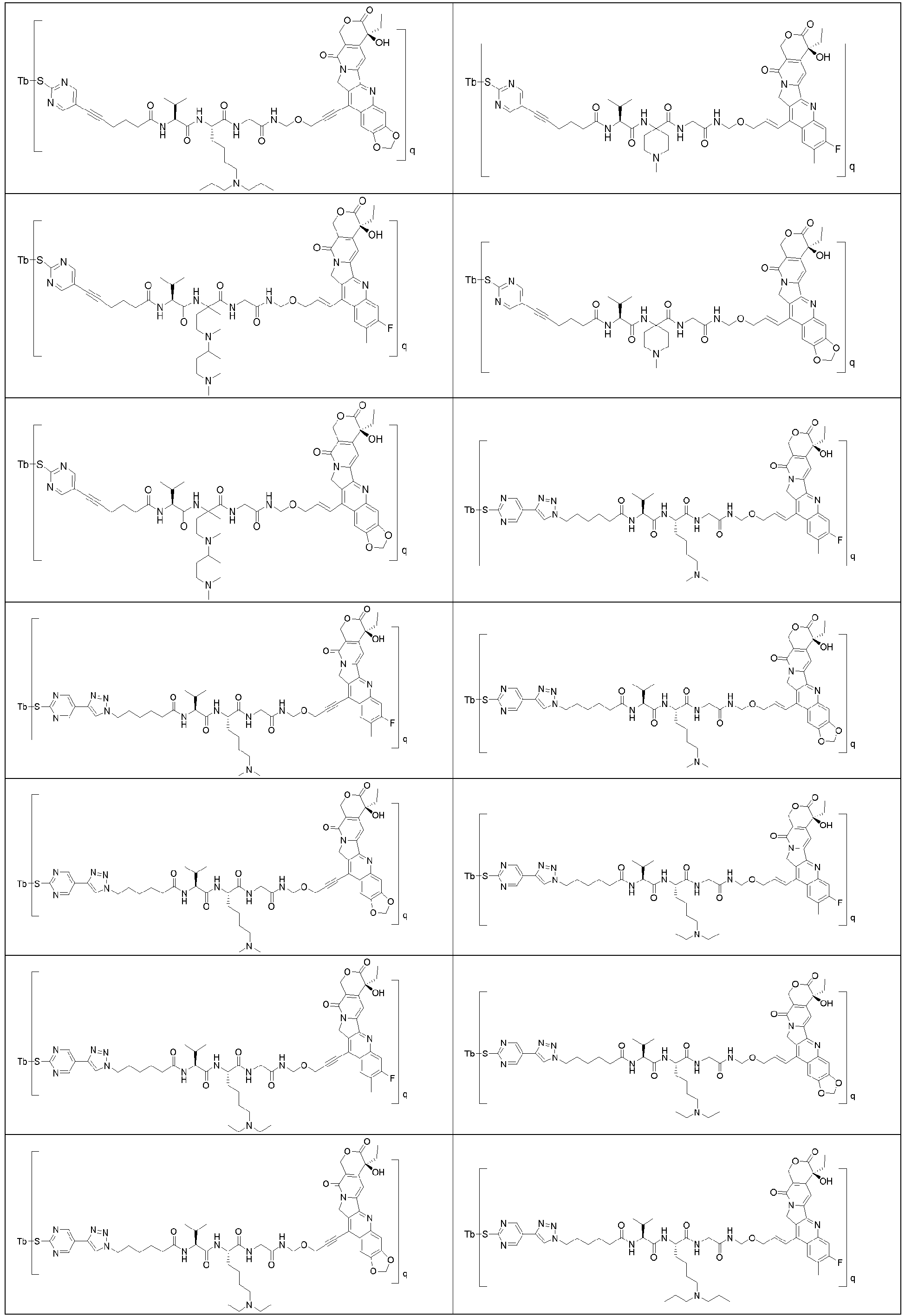

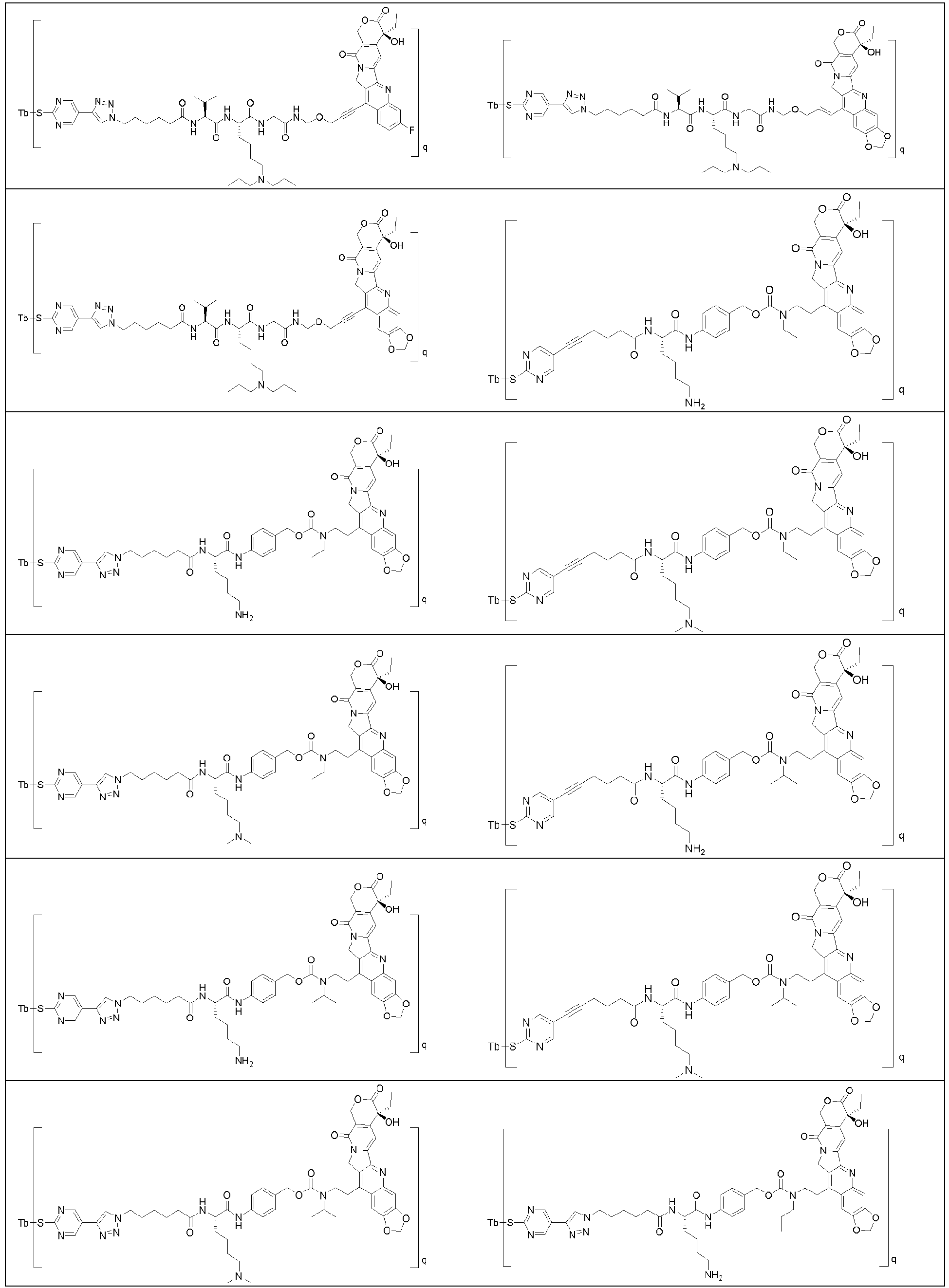

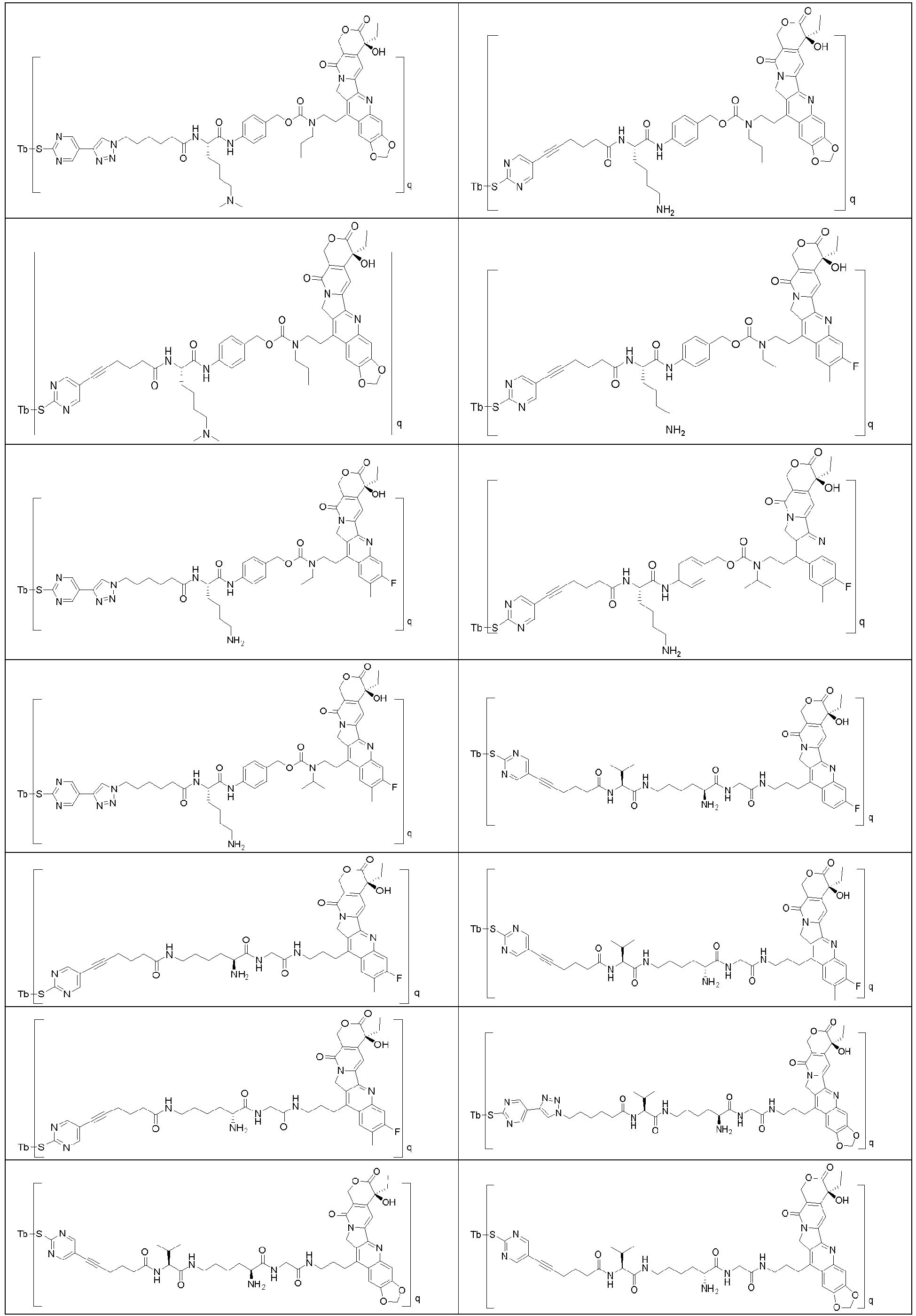

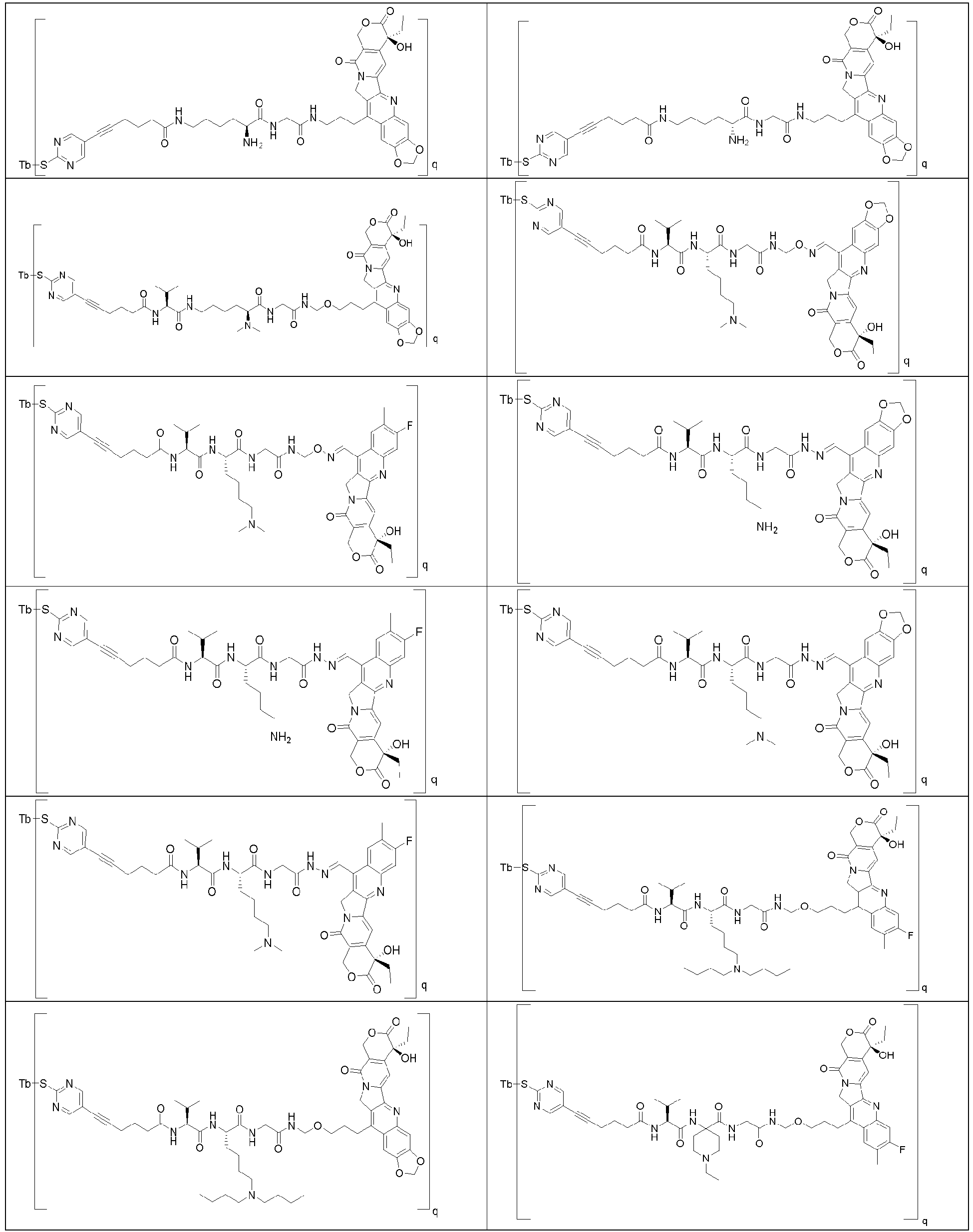

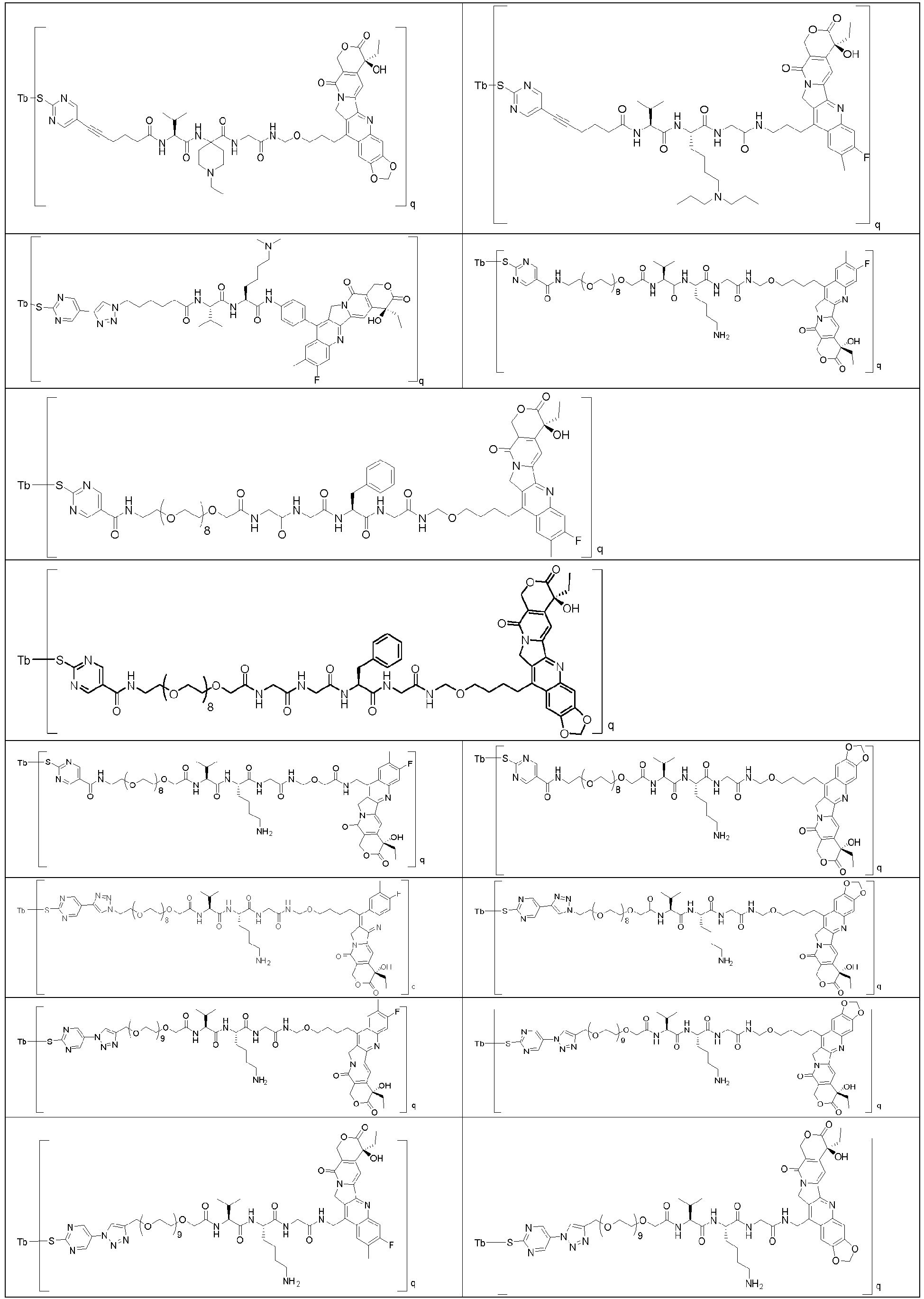

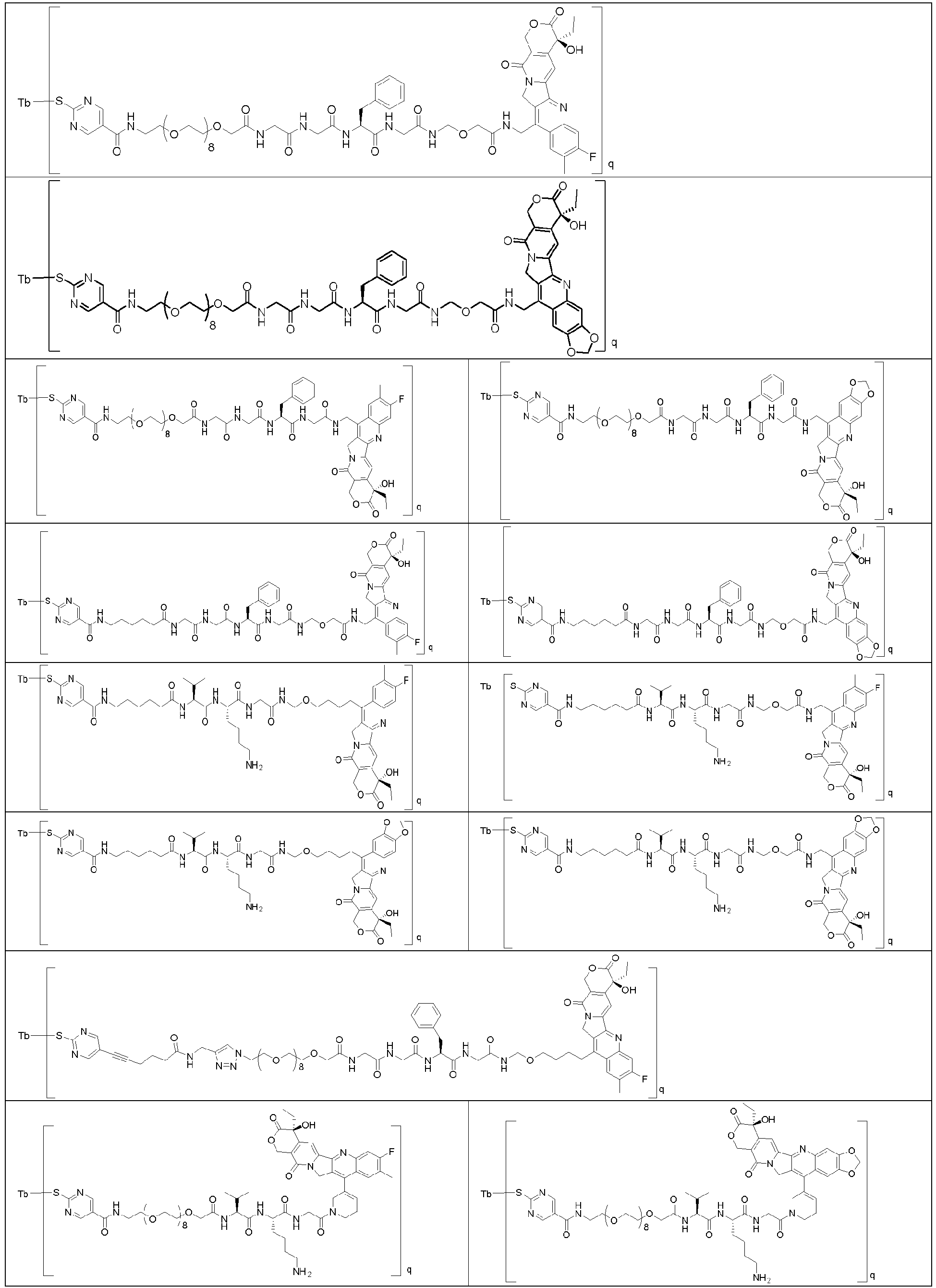

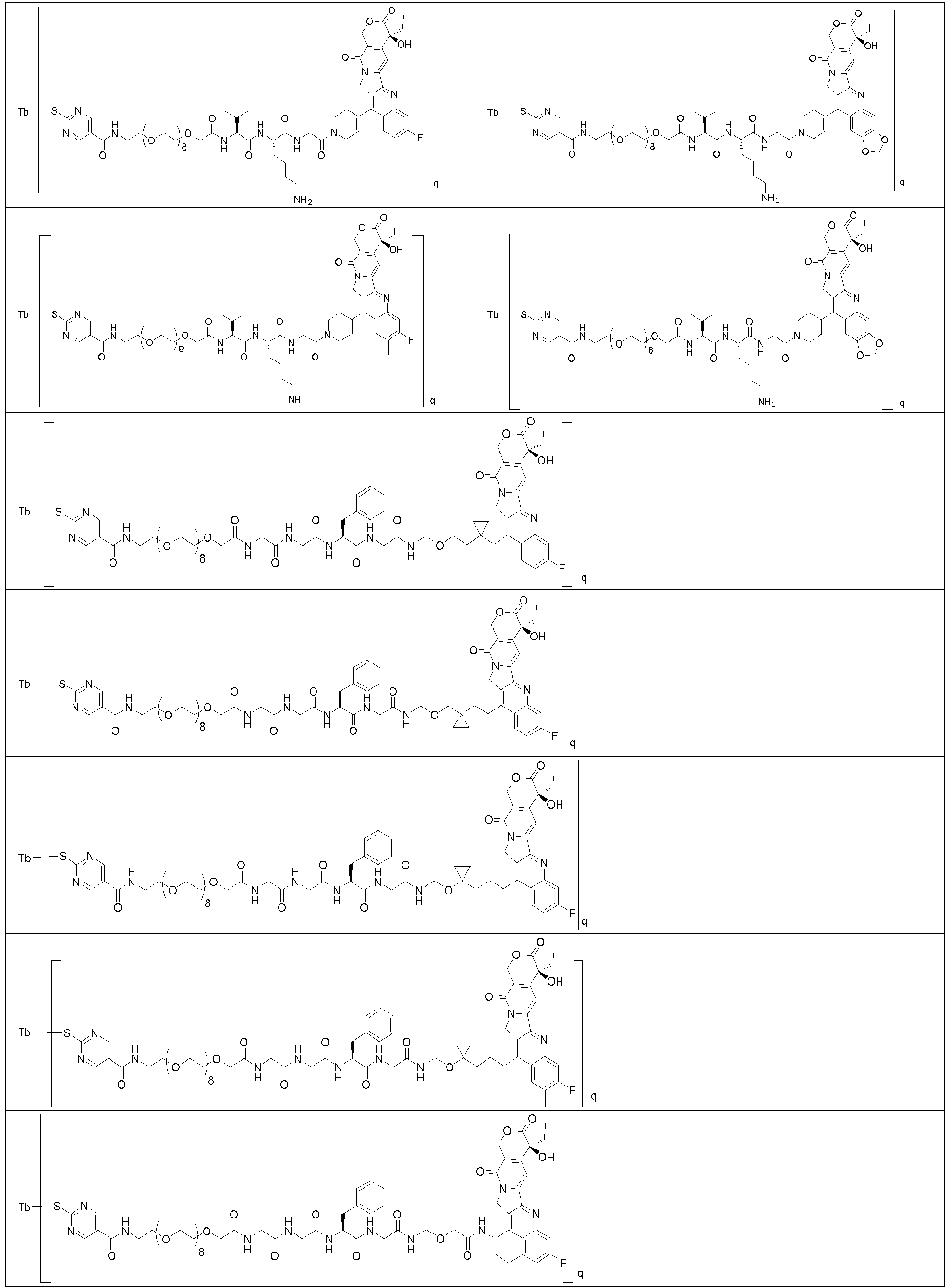

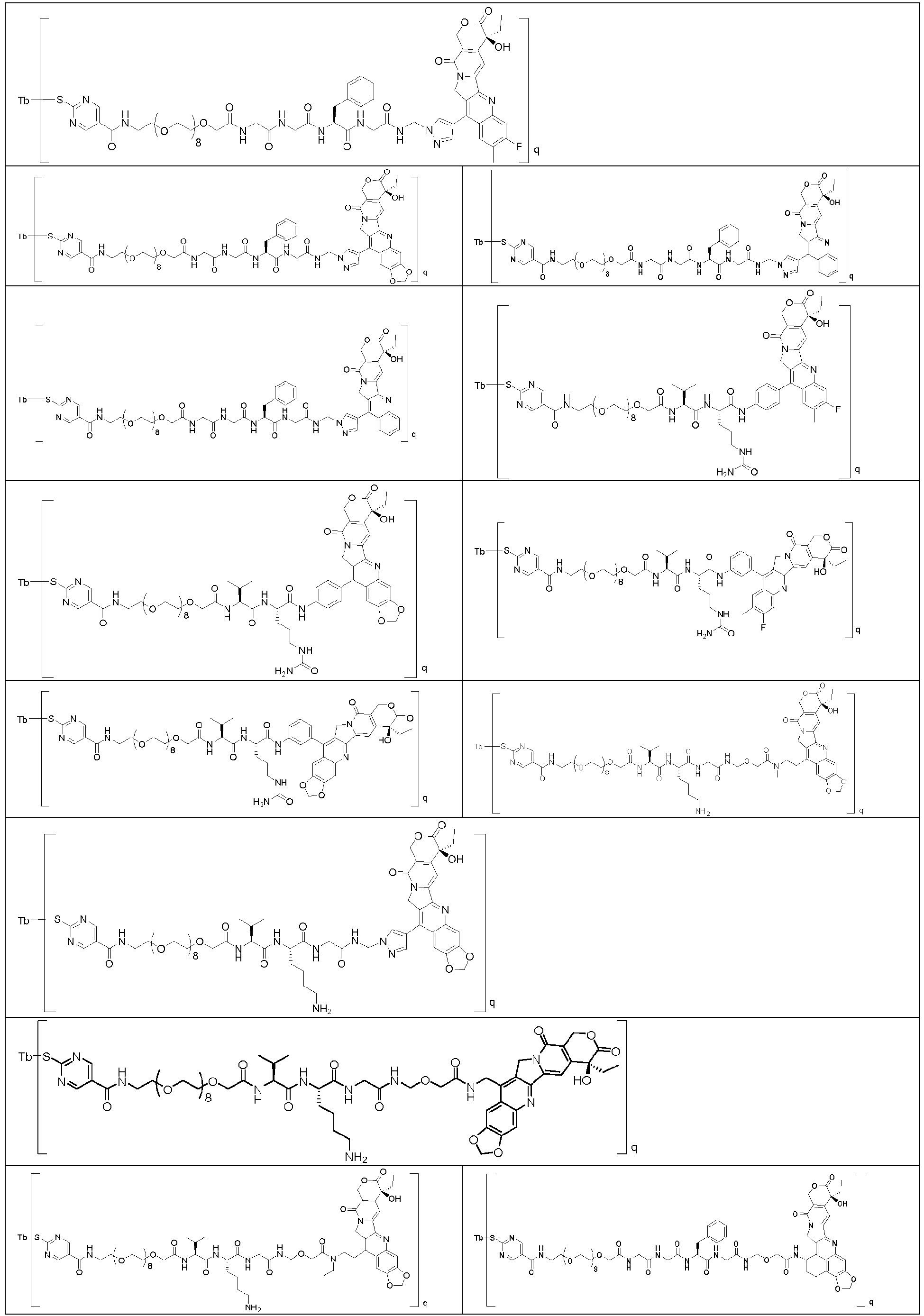

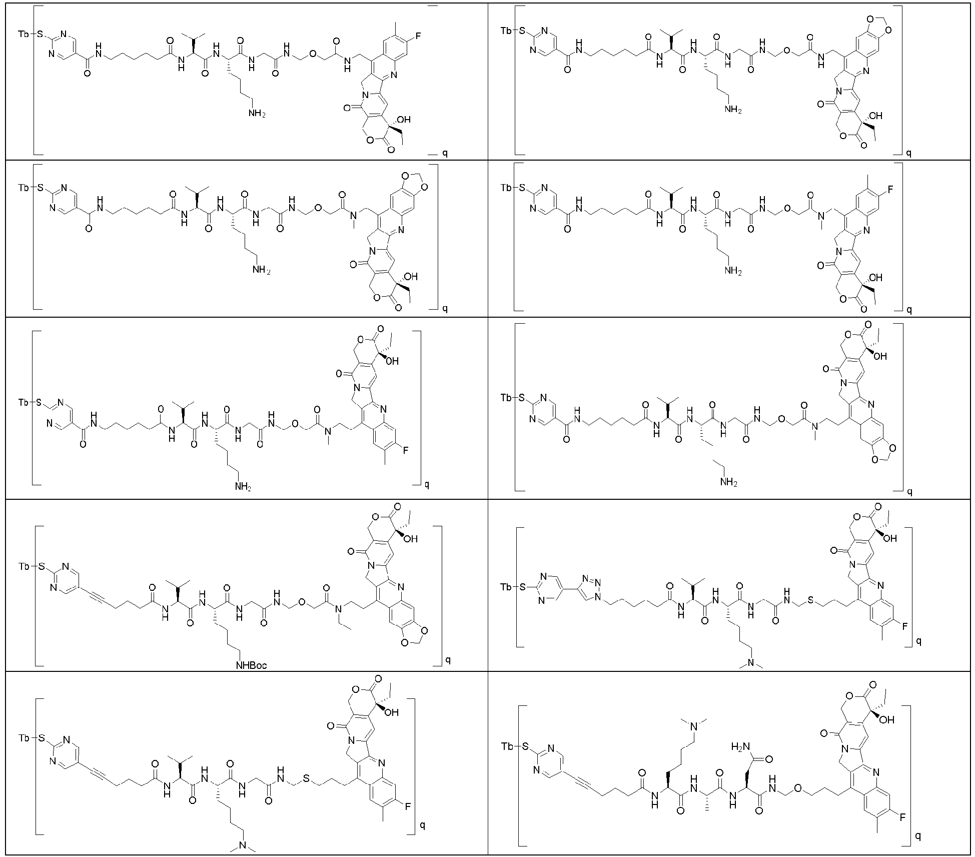

In some embodiments, Tb is an anti-NaPi2b antibody or an antigen-binding fragment thereof.

In some embodiments, the antibody or the antigen-binding fragment thereof and a monoclonal antibody or an antigen-binding fragment thereof comprise a Fab, a Fab', a F(ab')2, an Fd, an Fv (e.g., scFv), a dAb, a complementarity determining region fragment, a non-human antibody, a humanized antibody, a chimeric antibody, a fully human antibody, a probody, a monoclonal antibody, a bispecific antibody, or a multispecific antibody.

In some embodiments, Tb is an anti-NaPi2b antibody or an antigen-binding fragment thereof that has endocytic activity, no endocytic activity, or relatively weak endocytic activity.

In some embodiments, Tb is an anti-NaPi2b antibody or an antigen-binding fragment thereof that has endocytic activity.

In some embodiments, Tb is an anti-NaPi2b antibody or an antigen-binding fragment thereof that has no endocytic activity or weak endocytic activity.

In some embodiments, Tb is an anti-NaPi2b antibody or an antigen-binding fragment thereof.

In some embodiments, the anti-NaPi2b antibody is a non-human antibody, a humanized antibody, a chimeric antibody, or a fully human antibody.

In some embodiments, the anti-NaPi2b antibody is a monoclonal antibody, a bispecific antibody, or a multispecific antibody.

In some embodiments, the anti-NaPi2b antibody or the antigen-binding fragment thereof is a monoclonal antibody or an antigen-binding fragment thereof.

In some embodiments, the anti-NaPi2b antibody or the antigen-binding fragment thereof is selected from lifastuzumab or an antigen-binding fragment thereof, upifitamab or an antigen-binding fragment thereof, and antibody C1.

In some preferred embodiments, the anti-NaPi2b antibody is selected from murine antibodies 16G5B3, 60A12B3, 22H9C4, 66C12D12, and 34F2A10, and humanized antibodies thereof.

In some preferred embodiments, the anti-NaPi2b antibody is selected from the 60A12B3-hz1 antibody, the 22H9C4-hz1 antibody, the 66C12D12-hz1 antibody, and the 34F2A10-hz1 antibody.

The antibody or the antigen-binding fragment thereof of Tb may be prepared by various methods known in the art, such as by a genetic engineering recombination technique. For example, DNA molecules encoding the heavy chain and light chain genes of the antibody of the present disclosure are obtained by chemical synthesis or PCR amplification. The resulting DNA molecule is inserted into an expression vector, which is then transfected into a host cell. The transfected host cell is then cultured under specific conditions and expresses the antibody of the present disclosure.

In some preferred embodiments, in the antibody-drug conjugate, Tb is the anti-NaPi2b antibody or the antigen-binding fragment thereof according to a second aspect.

In the second aspect, the present disclosure provides an anti-NaPi2b antibody or an antigen-binding fragment thereof.

In some embodiments, the anti-NaPi2b antibody or the antigen-binding fragment thereof comprises the following complementarity determining regions (CDRs):

HCDR1 or a variant of a sequence thereof, HCDR2 or a variant of a sequence thereof, and HCDR3 or a variant of a sequence thereof comprised in a heavy chain variable region (VH) set forth in SEQ ID NO: 1, 17, 19, 34, 36, 52, 54, 71, 73, 74, or 33; and/or

LCDR1 or a variant of a sequence thereof, LCDR2 or a variant of a sequence thereof, and LCDR3 or a variant of a sequence thereof comprised in a light chain variable region (VL) set forth in SEQ ID NO: 2, 18, 20, 35, 37, 53, 55, 72, 75, or 51.

In certain embodiments, the anti-NaPi2b antibody or the antigen-binding fragment thereof comprises HCDR1 or a variant of a sequence thereof, HCDR2 or a variant of a sequence thereof, and HCDR3 or a variant of a sequence thereof comprised in the VH set forth in SEQ ID NO: 1 or 17; and/or LCDR1 or a variant of a sequence thereof, LCDR2 or a variant of a sequence thereof, and LCDR3 or a variant of a sequence thereof comprised in the VL set forth in SEQ ID NO: 2 or 18.

In certain embodiments, the anti-NaPi2b antibody or the antigen-binding fragment thereof comprises HCDR1 or a variant of a sequence thereof, HCDR2 or a variant of a sequence thereof, and HCDR3 or a variant of a sequence thereof comprised in the VH set forth in SEQ ID NO: 19 or 34; and/or LCDR1 or a variant of a sequence thereof, LCDR2 or a variant of a sequence thereof, and LCDR3 or a variant of a sequence thereof comprised in the VL set forth in SEQ ID NO: 20 or 35.

In certain embodiments, the anti-NaPi2b antibody or the antigen-binding fragment thereof comprises HCDR1 or a variant of a sequence thereof, HCDR2 or a variant of a sequence thereof, and HCDR3 or a variant of a sequence thereof comprised in the VH set forth in SEQ ID NO: 36 or 52; and/or LCDR1 or a variant of a sequence thereof, LCDR2 or a variant of a sequence thereof, and LCDR3 or a variant of a sequence thereof comprised in the VL set forth in SEQ ID NO: 37 or 53.

In certain embodiments, the anti-NaPi2b antibody or the antigen-binding fragment thereof comprises HCDR1 or a variant of a sequence thereof, HCDR2 or a variant of a sequence thereof, and HCDR3 or a variant of a sequence thereof comprised in the VH set forth in SEQ ID NO: 54, 71, or 73; and/or LCDR1 or a variant of a sequence thereof, LCDR2 or a variant of a sequence thereof, and LCDR3 or a variant of a sequence thereof comprised in the VL set forth in SEQ ID NO: 55 or 72.

In certain embodiments, the anti-NaPi2b antibody or the antigen-binding fragment thereof comprises HCDR1 or a variant of a sequence thereof, HCDR2 or a variant of a sequence thereof, and HCDR3 or a variant of a sequence thereof comprised in the VH set forth in SEQ ID NO: **74** or 33; and/or LCDR1 or a variant of a sequence thereof, LCDR2 or a variant of a sequence thereof, and LCDR3 or a variant of a sequence thereof comprised in the VL set forth in SEQ ID NO: 75 or 51.

In certain embodiments, the anti-NaPi2b antibody or the antigen-binding fragment thereof comprises HCDR1 or a variant of a sequence thereof, HCDR2 or a variant of a sequence thereof, and HCDR3 or a variant of a sequence thereof comprised in the VH set forth in SEQ ID NO: 17; and/or LCDR1 or a variant of a sequence thereof, LCDR2 or a variant of a sequence thereof, and LCDR3 or a variant of a sequence thereof comprised in the VL set forth in SEQ ID NO: 18.

In certain embodiments, the anti-NaPi2b antibody or the antigen-binding fragment thereof comprises HCDR1 or a variant of a sequence thereof, HCDR2 or a variant of a sequence thereof, and HCDR3 or a variant of a sequence thereof comprised in the VH set forth in SEQ ID NO: 34; and/or LCDR1 or a variant of a sequence thereof, LCDR2 or a variant of a sequence thereof, and LCDR3 or a variant of a sequence thereof comprised in the VL set forth in SEQ ID NO: 35.

In certain embodiments, the anti-NaPi2b antibody or the antigen-binding fragment thereof comprises HCDR1 or a variant of a sequence thereof, HCDR2 or a variant of a sequence thereof, and HCDR3 or a variant of a sequence thereof comprised in the VH set forth in SEQ ID NO: 52; and/or LCDR1 or a variant of a sequence thereof, LCDR2 or a variant of a sequence thereof, and LCDR3 or a variant of a sequence thereof comprised in the VL set forth in SEQ ID NO: 53.

In certain embodiments, the anti-NaPi2b antibody or the antigen-binding fragment thereof comprises HCDR1 or a variant of a sequence thereof, HCDR2 or a variant of a sequence thereof, and HCDR3 or a variant of a sequence thereof comprised in the VH set forth in SEQ ID NO: 71 or 73; and/or LCDR1 or a variant of a sequence thereof, LCDR2 or a variant of a sequence thereof, and LCDR3 or a variant of a sequence thereof comprised in the VL set forth in SEQ ID NO: 72.

In certain embodiments, the anti-NaPi2b antibody or the antigen-binding fragment thereof comprises HCDR1 or a variant of a sequence thereof, HCDR2 or a variant of a sequence thereof, and HCDR3 or a variant of a sequence thereof comprised in the VH set forth in SEQ ID NO: 33; and/or LCDR1 or a variant of a sequence thereof, LCDR2 or a variant of a sequence thereof, and LCDR3 or a variant of a sequence thereof comprised in the VL set forth in SEQ ID NO: 51.

In certain preferred embodiments, the variant of the sequence is a CDR having one or several amino acid substitutions, deletions, or additions (e.g., 1, 2, or 3 amino acid substitutions, deletions, or additions) compared with the CDR from which the variant is derived.

In certain preferred embodiments, the substitution is a conservative substitution.

In certain embodiments, the CDRs are defined according to the AbM, Chothia, Kabat or IMGT numbering system.

In certain embodiments, the anti-NaPi2b antibody or the antigen-binding fragment thereof comprises a heavy chain variable region (VH) and/or a light chain variable region (VL).

In certain embodiments, the anti-NaPi2b antibody or the antigen-binding fragment thereof of the present disclosure comprises the following heavy chain variable region (VH) and/or light chain variable region (VL), wherein the CDRs are defined according to the Kabat numbering system:
(a) a heavy chain variable region (VH) comprising 3 CDRs as follows: HCDR1 with the sequence of SEQ ID NO: 3 or a sequence having one or several amino acid substitutions, deletions, or additions (e.g., 1, 2, or 3 amino acid substitutions, deletions, or additions) compared therewith, HCDR2 with the sequence of SEQ ID NO: 4 or a sequence having one or several amino acid substitutions, deletions, or additions (e.g., 1, 2, or 3 amino acid substitutions, deletions, or additions) compared therewith, and HCDR3 with the sequence of SEQ ID NO: 6 or a sequence having one or several amino acid substitutions, deletions, or additions (e.g., 1, 2, or 3 amino acid substitutions, deletions, or additions) compared therewith; and/or
   a light chain variable region (VL) comprising 3 CDRs as follows: LCDR1 with the sequence of SEQ ID NO: 7 or a sequence having one or several amino acid substitutions, deletions, or additions (e.g., 1, 2, or 3 amino acid substitutions, deletions, or additions) compared therewith, LCDR2 with the sequence of SEQ ID NO: 9 or a sequence having one or several amino acid substitutions, deletions, or additions (e.g., 1, 2, or 3 amino acid substitutions, deletions, or additions) compared therewith, and LCDR3 with the sequence of SEQ ID NO: 10 or a sequence having one or several amino acid substitutions, deletions, or additions (e.g., 1, 2, or 3 amino acid substitutions, deletions, or additions) compared therewith;
(b) a heavy chain variable region (VH) comprising 3 CDRs as follows: HCDR1 with the sequence of SEQ ID NO: 3 or a sequence having one or several amino acid substitutions, deletions, or additions (e.g., 1, 2, or 3 amino acid substitutions, deletions, or additions) compared therewith, HCDR2 with the sequence of SEQ ID NO: 5 or a sequence having one or several amino acid substitutions, deletions, or additions (e.g., 1, 2, or 3 amino acid substitutions, deletions, or additions) compared therewith, and HCDR3 with the sequence of SEQ ID NO: 6 or a sequence having one or several amino acid substitutions, deletions, or additions (e.g., 1, 2, or 3 amino acid substitutions, deletions, or additions) compared therewith; and/or
   a light chain variable region (VL) comprising 3 CDRs as follows: LCDR1 with the sequence of SEQ ID NO: 8 or a sequence having one or several amino acid substitutions, deletions, or additions (e.g., 1, 2, or 3 amino acid substitutions, deletions, or additions) compared therewith, LCDR2 with the sequence of SEQ ID NO: 9 or a sequence having one or several amino acid substitutions, deletions, or additions (e.g., 1, 2, or 3 amino acid substitutions, deletions, or additions) compared therewith, and LCDR3 with the sequence of SEQ ID NO: 10 or a sequence having one or several amino acid substitutions, deletions, or additions (e.g., 1, 2, or 3 amino acid substitutions, deletions, or additions) compared therewith;
(c) a heavy chain variable region (VH) comprising 3 CDRs as follows: HCDR1 with the sequence of SEQ ID NO: 21 or a sequence having one or several amino acid substitutions, deletions, or additions (e.g., 1, 2, or 3 amino acid substitutions, deletions, or additions) compared therewith, HCDR2 with the sequence of SEQ ID NO: 22 or a sequence having one or several amino acid substitutions, deletions, or additions (e.g., 1, 2, or 3 amino acid substitutions, deletions, or additions) compared therewith, and HCDR3 with the sequence of SEQ ID NO: 24 or a sequence having one or several amino acid substitutions, deletions, or additions (e.g., 1, 2, or 3 amino acid substitutions, deletions, or additions) compared therewith; and/or
   a light chain variable region (VL) comprising 3 CDRs as follows: LCDR1 with the sequence of SEQ ID NO: 25 or a sequence having one or several amino acid substitutions, deletions, or additions (e.g., 1, 2, or 3 amino acid substitutions, deletions, or additions) compared therewith, LCDR2 with the sequence of SEQ ID NO: 26 or a sequence having one or several amino acid substitutions, deletions, or additions (e.g., 1, 2, or 3 amino acid substitutions, deletions, or additions) compared therewith, and LCDR3 with the sequence of SEQ ID NO: 27 or a sequence having one or several amino acid substitutions, deletions, or additions (e.g., 1, 2, or 3 amino acid substitutions, deletions, or additions) compared therewith;
(d) a heavy chain variable region (VH) comprising 3 CDRs as follows: HCDR1 with the sequence of SEQ ID NO: 21 or a sequence having one or several amino acid substitutions, deletions, or additions (e.g., 1, 2, or 3 amino acid substitutions, deletions, or additions) compared therewith, HCDR2 with the sequence of SEQ ID NO: 23 or a sequence having one or several amino acid substitutions, deletions, or additions (e.g., 1, 2, or 3 amino acid substitutions, deletions, or additions) compared therewith, and HCDR3 with the sequence of SEQ ID NO: 24 or a sequence having one or several amino acid substitutions, deletions, or additions (e.g., 1, 2, or 3 amino acid substitutions, deletions, or additions) compared therewith; and/or
   a light chain variable region (VL) comprising 3 CDRs as follows: LCDR1 with the sequence of SEQ ID NO: 25 or a sequence having one or several amino acid substitutions, deletions, or additions (e.g., 1, 2, or 3 amino acid substitutions, deletions, or additions) compared therewith, LCDR2 with the sequence of SEQ ID NO: 26 or a sequence having one or several amino acid substitutions, deletions, or additions (e.g., 1, 2, or 3 amino acid substitutions, deletions, or additions) compared therewith, and LCDR3 with the sequence of SEQ ID NO: 27 or a sequence having one or several amino acid substitutions, deletions, or additions (e.g., 1, 2, or 3 amino acid substitutions, deletions, or additions) compared therewith;
(e) a heavy chain variable region (VH) comprising 3 CDRs as follows: HCDR1 with the sequence of SEQ ID NO: 38 or a sequence having one or several amino acid substitutions, deletions, or additions (e.g., 1, 2, or 3 amino acid substitutions, deletions, or additions) compared therewith, HCDR2 with the sequence of SEQ ID NO: 39 or a sequence having one or several amino acid substitutions, deletions, or additions (e.g., 1, 2, or 3 amino acid substitutions, deletions, or additions) compared therewith, and HCDR3 with the sequence of SEQ ID NO: 41 or a sequence having one or several amino acid substitutions, deletions, or additions (e.g., 1, 2, or 3 amino acid substitutions, deletions, or additions) compared therewith; and/or
   a light chain variable region (VL) comprising 3 CDRs as follows: LCDR1 with the sequence of SEQ ID NO: 42 or a sequence having one or several amino acid substitutions, deletions, or additions (e.g., 1, 2, or 3 amino acid substitutions, deletions, or additions) compared therewith, LCDR2 with the sequence of SEQ ID NO: 44 or a sequence having one or several amino acid substitutions, deletions, or additions (e.g., 1, 2, or 3 amino acid substitutions, deletions, or additions) compared therewith, and LCDR3 with the sequence of SEQ ID NO: 45 or a sequence having one or several amino acid substitutions, deletions, or additions (e.g., 1, 2, or 3 amino acid substitutions, deletions, or additions) compared therewith;
(f) a heavy chain variable region (VH) comprising 3 CDRs as follows: HCDR1 with the sequence of SEQ ID NO: 38 or a sequence having one or several amino acid substitutions, deletions, or additions (e.g., 1, 2, or 3 amino acid substitutions, deletions, or additions) compared therewith, HCDR2 with the sequence of SEQ ID NO: 40 or a sequence having one or several amino acid substitutions, deletions, or additions (e.g., 1, 2, or 3 amino acid substitutions, deletions, or additions) compared therewith, and HCDR3 with the sequence of SEQ ID NO: 41 or a sequence having one or several amino acid substitutions, deletions, or additions (e.g., 1, 2, or 3 amino acid substitutions, deletions, or additions) compared therewith; and/or
   a light chain variable region (VL) comprising 3 CDRs as follows: LCDR1 with the sequence of SEQ ID NO: 42 or a sequence having one or several amino acid substitutions, deletions, or additions (e.g., 1, 2, or 3 amino acid substitutions, deletions, or additions) compared therewith, LCDR2 with the sequence of SEQ ID NO: 44 or a sequence having one or several amino acid substitutions, deletions, or additions (e.g., 1, 2, or 3 amino acid substitutions, deletions, or additions) compared therewith, and LCDR3 with the sequence of SEQ ID NO: 45 or a sequence having one or several amino acid substitutions, deletions, or additions (e.g., 1, 2, or 3 amino acid substitutions, deletions, or additions) compared therewith;
(g) a heavy chain variable region (VH) comprising 3 CDRs as follows: HCDR1 with the sequence of SEQ ID NO: 56 or a sequence having one or several amino acid substitutions, deletions, or additions (e.g., 1, 2, or 3 amino acid substitutions, deletions, or additions) compared therewith, HCDR2 with the sequence of SEQ ID NO: 57 or a sequence having one or several amino acid substitutions, deletions, or additions (e.g., 1, 2, or 3 amino acid substitutions, deletions, or additions) compared therewith, and HCDR3 with the sequence of SEQ ID NO: 60 or a sequence having one or several amino acid substitutions, deletions, or additions (e.g., 1, 2, or 3 amino acid substitutions, deletions, or additions) compared therewith; and/or
   a light chain variable region (VL) comprising 3 CDRs as follows: LCDR1 with the sequence of SEQ ID NO: 61 or a sequence having one or several amino acid substitutions, deletions, or additions (e.g., 1, 2, or 3 amino acid substitutions, deletions, or additions) compared therewith, LCDR2 with the sequence of SEQ ID NO: 63 or a sequence having one or several amino acid substitutions, deletions, or additions (e.g., 1, 2, or 3 amino acid substitutions, deletions, or additions) compared therewith, and LCDR3 with the sequence of SEQ ID NO: 64 or a sequence having one or several amino acid substitutions, deletions, or additions (e.g., 1, 2, or 3 amino acid substitutions, deletions, or additions) compared therewith;
(h) a heavy chain variable region (VH) comprising 3 CDRs as follows: HCDR1 with the sequence of SEQ ID NO: 56 or a sequence having one or several amino acid substitutions, deletions, or additions (e.g., 1, 2, or 3 amino acid substitutions, deletions, or additions) compared therewith, HCDR2 with the sequence of SEQ ID NO: 58 or a sequence having one or several amino acid substitutions, deletions, or additions (e.g., 1, 2, or 3 amino acid substitutions, deletions, or additions) compared therewith, and HCDR3 with the sequence of SEQ ID NO: 60 or a sequence having one or several amino acid substitutions, deletions, or additions (e.g., 1, 2, or 3 amino acid substitutions, deletions, or additions) compared therewith; and/or
   a light chain variable region (VL) comprising 3 CDRs as follows: LCDR1 with the sequence of SEQ ID NO: 62 or a sequence having one or several amino acid substitutions, deletions, or additions (e.g., 1, 2, or 3 amino acid substitutions, deletions, or additions) compared therewith, LCDR2 with the sequence of SEQ ID NO: 63 or a sequence having one or several amino acid substitutions, deletions, or additions (e.g., 1, 2, or 3 amino acid substitutions, deletions, or additions) compared therewith, and LCDR3 with the sequence of SEQ ID NO: 64 or a sequence having one or several amino acid substitutions, deletions, or additions (e.g., 1, 2, or 3 amino acid substitutions, deletions, or additions) compared therewith; or
(i) a heavy chain variable region (VH) comprising 3 CDRs as follows: HCDR1 with the sequence of SEQ ID NO: 56 or a sequence having one or several amino acid substitutions, deletions, or additions (e.g., 1, 2, or 3 amino acid substitutions, deletions, or additions) compared therewith, HCDR2 with the sequence of SEQ ID NO: 59 or a sequence having one or several amino acid substitutions, deletions, or additions (e.g., 1, 2, or 3 amino acid substitutions, deletions, or additions) compared therewith, and HCDR3 with the sequence of SEQ ID NO: 60 or a sequence having one or several amino acid substitutions, deletions, or additions (e.g., 1, 2, or 3 amino acid substitutions, deletions, or additions) compared therewith; and/or
   a light chain variable region (VL) comprising 3 CDRs as follows: LCDR1 with the sequence of SEQ ID NO: 62 or a sequence having one or several amino acid substitutions, deletions, or additions (e.g., 1, 2, or 3 amino acid substitutions, deletions, or additions) compared therewith, LCDR2 with the sequence of SEQ ID NO: 63 or a sequence having one or several amino acid substitutions, deletions, or additions (e.g., 1, 2, or 3 amino acid substitutions, deletions, or additions) compared therewith, and LCDR3 with the sequence of SEQ ID NO: 64 or a sequence having one or several amino acid substitutions, deletions, or additions (e.g., 1, 2, or 3 amino acid substitutions, deletions, or additions) compared therewith.

In certain embodiments, the anti-NaPi2b antibody or the antigen-binding fragment thereof of the present disclosure comprises the following heavy chain variable region (VH) and/or light chain variable region (VL), wherein the CDRs are defined according to the Kabat numbering system:
(a) the VH comprises HCDR1 with the sequence of SEQ ID NO: 3, HCDR2 with the sequence of SEQ ID NO: 4, and HCDR3 with the sequence of SEQ ID NO: 6; and/or
   the VL comprises LCDR1 with the sequence of SEQ ID NO: 7, LCDR2 with the sequence of SEQ ID NO: 9, and LCDR3 with the sequence of SEQ ID NO: 10;
(b) the VH comprises HCDR1 with the sequence of SEQ ID NO: 3, HCDR2 with the sequence of SEQ ID NO: 5, and HCDR3 with the sequence of SEQ ID NO: 6; and/or
   the VL comprises LCDR1 with the sequence of SEQ ID NO: 8, LCDR2 with the sequence of SEQ ID NO: 9, and LCDR3 with the sequence of SEQ ID NO: 10;
(c) the VH comprises HCDR1 with the sequence of SEQ ID NO: 21, HCDR2 with the sequence of SEQ ID NO: 22, and HCDR3 with the sequence of SEQ ID NO: 24; and/or
   the VL comprises LCDR1 with the sequence of SEQ ID NO: 25, LCDR2 with the sequence of SEQ ID NO: 26, and LCDR3 with the sequence of SEQ ID NO: 27;
(d) the VH comprises HCDR1 with the sequence of SEQ ID NO: 21, HCDR2 with the sequence of SEQ ID NO: 23, and HCDR3 with the sequence of SEQ ID NO: 24; and/or
   the VL comprises LCDR1 with the sequence of SEQ ID NO: 25, LCDR2 with the sequence of SEQ ID NO: 26, and LCDR3 with the sequence of SEQ ID NO: 27;
(e) the VH comprises HCDR1 with the sequence of SEQ ID NO: 38, HCDR2 with the sequence of SEQ ID NO: 39, and HCDR3 with the sequence of SEQ ID NO: 41; and/or
   the VL comprises LCDR1 with the sequence of SEQ ID NO: 42, LCDR2 with the sequence of SEQ ID NO: 44, and LCDR3 with the sequence of SEQ ID NO: 45;
(f) the VH comprises HCDR1 with the sequence of SEQ ID NO: 38, HCDR2 with the sequence of SEQ ID NO: 40, and HCDR3 with the sequence of SEQ ID NO: 41; and/or
   the VL comprises LCDR1 with the sequence of SEQ ID NO: 43, LCDR2 with the sequence of SEQ ID NO: 44, and LCDR3 with the sequence of SEQ ID NO: 45;
(g) the VH comprises HCDR1 with the sequence of SEQ ID NO: 56, HCDR2 with the sequence of SEQ ID NO: 57, and HCDR3 with the sequence of SEQ ID NO: 60; and/or
   the VL comprises LCDR1 with the sequence of SEQ ID NO: 61, LCDR2 with the sequence of SEQ ID NO: 63, and LCDR3 with the sequence of SEQ ID NO: 64;
(h) the VH comprises HCDR1 with the sequence of SEQ ID NO: 56, HCDR2 with the sequence of SEQ ID NO: 58, and HCDR3 with the sequence of SEQ ID NO: 60; and/or
   the VL comprises LCDR1 with the sequence of SEQ ID NO: 62, LCDR2 with the sequence of SEQ ID NO: 63, and LCDR3 with the sequence of SEQ ID NO: 64; or
(i) the VH comprises HCDR1 with the sequence of SEQ ID NO: 56, HCDR2 with the sequence of SEQ ID NO: 59, and HCDR3 with the sequence of SEQ ID NO: 60; and/or
   the VL comprises LCDR1 with the sequence of SEQ ID NO: 62, LCDR2 with the sequence of SEQ ID NO: 63, and LCDR3 with the sequence of SEQ ID NO: 64.

In certain embodiments, the anti-NaPi2b antibody or the antigen-binding fragment thereof of the present disclosure comprises the following heavy chain variable region (VH) and/or light chain variable region (VL), wherein the CDRs are defined according to the IMGT numbering system:
(a) a heavy chain variable region (VH) comprising 3 CDRs as follows: HCDR1 with the sequence of SEQ ID NO: 11 or a sequence having one or several amino acid substitutions, deletions, or additions (e.g., 1, 2, or 3 amino acid substitutions, deletions, or additions) compared therewith, HCDR2 with the sequence of SEQ ID NO: 12 or a sequence having one or several amino acid substitutions, deletions, or additions (e.g., 1, 2, or 3 amino acid substitutions, deletions, or additions) compared therewith, and HCDR3 with the sequence of SEQ ID NO: **14** or a sequence having one or several amino acid substitutions, deletions, or additions (e.g., 1, 2, or 3 amino acid substitutions, deletions, or additions) compared therewith; and/or
   a light chain variable region (VL) comprising 3 CDRs as follows: LCDR1 with the sequence of SEQ ID NO: 15 or a sequence having one or several amino acid substitutions, deletions, or additions (e.g., 1, 2, or 3 amino acid substitutions, deletions, or additions) compared therewith, LCDR2 with the sequence that is WAS or a sequence having one or several amino acid substitutions, deletions, or additions (e.g., 1, 2, or 3 amino acid substitutions, deletions, or additions) compared therewith, and LCDR3 with the sequence of SEQ ID NO: 10 or a sequence having one or several amino acid substitutions, deletions, or additions (e.g., 1, 2, or 3 amino acid substitutions, deletions, or additions) compared therewith;
(b) a heavy chain variable region (VH) comprising 3 CDRs as follows: HCDR1 with the sequence of SEQ ID NO: 11 or a sequence having one or several amino acid substitutions, deletions, or additions (e.g., 1, 2, or 3 amino acid substitutions, deletions, or additions) compared therewith, HCDR2 with the sequence of SEQ ID NO: 13 or a sequence having one or several amino acid substitutions, deletions, or additions (e.g., 1, 2, or 3 amino acid substitutions, deletions, or additions) compared therewith, and HCDR3 with the sequence of SEQ ID NO: 14 or a sequence having one or several amino acid substitutions, deletions, or additions (e.g., 1, 2, or 3 amino acid substitutions, deletions, or additions) compared therewith; and/or
   a light chain variable region (VL) comprising 3 CDRs as follows: LCDR1 with the sequence of SEQ ID NO: 15 or a sequence having one or several amino acid substitutions, deletions, or additions (e.g., 1, 2, or 3 amino acid substitutions, deletions, or additions) compared therewith, LCDR2 with the sequence that is WAS or a sequence having one or several amino acid substitutions, deletions, or additions (e.g., 1, 2, or 3 amino acid substitutions, deletions, or additions) compared therewith, and LCDR3 with the sequence of SEQ ID NO: 10 or a sequence having one or several amino acid substitutions, deletions, or additions (e.g., 1, 2, or 3 amino acid substitutions, deletions, or additions) compared therewith;
(c) a heavy chain variable region (VH) comprising 3 CDRs as follows: HCDR1 with the sequence of SEQ ID NO: 28 or a sequence having one or several amino acid substitutions, deletions, or additions (e.g., 1, 2, or 3 amino acid substitutions, deletions, or additions) compared therewith, HCDR2 with the sequence of SEQ ID NO: 29 or a sequence having one or several amino acid substitutions, deletions, or additions (e.g., 1, 2, or 3 amino acid substitutions, deletions, or additions) compared therewith, and HCDR3 with the sequence of SEQ ID NO: 31 or a sequence having one or several amino acid substitutions, deletions, or additions (e.g., 1, 2, or 3 amino acid substitutions, deletions, or additions) compared therewith; and/or
   a light chain variable region (VL) comprising 3 CDRs as follows: LCDR1 with the sequence of SEQ ID NO: 32 or a sequence having one or several amino acid substitutions, deletions, or additions (e.g., 1, 2, or 3 amino acid substitutions, deletions, or additions) compared therewith, LCDR2 with the sequence that is YTS or a sequence having one or several amino acid substitutions, deletions, or additions (e.g., 1, 2, or 3 amino acid substitutions, deletions, or additions) compared therewith, and LCDR3 with the sequence of SEQ ID NO: 27 or a sequence having one or several amino acid substitutions, deletions, or additions (e.g., 1, 2, or 3 amino acid substitutions, deletions, or additions) compared therewith;
(d) a heavy chain variable region (VH) comprising 3 CDRs as follows: HCDR1 with the sequence of SEQ ID NO: 28 or a sequence having one or several amino acid substitutions, deletions, or additions (e.g., 1, 2, or 3 amino acid substitutions, deletions, or additions) compared therewith, HCDR2 with the sequence of SEQ ID NO: 30 or a sequence having one or several amino acid substitutions, deletions, or additions (e.g., 1, 2, or 3 amino acid substitutions, deletions, or additions) compared therewith, and HCDR3 with the sequence of SEQ ID NO: 31 or a sequence having one or several amino acid substitutions, deletions, or additions (e.g., 1, 2, or 3 amino acid substitutions, deletions, or additions) compared therewith; and/or
   a light chain variable region (VL) comprising 3 CDRs as follows: LCDR1 with the sequence of SEQ ID NO: 32 or a sequence having one or several amino acid substitutions, deletions, or additions (e.g., 1, 2, or 3 amino acid substitutions, deletions, or additions) compared therewith, LCDR2 with the sequence that is YTS or a sequence having one or several amino acid substitutions, deletions, or additions (e.g., 1, 2, or 3 amino acid substitutions, deletions, or additions) compared therewith, and LCDR3 with the sequence of SEQ ID NO: 27 or a sequence having one or several amino acid substitutions, deletions, or additions (e.g., 1, 2, or 3 amino acid substitutions, deletions, or additions) compared therewith;
(e) a heavy chain variable region (VH) comprising 3 CDRs as follows: HCDR1 with the sequence of SEQ ID NO: 46 or a sequence having one or several amino acid substitutions, deletions, or additions (e.g., 1, 2, or 3 amino acid substitutions, deletions, or additions) compared therewith, HCDR2 with the sequence of SEQ ID NO: 47 or a sequence having one or several amino acid substitutions, deletions, or additions (e.g., 1, 2, or 3 amino acid substitutions, deletions, or additions) compared therewith, and HCDR3 with the sequence of SEQ ID NO: 49 or a sequence having one or several amino acid substitutions, deletions, or additions (e.g., 1, 2, or 3 amino acid substitutions, deletions, or additions) compared therewith; and/or
   a light chain variable region (VL) comprising 3 CDRs as follows: LCDR1 with the sequence of SEQ ID NO: 50 or a sequence having one or several amino acid substitutions, deletions, or additions (e.g., 1, 2, or 3 amino acid substitutions, deletions, or additions) compared therewith, LCDR2 with the sequence that is SAS or a sequence having one or several amino acid substitutions, deletions, or additions (e.g., 1, 2, or 3 amino acid substitutions, deletions, or additions) compared therewith, and LCDR3 with the sequence of SEQ ID NO: 45 or a sequence having one or several amino acid substitutions, deletions, or additions (e.g., 1, 2, or 3 amino acid substitutions, deletions, or additions) compared therewith;
(f) a heavy chain variable region (VH) comprising 3 CDRs as follows: HCDR1 with the sequence of SEQ ID NO: 46 or a sequence having one or several amino acid substitutions, deletions, or additions (e.g., 1, 2, or 3 amino acid substitutions, deletions, or additions) compared therewith, HCDR2 with the sequence of SEQ ID NO: 48 or a sequence having one or several amino acid substitutions, deletions, or additions (e.g., 1, 2, or 3 amino acid substitutions, deletions, or additions) compared therewith, and HCDR3 with the sequence of SEQ ID NO: 49 or a sequence having one or several amino acid substitutions, deletions, or additions (e.g., 1, 2, or 3 amino acid substitutions, deletions, or additions) compared therewith; and/or
   a light chain variable region (VL) comprising 3 CDRs as follows: LCDR1 with the sequence of SEQ ID NO: 50 or a sequence having one or several amino acid substitutions, deletions, or additions (e.g., 1, 2, or 3 amino acid substitutions, deletions, or additions) compared therewith, LCDR2 with the sequence that is SAS or a sequence having one or several amino acid substitutions, deletions, or additions (e.g., 1, 2, or 3 amino acid substitutions, deletions, or additions) compared therewith, and LCDR3 with the sequence of SEQ ID NO: 45 or a sequence having one or several amino acid substitutions, deletions, or additions (e.g., 1, 2, or 3 amino acid substitutions, deletions, or additions) compared therewith;
(g) a heavy chain variable region (VH) comprising 3 CDRs as follows: HCDR1 with the sequence of SEQ ID NO: 65 or a sequence having one or several amino acid substitutions, deletions, or additions (e.g., 1, 2, or 3 amino acid substitutions, deletions, or additions) compared therewith, HCDR2 with the sequence of SEQ ID NO: 66 or a sequence having one or several amino acid substitutions, deletions, or additions (e.g., 1, 2, or 3 amino acid substitutions, deletions, or additions) compared therewith, and HCDR3 with the sequence of SEQ ID NO: 68 or a sequence having one or several amino acid substitutions, deletions, or additions (e.g., 1, 2, or 3 amino acid substitutions, deletions, or additions) compared therewith; and/or
   a light chain variable region (VL) comprising 3 CDRs as follows: LCDR1 with the sequence of SEQ ID NO: 69 or a sequence having one or several amino acid substitutions, deletions, or additions (e.g., 1, 2, or 3 amino acid substitutions, deletions, or additions) compared therewith, LCDR2 with the sequence that is WAS or a sequence having one or several amino acid substitutions, deletions, or additions (e.g., 1, 2, or 3 amino acid substitutions, deletions, or additions) compared therewith, and LCDR3 with the sequence of SEQ ID NO: 64 or a sequence having one or several amino acid substitutions, deletions, or additions (e.g., 1, 2, or 3 amino acid substitutions, deletions, or additions) compared therewith;
(h) a heavy chain variable region (VH) comprising 3 CDRs as follows: HCDR1 with the sequence of SEQ ID NO: 65 or a sequence having one or several amino acid substitutions, deletions, or additions (e.g., 1, 2, or 3 amino acid substitutions, deletions, or additions) compared therewith, HCDR2 with the sequence of SEQ ID NO: 67 or a sequence having one or several amino acid substitutions, deletions, or additions (e.g., 1, 2, or 3 amino acid substitutions, deletions, or additions) compared therewith, and HCDR3 with the sequence of SEQ ID NO: 68 or a sequence having one or several amino acid substitutions, deletions, or additions (e.g., 1, 2, or 3 amino acid substitutions, deletions, or additions) compared therewith; and/or
   a light chain variable region (VL) comprising 3 CDRs as follows: LCDR1 with the sequence of SEQ ID NO: 69 or a sequence having one or several amino acid substitutions, deletions, or additions (e.g., 1, 2, or 3 amino acid substitutions, deletions, or additions) compared therewith, LCDR2 with the sequence that is WAS or a sequence having one or several amino acid substitutions, deletions, or additions (e.g., 1, 2, or 3 amino acid substitutions, deletions, or additions) compared therewith, and LCDR3 with the sequence of SEQ ID NO: 64 or a sequence having one or several amino acid substitutions, deletions, or additions (e.g., 1, 2, or 3 amino acid substitutions, deletions, or additions) compared therewith; or
(i) a heavy chain variable region (VH) comprising 3 CDRs as follows: HCDR1 with the sequence of SEQ ID NO: 76 or a sequence having one or several amino acid substitutions, deletions, or additions (e.g., 1, 2, or 3 amino acid substitutions, deletions, or additions) compared therewith, HCDR2 with the sequence of SEQ ID NO: 77 or a sequence having one or several amino acid substitutions, deletions, or additions (e.g., 1, 2, or 3 amino acid substitutions, deletions, or additions) compared therewith, and HCDR3 with the sequence of SEQ ID NO: 78 or a sequence having one or several amino acid substitutions, deletions, or additions (e.g., 1, 2, or 3 amino acid substitutions, deletions, or additions) compared therewith; and/or
   a light chain variable region (VL) comprising 3 CDRs as follows: LCDR1 with the sequence of SEQ ID NO: 79 or a sequence having one or several amino acid substitutions, deletions, or additions (e.g., 1, 2, or 3 amino acid substitutions, deletions, or additions) compared therewith, LCDR2 with the sequence that is HTS or a sequence having one or several amino acid substitutions, deletions, or additions (e.g., 1, 2, or 3 amino acid substitutions, deletions, or additions) compared therewith, and LCDR3 with the sequence of SEQ ID NO: 16 or a sequence having one or several amino acid substitutions, deletions, or additions (e.g., 1, 2, or 3 amino acid substitutions, deletions, or additions) compared therewith.

In certain embodiments, the anti-NaPi2b antibody or the antigen-binding fragment thereof of the present disclosure comprises the following heavy chain variable region (VH) and/or light chain variable region (VL), wherein the CDRs are defined according to the IMGT numbering system:
(a) the VH comprises HCDR1 with the sequence of SEQ ID NO: 11, HCDR2 with the sequence of SEQ ID NO: 12, and HCDR3 with the sequence of SEQ ID NO: 14; and/or
   the VL comprises LCDR1 with the sequence of SEQ ID NO: 15, LCDR2 with the sequence that is WAS, and LCDR3 with the sequence of SEQ ID NO: 10;
(b) the VH comprises HCDR1 with the sequence of SEQ ID NO: 11, HCDR2 with the sequence of SEQ ID NO: 13, and HCDR3 with the sequence of SEQ ID NO: 14; and/or
   the VL comprises LCDR1 with the sequence of SEQ ID NO: 15, LCDR2 with the sequence that is WAS, and LCDR3 with the sequence of SEQ ID NO: 10;
(c) the VH comprises HCDR1 with the sequence of SEQ ID NO: 28, HCDR2 with the sequence of SEQ ID NO: 29, and HCDR3 with the sequence of SEQ ID NO: 31; and/or
   the VL comprises LCDR1 with the sequence of SEQ ID NO: 32, LCDR2 with the sequence that is YTS, and LCDR3 with the sequence of SEQ ID NO: 27;
(d) the VH comprises HCDR1 with the sequence of SEQ ID NO: 28, HCDR2 with the sequence of SEQ ID NO: 30, and HCDR3 with the sequence of SEQ ID NO: 31; and/or
   the VL comprises LCDR1 with the sequence of SEQ ID NO: 32, LCDR2 with the sequence that is YTS, and LCDR3 with the sequence of SEQ ID NO: 27;
(e) the VH comprises HCDR1 with the sequence of SEQ ID NO: 46, HCDR2 with the sequence of SEQ ID NO: 47, and HCDR3 with the sequence of SEQ ID NO: 49; and/or
   the VL comprises LCDR1 with the sequence of SEQ ID NO: 50, LCDR2 with the sequence that is SAS, and LCDR3 with the sequence of SEQ ID NO: 45;
(f) the VH comprises HCDR1 with the sequence of SEQ ID NO: 46, HCDR2 with the sequence of SEQ ID NO: 48, and HCDR3 with the sequence of SEQ ID NO: 49; and/or
   the VL comprises LCDR1 with the sequence of SEQ ID NO: 50, LCDR2 with the sequence that is SAS, and LCDR3 with the sequence of SEQ ID NO: 45;
(g) the VH comprises HCDR1 with the sequence of SEQ ID NO: 65, HCDR2 with the sequence of SEQ ID NO: 66, and HCDR3 with the sequence of SEQ ID NO: 68; and/or
   the VL comprises LCDR1 with the sequence of SEQ ID NO: 69, LCDR2 with the sequence that is WAS, and LCDR3 with the sequence of SEQ ID NO: 64;
(h) the VH comprises HCDR1 with the sequence of SEQ ID NO: 65, HCDR2 with the sequence of SEQ ID NO: 67, and HCDR3 with the sequence of SEQ ID NO: 68; and/or
   the VL comprises LCDR1 with the sequence of SEQ ID NO: 69, LCDR2 with the sequence that is WAS, and LCDR3 with the sequence of SEQ ID NO: 64;
   or
(i) the VH comprises HCDR1 with the sequence of SEQ ID NO: 76, HCDR2 with the sequence of SEQ ID NO: 77, and HCDR3 with the sequence of SEQ ID NO: 78; and/or
   the VL comprises LCDR1 with the sequence of SEQ ID NO: 79, LCDR2 with the sequence that is HTS, and LCDR3 with the sequence of SEQ ID NO: 16.

In certain embodiments, the anti-NaPi2b antibody or the antigen-binding fragment thereof of the present disclosure comprises a heavy chain variable region (VH) and/or a light chain variable region (VL), wherein at least one of CDRs in the heavy chain variable region (VH) and/or the light chain variable region (VL) comprises a mutation compared with the CDR defined by the aforementioned Kabat numbering system or IMGT numbering system, the mutation being one or several amino acid substitutions, deletions, or additions, or any combination thereof (e.g., 1, 2, or 3 amino acid substitutions, deletions, or additions, or any combination thereof), and the anti-NaPi2b antibody or the antigen-binding fragment thereof of the present disclosure still retains the activity of binding to NaPi2b.

In certain preferred embodiments, the substitution of the present disclosure is a conservative substitution.

In certain embodiments, the anti-NaPi2b antibody or the antigen-binding fragment thereof binds to human NaPi2b, monkey NaPi2b, and/or rat NaPi2b.

In certain embodiments, the VH of the anti-NaPi2b antibody or the antigen-binding fragment thereof of the present disclosure comprises a framework region (FR) derived from a heavy chain variable region (VH) of a human immunoglobulin, and/or the VL of the antibody or the antigen-binding fragment thereof comprises a framework region (FR) derived from a light chain variable region (VL) of a human immunoglobulin. Thus, in certain embodiments, the antibody or the antigen-binding fragment thereof of the present disclosure is fully human. In certain embodiments, the antibody or the antigen-binding fragment thereof of the present disclosure is humanized.

In certain embodiments, the anti-NaPi2b antibody or the antigen-binding fragment thereof of the present disclosure comprises:
(a) a heavy chain framework region of a human immunoglobulin or a variant thereof, wherein the variant has at most 20 conservative amino acid substitutions (e.g., at most 20, at most 15, at most 10, or at most 5 conservative amino acid substitutions; e.g., 1, 2, 3, 4, 5, 6, 7, 8, 9, or 10 conservative amino acid substitutions) compared with the amino acid sequence encoded by the germline antibody gene from which it is derived; and/or
(b) a light chain framework region of a human immunoglobulin or a variant thereof, wherein the variant has at most 20 conservative amino acid substitutions (e.g., at most 20, at most 15, at most 10, or at most 5 conservative amino acid substitutions; e.g., 1, 2, 3, 4, 5, 6, 7, 8, 9, or 10 conservative amino acid substitutions) compared with the amino acid sequence encoded by the germline antibody gene from which it is derived.

In certain embodiments, the degree of humanization of the anti-NaPi2b antibody or the antigen-binding fragment thereof of the present disclosure is at least 75%, at least 80%, at least 85%, at least 90%, at least 91%, at least 92%, at least 93%, at least 94%, at least 95%, at least 96%, at least 97%, at least 98%, or at least 99%.

In certain embodiments, the anti-NaPi2b antibody or the antigen-binding fragment thereof of the present disclosure comprises:
(a) a heavy chain variable region (VH) comprising or consisting of an amino acid sequence selected from the following:
   (i) the sequence set forth in SEQ ID NO: 1, 17, 19, 34, 36, 52, 54, 71, 73, 74, or 33;
   (ii) a sequence having one or several amino acid substitutions, deletions, or additions, or any combination thereof (e.g., 1, 2, 3, 4, 5, 6, 7, 8, 9, or 10 amino acid substitutions, deletions, or additions, or any combination thereof), compared with the sequence set forth in SEQ ID NO: 1, 17, 19, 34, 36, 52, 54, 71, 73, 74, or 33; or
   (iii) a sequence having at least 70%, at least 75%, at least 80%, at least 85%, at least 90%, at least 91%, at least 92%, at least 93%, at least 94%, at least 95%, at least 96%, at least 97%, at least 98%, or at least 99% sequence identity compared with the sequence set forth in SEQ ID NO: 1, 17, 19, 34, 36, 52, 54, 71, 73, 74, or 33;
      and/or
(b) a light chain variable region (VL) comprising or consisting of an amino acid sequence selected from the following:
   (iv) the sequence set forth in SEQ ID NO: 2, 18, 20, 35, 37, 53, 55, 72, 75, or 51;
   (v) a sequence having one or several amino acid substitutions, deletions, or additions, or any combination thereof (e.g., 1, 2, 3, 4, 5, 6, 7, 8, 9, or 10 amino acid substitutions, deletions, or additions, or any combination thereof), compared with the sequence set forth in SEQ ID NO: 2, 18, 20, 35, 37, 53, 55, 72, 75, or 51; or
   (vi) a sequence having at least 70%, at least 75%, at least 80%, at least 85%, at least 90%, at least 91%, at least 92%, at least 93%, at least 94%, at least 95%, at least 96%, at least 97%, at least 98%, or at least 99% sequence identity compared with the sequence set forth in SEQ ID NO: 2, 18, 20, 35, 37, 53, 55, 72, 75, or 51.

In certain embodiments, the anti-NaPi2b antibody or the antigen-binding fragment thereof of the present disclosure comprises the VH set forth in SEQ ID NO: 1 or 17, and/or the VL set forth in SEQ ID NO: 2 or 18.

In certain embodiments, the anti-NaPi2b antibody or the antigen-binding fragment thereof of the present disclosure comprises the VH set forth in SEQ ID NO: 19 or 34, and/or the VL set forth in SEQ ID NO: 20 or 35.

In certain embodiments, the anti-NaPi2b antibody or the antigen-binding fragment thereof of the present disclosure comprises the VH set forth in SEQ ID NO: 36 or 52, and/or the VL set forth in SEQ ID NO: 37 or 53.

In certain embodiments, the anti-NaPi2b antibody or the antigen-binding fragment thereof of the present disclosure comprises the VH set forth in SEQ ID NO: 54, 71, or 73, and/or the VL set forth in SEQ ID NO: 55 or 72.

In certain embodiments, the anti-NaPi2b antibody or the antigen-binding fragment thereof of the present disclosure comprises the VH set forth in SEQ ID NO: 74 or 33, and/or the VL set forth in SEQ ID NO: 75 or 51.

In certain embodiments, the anti-NaPi2b antibody or the antigen-binding fragment thereof of the present disclosure comprises a VH having at least 70%, at least 80%, at least 85%, at least 90%, at least 91%, at least 92%, at least 93%, at least 94%, at least 95%, at least 96%, at least 97%, at least 98%, or at least 99% sequence identity compared with the VH of any one of the 5 scheme groups described above; and/or a VL having at least 70%, at least 80%, at least 85%, at least 90%, at least 91%, at least 92%, at least 93%, at least 94%, at least 95%, at least 96%, at least 97%, at least 98%, or at least 99% sequence identity compared with the VL of that same group; or

in certain embodiments, the anti-NaPi2b antibody or the antigen-binding fragment thereof of the present disclosure comprises a VH having one or several amino acid substitutions, deletions, or additions, or any combination thereof (e.g., 1, 2, 3, 4, 5, 6, 7, 8, 9, or 10 amino acid substitutions, deletions, or additions, or any combination thereof), compared with the VH of any one of the 5 scheme groups described above; and/or a VL having one or several amino acid substitutions, deletions, or additions, or any combination thereof (e.g., 1, 2, 3, 4, 5, 6, 7, 8, 9, or 10 amino acid substitutions, deletions, or additions, or any combination thereof), compared with the VL of that same group; in certain preferred embodiments, the substitution is a conservative substitution.

In certain embodiments, the anti-NaPi2b antibody or the antigen-binding fragment thereof of the present disclosure comprises:
(a) the VH with the sequence set forth in SEQ ID NO: 1 and the VL with the sequence set forth in SEQ ID NO: 2;
(b) the VH with the sequence set forth in SEQ ID NO: 17 and the VL with the sequence set forth in SEQ ID NO: 18;
(c) the VH with the sequence set forth in SEQ ID NO: 19 and the VL with the sequence set forth in SEQ ID NO: 20;
(d) the VH with the sequence set forth in SEQ ID NO: 34 and the VL with the sequence set forth in SEQ ID NO: 35;
(e) the VH with the sequence set forth in SEQ ID NO: 36 and the VL with the sequence set forth in SEQ ID NO: 37;
(f) the VH with the sequence set forth in SEQ ID NO: 52 and the VL with the sequence set forth in SEQ ID NO: 53;
(g) the VH with the sequence set forth in SEQ ID NO: 54 and the VL with the sequence set forth in SEQ ID NO: 55;
(h) the VH with the sequence set forth in SEQ ID NO: 71 and the VL with the sequence set forth in SEQ ID NO: 72;
(i) the VH with the sequence set forth in SEQ ID NO: 73 and the VL with the sequence set forth in SEQ ID NO: 72;
(j) the VH with the sequence set forth in SEQ ID NO: 74 and the VL with the sequence set forth in SEQ ID NO: 75;
(k) the VH with the sequence set forth in SEQ ID NO: 33 and the VL with the sequence set forth in SEQ ID NO: 51;
(l) a heavy chain variable region (VH) and a light chain variable region (VL), wherein the heavy chain variable region (VH) and the light chain variable region (VL) independently have at least 70%, at least 80%, at least 85%, at least 90%, at least 91%, at least 92%, at least 93%, at least 94%, at least 95%, at least 96%, at least 97%, at least 98%, or at least 99% sequence identity compared with the VH and the VL in any one of groups (a) to (k), respectively; or
(m) a heavy chain variable region (VH) and a light chain variable region (VL), wherein the heavy chain variable region (VH) and the light chain variable region (VL) independently have one or several amino acid substitutions, deletions, or additions, or any combination thereof (e.g., 1, 2, 3, 4, 5, 6, 7, 8, 9, or 10 amino acid substitutions, deletions, or additions, or any combination thereof), compared with the VH and the VL in any one of groups (a) to (k), respectively. In certain preferred embodiments, the substitution is a conservative substitution.

In certain embodiments, the anti-NaPi2b antibody of the present disclosure is a chimeric antibody, a humanized antibody, or a fully human antibody. In certain embodiments, the antibody or the antigen-binding fragment thereof of the present disclosure is selected from a Fab, a Fab', a (Fab')₂, an Fv fragment (e.g., scFv or disulfide-linked Fv (dsFv)), a diabody, and a multispecific antibody. In certain embodiments, the anti-NaPi2b antibody of the present disclosure is an scFv.

In certain embodiments, a heavy chain of the anti-NaPi2b antibody or the antigen-binding fragment thereof of the present disclosure comprises a heavy chain constant region (CH) of a human immunoglobulin or a variant thereof, wherein the variant has at most 50 conservative amino acid substitutions (e.g., at most 45, at most 40, at most 35, at most 30, at most 25, at most 20, at most 15, at most 10, or at most 5 conservative amino acid substitutions; e.g., 1, 2, 3, 4, 5, 6, 7, 8, 9, or 10 conservative amino acid substitutions) compared with the wild-type sequence from which it is derived. In certain embodiments, a light chain of the anti-NaPi2b antibody or the antigen-binding fragment thereof of the present disclosure comprises a light chain constant region (CL) of a human immunoglobulin or a variant thereof, wherein the variant has at most 50 conservative amino acid substitutions (e.g., at most 45, at most 40, at most 35, at most 30, at most 25, at most 20, at most 15, at most 10, or at most 5 conservative amino acid substitutions; e.g., 1, 2, 3, 4, 5, 6, 7, 8, 9, or 10 conservative amino acid substitutions) compared with the wild-type sequence from which it is derived.

In some embodiments, the constant region is altered, for example, by mutation, to modify properties of the anti-NaPi2b antibody molecule (e.g., to alter one or more of the following characteristics: Fc receptor binding, antibody glycosylation, the number of cysteine residues, effector cell functions, or complement functions). Functional alterations can be achieved by replacing at least one amino acid residue in the constant region of the antibody with a different residue, such as altering the affinity of the antibody for effector ligands (e.g., FcR or complement C1q), thereby altering effector functions (e.g., reducing them). The Fc region of an antibody mediates several important effector functions, such as ADCC, phagocytosis (ADCP), CDC, etc.

In certain embodiments, the anti-NaPi2b antibody or the antigen-binding fragment thereof of the present disclosure has a heavy chain constant region (CH) selected from, for example, heavy chain constant regions of IgG1, IgG2, IgG3, IgG4, IgM, IgA1, IgA2, IgD, and IgE, particularly selected from, for example, heavy chain constant regions of IgG1, IgG2, IgG3, and IgG4, and more particularly selected from a heavy chain constant region of IgG1 (e.g., human IgG1). In some embodiments, the human IgG1 heavy chain constant region is set forth in SEQ ID NO: 70. In some embodiments, the anti-NaPi2b antibody or the antigen-binding fragment thereof of the present disclosure has a light chain constant region selected from, for example, κ and λ light chain constant regions, preferably a κ light chain constant region (e.g., human κ light chain constant region). In some embodiments, the light chain constant region has the sequence set forth in SEQ ID NO: 80.

In some embodiments, the anti-NaPi2b antibody or the antigen-binding fragment thereof comprises the CH set forth in SEQ ID NO: 70 or a variant thereof, wherein the variant has at most 20 conservative amino acid substitutions (e.g., at most 20, at most 15, at most 10, or at most 5 conservative amino acid substitutions; e.g., 1, 2, 3, 4, 5, 6, 7, 8, 9, or 10 conservative amino acid substitutions) compared with SEQ ID NO: 70, or has at least 70%, at least 80%, at least 85%, at least 90%, at least 91%, at least 92%, at least 93%, at least 94%, at least 95%, at least 96%, at least 97%, at least 98%, or at least 99% sequence identity compared with SEQ ID NO: 70.

In some embodiments, the anti-NaPi2b antibody or the antigen-binding fragment thereof comprises a light chain constant region or a variant thereof. In some embodiments, the light chain constant region comprises a κ light chain constant region. In some embodiments, the light chain constant region comprises the light chain constant region (CL) set forth in SEQ ID NO: 80 or a variant thereof, wherein the variant has at most 20 conservative amino acid substitutions (e.g., at most 20, at most 15, at most 10, or at most 5 conservative amino acid substitutions; e.g., 1, 2, 3, 4, 5, 6, 7, 8, 9, or 10 conservative amino acid substitutions) compared with SEQ ID NO: 80, or has at least 70%, at least 80%, at least 85%, at least 90%, at least 91%, at least 92%, at least 93%, at least 94%, at least 95%, at least 96%, at least 97%, at least 98%, or at least 99% sequence identity compared with SEQ ID NO: 80.

In some embodiments, the anti-NaPi2b antibody or the antigen-binding fragment thereof comprises the heavy chain constant region (CH) set forth in SEQ ID NO: 70 and the light chain constant region (CL) set forth in SEQ ID NO: 80.

In certain embodiments, the anti-NaPi2b antibody or the antigen-binding fragment thereof of the present disclosure comprises:
(a) a heavy chain comprising or consisting of an amino acid sequence selected from the following:
   (i) a sequence comprising the VH sequence set forth in SEQ ID NO: 71 and the CH sequence set forth in SEQ ID NO: 70;
   (ii) a sequence having one or several amino acid substitutions, deletions, or additions, or any combination thereof (e.g., at most 50, at most 45, at most 40, at most 35, at most 30, at most 25, at most 20, at most 15, at most 10, or at most 5 amino acid substitutions, deletions, or additions, or any combination thereof; e.g., 1, 2, 3, 4, 5, 6, 7, 8, 9, or 10 amino acid substitutions, deletions, or additions, or any combination thereof), compared with the sequence shown in (i); or
   (iii) a sequence having at least 80%, at least 85%, at least 90%, at least 91%, at least 92%, at least 93%, at least 94%, at least 95%, at least 96%, at least 97%, at least 98%, or at least 99% sequence identity compared with the sequence shown in (i); and
(b) a light chain comprising an amino acid sequence selected from the following:
   (iv) a sequence comprising the VL sequence set forth in SEQ ID NO: 72 and the CL sequence set forth in SEQ ID NO: 80;
   (v) a sequence having one or several amino acid substitutions, deletions, or additions, or any combination thereof (e.g., at most 50, at most 45, at most 40, at most 35, at most 30, at most 25, at most 20, at most 15, at most 10, or at most 5 amino acid substitutions, deletions, or additions, or any combination thereof; e.g., 1, 2, 3, 4, 5, 6, 7, 8, 9, or 10 amino acid substitutions, deletions, or additions, or any combination thereof), compared with the sequence shown in (iv); or
   (vi) a sequence having at least 80%, at least 85%, at least 90%, at least 91%, at least 92%, at least 93%, at least 94%, at least 95%, at least 96%, at least 97%, at least 98%, or at least 99% sequence identity compared with the sequence shown in (iv).

In certain embodiments, the substitution described in (ii) or (v) is a conservative substitution.

In certain embodiments, the anti-NaPi2b antibody or the antigen-binding fragment thereof of the present disclosure comprises:
(a) a heavy chain comprising or consisting of an amino acid sequence selected from the following:
   (i) a sequence comprising the VH sequence set forth in SEQ ID NO: 73 and the CH sequence set forth in SEQ ID NO: 70;
   (ii) a sequence having one or several amino acid substitutions, deletions, or additions, or any combination thereof (e.g., at most 50, at most 45, at most 40, at most 35, at most 30, at most 25, at most 20, at most 15, at most 10, or at most 5 amino acid substitutions, deletions, or additions, or any combination thereof; e.g., 1, 2, 3, 4, 5, 6, 7, 8, 9, or 10 amino acid substitutions, deletions, or additions, or any combination thereof), compared with the sequence shown in (i); or
   (iii) a sequence having at least 80%, at least 85%, at least 90%, at least 91%, at least 92%, at least 93%, at least 94%, at least 95%, at least 96%, at least 97%, at least 98%, or at least 99% sequence identity compared with the sequence shown in (i); and
(b) a light chain comprising or consisting of an amino acid sequence selected from the following:
   (iv) a sequence comprising the VL sequence set forth in SEQ ID NO: 72 and the CL sequence set forth in SEQ ID NO: 80;
   (v) a sequence having one or several amino acid substitutions, deletions, or additions, or any combination thereof (e.g., at most 50, at most 45, at most 40, at most 35, at most 30, at most 25, at most 20, at most 15, at most 10, or at most 5 amino acid substitutions, deletions, or additions, or any combination thereof; e.g., 1, 2, 3, 4, 5, 6, 7, 8, 9, or 10 amino acid substitutions, deletions, or additions, or any combination thereof), compared with the sequence shown in (iv); or
   (vi) a sequence having at least 80%, at least 85%, at least 90%, at least 91%, at least 92%, at least 93%, at least 94%, at least 95%, at least 96%, at least 97%, at least 98%, or at least 99% sequence identity compared with the sequence shown in (iv).

In certain embodiments, the substitution described in (ii) or (v) is a conservative substitution.

In certain embodiments, the anti-NaPi2b antibody or the antigen-binding fragment thereof of the present disclosure comprises:
(a) a heavy chain comprising or consisting of an amino acid sequence selected from the following:
   (i) a sequence comprising the VH sequence set forth in SEQ ID NO: 17 and the CH sequence set forth in SEQ ID NO: 70;
   (ii) a sequence having one or several amino acid substitutions, deletions, or additions, or any combination thereof (e.g., at most 50, at most 45, at most 40, at most 35, at most 30, at most 25, at most 20, at most 15, at most 10, or at most 5 amino acid substitutions, deletions, or additions, or any combination thereof; e.g., 1, 2, 3, 4, 5, 6, 7, 8, 9, or 10 amino acid substitutions, deletions, or additions, or any combination thereof), compared with the sequence shown in (i); or
   (iii) a sequence having at least 80%, at least 85%, at least 90%, at least 91%, at least 92%, at least 93%, at least 94%, at least 95%, at least 96%, at least 97%, at least 98%, or at least 99% sequence identity compared with the sequence shown in (i); and
(b) a light chain comprising or consisting of an amino acid sequence selected from the following:
   (iv) a sequence comprising the VL sequence set forth in SEQ ID NO: 18 and the CL sequence set forth in SEQ ID NO: 80;
   (v) a sequence having one or several amino acid substitutions, deletions, or additions, or any combination thereof (e.g., at most 50, at most 45, at most 40, at most 35, at most 30, at most 25, at most 20, at most 15, at most 10, or at most 5 amino acid substitutions, deletions, or additions, or any combination thereof; e.g., 1, 2, 3, 4, 5, 6, 7, 8, 9, or 10 amino acid substitutions, deletions, or additions, or any combination thereof), compared with the sequence shown in (iv); or
   (vi) a sequence having at least 80%, at least 85%, at least 90%, at least 91%, at least 92%, at least 93%, at least 94%, at least 95%, at least 96%, at least 97%, at least 98%, or at least 99% sequence identity compared with the sequence shown in (iv).

In certain embodiments, the substitution described in (ii) or (v) is a conservative substitution.

In certain embodiments, the anti-NaPi2b antibody or the antigen-binding fragment thereof of the present disclosure comprises a heavy chain and a light chain,
wherein the heavy chain comprises or consists of the following sequences:
   (i) the sequence set forth in SEQ ID NO: 81;
   (ii) a sequence having one or several amino acid substitutions, deletions, or additions, or any combination thereof (e.g., at most 50, at most 45, at most 40, at most 35, at most 30, at most 25, at most 20, at most 15, at most 10, or at most 5 amino acid substitutions, deletions, or additions, or any combination thereof; e.g., 1, 2, 3, 4, 5, 6, 7, 8, 9, or 10 amino acid substitutions, deletions, or additions, or any combination thereof), compared with the sequence shown in (i); or
   (iii) a sequence having at least 80%, at least 85%, at least 90%, at least 91%, at least 92%, at least 93%, at least 94%, at least 95%, at least 96%, at least 97%, at least 98%, or at least 99% sequence identity compared with the sequence shown in (i); and
the light chain comprises or consists of the following sequences:
   (iv) the sequence set forth in SEQ ID NO: 82;
   (v) a sequence having one or several amino acid substitutions, deletions, or additions, or any combination thereof (e.g., at most 50, at most 45, at most 40, at most 35, at most 30, at most 25, at most 20, at most 15, at most 10, or at most 5 amino acid substitutions, deletions, or additions, or any combination thereof; e.g., 1, 2, 3, 4, 5, 6, 7, 8, 9, or 10 amino acid substitutions, deletions, or additions, or any combination thereof), compared with the sequence shown in (iv); or
   (vi) a sequence having at least 80%, at least 85%, at least 90%, at least 91%, at least 92%, at least 93%, at least 94%, at least 95%, at least 96%, at least 97%, at least 98%, or at least 99% sequence identity compared with the sequence shown in (iv).

In certain preferred embodiments, the substitution described in (ii) or (v) is a conservative substitution.

In certain embodiments, the anti-NaPi2b antibody or the antigen-binding fragment thereof of the present disclosure comprises a heavy chain and a light chain,
wherein the heavy chain comprises or consists of the following sequences:
   (i) the sequence set forth in SEQ ID NO: 83;
   (ii) a sequence having one or several amino acid substitutions, deletions, or additions, or any combination thereof (e.g., at most 50, at most 45, at most 40, at most 35, at most 30, at most 25, at most 20, at most 15, at most 10, or at most 5 amino acid substitutions, deletions, or additions, or any combination thereof; e.g., 1, 2, 3, 4, 5, 6, 7, 8, 9, or 10 amino acid substitutions, deletions, or additions, or any combination thereof), compared with the sequence shown in (i); or
   (iii) a sequence having at least 80%, at least 85%, at least 90%, at least 91%, at least 92%, at least 93%, at least 94%, at least 95%, at least 96%, at least 97%, at least 98%, or at least 99% sequence identity compared with the sequence shown in (i); and
the light chain comprises or consists of the following sequences:
   (iv) the sequence set forth in SEQ ID NO: 84;
   (v) a sequence having one or several amino acid substitutions, deletions, or additions, or any combination thereof (e.g., at most 50, at most 45, at most 40, at most 35, at most 30, at most 25, at most 20, at most 15, at most 10, or at most 5 amino acid substitutions, deletions, or additions, or any combination thereof; e.g., 1, 2, 3, 4, 5, 6, 7, 8, 9, or 10 amino acid substitutions, deletions, or additions, or any combination thereof), compared with the sequence shown in (iv); or
   (vi) a sequence having at least 80%, at least 85%, at least 90%, at least 91%, at least 92%, at least 93%, at least 94%, at least 95%, at least 96%, at least 97%, at least 98%, or at least 99% sequence identity compared with the sequence shown in (iv).

In certain preferred embodiments, the substitution described in (ii) or (v) is a conservative substitution.

In certain embodiments, provided is the anti-NaPi2b antibody or the antigen-binding fragment thereof, wherein the antibody comprises a heavy chain and a light chain selected from the following groups:
(a) a heavy chain comprising the VH set forth in SEQ ID NO: 71 and the CH set forth in SEQ ID NO: 70, and a light chain comprising the VL set forth in SEQ ID NO: 72 and the CL set forth in SEQ ID NO: 80;
(b) a heavy chain comprising the VH set forth in SEQ ID NO: 73 and the CH set forth in SEQ ID NO: 70, and a light chain comprising the VL set forth in SEQ ID NO: 72 and the CL set forth in SEQ ID NO: 80, preferably the heavy chain set forth in SEQ ID NO: 81 and the light chain set forth in SEQ ID NO: 82; and
(c) a heavy chain comprising the VH set forth in SEQ ID NO: 17 and the CH set forth in SEQ ID NO: 70, and a light chain comprising the VL set forth in SEQ ID NO: 18 and the CL set forth in SEQ ID NO: 80, preferably the heavy chain set forth in SEQ ID NO: 83 and the light chain set forth in SEQ ID NO: 84.

In certain embodiments, the antibody provided by the present disclosure is a multispecific antibody that binds to NaPi2b and one or more other antigens. In certain preferred embodiments, the multispecific antibody is a bispecific antibody or a trispecific antibody or a tetraspecific antibody. In some embodiments, the multispecific antibody of the present disclosure comprises the anti-NaPi2b antibody or the antigen-binding fragment thereof described above, and an additional antibody or a fragment thereof or an antibody analog thereof.

### Antibody derivatives of the present disclosure

The anti-NaPi2b antibody or the antigen-binding fragment thereof of the present disclosure may be derivatized, for example, by being attached to another molecule (e.g., another polypeptide or protein). In general, derivatization (e.g., labeling) of an antibody or an antigen-binding fragment thereof does not adversely affect its binding to NaPi2b. Thus, the anti-NaPi2b antibody or the antigen-binding fragment thereof of the present disclosure is also intended to include such derivatized forms. For example, the anti-NaPi2b antibody or the antigen-binding fragment thereof of the present disclosure may be attached (through chemical coupling, genetic fusion, non-covalent linking, or other methods) to one or more other molecular groups, such as another antibody (e.g., to form a bispecific antibody), a detection reagent, a pharmaceutical reagent, and/or a protein or polypeptide capable of mediating the binding of the antibody or the antigen-binding fragment to another molecule (e.g., avidin or a polyhistidine tag).

One type of derivatized antibody (e.g., bispecific antibody) is produced by cross-linking 2 or more antibodies (of the same type or different types). Methods for obtaining bispecific antibodies are well known in the art, and examples thereof include, but are not limited to, chemical cross-linking methods, cell engineering methods (hybridoma method), or genetic engineering methods.

Another type of derivatized antibody is a labeled antibody. For example, the antibody or the antigen-binding fragment thereof of the present disclosure may be attached to a detectable label. The detectable label of the present disclosure may be any substance that can be detected through fluorescent, spectral, photochemical, biochemical, immunological, electrical, optical, or chemical means. Such labels are well known in the art, and examples thereof include, but are not limited to, enzymes (e.g., horseradish peroxidase, alkaline phosphatase, β-galactosidase, urease, glucose oxidase, etc.), redionuclides (e.g., 3H, 125I, 35S, 14C, or 32P), fluorescent dyes (e.g., fluorescein isothiocyanate (FITC), fluorescein, tetramethylrhodamine isothiocyanate (TRITC), phycoerythrin (PE), Texas Red, rhodamine, quantum dots, or cyanine dye derivatives (e.g., Cy7 and Alexa 750)), acridinium ester compounds, magnetic beads (e.g., Dynabeads^{®}), calorimetric labels such as colloidal gold or colored glass or plastic (e.g., polystyrene, polypropylene, latex, etc.) beads, and biotin for binding to avidin (e.g., streptavidin) modified by the label(s) described above. Patents teaching the use of such labels include, but are not limited to, U.S. Pat. Nos. 3,817,837, 3,850,752, 3,939,350, 3,996,345, 4,277,437, 4,275,149, and 4,366,241 (which are incorporated herein by reference in their entirety). The detectable labels described above can be detected by methods known in the art. For example, the radioactive labels can be detected using photographic film or a scintillation counter, and the fluorescent labels can be detected using a photodetector to detect the emitted light. The enzyme labels are generally detected by providing a substrate for the enzyme and detecting the reaction product produced by the action of the enzyme on the substrate, and the calorimetric labels are detected by simply visualizing colored labels. In certain embodiments, such labels can be suitable for immunological detection (e.g., enzyme-linked immunoassay, radioimmunoassay, fluorescent immunoassay, chemiluminescent immunoassay, etc.). In certain embodiments, the detectable labels described above can be attached to the antibody or the antigen-binding fragment thereof of the present disclosure via linkers of varying lengths to reduce potential steric hindrance.

In addition, the antibody or the antigen-binding fragment thereof of the present disclosure may also be derivatized with chemical groups, such as polyethylene glycol (PEG), methyl or ethyl, or glycosyl. These groups can be used to improve the biological properties of the antibody, such as increasing serum half-life.

### Preparation of antibodies

The antibodies of the present disclosure can be prepared by various methods known in the art, such as by a genetic engineering recombination technique. For example, DNA molecules encoding the heavy chain and light chain genes of the antibody of the present disclosure are obtained by chemical synthesis or PCR amplification. The resulting DNA molecule is inserted into an expression vector, which is then transfected into a host cell. The transfected host cell is then cultured under specific conditions and expresses the antibody of the present disclosure.

The antigen-binding fragments of the present disclosure can be obtained by hydrolysis of an intact antibody molecule (see Morimoto et al., J. Biochem. Biophys. Methods, 24: 107-117 (1992) and Brennan et al., Science, 229: 81 (1985)). In addition, these antigen-binding fragments can be produced directly from recombinant host cells (reviewed in Hudson, Curr. Opin. Immunol., 11: 548-557 (1999); Little et al., Immunol. Today, 21: 364-370 (2000)). For example, Fab' fragments can be obtained directly from host cells; Fab' fragments can be chemically coupled to form F(ab')₂ fragments (Carter et al., Bio/Technology, 10: 163-167 (1992)). In addition, Fv, Fab, or F(ab')₂ fragments can also be isolated directly from the culture medium of recombinant host cells. Other techniques for preparing these antigen-binding fragments are well known to those of ordinary skill in the art.

In a third aspect, the present disclosure provides an isolated nucleic acid molecule comprising a nucleotide sequence encoding the antibody or the antigen-binding fragment thereof, or the heavy chain variable region and/or the light chain variable region thereof, or one or more CDRs thereof according to the second aspect of the present disclosure; in certain embodiments, the nucleotide sequence can be replaced according to codon degeneracy. In certain embodiments, the nucleotide sequence is codon-optimized.

In certain embodiments, the isolated nucleic acid molecule of the present disclosure comprises: (i) a first nucleic acid and a second nucleic acid encoding the heavy chain variable region and the light chain variable region, respectively, of the antibody or the antigen-binding fragment thereof according to the second aspect of the present disclosure, or (ii) a first nucleic acid and a second nucleic acid encoding the heavy chain variable region and the heavy chain constant region, as well as the light chain variable region and the light chain constant region, respectively, of the antibody or the antigen-binding fragment thereof according to the second aspect of the present disclosure, or (iii) a first nucleic acid and a second nucleic acid encoding the heavy chain and the light chain, respectively, of the antibody or the antigen-binding fragment thereof according to the second aspect of the present disclosure. In certain embodiments, the first nucleic acid and the second nucleic acid comprise nucleic acids with degenerate sequences or substantially identical sequences relative to the first nucleic acid and the second nucleic acid in any one of (i) to (iii) described above. In certain embodiments, the degenerate sequence or the substantially identical sequence refers to a sequence having at least about 85%, 90%, 95%, 99%, or higher sequence identity, or having one or more nucleotide substitutions, or having a difference in no more than 3, 6, 15, 30, or 45 nucleotides, compared with the nucleic acid molecule in (i) to (iii).

In a fourth aspect, provided is a vector (e.g., cloning vector or expression vector) comprising the isolated nucleic acid molecule according to the third aspect of the present disclosure. In certain embodiments, the vector of the present disclosure is a cloning vector or an expression vector. In certain embodiments, the vector of the present disclosure is, for example, a plasmid, a cosmid, a phage, a lentivirus, etc. In certain embodiments, the vector is capable of expressing the antibody or the antigen-binding fragment thereof of the present disclosure in a subject (e.g., a mammal, such as a human).

In a fifth aspect, provided is a host cell comprising the isolated nucleic acid molecule according to the third aspect of the present disclosure or the vector according to the fourth aspect of the present disclosure. The host cell may be a eukaryotic cell (e.g., mammalian cell, insect cell, or yeast cell) or a prokaryotic cell (e.g., *E*. *coli*)*.* Suitable eukaryotic cells include, but are not limited to, NS0 cells, SP2/0 cells, Vero cells, Hela cells, COS cells, CHO cells, HEK293 cells, BHK cells, and MDCKII cells. Suitable insect cells include, but are not limited to, Sf9 cells. In certain embodiments, the host cell of the present disclosure is a mammalian cell, such as CHO (e.g., CHO-K1, CHO-S, CHO DXB11, or CHO DG44).

In a sixth aspect, provided is a method for preparing the antibody or the antigen-binding fragment thereof according to the second aspect of the present disclosure, or the multispecific antibody of the present disclosure, which comprises culturing the host cell of the present disclosure under conditions allowing expression of the antibody or the antigen-binding fragment thereof, or the multispecific antibody, and isolating the antibody or the antigen-binding fragment thereof from the cultured host cell culture.

### Antibody-drug conjugate

In a seventh aspect, provided is an anti-NaPi2b antibody-drug conjugate, a stereoisomer thereof, a prodrug thereof, a pharmaceutically acceptable salt thereof, or a pharmaceutically acceptable solvate thereof, wherein the antibody is the antibody or the antigen-binding fragment thereof that binds to NaPi2b described above.

In some embodiments, the antibody-drug conjugate is selected from: 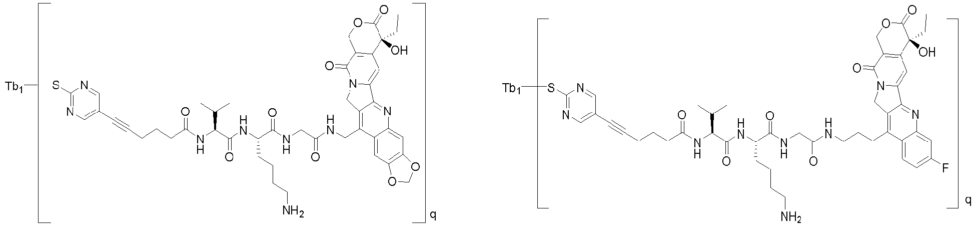 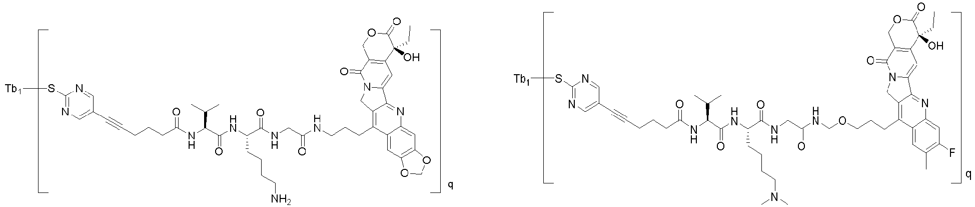 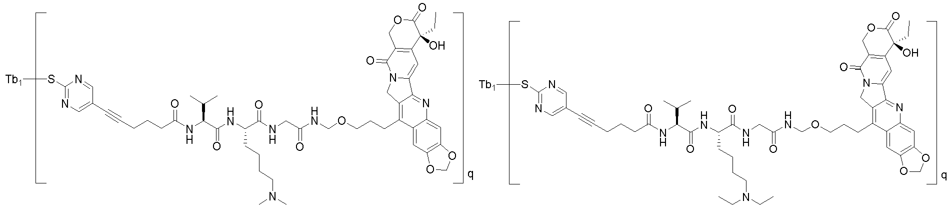 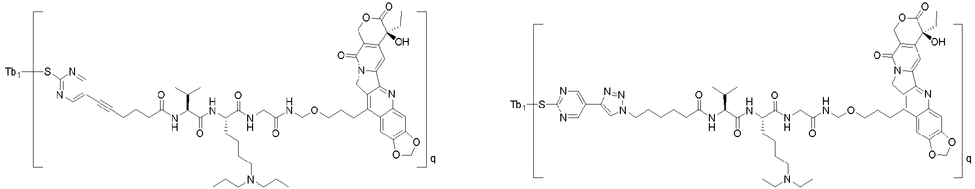 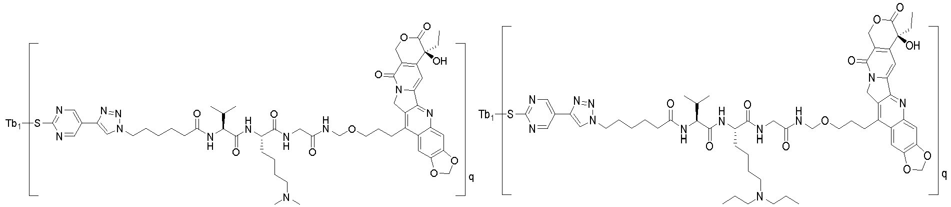 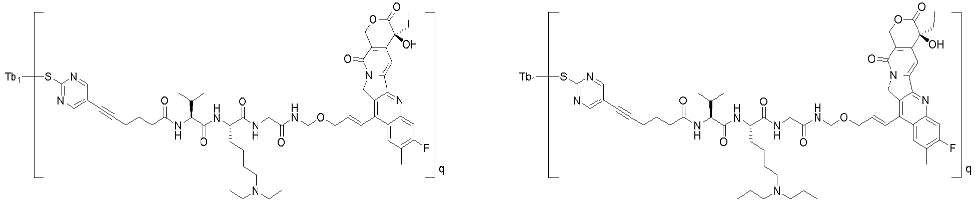 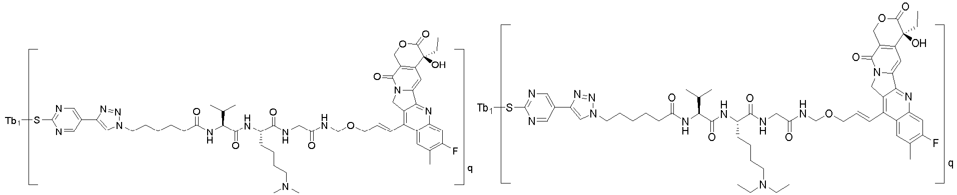 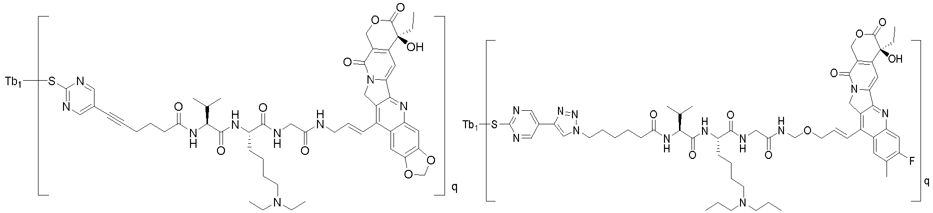 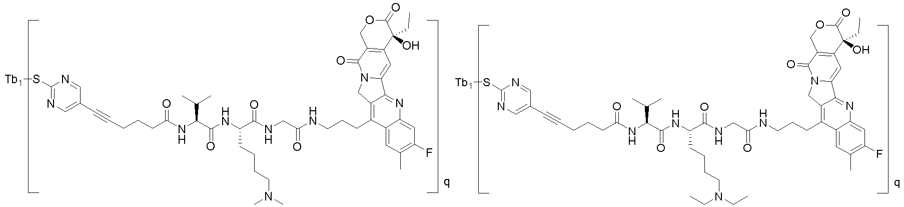 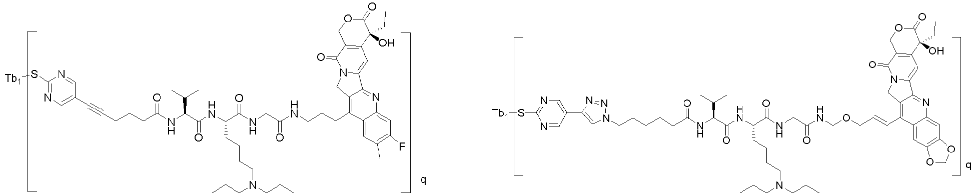 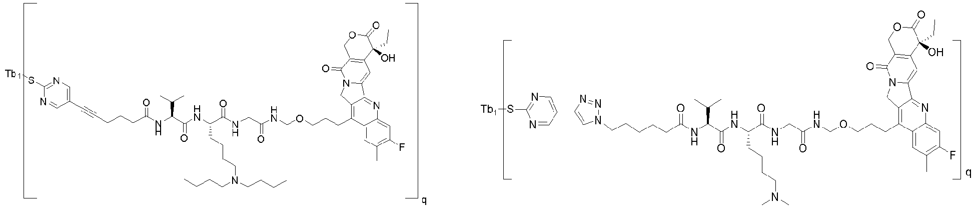 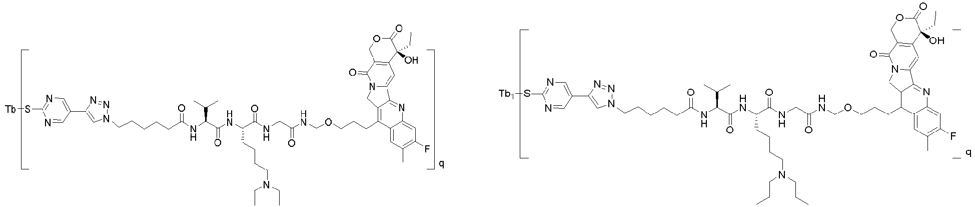 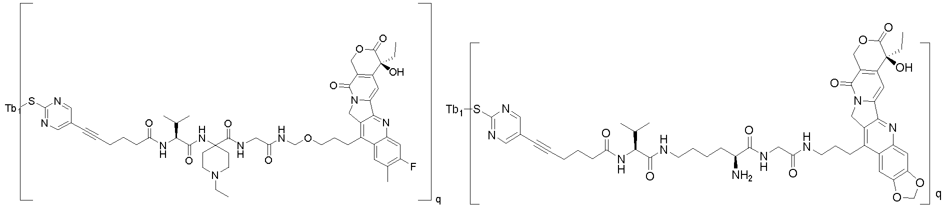 wherein
Tb₁ is an anti-NaPi2b antibody or an antigen-binding fragment thereof, such as lifastuzumab, upifitamab, 60A12B3-hz1, 22H9C4-hz1, 66C12D12-hz1, 34F2A10-hz1, C1, 16G5B3-hz2, and 16G5B3-hz1 antibody.
q is selected from any numerical value between 0.1 and 16.0, preferably any numerical value between 2 and 8. In some embodiments, q is 1, 2, 3, 4, 5, 6, 7, 8, 9, or 10. In some preferred embodiments, q is 2, 4, 6, or 8.

### Preparation of antibody-drug conjugate

In an eighth aspect of the present disclosure, the present disclosure provides a method for preparing the aforementioned antibody-drug conjugate, which comprises:
subjecting Tb and a drug-linker conjugate of formula III 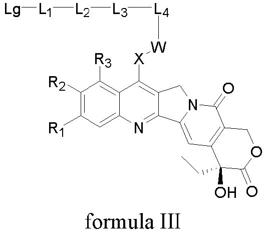
to a conjugation reaction in a suitable solvent and under suitable conditions,
wherein:
   Tb is as defined in any embodiment specifically described above and herein;
   R₁, R₂, R₃, X, W, L₁, L₂, L₃, and L₄ are as defined in any embodiment specifically described above and hereinLg is a leaving group, and Lg is selected from halogen, a sulfone group, a tertiary amine salt group (Me₃N⁺ or Et₃N⁺), a diazonium salt group, -OMs, MeSO₂-, and CF₃SO₃-.

In some embodiments, Lg is selected from F, Cl, and MeSO2-.

In some embodiments, Lg is selected from F and MeSO2-.

In some embodiments, the method comprises a step of subjecting Tb and the drug-linker conjugate of formula III 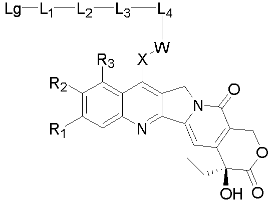 to the conjugation reaction in the suitable solvent and under the suitable conditions to form a C-S bond.

In some embodiments, Tb and the drug-linker conjugate are in a molar ratio of 1:(1-20), such as 1:2, 1:3, 1:4, 1:5, 1:6, 1:7, 1:8, 1:9, 1:10, 1:12, 1:14, 1:16, 1:18, 1:(10-20), 1:(12-20), 1:(14-20), 1:(16-20), or 1:(18-20).

In some embodiments, the conjugation reaction is carried out in water and/or an organic solvent.

In some embodiments, the organic solvent is selected from *N,N-*dimethylformamide, dimethyl sulfoxide, N-methylpyrrolidone, nitriles (e.g., acetonitrile), alcohols (e.g., methanol and ethanol), and any combination thereof.

In some embodiments, the method further comprises a step of purifying the conjugate product.

In some embodiments, the conjugate product is purified by chromatography.

In some embodiments, the chromatography comprises one or more of ion exchange chromatography, hydrophobic chromatography, reversed-phase chromatography, or affinity chromatography.

In a ninth aspect of the present disclosure, the present disclosure provides a population of antibody-drug conjugates, comprising the antibody-drug conjugate, the stereoisomer thereof, the prodrug thereof, the pharmaceutically acceptable salt thereof, or the pharmaceutically acceptable solvate thereof described above in the present disclosure, wherein the antibody-drug conjugates have one, two, or more q values.

In some embodiments, when the antibody-drug conjugates in the population have a single q value, the q value is equal to the DAR value.

In some embodiments, when the antibody-drug conjugates in the population have two or more q values, the proportion of antibody-drug conjugates with a specific q value among all antibody-drug conjugates in the population is greater than 50%, 60%, 70%, 80%, 90%, 95%, 96%, 97%, 98%, or 99%.

In some embodiments, the drug-to-antibody ratio (DAR) in the antibody-drug conjugates in the population is selected from integers and decimals between 1 and 10.

In some embodiments, the drug-to-antibody ratio (DAR) of the population is selected from 1.5-2.5, 3.5-4.5, 5.5-6.5, and 7.5-8.5.

In some embodiments, the drug-to-antibody ratio (DAR) of the population is selected from about 2.0, 4.0, 6.0, and 8.0.

In some embodiments, the drug-to-antibody ratio (DAR) in the antibody-drug conjugates in the population is selected from 2, 2.5, 3, 3.5, 4, 4.5, 5, 5.5, 6, 6.5, 7, 7.2, 7.4, 7.5, 7.6, 7.7, 7.8, 7.9, 8.0, 8.1, 8.2, 8.3, 8.4, 8.5, 8.7, 8.9, and 9.

In some embodiments, the population comprises ADCs having a DAR distribution from 1 to 8, such as 1.5, 2, 4, 6, and 8 (i.e., drug-loaded species of 1.5, 2, 4, 6, and 8). Notably, degradation products may be produced, such that the population may also comprise DARs of 1, 3, 5, and 7. In addition, the population may also have a DAR greater than 8. The antibody-drug conjugate is produced by the reduction of an interchain disulfide followed by conjugation. In some embodiments, the antibody-drug conjugate comprises the following two: an antibody-drug conjugate with a DAR of 4 or less (i.e., drug-loaded species of 4 or less) and an antibody-drug conjugate with a DAR of 6 or more (i.e., drug-loaded species of 6 or more).

In a tenth aspect of the present disclosure, the present disclosure provides a pharmaceutical composition comprising the antibody-drug conjugate, the stereoisomer thereof, the prodrug thereof, the pharmaceutically acceptable salt thereof, or the pharmaceutically acceptable solvate thereof according to the first aspect or the seventh aspect, the anti-NaPi2b antibody or the antigen-binding fragment thereof according to the second aspect, the nucleic acid according to the third aspect, the vector according to the fourth aspect, the host cell according to the fifth aspect, or the population of antibody-drug conjugates according to the ninth aspect described above, and optionally one or more pharmaceutical excipients.

In certain embodiments, the pharmaceutical composition comprises a therapeutically effective amount of the antibody-drug conjugate, the stereoisomer thereof, the prodrug thereof, the pharmaceutically acceptable salt thereof, or the pharmaceutically acceptable solvate thereof according to the first aspect or the seventh aspect, the anti-NaPi2b antibody or the antigen-binding fragment thereof according to the second aspect, the nucleic acid according to the third aspect, the vector according to the fourth aspect, the host cell according to the fifth aspect, or the population of antibody-drug conjugates according to the ninth aspect described above.

In certain embodiments, the pharmaceutical composition comprises the antibody-drug conjugate, the stereoisomer thereof, the prodrug thereof, the pharmaceutically acceptable salt thereof, or the pharmaceutically acceptable solvate thereof according to the first aspect or the seventh aspect described above and a pharmaceutically acceptable carrier and/or excipient.

In certain embodiments, the pharmaceutical composition of the present disclosure comprises the population of antibody-drug conjugates according to the ninth aspect and a pharmaceutically acceptable carrier and/or excipient. In certain preferred embodiments, the pharmaceutical composition of the present disclosure comprises the population of antibody-drug conjugates described above, or comprises the population of antibody-drug conjugates and a buffer. In certain further preferred embodiments, the pharmaceutical composition of the present disclosure further comprises an excipient and/or a surfactant.

In certain embodiments, the pharmaceutical composition of the present disclosure comprises the anti-NaPi2b antibody or the antigen-binding fragment thereof of the present disclosure and a pharmaceutically acceptable carrier and/or excipient. In certain preferred embodiments, the pharmaceutical composition of the present disclosure comprises the anti-NaPi2b antibody or the antigen-binding fragment thereof and a buffer. In certain further preferred embodiments, the pharmaceutical composition of the present disclosure further comprises an excipient and/or a surfactant.

In some embodiments, the buffer is a histidine buffer. In certain preferred embodiments, the buffer is a 20 mM histidine buffer at pH 6.0.

In certain embodiments, the pharmaceutical composition of the present disclosure comprises the host cell of the present disclosure and a pharmaceutically acceptable carrier and/or excipient, wherein the host cell comprises the isolated nucleic acid molecule or the vector as previously mentioned.

In certain embodiments, the pharmaceutical composition of the present disclosure comprises the multispecific antibody of the present disclosure and a pharmaceutically acceptable carrier and/or excipient.

In some embodiments, the drug-to-antibody ratio (DAR) in the pharmaceutical composition is selected from integers and decimals between 1 and 10.

In some embodiments, the drug-to-antibody ratio (DAR) in the pharmaceutical composition is selected from 1.5-2.5, 3.5-4.5, 5.5-6.5, and 7.5-8.5.

In some embodiments, the drug-to-antibody ratio (DAR) in the pharmaceutical composition is selected from about 2.0, 4.0, 6.0, and 8.0.

In some embodiments, the drug-to-antibody ratio (DAR) in the antibody-drug conjugate in the pharmaceutical composition is selected from 2, 2.5, 3, 3.5, 4, 4.5, 5, 5.5, 6, 6.5, 7, 7.2, 7.4, 7.5, 7.6, 7.7, 7.8, 7.9, 8.0, 8.1, 8.2, 8.3, 8.4, 8.5, 8.7, 8.9, and 9.

In an eleventh aspect, provided is use of the antibody or the antigen-binding fragment thereof of the present disclosure in the manufacture of a kit for detecting the presence or level of NaPi2b in a sample. In another aspect, the present disclosure provides a diagnostic or therapeutic kit comprising one or more of the following substances: the antibody or the antigen-binding fragment thereof, the nucleic acid, the vector, the host cell, the multispecific antibody, the antibody-drug conjugate, the population of antibody-drug conjugates, or the pharmaceutical composition of the present disclosure. Optionally, the diagnostic or therapeutic kit further comprises an instruction for use.

In a twelfth aspect of the present disclosure, the present disclosure provides use of the aforementioned antibody-drug conjugate composition or the aforementioned pharmaceutical composition in the manufacture of a medicament for treating and/or preventing a disease associated with abnormal cell activity (e.g., a cancer disease). The antibody-drug conjugate composition or the aforementioned pharmaceutical composition may be in a therapeutically effective amount.

In some embodiments, provided is use of the anti-NaPi2b antibody or the antigen-binding fragment thereof, the nucleic acid, the vector, the host cell, or the multispecific antibody of the present disclosure in the manufacture of a medicament for modulating (inhibiting or blocking) activity of NaPi2b.

In some embodiments, provided is use of the anti-NaPi2b antibody or the antigen-binding fragment thereof, the nucleic acid, the vector, the host cell, or the multispecific antibody of the present disclosure in the manufacture of a medicament for treating or preventing a disease associated with activity of NaPi2b.

In some embodiments, provided is use of the anti-NaPi2b antibody or the antigen-binding fragment thereof, the nucleic acid, the vector, the host cell, the antibody-drug conjugate, or the multispecific antibody of the present disclosure in the manufacture of a medicament for treating or preventing a tumor associated with activity of NaPi2b.

In some embodiments, provided is use of the antibody-drug conjugate, the stereoisomer thereof, the prodrug thereof, the pharmaceutically acceptable salt thereof, or the pharmaceutically acceptable solvate thereof according to the first aspect or the seventh aspect, the anti-NaPi2b antibody or the antigen-binding fragment thereof according to the second aspect, the nucleic acid according to the third aspect, the vector according to the fourth aspect, the host cell according to the fifth aspect, the population of antibody-drug conjugates according to the ninth aspect, or the pharmaceutical composition according to the tenth aspect described above in the present disclosure in the manufacture of a medicament for treating or preventing a tumor.

In some embodiments, provided is use of the antibody-drug conjugate, the stereoisomer thereof, the prodrug thereof, the pharmaceutically acceptable salt thereof, or the pharmaceutically acceptable solvate thereof according to the first aspect or the seventh aspect, the population of antibody-drug conjugates according to the ninth aspect, or the pharmaceutical composition according to the tenth aspect described above in the present disclosure in the manufacture of a medicament for treating or preventing a tumor associated with a target of Tb.

In some embodiments, provided is use of the antibody-drug conjugate, the stereoisomer thereof, the prodrug thereof, the pharmaceutically acceptable salt thereof, or the pharmaceutically acceptable solvate thereof according to the first aspect or the seventh aspect, the population of antibody-drug conjugates according to the ninth aspect, or the pharmaceutical composition according to the tenth aspect described above in the present disclosure in the manufacture of a medicament for treating or preventing a tumor associated with NaPi2b.

In some preferred embodiments, provided is use of the antibody-drug conjugate, the stereoisomer thereof, the prodrug thereof, the pharmaceutically acceptable salt thereof, or the pharmaceutically acceptable solvate thereof according to the first aspect or the seventh aspect, the anti-NaPi2b antibody or the antigen-binding fragment thereof according to the second aspect, the nucleic acid according to the third aspect, the vector according to the fourth aspect, the host cell according to the fifth aspect, the population of antibody-drug conjugates according to the ninth aspect, or the pharmaceutical composition according to the tenth aspect described above in the present disclosure in the manufacture of a medicament for treating or preventing a tumor associated with NaPi2b.

In a thirteenth aspect of the present disclosure, the present disclosure provides a method for using the antibody-drug conjugate, the stereoisomer thereof, the prodrug thereof, the pharmaceutically acceptable salt thereof, or the pharmaceutically acceptable solvate thereof according to the first aspect or the seventh aspect, the anti-NaPi2b antibody or the antigen-binding fragment thereof according to the second aspect, the nucleic acid according to the third aspect, the vector according to the fourth aspect, the host cell according to the fifth aspect, the population of antibody-drug conjugates according to the ninth aspect, or the pharmaceutical composition according to the tenth aspect described above to treat and/or prevent a disease associated with abnormal cell activity (e.g., a tumor).

In the twelfth and thirteenth aspects described above, the tumor is selected from esophageal cancer (e.g., esophageal adenocarcinoma and esophageal squamous cell carcinoma), brain tumor, lung cancer (e.g., small cell lung cancer, non-small cell lung cancer, or lung adenocarcinoma), squamous cell carcinoma, bladder cancer, gastric cancer, ovarian cancer, peritoneal cancer, pancreatic cancer, breast cancer, head and neck cancer, cervical cancer, endometrial cancer, colon cancer (e.g., human colon adenocarcinoma), rectal cancer, colorectal cancer, liver cancer, kidney cancer, urothelial cancer, epidermal cancer, non-Hodgkin's lymphoma, central nervous system tumor (e.g., neuroglioma, glioblastoma multiforme, glioma, or sarcoma), prostate cancer, and thyroid cancer.

In some embodiments, the tumor is a tumor associated with NaPi2b.

In some embodiments, the tumor is a tumor associated with NaPi2b.

In the present disclosure, unless otherwise indicated, scientific and technical terms used herein have the meanings commonly understood by those skilled in the art. In addition, the laboratory operations of cell culture, molecular genetics, nucleic acid chemistry, and immunology used herein are the routine procedures widely used in the corresponding fields. Also, in order to better understand the present disclosure, the definitions and interpretations of the related terms are provided as follows.

As used herein, examples of the term "pharmaceutically acceptable salt" are organic acid addition salts formed from organic acids that produce pharmaceutically acceptable anions, including but not limited to formate, acetate, propionate, benzoate, maleate, fumarate, succinate, tartrate, citrate, ascorbate, α-ketoglutarate, α-glycerophosphate, alkylsulfonate, or arylsulfonate; preferably, the alkylsulfonate is methylsulfonate or ethylsulfonate; the arylsulfonate is benzene sulfonate or p-toluenesulfonate. Suitable inorganic salts may also be formed, including but not limited to hydrochloride, hydrobromide, hydroiodide, nitrate, bicarbonate and carbonate, sulfate or phosphate, etc.

As used herein, the term "pharmaceutically acceptable carrier and/or excipient" refers to a carrier and/or excipient that is pharmacologically and/or physiologically compatible with the subject and the active ingredient. Such carriers and/or excipients are well known in the art (see, e.g., Remington 's Pharmaceutical Sciences, edited by Gennaro AR, 19th Ed., Pennsylvania: Mack Publishing Company, 1995), including but not limited to pH regulators, surfactants, adjuvants, ionic strength enhancers, diluents, osmotic pressure-maintaining reagents, absorption-retarding reagents, and preservatives.

Pharmaceutically acceptable salts may be obtained using standard procedures well known in the art, for example, by reacting a sufficient amount of an alkaline compound with a suitable acid providing a pharmaceutically acceptable anion.

In the present disclosure, the pharmaceutical excipients refer to excipients and additives used in the manufacture of a pharmaceutical product and in the formulation of a pharmaceutical formula, and refer to substances, other than the active ingredient, which have been rationally evaluated for safety and are contained in a pharmaceutical formulation. In addition to shaping, serving as carriers, and enhancing stability, pharmaceutical excipients also have such important functions as solubilization, aiding dissolution, sustaining or controlling release, etc. They are important ingredients that possibly affect the quality, safety, and effectiveness of pharmaceutical products. According to the origin, pharmaceutical excipients may be classified into natural products, semi-synthetic products, and total synthetic products. According to the effect and use, pharmaceutical excipients may be classified into solvents, propellants, solubilizers, cosolvents, emulsifiers, colorants, binders, disintegrants, fillers, lubricants, wetting agents, osmotic pressure regulators, stabilizers, glidants, flavoring agents, preservatives, suspending agents, coating materials, fragrances, anti-adhesive agents, antioxidants, chelating agents, permeation promoters, pH regulators, buffers, plasticizers, surfactants, foaming agents, defoaming agents, thickeners, inclusion agents, humectants, absorbents, diluents, flocculants, deflocculants, filter aids, release retardants, etc; according to the administration route, pharmaceutical excipients may be classified into pharmaceutical excipients for oral administration, injection, mucosal administration, transdermal or local administration, nasal or oral inhalation administration, ocular administration, etc. The same pharmaceutical excipient may be used in pharmaceutical formulations for different administration routes, and may have different effects and uses.

The pharmaceutical composition may be prepared into various suitable dosage forms according to the administration route, for example, tablets, capsules, granules, oral solutions, oral suspensions, oral emulsions, powders, tinctures, syrups, injections, suppositories, ointments, creams, pastes, ophthalmic formulations, pills, implants, aerosols, powder aerosols, sprays, etc. Among them, the pharmaceutical composition or suitable dosage form may contain 0.01 mg to 1000 mg of the compound of the present disclosure or the pharmaceutically acceptable salt thereof or conjugate thereof, suitably 0.1 mg to 800 mg, preferably 0.5 mg to 500 mg, preferably 0.5 mg to 350 mg, and particularly preferably 1 mg to 250 mg.

The pharmaceutical composition may be administered in the form of an injection, including an injectable liquid, a sterile powder for injection, and a concentrated solution for injection. The carriers and solvents that may be used include water, Ringer's solution, and isotonic sodium chloride solution. In addition, sterilized non-volatile oils may also be used as solvents or suspending media, such as monoglycerides or diglycerides.

As used herein, the term "treat", "treating", or "treatment" usually refers to acquiring needed pharmacological effects and/or physiological effects. In terms of fully or partially preventing a disease or a symptom thereof, the effect may be preventive; and/or in terms of partially or fully stabilizing or curing the disease and/or a side effect caused by the disease, the effect may be therapeutic. As used herein, "treat", "treating", or "treatment" encompasses any treatment to a disease in a patient, including (a) preventing the occurrence of a disease or a symptom in a patient susceptible to the disease or the symptom that has not yet been diagnosed; (b) inhibiting a symptom of a disease, i.e., arresting its progression; or (c) relieving a symptom of a disease, i.e., causing regression of the disease or the symptom.

In the present disclosure, the term "individual" includes a human or non-human animal. Exemplary human individuals include human individuals with a disease (e.g., the disease described herein), which are referred to as patients, or normal individuals. In the present disclosure, the term "non-human animal" includes all vertebrates, such as non-mammals (e.g., birds, amphibians, and reptiles) and mammals, such as non-human primates, livestock and/or domesticated animals (e.g., sheep, dogs, cats, cows, pigs, etc.).

In the present disclosure, the term "effective dose" refers to the amount of a compound that can relieve one or more symptoms of a condition to be treated to some extent after administration.

In the present disclosure, the term "bioactive substance", "bioactive molecule", or "drug molecule" refers to a substance that inhibits or prevents cell functions and/or causes cell death or destruction, and in some embodiments of the present disclosure, the bioactive substance, the bioactive molecule, or the drug molecule in the conjugate is a molecule having anti-tumor bioactivity, for example: radioisotopes, such as At²¹¹, I¹³¹, I¹²⁵, Y⁹⁰, Re¹⁸⁶, Re¹⁸⁸, Sm¹⁵³, Bi²¹², P³², Pb²¹², and radioisotopes of Lu; metal complexes, such as metal platinum complexes, metal gold complexes, oxaliplatin, etc.; glycopeptide antibiotics, such as bleomycin and pingyangmycin; DNA topoisomerase inhibitors, such as topoisomerase I inhibitors, camptothecin, hydroxycamptothecin, 9-aminocamptothecin, SN-38, irinotecan, topotecan, belotecan, rubitecan, topoisomerase II inhibitors, actinomycin D, adriamycin, doxorubicin, duocarmycin, daunorubicin, mitoxantrone, podophyllotoxin, etoposide, etc.; drugs that interfere with DNA synthesis, such as methotrexate, 5-fluorouracil, cytarabine, gemcitabine, mercaptopurine, pentostatin, fludarabine, cladribine, nelarabine, etc.; drugs that act on structural proteins, such as tubulin inhibitors, vinca alkaloids, vincristine, vinblastine, paclitaxel, docetaxel, cabazitaxel, etc.; tumor signaling pathway inhibitors, such as serine/threonine kinase inhibitors, tyrosine kinase inhibitors, aspartate kinase inhibitors, or histidine kinase inhibitors, etc.; and also includes proteasome inhibitors, histone deacetylase inhibitors, tumor angiogenesis inhibitors, cyclin inhibitors, maytansine derivatives, calicheamicin derivatives, auristatin derivatives, pyrrolobenzodiazepine (PBD) derivatives, melphalan, mitomycin C, chlorambucil, or other active substances that inhibit tumor cell growth and promote tumor cell apoptosis and necrosis; enzymes and fragments thereof, such as nucleolytic enzymes; antibiotics; toxins, such as small molecule toxins or enzymatically active toxins of bacterial, fungal, plant, or animal origin, including fragments and/or variants thereof; growth inhibitors; and drug modules. The term "toxin" refers to a substance that can have a deleterious effect on the growth or proliferation of a cell.

In the present disclosure, the term "linker" refers to a fragment that attaches a bioactive molecule (drug molecule) to a targeting moiety.

In the present disclosure, the term "targeting moiety" refers to a moiety in a conjugate that is capable of specifically binding to a target (or a moiety of a target) on the surface of a cell. The conjugate can be delivered to a specific cell population by the interaction of the targeting moiety with the target.

In the present disclosure, the antibody or the antigen-binding fragment thereof includes a derivatized antibody or an antigen-binding fragment thereof, such as an antibody or an antigen-binding fragment thereof having a sulfhydryl group, wherein the derivatization provides the antibody with a group or ability to react with a drug-linker conjugate. The sulfhydryl group -SH may be derivatized by opening a disulfide bond (e.g., by reduction using the reducing agent TCEP).

The terms "cancer" and "tumor" are used herein with the same meaning.

The term "gene" as used herein includes not only a DNA but also an mRNA thereof, a cDNA thereof, and a cRNA thereof.

The term "polynucleotide" as used herein is used with the same meaning as nucleic acid and also includes DNA, RNA, probes, oligonucleotides, and primers.

The terms "polypeptide" and "protein" are used herein without distinction.

The term "cell" as used herein also includes cells within an animal individual and cells in culture.

The NaPi2b involved in the anti-NaPi2b antibody in the present disclosure may be NaPi2b as is conventional in the art, and of course, also denotes variants of NaPi2b.

For the term "NaPi2b", NaPi2b is encoded by the SLC34A2 gene, consists of 690 amino acids, and is structurally 8-transmembrane with 4 extracellular loops. Sodium-dependent phosphate transport protein 2B (NaPi2b) maintains Pi homeostasis *in vivo* by selectively transporting bivalent Pi (HPO₄²⁻). NaPi2b of human shows 85.3% homology to that of rat and 95.8% homology to that of cynomolgus monkey. NaPi2b has a high amino acid sequence similarity with its family members NaPi2a/2c (51.29% homology to NaPi2a and 44.64% homology to NaPi2c), especially in the transmembrane region where the homology is > 80%.

KD refers to the dissociation constant obtained from the ratio of Kd (the dissociation rate of the specific binding molecule-target protein interaction) to Ka (the association rate of the specific binding molecule-target protein interaction) (or Kd/Ka, expressed in molar concentration (M)). The KD values may be determined using methods well established in the art. A preferred method for determining the KD of a binding molecule is by using surface plasmon resonance, such as with biosensor systems like the Biacore TM (GE Healthcare Life Sciences) system.

As used herein, the percent homology between two amino acid sequences is equal to the percent identity between the two sequences. The percent sequence identity between two sequences is a function of the number of identical positions shared by the sequences (i.e., % homology = number of identical positions/total number of positions × 100), where the number of gaps and the length of each gap are taken into account and need to be introduced for optimal alignment of the two sequences. Sequence comparison and determination of percent identity between sequences can be performed by methods generally known in the art, and such sequence comparison and determination of percent identity can be achieved using mathematical algorithms. For example, the algorithm of Meyers and Miller, 1988, Comput. Appl. Biosci., 4: 11-17 (which has been integrated into the ALIGN program (version 2.0)) can be used to determine the percent identity between amino acid sequences and/or nucleotide sequences. In addition, the GAP program (using its default parameters) in the GCG software package, available online from Accelrys, can be used to determine percent identity between amino acid sequences or nucleotide sequences. In one embodiment, the two sequences are of equal length.

The term "epitope" refers to a portion of an antigenic polypeptide or protein that has antigenic or immunogenic activity in the body of an animal, preferably a mammal. Epitopes of the antibody or the antigen-binding fragment thereof of the present disclosure can be determined by techniques in the prior art, such as synthetic peptide methods, immunoinformatics prediction, determination of polypeptide activity, epitope peptide scanning methods, phage display technology, X-ray diffraction and nuclear magnetic resonance analysis, and antibody homology modeling protein docking prediction methods. The phrase "antibodies that bind to the same epitope" as used herein means different antibodies that bind to a common epitope. If a second antibody binds to a portion of the peptide or a portion of the tertiary structure that a first antibody binds to, it can be determined that the first antibody and the second antibody bind to the same epitope.

In the present disclosure, the term "antibody" is interpreted in its broadest sense and includes intact monoclonal antibodies, polyclonal antibodies, and multispecific antibodies (e.g., bispecific antibodies) formed from at least two intact antibodies, so long as they possess the desired bioactivity. In the present disclosure, "antibody" and "immunoglobulin" may be used interchangeably. The "antibody molecule" or "antibody" as described herein refers to an immunoglobulin molecule and an immunologically active portion of an immunoglobulin molecule, i.e., a molecule comprising an antigen-binding site that immunospecifically binds to an antigen. Thus, the term antibody broadly encompasses not only an intact antibody molecule, but also a fragment of the antibody and a variant (including derivative) of the antibody and the antibody fragment. When an "antibody molecule" or "antibody" is used in the same context as an antigen-binding fragment, the "antibody molecule" or "antibody" refers to an intact antibody molecule or a full-length antibody. The term antibody molecule as used in this specification includes, for example, but is not limited to, a single-chain Fv (scFv), a Fab fragment, a Fab' fragment, a F(ab')2, a disulfide-linked Fv (sdFv), an Fv, and an intact antibody or a full-length antibody. The term "single-chain Fv" or "scFv" refers to a polypeptide comprising a VL domain of an antibody attached to a VH domain of the antibody. For example, an antibody that immunospecifically binds to NaPi2b can cross-react with other antigens. Preferably, an antibody that immunospecifically binds to NaPi2b does not cross-react with other antigens. Immunospecific binding may be identified, such as by immunoassay or other methods known to those skilled in the art. An "intact" antibody or a "full-length" antibody refers to a protein comprising two heavy chains (H) and two light chains (L) which are interconnected by disulfide bonds, wherein the protein comprises: (1) for the heavy chain, a variable region (abbreviated herein as "VH") and a heavy chain constant region comprising three domains CH1, CH2, and CH3; and (2) for the light chain, a light chain variable region (abbreviated herein as "VL") and a light chain constant region comprising one domain CL. The antibodies of the present disclosure include, but are not limited to, monoclonal, multispecific, human or chimeric antibodies, single-chain antibodies, Fab fragments, F(ab') fragments, anti-idiotypic (anti-Id) antibodies (including, e.g., anti-Id antibodies to antibodies of the present disclosure), and epitope-binding fragments of any of the antibodies described above. The immunoglobulin molecules of the present disclosure may be of any type (e.g., IgG, IgE, IgM, IgD, IgA, and IgY), class (e.g., IgG1, IgG2, IgG3, IgG4, IgA1, and IgA2), or subclass of immunoglobulin. Preferably, the antibody of the present disclosure comprises or consists of a VH domain, a VH CDR (often referred to herein as HCDR), a VL domain, or a VL CDR (often referred to herein as LCDR) having any one of the amino acid sequences set forth in the sequence and detailed information tables, or a fragment or variant thereof.

In the present disclosure, the term "monoclonal antibody" refers to an antibody from a population of substantially homogeneous antibodies, i.e., the individual antibodies constituting the population are identical except for a small number of natural mutations that may be present. Monoclonal antibodies have high specificity for one determinant (epitope) of an antigen, while polyclonal antibodies in contrast thereto comprise different antibodies against different determinants (epitopes). In addition to specificity, another advantage of monoclonal antibodies is that they can be synthesized without contamination by other antibodies. The modifier "monoclonal" used herein indicates that the antibody is characterized as being from a substantially homogeneous population of antibodies, and should not be construed as requiring a particular preparation method.

In some embodiments of the present disclosure, monoclonal antibodies further particularly include chimeric antibodies, i.e., a portion of the heavy chain and/or light chain is identical or homologous to a type, a class, or a subclass of antibodies, and the remainder is identical or homologous to another type, another class, or another subclass of antibodies, so long as they possess the desired bioactivity (see, e.g., US 4,816,567; and Morrison et al., 1984, PNAS, 81: 6851-6855). Chimeric antibodies that may be used in the present disclosure include primatized antibodies comprising variable region antigen-binding sequences from non-human primates (e.g., ancient monkeys, chimpanzees, etc.) and human constant region sequences.

The term "antigen-binding fragment" refers to a portion of an antibody, preferably an antigen-binding region or a variable region. Examples of the antibody fragment include a Fab, a Fab', a F(ab')₂, an Fd, an Fv, a dAb, a complementarity determining region fragment, a diabody, a linear antibody, and a single-chain antibody molecule. The term "antigen-binding fragment" as used herein refers to a partial fragment of an antibody having antigen-binding activity, wherein the fragment has full or partial function of the antibody, including but not limited to, a single-chain Fv (scFv), a Fab, a Fab', a F(ab')2, a disulfide-linked Fv (sdFv), an Fv, a di-scFv, etc. The term also includes a Fab', which is a monovalent fragment of the variable region of the antibody obtained by treating the F(ab')2 under reducing conditions. However, the term is not limited to these molecules, as long as the fragment has a binding affinity for an antigen. In addition, these functional fragments include not only fragments obtained by treating the full-length molecule of the antibody protein with an appropriate enzyme, but also proteins produced in an appropriate host cell using a genetically modified antibody gene.

The term "Fab'" as used herein refers to a monovalent fragment of the variable region of the antibody obtained by treating the F(ab')2 under the reducing conditions described above. However, the Fab' of the present disclosure also includes the Fab' produced using the genetically modified antibody gene.

The term "scFv" as used herein refers to a single polypeptide chain comprising VL and VH domains, wherein the VL and VH are attached through a linker or directly attached (see, e.g., Bird et al., Science 242: 423-426 (1988); Huston et al., Proc. Natl. Acad. Sci. USA 85: 5879-5883 (1988); and Pluckthun, The Pharmacology of Monoclonal Antibodies, Vol. 113, edited by Roseburg and Moore, Springer-Verlag, New York, pp. 269-315 (1994)). Such scFv molecules may have a general structure: NH2-VL-linker-VH-COOH or NH2-VH-linker-VL-COOH. An appropriate linker in the prior art consists of GGGGS amino acid sequence repeats or a variant thereof. For example, a linker having the amino acid sequence (GGGGS)4 may be used, but variants thereof may also be used (Holliger et al., (1993), Proc. Natl. Acad. Sci. USA, 90: 6444-6448). Other linkers that may be used in the present disclosure are described by Alfthan et al., (1995), Protein Eng., 8: 725-731; Choi et al., (2001), Eur. J. Immunol., 31: 94-106; Hu et al., (1996), Cancer Res., 56: 3055-3061; Kipriyanov et al., (1999), J. Mol. Biol., 293: 41-56; and Roovers et al., (2001), Cancer Immunol*.* In some cases, a disulfide bond may also be present between the VH and VL of the scFv. As used herein, the term "di-scFv" refers to an antibody fragment formed by connecting two scFvs.

As used herein, the antibody or the antigen-binding fragment thereof, even if comprising a variant, an amino acid substitution, deletion, or addition, still retains the activity of binding to the antigen.

The term "bispecific antibody", also known as "bifunctional antibody conjugate", refers to a conjugate formed from a first antibody (fragment) and a second antibody (fragment) via a coupling arm, and the conjugate retains the activity of each respective antibody and thus has bifunctionality and bispecificity.

The term "multispecific antibody" includes, for example, a trispecific antibody and a tetraspecific antibody, the former being an antibody with three different kinds of antigen-binding specificity, and the latter being an antibody with four different kinds of antigen-binding specificity.

The term "intact antibody" or "full-length antibody" refers to an antibody comprising antigen-binding variable regions, a light chain constant region (CL), and heavy chain constant regions (CH1, CH2, and CH3). The constant region may be a native sequence (e.g., human native constant region sequence) or a variant of an amino acid sequence thereof. The intact antibody is preferably an intact antibody with one or more effector functions.

The term "probody" is a modified antibody, comprising an antibody or an antibody fragment, capable of specifically binding to its target and capable of being coupled to a masking group, wherein the masking group means that the cleavage constant of the binding capacity of the antibody or the antibody fragment to its target is at least 100 times or 1000 times or 10000 times greater than that of the binding capacity of the antibody or the antibody fragment without the coupled masking group to its target.

In the present disclosure, "humanized" forms of non-human (e.g., murine) antibodies refer to chimeric antibodies that comprise minimal non-human immunoglobulin sequences. Most humanized antibodies are human recipient immunoglobulins with the hypervariable region residues replaced by non-human (e.g., mouse, rat, rabbit, or non-human primate) hypervariable region residues having the desired specificity, affinity, and function (donor antibody). In some embodiments, framework region (FR) residues of the human immunoglobulin are also replaced by non-human residues. Furthermore, humanized antibodies may further comprise residues that are not present in the recipient antibody or the donor antibody. These modifications are made to further optimize the properties of the antibody. A humanized antibody generally comprises at least one, usually two, variable regions, in which all or nearly all of the hypervariable loops correspond to those of a non-human immunoglobulin, while the FRs are entirely or nearly entirely human immunoglobulin sequences. The humanized antibody may further comprise at least one portion of an immunoglobulin constant region (Fc), typically a human immunoglobulin Fc. For details see, e.g., Jones et al., 1986, Nature, 321: 522-525; Riechmann et al., 1988, Nature, 332: 323-329; and Presta, 1992, Curr Op Struct Bwl 2: 593-596.

Intact antibodies may be divided into different "classes" according to the amino acid sequences of the heavy chain constant regions. The main five classes are IgA, IgD, IgE, IgG, and IgM, several of which may also be divided into different "subclasses" (isotypes), such as IgG1, IgG2, IgG3, IgG4, IgA1, and IgA2. Different classes of heavy chain constant regions of antibodies are referred to as α, β, ε, γ, and µ, respectively. Different classes of subunit structures and three-dimensional configurations of immunoglobulins are well known in the art.

Herein, the CDRs contained in the antibody or the antigen-binding fragment thereof of the present disclosure can be determined according to various numbering systems known in the art. In certain embodiments, the CDRs contained in the antibody or the antigen-binding fragment thereof of the present disclosure are preferably determined by the Kabat, Chothia, or AbM or IMGT numbering system.

As used herein, the term "framework residue region" or "FR residue" refers to those amino acid residues in the antibody variable region other than the CDR residues defined above.

As used herein, the term "germline antibody gene" is a coding gene of an immunoglobulin expressed in non-lymphoid cells, which has not undergone a maturation process that results in genetic rearrangement and maturation for expression of a specific immunoglobulin. One advantage provided by various embodiments of the present disclosure derives from the recognition that germline antibody genes encode amino acid sequences that retain more of the characteristic important amino acid sequence structures of an individual of an animal species than amino acid sequences encoded by mature antibody genes. Thus, the amino acid sequences encoded by the germline antibody genes are less likely to be recognized as foreign substances by the species when therapeutically applied to the species.

As for the amino acid substitution, a conservative amino acid substitution is preferred in this specification. The conservative amino acid substitution refers to a substitution that occurs within a set of amino acids related to an amino acid side chain. Preferred amino acid sets are as follows: an acidic set (aspartic acid and glutamic acid); a basic set (lysine, arginine, and histidine); a nonpolar set (alanine, valine, leucine, isoleucine, proline, phenylalanine, methionine, and tryptophan); and an uncharged polar family (glycine, asparagine, glutamine, cysteine, serine, threonine, and tyrosine). More preferred amino acid sets are as follows: aliphatic hydroxy (serine and threonine); an amide-containing set (asparagine and glutamine); an aliphatic set (alanine, valine, leucine, and isoleucine); and an aromatic set (phenylalanine, tryptophan, and tyrosine). Such amino acid substitutions are preferably made within a set that does not compromise properties of the substance having the original amino acid sequence.

In addition, it is known that the lysine residue at the carboxyl terminus of the heavy chain of the antibody produced in a cultured mammalian cell is deleted (Journal of Chromatography A, 705: 129-134 (1995)), and it is also known that 2 amino acid residues (glycine and lysine) at the carboxyl terminus of the heavy chain of the antibody produced in a cultured mammalian cell are deleted, and a proline residue newly located at the carboxyl terminus is amidated (Analytical Biochemistry, 360: 75-83 (2007)). However, such deletions and modifications of the heavy chain sequence do not affect the antigen-binding affinity and effector functions of the antibody (activation of complement, antibody-dependent cellular cytotoxicity, etc.).

The writing of the twenty conventional amino acids referred to herein follows conventional usage. See, e.g., Immunology-A Synthesis (2nd Edition, E. S. Golub and D. R. Gren, Eds., Sinauer Associates, Sunderland, Mass. (1991)), which is incorporated herein by reference. Herein, the terms "polypeptide" and "protein" have the same meaning and are used interchangeably. Besides, in the present disclosure, amino acids are generally represented by single-letter and three-letter abbreviations well known in the art. For example, alanine can be represented by A or Ala; arginine can be represented by R or Arg; glycine can be represented by G or Gly; glutamine can be represented by Q or Gln.

As used herein, the term "prevent", "preventing", or "prevention" refers to a method performed to arrest or delay the occurrence of a disease or condition or symptom (e.g., tumor and infectious disease) in a subject. As used herein, the term "treat", "treating", or "treatment" refers to a method performed to achieve beneficial or desired clinical outcomes. For purposes of the present disclosure, the beneficial or desired clinical outcomes include, but are not limited to, alleviation of symptoms, diminishment of extent of disease, stabilized (i.e., not worsening) state of disease, delay or slowing of disease progression, amelioration or palliation of state of disease, and remission (whether partial or total) of symptoms, whether detectable or undetectable. In addition, "treat", "treating", or "treatment" may also refer to prolonging survival as compared with the expected survival (if no treatment is received).

As used herein, the term "subject" refers to a mammal, for example, a primate mammal, such as a non-human primate mammal or a human. In certain embodiments, the subject (e.g., human) is suffering from a tumor and an infectious disease, or at risk of suffering from the disease described above.

As used herein, the term "effective amount" refers to an amount sufficient to achieve, or at least partially achieve, a desired effect. For example, a prophylactically effective amount against a disease (e.g., tumor and infectious disease) refers to an amount sufficient to prevent, arrest, or delay the occurrence of the disease (e.g., tumor and infectious disease); a therapeutically effective amount against a disease refers to an amount sufficient to cure or at least partially arrest the disease and complications thereof in patients suffering from the disease. It is undoubtedly within the ability of those skilled in the art to determine such an effective amount. For example, the amount effective for the therapeutic purpose will depend on the severity of the disease to be treated, the overall state of the patient's own immune system, the general condition of the patient such as age, body weight, and gender, the route of administration, and other treatments given concurrently, etc.

As used herein, the term "effector function" refers to those bioactivities attributable to an Fc region (either a native sequence Fc region or an amino acid sequence variant Fc region) of an antibody, which vary with the antibody isotype.

The term "pharmaceutically acceptable" means that a molecule, a molecular fragment, or a composition, when properly administered to an animal or human, does not produce detrimental, allergic, or other adverse reactions. Specific examples of some substances that can serve as pharmaceutically acceptable carriers or components thereof include sugars (e.g., lactose), starch, cellulose and derivatives thereof, vegetable oils, gelatin, polyols (e.g., propylene glycol), alginic acid, etc.

The term "population of antibody-drug conjugates" refers to a mixture composed of a set or group of the antibody-drug conjugates, the stereoisomers thereof, the prodrugs thereof, the pharmaceutically acceptable salts thereof, the tautomers thereof, or the pharmaceutically acceptable solvates thereof of the present disclosure, wherein the q values of the antibody-drug conjugates may be the same or different. In addition, it may also be referred to as an "antibody-drug conjugate mixture".

The *in vivo* therapeutic effect of the antibody and the antibody-drug conjugate on cancer can be determined using experimental animals, such as administering the antibody to nude mice implanted with a tumor cell line expressing NaPi2b, and measuring any changes in cancer cells.

In the present disclosure, although in most cases, amino acid substitutions in antibodies are substitutions with L-amino acids, they are not limited thereto. In some embodiments, an antibody peptide chain may comprise one or more D-amino acids. Peptides comprising D-amino acids are more stable and less susceptible to degradation in the oral cavity, intestine, or plasma than peptides comprising only L-amino acids.

Monoclonal antibodies used in the present disclosure may be produced by many methods. For example, monoclonal antibodies used in the present disclosure may be obtained by the hybridoma method using many species including mouse, hamster, rat, and human cells (see, e.g., Kohler et al., 1975, Nature, 256: 495), or prepared by recombinant DNA techniques (see, e.g., US 4,816,567), or isolated from phage antibody libraries (see, e.g., Clackson et al., 1991, Nature, 352: 624-628; and Marks et al., 1991, Journal of Molecular Biology, 222: 581-597).

Herein, unless otherwise expressly indicated, the descriptive phrases "each ... be independently selected from" and "... be each independently selected from" are used interchangeably throughout the present disclosure and should be understood in a broad sense, and it may mean that specific items expressed by the same or different symbols in different groups do not affect each other, or that specific items expressed by the same or different symbols in the same group do not affect each other.

Herein, the term "direct bond" means that the groups on both sides are directly attached, for example, in the compound of formula II 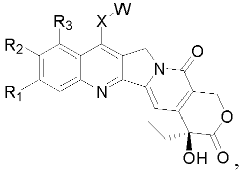 if X is a direct bond, the compound has a structural formula of 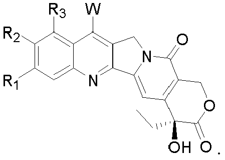 The remaining direct bonds may be understood with reference to the aforementioned content.

Herein, the term "absent" means that the group is absent, for example, in the compound of formula II 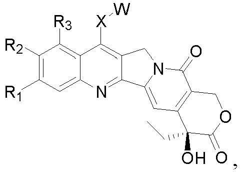 if W is absent, the compound has a structural formula of

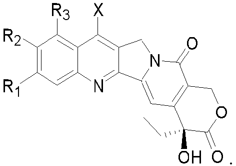

In the compound of formula II 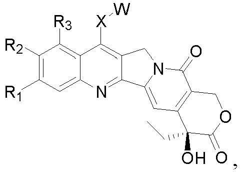 R₁ and R₂, together with the carbon atoms to which they are attached, form 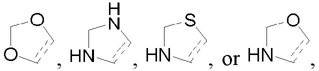 wherein the "dotted line" bond indicates the position at which the heterocyclic ring is fused to the phenyl ring, for example, forming

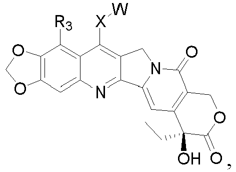

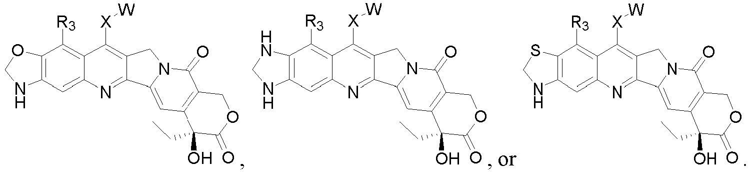

Herein, in the drug-linker conjugate of formula III 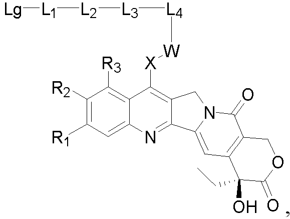 when L₄ is 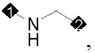 labels 1 and 2 therein indicate the attachment positions of L₄ to the remaining groups; specifically, position 1 is attached to L₃, and position 2 is attached to the drug molecule, i.e., 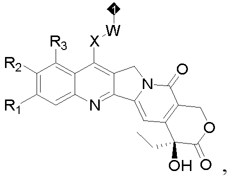 forming 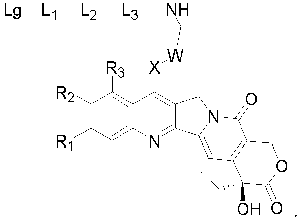 The remaining labels 1 and 2 may be understood with reference to the aforementioned content.

Herein, in the compound of formula II 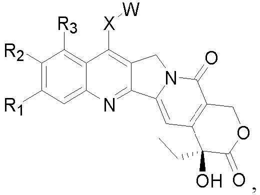 R₃ and X, together with the carbon atoms to which they are attached, form 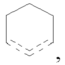 wherein the dotted line indicates the position at which the carbocyclic ring is fused to the phenyl ring and the pyridine ring, forming

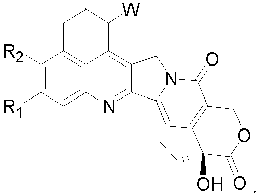

Herein, X is defined, for example, as "X is selected from optionally substituted 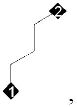 wherein the substituent is selected from 1 or 2 C1-4 alkyl (e.g., methyl)", then X may be, for example, 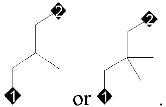 Other similar definitions of X may be understood with reference to the aforementioned content.

Herein, X is defined, for example, as "X is selected from optionally substituted 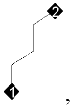 wherein the substituent is selected from 2 C1-4 alkyl (e.g., methyl), together with the carbon atoms to which they are both attached, forming C3-6 cycloalkyl (e.g., cyclopropyl)", then X may be, for example, 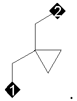 Other similar definitions of X may be understood with reference to the aforementioned content.

In the structure of the amino acid residue represented by AA¹ 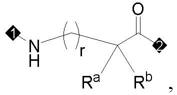 if r is 0, it will be understood by those skilled in the art that the structure of the amino acid residue represented by AA¹ will become

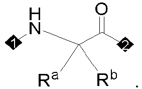

In the structure 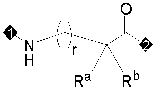 of the amino acid residue represented by AA¹, if R^{a} and R^{b}, together with the carbon atom to which they are both attached, form a 4-10 membered heterocyclic ring, the 4-10 membered heterocyclic ring is optionally substituted with one or more R⁰, wherein the term "the 4-10 membered heterocyclic ring is optionally substituted with one or more R⁰" means that the 4-10 membered heterocyclic ring may be unsubstituted or substituted with one or more R⁰, and in the more R⁰, the definition of each R⁰ may be identical or different. Other similar definitions may be understood with reference to the aforementioned content.

Herein, for example, when L₃ is selected from Lys, Val-Cit, Ala-Ala-Asn, Ala-Ala-Asp, Gly-Gly-Phe-Gly, Val-Lys-Gly, Val-Ala, and Lys-Ala-Asn, the meaning of "the distal amino of the lysine (Lys) being optionally substituted with 1, 2, or 3 substituents selected from tert-butoxycarbonyl, C1-6 alkyl (preferably methyl), and O" is that the distal amino of Lys in each of the options of L₃ is optionally substituted with 1, 2, or 3 substituents selected from tert-butoxycarbonyl, C1-6 alkyl (preferably methyl), and O, wherein the "distal amino of Lys" refers to the naked amino -NH₂ in the lysine residue 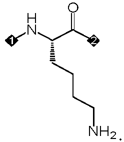 "The distal amino of the lysine (Lys) being optionally substituted with 1, 2, or 3 substituents selected from tert-butoxycarbonyl, C1-6 alkyl (preferably methyl), and O" means that the distal amino of the lysine (Lys) may be unsubstituted, or substituted with 1, 2, or 3 substituents selected from tert-butoxycarbonyl, C1-6 alkyl (preferably methyl), and O; for example, it may be substituted with 1 tert-butoxycarbonyl, i.e., resulting in 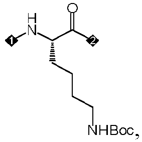 or with 2 methyl, i.e., resulting in 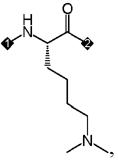 or with 2 methyl and 1 O simultaneously, i.e., resulting in 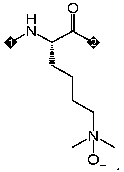 It should be noted that "the distal amino of the lysine (Lys) being substituted with O" means that the distal amino of the lysine (Lys) is substituted with oxo, i.e., resulting in 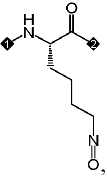 and if the distal amino is further substituted with two methyl, L₃ becomes

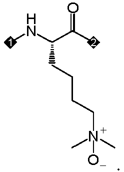

In each part of this specification, substituents for the compounds of the present disclosure are disclosed according to group types or ranges. It is specifically noted that each independent sub-combination of the various members of these group types and ranges is included in the present disclosure. For example, the term "C1-6 alkyl" refers specifically to independently disclosed methyl, ethyl, C3 alkyl, C4 alkyl, C5 alkyl, and C6 alkyl.

Herein, the term "C1-6 alkyl" refers to linear or branched alkyl containing 1-6 carbon atoms, including, for example, "C1-3 alkyl" or "C1-4 alkyl", methyl, ethyl, etc., and specific examples include, but are not limited to, methyl, ethyl, n-propyl, isopropyl, n-butyl, isobutyl, *sec*-butyl, *tert*-butyl, pentyl, and hexyl.

Herein, the term "C₁₋₄ alkyl" refers to linear or branched alkyl containing 1-4 carbon atoms, including, for example, "C₁₋₃ alkyl", methyl, ethyl, etc., and specific examples include, but are not limited to, methyl, ethyl, n-propyl, isopropyl, *n*-butyl, isobutyl, sec-butyl, and *tert*-butyl.

Herein, the term "C₂₋₆ alkenyl" refers to linear, branched, or cyclic alkenyl containing at least one double bond and 2-6 carbon atoms, including, for example, "C₂₋₄ alkenyl", etc. Examples of the C₂₋₆ alkenyl include, but are not limited to, ethenyl, 1-propenyl, 2-propenyl, 1-butenyl, 2-butenyl, 1,3-butadienyl, 1-pentenyl, 2-pentenyl, 3-pentenyl, 1,3-pentadienyl, 1,4-pentadienyl, 1-hexenyl, 2-hexenyl, 3-hexenyl, 1,4-hexadienyl, cyclopentenyl, 1,3-cyclopentadienyl, cyclohexenyl, 1,4-cyclohexadienyl, etc.

Herein, the term "C₂₋₆ alkynyl" refers to linear or branched alkynyl containing at least one triple bond and 2-6 carbon atoms, including, for example, "C₂₋₄ alkynyl", etc. Examples of the C₂₋₆ alkynyl include, but are not limited to, ethynyl, propynyl, 2-butynyl, 2-pentynyl, 3-pentynyl, 4-methyl-2-pentynyl, 2-hexynyl, 3-hexynyl, 5-methyl-2-hexynyl, etc.

Herein, the term "halogen" includes fluorine, chlorine, bromine, and iodine.

Herein, the term "3-6 membered cycloalkyl" or "C₃₋₆ cycloalkyl" refers to saturated cyclic alkyl containing 3-6 carbon atoms and includes cyclopropanyl (i.e., cyclopropyl), cyclobutanyl (i.e., cyclobutyl), cyclopentanyl (i.e., cyclopentyl), and cyclohexyl.

Herein, the term "3-7 membered carbocycloalkyl" or "C₃₋₇ cycloalkyl" refers to saturated cyclic alkyl containing 3-7 carbon atoms, including cyclopropanyl, cyclobutanyl, cyclopentanyl, cyclohexyl, and cycloheptyl.

Herein, the term "C1-6 alkoxy" refers to alkyl as defined above which is attached to the parent molecular moiety via an oxygen atom. Specific examples include, but are not limited to, methoxy, ethoxy, propoxy, isopropoxy, n-propoxy, isopropoxy, *n*-butoxy, isobutoxy, *tert*-butoxy, pentoxy, hexoxy, etc.

Herein, the term "C₁₋₄ alkoxy" refers to alkyl as defined above which is attached to the parent molecular moiety via an oxygen atom. Specific examples include, but are not limited to, methoxy, ethoxy, propoxy, isopropoxy, n-propoxy, isopropoxy, *n*-butoxy, isobutoxy, *tert*-butoxy, etc.

Herein, the term "4-10 membered heterocyclyl" refers to a cyclic group containing 4-10 ring atoms (at least one of which is a heteroatom, e.g., a nitrogen atom, an oxygen atom, or a sulfur atom). The term "4-6 membered heterocyclyl" refers to a cyclic group containing 4-6 ring atoms (at least one of which is a heteroatom, e.g., a nitrogen atom, an oxygen atom, or a sulfur atom). Optionally, the ring atom (e.g., the carbon atom, the nitrogen atom, or the sulfur atom) in the cyclic structure may be substituted with oxo. "4-8 membered heterocyclyl" includes, for example, "4-8 membered nitrogen-containing heterocyclyl", "4-8 membered oxygen-containing heterocyclyl", "4-7 membered heterocyclyl", "4-7 membered oxygen-containing heterocyclyl", "4-7 membered heterocyclyl", "4-6 membered heterocyclyl", "5-7 membered heterocyclyl", "5-6 membered heterocyclyl", and "5-6 membered nitrogen-containing heterocyclyl", and includes, but are not limited to, oxocyclobutanyl, pyrrolidinyl, tetrahydrofuranyl, piperidinyl, piperazinyl, tetrahydropyranyl, homopiperazinyl, etc.

Herein, the term "4-10 membered heterocyclic ring" refers to a ring containing 4-10 ring atoms (at least one of which is a heteroatom, e.g., a nitrogen atom, an oxygen atom, or a sulfur atom). The term "5-6 membered heterocyclic ring" refers to a ring containing 5-6 ring atoms (at least one of which is a heteroatom, e.g., a nitrogen atom, an oxygen atom, or a sulfur atom), including but not limited to, pyrrolidine, tetrahydrofuran, piperidine, piperazine, tetrahydropyran, and other rings.

Herein, the term "aryl" refers to a monocyclic or polycyclic hydrocarbon group having aromaticity, such as 6-10 membered aryl, 5-8 membered aryl, etc. Specific examples include, but are not limited to, phenyl, naphthyl, anthryl, phenanthryl, etc. The "6-10 membered aryl" refers to aryl containing 6-10 ring atoms. The "C6-10 aryl" refers to aryl containing 6-10 carbon atoms.

Herein, the term "heteroaryl" refers to a cyclic group having aromaticity, wherein at least one ring atom is a heteroatom, such as a nitrogen atom, an oxygen atom, or a sulfur atom. Optionally, the ring atom (e.g., the carbon atom, the nitrogen atom, or the sulfur atom) in the cyclic structure may be substituted with oxo. Specific examples include, but are not limited to, 5-10 membered heteroaryl, 5-6 membered heteroaryl, 5-10 membered nitrogen-containing heteroaryl, 6-10 membered oxygen-containing heteroaryl, 6-8 membered nitrogen-containing heteroaryl, 5-8 membered oxygen-containing heteroaryl, etc, such as furanyl, thienyl, pyrrolyl, thiazolyl, isothiazolyl, thiadiazolyl, oxazolyl, isoxazolyl, oxadiazolyl, imidazolyl, pyrazolyl, 1,2,3-triazolyl, 1,2,4-triazolyl, 1,2,3-oxadiazolyl, 1,2,4-oxadiazolyl, 1,2,5-oxadiazolyl, 1,3,4-oxadiazolyl, pyridinyl, 2-pyridonyl, 4-pyridonyl, pyrimidinyl, 1,4-dioxacyclohexadienyl, 2*H*-1,2-oxazinyl, 4*H*-1,2-oxazinyl, 6*H*-1,2-oxazinyl, 4*H*-1,3-oxazinyl, 6*H-*1,3-oxazinyl, 4*H*-1,4-oxazinyl, pyridazinyl, pyrazinyl, 1,2,3-triazinyl, 1,3,5-triazinyl, 1,2,4,5-tetrazinyl, azepinyl, 1,3-diazepinyl, azocinyl, etc.

The bond in the structural formula represented by a wave line "~~" herein is intended to represent that the structure represents a cis or trans isomer, or a mixture of cis and trans isomers in any proportion.

The term "drug-to-antibody ratio" or "DAR" refers to the ratio of the drug to the antibody in a population (or mixture) or composition or ADC molecule, such as a small molecule toxin attached to an antibody of an ADC. The DAR of an ADC may be in the range of 1 to 16, but higher loads (e.g., 20) are also possible depending on the number of linking sites on the antibody. The term DAR may be used when referring to the number of drugs loaded onto a single antibody, or alternatively, when referring to an average or mean DAR for a set of ADCs. It should be understood that the latter is commonly referred to as an average DAR. In the process of determining DAR values by mass spectrometry, the antibodies have been reduced into separate heavy and light chains, wherein DAR1 represents a conjugate comprising a light chain or heavy chain conjugated to 1 toxin molecule; DAR2 represents a conjugate comprising a light chain or heavy chain conjugated to 2 toxin molecules; DAR3 represents a conjugate comprising a light chain or heavy chain conjugated to 3 toxin molecules.

### Beneficial effects of the present disclosure

Through extensive research on multiple target antibody-drug conjugates (ADCs), the present disclosure provides enrichment in tumor microenvironment, the unique *in vivo* enzyme digestion characteristics of a linker, and the conjugation manner between the linker and a targeting moiety. By combining these with extensive screening and verification of *in vivo* and *in vitro* efficacy, a novel antibody-bioactive molecule conjugate is obtained. The conjugate obtained in the manner described above is capable of achieving a variety of surprising technical effects as follows:

the conjugate obtained according to the manner described above has better solubility and excellent chemical stability, such as no occurrence of reversible Michael addition reaction caused by the maleimide linkage manner in conventional ADCs, and thus can achieve a high drug-to-antibody ratio, and in some embodiments, the DAR value of the conjugate can reach 6 to 8;

the conjugation efficiency is extremely high, and in some embodiments, the conjugation efficiency can reach or exceed 90%;

through extensive research, a class of linkers with high plasma stability has been discovered, which can be cleaved in a tumor microenvironment (both inside and outside of a tumor cell), thus achieving good anti-tumor effects even in a tumor with low or no antigen expression;

the conjugate (ADC) obtained according to the manner described above improves the exposure amount of the entire ADC molecule in a relatively acidic tumor environment by adjusting the physicochemical properties of the linker and the entire ADC molecule, such that the ADC has better tumor tissue targeting property, i.e., the enrichment capacity in the tumor microenvironment, which increases the ratio of bioactive molecule concentration within the tumor to that in the blood and reduces the mechanism-related toxicity of the ADC molecule (toxicity of the ADC after binding to cell surface antigens in non-tumor tissues and endocytosis, also known as "on-target toxicity"), thus achieving a higher therapeutic index;

the conjugate obtained according to the manner described above has high stability in circulation *in vivo,* reducing the detachment of drug molecules in non-target tissues and weakening the "off-target" toxicity caused by the detachment of toxins in the non-target tissues;

the bioactive molecule of the conjugate has higher anti-tumor cell activity, such that the ADC has an excellent by-stander effect and can more effectively kill tumor cells with high antigen expression as well as tumor cells with low or no antigen expression in tumor tissues;

the toxin-linker of the present disclosure can form an antibody-drug conjugate with an antibody that has no endocytosis capacity by utilizing the extracellular cleavage capacity of the linker in a tumor microenvironment, and such an antibody-drug conjugate still has high anti-tumor activity;

the toxin-linker of the present disclosure can form an antibody-drug conjugate with an antibody that has no endocytosis capacity or extracellular tumor antigen binding capacity by utilizing the extracellular cleavage capacity of the linker in a tumor microenvironment and the enrichment capacity of the linker in the tumor microenvironment, and such an antibody-drug conjugate still has high anti-tumor activity;

the anti-NaPi2b antibody provided by the present disclosure has an extremely high degree of humanization or is a fully human antibody, and thus can be safely administered to a human subject without eliciting an immunogenic response.

In conclusion, the ADC of the present disclosure has great clinical value.

### BRIEF DESCRIPTION OF THE DRAWINGS

FIG. 1 shows an evaluation of the affinity of anti-NaPi2b humanized antibodies by flow cytometry.
FIG. 2 shows an assay for the endocytic activity of anti-NaPi2b humanized antibodies.
FIG. 3A shows an assay for the affinity of the 66C12D12-hz1 antibody in serum.
FIG. 3B shows an assay for the affinity of the 16G5B3-hz2 antibody in serum.
FIG. 4 shows a test of the *in vivo* efficacy of anti-human NaPi2b ADCs in the HT29-h.NaPi2b CDX model.
FIG. 5 shows the body weight changes of mice during administration of anti-human NaPi2b ADCs in the HT29-h.NaPi2b CDX model.
FIG. 6 shows a test of the *in vivo* efficacy of anti-human NaPi2b ADCs in the IGR-OV1 CDX model.
FIG. 7 shows the body weight changes of mice during the administration of anti-human NaPi2b ADCs in the IGR-OV1 CDX model.

### DETAILED DESCRIPTION OF THE PREFERRED EMBODIMENT

The present disclosure is further illustrated by the following specific examples, which, however, are not intended to limit the present disclosure. Based on the teachings of the present disclosure, those skilled in the art can make various modifications or improvements without departing from the basic spirit and scope of the present disclosure. Reagents or instruments without specified manufacturers used herein are conventional products that are commercially available.

The abbreviations in the present disclosure have the following meanings:

| | | | |
|---|---|---|---|
| OMs | Methanesulfonate group | FA | Formic acid |
| OTs | *p*-Toluenesulfonate group | ACN | Acetonitrile |
| OTf | Trifluoromethanesulfonate group | CCK8 reagent | 2-(2-Methoxy-4-nitrophenyl)-3-(4-nitrophenyl)-5-(2,4-disulfophenyl)-2*H-*tetrazolium monosodium salt |
| TBS | *tert*-Butyldimethylsilyl | FBS | Fetal bovine serum |
| MMT | *p*-Methoxyphenyl diphenylmethyl | DMSO | Dimethyl sulfoxide |
| PB/PBS | Phosphate buffer | HBTU | *O*-Benzotriazol-tetramethyluronium hexafluorophosphate |
| HOBT | 1-Hydroxybenzotriazole | HATU | *O*-(7-Azabenzotriazol-1-yl)-*N,N*,*N'*,*N*'-tetramethyluronium hexafluorophosphate |
| NBS | *N*-Bromosuccinimide | TFA | Trifluoroacetic acid |
| PPTS | Pyridinium *p*-toluenesulfonate | DCM | Dichloromethane |
| THF | Tetrahydrofuran | DMF | *N,N*-Dimethylformamide |
| EDC | 1-(3-Dimethylaminopropyl)-3-ethylcarbodiimide hydrochloride | IgG | Immunoglobulin G |
| CDR | Complementarity determining region in a variable region of an immunoglobulin | HRP | Horseradish peroxidase |
| FR | Antibody framework region: amino acid residues in a variable region of an antibody other than CDR residues | hFc | Fc fragment of a human IgG antibody |
| VH | Heavy chain variable region of an antibody | KD | Equilibrium dissociation constant |
| VL | Light chain variable region of an antibody | HCDR1 | Complementarity determining region 1 in a heavy chain variable region of an immunoglobulin |
| AbM | AbM CDR definitions derived from Martin's related studies (Martin ACR, Cheetham JC, Rees AR (1989) Modelling antibody hypervariable loops: A combined algorithm. Proc Natl Acad Sci USA 86: 9268-9272), which incorporate partial definitions of both Kabat and Chothia. | HCDR2 | Complementarity determining region 2 in a heavy chain variable region of an immunoglobulin |
| Kabat | An immunoglobulin alignment and numbering system proposed by Elvin A. Kabat (see, e.g., Kabat et al., Sequences of Proteins of Immunological Interest, 5th Ed. Public Health Service, National Institutes of Health, Bethesda, Md., 1991). | HCDR3 | Complementarity determining region 3 in a heavy chain variable region of an immunoglobulin |
| Chothia | An immunoglobulin numbering system proposed by Chothia et al., which is a classical rule for identifying CDR region boundaries based on the position of structural loop regions (see, e.g., Chothia & Lesk (1987) J. Mol. Biol. 196: 901-917; Chothia et al., (1989) Nature 342: 878-883). | LCDR1 | Complementarity determining region 1 in a light chain variable region of an immunoglobulin |
| IMGT | A numbering system based on the international ImMunoGeneTics information system^{®} (IMGT) initiated by Lefranc et al., see Lefranc et al., Dev. Comparat. Immunol., 27: 55-77, 2003. | LCDR2 | Complementarity determining region 2 in a light chain variable region of an immunoglobulin |
| mAb | Monoclonal antibody | LCDR3 | Complementarity determining region 3 in a light chain variable region of an immunoglobulin |
| EC50 | Concentration that gives 50% efficacy or binding | SEC-HPLC | Size-exclusion high-performance liquid chromatography |
| ELISA | Enzyme-linked immunosorbent assay | HC | Immunoglobulin heavy chain |
| PCR | Polymerase chain reaction | LC | Immunoglobulin light chain |

### Sequences and specific information thereof:

| SEQ ID No. | Name | Sequence |
|---|---|---|
| 1 | 66C12D12 VH | QIQLVQSGAELVRPGASVKMSCKTSGYTFTTYNVH WVKQTPRQGLEWIGAISPGNGLTSYTQKFKAKATLT VDKSSSTAYMQLSSLTSEDSAVYFCARGGFRLGSMD YWGQGTSVTVSS |
| 2 | 66C12D12 VL | DIVMTQSHKFMSTSVGDRVSVTCKASQNVGTSVAW YQQRPGQSPKELIYWASNRFTGVPDRFTGSGSGTDF TLTITNVQSEDLADYFCQQYSSPPLTFGAGTKLELK |
| 3 | 66C12D12/66C12D12-hz1 Kabat HCDR1 | TYNVH |
| 4 | 66C12D12 Kabat HCDR2 | AISPGNGLTSYTQKFKA |
| 5 | 66C12D12 hz1 Kabat HCDR2 | AISPGSGLTSYAQKFQG |
| 6 | 66C12D12/66C12D12-hz1 Kabat HCDR3 | GGFRLGSMDY |
| 7 | 66C12D12 Kabat LCDR1 | KASQNVGTSVA |
| 8 | 66C12D12 hz1 Kabat LCDR1 | SASQNVGTSVA |
| 9 | 66C12D12/66C12D12-hz1 Kabat LCDR2 | WASNRFT |
| 10 | 66C12D12/66C12D12-hz1 Kabat/ IMGT LCDR3 | QQYSSPPLT |
| 11 | 66C12D12/66C12D12-hz1 IMGT HCDR1 | GYTFTTYN |
| 12 | 66C12D12 IMGT HCDR2 | ISPGNGLT |
| 13 | 66C12D12 hz1 IMGT HCDR2 | ISPGSGLT |
| 14 | 66C12D12/66C12D12-hz1 IMGT HCDR3 | ARGGFRLGSMDY |
| 15 | 66C12D12/66C12D12-hz1 IMGT LCDR1 | QNVGTS |
| / | 66C12D12/66C12D12-hz1 IMGT LCDR2 | WAS |
| 16 | 60A12B3/60A12B3 hz1 IMGT LCDR3 | QHYSKLPLT |
| 17 | 66C12D12-hz1 VH | QVQLVESGAEVKQPGESVKVSCKTSGYTFTTYNVH WVRQAPGQGLEWMGAISPGSGLTSYAQKFQGRVTL TVDKSTSTAYMELSSLRSEDTAVYYCARGGFRLGSM DYWGQGTLVTVSS |
| 18 | 66C12D12-hz1 VL | DYWGQGTLVTVSS DIQMTQSPSFLSASVGDRVTITCSASQNVGTSVAWYQ QKPGQSPKELIYWASNRFTGVPSRFSGSGSGTDFTLTI SSLQPEDFATYYCQQYSSPPLTFGQGTKVEIK |
| 19 | 34F2A10 VH | QAYLQQSGADLVRPGASVKMSSKASGSSFTTYIIHWI KQTPGQGLEWIGAIYPGNGYTSYNQKFKGKATLTVD KSYNTAYIQLSSLSSEDSAVYFCARGNYGNYEGFTY WGQGTLVTVSA |
| 20 | 34F2A10 VL | DIQMTQTTSSLSVSLGDRITISCSASQAIYKYLNWYQ QKPDGTVKLLIYYTSTLHSGVPSRFSGSGSGTDYSLTI SNLEPEDIASYYCQQYNKFPFTFGSGTKLEIK |
| 21 | 34F2A10/34F2A10-hz1 Kabat HCDR1 | TYIIH |
| 22 | 34F2A10 Kabat HCDR2 | AIYPGNGYTSYNQKFKG |
| 23 | 34F2A10-hz1 Kabat HCDR2 | AIYPGSGYTSYAQKFQG |
| 24 | 34F2A10/34F2A10-hz1 Kabat HCDR3 | GNYGNYEGFTY |
| 25 | 34F2A10/34F2A10-hz1 Kabat LCDR1 | SASQAIYKYLN |
| 26 | 34F2A10/34F2A10-hz1 Kabat LCDR2 | YTSTLHS |
| 27 | 34F2A10/34F2A10-hz1 Kabat/IMGT LCDR3 | QQYNKFPFT |
| 28 | 34F2A10/34F2A10-hz1 IMGT HCDR1 | GSSFTTYI |
| 29 | 34F2A10 IMGT HCDR2 | IYPGNGYT |
| 30 | 34F2A10-hz1 IMGT HCDR2 | IYPGSGYT |
| 31 | 34F2A10/34F2A10-hz1 IMGT HCDR3 | ARGNYGNYEGFTY |
| 32 | 34F2A10/34F2A10-hz1 IMGT LCDR1 | QAIYKY |
| / | 34F2A10/34F2A10-hz1 IMGT LCDR2 | YTS |
| 33 | 60A12B3-hz1 VH | QVQLVESGPGLVQPSETLSLTCTVSGFSLTNYGVHW VRQPPGKGLEWIGVIWSGGRTNYNPSLQSRVTISKD NSTNQVSLKLSSVTAADTAVYYCAKRDGIYGDYWG QGTLVTVSS |
| 34 | 34F2A10-hz1 VH | QVQLVESGAEVKQPGESVKVSCKASGSSFTTYIIHWI RQAPGQGLEWMGAIYPGSGYTSYAQKFQGRVTLTV DKSTSTAYMELSSLRSEDTAVYFCARGNYGNYEGFT YWGQGTLVTVSS |
| 35 | 34F2A10-hz1 VL | DIQMTQSPSSLSASVGDRVTITCSASQAIYKYLNWYQ QKPGQAPKLLIYYTSTLHSGVPSRFSGSGSGTDYTLTI SSLQPEDFATYYCQQYNKFPFTFGQGTKVEIK |
| 36 | 22H9C4 VH | QVQLQQSGAELVRPGASVKMSCKASGYTFTSNNLH WVKLTPRQGLEWIGAVYPGNGDTSYTQKFKGKATLT VDKSSSIAYMQLSSPTSEDSAVYFCARGLPYLGAMD YWGQGTSVTVSS |
| 37 | 22H9C4 VL | [?] DIVMTQSQRFMSTTVGDRVSITCKASQNVGIFVTWY QQKPGQSPKLLIYSASSRYTGVPDRFTGSGSGTDFTL TISNMQSEDLADYFCQQYSRSPLSFGAGTKLELK |
| 38 | 22H9C4/22H9C4-hz1 Kabat HCDR1 | SNNLH |
| 39 | 22H9C4 Kabat HCDR2 | AVYPGNGDTSYTQKFKG |
| 40 | 22H9C4-hz1 Kabat HCDR2 | AVYPGSGDTSYSQKFQG |
| 41 | 22H9C4/22H9C4-hz1 Kabat HCDR3 | GLPYLGAMDY |
| 42 | 22H9C4 Kabat LCDR1 | KASQNVGIFVT |
| 43 | 22H9C4-hz1 Kabat LCDR1 | SASQNVGIFVT |
| 44 | 22H9C4/22H9C4-hz1 Kabat LCDR2 | SASSRYT |
| 45 | 22H9C4/22H9C4-hz1 Kabat/IMGT LCDR3 | QQYSRSPLS |
| 46 | 22H9C4/22H9C4-hz1 IMGT HCDR1 | GYTFTSNN |
| 47 | 22H9C4 IMGT HCDR2 | VYPGNGDT |
| 48 | 22H9C4-hz1 IMGT HCDR2 | VYPGSGDT |
| 49 | 22H9C4/22H9C4-hz1 IMGT HCDR3 | ARGLPYLGAMDY |
| 50 | 22H9C4/22H9C4-hz1 IMGT LCDR1 | QNVGIF |
| / | 22H9C4/22H9C4-hz1 IMGT LCDR2 | SAS |
| 51 | 60A12B3-hz1 VL | DIQMTQSPSSLSASVGDRVTITCSASQDIGTYLNWYQ QKPGQAPKLLIYHTSRLHSGVPSRFSGSGSGTDFTLTI SSLOPEDFATYYCOHYSKLPLTFGOGTKVEIK |
| 52 | 22H9C4-hz1 VH | QVQLVESGAEVKQPGESVKVSCKASGYTFTSNNLH WVRQAPGQGLEWMGAVYPGSGDTSYSQKFQGRVT LTVDKSASTAYMELSSLRSEDTAVYFCARGLPYLGA MDYWGQGTLVTVSS |
| 53 | 22H9C4-hz1 VL | DIQMTQSPSFLSASVGDRVTITCSASQNVGIFVTWYQ QKPGQSPKLLIYSASSRYTGVPSRFSGSGSGTDFTLTI SSLQPEDFATYYCQQYSRSPLSFGQGTKVEIK |
| 54 | 16G5B3 VH | QVQLQQSGAELVRPGASVKMSCKASGYTFASYNMH WVKKTPRQGLEWIGAIYPGNGNTSYNQKFKGKATL TVDKSSSTAYMQLSSLTSEDSAVYFCVRGGRGLGTM DYWGQGTSVTVSS |
| 55 | 16G5B3 VL | DIVMTQSHKFMSTSVGDRVSITCKASQDVGSSVAWY QQKPGQSPKLLIYWASTRHTGVPDRFTGSGSGTDFT LTISNVQSEDLADYFCQHYRNIPLTFGAGTKLELK |
| 56 | 16G5B3/16G5B3-hz1/16G5B3-hz2 Kabat HCDR1 | SYNMH |
| 57 | 16G5B3 Kabat HCDR2 | AIYPGNGNTSYNQKFKG |
| 58 | 16G5B3-hz1 Kabat HCDR2 | AIYPGSGNTSYSQKFQG |
| 59 | 16G5B3-hz2 Kabat HCDR2 | AIYPGSGNTGYSQKFQG |
| 60 | 16G5B3/16G5B3-hz1/16G5B3-hz2 Kabat HCDR3 | GGRGLGTMDY |
| 61 | 16G5B3 Kabat LCDR1 | KASQDVGSSVA |
| 62 | 16G5B3-hz1/16G5B3-hz2 Kabat LCDR1 | SASQDVGSSVA |
| 63 | 16G5B3/16G5B3-hz1/16G5B3-hz2 Kabat LCDR2 | WASTRHT |
| 64 | 16G5B3/16G5B3-hz1/16G5B3-hz2 Kabat/IMGT LCDR3 | QHYRNIPLT |
| 65 | 16G5B3/16G5B3-hz1/16G5B3-hz2 IMGT HCDR1 | GYTFASYN |
| 66 | 16G5B3 IMGT HCDR2 | IYPGNGNT |
| 67 | 16G5B3-Hz1/ 16G5B3-Hz2 IMGT HCDR2 | IYPGSGNT |
| 68 | 16G5B3/16G5B3-hz1/16G5B3-hz2 IMGT HCDR3 | VRGGRGLGTMDY |
| 69 | 16G5B3/16G5B3 hz1/16G5B3 hz2 IMGT LCDR1 | QDVGSS |
| / | 16G5B3/16G5B3 hz1/16G5B3 hz2 IMGT LCDR2 | WAS |
| 70 | IgG1 CH heavy chain constant region | |
| 71 | 16G5B3-hz1 VH | QVQLVESGAEVKQPGESVKVSCKASGYTFASYNMH WVRQAPGQGLEWMGAIYPGSGNTSYSQKFQGRVTL TVDKSASTAYMELSSLRSEDTAVYFCVRGGRGLGTM DYWGQGTLVTVSS |
| 72 | 16G5B3-hz1/16G5B3-hz2 VL | DIQMTQSPSFLSASVGDRVTITCSASQDVGSSVAWYQ QKPGQSPKLLIYWASTRHTGVPSRFSGSGSGTDFTLTI SSLQPEDFATYYCQHYRNIPLTFGQGTKVEIK |
| 73 | 16G5B3-hz2 VH | QVQLVESGAEVKQPGESVKVSCKASGYTFASYNMH WVRQAPGQGLEWMGAIYPGSGNTGYSQKFQGRVT LTVDKSASTAYMELSSLRSEDTAVYFCVRGGRGLGT MDYWGQGTLVTVSS |
| 74 | 60A12B3 VH | QVQLKESGPVLVAPSQSLSITCTVSGFSLTNYGVHWV RQPPGKGLEWLGVIWSGGRTNYNSALMSRLSISKDN |
| | | SKSQVFLKMNSLQTDDTAMYYCAKRDGIYGDYWG QGTSVTVSS |
| 75 | 60A12B3 VL | DIQMTQTTSSLSASLGDRVTISCRASQDIGTYLNWYQ QKPDGTVTLLIYHTSRLHSGVPSRFSGSGSGTDFSLTI SSLEPEDIATYYCQHYSKLPLTFGAGTKLELK |
| 76 | 60A12B3/60A12B3 hz1 IMGT HCDR1 | GFSLTNYG |
| 77 | 60A12B3/60A12B3 hz1 IMGT HCDR2 | IWSGGRT |
| 78 | 60A12B3/60A12B3 hz1 IMGT HCDR3 | AKRDGIYGDY |
| 79 | 60A12B3/60A12B3 hz1 IMGT LCDR1 | QDIGTY |
| / | 60A12B3/60A12B3 hz1 IMGT LCDR2 | HTS |
| 80 | Kappa CL light chain constant region | |
| 81 | 16G5B3-hz2 HC heavy chain | QVQLVESGAEVKQPGESVKVSCKASGYTFASYNMH WVRQAPGQGLEWMGAIYPGSGNTGYSQKFQGRVT LTVDKSASTAYMELSSLRSEDTAVYFCVRGGRGLGT MDYWGQGTLVTVSSASTKGPSVFPLAPSSKSTSGGT AALGCLVKDYFPEPVTVSWNSGALTSGVHTFPAVLQ SSGLYSLSSVVTVPSSSLGTQTYICNVNHKPSNTKVD KKVEPKSCDKTHTCPPCPAPELLGGPSVFLFPPKPKD TLMISRTPEVTCVVVDVSHEDPEVKFNWYVDGVEV HNAKTKPREEQYNSTYRVVSVLTVLHQDWLNGKEY KCKVSNKALPAPIEKTISKAKGQPREPQVYTLPPSRD ELTKNQVSLTCLVKGFYPSDIAVEWESNGQPENNYK TTPPVLDSDGSFFLYSKLTVDKSRWQQGNVFSCSVM HEALHNHYTQKSLSLSPGK |
| 82 | 16G5B3-hz2 LC light chain | DIQMTQSPSFLSASVGDRVTITCSASQDVGSSVAWYQ QKPGQSPKLLIYWASTRHTGVPSRFSGSGSGTDFTLTI SSLQPEDFATYYCQHYRNIPLTFGQGTKVEIKRTVAA PSVFIFPPSDEQLKSGTASVVCLLNNFYPREAKVQWK VDNALQSGNSQESVTEQDSKDSTYSLSSTLTLSKAD YEKHKVYACEVTHQGLSSPVTKSFNRGEC |
| 83 | 66C12D12-hz1 HC heavy chain | QVQLVESGAEVKQPGESVKVSCKTSGYTFTTYNVH WVRQAPGQGLEWMGAISPGSGLTSYAQKFQGRVTL TVDKSTSTAYMELSSLRSEDTAVYYCARGGFRLGSM DYWGQGTLVTVSSASTKGPSVFPLAPSSKSTSGGTA ALGCLVKDYFPEPVTVSWNSGALTSGVHTFPAVLQS SGLYSLSSVVTVPSSSLGTQTYICNVNHKPSNTKVDK KVEPKSCDKTHTCPPCPAPELLGGPSVFLFPPKPKDT LMISRTPEVTCVVVDVSHEDPEVKFNWYVDGVEVH NAKTKPREEQYNSTYRVVSVLTVLHQDWLNGKEYK CKVSNKALPAPIEKTISKAKGQPREPQVYTLPPSRDE LTKNQVSLTCLVKGFYPSDIAVEWESNGQPENNYKT TPPVLDSDGSFFLYSKLTVDKSRWQQGNVFSCSVMH EALHNHYTQKSLSLSPGK |
| 84 | 66C12D12-hz1 LC light chain | DIQMTQSPSFLSASVGDRVTITCSASQNVGTSVAWYQ QKPGQSPKELIYWASNRFTGVPSRFSGSGSGTDFTLTI SSLQPEDFATYYCQQYSSPPLTFGQGTKVEIKRTVAA PSVFIFPPSDEQLKSGTASVVCLLNNFYPREAKVQWK VDNALQSGNSQESVTEQDSKDSTYSLSSTLTLSKAD YEKHKVYACEVTHQGLSSPVTKSFNRGEC |
| 85 | hFc | PKSSDKTHTCPPCPAPELLGGPSVFLFPPKPKDTLMIS RTPEVTCVVVDVSHEDPEVKFN WYVDGVEVHNAKTKPREEQYNSTYRVVSVLTVLHQ DWLNGKEYKCKVSNKALPAPIEKTI SKAKGQPREPQVYTLPPSRDELTKNQVSLTCLVKGF YPSDIAVEWESNGQPENNYKTTPP VLDSDGSFFLYSKLTVDKSRWQQGNVFSCSVMHEAL HNHYTQKSLSLSPGK |
| 86 | mFc | GGGGSPRGPTIKPCPPCKCPAPNLLGGPSVFIFPPKIK DVLMISLSPIVTCVVVDVSEDD PDVQISWFVNNVEVHTAQTQTHREDYNSTLRVVSAL PIQHQDWMSGKEFKCKVNNKDLPA PIERTISKPKGSVRAPQVYVLPPPEEEMTKKQVTLTC MVTDFMPEDIYVEWTNNGKTELN YKNTEPVLDSDGSYFMYSKLRVEKKNWVERNSYSC SVVHEGLHNHHTTKSFSRTPGK |

The present disclosure is described with reference to the following examples, which are intended to illustrate (but not to limit) the present disclosure.

Unless otherwise indicated, the molecular biology experimental methods and immunoassays, as used in the present disclosure, are substantially performed with reference to the methods described in J. Sambrook et al., Molecular Cloning: A Laboratory Manual, 2nd Edition, Cold Spring Harbor Laboratory Press, 1989, and F. M. Ausubel et al., Short Protocols in Molecular Biology, 3rd Edition, John Wiley & Sons, Inc., 1995. Those skilled in the art know that the examples describe the present disclosure by way of example and are not intended to limit the protection scope claimed in the present disclosure.

### Preparation schemes

The structures of the compounds described in the following examples were determined by nuclear magnetic resonance (¹H NMR) or mass spectrometry (MS).

Nuclear magnetic resonance (¹H NMR) was determined using a Bruker 400 MHz nuclear magnetic resonance spectrometer, the solvents for the determination were deuterated methanol (CD₃OD), deuterated chloroform (CDCl₃), or hexadeuterodimethyl sulfoxide (DMSO-d₆), and the internal standard substance was tetramethylsilane (TMS).

The abbreviations in the nuclear magnetic resonance (NMR) spectra used in the examples are shown as follows:
s: singlet; d: doublet; t: triplet; q: quartet; dd: double doublet; qd: quartet doublet; ddd: double double doublet; ddt: double double triplet; dddd: double double double doublet; m: multiplet; br: broad; J: coupling constant; Hz: Hertz; DMSO-d₆: hexadeuterodimethyl sulfoxide. δ values were expressed in ppm.

Mass spectrometry (MS) was performed using an Agilent (ESI) mass spectrometer (model: Agilent 6120B).

### I. Synthesis of Bioactive Molecules and Intermediates Used in the Synthesis of "Drug-Linker Compounds"

### A. Synthesis of bioactive molecules

### Example A1.1: Synthesis of (S)-4-ethyl-8-fluoro-4-hydroxy-9-methyl-11-(1H-pyrazol-4-yl)-1,12-dihydro-14H-pyrano[3',4':6,7]indolizino[1,2-b]quinoline-3,14(4H)-dione (A1.1)

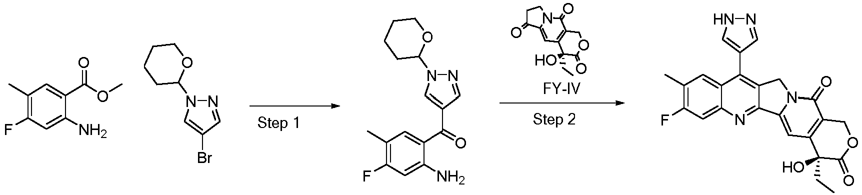

Step 1: 4-Bromo-1-(tetrahydro-2H-pyran-2-yl)-1H-pyrazole (4.6 g) was dissolved in anhydrous tetrahydrofuran (50 mL). The solution was cooled to -78 °C under a nitrogen atmosphere with dry ice-acetone, and then n-butyllithium (12 mL, 2 M) was added dropwise. The reaction solution was stirred at -78 °C for 20 min, and then methyl 2-amino-4-fluoro-5-methylbenzoate (1.83 g) was added. After the addition, the mixture was naturally warmed to room temperature and stirred continuously for 5 h. After the reaction was quenched by adding methanol (3 mL), ethyl acetate (200 mL) was added, and the solution was washed with water (100 mL × 3). The organic phase was dried, then the organic solvent was removed, and the residue was separated by silica gel column chromatography to obtain the target product (2-amino-4-fluoro-5-methylphenyl)(1-(tetrahydro-2*H*-pyran-2-yl)-1*H-*pyrazol-4-yl)methanone. ESI-MS (m/z): 304 [M+H]⁺.

Step 2: (*S*)-4-Ethyl-4-hydroxy-7,8-dihydro-1H-pyrano[3,4-*f*]indolizine-3,6,10(4*H*)-trione (2.63 g), (2-amino-4-fluoro-5-methylphenyl)(1-(tetrahydro-2*H*-pyran-2-yl)-1*H*-pyrazol-4-yl)methanone (3.03 g), and *p-*toluenesulfonic acid (1.74 g) were dissolved in dichloromethane (50 mL), then the solvent was removed, and the mixture was heated to 120 °C under a nitrogen atmosphere and allowed to react for 4 h. The mixture was dissolved in ethyl acetate (300 mL), the organic phase was washed with water (100 mL × 2) and dried, and then the organic solvent was removed. The residue was separated by silica gel column chromatography to obtain the target product (*S*)-4-ethyl-8-fluoro-4-hydroxy-9-methyl-11-(1*H*-pyrazol-4-yl)-1,12-dihydro-14*H*-pyrano[3',4':6,7]indolizino[1,2-b]quinoline-3,14(4*H*)-dione (A1.1). ESI-MS (m/z): 447 [M+H]⁺.

### Example A1.2: Synthesis of (S)-7-ethyl-7-hydroxy-14-(1H-pyrazol-4-yl)-10,13-dihydro-11H-[1,3]dioxolo[4,5-g]pyrano[3',4':6,7]indolizino[1,2-b]quinoline-8,11(7H)-dione (A1.2)

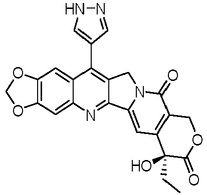

Following the same procedures and reaction conditions as in Example A1.1, but replacing methyl 2-amino-4-fluoro-5-methylbenzoate with methyl 6-aminobenzo[*d*][1,3]dioxolo-5-carboxylate, the target product (*S*)-7-ethyl-7-hydroxy-14-(1*H*-pyrazol-4-yl)-10,13-dihydro-11H-[1,3]dioxolo[4,5-g]pyrano[3',4':6,7]indolizino[1,2-b]quinoline-8,11(7*H*)-dione (A1.2) was obtained. ESI-MS (m/z): 459 [M+H]⁺.

### Example A1.3: Synthesis of (S)-14-(3-aminophenyl)-7-ethyl-7-hydroxy-10,13-dihydro-11H-[1,3]dioxolo[4,5-g]pyrano[3',4':6,7]indolizino[1,2-b]quinoline-8,11(7H)-dione (A1.3)

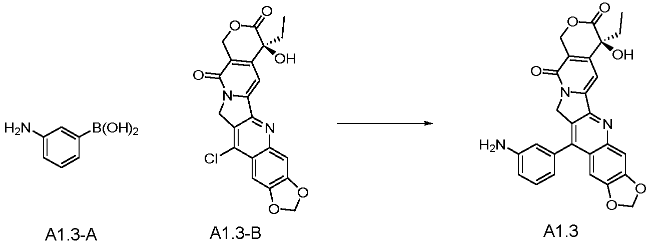

Compound (A1.3-A) (1.4 g), compound (A1.3-B) (2.2 g), Pd₂(DBA)₃ (300 mg), tricyclohexylphosphine (300 mg), and potassium acetate (1.1 g) were added sequentially to a mixed solvent of dioxane (30 mL) and water (5 mL), and the mixture was heated and stirred at 100 °C for 12 h under a nitrogen atmosphere. After the mixture was cooled, ethyl acetate (200 mL) was added, and the mixture was washed with water (100 mL) and dried. Then, the residue was separated by silica gel column chromatography to obtain the target product (S)-14-(3-aminophenyl)-7-ethyl-7-hydroxy-10,13-dihydro-11*H*-[1,3]dioxolo[4,5-*g*]pyrano[3',4':6,7]indolizino[1,2-*b*]quinoline-8,11(7*H*)-dione (A1.3). ESI-MS (m/z): 484 [M+H]⁺.

### Example A1.4: Synthesis of (S)-14-(4-aminophenyl)-7-ethyl-7-hydroxy-10,13-dihydro-11H-[1,3]dioxolo[4,5-g]pyrano[3',4':6,7]indolizino[1,2-b]quinoline-8,11(7H)-dione (A1.4)

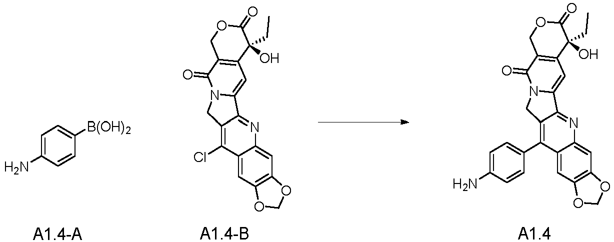

Compound (A1.4-A) (1.4 g), compound (A1.4-B) (2.2 g), Pd₂(DBA)₃ (300 mg), tricyclohexylphosphine (300 mg), and potassium acetate (1.1 g) were added sequentially to a mixed solvent of dioxane (30 mL) and water (5 mL), and the mixture was heated and stirred at 100 °C for 12 h under a nitrogen atmosphere. After the mixture was cooled, ethyl acetate (200 mL) was added, and the mixture was washed with water (100 mL) and dried. Then, the residue was separated by silica gel column chromatography to obtain the target product (*S*)-14-(4-aminophenyl)-7-ethyl-7-hydroxy-10,13-dihydro-11*H*-[1,3]dioxolo[4,5-*g*]pyrano[3',4':6,7]indolizino[1,2-*b*]quinoline-8,11(7*H*)-dione (A1.4).
ESI-MS (m/z): 484 [M+H]⁺;
¹H NMR (400 MHz, DMSO) δ 7.56 (s, 1H), 7.34 (d, *J* = 8.0 Hz, 2H), 7.27 (s, 1H), 7.14 (s, 1H), 6.89 (d, *J* = 7.8 Hz, 2H), 6.26 (s, 2H), 5.40 (s, 2H), 5.05 (s, 2H), 1.96 - 1.78 (m, 2H), 0.88 (t, *J* = 7.2 Hz, 3H).

### Example A1.5: Synthesis of (S)-14-(3-aminopropyl)-7-ethyl-7-hydroxy-10,13-dihydro-11H-[1,3]dioxolo[4,5-g]pyrano[3',4':6,7]indolizino[1,2-b]quinoline-8,11(7H)-dione (A1.5)

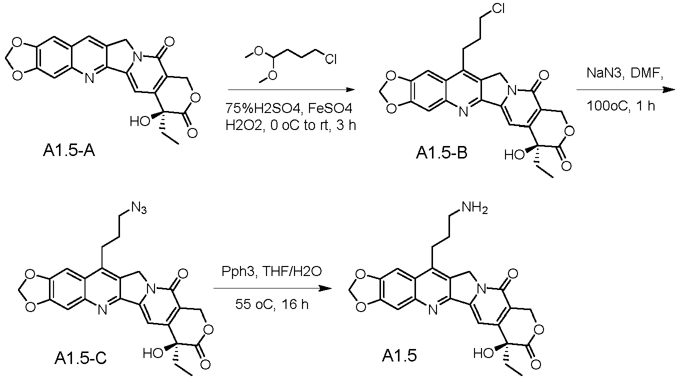

### Step 1: Synthesis of (S)-14-(3-chloropropyl)-7-ethyl-7-hydroxy-10,13-dihydro-11H-[1,3]dioxolo[4,5-g]pyrano[3',4':6,7]indolizino[1,2-b]quinoline-8,11(7H)-dione

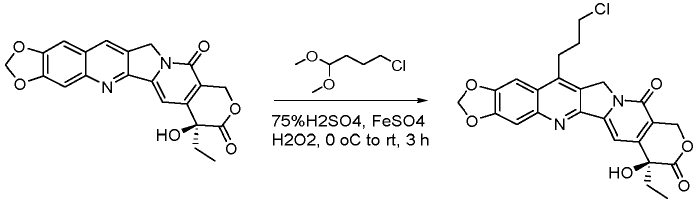

To a 75% sulfuric acid solution (5 mL) of compound (*S*)-7-ethyl-7-hydroxy-10,13-dihydro-11*H-*[1,3]dioxolo[4,5-g]pyrano[3',4':6,7]indolizino[1,2-b]quinoline-8,11(7*H*)-dione (A1.5-A, 500 mg) were added ferrous sulfate heptahydrate (570 mg of ferrous sulfate heptahydrate dissolved in 1 mL of water) and 4,4-dimethoxychlorobutane (3.89 g) under an ice bath. After the reaction solution was stirred for 3 min, hydrogen peroxide (29%, 2.5 mL) was added dropwise. The reaction solution was stirred at 0 °C for 5 min, then warmed to room temperature, and stirred for another 3 h. The reaction solution was diluted with water (50 mL) and extracted with ethyl acetate (80 mL × 2). The organic phase was washed with saturated brine, dried over anhydrous sodium sulfate, and concentrated to obtain a crude product. The crude product was further purified using a C18 column (acetonitrile/0.05% formic acid in water: 5%-60%) to obtain the target compound (yellow solid, 400 mg, yield: 67%).
LCMS (ESI) [M+H]⁺: 468.9;
¹H NMR (400 MHz, DMSO-d6) δ 7.65 (s, 1H), 7.51 (s, 1H), 7.24 (s, 1H), 6.50 (s, 1H), 6.30 (s, 2H), 5.42 (s, 2H), 5.26 (s, 2H), 3.81 (d, *J=* 5.9 Hz, 2H), 3.22 (s, 2H), 1.98 (d, *J* = 6.7 Hz, 4H), 0.88 (t, *J* = 7.2 Hz, 3H).

### Step 2: Synthesis of (S)-14-(3-azidopropyl)-7-ethyl-7-hydroxy-10,13-dihydro-11H-[1,3]dioxolo[4,5-g]pyrano[3',4':6,7]indofizino[1,2-b]quinoline-8,11(7H)-dione

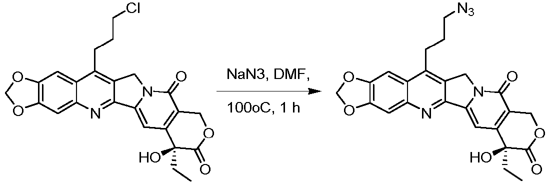

To a solution of compound (*S*)-14-(3-chloropropyl)-7-ethyl-7-hydroxy-10,13-dihydro-11*H-*[1,3]dioxolo[4,5-g]pyrano[3',4':6,7]indolizino[1,2-*b*]quinoline-8,11(7H)-dione (200 mg) in *N,N-*dimethylformamide (3 mL) was added sodium azide (284 mg), and the reaction solution was stirred at 100 °C for 1 h. Water (30 mL) was added to the reaction solution, and the mixture was extracted with ethyl acetate (60 mL × 2). The organic phase was washed with saturated brine, dried over anhydrous sodium sulfate, and concentrated to obtain the target compound (*S*)-14-(3-azidopropyl)-7-ethyl-7-hydroxy-10,13-dihydro-11*H*-[1,3]dioxolo[4,5-g]pyrano[3',4':6,7]indolizino[1,2-*b*]quinoline-8,11(7*H*)-dione (yellow solid, 180 mg, yield: 88%).
LCMS (ESI) [M+H]⁺: 476;
¹H NMR (400 MHz, DMSO-d6) δ 7.65 (s, 1H), 7.51 (s, 1H), 7.24 (s, 1H), 6.48 (s, 1H), 6.29 (s, 2H), 5.42 (s, 2H), 5.25 (s, 2H), 3.53 - 3.49 (m, 2H), 3.16 - 3.12 (m, 2H), 1.93 - 1.80 (m, 4H), 0.88 (t, *J=* 7.2 Hz, 3H).

### Step 3: Synthesis of (S)-14-(3-aminopropyl)-7-ethyl-7-hydroxy-10,13-dihydro-11H-[1,3]dioxolo[4,5-g]pyrano[3',4':6,7]indolizino[1,2-b]quinoline-8,11(7H)-dione

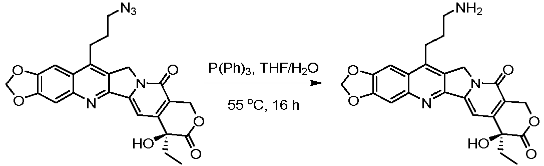

To a mixed solution of compound (S)-14-(3-azidopropyl)-7-ethyl-7-hydroxy-10,13-dihydro-11*H-*[1,3]dioxolo[4,5-g]pyrano[3',4':6,7]indolizino[1,2-*b*]quinoline-8,11(7H)-dione (130 mg, 0.274 mmol) in tetrahydrofuran (3 mL) and water (1 mL) was added triphenylphosphine (108 mg, 0.411 mmol), and the mixture was allowed to react at 55 °C for 16 h. The reaction was completed as detected by LCMS. Water (5 mL) was added to the reaction solution, and the mixture was adjusted to acidity with 2 N hydrochloric acid (3 mL) and extracted with ethyl acetate (10 mL). The aqueous phase was purified by preparative high-performance liquid chromatography (acetonitrile/0.05% formic acid in water) to obtain the target compound (*S*)-14-(3-aminopropyl)-7-ethyl-7-hydroxy-10,13-dihydro-11*H*-[1,3]dioxolo[4,5-g]pyrano[3',4':6,7]indolizino[1,2-*b*]quinoline-8,11(7*H*)-dione (A1.5, yellow solid, 15 mg, yield: 12%).
LCMS (ESI) [M+H]⁺: 449.9;
¹H NMR (400 MHz, DMSO-d6) δ 7.72 (s, 3H), 7.53 (s, 1H), 7.25 (s, 1H), 6.50 (s, 1H), 6.30 (s, 2H), 5.43 (s, 2H), 5.24 (s, 2H), 3.15 (d, *J* = 6.4 Hz, 2H), 3.03 (t, *J* = 6.9 Hz, 2H), 1.96 - 1.81 (m, 4H), 0.88 (t, *J* = 7.3 Hz, 3H).

### Example A1.6: Synthesis of (S)-N-ethyl-N-(2-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)ethyl)-2-hydroxyacetamide (A1.6)

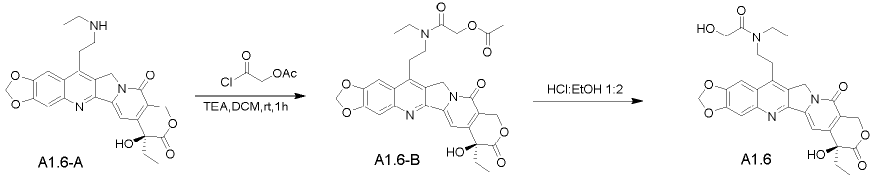

### Step 1: Preparation of (S)-2-(ethyl(2-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)ethyl)amino)-2-oxoethyl acetate (A1.6-B):

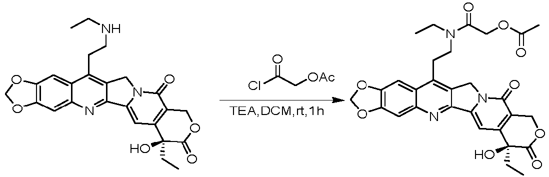

Compound (*S*)-7-ethyl-14-(2-(ethylamino)ethyl)-7-hydroxy-10,13-dihydro-11*H*-[1,3]dioxolo[4,5-g]pyrano[3',4':6,7]indolizino[1,2-b]quinoline-8,11(7*H*)-dione (A1.6-A, 50 mg), acetoxyacetyl chloride (73 mg), and triethylamine (50 mg) were added sequentially to dichloromethane (5 mL), and the mixture was stirred at room temperature for 1 h. The reaction solution was concentrated by rotary evaporation to remove the solvent to obtain a crude product of the compound (75 mg) as an oil.

LCMS (ESI) [M+H]⁺: 564.2.

### Step 2: Synthesis of (S)-N-ethyl-N-(2-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)ethyl)-2-hydroxyacetamide (A1.6)

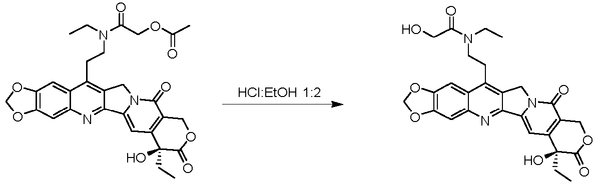

The crude product of compound (*S*)-2-(ethyl(2-(7-ethyl-7-hydroxy-8,11-dioxo-7,8,11,13-tetrahydro-10*H-*[1,3]dioxolo[4,5-g]pyrano[3',4':6,7]indolizino[1,2-*b*]quinolin-14-yl)ethyl)amino)-2-oxoethyl acetate (75 mg, 0.13 mmol) was dissolved in a mixed solution of concentrated hydrochloric acid and anhydrous ethanol (volume ratio: 1/2, 3 mL), and then the reaction solution was refluxed at 85 °C for 1 h. The reaction was completed as detected by LCMS. The reaction solution was concentrated, and the crude product was purified by preparative chromatography (0.01% trifluoroacetic acid in water, acetonitrile) to obtain the target compound (*S*)-*N*-ethyl-N-(2-(7-ethyl-7-hydroxy-8,11-dioxo-7,8,11,13-tetrahydro-10*H*-[1,3]dioxolo[4,5-*g*]pyrano[3',4':6,7]indolizino[1,2*-b*]quinolin-14-yl)ethyl)-2-hydroxyacetamide (A1.6) (15 mg, yield: 26%) as a white solid.
LCMS (ESI) [M+H]⁺: 522.0;
¹H NMR (400 MHz, DMSO) δ 7.92 (s, 1H), 7.53 (m, 1H), 7.25 (s, 1H), 6.50 (s, 1H), 6.31 (s, 2H), 5.43 (s, 2H), 5.34 (m, 2H), 4.65 (m, 1H), 4.07 (m, 2H), 3.52 (m, 2H), 3.41 (m, 2H), 2.00 (m, 2H), 1.88 (m, 2H), 1.16 - 1.04 (m, 3H), 0.89 - 0.83 (m, 3H).

### Example A1.7: Synthesis of (S)-N-methyl-N-(2-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)ethyl)-2-hydroxyacetamide (A1.7)

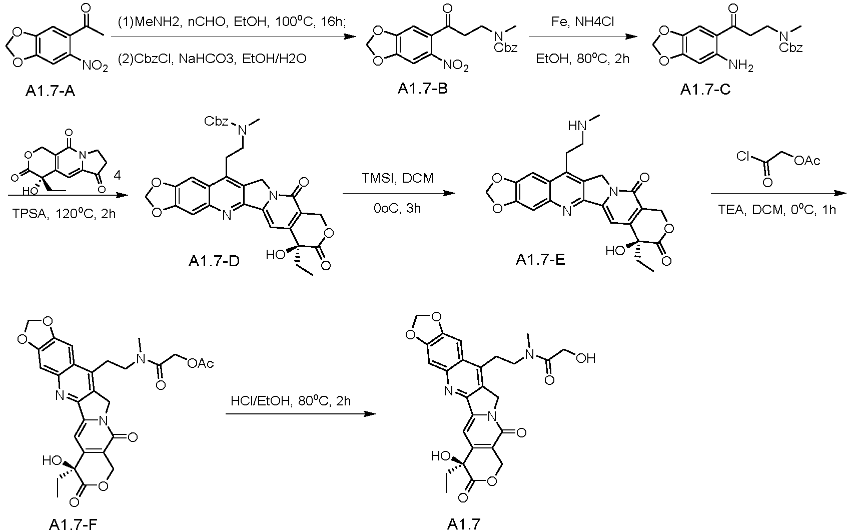

### Step 1: Preparation of compound benzyl methyl(3-(6-nitrobenzo[d][1,3]dioxol-5-yl)-3-oxopropyl)carbamate

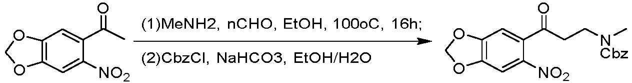

Compound 1-(6-nitrobenzo[d][1,3]dioxol-5-yl)ethan-1-one (A1.7-A, 500 mg), methylamine hydrochloride (1.6 g), and paraformaldehyde (714 mg) were dissolved in ethanol (8 mL), and the resulting solution was allowed to react at 100 °C for 16 h in a closed container. The reaction solution was cooled to room temperature and concentrated under reduced pressure, the resulting residue was dissolved in dichloromethane (80 mL), and the organic phase was extracted with water (50 mL × 3). The resulting aqueous phase was adjusted to pH = 9 with sodium bicarbonate, and then benzyl chloroformate (513 mg, 3.0 mmol) was added. The mixture was allowed to react at room temperature for 16 h. The reaction solution was extracted with ethyl acetate (30 mL × 3). The organic phase was washed with saturated brine, dried over anhydrous sodium sulfate, filtered under vacuum, and concentrated to obtain a crude product. The crude product was separated and purified using a C18 column (acetonitrile/water containing 0.05% formic acid = 5%-95%) to obtain the target compound (A1.7-B, 180 mg, yield: 23%).

LCMS (ESI) [M+H]⁺: 387.1.

¹H NMR (400 MHz, CDCl₃) δ 7.56 (s, 1H), 7.35 (m, 5H), 7.32 (m, 1H), 6.17 (s, 2H), 5.13 (s, 2H), 3.71 (m, 2H), 3.03 (m, 3H), 2.99 - 2.86 (m, 2H).

### Step 2: Preparation of compound benzyl (3-(6-aminobenzo[d][1,3]dioxol-5-yl)-3-oxopropyl)(methyl)carbamate (A1.7-C)

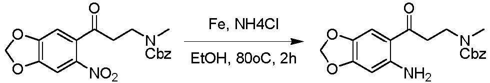

Compound A1.7-B benzyl methyl(3-(6-nitrobenzo[*d*][1,3]dioxol-5-yl)-3-oxopropyl)carbamate (180 mg, 0.47 mmol) was dissolved in a mixed solution of saturated aqueous ammonium chloride solution (8 mL) and ethanol (8 mL), and then iron powder (130 mg) was added to the reaction solution. The reaction mixture was stirred at 80 °C for 2 h. The reaction solution was filtered, the filtrate was concentrated under reduced pressure to obtain a solid, and the crude product was separated and purified by TLC (petroleum ether:ethyl acetate = 2/1) to obtain the target compound A1.7-C (76 mg).

LCMS (ESI) [M+H]⁺: 357.0.

### Step 3: Preparation of benzyl (S)-(2-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)ethyl)(methyl)carbamate (A1.7-D)

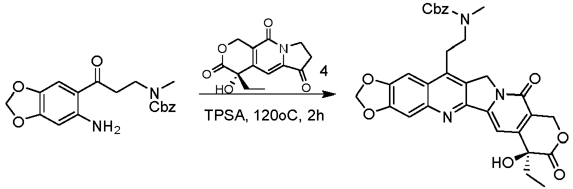

Compound A1.7-C benzyl (3-(6-aminobenzo[*d*][1,3]dioxol-5-yl)-3-oxopropyl)(methyl) carbamate (38 mg), compound (*S*)-4-ethyl-4-hydroxy-7,8-dihydro-1*H*-pyrano[3,4-*f*]indolizine-3,6,10(4*H*)-trione (29 mg), and *p-*toluenesulfonic acid (23 mg) were dissolved in a dichloromethane solution (5 mL) at room temperature. The resulting solution was clarified and mixed well, and then concentrated under reduced pressure. A vacuum was created using an oil pump, and the reaction solution was allowed to react under vacuum at 120 °C for 2 h. The reaction solution was cooled to room temperature, and water (30 mL) was added. The mixture was extracted with dichloromethane (30 mL × 3). The organic phases were combined, dried over anhydrous sodium sulfate, and filtered sequentially, and the filtrate was concentrated under reduced pressure to obtain the target compound A1.7-D (50 mg).

LCMS (ESI) [M+H]⁺ = 584.0.

### Step 4: Preparation of (S)-7-ethyl-7-hydroxy-14-(2-(methylamino)ethyl)-10,13-dihydro-11H-[1,3]dioxolo[4,5-g]pyrano[3',4':6,7]indolizino[1,2-b]quinoline-8,11(7H)-dione (A1.7-E)

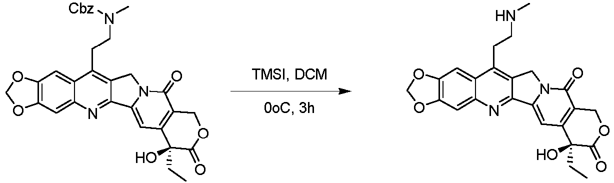

Compound A1.7-D benzyl (*S*)-(2-(7-ethyl-7-hydroxy-8,11-dioxo-7,8,11,13-tetrahydro-10*H-*[1,3]dioxolo[4,5-*g*]pyrano[3',4':6,7]indolizino[1,2-*b*]quinolin-14-yl)ethyl)(methyl)carbamate (50 mg) was dissolved in a dichloromethane solution (5 mL) at room temperature, and trimethylsilyl iodide (51 mg, 0.26 mmol) was added at 0 °C. The mixture was allowed to react for 3 h. The reaction solution was concentrated to remove the solvent, and the residue was purified by preparative high-performance liquid chromatography (acetonitrile/water containing 0.05% formic acid) to obtain the compound A1.7-E (15 mg) as a brown solid.

LCMS (ESI) [M+H]⁺: 450.0.

### Step 5: Preparation of compound (S)-2-((2-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)ethyl)(methyl)amino)-2-oxoethyl acetate (A1.7-F)

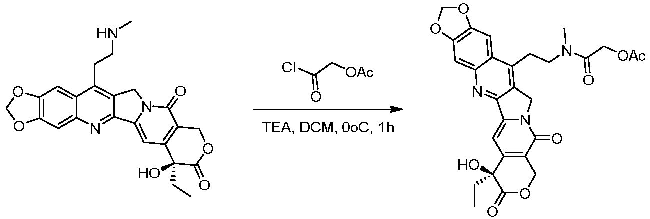

Compound A1.7-E (*S*)-7-ethyl-7-hydroxy-14-(2-(methylamino)ethyl)-10,13-dihydro-11*H-*[1,3]dioxolo[4,5-g]pyrano[3',4':6,7]indolizino[1,2-b]quinoline-8,11(7*H*)-dione (15 mg) and triethylamine (15 mg) were dissolved in a dichloromethane solution (5 mL), and acetoxyacetyl chloride (20 mg) was added at 0 °C. The mixture was allowed to react for 1 h. The reaction solution was concentrated to remove the solvent to obtain a crude product, and the resulting solid was used directly in the next step without further purification.

LCMS (ESI) [M5+H]⁺ = 550.1.

### Step 6: Preparation of compound (S)-N-(2-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)ethyl)-2-hydroxy-N-methylacetamide (A1.7)

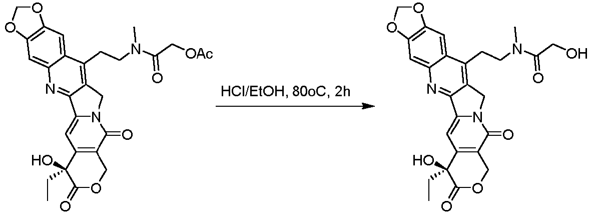

Compound A1.7-F (S)-2-((2-(7-ethyl-7-hydroxy-8,11-dioxo-7,8,11,13-tetrahydro-10*H*-[1,3]dioxolo[4,5-g]pyrano[3',4':6,7]indolizino[1,2-*b*]quinolin-14-yl)ethyl)(methyl)amino)-2-oxoethyl acetate (15 mg) was dissolved in an ethanol solution (5 mL), and concentrated hydrochloric acid (1.5 mL) was added. The mixture was allowed to react at 80 °C for 2 h. The reaction solution was concentrated to remove the solvent, and the residue was purified by preparative high-performance liquid chromatography (acetonitrile/water containing 0.05% formic acid) to obtain compound A1.7 (1.6 mg) as a white solid.

LCMS (ESI) [M+H]⁺: 508.2.

### Example A1.8: Synthesis of (S)-N-isopropyl-N-(2-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)ethyl)-2-hydroxyacetamide (A1.8)

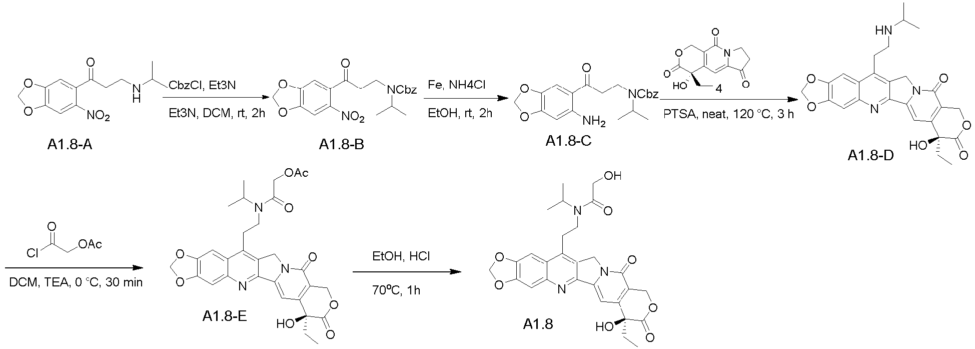

### Step 1: Preparation of compound benzyl isopropyl(3-(6-nitrobenzo[d][1,3]dioxol-5-yl)-3-oxopropyl)carbamate (A1.8-B)

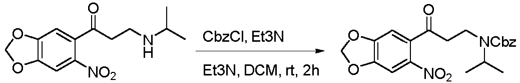

To a solution of compound (A1.8-A) 3-(isopropylamino)-1-(6-nitrobenzo[d][1,3]dioxol-5-yl)propan-1-one (900 mg) in dichloromethane (10 mL) were added sequentially triethylamine (1.8 g) and benzyloxycarbonyl chloride (734 mg, 4.3 mmol), and the mixture was allowed to react at room temperature for 2 h. The reaction solution was diluted with water (50 mL) and extracted with dichloromethane (50 mL × 3). The organic phase was washed with saturated brine, dried over anhydrous sodium sulfate, and concentrated to obtain a crude product. The crude product was separated and purified by column chromatography (petroleum ether:ethyl acetate = 3/1) to obtain the target compound (A1.8-B) (600 mg).

LCMS (ESI) [M+H]⁺: 414.9;

¹H NMR (400 MHz, DMSO-d6) δ 7.72 (s, 1H), 7.34 (br s, 6H), 6.31 (s, 2H), 5.08 (s, 2H), 4.14 - 4.10 (m, 1H), 3.48 (d, *J* = 7.9 Hz, 2H), 3.03 (s, 2H), 1.13 - 1.11 (m, 6H).

### Step 2: Preparation of compound benzyl (3-(6-aminobenzo[d][1,3]dioxol-5-yl)-3-oxopropyl)(isopropyl)carbatnate (A1.8-C)

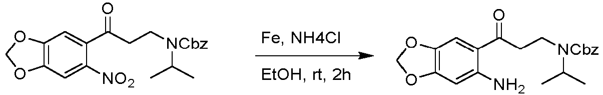

Compound (A1.8-B) benzyl isopropyl(3-(6-nitrobenzo[*d*][1,3]dioxol-5-yl)-3-oxopropyl)carbamate (200 mg, 0.48 mmol) was dissolved in a mixed solution of saturated ammonium chloride (3 mL) and ethanol (3 mL), and then iron powder (135 mg) was added to the reaction solution. The reaction mixture was stirred at room temperature for 2 h. The reaction solution was filtered, the filtrate was concentrated under reduced pressure to obtain a solid, and the crude product was separated and purified by column chromatography (petroleum ether:ethyl acetate = 3/1) to obtain the target compound (A1.8-C) (65 mg).

LCMS (ESI) [M+H]⁺: 395.2.

### Step 3: Preparation of (S)-7-ethyl-7-hydroxy-14-(2-(isopropylamino)ethyl)-10,13-dihydro-11H-[1,3]dioxolo[4,5-g]pyrano[3',4':6,7]indolizino[1,2-b]quinoline-8,11(7H)-dione (A1.8-D)

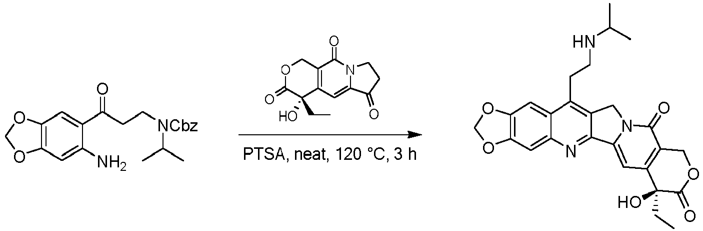

Compound (A1.8-C) benzyl (3-(6-aminobenzo[*d*][1,3]dioxol-5-yl)-3-oxopropyl)(isopropyl)carbamate (65 mg), compound (*S*)-4-ethyl-4-hydroxy-7,8-dihydro-1*H*-pyrano[3,4-*f*]indolizine-3,6,1-(4*H*)-trione (45 mg), and *p*-toluenesulfonic acid (33 mg, 0.17 mmol) were dissolved in a dichloromethane solution (10 mL) at room temperature. The resulting solution was clarified and mixed well, and then concentrated under reduced pressure. A vacuum was created using an oil pump, and the reaction solution was allowed to react under vacuum at 120 °C for 2 h. The reaction solution was cooled to room temperature, and water (50 mL) was added. The mixture was extracted with dichloromethane (30 mL × 3). The organic phases were combined, dried over anhydrous sodium sulfate, and filtered sequentially, and the filtrate was concentrated under reduced pressure. The crude product was separated and purified by column chromatography (dichloromethane/methanol = 10/1) to obtain the target compound (A1.8-D) (40 mg).
LCMS (ESI) [M+H]⁺: 478.0;
¹H NMR (400 MHz, DMSO-d6) δ 8.46 (s, 1H), 7.67 (s, 1H), 7.57 (s, 1H), 7.27 (s, 1H), 6.52 (s, 1H), 6.33 (s, 2H), 5.44 (s, 2H), 5.35 (s, 2H), 3.41 (br s, 2H), 3.23 (br s, 3H), 1.94 - 1.78 (m, 2H), 1.25 (d, *J* = 6.4 Hz, 6H), 0.88 (t, *J* = 7.3 Hz, 3H).

### Step 4: Preparation of compound (S)-2-((2-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)ethyl)(isopropyl)amino)-2-oxoethyl acetate (A1.8-E)

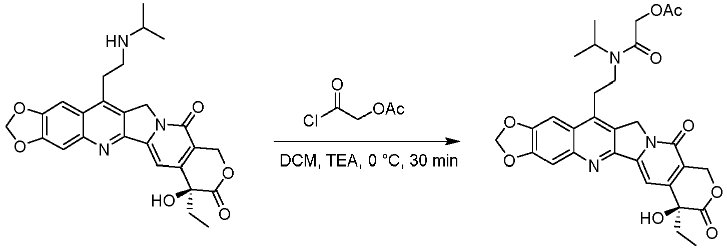

To a solution of compound (S)-7-ethyl-7-hydroxy-14-(2-(isopropylamino)ethyl)-10,13-dihydro-11H-[1,3]dioxolo[4,5-g]pyrano[3',4':6,7]indolizino[1,2-b]quinoline-8,11(7H)-dione (A1.8-D) (40 mg) in dichloromethane (2 mL) were added triethylamine (35 mg) and 2-chloro-2-oxoethyl acetate (57 mg) under an ice bath, and the mixture was allowed to react at 0 °C for 30 min. The reaction solution was concentrated under reduced pressure to obtain compound A1.8-E, which was used directly in the next step.

LCMS (ESI) [M+H]⁺: 578.0.

### Step 5: Preparation of compound (S)-N-(2-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)ethyl)-2-hydroxy-N-isopropylacetamide (A1.8)

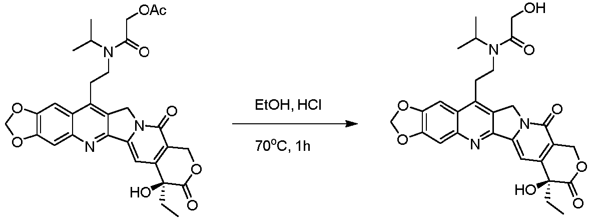

To a solution of compound (*S*)-2-((2-(7-ethyl-7-hydroxy-8,11-dioxo-7,8,11,13-tetrahydro-10*H-*[1,3]dioxolo[4,5-g]pyrano[3',4':6,7]indolizino[1,2-b]quinolin-14-yl)ethyl)(isopropyl)amino)-2-oxoethyl acetate (A1.8-E, 50 mg) in ethanol (3 mL) was added concentrated hydrochloric acid (0.5 mL), and the mixture was allowed to react at 70 °C for 1 h. The reaction solution was concentrated to obtain a crude product. The crude product was then purified by preparative high-performance liquid chromatography (acetonitrile/0.05% formic acid in water) to obtain the target compound (A1.8, 3 mg).
LCMS (ESI) [M+H]⁺: 464.0;
¹H NMR (400 MHz, DMSO-d6) δ 7.99 (s, 1H), 7.53 (s, 1H), 7.26 (s, 1H), 6.31 (s, 2H), 5.43 (s, 2H), 5.36 (s, 2H), 4.22 (s, 2H), 3.99 - 3.94 (m, 1H), 3.44 (dd, *J* = 16.7, 7.8 Hz, 2H), 3.33 - 3.21 (m, 2H), 1.95 - 1.79 (m, 2H), 1.19 (dd, *J =* 16.4, 5.8 Hz, 6H), 0.88 (t, *J =* 7.2 Hz, 3H).

### Example A1.9: Synthesis of (S)-7-ethyl-7-hydroxy-14-(3-hydroxypropyl)-10,13-dihydro-11H-[1,3]dioxolo[4,5-g]pyrano[3',4':6,7]indolizino[1,2-b]quinoline-8,11(7H)-dione (A1.9)

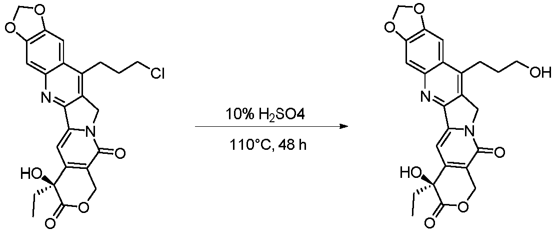

Compound (*S*)-7-ethyl-7-hydroxy-14-(3-chloropropyl)-10,13-dihydro-11*H-*[1,3]dioxolo[4,5-g]pyrano[3',4':6,7]indolizino[1,2-*b*]quinoline-8,11(7*H*)-dione (100 mg, 0.213 mmol) was dissolved in a 10% sulfuric acid solution (5 mL), and the reaction solution was allowed to react at 110 °C for 48 h. A saturated sodium bicarbonate solution (30 mL) was added to the reaction solution, and the mixture was extracted with dichloromethane (10 mL × 5), dried over anhydrous sodium sulfate, filtered under vacuum, and concentrated under reduced pressure to obtain a crude product. The crude product was purified by preparative high-performance liquid chromatography (acetonitrile/water containing 0.05% formic acid) to obtain (*S*)-7-ethyl-7-hydroxy-14-(3-hydroxypropyl)-10,13-dihydro-11*H*-[1,3]dioxolo[4,5-*g*]pyrano[3',4':6,7]indolizino[1,2-*b*]quinoline-8,11(7*H*)-dione (A1.9, 1.78 mg).
LCMS (ESI) [M+H]⁺: 451.0;
¹H NMR (400 MHz, DMSO-*d6*) δ 7.63 (s, 1H), 7.50 (s, 1H), 7.24 (s, 1H), 6.48 (s, 1H), 6.28 (s, 2H), 5.47 - 5.37 (m, 2H), 5.32 - 5.19 (m, 2H), 3.51 - 3.46 (m, 2H), 3.17 - 3.13 (m, 2H), 1.92 - 1.76 (m, 4H), 0.90 - 0.84 (m, 3H).

### Example A1.10: Synthesis of (S)-4-ethyl-8-fluoro-4-hydroxy-11-(3-hydroxypropyl)-9-methyl-1,12-dihydro-14H-pyrano[3',4':6,7]indolizino[1,2-b]quinoline-3,14(4H)-dione (A1.10)

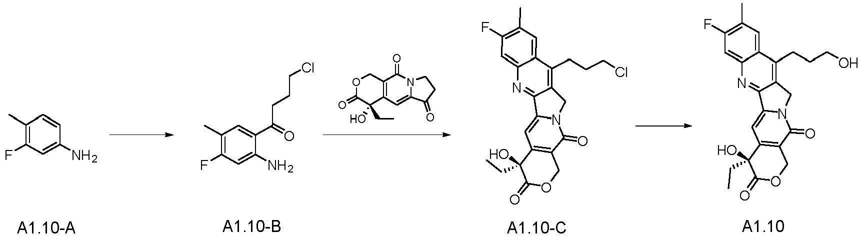

### Step 1:

To a solution of compound A1.10-A (10 g) in 1,2-dichloroethane (200 mL) were added dropwise 1 mol/L boron trichloride (96 mL) and 4-chlorobutyronitrile (9.9 g) at 0 °C. The mixture was stirred at 80 °C for 2 h. The reaction solution was cooled to room temperature, and 2 mol/L hydrochloric acid (90 mL) was added. The mixture was stirred at reflux at 80 °C for 0.5 h. The reaction solution was cooled to room temperature, diluted with a small amount of water, and extracted with dichloromethane (200 mL × 3). The organic phase was dried over anhydrous sodium sulfate, filtered under vacuum, and concentrated to obtain a crude product. The crude product was separated and purified by column chromatography (petroleum ether:ethyl acetate = 10/1) to obtain the target compound A1.10-B (4 g).

LCMS (ESI) [M+H]⁺: 230.0.

### Step 2:

(*S*)-4-Ethyl-4-hydroxy-7,8-dihydro-1*H*-pyrano[3,4-*f*]indolizine-3,6,10(4*H*)-trione (35 mg) and *p-*toluenesulfonic acid monohydrate (41.4 mg) were added to compound A1.10-B (50 mg), and the mixture was dissolved in a dichloromethane solution (30 mL). The resulting solution was clarified and mixed well, and then concentrated under reduced pressure. A vacuum was created using an oil pump, and the reaction solution was allowed to react under vacuum at 120 °C for 3 h. The reaction was completed as detected by LCMS. The reaction solution was cooled to room temperature, and water (20 mL) was added. The mixture was extracted with dichloromethane (20 mL × 3). The organic phases were combined, dried over anhydrous sodium sulfate, and filtered sequentially, and the filtrate was concentrated under reduced pressure to obtain a crude product. The crude product was separated and purified by column chromatography (dichloromethane/methanol = 20/1) to obtain the target compound A1.10-C (80 mg) as a white solid.

LCMS (ESI) [M+H]⁺: 457.0.

### Step 3:

Compound A1.10-C (75 mg) was dissolved in hexamethylphosphoric triamide, and purified water (0.8 mL) was added. The reaction solution was stirred at 100 °C for 72 h. The reaction was completed as detected by LCMS. The solution was purified by preparative chromatography (0.01% TFA in water, MeCN) to obtain the target compound (10 mg).
LCMS (ESI) [M+H]⁺: 439.2;
¹H NMR (400 MHz, DMSO-d6) δ 8.22 (d*, J =* 8.4 Hz, 1H), 7.87 (d, *J* = 10.9 Hz, 1H), 7.31 (s, 1H), 6.50 (s, 1H), 5.43 (s, 2H), 5.30 (s, 2H), 4.67 (t, *J* = 4.9 Hz, 1H), 3.55 - 3.47 (m, 2H), 3.28 - 3.20 (m, 2H), 2.51 (s, 3H), 1.93 - 1.81 (m, 4H), 0.88 (t, *J =* 7.3 Hz, 3H).

### Example A1.11: Synthesis of (S)-4-ethyl-8-fluoro-4-hydroxy-11-(3-aminopropyl)-9-methyl-1,12-dihydro-14H-pyrano[3',4':6,7]indolizino[1,2-b]quinoline-3,14(4H)-dione (A1.11)

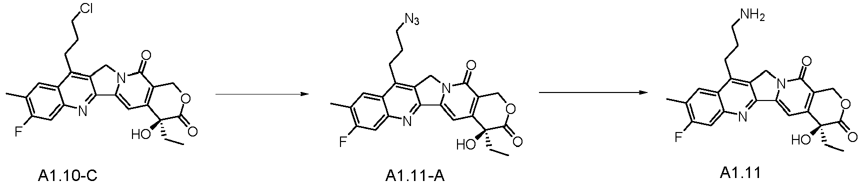

### Step 1:

To a solution of A1.10-C (395 mg) in *N,N*-dimethylformamide (10 mL) was added sodium azide (432 mg), and the reaction solution was allowed to react at 80 °C for 16 h, then cooled to room temperature, diluted with water (50 mL), and extracted with ethyl acetate (80 mL × 3). The organic phases were combined, dried over anhydrous sodium sulfate, filtered under vacuum, and concentrated to obtain the target product A1.11-A (290 mg).

LCMS (ESI) [M+H]⁺: 464.0.

### Step 2:

Compound A1.11-A (93 mg) was dissolved in tetrahydrofuran (5 mL), triphenylphosphine (78 mg) was added, and the mixture was allowed to react at room temperature for 4 h. Hydrochloric acid (4 M, 1 mL) was then added to the reaction solution, and the reaction solution was heated to 55 °C and allowed to react for 16 h. The reaction was completed as monitored by LCMS. The reaction solution was directly concentrated, and the crude product was purified by reversed-phase column chromatography (mobile phase A: 0.05% aqueous formic acid solution, mobile phase B: acetonitrile) to obtain the target product A1.11 (28 mg, yield: 36%) as a white solid.
LCMS (ESI) [M+H]⁺: 438.4;
¹H NMR (400 MHz, CD₃OD) δ 8.52 (s, 1H), 8.16 (d, *J* = 7.8 Hz, 1H), 7.76 (d, *J* = 10.7 Hz, 1H), 7.63 (s, 1H), 5.49 (ABq, *J* = 78.9, 16.3 Hz, 2H), 5.31 (br s, 2H), 3.35 - 3.32 (m, 2H), 3.22 - 3.10 (m, 2H), 2.55 (s, 3H), 2.13 - 2.11 (m, 2H), 1.96 - 1.94 (m, 2H), 1.01 (t, *J =* 7.4 Hz, 3H).

### Example A1.12: Synthesis of (S,E)-14-(3-amino-1-propen-1-yl)-7-ethyl-7-hydroxy-10,13-dihydro-11H-[1,3]dioxolo[4,5-g]pyrano[3',4':6,7]indolizino[1,2-b]quinoline-8,11(7H)-dione (A1.12)

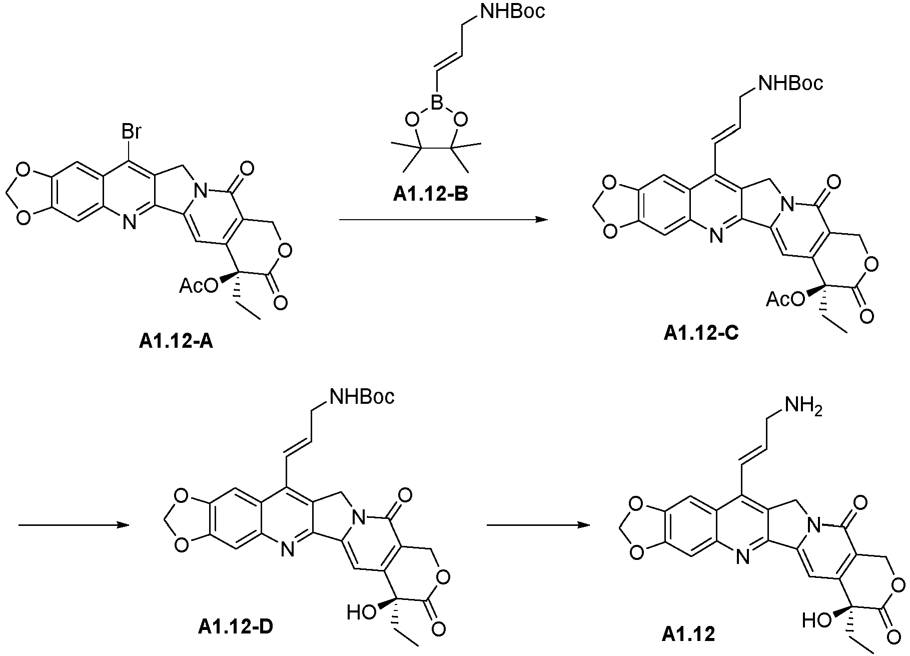

### Step 1:

Compound A1.12-A (200 mg, 0.39 mmol), compound A1.12-B (112 mg, 0.39 mmol), cesium fluoride (152 mg, 0.975 mmol), and tetrakis(triphenylphosphine)palladium (45 mg, 0.039 mmol) were added to a 1,4-dioxane solution (8 mL). The mixture was allowed to react at 120 °C for 0.5 h under a nitrogen atmosphere under microwave irradiation. The reaction was completed as detected by LCMS. The reaction solution was diluted with a mixed solution of dichloromethane (20 mL) and methanol (10 mL), and filtered. The filtrate was concentrated, and the crude product was purified by preparative TLC (dichloromethane:methanol = 30:1) to obtain the target compound (*S,E*)-14-(3-((*tert*-butoxycarbonyl)amino)-1-propen-1-yl)-7-ethyl-8,11-dioxo-7,8,11,13-tetrahydro-10*H*-[1,3]dioxolo[4,5-*g*]pyrano[3',4':6,7]indolizino[1,2-b]quinolin-7-yl acetate (A1.12-C, 60 mg, yield: 26%) as a brown solid. LCMS (ESI) [M+H]⁺ = 590.3;

¹H NMR (400 MHz, DMSO-d6) δ 7.63 (s, 1H), 7.49 (s, 1H), 7.35 (s, 1H), 7.09 (d, *J* = 16.7 Hz, 1H), 6.93 (s, 1H), 6.45 (d, *J* = 16.6 Hz, 1H), 6.30 (s, 2H), 5.47 (s, 2H), 5.34 - 5.24 (m, 2H), 3.94 (s, 2H), 2.21 (br s, 3H), 2.03 - 1.96 (m, 2H), 1.45 (s, 9H), 0.91 (t, *J* = 6.7 Hz, 3H).

### Step 2:

To a solution of compound (*S,E*)-14-(3-((*tert*-butoxycarbonyl)amino)-1-propen-1-yl)-7-ethyl-8,11-dioxo-7,8,11,13-tetrahydro-10*H*-[1,3]dioxolo[4,5-g]pyrano[3',4':6,7]indolizino[1,2-*b*]quinolin-7-yl acetate (A1.12-C, 50 mg, 0.085 mmol) in methanol (15 mL) was added sodium methoxide (9.2 mg, 0.17 mmol), and the mixture was stirred at 50 °C for 2 h. The reaction was completed as detected by LCMS. The reaction solution was concentrated to obtain a crude product of the target compound *tert-*butyl (*S,E*)-(3-(7-ethyl-7-hydroxy-8,11-dioxo-7,8,11,13-tetrahydro-10*H*-[1,3]dioxolo[4,5-*g*]pyrano[3',4':6,7]indolizino[1,2-*b*]quinolin-14-yl)allyl)carbamate (A1.12-D, 50 mg) as a brown solid. LCMS (ESI) [M+H]⁺ = 548;

### Step 3:

To a solution of compound *tert-*butyl (*S,E*)-(3-(7-ethyl-7-hydroxy-8,11-dioxo-7,8,11,13-tetrahydro-10*H-*[1,3]dioxolo[4,5-g]pyrano[3',4':6,7]indolizino[1,2-*b*]quinolin-14-yl)allyl)carbamate (A1.12-D, 50 mg, 0.091 mmol) in dichloromethane (2 mL) was added trifluoroacetic acid (1 mL), and the mixture was stirred at room temperature for 30 min. The reaction was completed as detected by LCMS. The reaction solution was concentrated, and the crude product was purified by preparative high-performance liquid chromatography (acetonitrile/0.05% formic acid in water) to obtain the target compound (S,E)-14-(3-aminoprop-1-en-1-yl)-7-ethyl-7-hydroxy-10,13-dihydro-11H-[1,3]dioxolo[4,5-g]pyrano[3',4':6,7]indolizino[1,2-b]quinoline-8,11(7H)-dione (A1.12) (8.1 mg, yield: 21%) as a brown solid.
LCMS (ESI) [M+H]⁺ = 448.3;
¹H NMR (400 MHz, DMSO-d6) δ 8.20 (s, 2H), 7.72 (s, 1H), 7.54 (s, 1H), 7.40 (d, *J* = 16.5 Hz, 1H), 7.27 (s, 1H), 6.52 - 6.46 (m, 2H), 6.31 (s, 2H), 5.42 (s, 2H), 5.27 (s, 2H), 3.86 (br s, 2H), 1.90 - 1.83 (m, 2H), 0.88 (t, *J* = 7.1 Hz, 3H).

### Example A1.13: Synthesis of (S,E)-14-(3-hydroxy-1-propen-1-yl)-7-ethyl-7-hydroxy-10,13-dihydro-11H-[1,3]dioxolo[4,5-g]pyrano[3',4':6,7]indolizino[1,2-b]quinoline-8,11(7H)-dione (A1.13)

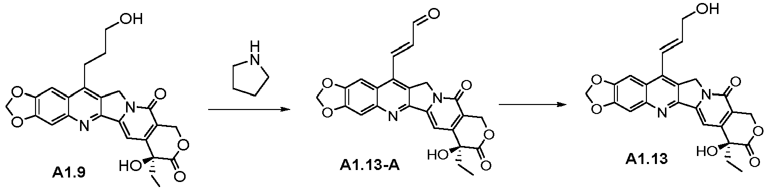

### Step 1:

Compound A1.9 (200 mg, 0.444 mmol) was dissolved in dimethyl sulfoxide (2 mL), and IBX (311 mg, 1.11 mmol) was added. The reaction solution was stirred at room temperature for 2 h and then supplemented with IBX (186 mg, 0.666 mmol). Then, tetrahydropyrrole (6.3 mg, 0.089 mmol) and acetonitrile (3 mL) were added to the reaction solution, and the reaction solution was stirred at room temperature overnight. The reaction was completed as detected by LCMS. The solution was extracted with ethyl acetate (20 mL × 3). The organic phase was washed with saturated brine, dried over anhydrous sodium sulfate, filtered under vacuum, and concentrated to dryness by rotary evaporation to obtain a crude product. The crude product was purified by silica gel column chromatography (DCM:MeOH = 50:1 to 10:1) to obtain the target compound (S,E)-14-(3-oxo-1-propen-1-yl)-7-ethyl-7-hydroxy-10,13-dihydro-11*H*-[1,3]dioxolo[4,5-*g*]pyrano[3',4':6,7]indolizino[1,2-*b*]quinoline-8,11(7*H*)-dione (A1.13-A, 100 mg, purity: 50.0%, yield: 25.0%) as a yellow solid. LCMS (ESI) [M+H]⁺ = 447.1.

### Step 2:

Compound A1.13-A (40 mg, 0.09 mmol) was dissolved in tetrahydrofuran (1 mL), and then sodium cyanoborohydride (28 mg, 0.448 mmol) was added to the reaction solution. The mixture was stirred at room temperature overnight. The reaction was completed as detected by LCMS. The reaction solution was concentrated to obtain a crude product. The crude product was purified by prep-HPLC (HCl in water/MeCN) to obtain the target compound (3.56 mg, purity: 93.6%, yield: 9.0%) as a pale yellow solid.
LCMS (ESI) [M+H]⁺ = 449.2;
1H NMR (400 MHz, DMSO-d6) δ 7.61 (s, 1H), 7.50 (s, 1H), 7.24 (s, 1H), 7.20 (d, *J* = 16.4 Hz, 1H), 6.69 - 6.59 (m, 1H), 6.49 (s, 1H), 6.30 (s, 2H), 5.42 (s, 2H), 5.26 (s, 2H), 5.16 (br s, 1H), 4.34 (br s, 2H), 1.93 - 1.81 (m, 2H), 0.88 (t, *J =* 7.3 Hz, 3H).

### Example A1.14: Synthesis of (S,E)-4-ethyl-8-fluoro-4-hydroxy-11-(3-hydroxy-1-propen-1-yl)-9-methyl-1,12-dihydro-14H-pyrano[3',4':6,7]indolizino[1,2-b]quinoline-3,14(4H)-dione (A1.14)

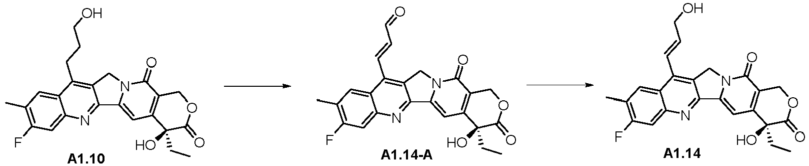

### Step 1:

Compound (*S*)-4-ethyl-8-fluoro-4-hydroxy-11-(3-hydroxypropyl)-9-methyl-1,12-dihydro-14*H-*pyrano[3',4':6,7]indolizino[1,2-*b*]quinoline-3,14(4*H*)-dione (A1.10, 100 mg, 0.228 mmol), DMSO (0.5 mL), and acetonitrile (0.75 mL) were added to a 25 mL single-necked flask, and IBX (160 mg, 0.571 mmol) and (*R*)-diphenyl(pyrrolidin-2-yl)methanol (12 mg, 0.047 mmol) were added sequentially. The mixture was stirred at room temperature for 16 h. Then, a mixed solvent of methanol and dichloromethane (200 mL) (DCM:MeOH = 10:1) was added to the reaction solution, and the resulting mixture was extracted with saturated brine (300 mL). The organic phases were combined, washed with water, dried, and filtered. The filtrate was evaporated to dryness under reduced pressure to remove the solvent to obtain a crude product. The crude product was purified by Flash chromatography (DCM:MeOH = 10:1) to obtain the target compound (*S,E*)-3-(4-ethyl-8-fluoro-4-hydroxy-9-methyl-3,14-dioxo-3,4,12,14-tetrahydro-1*H*-pyrano[3',4':6,7]indolizino[1,2-*b*]quinolin-11-yl)acrylaldehyde (A1.14-A, 40 mg, yield: 40%).

LCMS (ESI) [M+H]⁺ = 435.1.

### Step 2:

Compound (*S,E*)-3-(4-ethyl-8-fluoro-4-hydroxy-9-methyl-3,14-dioxo-3,4,12,14-tetrahydro-1*H-*pyrano[3',4':6,7]indolizino[1,2-*b*]quinolin-11-yl)acrylaldehyde (A1.14-A, 40 mg, 0.228 mmol) and anhydrous THF (5 mL) were added to a 50 mL three-necked flask, and the mixture was cooled to -78 °C under an N₂ atmosphere. A solution of lithium tri-sec-butylborohydride in THF (0.11 mL, 1 N) was added dropwise and slowly, and the mixture was stirred for another 1 h with the temperature maintained at -78 °C. After the reaction was completed, a saturated aqueous ammonium chloride solution was added to quench the reaction. The resulting mixture was warmed to room temperature, diluted with saturated brine (50 mL), and extracted with ethyl acetate (20 mL × 3). The organic phases were combined, washed with water, dried, and filtered. The filtrate was evaporated to dryness under reduced pressure to remove the solvent to obtain (*S,E*)-4-ethyl-8-fluoro-4-hydroxy-11-(3-hydroxy-1-propen-1-yl)-9-methyl-1,12-dihydro-14*H*-pyrano[3',4':6,7]indolizino[1,2-b]quinoline-3,14(4*H*)-dione (A1.14, 28 mg) as an off-white solid.

LCMS (ESI) [M+H]⁺ = 437.1.

### Example A1.15: Synthesis of (S)-7-ethyl-7-hydroxy-14-(2-(n-propylamino)ethyl)-10,13-dihydro-11H-[1,3]dioxolo[4,5-g]pyrano[3',4':6,7]indolizino[1,2-b]quinoline-8,11(7H)-dione (A1.15a) and (S)-N-(2-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)ethyl)-2-hydroxy-N-propylacetamide (A1.15b)

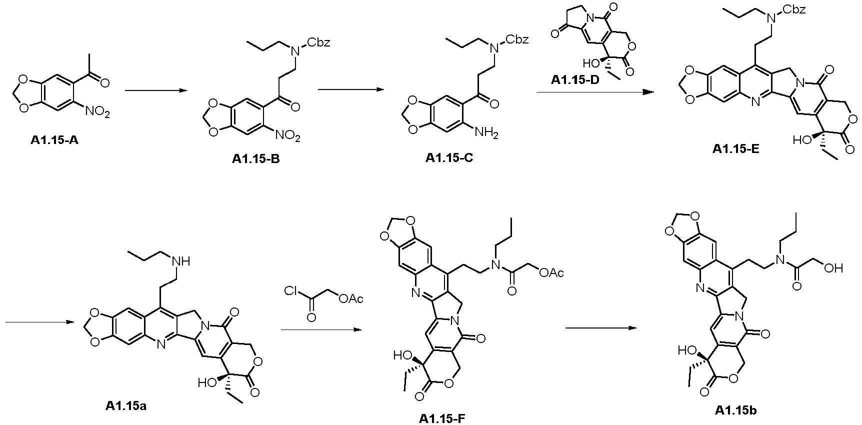

### Step 1:

Compound A1.15-A (5.0 g, 23.9 mmol), propylamine hydrochloride (22.8 g, 239 mmol), and paraformaldehyde (7.18 g, 239 mmol) were dissolved in ethanol (100 mL), and the resulting solution was allowed to react at 110 °C for 12 h in a closed container. The reaction was completed as detected by LCMS. The reaction solution was cooled to room temperature and concentrated under reduced pressure. Dichloromethane (100 mL) was added to the reaction solid, and the mixture was washed with water (80 mL × 3). The aqueous phase was adjusted to pH = 9 with sodium bicarbonate. Benzyloxycarbonyl chloride (3.7 g, 21.8 mmol) was added to the aqueous solution, and the mixture was allowed to react at room temperature for 2 h. The reaction was completed as detected by LCMS. The mixture was extracted with dichloromethane (100 mL × 3). The organic phase was washed with saturated brine, dried over anhydrous sodium sulfate, filtered under vacuum, and concentrated to obtain a crude product. The crude product was separated and purified by column chromatography (petroleum ether:ethyl acetate = 10/1) to obtain the target compound A1.15-B (1.5 g, yield: 20.1%) as a yellow oily liquid.
LCMS (ESI) [M+H]⁺ = 415.1;
1H NMR (400 MHz, CDCl₃) δ 7.56 (s, 1H), 7.35 (m, 6H), 6.18 (s, 2H), 5.17 - 5.12 (m, 2H), 3.69 - 3.67 (m, 2H), 3.32 - 3.30 (m, 2H), 3.09 - 2.90 (m, 2H), 1.59 (m, 2H), 0.90 (m, 3H).

### Step 2:

Compound A1.15-B (1.5 g, 3.63 mmol) was dissolved in a mixed solution of saturated ammonium chloride (20 mL) and ethanol (20 mL), and then iron powder (1.02 g, 18.1 mmol) was added to the reaction solution. The reaction mixture was stirred at 80 °C for 1 h. The reaction was completed as detected by LCMS. The reaction solution was filtered, and the filtrate was concentrated under reduced pressure to obtain a solid. The crude product was separated and purified by reversed-phase chromatography using a C18 column (acetonitrile/0.05% FAin water: 5% to 55%) to obtain the target compound A1.15-C (600 mg, yield: 43.0%) as a yellow solid.

LCMS (ESI) [M+H]⁺ = 385.2, t_{R} = 1.329 min.

### Step 3:

Compound A1.15-C (350 mg, 0.91 mmol), compound (*S*)-4-ethyl-4-hydroxy-7,8-dihydro-1*H*-pyrano[3,4-*f*]indolizine-3,6,10(4*H*)-trione (A1.15-D, 218 mg, 0.83 mmol), and *p*-toluenesulfonic acid (170 mg, 0.87 mmol) were dissolved in a dichloromethane solution (5 mL) at room temperature. The resulting solution was clarified and mixed well, and then concentrated under reduced pressure. A vacuum was created using an oil pump, and the reaction solution was allowed to react under vacuum at 120 °C for 2 h. The reaction was completed as detected by LCMS. The reaction solution was cooled to room temperature, and water (50 mL) was added. The mixture was extracted with dichloromethane (30 mL × 3). The organic phases were combined, dried over anhydrous sodium sulfate, and filtered sequentially, and the filtrate was concentrated under reduced pressure to obtain compound A1.15-E (390 mg, yield: 76%) as a brown solid.

LCMS (ESI) [M+H]⁺ = 612.0.

### Step 4:

Compound A1.15-E (390 mg, 0.638 mmol) was dissolved in a dichloromethane solution (5 mL) at room temperature, and trimethylsilyl iodide (510 mg, 2.55 mmol) was added dropwise at 0 °C under a nitrogen atmosphere. The reaction system was stirred at room temperature for 2 h. Diethyl ether (2 mL) and concentrated hydrochloric acid (4 mL) were added to the reaction solution, and the mixture was stirred for 30 min. The reaction system was adjusted to pH = 9 with saturated sodium bicarbonate, and extracted with dichloromethane (50 mL × 5). The organic phases were combined, dried over anhydrous sodium sulfate, and filtered, and the filtrate was concentrated under reduced pressure to obtain a crude product. The crude product was purified by preparative high-performance liquid chromatography (acetonitrile/water containing 0.05% formic acid) to obtain compound A1.15a (200 mg, yield: 66.1%) as a creamy-white solid.

LCMS (ESI) [M+H]⁺ = 478.2;

1H NMR (400 MHz, DMSO-d6) δ 8.30 (s, 1H, HCO₂H), 7.67 (s, 1H), 7.51 (s, 1H), 7.23 (s, 1H), 6.62 - 6.41 (m, 1H), 6.29 (s, 2H), 5.42 (s, 2H), 5.28 (s, 2H), 3.27 - 3.25 (m, 2H), 2.91 - 2.79 (m, 2H), 2.58 - 2.56 (m, 2H), 1.91 - 1.79 (m, 2H), 1.44 - 1.42 (m, 2H), 0.90 - 0.84 (m, 6H).

### Step 5:

Compound A1.15a (120 mg, 0.251 mmol) was dissolved in dichloromethane (5 mL) at room temperature, and compounds acetoxyacetyl chloride (167 mg, 1.25 mmol) and triethylamine (133 mg, 1.25 mmol) were added dropwise at 0 °C. The reaction mixture was stirred at 0 °C for 30 min. The reaction was completed as detected by LCMS. The reaction solution was concentrated under reduced pressure to obtain a crude product of compound A1.15-F (120 mg, yield: 82.8%) as a yellow solid. LCMS (ESI) [M+H]⁺ = 578.3.

### Step 6:

Compound A1.15-F (120 mg, 0.207 mmol) was dissolved in ethanol (4 mL) at room temperature, and then concentrated hydrochloric acid (2 mL) was added to the reaction solution. The reaction mixture was stirred at 70 °C for 1 h. The reaction was completed as detected by LCMS. Water (20 mL) was added to the reaction solution, and the mixture was extracted with dichloromethane (30 mL × 5). The organic phases were combined, dried over anhydrous sodium sulfate, and filtered sequentially, and the filtrate was concentrated under reduced pressure to obtain a crude product. The crude product was purified by preparative high-performance liquid chromatography (acetonitrile/water containing 0.05% formic acid) to obtain compound A1.15b (20 mg, yield: 18.1%) as a creamy-white solid.
LCMS (ESI) [M+H]⁺ = 536.2;
1H NMR (400 MHz, DMSO-d6) δ 7.93 (s, 0.7H), 7.69 (s, 0.3H), 7.51 (s, 1H), 7.24 (s, 1H), 6.49 (s, 1H), 6.30 (s, 2H), 5.42 (s, 2H), 5.35 (s, 1.5H), 5.29 (s, 0.5H), 4.65 - 4.62 (m, 1H), 4.13 - 3.97 (m, 2H), 3.51 - 3.49 (m, 2H), 3.33 - 3.30 (m, 2H), 3.24 - 3.20 (m, 2H), 1.86 - 1.84 (m, 2H), 1.60 - 1.52 (m, 2H), 0.89 - 0.86 (m, 6H).

### Example A1.16: Synthesis of (S)-4-ethyl-8-fluoro-4-hydroxy-11-(3-isopropylaminopropyl)-9-methyl-1,12-dihydro-14H-pyrano[3',4':6,7]indolizino[1,2-b]quinoline-3,14(4H)-dione (A1.16)

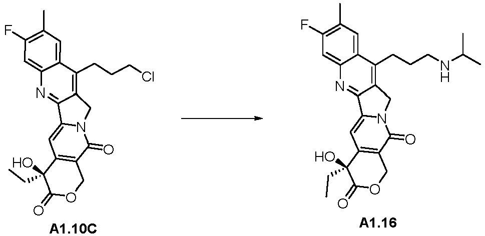

To a solution of A1.10-C (200 mg, 0.439 mmol) and isopropylamine (50 mg, 0.877 mmol) in DMF (10 mL) were added diisopropylethylamine (170 mg, 1.32 mmol) and sodium iodide (99 mg, 0.659 mmol), and the reaction mixture was allowed to react at 50 °C for 2 h in a sealed tube. Ethyl acetate was added to the reaction solution, and the organic phase was washed with saturated brine, dried over anhydrous sodium sulfate, filtered under vacuum, and concentrated under reduced pressure to obtain a crude product. The crude product was separated and purified by pre-TLC (dichloromethane:methanol = 10:1) to obtain a yellow solid, which was further purified by preparative high-performance liquid chromatography (acetonitrile/water containing 0.05% formic acid) to obtain the target compound (*S*)-4-ethyl-8-fluoro-4-hydroxy-11-(3-isopropylaminopropyl)-9-methyl-1,12-dihydro-14*H-*pyrano[3',4':6,7]indolizino[1,2-*b*]quinoline-3,14(4*H*)-dione (A1.16, 2.03 mg).
LCMS (ESI) [M+H]⁺ = 480.2;
¹H NMR (400 MHz, DMSO-d6) δ 8.28 (d, *J* = 8.0 Hz, 1H), 8.27 (s, 1H, HCO2H), 7.92 (d, *J* = 11.0 Hz, 1H), 7.33 (s, 1H), 6.53 (s, 1H), 5.45 (s, 2H), 5.30 (s, 2H), 33.30 - 3.22 (m, 3H), 3.18 - 3.10 (m, 2H), 2.54 (s, 3H), 2.06 - 1.94 (m, 2H), 1.93 - 1.80 (m, 2H), 1.26 - 1.20 (m, 6H), 0.88 (t, *J =* 7.3 Hz, 3H).

### Example A1.17: Synthesis of (S)-4-ethyl-8-fluoro-4-hydroxy-11-(3-cyclopropylaminopropyl)-9-methyl-1,12-dihydro-14H-pyrano[3',4':6,7]indolizino[1,2-b]quinoline-3,14(4H)-dione (A1.17)

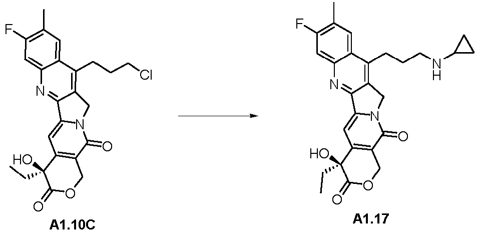

To a solution of A1.10C (200 mg, 0.439 mmol) and cyclopropylamine (52 mg, 0.881 mmol) in DMF (10 mL) were added diisopropylethylamine (165 mg, 1.28 mmol) and sodium iodide (96 mg, 0.640 mmol), and the reaction mixture was allowed to react at 50 °C for 2 h in a sealed tube. The reaction was completed as monitored by LCMS. Ethyl acetate was added to the reaction solution, and the organic phase was washed with saturated brine, dried over anhydrous sodium sulfate, filtered under vacuum, and concentrated under reduced pressure to obtain a crude product. The crude product was separated and purified by TLC (dichloromethane:methanol = 10:1), followed by further purification by preparative high-performance liquid chromatography (acetonitrile/water containing 0.05% formic acid) to obtain the target compound (*S*)-4-ethyl-8-fluoro-4-hydroxy-11-(3-cyclopropylaminopropyl)-9-methyl-1,12-dihydro-14*H*-pyrano[3',4':6,7]indolizino[1,2-*b*]quinoline-3,14(4*H*)-dione (A1.17, 5.12 mg).
LCMS (ESI) [M+H]⁺ = 478.2;
¹H NMR (400 MHz, DMSO-d6) δ 8.22 (br s, 2H, one is HCO2H), 7.86 (br s, 1H), 7.30 (s, 1H), 6.51 (s, 1H), 5.43 (s, 2H), 5.28 (s, 2H), 3.24 - 3.19 (m, 2H), 2.75 - 2.68 (m, 2H), 2.49 (s, 3H), 2.16 - 2.09 (m, 1H), 1.95 - 1.76 (m, 4H), 0.91 - 0.83(t, *J* = 7.3 Hz, 3H), 0.43 - 0.33 (m, 2H), 0.31 - 0.21 (m, 2H).

### Example A1.18: Synthesis of (S)-4-ethyl-8-fluoro-4-hydroxy-11-(4-aminophenyl)-9-methyl-1,12-dihydro-14H-pyrano[3',4':6,7]indolizino[1,2-b]quinoline-3,14(4H)-dione (A1.18)

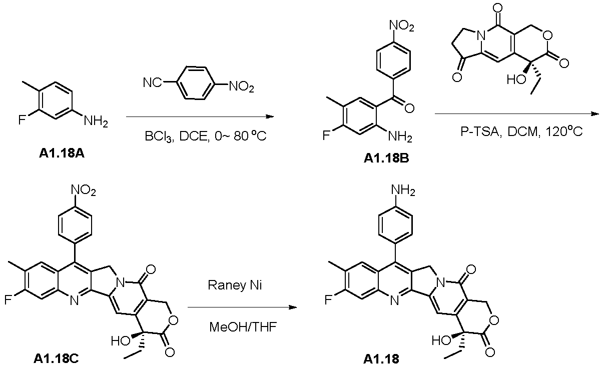

### Step 1:

3-Fluoro-4-methylaniline (A1.18A, 2.0 g, 16.3 mmol) was dissolved in 1,2-dichloroethane (40 mL) under a nitrogen atmosphere, and boron trichloride (19.2 mL, 19.2 mmol) was added dropwise at 0 °C. *p*-Nitrobenzonitrile (2.8 g, 19.2 mmol) was then added slowly, and after the addition, the reaction solution was heated to 80 °C and stirred overnight. The reaction solution was cooled to room temperature, and hydrochloric acid (40 mL, 2 M) was added. The mixture was then heated to 80 °C and stirred for another 30 min. After the reaction was completed as detected by LCMS., water (80 mL) was added to the reaction solution, and the mixture was extracted with dichloromethane (100 mL × 3). The organic phases were combined, dried over anhydrous sodium sulfate, filtered, and concentrated to dryness by rotary evaporation, and the crude product was purified by silica gel column chromatography (PE:EA = 10:1) to obtain the target compound (A1.18B, 2.0 g, yield: 46.5%).
LCMS (ESI) [M+H]⁺ = 275.1;
¹H NMR (400 MHz, DMSO) δ 8.34 (d, *J* = 8.6 Hz, 2H), 7.77 (d, *J* = 8.6 Hz, 2H), 7.39 (s, 2H), 7.11 (d, *J* = 8.8 Hz, 1H), 6.63 (d, *J* = 12.4 Hz, 1H), 2.00 (s, 3H).

### Step 2:

Compound A1.18B (620 mg, 2.28 mmol), (*S*)-4-ethyl-4-hydroxy-7,8-dihydro-1*H*-pyrano[3,4-f]indolizine-3,6,10(4*H*)-trione (600 mg, 2.28 mmol), and *p*-toluenesulfonic acid (560 mg, 2.96 mmol) were added to a reaction flask and dissolved well in dichloromethane. The resulting solution was then concentrated to dryness by rotary evaporation to remove the dichloromethane. A vacuum was created, and the residue was heated to 120 °C under vacuum and incubated at this temperature for six hours. After the reaction was completed as detected by LCMS, methanol (10 mL) was added to the system, and then water (80 mL) was added, which resulted in a large amount of precipitate. The precipitate was collected by filtration and then dried to obtain the target compound (A1.18C, 800 mg, yield: 80.0%) as a yellow solid.
LCMS (ESI) [M+H]⁺ = 502.2;
¹H NMR (400 MHz, DMSO) δ 8.52 (d, *J* = 8.2 Hz, 2H), 8.13 - 7.89 (m, 3H), 7.64 (d, *J* = 8.4 Hz, 1H), 7.37 (d, *J* = 13.4 Hz, 1H), 6.54 (s, 1H), 5.41 (s, 2H), 5.06 (m, 2H), 2.40 (s, 3H), 1.94 - 1.84 (m, 2H), 0.87 (m, 3H).

### Step 3:

Raney Ni (609 mg, 10.5 mmol) was added to a mixed solution of compound A1.18C (1.0 g, 2.1 mmol) in methanol (25 mL) and tetrahydrofuran (50 mL), and the reaction solution was stirred under a hydrogen atmosphere at room temperature for 5 h. The reaction was completed as detected by LCMS. The reaction solution was filtered, and the filtrate was concentrated to dryness by rotary evaporation to obtain the target compound (S)-4-ethyl-8-fluoro-4-hydroxy-11-(4-aminophenyl)-9-methyl-1,12-dihydro-14*H*-pyrano[3',4':6,7]indolizino[1,2-*b*]quinoline-3,14(4H)-dione (A1.18, 650 mg, yield: 67%) as a yellow solid.
LCMS (ESI) [M+H]⁺ = 472.3;
¹H NMR (400 MHz, DMSO) δ 7.93 - 7.81 (m, 2H), 7.40 - 7.25 (m, 3H), 6.81 (d, *J* = 8.2 Hz, 2H), 6.51 (s, 1H), 5.62 (s, 2H), 5.41 (s, 2H), 5.10 (s, 2H), 2.41 (s, 3H), 1.93 - 1.80 (m, 2H), 0.88 (t, *J* = 7.2 Hz, 3H).

### B. Synthesis of intermediates containing bioactive molecular fragments

### Example B1.1: Synthesis of (S)-2-amino-N-((4-(4-ethyl-8-fluoro-4-hydroxy-9-methyl-3,14-dioxo-3,4,12,14-tetrahydro-1H-pyrano[3',4':6,7]indolizino[1,2-b]quinolin-11-yl)butoxy)methyl)acetamide (B1.1)

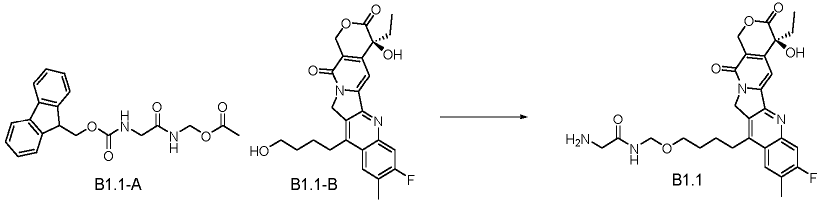

Compound (B1.1-A) (368 mg), compound (B1.1-B) (452 mg), and pyridinium *p*-toluenesulfonate (PPTS) (25 mg) were refluxed in dichloromethane (20 mL) for 20 h and then washed with an aqueous sodium bicarbonate solution and an aqueous hydrochloric acid solution sequentially. The mixture was concentrated under reduced pressure to remove the organic solvent, and the residue was dissolved in DMF (5 mL). Piperidine (1 mL) was added, and the compound mixture was stirred for 20 min and concentrated under reduced pressure to remove most of the low-boiling point components. The residue was separated by preparative HPLC to obtain the target product (*S*)-2-amino-*N*-((4-(4-ethyl-8-fluoro-4-hydroxy-9-methyl-3,14-dioxo-3,4,12,14-tetrahydro-1*H-*pyrano[3',4':6,7]indolizino[1,2-b]quinolin-11-yl)butoxy)methyl)acetamide (B1.1).

ESI-MS (m/z): 539 [M+H]⁺.

### Example B1.2: Synthesis of (S)-2-((2-aminoacetamido)methoxy)-N-ethyl-N-(2-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)ethyl)acetamide (B1.2)

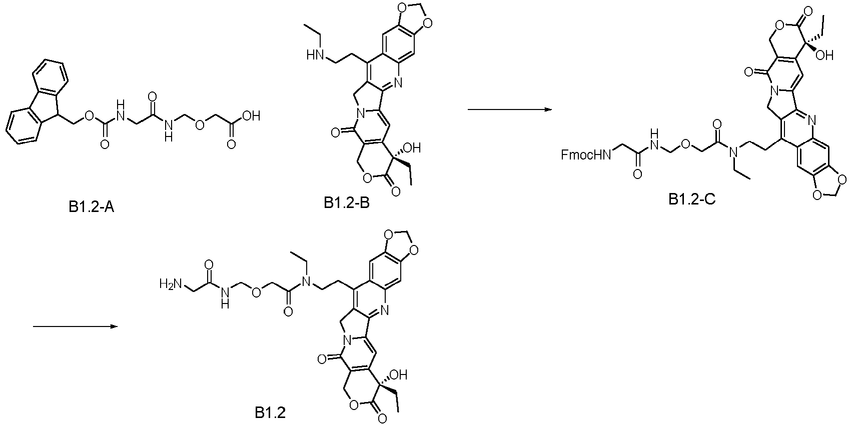

### Step 1:

Compound B1.2-A (274 mg), diisopropylethylamine (334 mg), and HBTU (369 mg) were added sequentially to *N,N*-dimethylformamide (10 mL), and then compound B1.2-B (300 mg) was added. The reaction solution was stirred at room temperature for 2 h. Ethyl acetate (50 mL) was added to the reaction solution, and the mixture was washed with saturated brine (30 mL × 3), dried over anhydrous sodium sulfate, and filtered under vacuum. The filtrate was concentrated under reduced pressure to obtain a solid, and the crude product was separated and purified by column chromatography (dichloromethane:methanol = 10/1) to obtain the target compound B1.2-C (300 mg).
LCMS (ESI) [M+H]⁺: 830.2;
¹H NMR (400 MHz, DMSO-d6) δ 8.74 (s, 1H), 7.91 - 7.84 (m, 2H), 7.80 - 7.67 (m, 2H), 7.65 - 7.54 (m, 2H), 7.53 - 7.46 (m, 1H), 7.45 - 7.37 (m, 2H), 7.36 - 7.27 (m, 2H), 7.25 - 7.23 (m, 1H), 6.57 - 6.44 (m, 1H), 6.29 (s, 2H), 5.50 - 5.19 (m, 4H), 4.71 - 4.57 (m, 2H), 4.32 - 3.97 (m, 7H), 3.80 - 3.53 (m, 4H), 3.20 - 3.14 (m, 2H), 1.92 - 1.80 (m, 2H), 1.28 - 1.22 (m, 3H), 0.87 - 0.82 (m, 3H).

### Step 2:

To a solution of compound B1.2-C (300 mg) in *N,N*-dimethylformamide (4 mL) was added piperidine (1 mL), and the reaction solution was stirred at room temperature for 20 min and concentrated under reduced pressure to remove low-boiling point components to obtain the target product, which was directly used in the next step of synthesis.

LCMS (ESI) [M+H]⁺ = 608.0.

### Example B1.3: Synthesis of (S)-2-amino-N-((2-(((4-ethyl-8-fluoro-4-hydroxy-9-methyl-3,14-dioxo-3,4,12,14-tetrahydro-1H-pyrano[3',4':6,7]indolizino[1,2-b]quinolin-11-yl)methyl)amino)-2-oxoethoxy)methyl)acetamide (B1.3)

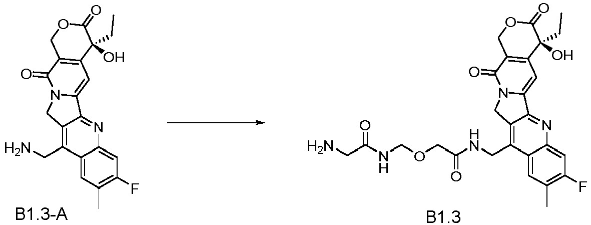

Following the same procedures and reaction conditions as in Example B1.1, but replacing (B1.2-B) with compound (B1.3-A), the target product (*S*)-2-amino-*N*-((2-(((4-ethyl-8-fluoro-4-hydroxy-9-methyl-3,14-dioxo-3,4,12,14-tetrahydro-1*H*-pyrano[3',4':6,7]indolizino[1,2-*b*]quinolin-11-yl)methyl)amino)-2-oxoethoxy)methyl)acetamide (B1.3) was obtained.

ESI-MS (m/z): 554 [M+H]⁺.

### Example B1.4: Synthesis of (S)-2-amino-N-((4-ethyl-8-fluoro-4-hydroxy-9-methyl-3,14-dioxo-3,4,12,14-tetrahydro-1H-pyrano[3',4':6,7]indolizino[1,2-b]quinolin-11-yl)methyl)acetamide (B1.4)

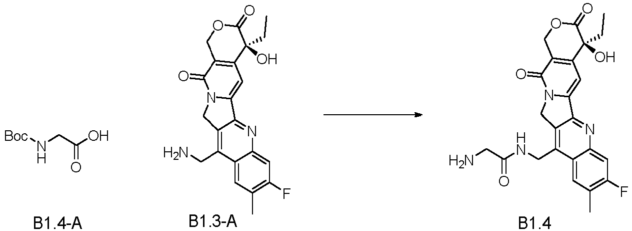

To a solution of compound (B1.4-A) (175 mg) and compound (B1.3-A) (409 mg) in DMF (5 mL) were added DIPEA (200 µL) and HBTU (420 mg). The compound mixture was stirred at room temperature for 20 h, and ethyl acetate (100 mL) was added. The resulting mixture was washed with water (100 mL × 3) and concentrated under reduced pressure to remove the organic solvent, and then a 1:1 mixture of DCM/TFA (10 mL) was added to the residue. The resulting mixture was left to stand at room temperature for 20 min and concentrated under reduced pressure to remove the low-boiling point components, and the residue was separated by preparative HPLC to obtain the target product (*S*)-2-amino-*N*-((4-ethyl-8-fluoro-4-hydroxy-9-methyl-3,14-dioxo-3,4,12,14-tetrahydro-1*H-*pyrano[3',4':6,7]indolizino[1,2-*b*]quinolin-11-yl)methyl)acetamide (B1.4).

ESI-MS (m/z): 467 [M+H]⁺.

### Example B1.5: Synthesis of (S)-2-amino-N-((7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)methyl)acetamide (B1.5)

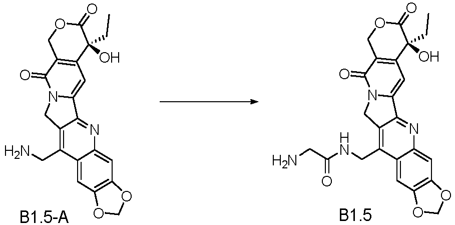

B1.5-A (200 mg) was dissolved in *N,N*-dimethylformamide (5 mL), and *N,N*-diisopropylethylamine (154.80 mg) and 2,5-dioxopyrrolidin-1-yl(*tert*-butoxycarbonyl)glycine (157 mg) were added sequentially to the reaction solution. The reaction solution was then stirred at 25 °C for 60 min. Water (30 mL) was added to the reaction solution, and the mixture was filtered under vacuum. The filter cake was dried to obtain the product as a white solid (ESI-MS (m/z): 579.4 [M+H]⁺). The above white solid product was dissolved in a mixed solution of trifluoroacetic acid and dichloromethane (volume ratio: 1:3; 4 mL), and the reaction solution was stirred at room temperature for 1 h. The reaction solution was then concentrated to obtain a crude product. The crude product was separated and purified by reversed-phase chromatography using a C18 column (acetonitrile/0.05% aqueous formic acid solution: 5% to 50%) to obtain the target product (*S*)-2-amino-*N*-((7-ethyl-7-hydroxy-8,11-dioxo-7,8,11,13-tetrahydro-10*H-*[1,3]dioxolo[4,5-g]pyrano[3',4':6,7]indolizino[1,2-*b*]quinolin-14-yl)methyl)acetamide (B1.5, 110 mg).

ESI-MS (m/z): 479.3 [M+H]⁺.

### Example B1.6: Synthesis of (S)-2-amino-N-((4-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)-1H-pyrazol-1-yl)methyl)acetamide (B1.6)

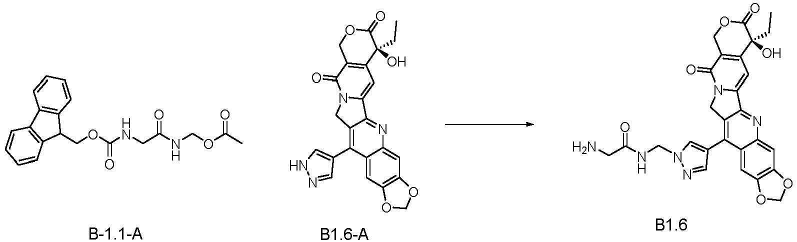

Following the same procedures and reaction conditions as in Example B1.1, but replacing compound (B1.1-B) with compound (B1.6-A), the target product (*S*)-2-amino-*N*-((4-(7-ethyl-7-hydroxy-8,11-dioxo-7,8,11,13-tetrahydro-10*H*-[1,3]dioxolo[4,5-*g*]pyrano[3',4':6,7]indolizino[1,2-*b*]quinolin-14-yl)-1*H*-pyrazol-1-yl)methyl)acetamide (B1.6) was obtained.

ESI-MS (m/z): 545 [M+H]⁺.

### Example B1.7: Synthesis of (S)-2-amino-N-((2-(((7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)methyl)amino)-2-oxoethoxy)methyl)acetamide (B1.7)

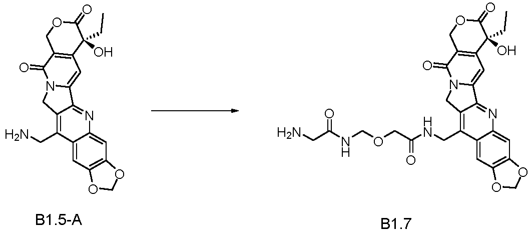

Following the same procedures and reaction conditions as in Example B1.2, but replacing compound (B1.2-B) with compound (B1.5-A), a mixture containing the target product (*S*)-2-amino-*N*-((2-(((7-ethyl-7-hydroxy-8,11-dioxo-7,8,11,13-tetrahydro-10*H-*[1,3]dioxolo[4,5-*g*]pyrano[3',4':6,7]indolizino[1,2-*b*]quinolin-14-yl)methyl)amino)-2-oxoethoxy)methyl)acetamide (B1.7) was obtained. The mixture was directly used in the next step of synthesis.

ESI-MS (m/z): 566 [M+H]⁺.

### Example B1.8: Synthesis of (S)-2-amino-N-(3-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)phenyl)acetamide (B1.8)

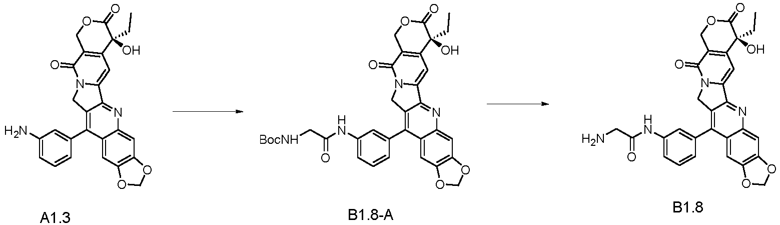

Following the same procedures and reaction conditions as in Example B1.4, but replacing compound (B1.3-A) with compound (A1.3), the target product (*S*)-2-amino-*N*-(3-(7-ethyl-7-hydroxy-8,11-dioxo-7,8,11,13-tetrahydro-10*H*-[1,3]dioxolo[4,5-*g*]pyrano[3',4':6,7]indolizino[1,2-*b*]quinolin-14-yl)phenyl)acetamide (B1.8) was obtained.

ESI-MS (m/z): 541 [M+H]⁺.

### Example B1.9: Synthesis of (S)-2-amino-N-(4-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)phenyl)acetamide (B1.9)

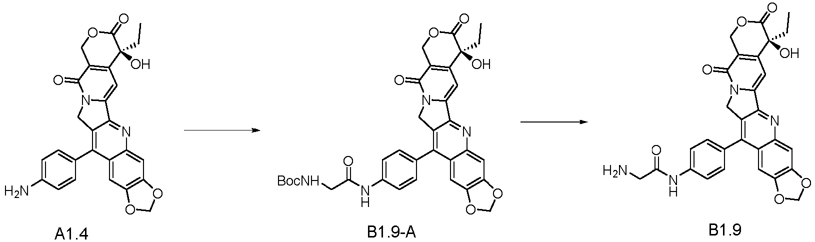

Following the same procedures and reaction conditions as in Example B1.4, but replacing compound (B1.3-A) with compound (A1.4), the target product (*S*)-2-amino-*N*-(4-(7-ethyl-7-hydroxy-8,11-dioxo-7,8,11,13-tetrahydro-10*H*-[1,3]dioxolo[4,5-*g*]pyrano[3',4':6,7]indolizino[1,2-*b*]quinolin-14-yl)phenyl)acetamide (B1.9) was obtained. Alternatively, the target product was obtained by using the following reaction conditions.

Compound A1.4 (60 mg, 0.12 mmol) was dissolved in *N,N*-dimethylformamide (3 mL), and (*tert-*butoxycarbonyl)glycine (26 mg, 0.15 mmol), HATU (56 mg, 0.15 mmol), and *N,N*-diisopropylethylamine (48 mg, 0.37 mmol) were added sequentially. The mixture was stirred at room temperature for 1 h. The reaction was completed as detected by TLC. TFA (1.0 mL) was then added directly to the above reaction solution. The mixture was stirred at room temperature for another 1 h. The reaction was completed as detected by LCMS. The reaction solution was concentrated to remove the trifluoroacetic acid to obtain a crude product. The crude product was purified by preparative chromatography (0.01% aqueous trifluoroacetic acid solution, acetonitrile) to obtain the target product (*S*)-2-amino-*N*-(4-(7-ethyl-7-hydroxy-8,11-dioxo-7,8,11,13-tetrahydro-10*H*-[1,3]dioxolo[4,5-g]pyrano[3',4':6,7]indolizino[1,2-*b*]quinolin-14-yl)phenyl)acetamide (B1.9) (26.3 mg, yield: 38%) as a yellow solid.

ESI-MS (m/z): 541 [M+H]⁺;

¹H NMR (400 MHz, DMSO) δ 10.71 (s, 1H), 8.16 (s, 2H), 7.86 (d, *J* = 8.6 Hz, 2H), 7.67-7.58 (m, 3H), 7.29 (s, 1H), 7.04 (s, 1H), 6.50 (s, 1H), 6.28 (s, 2H), 5.40 (s, 2H), 5.05 (s, 2H), 3.87 (s, 2H), 1.93-1.81 (m, 2H), 0.88 (t, *J =* 7.3 Hz, 3H).

### Example B1.10: Synthesis of (S)-2-amino-N-(3-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)propyl)acetamide (B1.10)

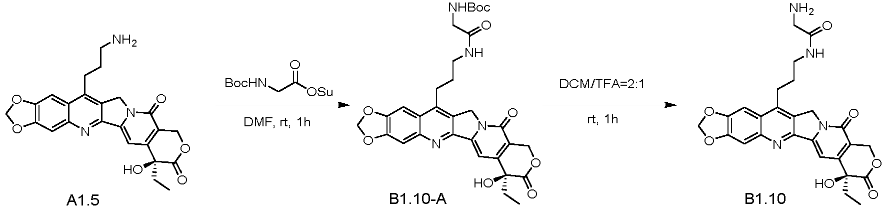

### Step 1:

To a solution of compound A1.5 (200 mg) in *N,N*-dimethylformamide (5 mL) were added triethylamine (67 mg) and 2,5-dioxopyrrolidin-1-yl(*tert*-butoxycarbonyl)glycine (182 mg) at room temperature, and the mixture was allowed to react at room temperature for 1 h. The reaction solution was diluted with water (30 mL) and extracted with ethyl acetate (30 mL × 2). The organic phase was washed with saturated brine, dried over anhydrous sodium sulfate, and concentrated to obtain a crude product. The crude product was further purified using a C18 column (acetonitrile/0.05% formic acid in water: 5%-60%) to obtain the target compound (B1.10-A, 100 mg).

LCMS (ESI) [M+H]⁺: 607.

### Step 2:

To a solution of compound B1.10-A (100 mg) in dichloromethane (4 mL) was added trifluoroacetic acid (2 mL), and the mixture was stirred at room temperature for 1 h. The reaction solution was concentrated, and the crude product was added to a solution of *N,N*-dimethylformamide (3 mL) and further purified using a C18 column (acetonitrile/0.05% formic acid in water: 5%-60%) to obtain the target compound (*S*)-2-amino-*N*-(3-(7-ethyl-7-hydroxy-8,11-dioxo-7,8,11,13-tetrahydro-10*H*-[1,3]dioxolo[4,5-*g*]pyrano[3',4':6,7]indolizino[1,2-*b*]quinolin-14-yl)propyl)acetamide (B1.10, 80 mg).
LCMS (ESI) [M+H]⁺: 507;
¹H NMR (400 MHz, DMSO-d6) δ 8.26 (s, 1H), 7.64 (s, 1H), 7.51 (s, 1H), 7.24 (s, 1H), 6.51 (s, 1H), 6.29 (s, 2H), 5.42 (s, 2H), 5.23 (s, 2H), 3.31 (s, 2H), 3.30 -3.27 (m, 2H), 3.13 - 3.07 (m, 2H), 2.04 - 1.76 (m, 4H), 0.87 (t, *J =* 7.3 Hz, 3H).

### Example B1.11: Synthesis of (S)-2-((2-aminoacetamido)methoxy)-N-isopropyl-N-(2-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)ethyl)acetamide (B1.11)

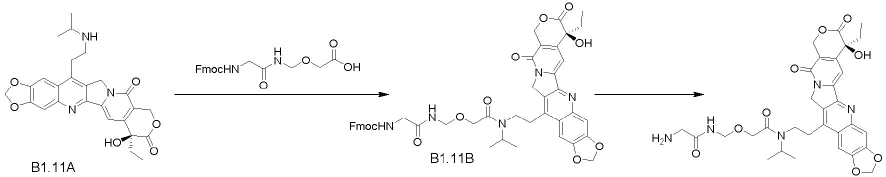

### Step 1:

Compound 1-(9*H*-fluoren-9-yl)-3,6-dioxo-2,9-dioxa-4,7-diazaundecan-11-oic acid (242 mg), diisopropylethylamine (330 mg), and *N,N,N',N'*-tetramethyluronium hexafluorophosphate (359 mg) were dissolved in an *N,N*-dimethylformamide solution (10 mL). Compound (*S*)-7-ethyl-7-hydroxy-14-(2-(isopropylamino)ethyl)-10,13-dihydro-11*H*-[1,3]dioxolo[4,5-*g*]pyrano[3',4':6,7]indolizino[1,2-*b*]quinoline-8,11(7*H*)-dione (300 mg) was then added, and the reaction mixture was allowed to react at room temperature for 2 h. Ethyl acetate (50 mL) was added to the reaction solution, and the mixture was washed with saturated brine (30 mL × 3), dried over anhydrous sodium sulfate, and filtered under vacuum. The filtrate was concentrated under reduced pressure to obtain a solid, and the crude product was separated and purified by column chromatography (dichloromethane:methanol = 10/1) to obtain the target compound B1.11B (220 mg).

LCMS (ESI) [M+H]⁺: 844.0.

### Step 2: Preparation of (S)-2-((2-aminoacetamido)methoxy)-N-isopropyl-N-(2-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)ethyl)acetamide

To a solution of compound B1.11B (220 mg, 0.261 mmol) in *N,N*-dimethylformamide (4 mL) was added piperidine (1 mL), and the reaction mixture was stirred at room temperature for 20 min. The reaction was completed as detected by LCMS. The reaction solution was concentrated under reduced pressure to obtain the target compound (220 mg) as a brown solid, and the product was directly used in the next step of synthesis without purification.

LCMS (ESI) [M+H]⁺: 622.1.

### Example B1.12: Synthesis of (S)-2-amino-N-((3-(4-ethyl-8-fluoro-4-hydroxy-9-methyl-3,14-dioxo-3,4,12,14-tetrahydro-1H-pyrano[3',4':6,7]indolizino[1,2-b]quinolin-11-yl)propoxy)methyl)acetamide (B1.12)

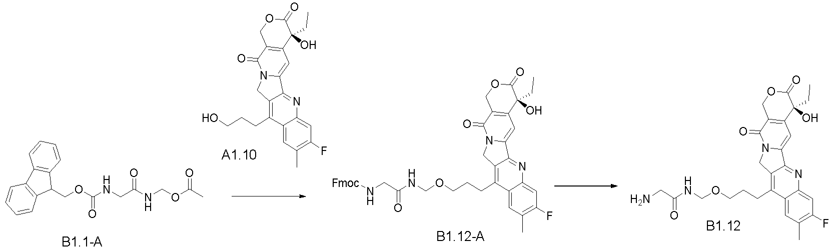

### Step 1:

Compound A1.10 (160 mg, 0.365 mmol) was dissolved in *N,N*-dimethylformamide (3 mL), and (2-((((9*H-*fluoren-9-yl)methoxy)carbonyl)amino)acetamido)methyl acetate (B1.1-A, 672 mg, 1.83 mmol) was added. Then, hydrochloric acid-ethyl acetate (0.073 mL, 3 M) was added to the reaction solution, and the reaction solution was stirred at room temperature overnight. The reaction was completed as detected by LCMS. The reaction solution was directly purified by reversed-phase chromatography (acetonitrile/0.05% FA in water: 5% to 50%) to obtain the target compound (80 mg, yield: 29.0%) as a white solid.

LCMS (ESI) [M+H]⁺ = 747.4;

¹H NMR (400 MHz, DMSO-d6) δ 8.74 (t, *J* = 6.4 Hz, 1H), 8.28 - 8.17 (m, 1H), 7.99 - 7.88 (m, 3H), 7.73 (d, *J* = 7.3 Hz, 2H), 7.63 (t, *J* = 5.7 Hz, 1H), 7.44 (t, *J* = 7.4 Hz, 2H), 7.35 (t, *J* = 7.2 Hz, 3H), 6.58 (s, 1H), 5.48 (s, 2H), 5.29 (s, 2H), 4.66 (d, *J* = 6.3 Hz, 2H), 4.32 (d, *J* = 6.9 Hz, 2H), 4.26 (d, *J* = 6.1 Hz, 1H), 3.71 (d, *J* = 5.8 Hz, 2H), 3.57 (t, *J =* 5.7 Hz, 2H), 3.29 - 3.20 (m, 2H), 2.55 (s, 3H), 2.00 - 1.86 (m, 4H), 0.93 (t, *J* = 7.2 Hz, 3H).

Step 2: B1.12-A (240 mg) was dissolved in DMF (5 mL), and piperidine (1 mL) was added. The compound mixture was stirred for 20 min and concentrated under reduced pressure to remove the low-boiling point components, and the residue was directly used in the next step of synthesis.

ESI-MS (m/z): 525.2 [M+H]⁺.

A small amount of the crude product was purified by reversed-phase chromatography (acetonitrile/0.05% FA in water: 5% to 50%) to obtain the target compound.
ESI-MS (m/z): 525.1 [M+H]⁺;
¹H NMR (400 MHz, DMSO) δ 9.13 (t, *J* = 6.6 Hz, 1H), 8.21 (d, *J* = 8.1 Hz, 1H), 8.02 (brs, 2H), 7.89 (d, *J* = 10.8 Hz, 1H), 7.32 (s, 1H), 6.54 (s, 1H), 5.44 (s, 2H), 5.28 (s, 2H), 4.66 (d, *J* = 6.5 Hz, 2H), 3.64 (s, 2H), 3.53 (t, *J* = 6.1 Hz, 2H), 3.25-3.18 (m, 2H), 2.52 (s, 3H), 1.98-1.84 (m, 4H), 0.88 (t, *J* = 7.3 Hz, 3H).

### Example B1.13: Synthesis of (S)-2-amino-N-(3-(4-ethyl-8-fluoro-4-hydroxy-9-methyl-3,14-dioxo-3,4,12,14-tetrahydro-1H-pyrano[3',4':6,7]indolizino[1,2-b]quinolin-11-yl)propyl)acetamide (B1.13)

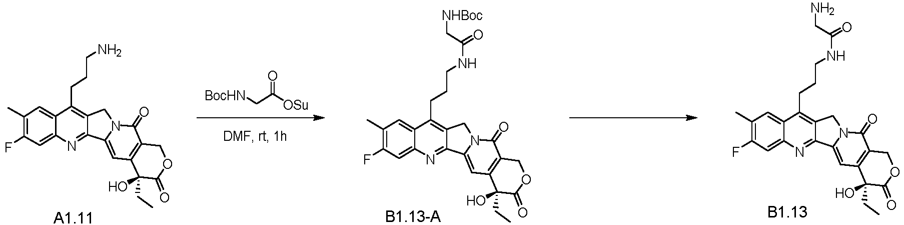

### Step 1:

To a solution of compound A1.11 (200 mg) in *N,N*-dimethylformamide (5 mL) were added triethylamine (67 mg) and 2,5-dioxopyrrolidin-1-yl(*tert-*butoxycarbonyl)glycine (182 mg) at room temperature, and the mixture was allowed to react at room temperature for 1 h. The reaction solution was diluted with water (30 mL) and extracted with ethyl acetate (30 mL × 2). The organic phase was washed with saturated brine, dried over anhydrous sodium sulfate, and concentrated to obtain a crude product. The crude product was further purified using a C18 column (acetonitrile/0.05% formic acid in water: 5%-60%) to obtain the target compound (B1.13-A, 104 mg).

LCMS (ESI) [M+H]⁺: 595.

### Step 2:

To a solution of compound B1.13-A (100 mg) in dichloromethane (4 mL) was added trifluoroacetic acid (2 mL), and the mixture was stirred at room temperature for 1 h. The reaction solution was concentrated, and the crude product was added to a solution of *N,N*-dimethylformamide (3 mL) and further purified using a C18 column (acetonitrile/0.05% formic acid in water: 5%-60%) to obtain the target compound (*S*)-2-amino-*N*-(3-(4-ethyl-8-fluoro-4-hydroxy-9-methyl-3,14-dioxo-3,4,12,14-tetrahydro-1*H*-pyrano[3',4':6,7]indolizino[1,2-*b*]quinolin-11-yl)propyl)acetamide (B1.13, 88 mg).

LCMS (ESI) [M+H]⁺: 495.

### Example B1.14: Synthesis of (S)-2-amino-N-((3-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)propoxy)methyl)acetamide (B1.14)

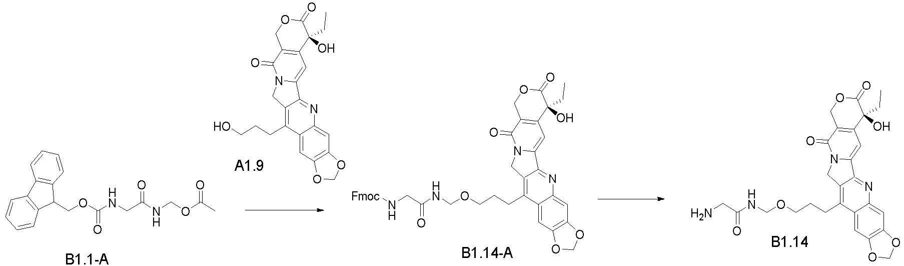

Step 1: Compound (B1.1-A) (368 mg), compound (A1.9) (440 mg), and pyridinium *p*-toluenesulfonate (PPTS) (25 mg) were refluxed in dichloromethane (20 mL) for 20 h and then washed with an aqueous sodium bicarbonate solution and an aqueous hydrochloric acid solution sequentially. The mixture was concentrated under reduced pressure to remove the organic solvent to obtain a crude product. The crude product was separated and purified by column chromatography (dichloromethane/methanol = 10/1) to obtain the target compound B1.14A (240 mg).

LCMS (ESI) [M+H]⁺: 759.5.

Step 2: B1.14-A (240 mg) was dissolved in DMF (5 mL), and piperidine (1 mL) was added. The compound mixture was stirred for 20 min and concentrated under reduced pressure to remove the low-boiling point components, and the residue was directly used in the next step of synthesis. A small amount of the crude product was purified by reversed-phase chromatography (acetonitrile/0.05% FA in water: 5% to 50%) to obtain the target compound.

ESI-MS (m/z): 537.4 [M+H]⁺;

¹H NMR (400 MHz, DMSO-d6) δ 9.13 (t, 1H), 8.04 (br, 2H), 7.58 (s, 1H), 7.51 (s, 1H), 7.25 (s, 1H), 6.29 (s, 2H), 5.43 (S, 2H), 5.21 (s, 2H), 4.65 (d, 2H), 3.63 (m, 2H), 3.53 (m, 2H), 3.11 (m, 2H), 1.87 (m, 4H), 0.88 (t, 3H).

### Example B1.15: (S,E)-2-Amino-N-(3-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)allyl)acetamide

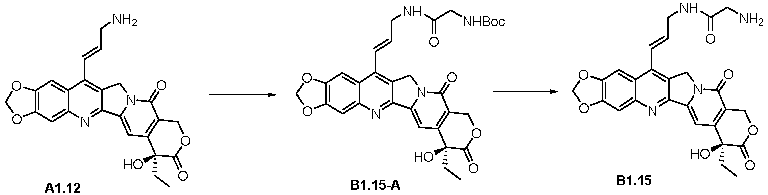

### Step 1:

To a solution of compound A1.12 (200 mg, 0.447 mmol) in *N,N*-dimethylformamide (5 mL) were added triethylamine (135 mg, 1.341 mmol) and *N*-hydroxy-2,5-dioxopyrrolidine *tert*-butoxycarbonyl glycinate (183 mg, 0.671 mmol) at room temperature, and the mixture was allowed to react at room temperature for 1 h. The reaction was completed as detected by LCMS. Water (20 mL) was added to the reaction solution, and the mixture was extracted with ethyl acetate (20 mL × 3). The organic phase was washed with saturated brine, dried over anhydrous sodium sulfate, and concentrated to obtain the target compound B1.15A (130 mg, yield: 48%) as a brown solid.

LCMS (ESI) [M+H]⁺ =605.6.

### Step 2:

Compound B1.15-A (130 mg, 0.215 mmol) was dissolved in a solution of hydrochloric acid in 1,4-dioxane (5 mL) at room temperature, and the resulting solution was allowed to react at room temperature for 30 min. The reaction was completed as detected by LCMS. The reaction solution was purified by reversed-phase chromatography to obtain the target compound (60 mg, yield: 52%) as a brown solid.
LCMS (ESI) [M+H]⁺ =505.2;
¹H NMR (400 MHz, DMSO-d6) δ 8.85 (s, 1H), 8.15 (s, 2H), 7.70 (s, 1H), 7.54 (s, 1H), 7.26 (s, 1H), 7.21 (d, *J =* 16.2 Hz, 1H), 6.51 (d, *J* = 16.2 Hz, 1H), 6.31 (s, 2H), 5.42 (s, 2H), 5.28 (s, 2H), 4.19 (s, 2H), 1.86 - 1.82 (m, 4H), 0.87 (t, *J* = 7.3 Hz, 3H).

### Example B1.16: (S,E)-2-Amino-N-(((3-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)allyl)oxy)methyl)acetamide

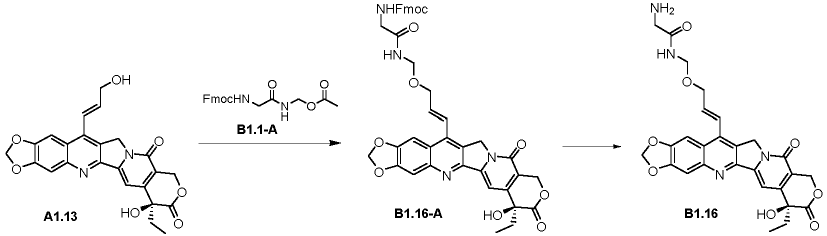

### Step 1:

The starting material A1.13 (150 mg, 0.335 mmol) was dissolved in toluene (3 mL) under a nitrogen atmosphere, and (2-((((9*H*-fluoren-9-yl)methoxy)carbonyl)amino)acetamido)methyl acetate (B1.1-A, 308 mg, 0.84 mmol) was added. Then, zinc acetate (123 mg, 0.67 mmol) was added to the reaction solution, and the reaction solution was stirred at 100 °C overnight. The reaction was completed as detected by LCMS. The reaction solution was directly purified by reversed-phase chromatography (acetonitrile/0.05% formic acid in water: 5% to 50%) to obtain the target compound (9*H*-fluoren-9-yl)methyl(*S,E*)-(2-((((3-(7-ethyl-7-hydroxy-8,11-dioxo-7,8,11,13-tetrahydro-10*H*-[1,3]dioxolo[4,5-g]pyrano[3',4':6,7]indolizino[1,2-*b*]quinolin-14-yl)allyl)oxy)methyl)amino)-2-oxoethyl)carbamate (B1.16-A, 80 mg, yield: 31%) as a white solid.

LCMS (ESI) [M+H]⁺ = 757.4.

### Step 2:

Compound B1.16-A (80 mg, 0.11 mmol) was dissolved in N,N-dimethylformamide (2 mL), and then piperidine (0.05 mL) was added to the reaction solution. The mixture was stirred at room temperature for 1 h. The reaction was completed as detected by LCMS. The reaction solution was concentrated to obtain a crude product. The crude product was separated and purified by reversed-phase chromatography using a C18 column (acetonitrile/0.05% ammonium hydroxide in water: 5% to 50%) to obtain the target compound (35 mg, yield: 62%) as a brown solid.

LCMS (ESI) [M+H]⁺ = 535.2, tR = 1.070 min.

### Example B1.17: (S,E)-2-Amino-N-(((3-(4-ethyl-8-fluoro-4-hydroxy-9-methyl-3,14-oxo-3,4,12,14-tetrahydro-1H-pyrano[3',4':6,7]indolizino[1,2-b]quinolin-11-yl)allyl)oxy)methyl)acetamide

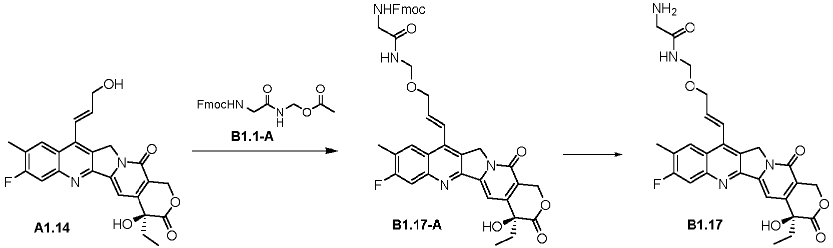

### Step 1:

Compound A1.14 (80 mg, 0.183 mmol) and compound (2-((((9*H*-fluoren-9-yl)methoxy)carbonyl)amino)acetamido)methyl acetate (B1.1-A, 135 mg, 0.367 mmol) were dissolved in toluene (4 mL), and zinc acetate (190 mg, 1.03 mmol) was added. The reaction solution was stirred at 100 °C for 48 h and then cooled to room temperature. Water (10 mL) was added, and the mixture was extracted with ethyl acetate (10 mL × 3). The organic phases were combined, dried over anhydrous sodium sulfate, filtered, and concentrated to obtain a crude product. The crude product was purified by reversed-phase chromatography (0.05% FA in water/acetonitrile: 5% to 100%) to obtain the target compound (9*H*-fluoren-9-yl)methyl (*S,E*)-(2-((((3-(4-ethyl-8-fluoro-4-hydroxy-9-methyl-3,14-dioxo-3,4,12,14-tetrahydro-1*H*-pyrano[3',4':6,7]indolizino[1,2-*b*]quinolin-11-yl)allyl)oxy)methyl)amino)-2-oxoethyl)carbamate (B1.17-A, 30 mg) as a white solid.

LCMS (ESI) [M+H]⁺ = 745.2;

¹H NMR (400 MHz, DMSO) δ 8.82 (m 1H), 8.21 (m, 1H), 7.89 (m, 2H), 7.84 (d, *J* = 7.8 Hz, 2H), 7.68 (m, 1H), 7.61 (m, 1H), 7.42 (m, 1H), 7.37 (m, 1H), 7.33 - 7.28 (m, 4H), 6.72 - 6.55 (m, 1H), 6.53 (s, 1H), 5.42 (s, 2H), 5.28 (s, 2H), 4.75 (m, 2H), 4.34 (m, 2H), 4.28 - 4.24 (m, 2H), 4.20 (m, 1H), 3.70 (d, *J* = 6.1 Hz, 2H), 2.47 (s, 3H), 1.90 - 1.82 (m, 2H), 0.88 (t, *J =* 7.3 Hz, 3H).

### Step 2:

Compound 4 (25 mg, 0.034 mmol) was dissolved in N,N-dimethylformamide (2 mL), and then diethylamine (0.2 mL) was added. After the addition, the reaction solution was stirred at room temperature for 1 h. After the reaction was completed as detected by LCMS, the reaction solution was directly evaporated to dryness under reduced pressure to remove the solvent to obtain a crude product of the target compound (*S,E*)-2-amino-*N-*(((3-(4-ethyl-8-fluoro-4-hydroxy-9-methyl-3,14-oxo-3,4,12,14-tetrahydro-1*H*-pyrano[3',4':6,7]indolizino[1,2-*b*]quinolin-11-yl)allyl)oxy)methyl)acetamide as a yellow viscous substance.

LCMS (ESI) [M+H]⁺ = 523.2;

LCMS (ESI) [M+H]⁺ = 523.1, t_{R} = 0.446 min, 1.311 min.

¹H NMR (400 MHz, DMSO) δ 9.26 (t, *J* = 6.5 Hz, 1H), 8.23 (d, *J* = 8.2 Hz, 1H), 8.06 (s, 2H), 7.91 (m, 1H), 7.40 (d, *J* = 16.3 Hz, 1H), 7.34 (s, 1H), 6.70 - 6.60 (m, 1H), 6.54 (s, 1H), 5.44 (s, 2H), 5.35 (s, 2H), 4.80 (d,*J* = 6.6 Hz, 2H), 4.38 (d, *J* = 3.9 Hz, 2H), 3.68 (d, 2H), 2.52 (s, 3H), 1.90 - 1.84 (m, 2H), 0.88 (m, 3H).

### C. Synthesis of intermediates containing molecular fragments with conjugation linking segments

### Example C1.1: N⁶-(tert-Butoxycarbonyl)-N²-((1-(2-(methylthio)pyrimidin-5-yl)-1-oxo-5,8,11,14,17,20,23,26,29-nonaoxa-2-aza-hentriacontanoyl)-L-valyl)-L-lysine (compound C1.1)

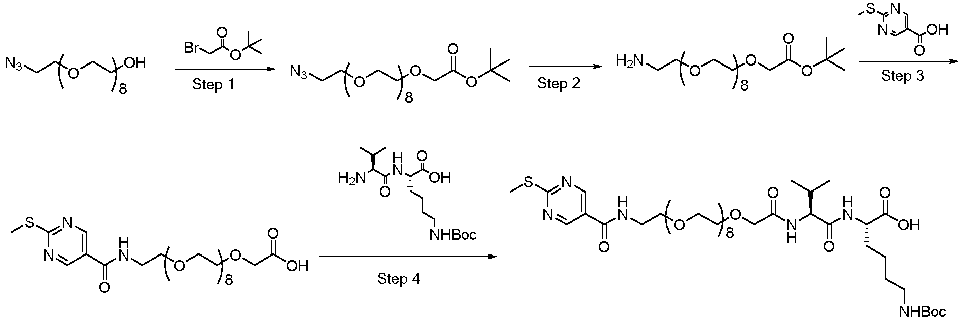

### Step 1: tert-Butyl 29-azido-3,6,9,12,15,18,21,24,27-nonaoxa-nonacosanoate

To a solution of 26-azido-3,6,9,12,15,18,21,24-octaoxa-26-alkan-1-ol (4.39 g) in DMF (40 mL) was added NaH (0.6 g, 60%). After the mixture was stirred for 30 min, *tert-*butyl bromoacetate (2.4 g) was added. The mixture was stirred under dry conditions for 20 h. Then, ethyl acetate (200 mL) and water (200 mL, added slowly at first) were added to the mixture. The organic phase was washed with water (100 mL × 3), dried over anhydrous sodium sulfate, concentrated under reduced pressure to remove the solvent, and the residue was separated by silica gel column chromatography to obtain the target product *tert-*butyl 29-azido-3,6,9,12,15,18,21,24,27-nonaoxa-nonacosanoate.

ESI-MS *(m*/*z):* 554 [M + H]⁺.

### Step 2: tert-Butyl 29-amino-3,6,9,12,15,18,21,24,27-nonaoxa-nonacosanoate

To a solution of *tert-*butyl 29-azido-3,6,9,12,15,18,21,24,27-nonaoxa-nonacosanoate (1.16 g) in ethyl acetate (20 mL) was added palladium on carbon catalyst (Pd/C, 10%, 100 mg). The above solution was stirred under a hydrogen atmosphere for 5 h, then filtered to remove the palladium on carbon, and concentrated under reduced pressure to remove the solvent to obtain the target product.

ESI-MS *(m*/*z):* 528 [M + H]⁺.

### Step 3: 1-(2-(Methylthio)pyrimidin-5-yl)-1-oxo-5,8,11,14,17,20,23,26,29-nonaoxa-2-aza-hentriacontanoic acid

*tert-*Butyl 29-amino-3,6,9,12,15,18,21,24,27-nonaoxa-nonacosanoate (527 mg) and 2-methylthio-pyrimidine-5-carboxylic acid (170 mg) were added to anhydrous DMF (10 mL), and then DIPEA (0.2 mL) and HBTU (420 mg) were added sequentially to the above solution while cooling under an ice bath. The above mixture was stirred at room temperature for 20 h, then diluted with ethyl acetate (100 mL), and washed with water (100 mL × 4). The organic phase was dried over anhydrous sodium sulfate and then concentrated under reduced pressure to remove the organic solvent. The residue was dissolved in DCM (10 mL), and then TFA (10 mL) was added. The mixture was stirred at room temperature for 1 h and then concentrated under reduced pressure to remove the low-boiling point components. The residue was separated by preparative HPLC to obtain 1-(2-(methylthio)pyrimidin-5-yl)-1-oxo-5,8,11,14,17,20,23,26,29-nonaoxa-2-aza-hentriacontanoic acid.

ESI-MS *(m*/*z):* 624 [M + H]⁺.

### Step 4: N⁶-(tert-Butoxycarbonyl)-N²-((1-(2-(methylthio)pyrimidin-5-yl)-1-oxo-5,8,11,14,17,20,23,26,29-nonaoxa-2-aza-hentriacontanoyl)-L-valyl)-L-lysine

1-(2-(Methylthio)pyrimidin-5-yl)-1-oxo-5,8,11,14,17,20,23,26,29-nonaoxa-2-aza-31-hentriacontanoic acid (623 mg) was dissolved in DMF (10 mL), and DIPEA (300 µL) and HBTU (420 mg) were added at 0 °C. The mixture was stirred for 30 min, and then the valine-(Boc)lysine dipeptide compound (345 mg) was added. The reaction solution was stirred for 20 h, and then ethyl acetate (100 mL) was added. The mixture was washed with diluted hydrochloric acid (20 mL × 3). The organic phase was dried and then concentrated under reduced pressure to remove the organic solvent, and the crude product was separated by preparative HPLC to obtain the target compound *N*⁶-(*tert*-butoxycarbonyl)-*N*²-((1-(2-(methylthio)pyrimidin-5-yl)-1-oxo-5,8,11,14,17,20,23,26,29-nonaoxa-2-aza-hentriacontanoyl)-*L*-valyl)-*L*-lysine (compound C1.1).

ESI-MS *(m*/*z):* 951 [M + H]⁺.

### Example C1.2: N⁶-(tert-Butoxycarbonyl)-N²-((29-(4-(2-(methylthio)pyrimidin-5-yl)-1H-1,2,3-triazol-1-yl)-3,6,9,12,15,18,21,24,27-nonaoxa-nonacosanoyl)-L-valyl)-L-lysine (compound C1.2)

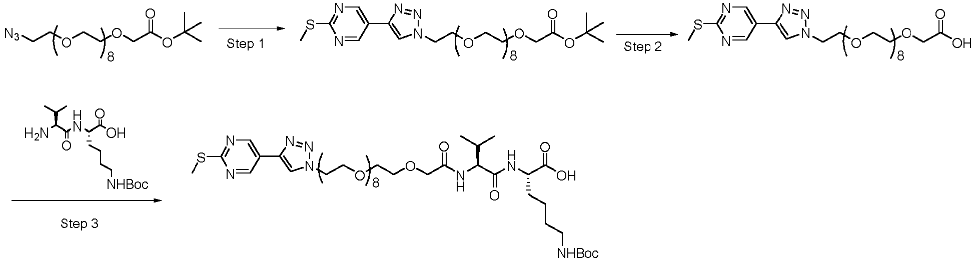

### Step 1: tert-Butyl 29-(4-(2-(methylthio)pyrimidin-5-yl)-1H-1,2,3-triazol-1-yl)-3,6,9,12,15,18,21,24,27-nonaoxa-nonacosanoate

*tert-Butyl* 29-azido-3,6,9,12,15,18,21,24,27-nonaoxa-nonacosanoate (1.7 g) and 2-methylthio-5-ethynylpyrimidine (450 mg) were dissolved in DMSO-water (20 mL, 4:1), and cuprous bromide (50 mg) was added to the mixture. The reaction solution was stirred at room temperature for 2 h, and then ethyl acetate (100 mL) was added. The mixture was washed with water (100 mL × 3), dried, and then concentrated under reduced pressure to remove the organic solvent, and the crude product was separated by silica gel column chromatography to obtain the target product *tert-*butyl 29-(4-(2-(methylthio)pyrimidin-5-yl)-1*H*-1,2,3-triazol-1-yl)-3,6,9,12,15,18,21,24,27-nonaoxa-nonacosanoate.

MS *(m*/*z):* 704 [M + H]⁺.

### Step 2: 29-(4-(2-(Methylthio)pyrimidin-5-yl)-1H-1,2,3-triazol-1-yl)-3,6,9,12,15,18,21,24,27-nonaoxa-nonacosanoic acid

*tert-Butyl* 29-(4-(2-(methylthio)pyrimidin-5-yl)-1H-1,2,3-triazol-1-yl)-3,6,9,12,15,18,21,24,27-nonaoxa-nonacosanoate (1 g) was dissolved in dichloromethane (10 mL), and then TFA (5 mL) was added. The mixture was left to stand at room temperature for 1 h and then concentrated under reduced pressure to remove the low-boiling point components to obtain the target product 29-(4-(2-(methylthio)pyrimidin-5-yl)-1H-1,2,3-triazol-1-yl)-3,6,9,12,15,18,21,24,27-nonaoxa-nonacosanoic acid.

MS *(m*/*z):* 648 [M + H]⁺.

### Step 3: N⁶-(tert-Butoxycarbonyl)-N²-((29-(4-(2-(methylthio)pyrimidin-5-yl)-1H-1,2,3-triazol-1-yl)-3,6,9,12,15,18,21,24,27-nonaoxa-nonacosanoyl)-L-valyl)-L-lysine

29-(4-(2-(Methylthio)pyrimidin-5-yl)-1*H*-1,2,3-triazol-1-yl)-3,6,9,12,15,18,21,24,27-nonaoxa-nonacosanoic acid(647 mg) was dissolved in DMF (10 mL), and DIPEA (300 µL) and HBTU (420 mg) were added at 0 °C. The mixture was stirred for 30 min, and then the valine-lysine dipeptide compound (345 mg) was added. The reaction solution was stirred for 20 h, and then ethyl acetate (100 mL) was added. The mixture was washed with diluted hydrochloric acid (20 mL × 3). The organic phase was dried and then concentrated under reduced pressure to remove the organic solvent, and the crude product was separated by preparative HPLC to obtain the target compound *N*⁶-(*tert*-butoxycarbonyl)-*N*²-((29-(4-(2-(methylthio)pyrimidin-5-yl)-1*H*-1,2,3-triazol-1-yl)-3,6,9,12,15,18,21,24,27-nonaoxa-nonacosanoyl)-*L*-valyl)-*L*-lysine (compound C1.2).

ESI-MS *(m*/*z):* 975 [M + H]⁺.

### Example C1.3: (1-(2-(Methylthio)pyrimidin-5-yl)-1-oxo-5,8,11,14,17,20,23,26,29-nonaoxa-2-aza-hentriacontanoyl)-glycyl-glycyl-L-phenylalanine (compound C1.3)

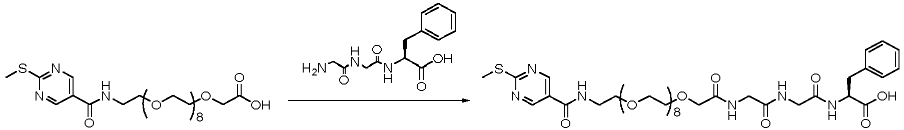

Following the same procedures and reaction conditions as in step 4 of Example C1.1, the target product (1-(2-(methylthio)pyrimidin-5-yl)-1-oxo-5,8,11,14,17,20,23,26,29-nonaoxa-2-aza-hentriacontanoyl)-glycyl-glycyl-*L*-phenylalanine (compound C1.3) was obtained using the corresponding starting materials.

ESI-MS (m/z): 885 [M+H]⁺.

### Example C1.4: (36-(2-(Methylthio)pyrimidin-5-yl)-31-oxo-3,6,9,12,15,18,21,24,27-nonaoxa-30-aza-hexatriacontan-(35-yn)-oyl)glycylglycyl-phenylalanine (compound C1.4)

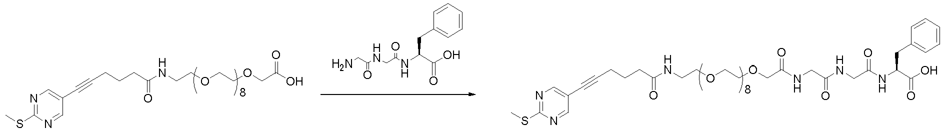

Following the same procedures and reaction conditions as in step 4 of Example C1.1, the target product (36-(2-(methylthio)pyrimidin-5-yl)-31-oxo-3,6,9,12,15,18,21,24,27-nonaoxa-30-aza-hexatriacontan-(35-yn)-oyl)glycylglycyl-phenylalanine (compound C1.4) was obtained using the starting materials shown in the reaction formula.

ESI-MS (m/z): 951 [M+H]⁺.

### Example C1.5: N⁶-(tert-Butoxycarbonyl)-N²-((36-(2-(methylthio)pyrimidin-5-yl)-31-oxo-3,6,9,12,15,18,21,24,27-nonaoxa-30-aza-hexatriacontan-(35-yn)-oyl)-L-valyl)-L-lysine (C1.5)

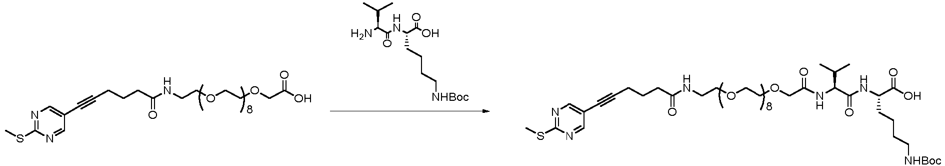

Following the same procedures and reaction conditions as in step 4 of Example C1.1, the target product *N*⁶-(tert-butoxycarbonyl)-*N*²-((36-(2-(methylthio)pyrimidin-5-yl)-31-oxo-3,6,9,12,15,18,21,24,27-nonaoxa-30-aza-hexatriacontan-(35-yn)-oyl)-*L*-valyl)-*L*-lysine (compound C1.5) was obtained using the compounds shown in the reaction formula as the starting materials.

ESI-MS (m/z): 1017 [M+H]⁺.

### Example C1.6: N⁶-(tert-Butoxycarbonyl)-N²-((6-(2-(methylthio)pyrimidin-5-yl)-hex-5-ynoyl)-L-valyl)-L-lysine (compound C1.6)

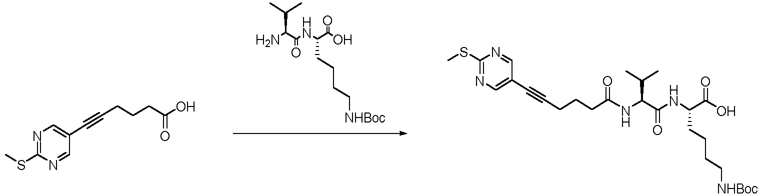

6-(2-(Methylthio)pyrimidin-5-yl)hex-5-ynoic acid (920 mg) and *N*-hydroxysuccinimide (537 mg) were dissolved in dichloromethane (50 mL), and then dicyclohexylcarbodiimide (963 mg) was added. The reaction solution was stirred at room temperature for 1 h and then concentrated under reduced pressure to remove the dichloromethane. The residue was dissolved in *N,N-*dimethylformamide (50 mL), and then *N*²-*L*-valyl-*N*⁶-(Boc)-*L-*lysine (1.5 g) was added. The reaction solution was stirred at room temperature for 16 h and then poured into 200 mL of water. The aqueous solution was adjusted to pH = 11 with a saturated sodium bicarbonate solution and extracted twice with ethyl acetate. The organic phase was discarded, and the aqueous phase was adjusted to pH = 4-5 with citric acid and extracted three times with ethyl acetate (100 mL × 3). The organic phase was washed with saturated brine (100 mL), dried over anhydrous sodium sulfate, filtered under vacuum, and concentrated by rotary evaporation to obtain 2.8 g of a crude product. The crude product was separated and purified by flash chromatography (DCM:MeOH = 30:1) to obtain the target compound *N*⁶-(*tert*-butoxycarbonyl)-*N*²-((6-(2-(methylthio)pyrimidin-5-yl)-hex-5-ynoyl)-*L*-valyl)-*L*-lysine (1.0 g) (compound C1.6).

ESI-MS (m/z): 564.3 [M+H]⁺.

¹H NMR (400 MHz, DMSO-*d*₆) δ 12.45 (s, 1H), 8.68 (s, 2H), 8.12 (d, *J* = 7.3 Hz, 1H), 7.88 (d, *J* = 8.9 Hz, 1H), 6.76 (s, 1H), 4.27 - 4.19 (m, 1H), 4.11 (d, *J* = 4.9 Hz, 1H), 2.88 (d, *J* = 7.9 Hz, 3H), 2.73 (s, 1H), 2.59 (s, 1H), 2.52 (s, 4H), 2.40 - 2.24 (m, 3H), 2.03 - 1.88 (m, 2H), 1.77 (d, *J* = 3.2 Hz, 2H), 1.68 (d, *J* = 7.0 Hz, 1H), 1.60 - 1.49 (m, 1H), 1.36 (s, 15H), 0.85 (dd, *J* = 14.8, 6.7 Hz, 7H).

### Example C1.7: N⁶-(tert-Butoxycarbonyl)-N²-((6-(2-(methylsulfonyl)pyrimidin-5-yl)-hex-5-ynoyl)-L-valyl)-L-lysine (compound C1.7)

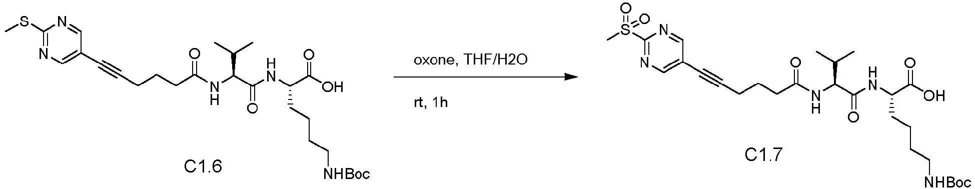

To a mixed solution of compound C1.6 (260 mg) in tetrahydrofuran (3 mL) and water (3 mL) was added potassium peroxymonosulfonate (1.414 g), and the mixture was stirred at room temperature for 1 h. The reaction solution was filtered, and the filtrate was purified using a C18 column (acetonitrile/0.05% formic acid in water: 5%-60%) to obtain the target compound *N*⁶-(*tert*-butoxycarbonyl)-*N*²-((6-(2-(methylsulfonyl)pyrimidin-5-yl)-hex-5-ynoyl)-L-valyl)-L-lysine (compound C1.7) (120 mg).

LCMS (ESI) [M+H]⁺: 596;

¹H NMR (400 MHz, DMSO-d6) δ 12.48 (s, 1H), 9.13 (s, 2H), 8.14 (d, *J* = 7.0 Hz, 1H), 7.90 (d,*J* = 9.1 Hz, 1H), 6.77 (s, 1H), 4.24 (t, *J* = 7.7 Hz, 1H), 4.12 (br s, 1H), 3.41 (s, 3H), 2.89 (d, *J* = 6.0 Hz, 2H), 2.43 - 2.29 (m, 2H), 2.03 - 1.93 (m, 2H), 1.82 (br s, 2H), 1.63 - 1.60 (m, 4H), 1.37 (s, 12H), 0.88 - 0.83 (m, 6H).

### Example C1.8: N⁶-(tert-Butoxycarbonyl)-N²-((6-(2-(methylthio)pyrimidine-5-carboxamido)hexanoyl)-L-valyl)-L-lysine (compound C1.8)

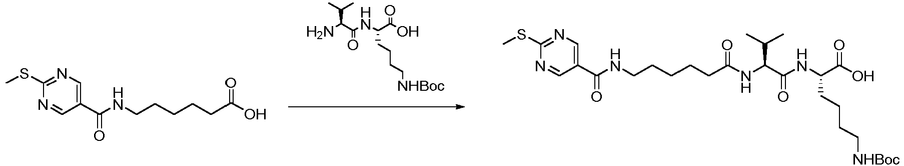

Following the same procedures and reaction conditions as in Example C1.6, the target product *N*⁶-(*tert-*butoxycarbonyl)-*N*²-((6-(2-(methylthio)pyrimidine-5-carboxamido)hexanoyl)-*L*-valyl)-*L*-lysine (compound C1.8) was obtained using the known compounds shown in the reaction formula as starting materials.

ESI-MS (m/z): 611 [M+H]⁺.

### Example C1.9: N⁶-(tert-Butoxycarbonyl)-N²-((6-(4-(2-(methylthio)pyrimidin-5-yl)-1H-1,2,3-triazol-1-yl)hexanoyl)-L-valyl)-L-lysine (compound C1.9)

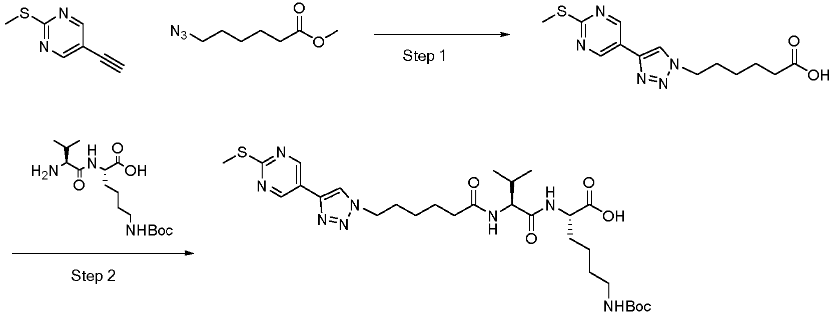

### Step 1: 6-(4-(2-(Methylthio)pyrimidin-5-yl)-1H-1,2,3-triazol-1-yl)hexanoic acid

2-Methylthio-5-ethynylpyrimidine (1.5 g) and methyl 6-azidohexanoate (1.71 g) were dissolved in a mixed solvent of *tert*-butanol and water (20 mL/25 mL) at room temperature, and sodium ascorbate (5.7 g) and cuprous bromide (2.9 g) were added. The mixture was stirred for 10 h. Ethyl acetate (200 mL) was added to the reaction solution, and the mixture was washed with water (100 mL × 5), dried, and then concentrated to remove the organic solvent. The residue was subjected to silica gel column chromatography to obtain methyl 6-(4-(2-(methylthio)pyrimidin-5-yl)-1*H*-1,2,3-triazol-1-yl)hexanoate (2.8 g). ESI-MS (m/z): 321.9 [M+H]⁺. The above product was dissolved in tetrahydrofuran (100 mL), and lithium hydroxide (1 M, 20 mL) was added. The mixture was stirred for 3 h, and then hydrochloric acid was added to adjust the pH of the reaction solution to 2, followed by the addition of ethyl acetate (200 mL) was added. The mixture was washed with water (100 mL × 3), dried, and concentrated to remove the organic solvent to obtain the target product 6-(4-(2-(methylthio)pyrimidin-5-yl)-1*H-*1,2,3-triazol-1-yl)hexanoic acid. ESI-MS (m/z): 308.4 [M+H]⁺.

### Step 2: N⁶-(tert-Butoxycarbonyl)-N²-((6-(4-(2-(methylthio)pyrimidin-5-yl)-1H-1,2,3-triazol-1-yl)hexanoyl)-L-valyl)-L-lysine

Compound 6-(4-(2-(methylthio)pyrimidin-5-yl)-1*H*-1,2,3-triazol-1-yl)hexanoic acid (370 mg) and *N-*hydroxysuccinimide (152 mg) were dissolved in dichloromethane (5 mL) and stirred, and then dicyclohexylcarbodiimide (273 mg) was added. Then, the reaction solution was stirred at room temperature for 1 h. Water (10 mL) was added to the reaction solution, and the mixture was extracted with ethyl acetate (20 mL × 2). The organic phase was washed with saturated brine, dried over anhydrous sodium sulfate, and filtered, and the filtrate was concentrated under reduced pressure to obtain a crude product. This crude product was dissolved in *N,N*-dimethylformamide (10 mL) and stirred, and compound *N*²-*L*-valyl-*N*⁶-(Boc)-*L*-lysine was added. The reaction solution was allowed to react at room temperature for 16 h, and citric acid was slowly added to the reaction solution to adjust the pH to about 5. Water was added, and then the mixture was extracted with ethyl acetate (3 × 10 mL). The organic phase was washed with saturated brine, dried over anhydrous sodium sulfate, and concentrated to remove the ethyl acetate to obtain the target product *N*⁶-(*tert*-butoxycarbonyl)-*N*²-((6-(4-(2-(methylthio)pyrimidin-5-yl)-1*H*-1,2,3-triazol-1-yl)hexanoyl)-*L*-valyl)-*L*-lysine (compound C1.9, 400 mg).
ESI-MS (m/z): 635.3 [M+H]⁺;
¹H NMR (400 MHz, DMSO-d6) δ 12.46 (s, 1H), 9.06 (s, 2H), 9.06 (s, 1H), 8.70 (s, 1H), 8.07 (d, *J* = 7.4 Hz, 1H), 7.77 (d, *J=* 9.0 Hz, 1H), 6.76 (t, *J* = 5.6 Hz, 1H), 4.41 (t, *J* = 7.0 Hz, 2H), 4.19 (dd, *J* = 8.9, 7.0 Hz, 1H), 4.14 - 4.05 (m, 1H), 2.92 - 2.85 (m, 2H), 2.56 (s, 3H), 2.24 - 2.10 (m, 2H), 1.97 - 1.81 (m, 3H), 1.71 - 1.64 (m, 1H), 1.61 - 1.50 (m, 3H), 1.36 (s, 9H), 1.28 - 1.22 (m, 6H), 0.88 - 0.76 (m, 6H).

### Example C1.10: (6-(2-(Methylthio)pyrimidin-5-yl)hex-(5-yn)-oyl)glycylglycyl-L-phenylalanine (compound C1.10)

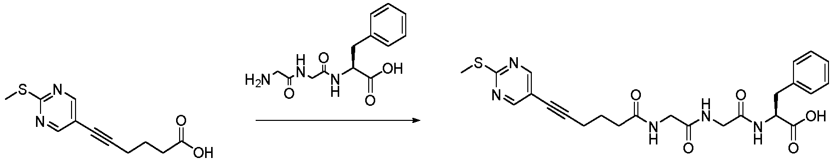

Following the same procedures and reaction conditions as in Example C1.6, the target product (6-(2-(methylthio)pyrimidin-5-yl)hex-5-ynoyl)glycylglycyl-*L*-phenylalanine (compound C1.10) was obtained using the known starting materials shown in the above reaction formula. ESI-MS (m/z): 498 [M+H]⁺.

Following the same procedures and reaction conditions as in Example C1.6, the target products listed in the table below were obtained using different reaction starting materials.

| Compound | Chemical structure | Chemical name | ESI-MS (*m*/*z*): [M + H]⁺ |
|---|---|---|---|
| C1.11 | 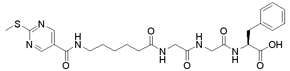 | (6-(2-(Methylthio)pyrimidine-5-carboxamido)hexanoyl)glycylglycyl-*L*-phenylalanine | 545 |
| C1.12 | 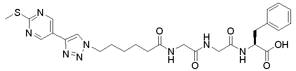 | (6-(4-(2-(Methylthio)pyrimidin-5-yl)-1*H*-1,2,3-triazol-1-yl)hexanoyl)glycylglycyl-*L-*phenylalanine | 569 |

Example C1.13: (29-(4-(2-(Methylthio)pyrimidin-5-yl)-1*H*-1,2,3-triazol-1-yl)-3,6,9,12,15,18,21,24,27-nonaoxa-29-alkanoyl)glycylglycyl-*L*-phenylalanine (compound C1.13)

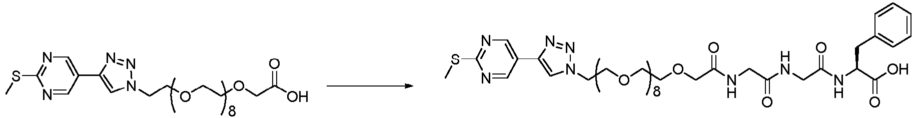

Following the same procedures and reaction conditions as in step 4 of Example C1.1, the target product (29-(4-(2-(methylthio)pyrimidin-5-yl)-1*H*-1,2,3-triazol-1-yl)-3,6,9,12,15,18,21,24,27-nonaoxa-29-alkanoyl)glycylglycyl-*L*-phenylalanine (compound C1.13) was obtained using known starting materials.

ESI-MS (m/z): 909 [M+H]⁺.

### Example C1.14: N⁶-(tert-Butoxycarbonyl)-N²-((39-(2-(methylthio)pyrimidin-5-yl)-5,34-dioxo-3,9,12,15,18,21, 24,27,30-nonaoxa-6,33-diazanonatriacontan-38-ynoyl)-L-valyl)-L-lysine (C1.14)

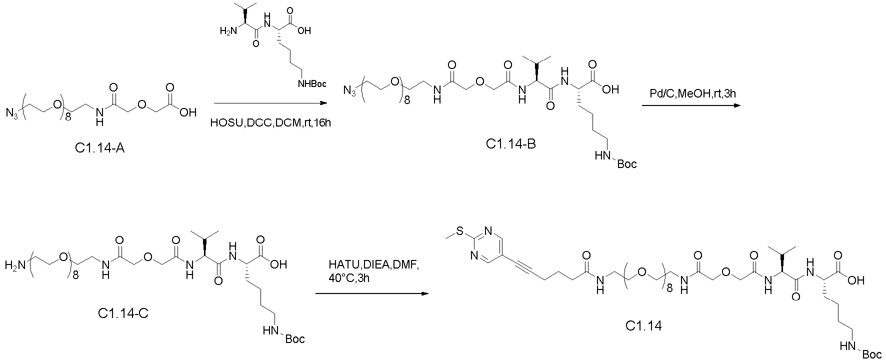

### Step 1:

Compound C1.14-A (336 mg) was dissolved in dichloromethane (5 mL), and N-hydroxysuccinimide (98 mg, 0.85 mmol) and dicyclohexylcarbodiimide (175 mg, 0.85 mmol) were added. The mixture was stirred at room temperature for 1 h, and then compound *N*²-*L*-valyl-*N*⁶-(Boc)-*L*-lysine (230 mg) was added to the reaction solution. The mixture was stirred at room temperature overnight and concentrated to remove the solvent to obtain a crude product. The crude product was separated and purified by reversed-phase chromatography using a C18 column (acetonitrile/0.01% FA in water: 5%-50%) to obtain the target compound (C1.14B, 290 mg) as a colorless oil.

LCMS (ESI) [M+H]⁺ = 882.3.

### Step 2:

Compound C1.14-B (300 mg) was dissolved in anhydrous methanol (5 mL), and Pd/C (10%, 60 mg) was added. The mixture was purged three times with hydrogen, stirred at room temperature overnight, filtered under vacuum, and concentrated to obtain the target compound C1.14-C (293 mg) as a colorless oil.

LCMS (ESI) [M+H]⁺: 856.1.

### Step 3:

6-(2-(Methylthio)pyrimidin-5-yl)hex-5-ynoic acid (84 mg) was dissolved in *N,N*-dimethylformamide (3 mL), and then *N,N*-diisopropylethylamine (61 mg) and HATU (108 mg) were added sequentially. The reaction solution was stirred at room temperature for 20 min, then compound C1.14-C (196 mg) was added, and the mixture was stirred at 40 °C for 3 h. The crude product was separated and purified by reversed-phase chromatography using a C18 column (acetonitrile/0.01% FA in water: 5%-65%) to obtain the target compound *N*⁶-(*tert*-butoxycarbonyl)-*N*²-((39-(2-(methylthio)pyrimidin-5-yl)-5,34-dioxo-3,9,12,15,18,21,24,27,30-nonaoxa-6,33-diazanonatriacontan-38-ynoyl)-*L*-valyl)-*L*-lysine (C1.14) (170 mg).
LCMS (ESI) [M+H]⁺: 1074.0;
¹H NMR (400 MHz, DMSO-d6) δ 8.68 (s, 2H), 8.23 (t, *J* = 5.7 Hz, 1H), 8.13 (d, *J* = 8.9 Hz, 1H), 7.99 (t, *J=* 5.6 Hz, 1H), 7.43 (d, J= 6.5 Hz, 1H), 6.69 (s, 1H), 4.09 (dd, *J* = 8.8, 6.5 Hz, 1H), 4.02 (s, 2H), 3.98 (d, J= 2.9 Hz, 2H), 3.74 - 3.70 (m, 1H), 3.50 (s, 30H), 3.45 (d, *J* = 6.2 Hz, 2H), 3.39 (d, *J* = 5.8 Hz, 2H), 3.26 (d, *J* = 6.1 Hz, 2H), 3.20 (q, *J* = 5.9 Hz, 2H), 2.81 (t, *J* = 6.6 Hz, 2H), 2.52 (s, 3H), 2.24 (t, *J* = 7.4 Hz, 2H), 2.12 - 2.06 (m, 1H), 1.79 - 1.74 (m, 2H), 1.67 - 1.59 (m, 1H), 1.50 (d, *J* = 5.3 Hz, 1H), 1.36 (s, 9H), 1.24 (s, 2H), 1.20 - 1.09 (m, 2H), 0.85 (t, *J =* 6.3 Hz, 6H).

### Example C1.15: N⁶-(tert-Butoxycarbonyl)-N²-((32-(4-(2-(methylthio)pyrimidin-5-yl)-1H-1,2,3-triazol-1-yl)-5-oxo-3,9,12,15,18,21,24,27,30-nonaoxa-6-azadotriacontanoyl)-L-valyl)-L-lysine (C1.15)

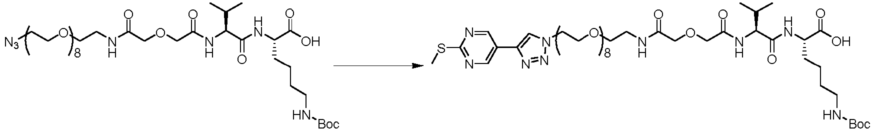

Compound C1.14-B (130 mg), 5-ethynyl-2-methylthiopyrimidine (44 mg), sodium ascorbate (3 mg), and copper sulfate (5 mg) were added to a mixed solution of *tert*-butanol (2 mL) and water (2 mL), and the reaction mixture was allowed to react under a nitrogen atmosphere at room temperature for 3 h. Water (10 mL) was added to the reaction solution, and the mixture was extracted with dichloromethane (30 mL × 3). The organic phase was washed with saturated brine, dried over anhydrous sodium sulfate, filtered under vacuum, and concentrated to obtain a crude product. The crude product was separated and purified by TLC (dichloromethane:methanol = 10:1) to obtain the target compound *N*⁶-(*tert*-butoxycarbonyl)-*N*²-((32-(4-(2-(methylthio)pyrimidin-5-yl)-1*H*-1,2,3-triazol-1-yl)-5-oxo-3,9,12,15,18,21,24,27,30-nonaoxa-6-azadotriacontanoyl)-*L*-valyl)-*L*-lysine (C1.15) (90 mg).

LCMS (ESI) [M+H]⁺ = 1032.3.

### Example C1.16: N⁶,N⁶-Dimethyl-N²-((6-(2-(methylthio)pyrimidin-5-yl)hex-5-ynoyl)-L-valyl)-L-lysine

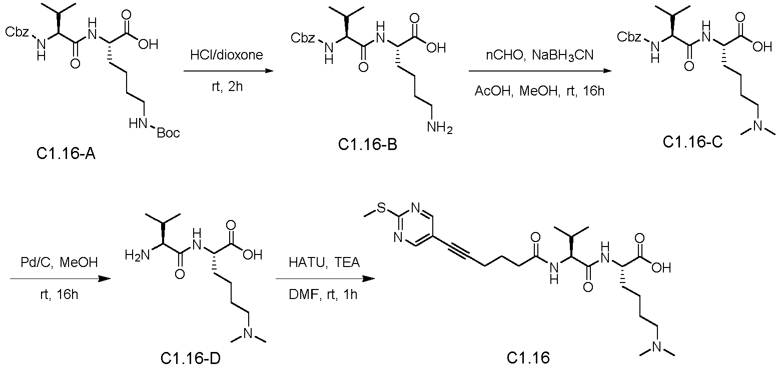

### Step 1:

A solution of hydrogen chloride in dioxane (100 mL) was added to compound C1.16-A (10.0 g), and the mixture was allowed to react at room temperature for 2 h. The reaction solution was concentrated under reduced pressure to obtain the target compound C1.16-B (8.0 g) as a white solid.

LCMS (ESI) [M+H]⁺: 380.1.

¹H NMR (400 MHz, DMSO) δ 8.18 (d, *J* = 7.4 Hz, 1H), 7.40 - 7.30 (m, 5H), 7.26 (d, *J* = 8.8 Hz, 1H), 5.08 - 4.99 (m, 2H), 4.23 - 4.11 (m, 1H), 3.94 - 3.88 (m, 1H), 2.78 - 2.73 (m, 2H), 2.03 - 1.94 (m, 1H), 1.77 - 1.51 (m, 4H), 1.44 - 1.31 (m, 2H), 0.87 (dd, *J* = 17.3, 6.6 Hz, 6H).

### Step 2:

Compound C1.16-B (3.0 g) and sodium acetate (1.90 g) were dissolved in a methanol solution (100 mL), and the resulting solution was allowed to react at room temperature for 10 min. Paraformaldehyde (2.8 g) was added to the reaction solution, and the mixture was stirred at room temperature for 30 min. Sodium cyanoborohydride (1.0 g) was further added to the reaction solution, and the reaction mixture was stirred at room temperature for 16 h. The reaction solution was filtered, and then the filtrate was separated and purified by reversed-phase chromatography using a C18 column (acetonitrile:0.05% aqueous formic acid solution: 5% to 55%) to obtain the target compound C1.16-C (1.70 g).
LCMS (ESI) [M+H]⁺: 408.1;
¹H NMR (400 MHz, DMSO) δ 7.86 (d, *J* = 7.3 Hz, 1H), 7.40 - 7.26 (m, 6H), 5.03 (s, 2H), 4.08 - 4.06 (m, 1H), 3.90 - 3.85 (m, 1H), 2.50 - 2.45 (m, 2H), 2.35 (s, 6H), 2.05 - 1.93 (m, 1H), 1.73 - 1.55 (m, 2H), 1.51 - 1.40 (m, 2H), 1.33 - 1.22 (m, 2H), 0.85 (dd, *J* = 16.5, 6.8 Hz, 6H).

### Step 3:

Compound C1.16-C (1.6 g) was dissolved in methanol (80 mL) at room temperature, and then Pd/C (10%, 0.16 g) was added to the reaction solution. The mixture was stirred under a hydrogen atmosphere at room temperature for 12 h. The reaction solution was filtered, and the filtrate was concentrated under reduced pressure to obtain the target compound C1.16-D (680 mg).

LCMS (ESI) [M+H]⁺: 274.2.

### Step 4:

6-(2-(Methylthio)pyrimidin-5-yl)hex-5-ynoic acid (944 mg) was dissolved in *N,N-*dimethylformamide (30 mL), and then N,N-diisopropylethylamine (1.3 g) and *O*-(7-benzotriazol-1-yl)-*N,N,N',N'*-tetramethyluronium hexafluorophosphate (HBTU, 1.8 g) were added sequentially. The reaction solution was stirred at room temperature for 20 min, then compound C1.16-D (1.1 g) was added, and the mixture was stirred at 40 °C for 3 h. The crude product was separated and purified by reversed-phase chromatography using a C18 column (acetonitrile/0.01% aqueous formic acid: 5%-65%) to obtain the target compound *N*⁶,*N*⁶-dimethyl-*N*²-((6-(2-(methylthio)pyrimidin-5-yl)hex-5-ynoyl)-*L*-valyl)-*L*-lysine (C1.16) (1.2 g) as a white solid.
LCMS (ESI) [M+H]⁺: 492.1;
¹H NMR (400 MHz, DMSO) δ 8.68 (s, 2H), 8.00 (d, *J* = 7.6 Hz, 1H), 7.91 (d, *J* = 9.0 Hz, 1H), 4.20 (t, 1H), 4.08 (dd, *J =* 12.7, 7.7 Hz, 1H), 2.47 - 2.40 (m, 4H), 2.37 - 2.31 (m, 2H), 2.30 (s, 6H), 2.01 - 1.92 (m, 1H), 1.82 - 1.73 (m, 2H), 1.71 - 1.56 (m, 2H), 1.49 - 1.37 (m, 2H), 1.33 - 1.23 (m, 2H), 0.88 - 0.82 (m, 6H).

### Example C1.17: N⁶,N⁶-Dimethyl-N²-((6-(2-(methylsulfonyl)pyrimidin-5-yl)hex-5-ynoyl)-L-valyl)-L-lysine (C1.17)

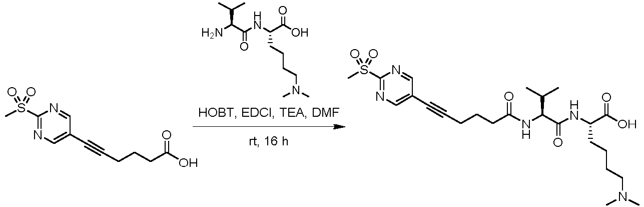

6-(2-(Methylsulfonyl)pyrimidin-5-yl)hex-5-ynoic acid (268 mg), compound C1.16-D (328 mg), and triethylamine (322 mg) were dissolved in *N,N*-dimethylformamide (5 mL). 1-Hydroxybenzotriazole (HOBT, 162 mg) and 1-(3-dimethylaminopropyl)-3-ethylcarbodiimide hydrochloride (EDCI, 229 mg) were then added,

and the reaction solution was stirred at room temperature for 16 h and then directly purified by reversed-phase chromatography using a C18 column (acetonitrile and 0.05% aqueous formic acid solution system) to obtain the target compound *N*⁶,*N*⁶-dimethyl-*N*²-((6-(2-(methylsulfonyl)pyrimidin-5-yl)hex-5-ynoyl)-*L*-valyl)-*L*-lysine (C1.17, white solid, 327 mg).

LCMS (ESI) [M+H]⁺: 524.4.

¹H NMR (400 MHz, DMSO) δ 9.13 (s, 2H), 7.95 (t, *J* = 8.8 Hz, 2H), 4.21 (dd, *J* = 8.8, 6.9 Hz, 1H), 4.08 - 4.03 (m, 1H), 3.41 (s, 3H), 2.55 (t, *J =* 7.0 Hz, 2H), 2.42 - 2.32 (m, 4H), 2.27 (s, 6H), 1.98 (dd, *J* = 13.6, 6.8 Hz, 1H), 1.86 - 1.77 (m, 2H), 1.74 - 1.55 (m, 2H), 1.47 - 1.37 (m, 2H), 1.31 - 1.23 (m, 2H), 0.85 (dd, *J* = 12.8, 6.8 Hz, 6H).

### Example C1.18: N²-(tert-Butoxycarbonyl)-N⁶-((6-(2-(methylsulfonyl)pyrimidin-5-yl)hex-5-ynoyl)-L-valyl)-L-lysine

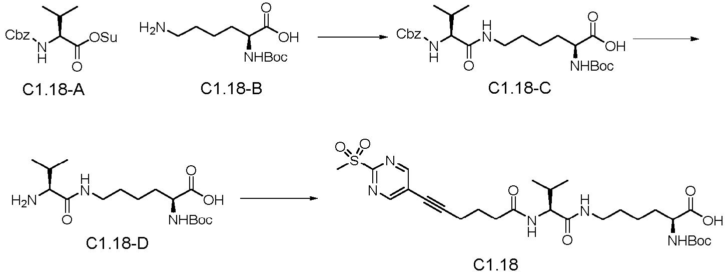

### Step 1:

Compound C1.18-B (3 g) was dissolved in dichloromethane (30 mL), and DIPEA (4 mL) was added, followed by the addition of compound C1.18-A (3.48 g). The mixture was stirred at room temperature for 20 h. Ethyl acetate (200 mL) was added to the reaction solution, and the mixture was washed with hydrochloric acid (0.1 M, 30 mL × 3) and water (30 mL × 3) sequentially, dried over anhydrous sodium sulfate, and concentrated under reduced pressure to obtain the target mixture C1.18-C (4.7 g).

### Step 2:

Compound C1.18-C (4.7 g) was dissolved in methanol (80 mL) at room temperature, and then Pd/C (10%, 0.6 g) was added to the reaction solution. The mixture was stirred under a hydrogen atmosphere at room temperature for 12 h. The reaction solution was filtered, and the filtrate was concentrated under reduced pressure to obtain the target compound C1.18-D (3.4 g). LCMS (ESI) [M+H]⁺: 346.2.

### Step 3:

6-(2-(Methylsulfonyl)pyrimidin-5-yl)hex-5-ynoic acid (2.68 g) was dissolved in N,N-dimethylformamide (50 mL), and then triethylamine (3 mL) and HBTU (3.8 g) were added. The mixture was stirred at room temperature for 10 min, then ethyl acetate (200 mL) was added, and the resulting mixture was washed with saturated sodium bicarbonate (20 mL × 2), hydrochloric acid (0.1 M, 50 mL × 2) and water (50 mL × 2) sequentially, dried over anhydrous sodium sulfate, and concentrated under reduced pressure to obtain a crude product of the intermediate. The crude product was then dissolved in DMF (30 mL), and DIPEA (1.6 mL) was added, followed by the addition of C1.18-D (3.4 g). The reaction solution was stirred at room temperature for 3 h, then ethyl acetate (200 mL) was added, and the resulting mixture was washed with hydrochloric acid (0.1 M, 50 mL × 2) and water (50 mL × 2) sequentially, dried over anhydrous sodium sulfate, and concentrated under reduced pressure to obtain a crude product. The crude product was separated and purified by silica gel column chromatography to obtain the target compound *N*²-(*tert*-butoxycarbonyl)-*N*⁶-((6-(2-(methylsulfonyl)pyrimidin-5-yl)hex-5-ynoyl)-*L*-valyl)-*L*-lysine (C1.18, white solid, 3.8 g).

LCMS (ESI) [M+H]⁺: 596.4.

### Example C1.19: N⁶,N⁶-Dimethyl-N²-((6-(4-(2-(methylsulfonyl)pyrimidin-5-yl)-1H-1,2,3-triazol-1-yl)hexanoyl)-L-valyl)-L-lysine

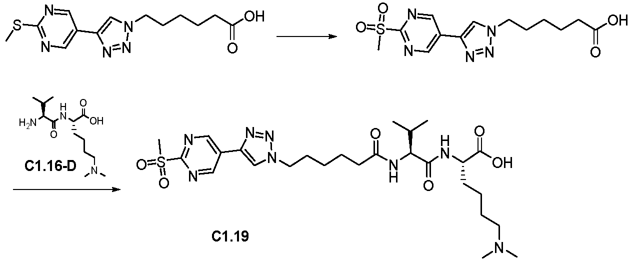

### Step 1:

Compound 6-(4-(2-(methylthio)pyrimidin-5-yl)-1*H*-1,2,3-triazol-1-yl)hexanoic acid (1.0 g, 3.3 mmol) was dissolved in a mixed solution of tetrahydrofuran and water (40 mL, 3:1), and potassium peroxymonosulfonate (10.0 g, 16.3 mmol) was added. The mixture was stirred at room temperature for 3 h. The reaction was completed as detected by LCMS. The reaction solution was filtered, the filter cake was washed with DMSO, and the filtrates were combined and purified by reversed-phase chromatography (C18, acetonitrile:0.1% formic acid = 5%-55%) to obtain 6-(4-(2-(methylsulfonyl)pyrimidin-5-yl)-1*H*-1,2,3-triazol-1-yl)hexanoic acid (750 mg, yield: 67.9%) as a white solid.
LCMS (ESI) [M+H] ⁺ = 340.1;
¹H NMR (400 MHz, DMSO-d6) δ 9.48 (s, 2H), 8.95 (s, 1H), 4.49 (t, *J* = 7.0 Hz, 2H), 3.45 (s, 3H), 2.22 (t, *J =* 7.3 Hz, 2H), 1.95 - 1.84 (m, 2H), 1.61 - 1.50 (m, 2H), 1.36 - 1.26 (m, 2H).

### Step 2:

Compound 6-(4-(2-(methylsulfonyl)pyrimidin-5-yl)-1*H*-1,2,3-triazol-1-yl)hexanoic acid (300 mg, 0.88 mmol) was dissolved in *N,N*-dimethylformamide (6 mL), and HATU (337 mg, 0.88 mmol) and DIPEA (286 mg, 2.21 mmol) were added. The mixture was stirred at room temperature for 30 min, then dipeptide C1.16-D (243 mg, 0.88 mmol) was added, and the resulting mixture was stirred at room temperature for 2 h. The reaction was completed as detected by LCMS. The reaction solution was separated and purified using a C18 column (acetonitrile/0.01% FA aqueous solution: 5%-50%) to obtain the target compound *N*⁶,*N*⁶-dimethyl-*N*²-((6-(4-(2-(methylsulfonyl)pyrimidin-5-yl)-1H-1,2,3-triazol-1-yl)hexanoyl)-L-valyl)-L-lysine (300 mg, yield: 57%) as a white solid.

LCMS (ESI) [M+H]⁺ = 595.5, t_{R} = 2.024 min.

¹H NMR (400 MHz, DMSO-d6) δ 9.49 (s, 2H), 9.01 (s, 1H), 7.85 (br s, 1H), 7.84 (d, *J* = 8.9 Hz, 1H), 4.48 (t, *J =* 6.8 Hz, 2H), 4.17 - 4.15 (m, 1H), 4.02 (br s, 1H), 3.44 (s, 3H), 2.42 (br s, 2H), 2.30 (s, 6H), 2.18 - 2.14 (m, 2H), 1.99 - 1.96 (m, 1H), 1.88 (dd, *J* = 14.6, 7.1 Hz, 2H), 1.67 (br s, 1H), 1.59 - 1.53 (m, 2H), 1.43 (br s, 2H), 1.28 - 1.26 (m, 5H), 0.83 - 0.89 (m, 6H).

### Example C1.20: N⁶,N⁶-Diethyl-N²-((6-(2-(methylsulfonyl)pyrimidin-5-yl)hex-5-ynoyl)-L-valyl)-L-lysine

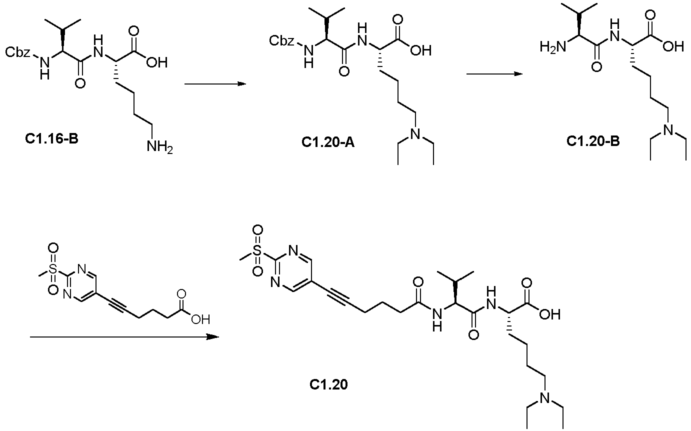

### Step 1:

Compound C1.16-B (5.0 g, 12.05 mmol) was dissolved in dichloromethane (100 mL), and acetaldehyde (3.2 g, 72.3 mmol) was added to the reaction solution. The mixture was stirred at room temperature for 10 min, and then sodium triacetoxyborohydride (12.8 g, 60.25 mmol) was added to the reaction solution. The reaction mixture was stirred at room temperature for 1 h. The reaction was completed as detected by LCMS. A saturated aqueous ammonium chloride solution was added to the reaction solution, and the mixture was stirred for one hour, concentrated to dryness by rotary evaporation, and filtered. The filtrate was then separated and purified by reversed-phase chromatography using a C18 column (acetonitrile:0.05% aqueous formic acid solution: 5% to 55%) to obtain the target compound C1.20-A (4.57 g, yield: 82.0%) as a white solid.

LCMS (ESI) [M+H]⁺ = 436.4;

¹H NMR (400 MHz, DMSO-d6) δ 7.70 (d, *J* = 7.0 Hz, 1H), 7.41 (d, *J* = 9.0 Hz, 1H), 7.38 - 7.26 (m, 5H), 5.08 - 4.99 (m, 2H), 4.00 (dd, *J* = 12.6, 6.5 Hz, 1H), 3.86 (dd, *J* = 8.6, 6.8 Hz, 1H), 2.74 (dd, *J* = 14.0, 6.9 Hz, 4H), 2.64 - 2.54 (m, 2H), 2.05 - 1.94 (m, 1H), 1.72 - 1.52 (m, 2H), 1.52 - 1.38 (m, 2H), 1.38 - 1.18 (m, 2H), 1.04 (t, *J* = 7.1 Hz, 6H), 0.87 - 0.81 (m, 6H).

### Step 2:

Compound C1.20-A (1.6 g, 3.68 mmol) was dissolved in methanol (80 mL) at room temperature, and then Pd/C (0.16 g) was added to the reaction solution. The mixture was stirred under a hydrogen atmosphere at room temperature for 12 h. The reaction was completed as detected by LCMS. The reaction solution was filtered, and the filtrate was concentrated under reduced pressure to obtain the target compound C1.20-B (900 mg, yield: 82%) as a creamy-white solid.

¹H NMR (400 MHz, DMSO-d6) δ 8.04 (br s, 1H), 4.02 - 3.99 (m, 1H), 3.10 (d, *J* = 4.5 Hz, 1H), 2.65 (q, *J* = 7.1 Hz, 4H), 2.55 - 2.51 (m, 2H), 2.06 - 1.93 (m, 1H), 1.73 - 1.54 (m, 2H), 1.47 - 1.38 (m, 2H), 1.30 - 1.21 (m, 2H), 1.01 (t, *J =* 7.1 Hz, 6H), 0.89 (d, *J =* 6.9 Hz, 3H), 0.79 (d, *J* = 6.8 Hz, 3H).

### Step 3:

Compound 6-(2-(methylsulfonyl)pyrimidin-5-yl)hex-5-ynoic acid (268 mg, 1 mmol) was dissolved in DMF (8 mL), and HATU (380 mg, 1 mmol) and triethylamine (322 mg, 2.5 mmol) were added sequentially. The mixture was stirred at room temperature for 20 min, and then compound C1.20-B (301 mg, 1 mmol) was added. The mixture was stirred at room temperature for another 30 min. After the reaction was completed as detected by LCMS, the reaction solution was directly purified by reversed-phase chromatography using a C18 column (acetonitrile and 0.05% aqueous formic acid solution system) to obtain the target compound (280 mg, yield: 51%) as a white solid.

LCMS (ESI) [M+H]⁺ = 552.3;

¹H NMR (400 MHz, DMSO-d6) δ 9.13 (s, 2H), 7.95 (d, *J* = 8.9 Hz, 1H), 7.86 (d, *J* = 7.2 Hz, 1H), 4.18 (dd, *J =* 8.8, 6.8 Hz, 1H), 4.02 (dd, *J* = 12.8, 7.2 Hz, 1H), 3.41 (s, 3H), 2.74 - 2.69 (m, 4H), 2.62 - 2.52 (m, 4H), 2.44 - 2.29 (m, 2H), 2.04 - 1.94 (m, 1H), 1.86 - 1.77 (m, 2H), 1.72 - 1.54 (m, 2H), 1.51 - 1.39 (m, 2H), 1.33 - 1.23 (m, 2H), 1.02 (t, *J* = 7.2 Hz, 6H), 0.87 - 0.82 (m, 6H).

### Example C1.21: N⁶,N⁶-Diethyl-N²-((6-(4-(2-(methylsulfonyl)pyrimidin-5-yl)-1H-1,2,3-triazol-1-yl)hexanoyl)-L-valyl)-L-lysine

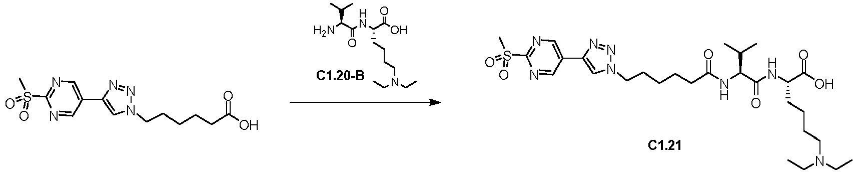

Compound 6-(4-(2-(methylsulfonyl)pyrimidin-5-yl)-1*H*-1,2,3-triazol-1-yl)hexanoic acid (500 mg, 1.475 mmol) was dissolved in *N,N*-dimethylformamide (10 mL), and then HATU (560 mg, 1.475 mmol) and *N,N-*diisopropylethylamine (476 mg, 3.688 mmol) were added. The mixture was stirred for 30 min, and then compound C1.20-B (444 mg, 1.475 mmol) was added, The reaction solution was stirred at room temperature for 1 h. After the reaction was completed as detected by LCMS, the reaction solution was directly purified by reversed-phase chromatography using a C18 column (acetonitrile and 0.05% aqueous formic acid solution system) to obtain the target compound *N*⁶,*N*⁶-diethyl-*N*²-((6-(4-(2-(methylsulfonyl)pyrimidin-5-yl)-1*H*-1,2,3-triazol-1-yl)hexanoyl)-*L-*valyl)-*L*-lysine (330 mg, yield: 34%) as a white solid.

LCMS (ESI) [M+H]⁺ = 623.4;

¹H NMR (400 MHz, DMSO) δ 9.49 (s, 2H), 9.04 (s, 1H), 7.84 (d, *J* = 8.9 Hz, 1H), 7.80 (d, *J =* 7.1 Hz, 1H), 4.47 (t, *J* = 7.0 Hz, 2H), 4.14 (dd, *J* = 8.8, 6.6 Hz, 1H), 4.06 - 3.95 (m, 1H), 3.44 (s, 3H), 2.67 - 2.64 (m, 4H), 2.55 (t, *J* = 7.4 Hz, 2H), 2.21 - 2.10 (m, 2H), 2.02 - 1.94 (m, 1H), 1.92 - 1.84 (m, 2H), 1.72 - 1.63 (m, 1H), 1.72 - 1.63 (m, 3H), 1.59 - 1.54 (m, 2H), 1.32 - 1.21 (m, 4H), 1.01 (t, *J* = 7.1 Hz, 6H), 0.81 (dd, *J* = 9.6, 6.8 Hz, 6H).

### Example C1.22: N⁶,N⁶-Dipropyl-N²-((6-(2-(methylsulfonyl)pyrimidin-5-yl)hex-5-ynoyl)-L-valyl)-L-lysine

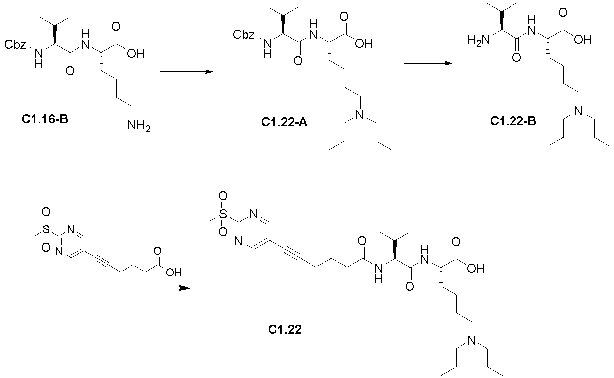

### Step 1:

Compound C1.16-B (5.0 g, 12 mmol) was dissolved in dichloromethane (100 mL), and *n-*propionaldehyde (4.2 g, 72.3 mmol) was added to the reaction solution. The mixture was stirred at room temperature for 10 min, and then sodium triacetoxyborohydride (12.8 g, 60.25 mmol) was added to the reaction solution. The reaction mixture was stirred at room temperature for 1 h. The reaction was completed as detected by LCMS. A saturated aqueous ammonium chloride solution was added to the reaction solution, and the mixture was stirred for one hour, concentrated to dryness by rotary evaporation, and filtered. The filtrate was then separated and purified by reversed-phase chromatography using a C18 column (acetonitrile:0.05% aqueous formic acid solution: 5% to 55%) to obtain the target compound C1.22-A (4.57 g, yield: 82.0%) as a white solid.
LCMS (ESI) [M+H]⁺ = 464.0;
¹H NMR (400 MHz, DMSO-d6) δ 7.81 (d, *J =* 7.3 Hz, 1H), 7.38 - 7.28 (m, 5H), 5.04 (d, *J* = 1.7 Hz, 2H), 4.12 - 4.02 (m, 1H), 3.94 - 3.82 (m, 1H), 2.65 - 2.52 (m, 6H), 2.06 - 1.94 (m, 1H), 1.76 - 1.64 (m, 1H), 1.64 - 1.53 (m, 1H), 1.52 - 1.40 (m, 6H), 1.34 - 1.18 (m, 2H), 0.92 - 0.80 (m, 12H).

### Step 2:

Compound C1.22-A (2.0 g, 4.32 mmol) was dissolved in methanol (80 mL) at room temperature, and then Pd/C (0.16 g) was added to the reaction solution. The mixture was stirred under a hydrogen atmosphere at room temperature for 12 h. The reaction was completed as detected by LCMS. The reaction solution was filtered, and the filtrate was concentrated under reduced pressure to obtain the target compound C1.22-B (1.2 g, yield: 85.5%) as a white solid.

### Step 3:

Compound 6-(2-(methylsulfonyl)pyrimidin-5-yl)hex-5-ynoic acid (100 mg, 0.373 mmol) was dissolved in N,N-dimethylformamide (1 mL), and then HATU (142 mg, 0.373 mmol) and *N,N*-diisopropylethylamine (120 mg, 0.93 mmol) were added. The system was stirred for 30 min, and then compound C1.22-B (122 mg, 0.371 mmol) was added. The reaction solution was stirred at room temperature for 1 h. After the reaction was completed as detected by LCMS, the reaction solution was directly purified by reversed-phase chromatography using a C18 column (acetonitrile and 0.05% aqueous formic acid solution system) to obtain the target compound *N*⁶,*N*⁶-dipropyl-*N*²-((6-(2-(methylsulfonyl)pyrimidin-5-yl)hex-5-ynoyl)-*L*-valyl)-*L*-lysine (50 mg, yield: 28%) as a pale yellow solid.
LCMS (ESI) [M+H]⁺ = 580.0;
¹H NMR (400 MHz, DMSO-d6) δ 8.24 (s, 2H), 7.98 - 7.93 (m, 2H), 4.24 - 4.16 (m, 1H), 4.10 (d*, J* = 5.2 Hz, 1H), 3.41 (s, 3H), 2.79 - 2.64 (m, 6H), 2.55 (t, *J* = 7.1 Hz, 2H), 2.45 - 2.26 (m, 2H), 2.06 - 1.91 (m, 1H), 1.89 - 1.78 (m, 2H), 1.76 - 1.66 (m, 1H), 1.64 - 1.57 (m, 1H), 1.57 - 1.42 (m, 6H), 1.37 - 1.24 (m, 2H), 0.93 - 0.78 (m, 12H).

### Example C1.23: N⁶,N⁶-Dipropyl-N²-((6-(4-(2-(methylsulfonyl)pyrimidin-5-yl)-1H-1,2,3-triazol-1-yl)hexanoyl)-L-valyl)-L-lysine

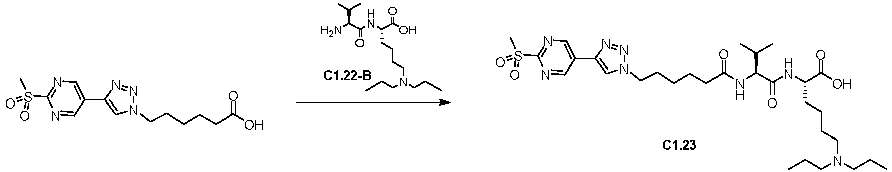

Compound 6-(4-(2-(methylsulfonyl)pyrimidin-5-yl)-1*H*-1,2,3-triazol-1-yl)hexanoic acid (500 mg, 1.475 mmol) was dissolved in *N,N*-dimethylformamide (10 mL), and then HATU (560 mg, 1.475 mmol) and *N,N-*diisopropylethylamine (476 mg, 3.688 mmol) were added. The system was stirred for 30 min, and then compound C1.22-B (485 mg, 1.475 mmol) was added, The reaction solution was stirred at room temperature for 1 h. After the reaction was completed as detected by LCMS, the reaction solution was directly purified by reversed-phase chromatography using a C18 column (acetonitrile and 0.05% aqueous formic acid solution system) to obtain the target compound *N*⁶,*N*⁶-dipropyl-*N*²-((6-(4-(2-(methylsulfonyl)pyrimidin-5-yl)-1*H*-1,2,3-triazol-1-yl)hexanoyl)-*L-*valyl)-*L*-lysine (320 mg, yield: 33%) as a white solid.
LCMS (ESI) [M+H]⁺ = 651.5;
¹H NMR (400 MHz, DMSO) δ 9.49 (s, 2H), 9.00 (s, 1H), 7.95 (d, *J* = 7.2 Hz, 1H), 7.79 (d, *J* = 8.9 Hz, 1H), 4.47 (t, *J =* 6.9 Hz, 2H), 4.21 - 4.11 (m, 1H), 4.09 - 4.01 (m, 1H), 3.44 (s, 3H), 2.45 - 2.35 (m, 6H), 2.23 - 2.09 (m, 2H), 1.97 - 1.86 (m, 3H), 1.72 - 1.64 (m, 1H), 1.61 - 1.50 (m, 3H), 1.43 - 1.34 (m, 6H), 1.32 - 1.22 (m, 4H), 0.86 - 0.77 (m, 12H).

### Example C1.24: tert-Butyl (S)-(6-((4-(hydroxymethyl)phenyl)amino)-5-(6-(2-(methylthio)pyrimidin-5-yl)hex-5-ynamido)-6-oxohexyl)carbamate

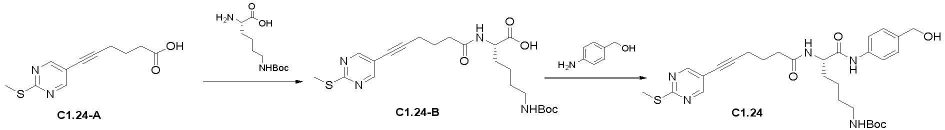

### Step 1:

Compound C1.24-A (3.70 g, 15.7 mmol), diisopropylethylamine (11.0 mL, 62.8 mmol), and HBTU (8.90 g, 23.6 mmol) were dissolved in N,N-dimethylformamide (30 mL), and the reaction solution was allowed to react at room temperature for 30 min. Then, compound Boc-protected lysine (3.86 g, 15.7 mmol) was added, and the reaction mixture was allowed to react at room temperature for 2 h. The reaction was completed as detected by LCMS. An aqueous citric acid solution (30 mL) was added to the reaction solution to adjust the pH to 5, and then the aqueous solution was extracted with dichloromethane (50 mL × 3). The organic phases were combined, dried over anhydrous sodium sulfate, filtered under vacuum, and concentrated under reduced pressure to obtain the target compound C1.24-B (7.0 g, crude product) as a yellow oil. LCMS (ESI) [M+H]⁺ = 465.1, t_{R} = 1.751 min.

### Step 2:

Compound C1.24-B (7.00 g, 5.60 mmol), diisopropylethylamine (10.7 mL, 60.4 mmol), and HBTU (8.60 g, 22.7 mmol) were dissolved in *N,N*-dimethylformamide (100 mL), and then *p*-aminobenzyl alcohol (3.71 g, 30.2 mmol) was added. The reaction mixture was allowed to react at room temperature for 2 h. The reaction was completed as detected by LCMS. Ethyl acetate (300 mL) was added to the reaction solution, and the mixture was washed with saturated brine (100 mL × 3), dried over anhydrous sodium sulfate, and filtered under vacuum. The filtrate was concentrated under reduced pressure to obtain a solid, and the crude product was separated and purified by column chromatography (dichloromethane:methanol = 10/1) to obtain the target compound *tert-*butyl (*S*)-(6-((4-(hydroxymethyl)phenyl)amino)-5-(6-(2-(methylthio)pyrimidin-5-yl)hex-5-ynamido)-6-oxohexyl)carbamate (6.00 g, yield: 69.9%) as a yellow oil.
LCMS (ESI) [M+H]⁺ = 570.1;
¹H NMR (400 MHz, CDCl₃) δ 9.24 (s, 1H), 8.46 (s, 2H), 7.51 - 7.47 (m, 2H), 7.31 - 7.23 (m, 3H), 7.02 - 6.95 (m, 1H), 4.83 (br s, 1H), 4.63 - 4.60 (m, 2H), 3.19 - 2.96 (m, 4H), 2.57 (s, 3H), 2.52 - 2.48 (m, 2H), 2.47 - 2.41 (m, 2H), 1.99 - 1.92 (m, 2H), 1.87 - 1.77 (m, 2H), 1.76 - 1.63 (m, 2H), 1.43 (s, 9H).

### Example C1.25: tert-Butyl (S)-(6-((4-(hydroxymethyl)phenyl)amino)-5-(6-(2-(methylthio)pyrimidin-5-yl)-1H-1,2,3-triazol-1-yl)hexanamido)-6-oxohexyl)carbamate

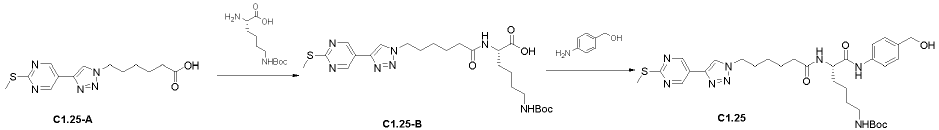

### Step 1:

Compound C1.25-A (1.60 g, 5.21 mmol), diisopropylethylamine (2.68 mL, 20.8 mmol), and HBTU (2.97 g, 7.82 mmol) were dissolved in N,N-dimethylformamide (30 mL), and the reaction solution was allowed to react at room temperature for 30 min. Then, Boc-protected lysine (1.28 g, 5.21 mmol) was added, and the reaction mixture was allowed to react at room temperature for 2 h. The reaction was completed as detected by LCMS. An aqueous citric acid solution (30 mL) was added to the reaction solution to adjust the pH to 5, and then the aqueous solution was extracted with dichloromethane (50 mL × 3). The organic phases were combined, dried over anhydrous sodium sulfate, filtered under vacuum, and concentrated under reduced pressure to obtain the target compound C1.25-B (3.0 g, crude product) as a yellow oil.

LCMS (ESI) [M+H]⁺ = 536.0;

### Step 2:

Compound C1.25-B (3.00 g, 5.60 mmol), diisopropylethylamine (2.89 g, 22.4 mmol), and HBTU (3.19 g, 8.40 mmol) were dissolved in *N,N*-dimethylformamide (30 mL). Then, *p*-aminobenzyl alcohol (1.38 g, 11.2 mmol) was added, and the reaction mixture was allowed to react at room temperature for 2 h. The reaction was completed as detected by LCMS. Ethyl acetate (200 mL) was added to the reaction solution, and the mixture was washed with saturated brine (80 mL × 3), dried over anhydrous sodium sulfate, and filtered under vacuum. The filtrate was concentrated under reduced pressure to obtain a solid, and the crude product was separated and purified by column chromatography (dichloromethane:methanol = 10/1) to obtain the target compound *tert-*butyl (*S*)-(6-((4-(hydroxymethyl)phenyl)amino)-5-(6-(2-(methylthio)pyrimidin-5-yl)-1*H*-1,2,3-triazol-1-yl)hexanamido)-6-oxohexyl)carbamate (2.90 g) as a yellow oil.
LCMS (ESI) [M+H]⁺ = 641.1;
¹H NMR (400 MHz, CDCl₃) δ 9.13 (s, 1H), 8.95 (s, 2H), 7.94 (s, 1H), 7.47 (d, *J* = 8.4 Hz, 2H), 7.24 (d, *J* = 8.4 Hz, 2H), 6.83 - 6.77 (m, 1H), 4.93 - 4.80 (m, 1H), 4.64 - 4.58 (m, 2H), 4.40 - 4.29 (m, 2H), 3.12 - 2.97 (m, 4H), 2.60 (s, 3H), 2.31 - 2.23 (m, 2H), 1.96 - 1.83 (m, 4H), 1.76 - 1.61 (m, 4H), 1.41 (s, 9H), 1.37 - 1.29 (m, 4H).

### Example C1.26: N⁶,N⁶-Dibutyl-N²-((6-(2-(methylsulfonyl)pyrimidin-5-yl)hex-5-ynoyl)-L-valyl)-L-lysine

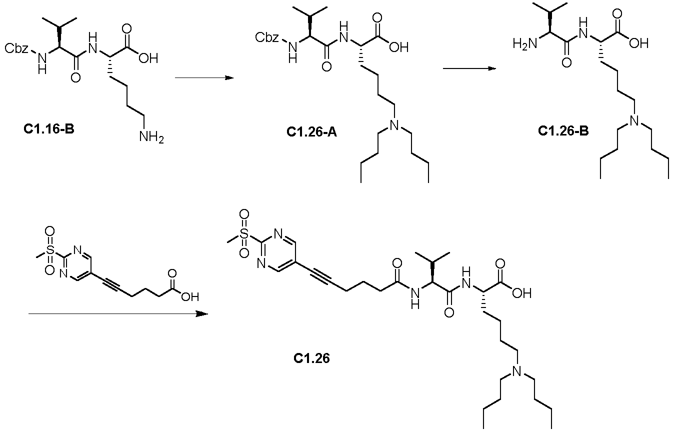

### Step 1:

Compound C1.16-B (10.0 g) was dissolved in a dichloromethane solution (200 mL), and n-butyraldehyde (10.4 g) was added to the reaction solution. The mixture was stirred at room temperature for 10 min, and then sodium triacetoxyborohydride (25.6 g) was added in portions to the reaction solution. The reaction mixture was stirred at room temperature for 1 h. The reaction was completed as detected by LCMS. A saturated aqueous ammonium chloride solution was added to the reaction solution, and the mixture was stirred for one hour, concentrated to dryness by rotary evaporation, and filtered. The filtrate was then separated and purified by reversed-phase chromatography using a C18 column (acetonitrile:0.05% aqueous formic acid solution: 5% to 55%) to obtain the target compound C1.26-A (4.9 g) as a white solid.
LCMS (ESI) [M+H]⁺ = 492.7;
¹H NMR (400 MHz, CDCl3) δ 7.35 - 7.30 (m, 5H), 5.14 - 5.08 (m, 2H), 4.33 - 4.30 (m, 1H), 4.14 - 4.12 (m, 1H), 2.94 - 2.80 (m, 6H), 2.13 - 2.11 (m, 1H), 1.85 - 1.82 (m, 2H), 1.58 - 1.55 (m, 4H), 1.40 - 1.22 (m, 8H), 0.98 - 0.87 (m, 12H).

### Step 2:

Compound C1.26-A (5.0 g) was dissolved in a solution of methanol (100 mL) at room temperature, and then Pd/C (10%, 1 g) was added to the reaction solution. The mixture was stirred under a hydrogen atmosphere for 12 h. The reaction was completed as detected by LCMS. The reaction solution was filtered, and the filtrate was concentrated under reduced pressure to obtain the target compound C1.26-B (3.68 g) as a white solid.

LCMS (ESI) [M+H]⁺ = 358.3.

### Step 3:

Compound 6-(2-(methylsulfonyl)pyrimidin-5-yl)hex-5-ynoic acid (1.0 g) was dissolved in *N,N-*dimethylformamide (15 mL), and then *N,N*-diisopropylethylamine (1.2 g) and HATU (1.4 g) were added sequentially. The reaction mixture was stirred for 30 min, and then compound C1.26-B (1.3 g) was added. The reaction solution was stirred at room temperature for 1 h. After the reaction was completed as detected by LCMS, the reaction solution was directly purified by preparative chromatography (0.01% aqueous trifluoroacetic acid solution, acetonitrile) to obtain the target compound *N*⁶,*N*⁶-dibutyl-*N*²-((6-(2-(methylsulfonyl)pyrimidin-5-yl)hex-5-ynoyl)-L-valyl)-L-lysine (C1.26, 500 mg) as a yellow solid.
LCMS (ESI) [M+H]⁺ = 608.7;
1H NMR (400 MHz, DMSO) δ 9.37 (s, 1H), 9.13 (s, 2H), 8.19 (d, *J* = 7.3 Hz, 1H), 7.92 (d, *J* = 8.8 Hz, 1H), 4.25 - 4.20 (m, 1H), 4.20 - 4.13 (m, 1H), 3.42 (s, 3H), 3.06 - 2.99 (m, 6H), 2.57 - 2.53 (m, 2H), 2.41 - 2.30 (m, 2H), 1.99 - 1.95 (m, 2H), 1.87 - 1.79 (m, 2H), 1.78 - 1.71 (m, 1H), 1.62 - 1.56 (m, 4H), 1.34 - 1.32 (m, 8H), 0.94 - 0.85 (m, 12H).

### Example C1.27: (S)-1-Ethyl-4-(3-methyl-2-(6-(2-(methylsulfonyl)pyrimidin-5-yl)hex-5-ynamido)butanamido)piperidine-4-carboxylic acid

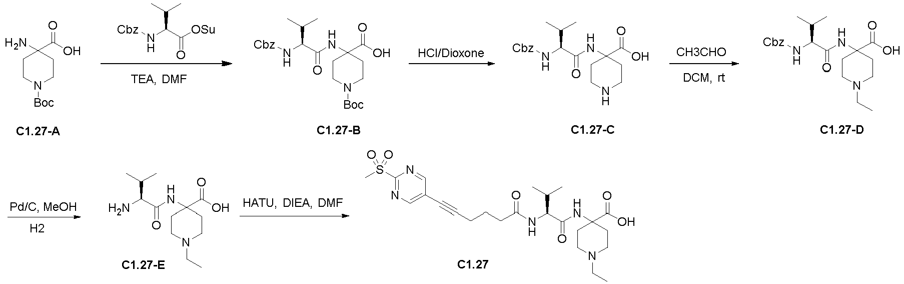

### Step 1:

Compound C1.27-A (5.0 g, 20.49 mmol) was dissolved in DMF (100 mL), and *N*-((benzyloxy)carbonyl)-*L*-valinyl 2,5-dioxopyrrolidine-*N*-hydroxy ester (7.13 g, 20.49 mmol) was added to the reaction solution. The mixture was stirred at 100 °C for 48 h. The reaction was completed as detected by LCMS. The reaction solution was separated and purified by reversed-phase chromatography using a C18 column (acetonitrile:0.05% aqueous formic acid solution: 5% to 55%) to obtain the target compound C1.27-B (1.3 g, yield: 13.3%) as a yellow solid.
LCMS (ESI) [M-boc]⁺ = 378.2;
¹H NMR (400 MHz, DMSO) δ 7.38 - 7.33 (m, 5H), 7.32 - 7.27 (m, 1H), 7.20 (d, *J* = 9.0 Hz, 1H), 5.14 - 5.02 (m, 2H), 3.97 - 3.87 (m, 1H), 3.70 - 3.59 (m, 2H), 3.08 - 2.95 (m, 2H), 2.04 - 1.97 (m, 1H), 1.95 - 1.87 (m, 2H), 1.76 - 1.69 (m, 2H), 1.40 (s, 9H), 0.91 - 0.84 (m, 6H).

### Step 2:

Compound C1.27-B (1.3 g, 2.71 mmol) was dissolved in an ethyl acetate solution (10 mL) at room temperature, and then dioxane-HCl (3 N, 10 mL) was added to the reaction solution. The mixture was stirred at room temperature for 1 h. The reaction was completed as detected by LCMS. The reaction solution was concentrated under reduced pressure to obtain the target compound C1.27-C (1.06 g, yield: 94.4%) as a yellow solid.

LCMS (ESI) [M+H]⁺ = 378.4.

### Step 3:

Compound C1.27-C (1.06 g, 2.81 mmol) was dissolved in dichloromethane (10 mL), and acetaldehyde (0.75 g, 16.87 mmol) was added to the reaction solution. The mixture was stirred at room temperature for 10 min, and then sodium triacetoxyborohydride (3 g, 14.05 mmol) was added in portions to the reaction solution. The reaction mixture was stirred at room temperature for 1 h. The reaction was completed as detected by LCMS. A saturated aqueous ammonium chloride solution was added to the reaction solution, and the mixture was stirred for one hour and distilled under reduced pressure to remove the dichloromethane. The residual aqueous phase was separated by reversed-phase chromatography (acetonitrile:0.05% aqueous formic acid solution: 5% to 55%) to obtain the target compound C1.27-D (1.13 g, yield: 99.23%) as a white solid.
LCMS (ESI) [M+H]⁺ = 406.1;
1H NMR (400 MHz, MeOD) δ 7.42 - 7.25 (m, 5H), 5.22 - 5.06 (m, 2H), 3.91 (d, *J =* 7.0 Hz, 1H), 3.52 - 3.36 (m, 2H), 3.25 - 2.93 (m, 4H), 2.66 - 2.54 (m, 1H), 2.47 - 2.36 (m, 1H), 2.27 - 2.14 (m, 2H), 2.13 - 2.06 (m, 1H), 1.35 - 1.29 (m, 4H), 0.98 - 0.90 (m, 6H).

### Step 4:

Compound C1.27-D (1.13 g, 2.78 mmol) was dissolved in a methanol solution (20 mL) at room temperature, and then Pd/C (0.5 g) was added to the reaction solution. The mixture was stirred under a hydrogen atmosphere for 12 h. The reaction was completed as detected by LCMS. The reaction solution was filtered, and the filtrate was concentrated under reduced pressure to obtain the target compound C1.27-E (0.58 g, yield: 76.18%) as a white solid. LCMS (ESI) [M+H]⁺ = 272.4.

### Step 5:

Compound 6-(2-(methylsulfonyl)pyrimidin-5-yl)hex-5-ynoic acid (1 g, 3.73 mmol) was dissolved in *N,N-*dimethylformamide (5 mL), and then *N,N*-diisopropylethylamine (1.2 g, 9.32 mmol) and HATU (1.42 g, 3.73 mmol) were added sequentially. The reaction solution was stirred for 30 min, and then compound C1.27-E (1.01 g, 3.73 mmol) was added. The reaction solution was stirred at room temperature for 1 h. After the reaction was completed as detected by LCMS, the reaction solution was directly purified by preparative chromatography (0.01% aqueous trifluoroacetic acid solution, acetonitrile) to obtain the target compound (S)-1-ethyl-4-(3-methyl-2-(6-(2-(methylsulfonyl)pyrimidin-5-yl)hex-5-ynamido)butanamido)piperidine-4-carboxylic acid (C1.27, 500 mg, yield: 28.7%) as a yellow solid.
LCMS (ESI) [M+H]⁺ = 522.5;
¹H NMR (400 MHz, DMSO) δ 9.12 (s, 2H), 8.08 (s, 1H), 7.88 (d, *J* = 9.0 Hz, 1H), 4.28 - 4.25 (m, 1H), 3.41 (s, 3H), 2.89 - 2.78 (m, 2H), 2.57 - 2.52 (m, 4H), 2.45 - 2.30 (m, 4H), 2.07 - 2.01 (m, 2H), 1.99 - 1.96 (m, 1H), 1.95 - 1.88 (m, 2H), 1.85 - 1.79 (m, 2H), 1.04 (t, *J =* 7.1 Hz, 3H), 0.88 - 0.83 (m, 6H).

### II. Synthesis of Drug-Linker Compounds

### Example 2.1: N-((S)-1-(((S)-6-Amino-1-((2-((((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)methyl)amino)-2-oxoethyl)amino)-1-oxohexan-2-yl)amino)-3-methyl-1-oxobutan-2-yl)-6-(2-(methylsulfonyl)pyrimidin-5-yl)hex-5-ynamide (DL-001)

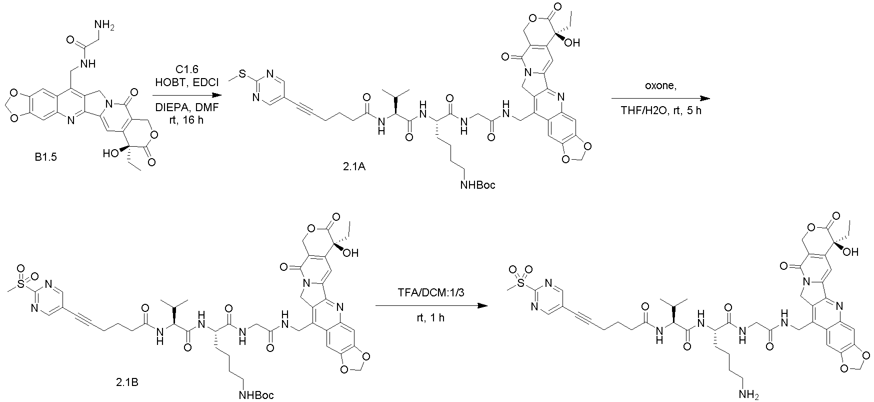

### Step 1:

Compound C1.6 (50 mg, 0.10 mol), compound B1.5 (71 mg, 0.12 mol), and *N,N*-diisopropylethylamine (39 mg, 0.30 mol) were dissolved in DMF (5 mL). Then, 1-hydroxybenzotriazole (16 mg, 0.12 mmol) and 1-(3-dimethylaminopropyl)-3-ethylcarbodiimide hydrochloride (23 mg, 0.12 mmol) were added sequentially, and the reaction solution was stirred at room temperature for 16 h and then diluted with 50 mL of ethyl acetate. The organic phase was washed three times with water (25 mL × 3), dried over anhydrous sodium sulfate, and concentrated to remove the ethyl acetate to obtain a crude product. The crude product was separated and purified by flash silica gel column chromatography (DCM:MeOH = 100:0 to 90:10) to obtain the target compound 2.1A (62 mg, yellow solid, yield: 58%).

LCMS (ESI) [M+H]⁺: 1024.8.

### Step 2:

Compound 2.1A (40 mg, 0.04 mmol) was dissolved in a mixed solution of tetrahydrofuran and water (volume ratio: 1/1; 4 mL), and potassium peroxymonosulfonate (246 mg, 0.4 mmol) was added. The reaction solution was stirred at room temperature for 5 h and then diluted with 30 mL of ethyl acetate. The organic phase was washed three times with water (25 mL × 3), dried over anhydrous sodium sulfate, and concentrated to remove the ethyl acetate to obtain the target compound 2.1B (30 mg, yellow solid, yield: 74%).

LCMS (ESI) [M+H]⁺ = 1055.9.

### Step 3:

Compound 2.1B (30 mg, 0.03 mmol) was dissolved in a mixed solution of trifluoroacetic acid and dichloromethane (volume ratio: 1:3; 4 mL), and the reaction solution was stirred at room temperature for 1 h and then concentrated under reduced pressure to obtain a crude product. The crude product was purified by preparative chromatography (0.01% TFA in water, MeCN) to obtain the target compound *N*-((*S*)-1-(((*S*)-6-amino-1-((2-((((*S*)-7-ethyl-7-hydroxy-8,11-dioxo-7,8,11,13-tetrahydro-10*H*-[1,3]dioxolo[4,5-*g*]pyrano[3',4':6,7]indolizino[1,2-*b*]quinolin-14-yl)methyl)amino)-2-oxoethyl)amino)-1-oxohexan-2-yl)amino)-3-methyl-1-oxobutan-2-yl)-6-(2-(methylsulfonyl)pyrimidin-5-yl)hex-5-ynamide (9 mg, white solid, yield: 33%).
LCMS (ESI) [M+H]⁺: 955.8;
¹H NMR (400 MHz, DMSO-d6) δ 9.09 (s, 2H), 8.54 (s, 1H), 8.15 (m, 2H), 7.84 (m, 1H), 7.80 (s, 1H), 7.64 (br s, 3H), 7.53 (s, 1H), 7.26 (s, 1H), 6.51 (s, 1H), 6.30 (d, *J* = 2.9 Hz, 2H), 5.59 - 5.46 (m, 2H), 5.43 (s, 2H), 4.87 - 4.78 (m, 1H), 4.70 - 4.60 (m, 1H), 4.17 - 4.03 (m, 2H), 3.82 - 3.58 (m, 2H), 3.41 (s, 3H), 2.80 - 2.70 (m, 2H), 2.40 - 2.22 (m, 2H), 1.93 - 1.68 (m, 6H), 1.65 - 1.57 (m, 1H), 1.56 - 1.45 (m, 3H), 1.36 - 1.22 (m, 2H), 0.87 (t, *J =* 7.3 Hz, 3H), 0.80 (d, *J* = 5.2 Hz, 6H).

Following the same procedures and reaction conditions as in Example 2.1, the target products listed in the table below for Examples 2.2 to 2.8 were obtained using different starting materials as shown.

| Example | Compound | Name | (m/z): [M+H]⁺ |
|---|---|---|---|
| Example 2.2 | 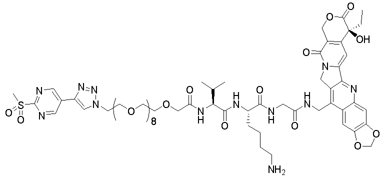 | *N*-((*S*)-1-(((*S*)-6-Amino-1-((2-((((*S*)-7-ethyl-7-hydroxy-8,11-dioxo-7,8,11,13-tetrahydro-10*H-*[1,3]dioxolo[4,5-*g*]pyrano[3',4':6,7]indolizino[1,2-*b*]quinolin-14-yl)methyl)amino)-2-oxoethyl)amino)-1-oxo-hexan-2-yl)amino)-3-methyl-1-oxo-butan-2-yl)-29-(4-(2-(methylsulfonyl)pyrimidin-5-yl)-1*H*-1,2,3-triazol-1-yl)-3,6,9,12,15,18,21,24,27-nonaoxanonacosanamide | 1366.9 |
| | DL-002 | | |
| | Starting materials: C1.2 and B1.5 | | |
| Example 2.3 | 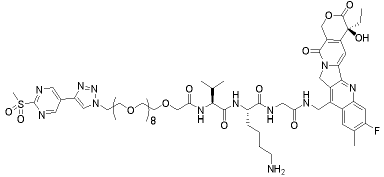 | *N*-((*S*)-1-(((*S*)-6-Amino-1-((2-((((*S*)-4-ethyl-8-fluoro-4-hydroxy-9-methyl-3,14-dioxo-3,4,12,14-tetrahydro-1*H-*pyrano[3',4':6,7]indolizino[1,2-*b*]quinolin-11-yl)methyl)amino)-2-oxoethyl)amino)-1-oxohexan-2-yl)amino)-3-methyl-1-oxobutan-2-yl)-29-(4-(2-(methylsulfonyl)pyrimidin-5-yl)-1*H*-1,2,3-triazol-1-yl)-3,6,9,12,15,18,21,24,27-nonaoxanonacosanamide | 1354.9 |
| | DL-003 | | |
| | Starting materials: C1.2 and B1.4 | | |
| Example 2.4 | 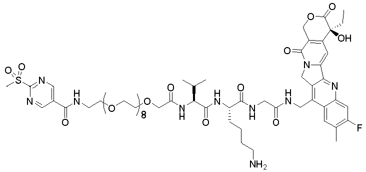 | *N*-((7*S*,10*S*)-7-(4-Aminobutyl)-1-((*S*)-4-ethyl-8-fluoro-4-hydroxy-9-methyl-3,14-dioxo-3,4,12,14-tetrahydro-1*H-*pyrano[3',4':6,7]indolizino[1,2-*b*] quinolin-11-yl)-10-isopropyl-3,6,9,12-tetraoxo-14,17,20,23,26,29,32,35,38-nonaoxa-2,5,8,11-tetraazatetracontan-40-yl)-2-(methylsulfonyl)pyrimidine-5-carboxamide | 1330.9 |
| | DL-004 | | |
| | Starting materials: C1.1 and B1.4 | | |
| Example 2.5 | 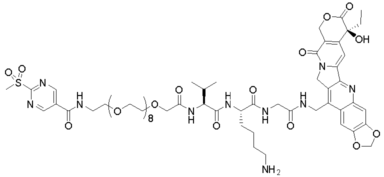 | *N*-((7*S*,10*S*)-7-(4-Aminobutyl)-1-((*S*)-7-ethyl-7-hydroxy-8,11-dioxo-7,8,11,13-tetrahydro-10*H-*[1,3]dioxolo[4,5-*g*]pyrano[3',4':6,7]indolizino[1,2-*b*]quinolin-14-yl)-10-isopropyl-3,6,9,12-tetraoxo-14,17,20,23,26,29,32,35,38-nonaoxa-2,5,8,11-tetraazatetracontan-40-yl)-2-(methylsulfonyl)pyrimidine-5-carboxamide | 1342.8 |
| | DL-005 | | |
| | Starting materials: C1.1 and B1.5 | | |
| Example 2.6 | 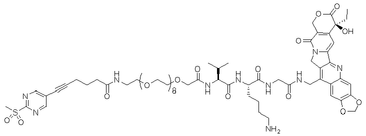 | *N*-((*S*)-1-(((*S*)-6-Amino-1-((2-((((*S*)-7-ethyl-7-hydroxy-8,11-dioxo-7,8,11,13-tetrahydro-10*H-*[1,3]dioxolo[4,5-*g*]pyrano[3',4':6,7]indolizino[1,2-*b*]quinolin-14-yl)methyl)amino)-2-oxoethyl)amino)-1-oxohexan-2-yl)amino)-3-methyl-1-oxobutan-2-yl)-29-(6-(2-(methylsulfonyl)pyrimidin-5-yl)hex-5-ynamido)-3,6,9,12,15,18,21,24,27-nonaoxanonacosanamide | 1408.5 |
| | DL-006 | | |
| | Starting materials: C1.5 and B1.5 | | |
| Example 2.7 | 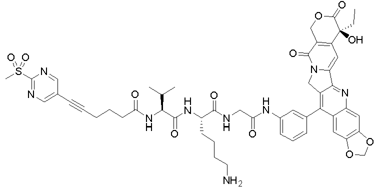 | *N*-((*S*)-1-(((*S*)-6-Amino-1-((2-((3-((*S*)-7-ethyl-7-hydroxy-8,11-dioxo-7,8,11,13-tetrahydro-10*H-*[1,3]dioxolo[4,5-*g*]pyrano[3',4':6,7]indolizino[1,2-*b*]quinolin-14-yl)phenyl)amino)-2-oxoethyl)amino)-1-oxohexan-2-yl)amino)-3-methyl-1-oxobutan-2-yl)-6-(2-(methylsulfonyl)pyrimidin-5-yl)hex-5-yn-amide | 1017.9 |
| | DL-007 | | |
| | Starting materials: C1.7 and B1.8 | | |
| Example 2.8 | 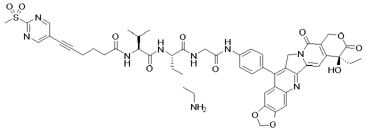 | *N*-((*S*)-1-(((*S*)-6-Amino-1-((2-((4-((*S*)-7-ethyl-7-hydroxy-8,11-dioxo-7,8,11,13-tetrahydro-10*H-*[1,3]dioxolo[4,5-*g*]pyrano[3',4':6,7]indolizino[1,2-*b*]quinolin-14-yl)phenyl)amino)-2-oxoethyl)amino)-1-oxohexan-2-yl)amino)-3-methyl-1-oxobutan-2-yl)-6-(2-(methylsulfonyl)pyrimidin-5-yl)hex-5-ynamide | 1017.8 |
| | DL-008 | | |
| | Starting materials: C1.7 and B1.9 | | |

### Example 2.9: (S)-6-Amino-N-(2-((((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)methyl)amino)-2-oxoethyl)-2-((S)-3-methyl-2-(6-(4-(2-(methylsulfonyl)pyrimidin-5-yl)-1H-1,2,3-triazol-1-yl)hexanamido)butanamido)hexanamide (DL-009)

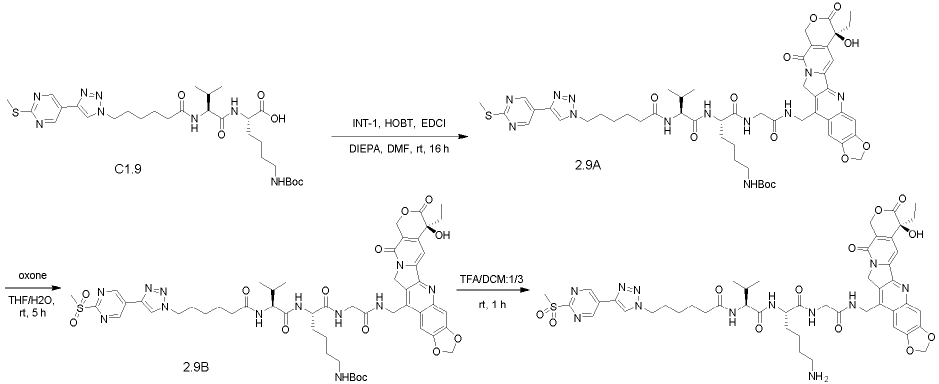

### Step 1:

Compound B1.5 (30 mg), compound C1.9 (48 mg), and *N,N*-diisopropylethylamine (23 mg) were dissolved in DMF (5 mL). 1-Hydroxybenzotriazole (9 mg) and 1-(3-dimethylaminopropyl)-3-ethylcarbodiimide hydrochloride (13 mg) were added to the above reaction, and the reaction mixture was stirred at room temperature for 16 h and then diluted with 50 mL of ethyl acetate. The organic phase was washed three times with water (25 mL × 3), dried over anhydrous sodium sulfate, and concentrated to remove the ethyl acetate to obtain a crude product. The crude product was separated and purified by flash chromatography (DCM:MeOH = 100:0 to 90:10) to obtain the target compound 2.9A (35 mg, pale yellow solid, yield: 43%).

LCMS (ESI) [M+H]⁺: 1095.8

### Step 2:

Compound 2.9A (35 mg) was dissolved in a mixed solution of tetrahydrofuran and water (volume ratio: 1/1; 4 mL), and potassium peroxymonosulfonate (184 mg) was added. The reaction solution was stirred at room temperature for 5 h and then diluted with 30 mL of ethyl acetate. The organic phase was washed three times with water (25 mL × 3), dried over anhydrous sodium sulfate, and concentrated to remove the ethyl acetate to obtain the target compound 2.9B (25 mg, pale yellow solid, yield: 71%).

LCMS (ESI) [M+H]⁺: 1126.9.

### Step 3:

Compound 2.9B (25 mg) was dissolved in a mixed solution of trifluoroacetic acid and dichloromethane (volume ratio: 1:3; 4 mL), and the reaction solution was stirred at room temperature for 1 h and then concentrated to obtain a crude product. The crude product was purified by prep-HPLC (0.01% TFA in water, MeCN) to obtain the target compound (*S*)-6-amino-*N*-(2-((((*S*)-7-ethyl-7-hydroxy-8,11-dioxo-7,8,11,13-tetrahydro-10*H-*[1,3]dioxolo[4,5-*g*]pyrano[3',4':6,7]indolizino[1,2-*b*]quinolin-14-yl)methyl)amino)-2-oxoethyl)-2-((*S*)-3-methyl-2-(6-(4-(2-(methylsulfonyl)pyrimidin-5-yl)-1*H*-1,2,3-triazol-1-yl)hexanamido)butanamido)hexanamide trifluoroacetate (5.1 mg).
LCMS (ESI) [M+H]⁺: 1026.9;
¹H NMR (400 MHz, DMSO) δ 9.46 (s, 2H), 8.91 (s, 1H), 8.53 (s, 1H), 8.18 (s, 1H), 8.11 - 8.10 (m, 1H), 7.79 (s, 1H), 7.73 - 7.72 (m, 1H), 7.63 (s, 3H), 7.53 (s, 1H), 7.26 (s, 1H), 6.52 (s, 1H), 6.29 (m, 2H), 5.61 - 5.46 (m, 2H), 5.43 (s, 2H), 4.85 - 4.83 (m, 1H), 4.63 - 4.60 (m 1H), 4.44 (s, 2H), 4.06 - 4.03 (m, 3H), 2.75 (s, 2H), 2.12 - 2.10 (m, 2H), 1.89 - 1.85 (m, 6H), 1.56 - 1.53 (m, 8H), 1.24 (br s, 4H), 0.87 - 0.84 (m, 3H), 0.77 (d, *J =* 6.1 Hz, 6H).

### Example 2.10: N-((7S,10S)-7-(4-Aminobutyl)-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)-10-isopropyl-3,6,9,12,16-pentaoxo-14,20,23,26,29,32,35,38,41-nonaoxa-2,5,8,11,17-pentaazatritetracontan-43-yl)-6-(2-(methylsulfonyl)pyrimidin-5-yl)hex-5-ynamide (DL-010)

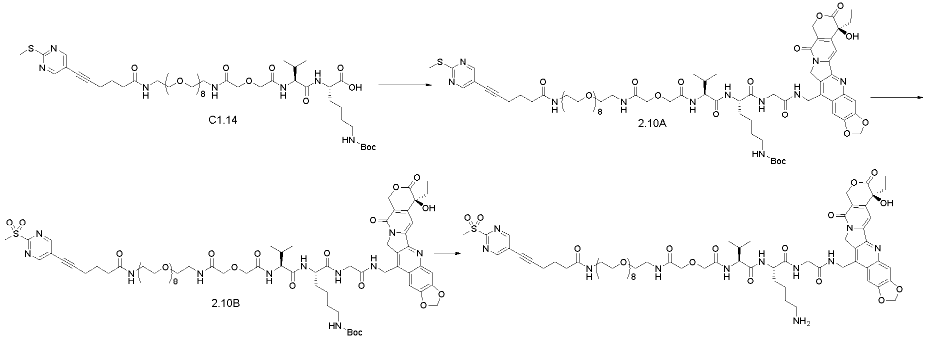

### Step 1:

Compound C1.14 (112 mg) was dissolved in N,N-dimethylformamide (2 mL), and then triethylamine (32 mg, 0.31 mmol), 1-hydroxybenzotriazole (17 mg, 0.13 mmol), and 1-(3-dimethylaminopropyl)-3-ethylcarbodiimide hydrochloride (24 mg, 0.13 mmol) were added sequentially. The mixture was stirred for 20 min, then compound B1.5 (50 mg) was added to the reaction solution, and the reaction solution was stirred at 40 °C overnight. Water (10 mL) was then added to the reaction solution, and the mixture was extracted with dichloromethane, dried over anhydrous sodium sulfate, filtered under vacuum, and concentrated to dryness by rotary evaporation to obtain a crude product. The crude product was separated and purified by reversed-phase chromatography using a C18 column (acetonitrile/0.01% FA in water: 0%-55%) to obtain the target compound 2.10A (75 mg, yield: 46%) as a white solid.

LCMS (ESI) [M+H]⁺: 1534.0.

### Step 2:

Compound 2.10A (75 mg) was dissolved in a solution of tetrahydrofuran and water (8 mL, 1:1), and potassium peroxymonosulfonate (211 mg) was added. The mixture was stirred at room temperature for 10 h. Dichloromethane (10 mL) was added to the reaction solution for extraction. The organic phase was separated, dried over anhydrous sodium sulfate, filtered under vacuum, and concentrated to dryness by rotary evaporation to obtain the target product 2.10B (50 mg, yield: 65%).

LCMS (ESI) [M+H]⁺: 1566.2.

### Step 3:

Compound 2.10B (50 mg) was dissolved in a mixed solution of trifluoroacetic acid and dichloromethane (volume ratio: 1:2; 4 mL), and then the reaction solution was stirred at room temperature for 1 h. The reaction solution was concentrated to obtain a crude product. The crude product was subjected to preparative chromatography (0.01% TFA in water, MeCN) to obtain the target compound *N*-((7*S*,10*S*)-7-(4-Aminobutyl)-1-((*S*)-7-ethyl-7-hydroxy-8,11-dioxo-7,8,11,13-tetrahydro-10*H*-[1,3]dioxolo[4,5-*g*]pyrano[3',4':6,7]indolizino[1,2-*b*]quinolin-14-yl)-10-isopropyl-3,6,9,12,16-pentaoxo-14,20,23,26,29,32,35,38,41-nonaoxa-2,5,8,11,17-pentaazatritetracontan-43-yl)-6-(2-(methylsulfonyl)pyrimidin-5-yl)hex-5-ynamide (10.5 mg, yield: 21%).
LCMS (ESI) [M+H]⁺: 1466.9;
¹H NMR (400 MHz, DMSO-d6) δ 9.12 (s, 2H), 8.62 (t, *J* = 5.7 Hz, 1H), 8.26 - 8.11 (m, 2H), 8.03 (t, *J* = 5.8 Hz, 1H), 7.93 (t, *J* = 5.3 Hz, 1H), 7.85 (d, *J* = 8.7 Hz, 1H), 7.79 (d, *J* = 5.6 Hz, 1H), 7.61 (s, 3H), 7.54 (s, 1H), 7.26 (s, 1H), 6.50 (s, 1H), 6.31 (d, *J=* 1.7 Hz, 2H), 5.46 (d, *J =* 17.2 Hz, 4H), 4.76 (dt, *J =* 14.8, 8.7 Hz, 2H), 4.20 - 4.15 (m, 4H), 4.00 (s, 3H), 3.95 (d, *J* = 5.5 Hz, 3H), 3.72 - 3.67 (m, 2H), 3.50 (d, *J* = 3.9 Hz, 30H), 3.41 (s, 3H), 3.25 - 3.19 (m, 4H), 2.76 (d, *J* = 5.6 Hz, 2H), 2.56 (t, *J=* 4.9 Hz, 2H), 2.27 (t, *J=* 7.3 Hz, 2H), 1.90 (dd, *J =* 16.1, 7.4 Hz, 2H), 1.84 - 1.78 (m, 2H), 1.63 (s, 1H), 1.53 - 1.48 (m, 2H), 1.30 (d, *J =* 7.7 Hz, 2H), 0.87 - 0.84 (m, 4H), 0.78 - 0.75 (m, 6H).

### Example 2.11: N-((S)-1-(((S)-6-Amino-1-((2-((3-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)propyl)amino)-2-oxoethyl)amino)-1-oxohexan-2-yl)amino)-3-methyl-1-oxobutan-2-yl)-6-(2-(methylsulfonyl)pyrimidin-5-yl)hex-5-ynamide (DL-011)

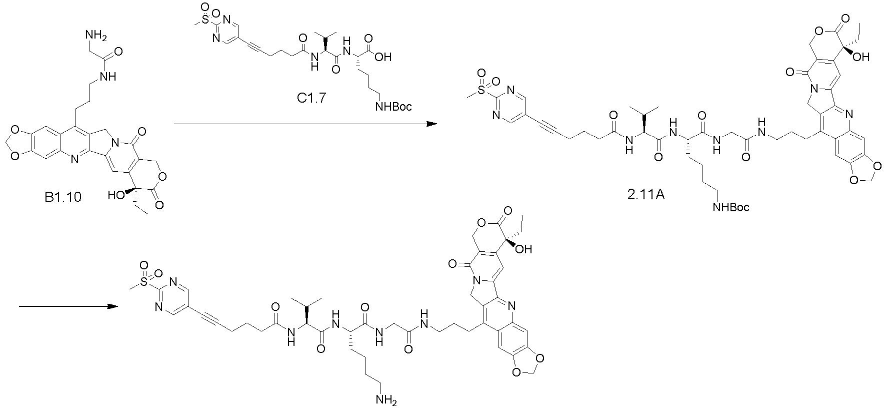

### Step 1:

To a solution of compound B1.10 (75 mg) in *N*,*N*-dimethylformamide (3 mL) were added sequentially compound C1.7 (107 mg), triethylamine (45 mg), HOBT (25 mg), and EDCI (35 mg), and the mixture was allowed to react at 35 °C for 4 h. Water (30 mL) was added to the reaction solution, and the mixture was extracted with ethyl acetate (30 mL × 2). The organic phase was dried over anhydrous sodium sulfate and concentrated to obtain the target compound 2.11A (60 mg, yield: 37%).

LCMS (ESI) [M+H]⁺: 1084;

### Step 2:

To a solution of compound 2.11A (60 mg) in dichloromethane (4 mL) was added a mixed solution of trifluoroacetic acid and dichloromethane (volume ratio: 1:2; 4 mL), and the mixture was stirred at room temperature for 1 h. The reaction solution was concentrated, and the crude product was purified by preparative high-performance liquid chromatography (acetonitrile/0.05% formic acid in water) to obtain the target compound *N*-((*S*)-1-(((*S*)-6-amino-1-((2-((3-((*S*)-7-ethyl-7-hydroxy-8,11-dioxo-7,8,11,13-tetrahydro-10*H*-[1,3]dioxolo[4,5-g]pyrano[3',4':6,7]indolizino[1,2-*b*]quinolin-14-yl)propyl)amino)-2-oxoethyl)amino)-1-oxohexan-2-yl)amino)-3-methyl-1-oxobutan-2-yl)-6-(2-(methylsulfonyl)pyrimidin-5-yl)hex-5-ynamide (9 mg, yield: 18%).
LCMS (ESI) [M+H]⁺: 983.9;
¹H NMR (400 MHz, DMSO-d6) *δ* 9.08 (s, 2H), 8.34 (s, 1H), 8.24 (s, 1H), 8.19 (d, *J =* 7.1 Hz, 1H), 8.00 (d, *J=* 8.3 Hz, 1H), 7.95 (s, 1H), 7.63 (s, 1H), 7.49 (d, *J=* 2.8 Hz, 1H), 7.24 (s, 1H), 6.50 (s, 1H), 6.28 (d, *J=* 1.9 Hz, 2H), 5.42 (s, 2H), 5.24 (s, 2H), 4.25 - 4.07 (m, 2H), 3.74 - 3.70 (m, 3H), 3.25 (d, *J=* 12.4 Hz, 3H), 3.08 (d, *J* = 7.6 Hz, 2H), 2.73 (s, 2H), 2.40 - 2.19 (m, 3H), 2.01 - 1.64 (m, 10H), 1.52 (br s, 4H), 1.34 (br s, 2H), 0.87 (t, *J=* 7.3 Hz, 3H), 0.83 - 0.75 (m, 6H).

### Example 2.12: (S)-6-Amino-N-(2-((((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)methyl)amino)-2-oxoethyl)-2-((S)-2-isopropyl-35-(4-(2-(methylsulfonyl)pyrimidin-5-yl)-1H-1,2,3-triazol-1-yl)-4,8-dioxo-6,12,15,18,21,24,27,30,33-nonaoxa-3,9-diazapentatriacontanamido)hexanamide (DL-012)

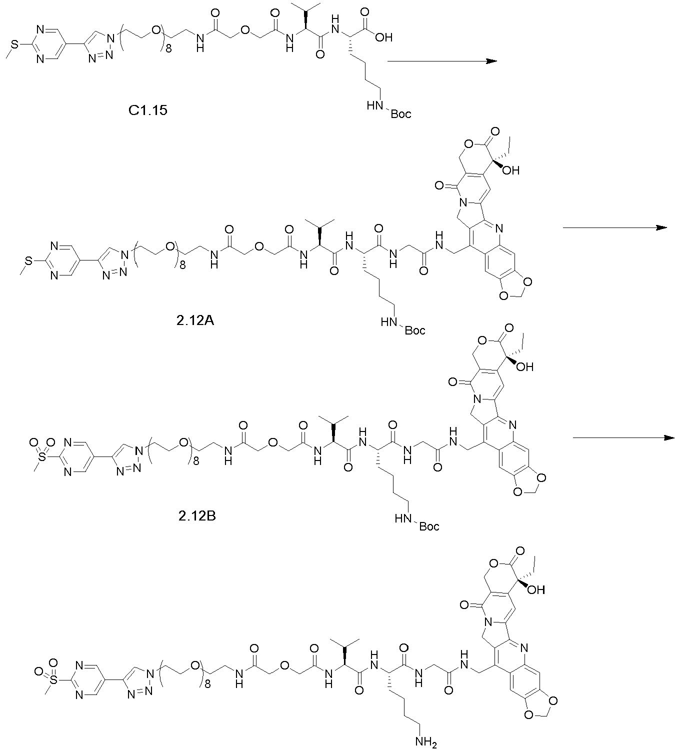

### Step 1:

Compound C1.15 (80 mg), compound B1.5 (37 mg), and triethylamine (23 mg, 0.231 mol) were dissolved in DMF (5 mL). Then, 1-hydroxybenzotriazole (12 mg) and 1-(3-dimethylaminopropyl)-3-ethylcarbodiimide hydrochloride (17 mg) were added, and the reaction solution was stirred at room temperature for 16 h and then diluted with 50 mL of ethyl acetate. The organic phase was washed three times with water (25 mL × 3), dried over anhydrous sodium sulfate, and concentrated to remove the ethyl acetate to obtain a crude product. The crude product was separated and purified by flash chromatography (DCM:MeOH = 100:0 to 90:10) to obtain the target compound 2.12A (75 mg, yield: 65%).

LCMS (ESI) [M+H]⁺: 1492.0.

### Step 2:

Compound 2.12A (50 mg) was dissolved in a mixed solution of tetrahydrofuran and water (volume ratio: 1/1; 4 mL), and potassium peroxymonosulfonate (202 mg) was added. The reaction solution was stirred at room temperature for 5 h and then diluted with 30 mL of ethyl acetate. The organic phase was washed three times with water (25 mL × 3), dried over anhydrous sodium sulfate, and concentrated to remove the ethyl acetate to obtain the target compound 2.12B (40 mg, yield: 80%).

LCMS (ESI) [M+H]⁺: 1524.1.

### Step 3:

Compound 2.12B (30 mg) was dissolved in a mixed solution of trifluoroacetic acid and dichloromethane (volume ratio: 1:3; 4 mL), and then the reaction solution was stirred at room temperature for 1 h. The reaction solution was then concentrated to obtain a crude product. The crude product was purified by prep-HPLC (water containing 0.01% trifluoroacetic acid, acetonitrile) to obtain the target compound (*S*)-6-amino-*N*-(2-((((*S*)-7-ethyl-7-hydroxy-8,11-dioxo-7,8,11,13-tetrahydro-10*H*-[1,3]dioxolo[4,5-g]pyrano[3',4':6,7]indolizino[1,2-*b*]quinolin-14-yl)methyl)amino)-2-oxoethyl)-2-((*S*)-2-isopropyl-35-(4-(2-(methylsulfonyl)pyrimidin-5-yl)-1*H*-1,2,3-triazol-1-yl)-4,8-dioxo-6,12,15,18,21,24,27,30,33-nonaoxa-3,9-diazapentatriacontanamido)hexanamide (9 mg, yield: 30%).

LCMS (ESI) [M+H]⁺: 1423.9.

### Example 2.13: N-((13S,16S)-13-(4-(Dimethylamino)butyl)-3-ethyl-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)-17-methyl-4,9,12,15-tetraoxo-6-oxa-3,8,11,14-tetraazaoctadecan-16-yl)-6-(2-(methylsulfonyl)pyrimidin-5-yl)hex-5-ynamide (DL-013)

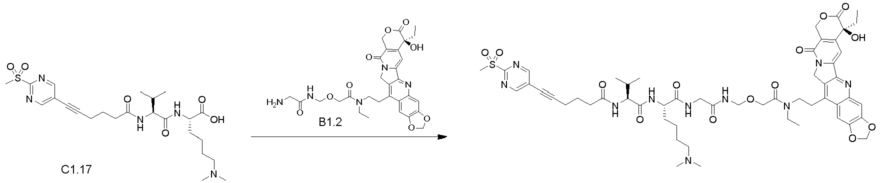

Compound C1.17 (50 mg), compound B1.2 (58 mg), and triethylamine (24 mg) were dissolved in *N,N-*dimethylformamide (2 mL). Then, 1-hydroxybenzotriazole (16 mg) and 1-(3-dimethylaminopropyl)-3-ethylcarbodiimide hydrochloride (22 mg) were added sequentially, and the reaction solution was stirred at room temperature for 16 h. The reaction solution was directly purified by preparative chromatography (0.01% aqueous formic acid solution, acetonitrile) to obtain the target compound (21 mg, yield: 10%).
LCMS (ESI) [M+H]⁺: 1113.0;
¹H NMR (400 MHz, MeOD) δ 8.93 (s, 2H), 8.40 - 8.33 (m, 1H), 8.30 - 8.25 (m, 1H), 8.25 - 8.21 (m, 1H), 7.65 (s, 1H), 7.48 (s, 1H), 7.34 (s, 1H), 6.22 (s, 2H), 5.47 (dd, *J =* 78.6, 16.4 Hz, 2H), 5.28 - 5.13 (m, 2H), 4.73 - 4.59 (m, 2H), 4.30 - 4.05 (m, 4H), 3.94 - 3.82 (m, 2H), 3.70 - 3.57 (m, 2H), 3.54 - 3.44 (m, 1H), 3.34 (s, 6H), 3.16 - 3.08 (m, 2H), 2.90 (s, 6H), 2.58 - 2.40 (m, 4H), 2.04 - 1.83 (m, 6H), 1.80 - 1.68 (m, 3H), 1.54 - 1.36 (m, 2H), 1.24 - 1.15 (m, 3H), 1.03 - 0.86 (m, 9H).

### Example 2.14: N-((13S,16S)-13-(4-(N-Oxo-N,N-dimethylamino)butyl)-3-ethyl-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)-17-methyl-4,9,12,15-tetraoxo-6-oxa-3,8,11,14-tetraazaoctadecan-16-yl)-6-(2-(methylsulfonyl)pyrimidin-5-yl)hex-5-ynamide (DL-014)

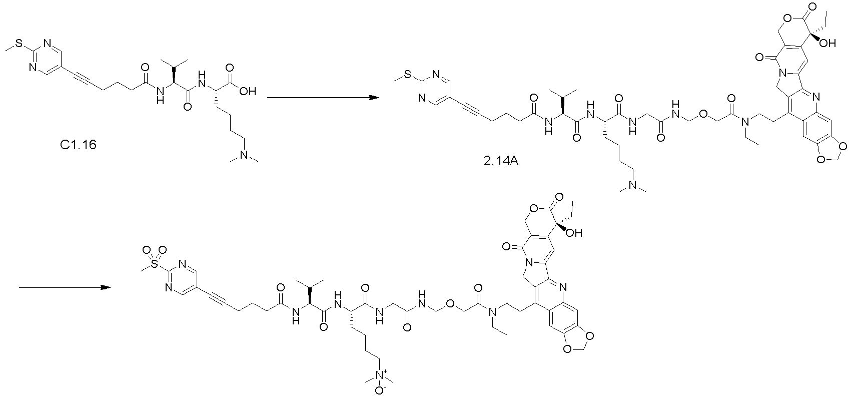

### Step 1:

Compound C1.16 (49 mg), compound B1.2 (71 mg), and *N,N-*diisopropylethylamine (39 mg) were dissolved in DMF (5 mL). Then, 1-hydroxybenzotriazole (16 mg) and 1-(3-dimethylaminopropyl)-3-ethylcarbodiimide hydrochloride (23 mg) were added sequentially, and after the addition, the reaction solution was stirred at room temperature for 16 h and then diluted with 50 mL of ethyl acetate. The organic phase was washed three times with water (25 mL × 3), dried over anhydrous sodium sulfate, and concentrated to remove the ethyl acetate to obtain a crude product. The crude product was separated and purified by flash silica gel column chromatography (DCM:MeOH = 100:0 to 90: 10) to obtain the target compound 2.14A (61 mg, yield: 60%).

LCMS (ESI) [M+H]⁺: 1081.6;

### Step 2:

Compound 2.14A (40 mg) was dissolved in a mixed solution of tetrahydrofuran and water (volume ratio: 1/1; 4 mL), then potassium peroxymonosulfonate (227 mg, 0.37 mmol) was added, and the reaction solution was stirred at room temperature for 5 h. The reaction was completed as detected by LCMS. The reaction solution was then diluted with 30 mL of ethyl acetate, and the organic phase was washed three times with water (25 mL × 3), dried over anhydrous sodium sulfate, and concentrated to remove the ethyl acetate to obtain a crude product of the compound, which was then purified by preparative chromatography (0.01% aqueous formic acid solution, acetonitrile) to obtain the target compound *N*-((13*S*,16*S*)-13-(4-(*N*-oxo-*N*,*N*-dimethylamino)butyl)-3-ethyl-1-((*S*)-7-ethyl-7-hydroxy-8,11-dioxo-7,8,11,13-tetrahydro-10*H*-[1,3]dioxolo[4,5-*g*]pyrano[3',4':6,7]indolizino[1,2-*b*]quinolin-14-yl)-17-methyl-4,9,12,15-tetraoxo-6-oxa-3,8,11,14-tetraazaoctadecan-16-yl)-6-(2-(methylsulfonyl)pyrimidin-5-yl)hex-5-ynamide (9 mg, yield: 33%).

LCMS (ESI) [M+H]⁺ = 1129.0.

### Example 2.15: N-((13S,16S)-13-(4-(N,N-Dimethylamino)butyl)-3-isopropyl-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)-17-methyl-4,9,12,15-tetraoxo-6-oxa-3,8,11,14-tetraazaoctadecan-16-yl)-6-(2-(methylsulfonyl)pyrimidin-5-yl)hex-5-ynamide (DL-015)

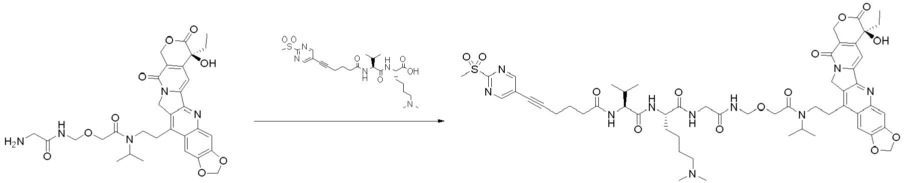

To a solution of compound *N*⁶,*N*⁶-dimethyl-*N*²-((6-(2-(methylsulfonyl)pyrimidin-5-yl)hex-5-ynoyl)-*L-*valyl)-*L*-lysine (42 mg, C1.17) and compound (*S*)-2-((2-aminoacetamido)methoxy)-*N*-isopropyl-*N*-(2-(7-ethyl-7-hydroxy-8,11-dioxo-7,8,11,13-tetrahydro-10*H*-[1,3]dioxolo[4,5-*g*]pyrano[3',4':6,7]indolizino[1,2-*b*]quinolin-14-yl)ethyl)acetamide (50 mg, B1.11) in N,N-dimethylformamide (10 mL) were added sequentially HBTU (31 mg) and diisopropylethylamine (21 mg). The reaction mixture was stirred at room temperature for 2 h. The reaction solution was filtered and then purified by preparative high-performance liquid chromatography (acetonitrile/water containing 0.05% trifluoroacetic acid) to obtain the target compound *N-*((13*S*,16*S*)-13-(4-(*N,N-*dimethylamino)butyl)-3-isopropyl-1-((*S*)-7-ethyl-7-hydroxy-8,11-dioxo-7,8,11,13-tetrahydro-10*H-*[1,3]dioxolo[4,5-*g*]pyrano[3',4':6,7]indolizino[1,2-*b*]quinolin-14-yl)-17-methyl-4,9,12,15-tetraoxo-6-oxa-3,8,11,14-tetraazaoctadecan-16-yl)-6-(2-(methylsulfonyl)pyrimidin-5-yl)hex-5-ynamide (15.2 mg).
LCMS (ESI) [M+H]⁺ = 1127.6;
¹H NMR (400 MHz, DMSO-d6) δ 9.10 (s, 2H), 8.73 (t, *J* = 8.7 Hz, 1H), 8.23 - 8.17 (m, 1H), 8.10 (*d, J =* 7.5 Hz, 1H), 7.97 - 7.93 (m, 2H), 7.52 (s, 1H), 7.25 (s, 1H), 6.50 (s, 1H), 6.30 (s, 2H), 5.42 (s, 2H), 5.36 (s, 2H), 4.66 (d, *J=* 6.6 Hz, 2H), 4.33 - 4.21 (m, 4H), 4.20 - 4.13 (m, 2H), 3.76 - 3.74 (m, 2H), 3.40 (s, 3H), 3.40 - 3.38 (m, 1H), 3.30 - 3.21 (m, 2H), 3.04 - 2.95 (m, 2H), 2.76 (s, 3H), 2.74 (s, 3H), 2.39 - 2.31 (m, 2H), 2.00 - 1.74 (m, 6H), 1.68 - 1.49 (m, 4H), 1.37 - 1.27 (m, 2H), 1.23 - 1.16 (m, 6H), 0.89 - 0.82 (m, 9H).

### Example 2.16: N-((10S,13S)-10-(4-(N,N-Dimethylamino)butyl)-1-(((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)amino)-14-methyl-1,6,9,12-tetraoxo-3-oxa-5,8,11-triazapentadecan-13-yl)-6-(2-(methylsulfonyl)pyrimidin-5-yl)hex-5-ynamide (DL-016)

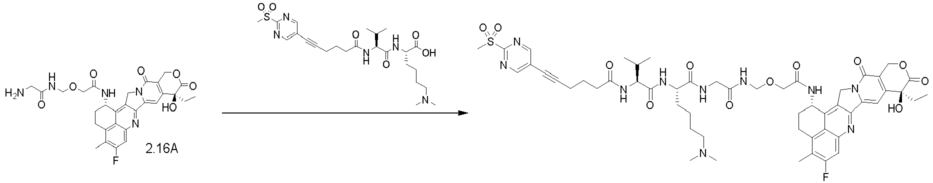

Compound *N*⁶,*N*⁶-dimethyl-*N*²-((6-(2-(methylsulfonyl)pyrimidin-5-yl)hexadecyl-5-ynoyl)-*L*-propionyl)-*L*-lysine (C1.17, 52.3 mg) was dissolved in *N*,*N*-dimethylformamide (2 mL), and then HBTU (38 mg, 0.10 mmol) and N,N-diisopropylethylamine (26 mg, 0.20 mmol) were added sequentially. After the addition, the reaction solution was stirred at room temperature for 30 min, and then compound 2-amino-*N*-((2-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolo[1,2-*b*]quinolin-1-yl)amino)-2-oxoethoxy)methyl)acetamide (2.16A, prepared according to the same method as reported on pages 147-148 in CN104755494A, 63 mg). After the reaction was completed as detected by LCMS, the reaction solution was directly purified by preparative chromatography (0.01% aqueous trifluoroacetic acid solution, acetonitrile) to obtain the target compound *N*-((10*S*,13*S*)-10-(4-(*N,N*-dimethylamino)butyl)-1-(((1*S*,9*S*)-9-ethyl-5-*fluoro-9-hydroxy-4-methyl-10,13-dioxo-2,3,9,10,13,15-hexahydro-1H,12H-*benzo[*de*]pyrano[3',4':6,7]indolizino[1,2-*b*]quinolin-1-yl)amino)-14-methyl-1,6,9,12-tetraoxo-3-oxa-5,8,11-triazapentadecan-13-yl)-6-(2-(methylsulfonyl)pyrimidin-5-yl)hex-5-ynamide (8.1 mg, yield: 7%).
LCMS (ESI) [M+H]⁺ = 1085.5;
¹H NMR (400 MHz, DMSO-d6) δ 9.31 (s, 1H), 9.11 (s, 2H), 8.72 (*t, J =* 6.8 Hz, 1H), 8.51 (*d, J =* 8.8 Hz, 1H), 8.19 *(t, J=* 6.8 Hz, 1H), 8.10 *(d, J=* 7.1 Hz, 1H), 7.91 *(d, J=* 8.6 Hz, 1H), 7.79 (d, *J* = 10.9 Hz, 1H), 7.32 (s, 1H), 6.53 (s, 1H), 5.63 - 5.54 (m, 1H), 5.42 (s, 2H), 5.20 (s, 2H), 4.68 - 4.58 (m, 2H), 4.26 - 4.12 (m, 2H), 4.01 (s, 2H), 3.73 (d, *J* = 5.4 Hz, 2H), 3.20 - 3.10 (m, 2H), 3.05 - 2.95 (m, 2H), 2.76 (d, *J =* 4.7 Hz, 6H), 2.55 - 2.53 (m, 2H), 2.42 - 2.28 (m, 6H), 2.21 - 2.13 (m, 2H), 2.02 - 1.77 (m, 6H), 1.76 - 1.62 (m, 2H), 1.62 - 1.51 (m, 3H), 1.37 - 1.21 (m, 2H), 0.90 - 0.80 (m, 9H).

### Example 2.17: tert-Butyl ((S)-3-ethyl-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)-13-((S)-3-methyl-2-(6-(2-(methylsulfonyl)pyrimidin-5-yl)hex-5-ynamido)butanamido)-4,9,12-trioxo-6-oxa-3,8,11-triazaheptadecan-17-yl)carbamate (DL-017)

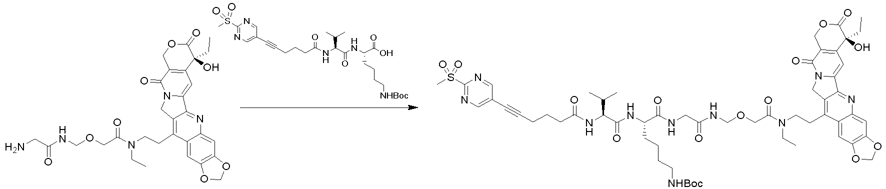

Compound C1.7 (90.0 mg), diisopropylethylamine (68.0 mg), and HATU (100 mg) were dissolved in N,N-dimethylformamide (5 mL). Then, compound B1.2 (80 mg) was added, and the mixture was allowed to react at room temperature for 2 h. The reaction was completed as detected by LCMS. Ethyl acetate (50 mL) was added to the reaction solution, and the mixture was washed with saturated brine (30 mL × 3), dried over anhydrous sodium sulfate, and filtered under vacuum. The filtrate was concentrated under reduced pressure to obtain a solid, and the crude product was purified by preparative high-performance liquid chromatography (acetonitrile/water containing 0.05% formic acid) to obtain the compound *tert*-butyl ((*S*)-3-ethyl-1-((*S*)-7-ethyl-7-hydroxy-8,11-dioxo-7,8,11,13-tetrahydro-10*H*-[1,3]dioxolo[4,5-*g*]pyrano[3',4':6,7]indolizino[1,2-*b*]quinolin-14-yl)-13-((*S*)-3-methyl-2-(6-(2-(methylsulfonyl)pyrimidin-5-yl)hex-5-ynamido)butanamido)-4,9,12-trioxo-6-oxa-3,8,11-triazaheptadecan-17-yl)carbamate (2.06 mg, yield: 10.6%) as a yellow solid.

LCMS (ESI) [M+H]⁺: 1184.9.

### Example 2.18: N-((11S,14S)-11-(4-(Dimethylamino)butyl)-1-((S)-4-ethyl-8-fluoro-4-hydroxy-9-methyl-3,14-dioxo-3,4,12,14-tetrahydro-1H pyrano[3',4':6,7]indofizino[1,2-b]quinolin-11-yl)-15-methyl-7,10,13-trioxo-4-oxa-6,9,12-triazahexadecan-14-yl)-6-(2-(methylsulfonyl)pyrimidin-5-yl)hex-5-ynamide (DL-018)

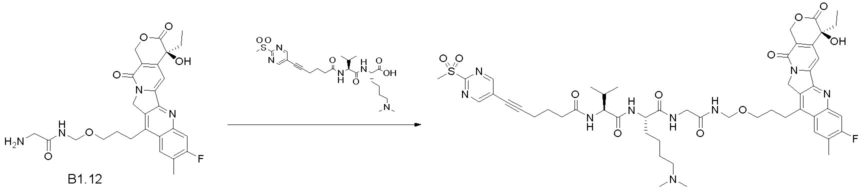

Compound *N*⁶,*N*⁶-dimethyl-*N*²-((6-(2-(methylsulfonyl)pyrimidin-3-yl)hex-5-ynoyl)-L-valyl)-L-lysine (126 mg) (C1.17), B1.12 (150 mg), 1-hydroxybenzotriazole (49 mg), 1-(3-dimethylaminopropyl)-3-ethylcarbodiimide hydrochloride (69 mg), and diisopropylethylamine (156 mg, 1.21 mmol) were dissolved in an N,N-dimethylformamide solution (10 mL), and the reaction solution was stirred at 40 °C for 12 h. The reaction was completed as detected by LCMS. The reaction solution was filtered and purified by preparative high-performance liquid chromatography (acetonitrile/water containing 0.05% formic acid) to obtain the compound *N*-((11*S*,14*S*)-11-(4-(dimethylamino)butyl)-1-((*S*)-4-ethyl-8-fluoro-4-hydroxy-9-methyl-3,14-dioxo-3,4,12,14-tetrahydro-1*H-*pyrano[3',4':6,7]indolizino[1,2-*b*]quinolin-11-yl)-15-methyl-7,10,13-trioxo-4-oxa-6,9,12-triazahexadecan-14-yl)-6-(2-(methylsulfonyl)pyrimidin-5-yl)hex-5-ynamide (DL-018) (12 mg, yield: 6.0%) as a yellow solid.
LCMS (ESI) [M+H]⁺ = 1030.6;
¹H NMR (400 MHz, DMSO-d6) δ 9.10 (s, 2H), 8.62 (t, *J* = 6.4 Hz, 1H), 8.23 - 8.13 (m, 2H), 8.07 (d, *J* = 7.2 Hz, 1H), 7.95 - 7.83 (m, 2H), 7.31 (s, 1H), 6.53 (s, 1H), 5.44 (s, 2H), 5.29 (s, 2H), 4.64 - 4.53 (m, 2H), 4.28 - 4.20 (m, 1H), 4.19 - 4.11 (m, 1H), 3.76 - 3.69 (m, 2H), 3.50 (t, *J =* 5.7 Hz, 2H), 3.41 (s, 3H), 3.23 - 3.19 (m, 2H), 2.67 - 2.62 (m, 2H), 2.57 - 2.54 (m, 2H), 2.54 (s, 3H), 2.42 - 2.26 (m, 3H), 2.02 - 1.77 (m, 6H), 1.68 - 1.66 (m, 1H), 1.58 - 1.45 (m, 3H), 1.36 - 1.25 (m, 2H), 0.88 (t, *J =* 7.4 Hz, 3H), 0.82 (t, *J =* 6.3 Hz, 6H).

### Example 2.19: N-((11S,14S)-11-(4-(Dimethylamino)butyl)-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)-15-methyl-7,10,13-trioxo-4-oxa-6,9,12-triazahexadecan-14-yl)-6-(2-(methylsulfonyl)pyrimidin-5-yl)hex-5-ynamide (DL-019)

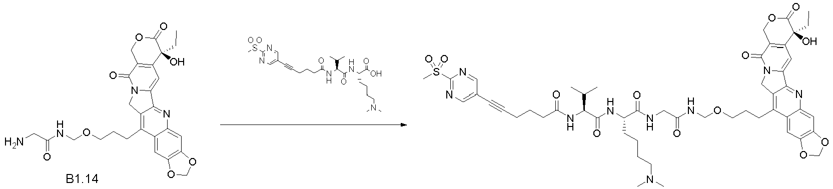

Compound B1.14 (100 mg, 0.186 mmol) and compound *N*⁶,*N*⁶-dimethyl-*N*²-((6-(2-(methylsulfonyl)pyrimidin-5-yl)hex-5-ynoyl)-*L*-valyl)-*L*-lysine (100 mg, 0.191 mmol) were dissolved in DMF (2 mL). Then, 1-hydroxybenzotriazole (38 mg, 0.280 mmol) and 1-(3-dimethylaminopropyl)-3-ethylcarbodiimide hydrochloride (53 mg, 0.280 mmol) were added, followed by the addition of triethylamine (56 mg, 0.559 mmol). After the addition, the reaction solution was stirred at room temperature for 16 h. After the reaction was completed as detected by LCMS, the reaction solution was purified by preparative chromatography (0.01% TFA in water, MeCN) to obtain the target compound *N*-((11*S,*14*S*)-11-(4-(dimethylamino)butyl)-1-((*S*)-7-ethyl-7-hydroxy-8,11-dioxo-7,8,11,13-tetrahydro-10*H*-[1,3]dioxolo[4,5-*g*]pyrano[3',4':6,7]indolizino[1,2-*b*]quinolin-14-yl)-15-methyl-7,10,13-trioxo-4-oxa-6,9,12-triazahexadecan-14-yl)-6-(2-(methylsulfonyl)pyrimidin-5-yl)hex-5-ynamide (20 mg, yield: 10%) as a yellow solid.
LCMS (ESI) [M+H]⁺ = 1042.5;
¹H NMR (400 MHz, DMSO-d6) δ 9.28 (s, 1H, TFA), 9.10 (s, 2H), 8.64 (br s, 1H), 8.19 (br s, 1H), 8.09 (d, *J =* 6.4 Hz, 1H), 7.92 (d, *J* = 7.8 Hz, 1H), 7.59 (s, 1H), 7.51 (s, 1H), 7.24 (s, 1H), 6.50 (s, 1H), 6.29 (s, 2H), 5.43 (s, 2H), 5.24 (s, 2H), 4.67 - 4.52 (m, 2H), 4.30 - 4.10 (m, 2H), 3.74 (br s, 2H), 3.50 (br s, 2H), 3.41 (s, 3H), 3.17 - 3.07 (m, 2H), 3.04 - 2.91 (m, 2H), 2.75 (s, 6H), 2.46 - 2.24 (m, 3H), 2.04 - 1.66 (m, 9H), 1.63 - 1.46 (m, 3H), 1.32 - 1.29 (m, 2H), 0.87 - 0.82 (m, 9H).

### Example 2.20: N-((S)-1-(((S)-6-Amino-1-((2-((3-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)propyl)amino)-2-oxoethyl)amino)-1-oxohexan-2-yl)amino)-3-methyl-1-oxobutan-2-yl)-6-(2-(methylsulfonyl)pyrimidin-5-yl)hex-5-ynamide (DL-020)

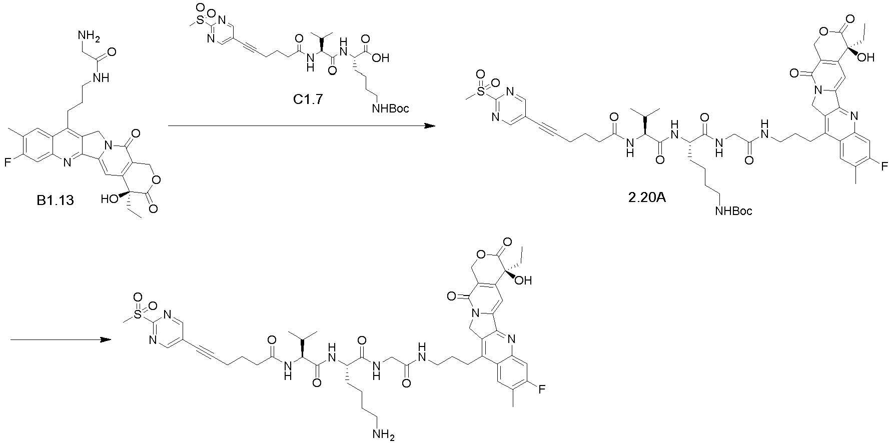

### Step 1:

To a solution of compound B1.13 (75 mg) in N,N-dimethylformamide (3 mL) were added sequentially compound C1.7 (107 mg), triethylamine (45 mg), HOBT (25 mg), and EDCI (35 mg), and the mixture was allowed to react at 35 °C for 4 h. Water (30 mL) was added to the reaction solution, and the mixture was extracted with ethyl acetate (30 mL × 2). The organic phase was dried over anhydrous sodium sulfate and concentrated to obtain the target compound 2.20A (76 mg).

LCMS (ESI) [M+H]⁺: 1072.5;

### Step 2:

To a solution of compound 2.20A (60 mg, 0.06 mmol) in dichloromethane (2 mL) was added trifluoroacetic acid (1 mL), and the mixture was stirred at room temperature for 1 h. The reaction was completed as detected by LCMS. The reaction solution was concentrated and purified by preparative chromatography (0.01% TFA in water, MeCN) to obtain the target compound *N*-((*S*)-1-(((*S*)-6-amino-1-((2-((3-((*S*)-4-ethyl-8-fluoro-4-hydroxy-9-methyl-3,14-dioxo-3,4,12,14-tetrahydro-1*H*-pyrano[3',4':6,7]indofizino[1,2-*b*]quinolin-11-yl)propyl)amino)-2-oxoethyl)amino)-1-oxohexan-2-yl)amino)-3-methyl-1-oxobutan-2-yl)-6-(2-(methylsulfonyl)pyrimidin-5-yl)hex-5-ynamide (43 mg, yield: 80%) as a yellow solid.
LCMS (ESI) [M+H]⁺: 972.9;
¹H NMR (400 MHz, DMSO-d6) δ 9.09 (s, 2H), 8.24 - 8.17 (m, 2H), 8.12 (d, *J =* 7.0 Hz, 1H), 7.99 - 7.93 (m, 1H), 7.88 - 7.84 (m, 2H), 7.64 (s, 3H, NH2-TFA), 7.31 (s, 1H), 6.52 (s, 1H), 5.44 (s, 2H), 5.30 (s, 2H), 4.25 - 4.18 (m, 1H), 4.15 - 4.11 (m, 1H), 3.76 - 3.71 (m, 2H), 3.41 (s, 3H), 3.32 - 3.26 (m, 2H), 3.21 - 3.17 (m, 1H), 2.79 - 2.70 (m, 2H), 2.52 (s, 3H), 2.40 - 2.24 (m, 3H), 1.94 - 1.82 (m, 7H), 1.80 - 1.73 (m, 2H), 1.58 - 1.49 (m, 3H), 1.36 - 1.31 (m, 1H), 1.18 - 1.14 (m, 1H), 0.87 (t, *J =* 7.4 Hz, 3H), 0.80 - 0.76 (m, 6H).

### Example 2.21: N-((S)-1-(((S)-5-Amino-6-((2-((3-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)propyl)amino)-2-oxoethyl)amino)-6-oxohexyl)amino)-3-methyl-1-oxobutan-2-yl)-6-(2-(methylsulfonyl)pyrimidin-5-yl)hex-5-ynamide

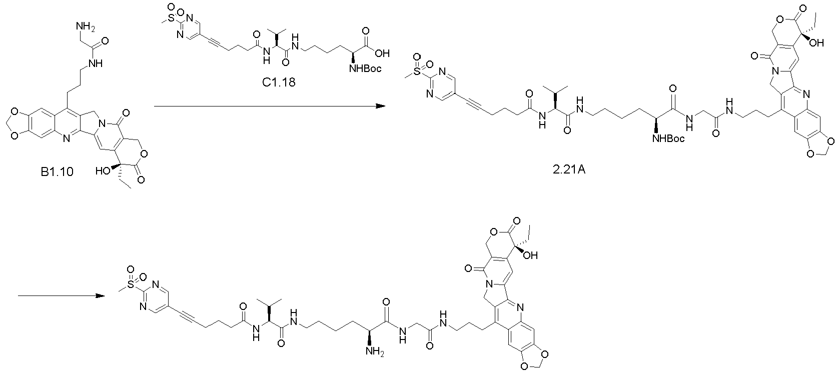

### Step 1:

Compound C1.18 (430 mg) was dissolved in *N*,*N*-dimethylformamide (5 mL), and then EDCI (160 mg), HOBT (115 mg), and triethylamine (214 mg) were added. After the addition, the reaction solution was stirred at room temperature for 20 min, then compound B1.10 (314 mg) was added, and the mixture was stirred at room temperature for 3 h. The reaction was completed as detected by LCMS. The mixture was then diluted with water and extracted with ethyl acetate, and the organic phase was concentrated to obtain a crude product of 2.21A (350 mg).

LCMS (ESI) [M+H]⁺: 1084.3;

### Step 2:

To a solution of compound 2.21A (350 mg) in dichloromethane (4 mL) was added a mixed solution of trifluoroacetic acid and dichloromethane (volume ratio: 1:2; 4 mL), and the mixture was stirred at room temperature for 1 h. The reaction solution was concentrated, and the crude product was purified by preparative high-performance liquid chromatography (acetonitrile/0.05% formic acid in water) to obtain the target compound *N-*((*S*)-1-(((*S*)-5-amino-6-((2-((3-((*S*)-7-ethyl-7-hydroxy-8,11-dioxo-7,8,11,13-tetrahydro-10*H*-[1,3]dioxolo[4,5-g]pyrano[3 ',4':6,7]indolizino[1,2-*b*]quinolin-14-yl)propyl)amino)-2-oxoethyl)amino)-6-oxohexyl)amino)-3-methyl-1-oxobutan-2-yl)-6-(2-(methylsulfonyl)pyrimidin-5-yl)hex-5-ynamide (100 mg).
LCMS (ESI) [M+H]⁺: 984.4;
¹H NMR (400 MHz, DMSO-d6) δ 9.10 (s, 2H), 8.69 (t, *J =* 6.1 Hz, 1H), 8.14 (t, *J =* 5.4 Hz, 1H), 8.08 (s, 3H, NH2-TFA), 7.95 (t, *J =* 4.9 Hz, 1H), 7.89 (d, *J =* 8.9 Hz, 1H), 7.66 (s, 1H), 7.51 (s, 1H), 7.25 (s, 1H), 6.48 (s, 1H), 6.29 (s, 2H), 5.42 (s, 2H), 5.25 (s, 2H), 4.09 (t, *J* = 8.1 Hz, 1H), 3.83 (d, *J =* 5.7 Hz, 2H), 3.40 (s, 3H), 3.33 - 3.27 (m, 2H), 3.15 - 3.06 (m, 3H), 2.97 - 2.94 (m, 1H), 2.57 - 2.53 (m, 2H), 2.43 - 2.28 (m, 3H), 1.95 - 1.68 (m, 9H), 1.46 - 1.28 (m, 4H), 0.88 (t, *J =* 7.3 Hz, 3H), 0.82 (d, *J =* 6.7 Hz, 6H).

### Example 2.22: N-((S)-1-(((S)-5-Amino-6-((2-((3-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)propyl)amino)-2-oxoethyl)amino)-6-oxohexyl)amino)-3-methyl-1-oxobutan-2-yl)-6-(2-(methylsulfonyl)pyrimidin-5-yl)hex-5-ynamide (DL-022)

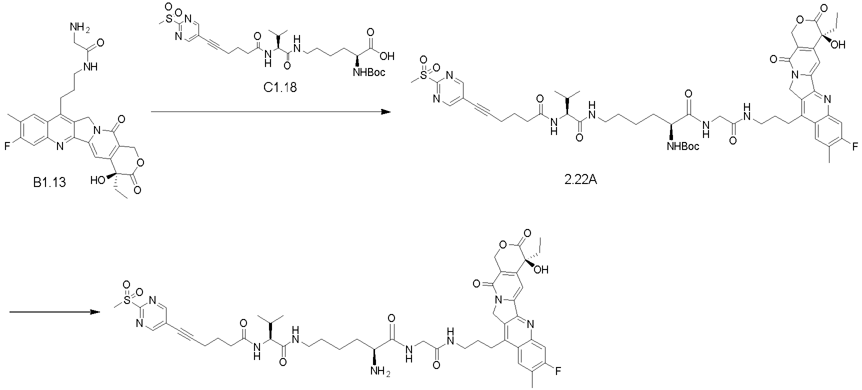

### Step 1:

Compound C1.18 (136 mg, 0.23 mmol) was dissolved in *N*,*N*-dimethylformamide (5 mL), and then EDCI (48 mg, 0.25 mmol), HOBT (34 mg, 0.25 mmol), and triethylamine (64 mg, 0.63 mmol) were added. After the addition, the reaction solution was stirred at room temperature for 20 min, then compound B1.13 (3105 mg, 0.19 mmol) was added, and the mixture was stirred at room temperature for 3 h. The reaction was completed as detected by LCMS. The mixture was then diluted with water and extracted with ethyl acetate, and the organic phase was concentrated to obtain a crude product. The resulting compound was dissolved in dichloromethane (4 mL), and then trifluoroacetic acid (2 mL) was added. The mixture was allowed to react for 1 h and then concentrated, and the crude product was purified by preparative chromatography to obtain the target compound *N*-((*S*)-1-(((*S*)-5-amino-6-((2-((3-((*S*)-4-ethyl-8-fluoro-4-hydroxy-9-methyl-3,14-dioxo-3,4,12,14-tetrahydro-1*H*-pyrano[3',4':6,7]indolizino[1,2-*b*]quinolin-11-yl)propyl)amino)-2-oxoethyl)amino)-6-oxohexyl)amino)-3-methyl-1-oxobutan-2-yl)-6-(2-(methylsulfonyl)pyrimidin-5-yl)hex-5-ynamide (DL-022) (88 mg).
LCMS (ESI) [M+H]⁺ = 972.9;
¹H NMR (400 MHz, DMSO-d6) δ 9.10 (s, 2H), 8.72 (t, *J* = 5.5 Hz, 1H), 8.23 (*d, J =* 8.4 Hz, 1H), 8.17 (t, *J* = 5.4 Hz, 1H), 8.11 (s, 3H, NH2-TFA), 7.97 (t, *J =* 5.6 Hz, 1H), 7.93 - 7.86 (m, 2H), 7.32 (s, 1H), 6.53 (s, 1H), 5.44 (s, 2H), 5.29 (s, 2H), 4.14 - 4.02 (m, 2H), 3.85 - 3.83 (m, 2H), 3.41 (s, 3H), 3.32 (d, *J* = 6.2 Hz, 2H), 3.24 - 3.16 (m, 2H), 3.11 - 2.94 (m, 2H), 2.55 (s, 3H), 2.35 - 2.30 (m, 3H), 1.92 - 1.69 (m, 10H), 1.43 - 1.29 (m, 4H), 0.88 (t, *J* = 7.3 Hz, 3H), 0.82 (d, *J* = 6.7 Hz, 6H).

### Example 2.23: (S)-6-amino-N-(2-((3-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)propyl)amino)-2-oxoethyl)-2-((S)-2-isopropyl-35-(4-(2-(methylsulfonyl)pyrimidin-5-yl)-1H-1,2,3-triazol-1-yl)-4,8-dioxo-6,12,15,18,21,24,27,30,33-nonaoxa-3,9-diazapentatriacontanamido)hexanamide (DL-023)

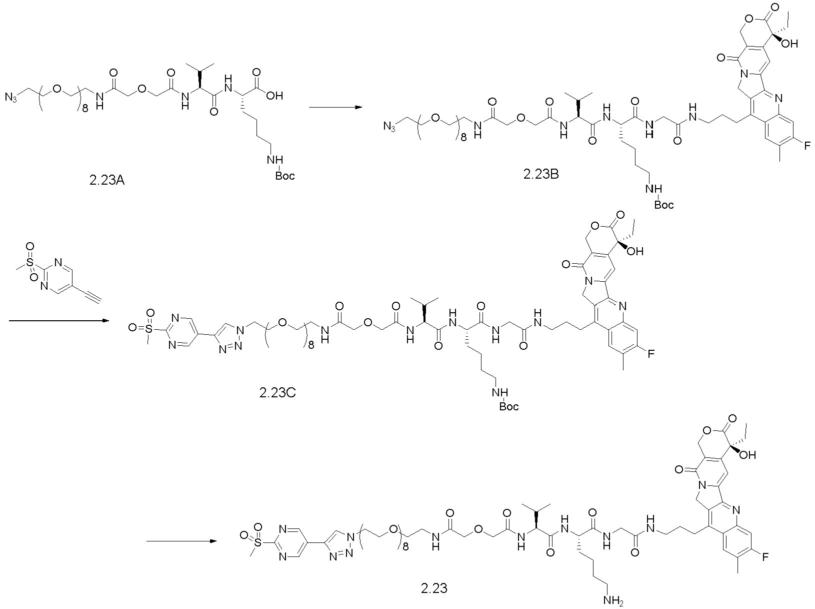

### Step 1:

To a solution of compound B1.13 (46 mg) in N,N-dimethylformamide (3 mL) were added sequentially compound 2.23A(100 mg), triethylamine (50 µL), HOBT (16 mg), and EDCI (23 mg), and the mixture was allowed to react at 35 °C for 4 h. Water (30 mL) was added to the reaction solution, and the mixture was extracted with ethyl acetate (30 mL × 2). The organic phase was dried over anhydrous sodium sulfate and concentrated to obtain the target compound 2.23B (105 mg).

LCMS (ESI) [M+H]⁺: 1358.4;

### Step 2:

Compound 2.23B (105 mg) and 2-methylsulfonyl-5-ethynylpyrimidine (45 mg) were dissolved in DMSO-water (2 mL, 4:1), and cuprous bromide (50 mg) was added to the mixture. The reaction solution was stirred at room temperature for 2 h, and then ethyl acetate (100 mL) was added. The mixture was washed with water (100 mL × 3), dried, and then concentrated under reduced pressure to remove the organic solvent, and the crude product was separated by silica gel column chromatography to obtain the target product 2.23C (84 mg). MS (*m*/*z*): 1540.8 [M + H]⁺.

### Step 3:

Compound 2.23C was dissolved in a mixed solution of trifluoroacetic acid and dichloromethane (volume ratio: 1:2; 4 mL), and then the reaction solution was stirred at room temperature for 30 min. The reaction was completed as detected by LCMS. The reaction solution was then concentrated to obtain a crude product. The crude product was subjected to preparative chromatography (0.01% TFA in water, MeCN) to obtain the target compound (*S*)-6-amino-*N*-(*2-*((3-((*S*)-4-ethyl-8-fluoro-4-hydroxy-9-methyl-3,14-dioxo-3,4,12,14-tetrahydro-1*H-*pyrano[3',4':6,7]indolizino[1,2-*b*]quinolin-11-yl)propyl)amino)-2-oxoethyl)-2-((S)-2-isopropyl-35-(4-(2-(methylsulfonyl)pyrimidin-5-yl)-1*H*-1,2,3-triazol-1-yl)-4,8-dioxo-6,12,15,18,21,24,27,30,33-nonaoxa-3,9-diazapentatriacontanamido)hexanamide (DL-023, 8 mg, trifluoroacetate, yield: 5%).
LCMS (ESI) [M+H]⁺ = 1440.8;
¹H NMR (400 MHz, DMSO-d6) δ 9.49 (s, 2H), 8.91 (s, 1H), 8.24 - 8.18 (m, 3H), 8.01 (t, *J* = 5.6 Hz, 1H), 7.96 (t, *J =* 5.5 Hz, 1H), 7.90 - 7.84 (m, 2H), 7.63 (s, 3H, TFA salt), 7.32 (s, 1H), 5.44 (s, 2H), 5.30 (s, 2H), 4.66 (t, *J=* 5.1 Hz, 2H), 4.21 (dd, *J=* 15.2, 7.9 Hz, 2H), 3.97 (s, 2H), 3.93 (s, 2H), 3.89 (t, *J* = 5.1 Hz, 2H), 3.75 - 3.72 (m, 2H), 3.56 - 3.54 (m, 2H), 3.54 - 3.38 (m, 30H), 3.33 - 3.13 (m, 5H), 2.75 (d, *J=* 7.1 Hz, 2H), 2.52 (s, 3H), 2.02 - 1.94 (m, 1H), 1.91 - 1.81 (m, 3H), 1.71 - 1.65 (m, 1H), 1.58 - 1.46 (m, 3H), 1.38 - 1.29 (m, 2H), 0.88 (t, *J=* 7.3 Hz, 3H), 0.81 - 0.75 (m, 6H).

### Example 2.24: N-((S)-1-(((S)-6-(Dimethylamino)-1-((2-((3-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)propyl)amino)-2-oxoethyl)amino)-1-oxohexan-2-yl)amino)-3-methyl-1-oxobutan-2-yl)-6-(2-(methylsulfonyl)pyrimidin-5-yl)hex-5-ynamide (DL-024)

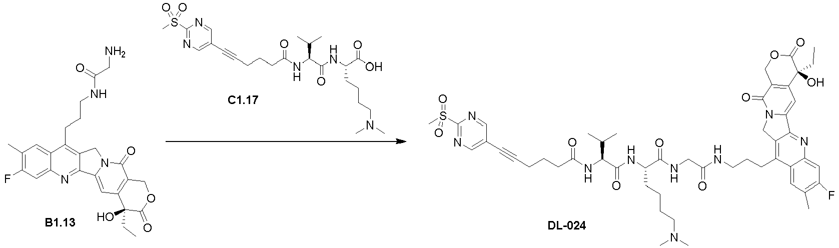

To a solution of compound B1.13 (40 mg, 0.081 mmol) and compound C1.17 (42 mg, 0.081 mmol) in N,N-dimethylformamide (2 mL) were added sequentially PyBOP (41 mg, 0.081) and DIPEA (20 mg, 0.16 mmol), and the reaction solution was stirred at room temperature for 2 h. The reaction was completed as detected by LCMS. The reaction solution was purified by preparative high-performance liquid chromatography (acetonitrile/0.05% aqueous trifluoroacetic acid) to obtain the compound N-((S)-1-(((S)-6-(dimethylamino)-1-((2-((3-((S)-4-ethyl-8-fluoro-4-hydroxy-9-methyl-3,14-dioxo-3,4,12,14-tetrahydro-1*H*-pyrano[3',4':6,7]indolizino[1,2-*b*]quinolin-11-yl)propyl)amino)-2-oxoethyl)amino)-1-oxohexan-2-yl)amino)-3-methyl-1-oxobutan-2-yl)-6-(2-(methylsulfonyl)pyrimidin-5-yl)hex-5-ynamide (11.54 mg, yield: 14%) as a yellow solid.
LCMS (ESI) [M+H]⁺ =1000.6;
¹H NMR (400 MHz, DMSO-d6) δ 9.42 (s, 1H), 9.08 (s, 2H), 8.27 - 8.18 (m, 2H), 8.14 (d, *J* = 6.9 Hz, 1H), 7.96 (t, *J* = 6.9 Hz, 1H), 7.91 - 7.77 (m, 2H), 7.31 (s, 1H), 5.44 (s, 2H), 5.29 (s, 2H), 4.15 - 4.12 (m, 1H), 3.81 - 3.65 (m, 3H), 3.41 (s, 3H), 3.33 - 3.25 (m, 2H), 3.22 - 3.15 (m, 2H), 3.03 - 2.95 (m, 2H), 2.77 - 2.71 (m, 6H), 2.38 - 2.23 (m, 3H), 1.92 - 1.81 (m, 7H), 1.78 - 1.73 (m, 2H), 1.62 - 1.56 (m, 3H), 1.38 - 1.29 (m, 2H), 0.92 - 0.84 (m, 3H), 0.84 - 0.72 (m, 6H).

### Example 2.25: N-((S)-1-(((S)-6-(Diethylamino)-1-((2-((3-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)propyl)amino)-2-oxoethyl)amino)-1-oxohexan-2-yl)amino)-3-methyl-1-oxobutan-2-yl)-6-(2-(methylsulfonyl)pyrimidin-5-yl)hex-5-ynamide (DL-025)

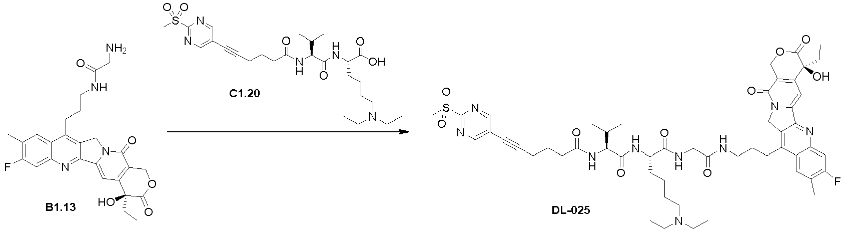

Compound C1.20 (30 mg, 0.054 mmol) and compound B1.13 (27 mg, 0.054 mmol) were dissolved in N,N-dimethylformamide (2 mL). Then, HBTU (22 mg, 0.054 mmol) and *N,N*-diisopropylethylamine (17 mg, 0.135 mmol) were added sequentially, and after the addition, the reaction solution was stirred at room temperature for 30 min. After the reaction was completed as detected by LCMS, the reaction solution was directly purified by preparative chromatography (0.01% aqueous trifluoroacetic acid solution, acetonitrile) to obtain the target compound *N*-((*S*)-1-(((*S*)-6-(diethylamino)-1-((2-((3-((*S*)-4-ethyl-8-fluoro-4-hydroxy-9-methyl-3,14-dioxo-3,4,12,14-tetrahydro-1*H*-pyrano[3',4':6,7]indolizino[1,2-*b*]quinolin-11-yl)propyl)amino)-2-oxoethyl)amino)-1-oxohexan-2-yl)amino)-3-methyl-1-oxobutan-2-yl)-6-(2-(methylsulfonyl)pyrimidin-5-yl)hex-5-ynamide (23 mg, yield: 42%) as a yellow solid.
LCMS (ESI) [M+H]⁺ = 1028.5;
¹H NMR (400 MHz, DMSO-d6) *δ* 9.08 (s, 2H), 9.02 (s, 1H), 8.21 (d, *J=* 7.4 Hz, 2H), 8.13 (d, *J =* 7.1 Hz, 1H), 7.97 (t, *J* = 5.4 Hz, 1H), 7.87 (d, *J* = 10.6 Hz, 2H), 7.31 (s, 1H), 6.52 (s, 1H), 5.44 (s, 2H), 5.30 (s, 2H), 4.28 - 4.18 (m, 1H), 4.14 (t, *J* = 7.7 Hz, 1H), 3.80 - 3.65 (m, 2H), 3.41 (s, 3H), 3.31 - 3.24 (m, 2H), 3.22 - 3.15 (m, 2H), 3.12 - 3.06 (m, 4H), 3.02 - 2.91 (m, 2H), 2.36 - 2.23 (m, 2H), 1.95 - 1.68 (m, 9H), 1.68 - 1.49 (m, 4H), 1.38 - 1.28 (m, 2H), 1.13 (t*, J =* 7.2 Hz, 6H), 0.86 (t, *J =* 7.2 Hz, 3H),0.79 - 0.76 (m, 6H).

### Example 2.26: N-((S)-1-(((S)-6-(Dipropylamino)-1-((2-((3-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)propyl)amino)-2-oxoethyl)amino)-1-oxohexan-2-yl)amino)-3-methyl-1-oxobutan-2-yl)-6-(2-(methylsulfonyl)pyrimidin-5-yl)hex-5-ynamide (DL-026)

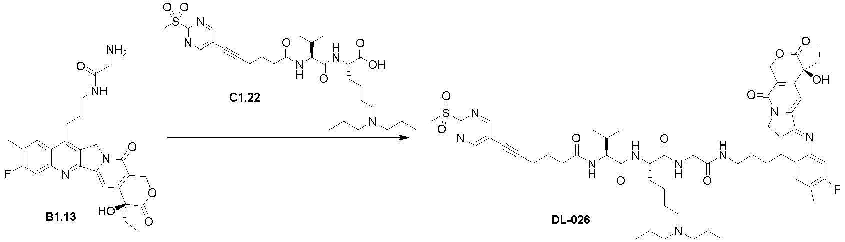

Compound C1.22 (31 mg, 0.054 mmol) and compound B1.13 (27 mg, 0.054 mmol) were dissolved in N,N-dimethylformamide (2 mL). Then, HBTU (22 mg, 0.054 mmol) and *N*,*N*-diisopropylethylamine (17 mg, 0.135 mmol) were added sequentially, and the reaction solution was stirred at room temperature for 30 min. After the reaction was completed as detected by LCMS, the reaction solution was directly purified by preparative chromatography (0.01% aqueous trifluoroacetic acid solution, acetonitrile) to obtain the target compound *N*-((*S*)-1-(((*S*)-6-(dipropylamino)-1-((2-((3-((*S*)-4-ethyl-8-fluoro-4-hydroxy-9-methyl-3,14-dioxo-3,4,12,14-tetrahydro-1*H-*pyrano[3',4':6,7]indolizino[1,2-*b*]quinolin-11-yl)propyl)amino)-2-oxoethyl)amino)-1-oxohexan-2-yl)amino)-3-methyl-1-oxobutan-2-yl)-6-(2-(methylsulfonyl)pyrimidin-5-yl)hex-5-ynamide (25 mg, yield: 45%) as a yellow solid.
LCMS (ESI) [M+H]⁺ = 1056.6;
H NMR (400 MHz, DMSO-d6) δ 9.09 (s, 2H), 8.27 - 8.19 (m, 2H), 8.13 (d, *J =* 7.3 Hz, 1H), 7.97 (t, *J* = 5.3 Hz, 1H), 7.91 - 7.84 (m, 2H), 7.31 (s, 1H), 6.53 (s, 1H), 5.44 (s, 2H), 5.29 (s, 2H), 4.24 (d, *J =* 6.7 Hz, 1H), 4.13 (t, *J* = 7.8 Hz, 1H), 3.73 (dd, *J* = 14.7, 5.9 Hz, 2H), 3.41 (s, 3H), 3.28 (d, *J* = 6.4 Hz, 2H), 3.19 (t, *J* = 7.6 Hz, 2H), 2.99 (br, 6H), 2.33 - 2.27 (m, 2H), 1.95 - 1.70 (m, 9H), 1.62 - 1.57 (m, 8H), 1.33 - 1.30 (m, 2H), 0.96 - 0.82 (m, 9H), 0.82 - 0.73 (m, 6H).

### Example 2.27: N-((S)-1-(((S)-6-(Diethylamino)-1-((2-(((E)-3-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)allyl)amino)-2-oxoethyl)amino)-1-oxohexan-2-yl)amino)-3-methyl-1-oxobutan-2-yl)-6-(2-(methylsulfonyl)pyrimidin-5-yl)hex-5-ynamide

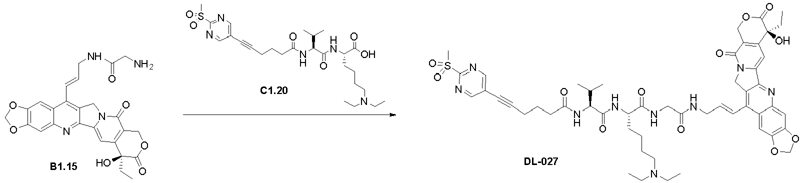

To a solution of compound B1.15 (40 mg, 0.074 mmol) and compound C1.20 (40 mg, 0.072 mmol) in *N*,*N*-dimethylformamide (4 mL) were added sequentially HBTU (30 mg, 0.079 mmol) and DIPEA (26 mg, 0.2 mmol). The reaction solution was allowed to react at room temperature for 1 h. The reaction was completed as detected by LCMS. The reaction solution was purified by preparative high-performance liquid chromatography (acetonitrile/0.05% aqueous trifluoroacetic acid solution) to obtain the compound N-((S)-1-(((S)-6-(diethylamino)-1-((2-(((*E*)-3-((*S*)-7-ethyl-7-hydroxy-8,11-dioxo-7,8,11,13-tetrahydro-10*H*-[1,3]dioxolo[4,5-g]pyrano[3',4':6,7]indolizino[1,2-*b*]quinolin-14-yl)allyl)amino)-2-oxoethyl)amino)-1-oxohexan-2-yl)amino)-3-methyl-1-oxobutan-2-yl)-6-(2-(methylsulfonyl)pyrimidin-5-yl)hex-5-ynamide (11.1 mg, yield: 14%).
LCMS (ESI) [M+H]⁺ =1038.6;
¹H NMR (400 MHz, DMSO-d6) δ 9.10 (s, 2H), 9.02 (s, 1H, TFA), 8.33 - 8.25 (m, 2H), 8.12 (d, *J =* 7.3 Hz, 1H), 7.92 (d, *J* = 8.4 Hz, 1H), 7.68 (d, *J* = 4.1 Hz, 1H), 7.52 (d, *J* = 2.8 Hz, 1H), 7.26 (s, 1H), 7.15 (d, *J* = 16.2 Hz, 1H), 6.52 - 6.50 (m, 2H), 6.30 (s, 2H), 5.43 (s, 2H), 5.28 - 5.24 (m, 2H), 4.27 (d, *J=* 6.2 Hz, 2H), 4.12 (br s, 2H), 3.81 (br s, 2H), 3.41 (s, 3H), 3.11 - 3.07 (m, 6H), 2.97 (br s, 2H), 2.53 - 2.50 (m, 1H), 2.39 - 2.24 (m, 4H), 1.85 - 1.79 (m, 8H), 1.16 (t, *J =* 6.4 Hz, 6H), 0.88 (t, *J =* 6.4 Hz, 3H), 0.78 (d, *J* = 6.4 Hz, 6H).

### Example 2.28: (S)-6-(Dimethylamino)-N-(2-(((3-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)propoxy)methyl)amino)-2-oxoethyl)-2-((S)-3-methyl-2-(6-(4-(2-(methylsulfonyl)pyrimidin-5-yl)-1H-1,2,3-triazol-1-yl)hexanamido)butanamido)hexanamide

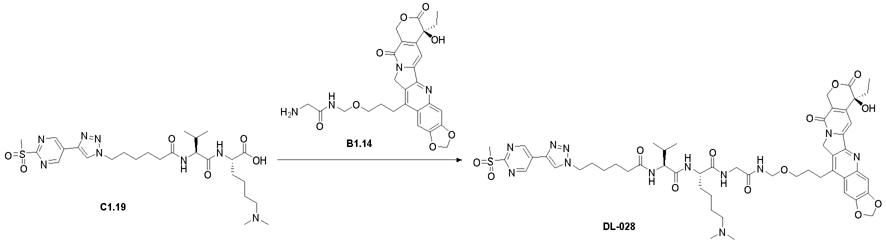

Compound C1.19 (40 mg, 0.06 mmol) was dissolved in N,N-dimethylformamide (1.5 mL), and HBTU (26 mg, 0.06 mmol), B1.14 (36 mg, 0.06 mmol), and DIPEA (66 mg, 0.17 mmol) were added sequentially. The mixture was stirred at room temperature for 1 h. The reaction was completed as detected by LCMS. The reaction solution was then separated and purified by preparative chromatography (acetonitrile/0.05% aqueous trifluoroacetic acid solution) to obtain the target compound (*S*)-6-(dimethylamino)-*N*-(2-(((3-((*S*)-7-ethyl-7-hydroxy-8,11-dioxo-7,8,11,13-tetrahydro-10*H*-[1,3]dioxolo[4,5-g]pyrano[3',4':6,7]indolizino[1,2-b]quinolin-14-yl)propoxy)methyl)amino)-2-oxoethyl)-2-((*S*)-3-methyl-2-(6-(4-(2-(methylsulfonyl)pyrimidin-5-yl)-1H-1,2,3-triazol-1-yl)hexanamido)butanamido)hexanamide (13.88 mg, TFA salt yield: 16%) as a beige solid.
LCMS (ESI) [M+H]⁺ = 1113.7;
¹H NMR (400 MHz, DMSO-d6) δ 9.46 (s, 2H), 9.29 (s, 1H, TFA salt), 8.92 (s, 1H), 8.63 (t, *J* = 6.6 Hz, 1H), 8.18 (br s, 1H), 8.03 (d, *J =* 7.6 Hz, 1H), 7.81 (d, *J =* 8.3 Hz, 1H), 7.59 (s, 1H), 7.50 (s, 1H), 7.24 (s, 1H), 6.48 (s, 1H), 6.28 (s, 2H), 5.42 (s, 2H), 5.24 (s, 2H), 4.64 - 4.55 (m, 2H), 4.46 (t, *J =* 7.0 Hz, 2H), 4.28 - 4.20 (m, 1H), 4.15 - 4.09 (m, 1H), 3.76 - 3.71 (m, 4H), 3.53 - 3.48 (m, 4H), 3.44 (s, 3H), 3.10 - 3.08 (m, 2H), 2.97 - 2.95 (m, 2H), 2.75 (d, *J =* 4.9 Hz, 6H), 2.20 - 2.09 (m, 2H), 1.92 - 1.83 (m, 5H), 1.68 - 1.65 (m, 1H), 1.63 - 1.50 (m, 5H), 0.90 - 0.74 (m, 9H).

### Example 2.29: (S)-6-(Diethylamino)-N-(2-(((3-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)propoxy)methyl)amino)-2-oxoethyl)-2-((S)-3-methyl-2-(6-(4-(2-(methylsulfonyl)pyrimidin-5-yl)-1H-1,2,3-triazol-1-yl)hexanamido)butanamido)hexanamide

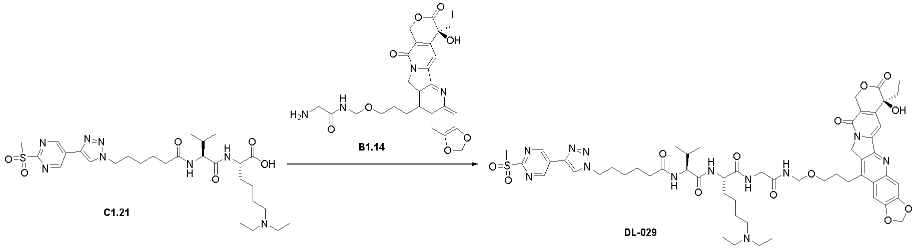

Compound C1.21 (58 mg, 0.09 mmol) and compound B1.14 (50 mg, 0.09 mmol) were dissolved in DMF (1 mL), and then HBTU (35 mg, 0.09 mmol) and *N*,*N*-diisopropylethylamine (30 mg, 0.23 mmol) were added. After the addition, the reaction solution was stirred at room temperature for 1 h. After the reaction was completed as detected by LCMS, the reaction solution was purified by preparative chromatography (0.01% TFA in water, MeCN) to obtain the target compound (*S*)-6-(diethylamino)-*N*-(2-(((3-((*S*)-7-ethyl-7-hydroxy-8,11-dioxo-7,8,11,13-tetrahydro-10*H*-[1,3]dioxolo[4,5-*g*]pyrano[3',4':6,7]indolizino[1,2-*b*]quinolin-14-yl)propoxy)methyl)amino)-2-oxoethyl)-2-((*S*)-3-methyl-2-(6-(4-(2-(methylsulfonyl)pyrimidin-5-yl)-1*H*-1,2,3-triazol-1-yl)hexanamido)butanamido)hexanamide (14 mg, yield: 13%) as a yellow solid.
LCMS (ESI) [M+H]⁺ = 1141.7;
¹H NMR (400 MHz, DMSO) δ 9.46 (s, 2H), 8.98 (s, 1H, TFA), 8.93 (s, 1H), 8.64 (t, *J* = 6.5 Hz, 1H), 8.18 (d, *J =* 5.7 Hz, 1H), 8.03 (d, *J =* 7.4 Hz, 1H), 7.81 (d, *J =* 8.5 Hz, 1H), 7.58 (s, 1H), 7.50 (d, *J =* 3.1 Hz, 1H), 7.24 (s, 1H), 6.52 (s, 1H), 6.28 (s, 2H), 5.42 (s, 2H), 5.23 (s, 2H), 4.62 - 4.55 (m, 2H), 4.46 (t, *J* = 6.9 Hz, 2H), 4.27 - 4.23 (m, 1H), 4.11 - 4.09 (m, 1H), 3.74 (d, *J =* 5.8 Hz, 2H), 3.49 (t, *J* = 5.8 Hz, 2H), 3.44 (s, 3H), 3.13 - 3.06 (m, 6H), 3.01 - 2.93 (m, 2H), 2.22 - 2.07 (m, 2H), 1.91 - 1.83 (m, 6H), 1.70- 1.51 (m, 5H), 1.33 - 1.21 (m, 6H), 1.16 (t, *J =* 7.2 Hz, 6H), 0.87 (t, *J =* 7.4 Hz, 3H), 0.82 - 0.76 (m, 6H).

### Example 2.30: (S)-6-(Dipropylamino)-N-(2-(((3-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)propoxy)methyl)amino)-2-oxoethyl)-2-((S)-3-methyl-2-(6-(4-(2-(methylsulfonyl)pyrimidin-5-yl)-1H-1,2,3-triazol-1-yl)hexanamido)butanamido)hexanamide

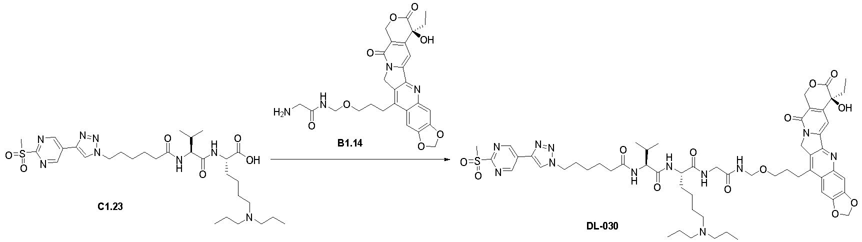

Compound C1.23 (61 mg, 0.09 mmol) and compound B1.14 (50 mg, 0.09 mmol) were dissolved in DMF (1 mL), and then HBTU (35 mg, 0.09 mmol) and *N*,*N*-diisopropylethylamine (30 mg, 0.23 mmol) were added. After the addition, the reaction solution was stirred at room temperature for 1 h. After the reaction was completed as detected by LCMS, the reaction solution was concentrated to obtain a crude product. The crude product was purified by preparative chromatography (0.01% TFA in water, MeCN) to obtain the target compound (*S*)-6-(dipropylamino)-*N*-2-(((3-((*S*)-7-ethyl-7-hydroxy-8,11-dioxo-7,8,11,13-tetrahydro-10*H*-[1,3]dioxolo[4,5-*g*]pyrano[3',4':6,7]indolizino[1,2-*b*]quinolin-14-yl)propoxy)methyl)amino)-2-oxoethyl)-2-((*S*)-3 -methyl-2-(6-(4-(2-(methylsulfonyl)pyrimidin-5-yl)-1*H*-1,2,3-triazol-1-yl)hexanamido)butanamido)hexanamide (21 mg, yield: 19%) as a yellow solid.
LCMS (ESI) [M+H]⁺ = 1169.8;
¹H NMR (400 MHz, DMSO) δ 9.46 (s, 2H), 9.01 (s, 1H, TFA), 8.93 (s, 1H), 8.64 (t, *J* = 6.4 Hz, 1H), 8.19 (t, *J* = 5.4 Hz, 1H), 8.02 (d, *J =* 7.3 Hz, 1H), 7.81 (d, *J* = 8.3 Hz, 1H), 7.58 (s, 1H), 7.51 (s, 1H), 7.24 (s, 1H), 6.50 (s, 1H), 6.28 (s, 2H), 5.42 (s, 2H), 5.23 (s, 2H), 4.60 - 4.58 (m, 2H), 4.46 (t, *J* = 6.9 Hz, 2H), 4.24 (d, *J* = 6.0 Hz, 1H), 4.13 - 4.09 (m, 1H), 3.74 (d, *J* = 5.7 Hz, 2H), 3.49 (t, *J* = 5.8 Hz, 2H), 3.44 (s, 3H), 3.10 (br s, 2H), 3.02 - 2.95 (m, 6H), 2.19 - 2.15 (m, 2H), 1.88 - 1.86 (m, 6H), 1.71 - 1.69 (m, 1H), 1.61 - 1.56 (m, 8H), 1.28 - 1.26 (m, 6H), 0.88 - 0.83 (m, 9H), 0.81 - 0.78 (m, 6H).

### Example 2.31: (S)-6-(Dimethylamino)-N-(2-(((3-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)propoxy)methyl)amino)-2-oxoethyl)-2-((S)-3-methyl-2-(6-(4-(2-(methylsulfonyl)pyrimidin-5-yl)-1H-1,2,3-triazol-1-yl)hexanamido)butanamido)hexanamide

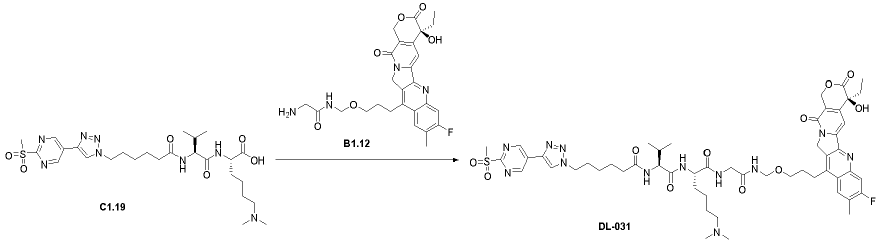

Compound C1.19 (113 mg, 0.19 mmol) and compound B1.12 (100 mg, 0.19 mmol) were dissolved in DMF (1 mL), and then HBTU (72 mg, 0.19 mmol) and *N*,*N*-diisopropylethylamine (61 mg, 0.48 mmol) were added. After the addition, the reaction solution was stirred at room temperature for 1 h. After the reaction was completed as detected by LCMS, the reaction solution was purified by preparative chromatography (0.01% TFA in water, acetonitrile) to obtain the target compound (*S*)-6-(dimethylamino)-*N*-(2-(((3-((*S*)-4-ethyl-8-fluoro-4-hydroxy-9-methyl-3,14-dioxo-3,4,12,14-tetrahydro-1*H*-pyrano[3',4':6,7]indolizino[1,2-*b*]quinolin-11-yl)propoxy)methyl)amino)-2-oxoethyl)-2-((*S*)-3-methyl-2-(6-(4-(2-(methylsulfonyl)pyrimidin-5-yl)-1*H*-1,2,3-triazol-1-yl)hexanamido)butanamido)hexanamide (16 mg, yield: 8%) as a white solid.
LCMS (ESI) [M+H]⁺= 1101.6;
¹H NMR (400 MHz, DMSO) δ 9.46 (s, 2H), 8.93 (s, 1H), 8.63 (t, *J* = 6.3 Hz, 1H), 8.20 - 8.28 (m, 2H), 8.04 (*d, J =* 7.2 Hz, 1H), 7.88 (*d, J =* 10.8 Hz, 1H), 7.81 (*d, J =* 8.3 Hz, 1H), 7.31 (s, 1H), 6.53 (s, 1H), 5.44 (s, 2H), 5.29 (s, 2H), 4.63 - 4.54 (m, 2H), 4.46 (t, *J =* 7.0 Hz, 2H), 4.29 - 4.19 (m, 1H), 4.15 - 4.08 (m, 1H), 3.73 (d, *J =* 6.9 Hz, 2H), 3.50 (t, *J =* 5.8 Hz, 2H), 3.44 (s, 3H), 3.24 - 3.14 (m, 2H), 3.01 - 2.91 (m, 2H), 2.72 (s, 6H), 2.53 (s, 3H), 2.23 - 2.07 (m, 2H), 1.93 - 1.84 (m, 6H), 1.72 - 1.64 (m, 1H), 1.61 - 1.45 (m, 6H), 1.34 - 1.22 (m, 4H), 0.87 (t, *J=* 7.4 Hz, 3H), 0.82 - 0.76 (m, 6H).

### Example 2.32: (S)-6-(Diethylamino)-N-(2-(((3-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)propoxy)methyl)amino)-2-oxoethyl)-2-((S)-3-methyl-2-(6-(4-(2-(methylsulfonyl)pyrimidin-5-yl)-1H-1,2,3-triazol-1-yl)hexanamido)butanamido)hexanamide

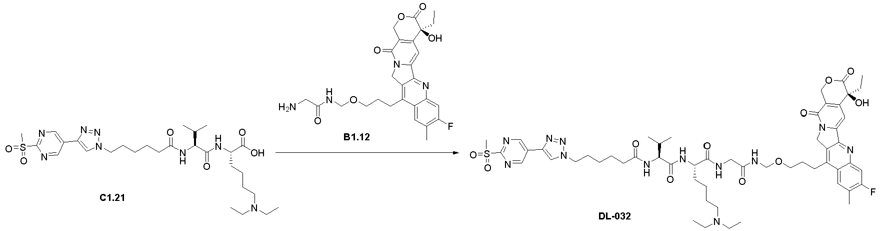

Compound C1.21 (59 mg, 0.10 mmol) and compound B1.12 (50 mg, 0.10 mmol) were dissolved in DMF (1 mL), and then HBTU (36 mg, 0.10 mmol) and N,N-diisopropylethylamine (31 mg, 0.24 mmol) were added. After the addition, the reaction solution was stirred at room temperature for 1 h. After the reaction was completed as detected by LCMS, the reaction solution was purified by preparative chromatography (0.01% formic acid solution, acetonitrile) to obtain the target compound (*S*)-6-(diethylamino)-*N*-(2-(((3-((*S*)-4-ethyl-8-fluoro-4-hydroxy-9-methyl-3,14-dioxo-3,4,12,14-tetrahydro-1*H*-pyrano[3',4':6,7]indolizino[1,2-*b*]quinolin-11-yl)propoxy)methyl)amino)-2-oxoethyl)-2-((*S*)-3-methyl-2-(6-(4-(2-(methylsulfonyl)pyrimidin-5-yl)-1H-1,2,3-triazol-1-yl)hexanamido)butanamido)hexanamide (33.8 mg, yield: 32%) as a white solid.
LCMS (ESI) [M+H]⁺ = 1129.7;
¹H NMR (400 MHz, DMSO) δ 9.46 (s, 2H), 8.93 (s, 1H), 8.61 (t, *J* = 6.4 Hz, 1H), 8.29 (s, 1H), 8.24 - 8.15 (m, 2H), 8.00 (d, *J* = 7.2 Hz, 1H), 7.85 (d, *J =* 10.5 Hz, 2H), 7.31 (s, 1H), 5.44 (s, 2H), 5.26 (s, 2H), 4.63 - 4.55 (m, 2H), 4.46 (t, *J=* 6.8 Hz, 2H), 4.23 - 4.18 (m, 1H), 4.11 (d, *J=* 7.9 Hz, 1H), 3.77 - 3.69 (m, 2H), 3.50 (t, *J* = 5.9 Hz, 2H), 3.44 (s, 3H), 3.17 - 3.15 (m, 3H), 2.61 (q, *J =* 6.7 Hz, 4H), 2.22 - 2.10 (m, 2H), 1.92 - 1.84 (m, 6H), 1.68 (d, *J* = 6.7Hz, 1H), 1.57 - 1.48 (m, 3H), 1.39 (d, *J* = 5.9 Hz, 2H), 1.30 - 1.22 (m, 4H), 0.97 (t, *J* = 6.9 Hz, 6H), 0.88 (*t, J =* 7.3 Hz, 3H), 0.82 - 0.77 (m, 6H).

### Example 2.33: (S)-6-(Dipropylamino)-N-(2-(((3-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)propoxy)methyl)amino)-2-oxoethyl)-2-((S)-3-methyl-2-(6-(4-(2-(methylsulfonyl)pyrimidin-5-yl)-1H-1,2,3-triazol-1-yl)hexanamido)butanamido)hexanamide

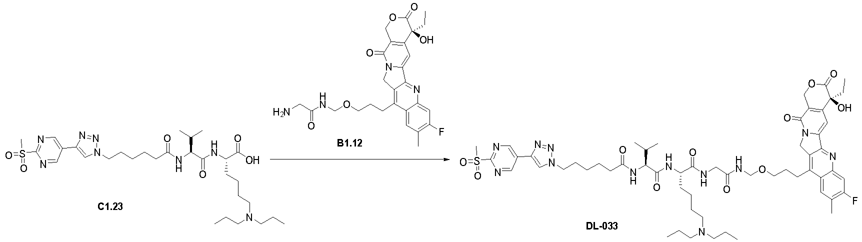

Compound C1.23 (62 mg, 0.10 mmol) and compound B1.12 (50 mg, 0.10 mmol) were dissolved in DMF (1 mL), and then HBTU (36 mg, 0.10 mmol) and *N*,*N*-diisopropylethylamine (31 mg, 0.24 mmol) were added. After the addition, the reaction solution was stirred at room temperature for 1 h. After the reaction was completed as detected by LCMS, the reaction solution was purified by preparative chromatography (0.01% TFA in water, MeCN) to obtain the target compound (*S*)-6-(dipropylamino)-*N*-(2-(((3-((*S*)-4-ethyl-8-fluoro-4-hydroxy-9-methyl-3,14-dioxo-3,4,12,14-tetrahydro-1*H*-pyrano[3',4':6,7]indofizino[1,2-*b*]quinolin-11-yl)propoxy)methyl)amino)-2-oxoethyl)-2-((S)-3-methyl-2-(6-(4-(2-(methylsulfonyl)pyrimidin-5-yl)-1*H*-1,2,3-triazol-1-yl)hexanamido)butanamido)hexanamide (35.2 mg, yield: 32%) as a yellow solid.
LCMS (ESI) [M+H]⁺ = 1157.8;
¹H NMR (400 MHz, DMSO) δ 9.46 (s, 2H), 9.05 (s, 1H, TFA), 8.93 (s, 1H), 8.64 (t, *J* = 6.7 Hz, 1H), 8.20 (d, *J* = 7.4 Hz, 2H), 8.03 (d, *J* = 7.2 Hz, 1H), 7.88 (d, *J* = 10.8 Hz, 1H), 7.81 (d, *J* = 8.4 Hz, 1H), 7.32 (s, 1H), 6.54 (s, 1H), 5.44 (s, 2H), 5.28 (s, 2H), 4.62 - 4.55 (m, 2H), 4.46 *(t, J=* 7.0 Hz, 2H), 4.24 (m, 1H), 4.13 - 4.07 (m, 1H), 3.74 (m, 2H), 3.50 (m, 2H), 3.44 (s, 3H), 3.23 - 3.16 (m, 2H), 3.01 - 2.95 (m, 6H), 2.50 (s, 3H), 2.22 - 2.09 (m, 2H), 1.95 - 1.84 (m, 8H), 1.67 - 1.56 (m, 9H), 1.33 - 1.23 (m, 6H), 0.91 - 0.86 (m, 9H), 0.82 - 0.77 (m, 6H).

### Example 2.34: N-((11S,14S)-11-(4-(Diethylamino)butyl)-1-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)-15-methyl-7,10,13-trioxo-4-oxa-6,9,12-triazahexadecan-14-yl)-6-(2-(methylsulfonyl)pyrimidin-5-yl)hex-5-ynamide (DL-034)

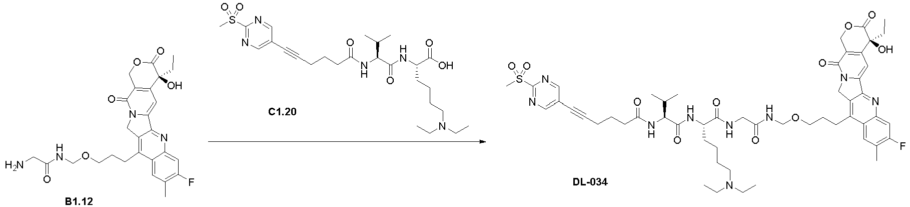

Compound (*S*)-2-amino-*N*-((3-(4-ethyl-8-fluoro-4-hydroxy-9-methyl-3,14-dioxo-3,4,12,14-tetrahydro-1*H*-pyrano[3',4':6,7]indolizino[1,2-*b*]quinolin-11-yl)propoxy)methyl)acetamide (B1.12, 30 mg, 0.057 mmol) and N6,N6-diethyl-N2-((6-(2-(methylsulfonyl)pyrimidin-5-yl)hex-5-ynoyl)-*L*-valyl)-*L*-lysine (C1.20, 31 mg, 0.057 mmol) were dissolved in DMF (1 mL). Then, HBTU (22 mg, 0.057 mmol) and *N*,*N*-diisopropylethylamine (18 mg, 0.143 mmol) were added sequentially, and after the addition, the reaction solution was stirred at room temperature for one hour. After the reaction was completed as detected by LCMS, the reaction solution was purified by preparative chromatography (0.01% aqueous trifluoroacetic acid solution, acetonitrile) to obtain the target compound *N*-((11*S*,14*S*)-11-(4-(diethylamino)butyl)-1-((*S*)-4-ethyl-8-fluoro-4-hydroxy-9-methyl-3,14-dioxo-3,4,12,14-tetrahydro-1*H*-pyrano[3',4':6,7]indolizino[1,2-*b*]quinolin-11-yl)-15-methyl-7,10,13-trioxo-4-oxa-6,9,12-triazahexadecan-14-yl)-6-(2-(methylsulfonyl)pyrimidin-5-yl)hex-5-ynamide trifluoroacetate (3.18 mg, yield: 5%) as a yellow solid.
LCMS (ESI) [M+H]⁺ = 1058.7;
¹H NMR (400 MHz, DMSO-d6) δ 9.10 (s, 2H), 8.65 (t, *J* = 6.5 Hz, 1H), 8.21 - 8.19 (m, 2H), 8.09 (d, *J =* 7.3 Hz, 1H), 7.92 (d, *J =* 8.6 Hz, 1H), 7.88 (d, *J =* 10.8 Hz, 1H), 7.32 (s, 1H), 6.53 (s, 1H), 5.44 (s, 2H), 5.28 (s, 2H), 4.64 - 4.56 (m, 2H), 4.29 - 4.22 (m, 1H), 4.15 (t, *J =* 7.4 Hz, 1H), 3.74 (d, *J* = 4.6 Hz, 2H), 3.51 - 3.49 (m, 2H), 3.24 - 3.18 (m, 2H), 3.13 - 3.07 (m, 4H), 3.01 - 2.95 (m, 2H), 2.55 (s, 3H), 2.40 - 2.31 (m, 3H), 1.99 - 1.74 (m, 8H), 1.65 - 1.49 (m, 4H), 1.38 - 1.25 (m, 2H), 1.16 (t, *J =* 7.2 Hz, 6H), 0.89 - 0.79 (m, 9H).

### Example 2.35: N-((11S,14S)-11-(4-(Dipropylamino)butyl)-1-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)-15-methyl-7,10,13-trioxo-4-oxa-6,9,12-triazahexadecan-14-yl)-6-(2-(methylsulfonyl)pyrimidin-5-yl)hex-5-ynamide (DL-035)

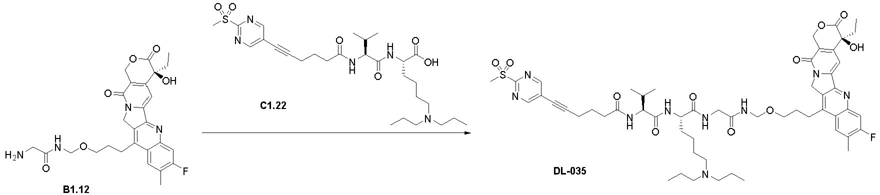

Compound (*S*)-2-amino-*N*-((3-(4-ethyl-8-fluoro-4-hydroxy-9-methyl-3,14-dioxo-3,4,12,14-tetrahydro-1*H*-pyrano[3',4':6,7]indolizino[1,2-b]quinolin-11-yl)propoxy)methyl)acetamide (B1.12, 30 mg, 0.057 mmol) and *N*²-((6-(2-(methylsulfonyl)pyrimidin-5-yl)hex-5-ynoyl)-L-propionyl)-*N*⁶,*N*⁶-dipropyl-*L*-lysine (C1.22, 33 mg, 0.057 mmol) were dissolved in DMF (1 mL). Then, HBTU (22 mg, 0.057 mmol) and *N*,*N*-diisopropylethylamine (18 mg, 0.143 mmol) were added sequentially, and after the addition, the reaction solution was stirred at room temperature for one hour. After the reaction was completed as detected by LCMS, the reaction solution was purified by preparative chromatography (0.01% aqueous trifluoroacetic acid solution, acetonitrile) to obtain the target compound *N*-((11*S*,14*S*)-11-(4-(dipropylamino)butyl)-1-((*S*)-4-ethyl-8-fluoro-4-hydroxy-9-methyl-3,14-dioxo-3,4,12,14-tetrahydro-1*H*-pyrano[3',4':6,7]indolizino[1,2-*b*]quinolin-11-yl)-15-methyl-7,10,13-trioxo-4-oxa-6,9,12-triazahexadecan-14-yl)-6-(2-(methylsulfonyl)pyrimidin-5-yl)hex-5-ynamide (DL-035) (10.65 mg, yield: 16%) as a yellow solid.
LCMS (ESI) [M+H]⁺ = 1086.7;
¹H NMR (400 MHz, DMSO-d6) δ 9.10 (s, 2H), 8.65 (t, *J* = 6.2 Hz, 1H), 8.28 - 8.16 (m, 2H), 8.08 (d, *J =* 6.8 Hz, 1H), 7.92 (d, *J* = 8.1 Hz, 1H), 7.88 (d, *J* = 10.9 Hz, 1H), 7.32 (s, 1H), 6.53 (s, 1H), 5.44 (s, 2H), 5.29 (s, 2H), 4.63 - 4.52 (m, 2H), 4.31 - 4.19 (m, 1H), 4.19 - 4.08 (m, 1H), 3.74 (d, *J =* 4.9 Hz, 2H), 3.50 (t, *J* = 5.2 Hz, 2H), 3.41 (s, 3H), 3.24 - 3.18 (m, 2H), 3.03 - 2.94 (m, 6H), 2.55 (s, 3H), 2.46 - 2.24 (m, 3H), 2.05 - 1.67 (m, 8H), 1.66 - 1.51 (m, 8H), 1.36 - 1.22 (m, 4H), 0.91 - 0.80 (m, 15H).

### Example 2.36: N-((11S,14S)-11-(4-(Diethylamino)butyl)-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)-15-methyl-7,10,13-trioxo-4-oxa-6,9,12-triazahexadecan-14-yl)-6-(2-(methylsulfonyl)pyrimidin-5-yl)hex-5-ynamide (DL-036)

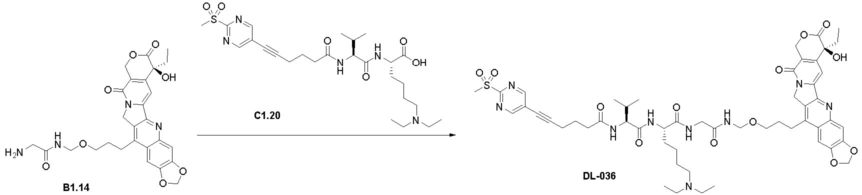

Compound C1.20 (40 mg, 0.075 mmol) and compound B1.14 (41 mg, 0.075 mmol) were dissolved in DMF (1 mL), and then HOBt (15.2 mg, 0.113 mmol) and 1-(3-dimethylaminopropyl)-3-ethylcarbodiimide hydrochloride (21.5 mg, 0.113 mmol) were added, followed by the addition of triethylamine (23 mg, 0.225 mmol). After the addition, the reaction solution was stirred at room temperature for 16 h. After the reaction was completed as detected by LCMS, the reaction solution was concentrated to obtain a crude product. The crude product was purified by preparative chromatography (0.01% TFA in water, MeCN) to obtain the target compound *N*-((11*S*,14*S*)-11-(4-(diethylamino)butyl)-1-((*S*)-7-ethyl-7-hydroxy-8,11-dioxo-7,8,11,13-tetrahydro-10*H*-[1,3]dioxolo[4,5-g]pyrano[3',4':6,7]indolizino[1,2-*b*]quinolin-14-yl)-15-methyl-7,10,13-trioxo-4-oxa-6,9,12-triazahexadecan-14-yl)-6-(2-(methylsulfonyl)pyrimidin-5-yl)hex-5-ynamide (16 mg, yield: 20%) as a yellow solid.
LCMS (ESI) [M+H]⁺ = 1070.6;
¹H NMR (400 MHz, DMSO-d6) δ 9.13 -9.08 (m, 2H), 9.01 (s, 1H), 8.64 (t, *J* = 6.5 Hz, 1H), 8.20 (t, *J* = 5.7 Hz, 1H), 8.09 (d, *J* = 7.4 Hz, 1H), 7.92 (d, *J* = 8.4 Hz, 1H), 7.59 (s, 1H), 7.51 (s, 1H), 7.24 (s, 1H), 6.50 (s, 1H), 6.29 (s, 2H), 5.43 (s, 2H), 5.24 (s, 2H), 4.65 - 4.53 (m, 2H), 4.26 (d, *J* = 6.5 Hz, 1H), 4.20 - 4.10 (m, 1H), 3.74 (d, *J=* 5.5 Hz, 2H), 3.50 (t, *J* = 5.8 Hz, 2H), 3.41 (s, 3H), 3.14 - 3.07 (m, 6H), 2.98 (s, 2H), 2.55 (d, *J =* 7.3 Hz, 2H), 2.41 - 2.29 (m, 2H), 2.03 - 1.89 (m, 2H), 1.89 - 1.77 (m, 7H), 1.61 - 1.56 (m, 2H), 1.32 (s, 2H), 1.16 (t, *J=* 7.2 Hz, 6H), 0.91 - 0.78 (m, 9H).

### Example 2.37: N-((11S,14S)-11-(4-(Di-n-propylamino)butyl)-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)-15-methyl-7,10,13-trioxo-4-oxa-6,9,12-triazahexadecan-14-yl)-6-(2-(methylsulfonyl)pyrimidin-5-yl)hex-5-ynamide (DL-037)

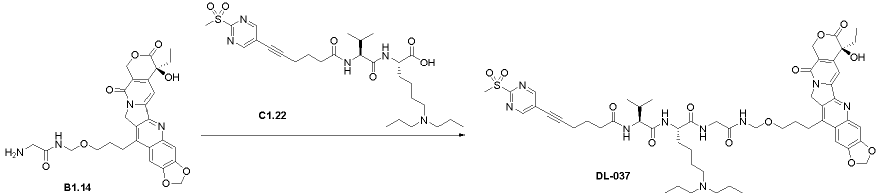

Compound C1.22 (43 mg, 0.074 mmol) and compound B1.14 (40 mg, 0.075 mmol) were dissolved in *N,N*-dimethylformamide (1 mL), and then HBTU (28 mg, 0.075 mmol) and *N,N*-diisopropylethylamine (24 mg, 0.187 mmol) were added sequentially. The reaction solution was stirred at room temperature for 1 h. After the reaction was completed as detected by LCMS, the reaction solution was directly purified by preparative chromatography (0.01% aqueous trifluoroacetic acid solution, acetonitrile) to obtain a trifluoroacetate of the target compound (8.5 mg, yield: 10%) as a yellow solid.
LCMS (ESI) [M+H]⁺ = 1098.6;
¹H NMR (400 MHz, DMSO-d6) δ 9.10 (s, 2H), 9.03 (s, 1H), 8.64 (t, *J* = 6.4 Hz, 1H), 8.19 (t, *J* = 5.9 Hz, 1H), 8.08 (d*, J* = 7.4 Hz, 1H), 7.92 (d, *J* = 8.5 Hz, 1H), 7.59 (s, 1H), 7.51 (s, 1H), 7.24 (s, 1H), 6.49 (s, 1H), 6.29 (s, 2H), 5.42 (s, 2H), 5.24 (s, 2H), 4.66 - 4.52 (m, 2H), 4.31 - 4.21 (m, 1H), 4.19 - 4.10 (m, 1H), 3.74 (d, *J =* 5.5 Hz, 2H), 3.49 - 3.48 (m, 2H), 3.40 (s, 3H), 3.15 - 3.06 (m, 2H), 3.02 - 2.95 (m, 6H), 2.59 - 2.52 (m, 3H), 2.41 - 2.29 (m, 2H), 2.05 - 1.90 (m, 2H), 1.91 - 1.77 (m, 6H), 1.63 - 1.57 (m, 6H), 1.31 - 1.29 (m, 2H), 0.92 - 0.80 (m, 15H).

Following the same procedures as in this example, the reaction solution was directly purified by preparative chromatography (0.1% aqueous formic acid solution, acetonitrile) and lyophilized to obtain a formate of the target compound (32.6 mg, yield: 39%) as a white solid.
LCMS (ESI) [M+H]⁺ = 1098.6;
¹H NMR (400 MHz, DMSO-d₆) δ 9.09 (s, 2H), 8.56 *(t, J=* 6.5 Hz, 1H), 8.17 (s, 1H), 8.14 (t, *J=* 5.7 Hz, 1H), 7.97 (d, *J* = 7.3 Hz, 1H), 7.89 (d, *J* = 8.6 Hz, 1H), 7.58 (s, 1H), 7.50 (s, 1H), 7.24 (s, 1H), 6.45 (s, 1H), 6.28 (s, 2H), 5.48 - 5.35 (m, 2H), 5.24 (s, 2H), 4.64 - 4.54 (m, 2H), 4.23 - 4.07 (m, 2H), 3.79 - 3.66 (m, 2H), 3.50 (t, *J* = 5.9 Hz, 2H), 3.40 (s, 3H), 3.14 - 3.08 (t, *J* = 8.2 Hz, 2H), 2.59 - 2.52 (m, 2H), 2.36 - 2.27 (m, 8H), 2.00 - 1.76 (m, 7H), 1.72- 1.62 (m, 1H), 1.58- 1.48 (m, 1H), 1.40 - 1.20 (m, 8H), 0.88 - 0.77 (m, 15H).

DL-037 formate (16 mg) was taken, purified by preparative HPLC (0.1% ammonium bicarbonate in water, acetonitrile), and lyophilized to obtain the target compound in its free form (DL-037, 10 mg, yield: 65%) as a white solid.
LCMS (ESI) [M+H]⁺ = 1098.6;
¹H NMR (400 MHz, DMSO-d6) δ 9.10 (s, 2H), 8.61 (t, *J* = 6.4 Hz, 1H), 8.21 (t, *J* = 5.6 Hz, 1H), 8.04 (d, J = 7.2 Hz, 1H), 7.95 (d, *J=* 8.5 Hz, 1H), 7.53 (s, 1H), 7.48 (s, 1H), 7.22 (s, 1H), 6.50 (s, 1H), 6.26 (s, 2H), 5.48 - 5.35 (m, 2H), 5.16 (s, 2H), 4.63 - 4.51 (m, 2H), 4.25 - 4.10 (m, 2H), 3.82 - 3.63 (m, 2H), 3.49 (t, *J* = 5.8 Hz, 2H), 3.40 (s, 3H), 3.06 (t, *J* = 8.0 Hz, 2H), 2.56 - 2.51 (m, 2H), 2.41 - 2.23 (m, 8H), 1.96 - 1.76 (m, 7H), 1.71 - 1.57 (m, 1H), 1.57 - 1.48 (m, 1H), 1.37 - 1.21 (m, 8H), 0.88 - 0.78 (m, 15H).

### Example 2.38: (S)-6-(Dipropylamino)-N-(2-(((((E)-3-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)allyl)oxy)methyl)amino)-2-oxoethyl)-2-((S)-3-methyl-2-(6-(4-(2-(methylsulfonyl)pyrimidin-5-yl)-1H-1,2,3-triazol-1-yl)hexanamido)butanamido)hexanamide

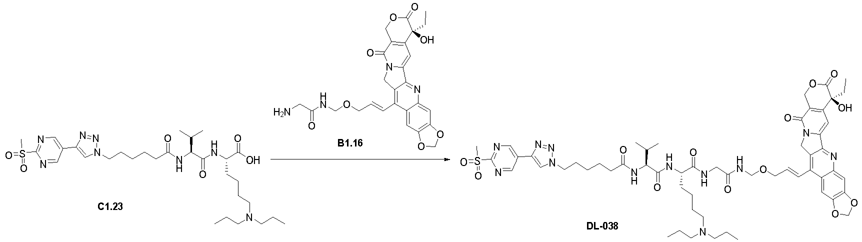

Compound B1.16 (30 mg, 0.06 mmol) and compound C1.23 (37 mg, 0.06 mmol) were dissolved in DMF (1 mL). Then, HBTU (21 mg, 0.06 mmol) and *N,N*-diisopropylethylamine (16 mg, 0.12 mmol) were added. After the addition, the reaction solution was stirred at room temperature for 1 h. After the reaction was completed as detected by LCMS, the reaction solution was purified by preparative chromatography (0.01% TFA in water, MeCN) to obtain the target compound (*S*)-6-(dipropylamino)-*N*-(2-(((((*E*)-3-((*S*)-7-ethyl-7-hydroxy-8,11-dioxo-7,8,11,13-tetrahydro-10*H*-[1,3]dioxolo[4,5-*g*]pyrano[3',4':6,7]indolizino[1,2-*b*]quinolin-14-yl)allyl)oxy)methyl)amino)-2-oxoethyl)-2-((*S*)-3-methyl-2-(6-(4-(2-(methylsulfonyl)pyrimidin-5-yl)-1*H*-1,2,3-triazol-1-yl)hexanamido)butanamido)hexanamide (4 mg, yield: 6%) as a yellow solid.
LCMS (ESI) [M+H]⁺ = 1167.6;
¹H NMR (400 MHz, DMSO) δ 9.45 (s, 2H), 8.98 (s, 1H, TFA), 8.92 (s, 1H), 8.74 (t, *J =* 6.8 Hz, 1H), 8.25 (t, *J =* 5.8 Hz, 1H), 8.05 (d, *J =* 7.3 Hz, 1H), 7.82 (d, *J =* 8.4 Hz, 1H), 7.63 (s, 1H), 7.53 (s, 1H), 7.26 (d, *J =* 12.0 Hz, 1H), 7.26 (s, 1H), 6.58 - 6.52 (m, 1H), 6.50 (s, 1H), 6.30 (s, 2H), 5.42 (s, 2H), 5.31 (s, 2H), 4.74 - 4.67 (m, 2H), 4.46 (m, 2H), 4.30 (m, 1H), 4.10 (m, 1H), 3.77 (m, 2H), 3.44 (s, 3H), 3.01 - 2.95 (m, 8H), 2.23 - 2.18 (m, 1H), 2.13 (m, 1H), 2.00 (m, 2H), 1.90 - 1.84 (m, 6H), 1.63 - 1.57 (m, 9H), 0.88 (m, 9H), 0.81 - 0.78 (m, 6H).

### Example 2.39: 4-((S)-6-Amino-2-(6-(2-(methylsulfonyl)pyrimidin-5-yl)hex-5-ynamido)hexanamido)benzyl ethyl(2-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)ethyl)carbamate

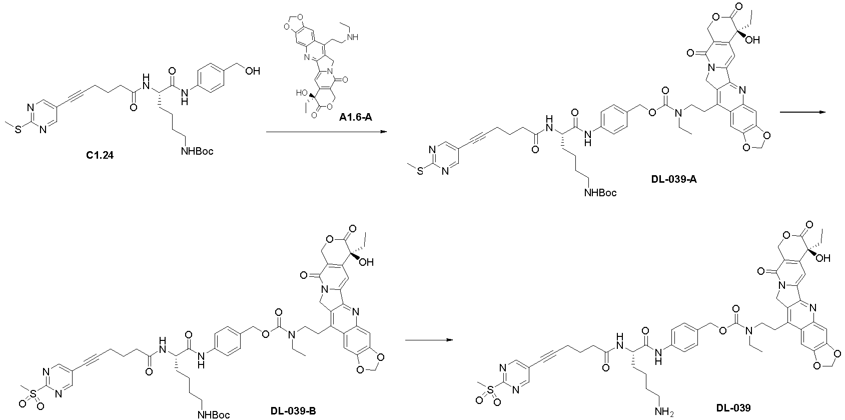

### Step 1:

To a solution of C1.24 (150 mg, 0.264 mmol) and triethylamine (89 mg, 0.88 mmol) in dichloromethane (10 mL) was added *N,N*-succinimidyl carbonate (DSC, 90 mg, 0.352 mmol) under an ice bath at 0 °C, and the reaction mixture was allowed to react under a nitrogen atmosphere at 0 °C for 30 min. Then, compound A1.6-A (82 mg, 0.176 mmol) was added dropwise, and the reaction mixture was allowed to react at room temperature for 12 h. The reaction was completed as monitored by LCMS. The reaction solution was concentrated under reduced pressure to obtain a crude product. The crude product was purified by preparative high-performance liquid chromatography (acetonitrile/water containing 0.05% formic acid) to obtain the compound DL-039-A (27 mg, yield: 14.4%) as a yellow oil.

LCMS (ESI) [M+H]⁺ = 1059.2;

### Step 2:

To a mixed solution of compound DL-039-A (60 mg, 0.057 mmol) in tetrahydrofuran (5 mL) and water (1 mL) was added potassium peroxymonosulfonate (Oxone, 349 mg, 0.567 mmol), and the reaction mixture was stirred at room temperature for 12 h. The reaction was completed as detected by TLC. Water (10 mL) was added to the reaction solution, and the mixture was extracted with dichloromethane (10 mL × 3). The organic phase was washed with saturated brine, dried over anhydrous sodium sulfate, filtered under vacuum, and concentrated under reduced pressure to obtain the target compound DL-039-B (60 mg, crude product) as a yellow solid.

LCMS (ESI) [M+H]⁺ = 1091.0;

### Step 3:

Compound DL-039-B (60 mg, 0.055 mmol) was dissolved in a dichloromethane solution (1 mL), and dichloroacetic acid (1 mL) was added to the reaction solution at 0 °C. The reaction mixture was stirred at room temperature for 3 h. The reaction was completed as detected by LCMS. The filtrate was concentrated under reduced pressure to obtain a crude product, which was then purified by preparative high-performance liquid chromatography (acetonitrile/water containing 0.05% trifluoroacetic acid) to obtain the compound 4-((*S*)-6-amino-2-(6-(2-(methylsulfonyl)pyrimidin-5-yl)hex-5-ynamido)hexanamido)benzyl ethyl(2-((*S*)-7-ethyl-7-hydroxy-8,11-dioxo-7,8,11,13-tetrahydro-10*H*-[1,3]dioxolo[4,5-*g*]pyrano[3',4':6,7]indolizino[1,2-*b*]quinolin-14-yl)ethyl)carbamate (2.44 mg, yield: 3.69%) as a yellow solid.

LCMS (ESI) [1/2M+H]⁺ = 496.3, t_{R} = 2.252 min.

### Example 2.40: 4-((S)-6-Amino-2-(6-(4-(2-(methylsulfonyl)pyrimidin-5-yl)-1H-1,2,3-triazol-1-yl)hexanamido)hexanamido)benzyl N-ethyl-N-(2-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)ethyl)carbamate (DL-040)

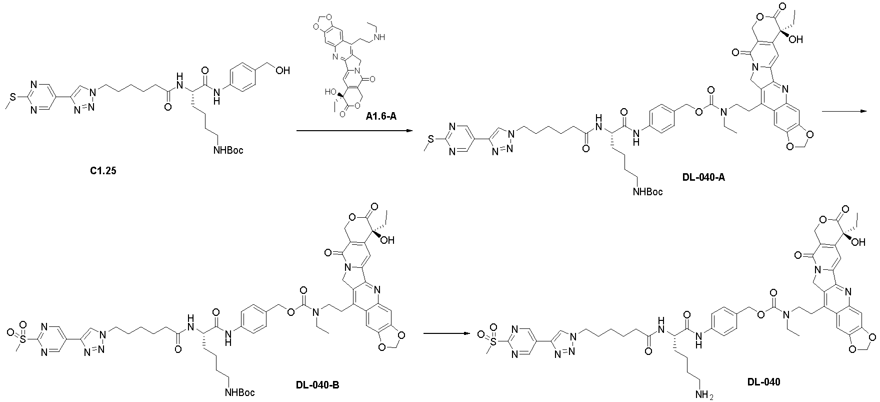

### Step 1:

To a solution of C1.25 (300 mg, 0.469 mmol) and triethylamine (126 mg, 1.25 mmol) in dichloromethane (10 mL) was added *N,N*-succinimidyl carbonate (DSC, 160 mg, 0.626 mmol) under an ice bath at 0 °C, and the reaction mixture was allowed to react at 0 °C for 30 min. Then, compound A1.6-A (145 mg, 0.313 mmol) was added dropwise, and the reaction mixture was allowed to react at room temperature for 12 h. The reaction was completed as monitored by LCMS. The reaction solution was concentrated under reduced pressure to obtain a crude product, which was then purified by preparative high-performance liquid chromatography (acetonitrile/water containing 0.05% formic acid) to obtain the compound DL-040-A (70 mg, yield: 19.8%) as a yellow oil. LCMS (ESI) [M+H]⁺ = 1130.1.

### Step 2:

To a mixed solution of compound DL-040-A (70 mg, 0.062 mmol) in tetrahydrofuran (5 mL) and water (1 mL) was added potassium peroxymonosulfonate (Oxone, 381 mg, 0.619 mmol), and the reaction mixture was stirred at room temperature for 12 h. The reaction was completed as detected by TLC. Water (10 mL) was added to the reaction solution, and the mixture was extracted with dichloromethane (10 mL × 3). The organic phase was washed with saturated brine, dried over anhydrous sodium sulfate, filtered under vacuum, and concentrated under reduced pressure to obtain the target compound DL-040-B (70 mg, crude product) as a yellow solid. LCMS (ESI) [M+H]⁺ = 1162.6, t_{R} = 1.710 min.

### Step 3:

Compound DL-040-B (70 mg, 0.060 mmol) was dissolved in a dichloromethane solution (1 mL), and dichloroacetic acid (1 mL) was added to the reaction solution at 0 °C. The reaction mixture was stirred at room temperature for 3 h. The reaction was completed as detected by LCMS. The filtrate was concentrated under reduced pressure to obtain a crude product, which was then purified by preparative high-performance liquid chromatography (acetonitrile/water containing 0.05% trifluoroacetic acid) to obtain the compound 4-((*S*)-6-amino-2-(6-(4-(2-(methylsulfonyl)pyrimidin-5-yl)-1*H*-1,2,3-triazol-1-yl)hexanamido)hexanamido)benzyl *N-*ethyl-*N*-(2-((*S*)-7-ethyl-7-hydroxy-8,11-dioxo0-7,8,11,13-tetrahydro-10*H*-[1,3|dioxolo[4,5-*g*]pyrano[3'.4":6,7]indolizino[1,2-*b*]quinolin-14-yl)ethyl)carbamate (5.54 mg, yield: 8.66%) as a yellow solid.
LCMS (ESI) [1/2M+H]⁺ = 1062.5, t_{R} = 4.052 min;
¹H NMR (400 MHz, DMSO-d6) δ 10.22 (br s, 1H), 9.46 (s, 2H), 8.93 (s, 1H), 8.22 - 8.03 (m, 1H), 7.85 (br s, 3H, NH2-TFA), 7.61 - 7.45 (m, 3H), 7.32 - 7.26 (m, 1H), 7.26 - 7.17 (m, 2H), 6.54 (s, 1H), 6.35 (s, 2H), 5.91 (s, 2H), 5.47 (s, 2H), 5.37 - 5.30 (m, 1H), 5.27 - 5.20 (m, 1H), 5.10 - 5.00 (m, 1H), 4.97 - 4.78 (m, 1H), 4.52 - 4.26 (m, 3H), 3.56 - 3.46 (m, 2H), 3.44 (s, 3H), 2.83 - 2.71 (m, 2H), 2.21 - 2.11 (m, 2H), 1.98 - 1.78 (m, 4H), 1.67 - 1.48 (m, 6H), 1.37 - 1.22 (m, 4H), 1.09 - 0.96 (m, 3H), 0.93 - 0.78 (m, 3H).

### Example 2.41: (S)-6-(Dimethylamino)-N-(2-(((((E)-3-((S)-4-ethyl-8-fluoro-4-hydroxy-9-methyl-3,14-dioxo-3,4,12,14-tetrahydro-1H-pyrano[3',4':6,7]indofizino[1,2-b]quinolin-11-yl)allyl)oxy)methyl)amino)-2-oxoethyl)-2-((S)-3-methyl-2-(6-(4-(2-(methylsulfonyl)pyrimidin-5-yl)-1H-1,2,3-triazol-1-yl)hexanamido)butanamido)hexanamide (DL-041)

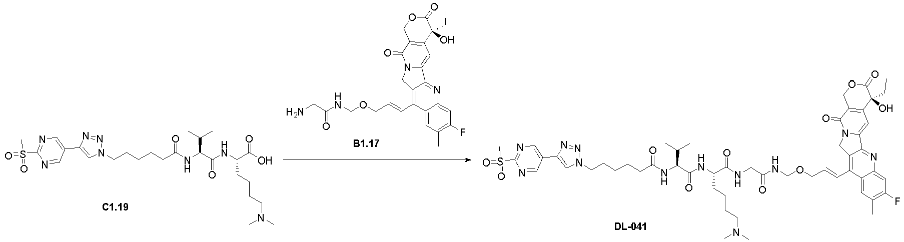

Compound B1.17 (30 mg) and compound C1.19 (37 mg) were dissolved in DMF (1 mL). Then, HBTU (21 mg) and *N*,*N*-diisopropylethylamine (16 mg) were added. After the addition, the reaction solution was stirred at room temperature for 1 h. After the reaction was completed as detected by LCMS, the reaction solution was purified by preparative chromatography (0.01% TFA in water, MeCN) to obtain the target compound (*S*)-6-(dimethylamino)-*N*-(2-(((((*E*)-3-((*S*)-4-ethyl-8-fluoro-4-hydroxy-9-methyl-3,14-dioxo-3,4,12,14-tetrahydro-1*H-*pyrano[3',4':6,7]indolizino[1,2-*b*]quinolin-11-yl)allyl)oxy)methyl)amino)-2-oxoethyl)-2-((*S*)-3-methyl-2-(6-(4-(2-(methylsulfonyl)pyrimidin-5-yl)-1*H*-1,2,3-triazol-1-yl)hexanamido)butanamido)hexanamide (11 mg).

LCMS (ESI) [M+H]⁺ = 1099.5.

### Example 2.42: (S)-6-(Diethylamino)-N-(2-(((((E)-3-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)allyl)oxy)methyl)amino)-2-oxoethyl)-2-((S)-3-methyl-2-(6-(4-(2-(methylsulfonyl)pyrimidin-5-yl)-1H-1,2,3-triazol-1-yl)hexanamido)butanamido)hexanamide (DL-042)

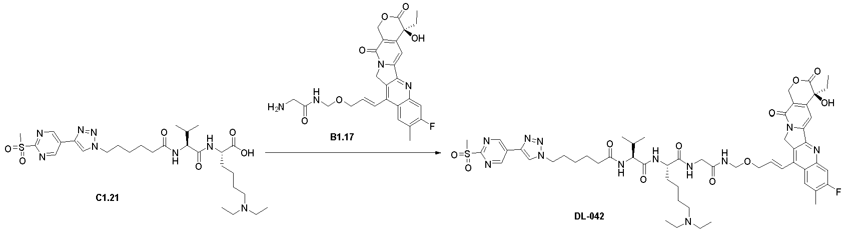

Compound B1.17 (30 mg) and compound C1.21 (37 mg) were dissolved in DMF (1 mL). Then, HBTU (21 mg) and *N*,*N*-diisopropylethylamine (16 mg) were added. After the addition, the reaction solution was stirred at room temperature for 1 h. After the reaction was completed as detected by LCMS, the reaction solution was purified by preparative chromatography (0.01% TFA in water, MeCN) to obtain the target compound (*S*)-6-(diethylamino)-*N*-(2-(((((*E*)-3-((*S*)-4-ethyl-8-fluoro-4-hydroxy-9-methyl-3,14-dioxo-3,4,12,14-tetrahydro-1*H-*pyrano[3',4':6,7]indolizino[1,2-*b*]quinolin-11-yl)allyl)oxy)methyl)amino)-2-oxoethyl)-2-((*S*)-3-methyl-2-(6-(4-(2-(methylsulfonyl)pyrimidin-5-yl)-1*H*-1,2,3-triazol-1-yl)hexanamido)butanamido)hexanamide (7 mg).

LCMS (ESI) [M+H]⁺ = 1127.5.

### Example 2.43: (S)-6-(Dipropylamino)-N-(2-(((((E)-3-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)allyl)oxy)methyl)amino)-2-oxoethyl)-2-((S)-3-methyl-2-(6-(4-(2-(methylsulfonyl)pyrimidin-5-yl)-1H-1,2,3-triazol-1-yl)hexanamido)butanamido)hexanamide (DL-043)

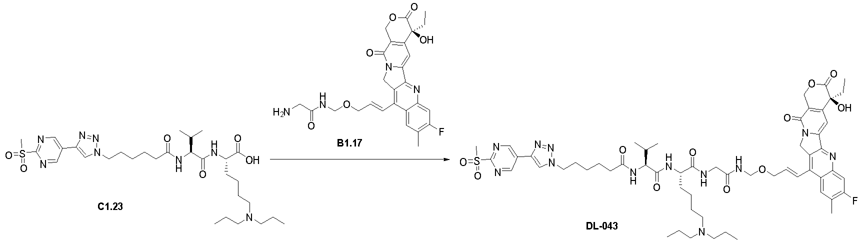

Compound B1.17 (17.5 mg, 0.033 mmol) and compound C1.23 (22 mg, 0.034 mmol) were dissolved in N,N-dimethylformamide (2 mL), and then HOBT (7 mg, 0.051 mmol), EDCI (10 mg, 0.051 mmol), and DIPEA (9 mg, 0.068 mmol) were added sequentially. After the addition, the reaction solution was stirred at room temperature for 1 h and then directly purified by preparative high-performance liquid chromatography (0.01% aqueous trifluoroacetic acid solution, acetonitrile), and the resulting solution was lyophilized to obtain the target compound (*S*)-6-(dipropylamino)-*N*-(2-(((((*E*)-3-((*S*)-4-ethyl-8-fluoro-4-hydroxy-9-methyl-3,14-dioxo-3,4,12,14-tetrahydro-1*H*-pyrano[3',4':6,7]indolizino[1,2-*b*]quinolin-11-yl)allyl)oxy)methyl)amino)-2-oxoethyl)-2-((*S*)-3-methyl-2-(6-(4-(2-(methylsulfonyl)pyrimidin-5-yl)-1*H*-1,2,3-triazol-1-yl)hexanamido)butanamido)hexanamide (7 mg, yield: 18%) as a pale yellow solid product.

LCMS (ESI) [M+H]⁺ = 1155.5.

### Example 2.44: N-((11S,14S,E)-11-(4-(Dimethylamino)butyl)-1-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)-15-methyl-7,10,13-trioxo-4-oxa-6,9,12-triazahexadec-1-en-14-yl)-6-(2-(methylsulfonyl)pyrimidin-5-yl)hex-5-ynamide (DL-044)

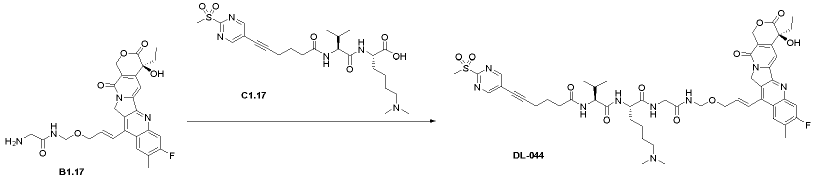

Compound B1.17 (60 mg) and compound C1.17 (80 mg) were dissolved in DMF (2 mL). Then, HBTU (45 mg) and *N,N*-diisopropylethylamine (35 mg) were added, and after the addition, the reaction solution was stirred at room temperature for 1 h. After the reaction was completed as detected by LCMS, the reaction solution was purified by preparative chromatography (0.01% TFA in water, MeCN) to obtain the target compound *N-*((11*S*,14*S*,*E*)-11-(4-(dimethylamino)butyl)-1-((*S*)-4-ethyl-8-fluoro-4-hydroxy-9-methyl-3,14-dioxo-3,4,12,14-tetrahydro-1*H*-pyrano[3',4':6,7]indofizino[1,2-*b*]quinolin-11-yl)-15-methyl-7,10,13-trioxo-4-oxa-6,9,12-triazahexadec-1-en-14-yl)-6-(2-(methylsulfonyl)pyrimidin-5-yl)hex-5-ynamide (21 mg).

LCMS (ESI) [M+H]⁺ = 1028.5.

### Example 2.45: N-((11S,14S,E)-11-(4-(Diethylamino)butyl)-1-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)-15-methyl-7,10,13-trioxo-4-oxa-6,9,12-triazahexadec-1-en-14-yl)-6-(2-(methylsulfonyl)pyrimidin-5-yl)hex-5-ynamide (DL-045)

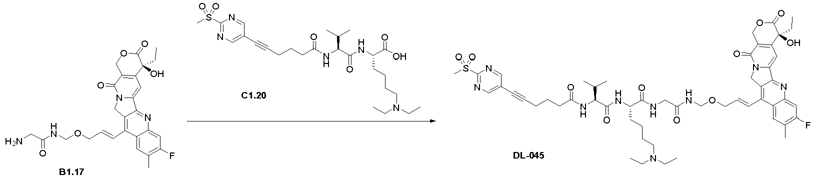

Compound B1.17 (60 mg) and compound C1.20 (80 mg) were dissolved in DMF (2 mL). Then, HBTU (45 mg) and *N,N*-diisopropylethylamine (35 mg) were added, and after the addition, the reaction solution was stirred at room temperature for 1 h. After the reaction was completed as detected by LCMS, the reaction solution was purified by preparative chromatography (0.01% TFA in water, MeCN) to obtain the target compound *N-*((11*S*,14*S*,*E*)-11-(4-(diethylamino)butyl)-1-((*S*)-4-ethyl-8-fluoro-4-hydroxy-9-methyl-3,14-dioxo-3,4,12,14-tetrahydro-1*H* pyrano[3',4':6,7]indofizino[1,2-*b*]quinolin-11-yl)-15-methyl-7,10,13-trioxo-4-oxa-6,9,12-triazahexadec-1-en-14-yl)-6-(2-(methylsulfonyl)pyrimidin-5-yl)hex-5-ynamide (29 mg).

LCMS (ESI) [M+H]⁺ = 1056.5.

### Example 2.46: N-((11S,14S,E)-11-(4-(Dipropylamino)butyl)-1-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)-15-methyl-7,10,13-trioxo-4-oxa-6,9,12-triazahexadec-1-en-14-yl)-6-(2-(methylsulfonyl)pyrimidin-5-yl)hex-5-ynamide (DL-046)

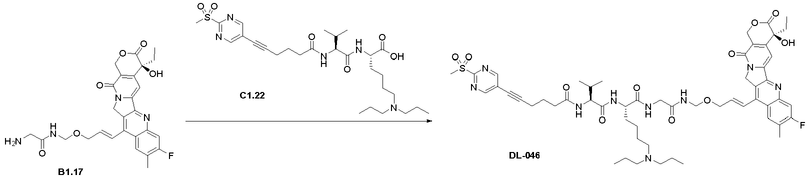

Compound B1.17 (60 mg) and compound C1.22 (80 mg) were dissolved in DMF (2 mL). Then, HBTU (45 mg) and *N,N*-diisopropylethylamine (35 mg) were added, and after the addition, the reaction solution was stirred at room temperature for 1 h. After the reaction was completed as detected by LCMS, the reaction solution was purified by preparative chromatography (0.01% TFA in water, MeCN) to obtain the target compound *N-*((11*S*,14*S*,*E*)-11-(4-(dipropylamino)butyl)-1-((S)-4-ethyl-8-fluoro-4-hydroxy-9-methyl-3,14-dioxo-3,4,12,14-tetrahydro-1*H*-pyrano[3',4':6,7]indofizino[1,2-*b*]quinolin-11-yl)-15-methyl-7,10,13-trioxo-4-oxa-6,9,12-triazahexadec-1-en-14-yl)-6-(2-(methylsulfonyl)pyrimidin-5-yl)hex-5-ynamide (30 mg).
LCMS (ESI) [M+H]⁺ = 1084.2;
¹H NMR (400 MHz, DMSO) δ 9.09 (s, 2H), 8.73 (*d, J =* 6.3 Hz, 1H), 8.30 - 8.24 (m, 2H), 8.05 (m, 1H), 7.94 - 7.88 (m, 2H), 7.38 - 7.33 (m, 2H), 6.67 - 6.60 (m, 1H), 6.53 (s, 1H), 5.44 (s, 2H), 5.37 (s, 2H), 4.73 (t, *J* = 6.2 Hz, 2H), 4.34 (*d*, *J* = 4.2 Hz, 2H), 4.26 - 4.20 (m, 1H), 4.17 - 4.13 (m, 1H), 3.86 - 3.69 (m, 4H), 3.40 (s, 3H), 2.41 - 2.30 (m, 8H), 2.02 - 1.94 (m, 2H), 1.89 - 1.85 (m, 2H), 1.82 - 1.77 (m, 2H), 1.74 - 1.63 (m, 2H), 1.60 - 1.50 (m, 2H), 1.47 - 1.39 (m, 8H), 0.90 - 0.86 (m, 6H), 0.85 - 0.80 (m, 9H).

### Example 2.47: N-((11S,14S,E)-11-(4-(Dipropylamino)butyl)-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)-15-methyl-7,10,13-trioxo-4-oxa-6,9,12-triazahexadec-1-en-14-yl)-6-(2-(methylsulfonyl)pyrimidin-5-yl)hex-5-ynamide (DL-047)

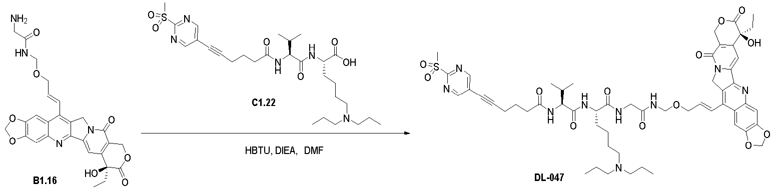

Compound B1.16 (45 mg, 0.08 mmol) and compound C1.22 (49 mg, 0.08 mmol) were dissolved in DMF (1 mL). Then, HBTU (32 mg, 0.08 mmol) and *N,N*-diisopropylethylamine (27 mg, 0.2 mmol) were added, and after the addition, the reaction solution was stirred at room temperature for 1 h. After the reaction was completed as detected by LCMS, the reaction solution was purified by preparative chromatography (0.01% trifluoroacetic acid in water, acetonitrile) to obtain the target compound *N*-((11*S*,14*S*,*E*)-11-(4-(dipropylamino)butyl)-1-((*S*)-7-ethyl-7-hydroxy-8,11-dioxo-7,8,11,13-tetrahydro-10*H*-[1,3]dioxolo[4,5-g]pyrano[3',4':6,7]indolizino[1,2-b]quinolin-14-yl)-15-methyl-7,10,13-trioxo-4-oxa-6,9,12-triazahexadec-1-en-14-yl)-6-(2-(methylsulfonyl)pyrimidin-5-yl)hex-5-ynamide (DL-047, 10 mg) as a yellow solid.
LCMS (ESI) [M+H]⁺ = 1096.6;
¹H NMR (400 MHz, DMSO) δ 9.10 (s, 2H), 9.05 (s, 1H, TFA), 8.75 (t, *J =* 5.3 Hz, 1H), 8.26 (t, *J* = 6.4 Hz, 1H), 8.11 (d, *J =* 7.6 Hz, 1H), 7.93 (d, *J* = 8.4 Hz, 1H), 7.63 (s, 1H), 7.53 (s, 1H), 7.26 - 2.24 (m, 2H), 6.65 - 6.43 (m, 2H), 6.30 (s, 2H), 5.42 (s, 2H), 5.30 (s, 2H), 4.74 - 4.69 (m, 1H), 4.31 - 4.29 (m, 2H), 4.26 - 424 (m, 1H), 4.19 - 4.12 (m, 1H), 3.79 - 2.76 (m, 1H), 3.40 (s, 3H), 3.02 - 2.95 (m, 8H), 2.55 (m, 2H), 2.39 - 2.32 (m, 2H), 2.01 - 1.93 (m, 2H), 1.92 - 1.77 (m, 5H), 1.62 - 1.58 (m, 6H), 1.36 - 1.30 (m, 2H), 0.91 - 0.83 (m, 15H).

### Example 2.48: N-((11S,14S)-11-(4-(Dibutylamino)butyl)-1-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)-15-methyl-7,10,13-trioxo-4-oxa-6,9,12-triazahexadecan-14-yl)-6-(2-(methylsulfonyl)pyrimidin-5-yl)hex-5-ynamide (DL-048)

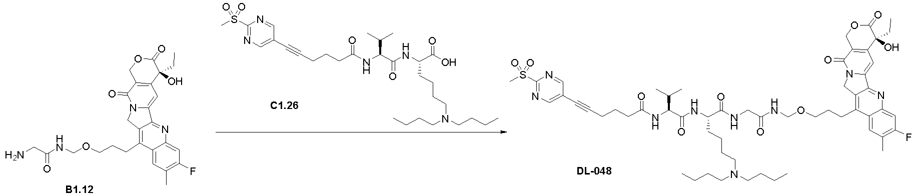

Compound C1.26 (50 mg, 0.08 mmol) and compound B1.12 (43 mg, 0.08 mmol) were dissolved in *N,N-*dimethylformamide (1.5 mL), and then DIPEA (26 mg, 0.21 mmol) and HBTU (31 mg, 0.08 mmol) were added sequentially. The mixture was stirred at room temperature for 1 h. After the reaction was completed as detected by LCMS, the reaction solution was separated and purified directly by preparative high-performance chromatography (0.01% trifluoroacetic acid in water, acetonitrile) to obtain the target compound (13.57 mg) as a yellow solid.
LCMS (ESI) [M+H]⁺ = 1114.6;
¹H NMR (400 MHz, DMSO) δ 9.09 (s, 2H), 9.05 (s, 1H, TFA), 8.64 (t, *J* = 7.2 Hz, 1H), 8.23 - 8.16 (m, 2H), 8.07 (d, *J =* 7.3 Hz, 1H), 7.93 - 7.85 (m, 2H), 7.32 (s, 1H), 6.52 (s, 1H), 5.44 (s, 2H), 5.29 (s, 2H), 4.61 - 4.59 (m, 2H), 4.24 - 4.22 (m, 1H), 4.19 - 4.10 (m, 1H), 3.73 - 3.70 (m, 2H), 3.50 (s, 3H), 3.21 - 3.18 (m, 6H), 3.00 - 2.98 (m, 8H), 2.54 (s, 3H), 1.97 - 1.78 (m, 7H), 1.56 - 1.52 (m, 8H), 1.36 - 1.25 (m, 6H), 0.93 - 0.78 (m, 15H).

### Example 2.49: (S)-6-(Dimethylamino)-N-(2-(((((E)-3-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)allyl)oxy)methyl)amino)-2-oxoethyl)-2-((S)-3-methyl-2-(6-(4-(2-(methylsulfonyl)pyrimidin-5-yl)-1H-1,2,3-triazol-1-yl)hexanamido)butanamido)hexanamide (DL-049)

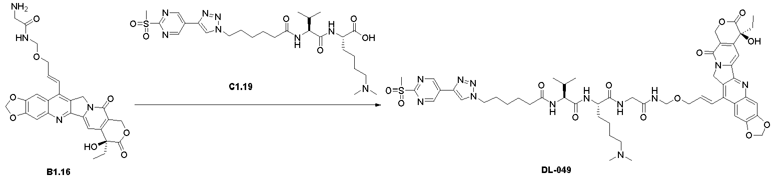

Compound B1.16 (45 mg, 0.08 mmol) and compound C1.19 (50 mg, 0.08 mmol) were dissolved in DMF (1 mL). Then, *N,N*-diisopropylethylamine (27 mg, 0.2 mmol) and HBTU (32 mg, 0.08 mmol) were added sequentially, and after the addition, the reaction solution was stirred at room temperature for 1 h. After the reaction was completed as detected by LCMS, the reaction solution was directly purified by preparative chromatography (0.01% aqueous formic acid solution, acetonitrile) to obtain the target compound (*S*)-6-(dimethylamino)-*N*-(2-(((((*E*)-3-((*S*)-7-ethyl-7-hydroxy-8,11-dioxo-7,8,11,13-tetrahydro-10*H*-[1,3]dioxolo[4,5-*g*]pyrano[3',4':6,7]indolizino[1,2-*b*]quinolin-14-yl)allyl)oxy)methyl)amino)-2-oxoethyl)-2-((*S*)-3-methyl-2-(6-(4-(2-(methylsulfonyl)pyrimidin-5-yl)-1*H*-1,2,3-triazol-1-yl)hexanamido)butanamido)hexanamide (DL-049, 3 mg) as a white solid.
LCMS (ESI) [M+H]⁺= 1111.1;
¹H NMR (400 MHz, DMSO) δ 9.45 (s, 2H), 8.92 (s, 1H), 8.69 (t, *J =* 6.6 Hz, 1H), 8.25 (t, *J =* 5.9 Hz, 1H), 8.19 (s, 1H, FA), 7.99 (d, *J =* 7.0 Hz, 1H), 7.83 (d, *J* = 8.5 Hz, 1H), 7.64 (s, 1H), 7.52 (s, 1H), 7.27 (m, 2H), 6.59 - 6.48 (m, 2H), 6.29 (s, 2H), 5.42 (s, 2H), 5.31 (s, 2H), 4.74 - 4.67 (m, 2H), 4.44 (m, 2H), 4.30 (d, *J=* 4.8 Hz, 2H), 4.21 (m, 1H), 4.12 (m, 1H), 3.83 - 3.70 (m, 4H), 3.43 (s, 3H), 2.25 (m, 2H), 2.15 (s, 6H), 2.04 - 1.97 (m, 2H), 1.90 - 1.85 (m, 4H), 1.55 - 1.51 (m, 3H), 1.39 - 1.34 (m, 6H), 0.90 - 0.83 (m, 3H), 0.80 (t, *J =* 7.2 Hz, 6H).

### Example 2.50: 1-Ethyl-N-(2-(((3-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)propoxy)methyl)amino)-2-oxoethyl)-4-((S)-3-methyl-2-(6-(2-(methylsulfonyl)pyrimidin-5-yl)hex-5-ynamido)butanamido)piperidine-4-carboxamide (DL-050)

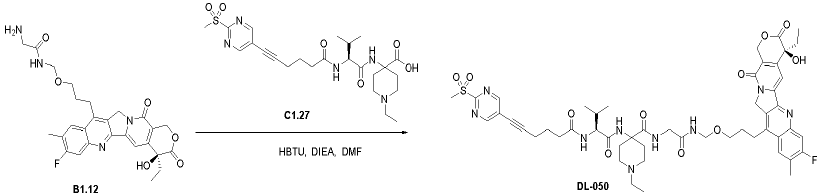

Compound B1.12 (50 mg, 0.10 mmol) and compound C1.27 (50 mg, 0.10 mmol) were dissolved in DMF (1 mL). Then, *N*,*N*-diisopropylethylamine (30 mg, 0.24 mmol) and HBTU (36 mg, 0.10 mmol) were added sequentially, and after the addition, the reaction solution was stirred at room temperature for 1 h. After the reaction was completed as detected by LCMS, the reaction solution was purified by preparative chromatography (0.01% FA in water, MeCN) to obtain the target compound 1-ethyl-*N*-(2-(((3-((*S*)-4-ethyl-8-fluoro-4-hydroxy-9-methyl-3,14-dioxo-3,4,12,14-tetrahydro-1*H*-pyrano[3',4':6,7]indolizino[1,2-*b*]quinolin-11-yl)propoxy)methyl)amino)-2-oxoethyl)-4-((*S*)-3-methyl-2-(6-(2-(methylsulfonyl)pyrimidin-5-yl)hex-5-ynamido)butanamido)piperidine-4-carboxamide (DL-050, 10 mg) as a white solid.
LCMS (ESI) [M+H]⁺ = 1028.6;
¹H NMR (400 MHz, DMSO) *δ* 9.08 (s, 2H), 8.42 (s, 1H, FA), 8.18 - 8.15 (m, 4H), 7.98 (t, *J* = 5.6 Hz, 1H), 7.86 (d, *J =* 10.9 Hz, 1H), 7.30 (s, 1H), 6.51 (s, 1H), 5.43 (s, 2H), 5.28 (s, 2H), 4.72 - 4.63 (m, 1H), 4.53 - 4.45 (m, 1H), 4.07 - 4.06 (m, 1H), 3.79 - 3.69 (m, 1H), 3.58 - 3.55 (m, 2H), 3.55 - 3.48 (m, 5H), 3.21 - 3.16 (m, 3H), 2.55 (s, 3H), 2.66 - 2.64 (m, 2H), 2.40 - 2.26 (m, 5H), 1.99 - 1.71 (m, 12H), 0.96 (t, *J =* 7.1 Hz, 3H), 0.93 - 0.85 (m, 9H).

### Example 2.51: (R)-6-(Dimethylamino)-N-(4-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)phenyl)-2-((S)-3-methyl-2-(6-(4-(2-(methylsulfonyl)pyrimidin-5-yl)-1H-1,2,3-triazol-1-yl)hexanamido)butanamido)hexanamide (DL-051) and (S)-6-(dimethylamino)-N-(4-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)phenyl)-2-((S)-3-methyl-2-(6-(4-(2-(methylsulfonyl)pyrimidin-5-yl)-1H-1,2,3-triazol-1-yl)hexanamido)butanamido)hexanamide (DL-052)

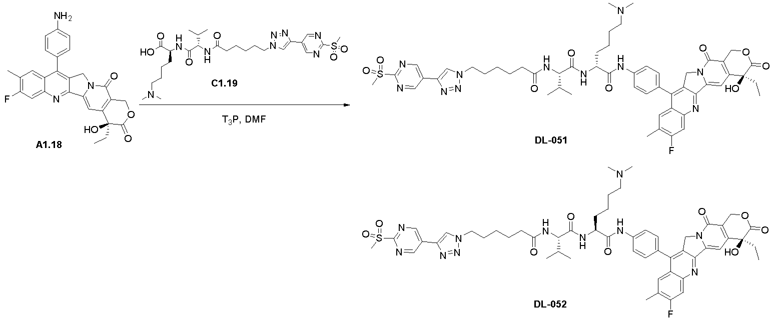

Compounds A1.18 (40 mg, 0.08 mmol) and C1.19 (71 mg, 0.12 mmol) were dissolved in *N,N-*dimethylformamide (3 mL). Then, T₃P (254 mg, 0.40 mmol, 50% w/w mixture with ethyl acetate) was added, and the reaction solution was stirred at room temperature for 1 h. After the reaction was completed as detected by LCMS, the reaction solution was directly purified by preparative chromatography (0.01% aqueous trifluoroacetic acid solution, acetonitrile) to obtain the target compounds (*R*)-6-(dimethylamino)-*N*-(4-((*S*)-4-ethyl-8-fluoro-4-hydroxy-9-methyl-3,14-dioxo-3,4,12,14-tetrahydro-1*H*-pyrano[3',4':6,7]indolizino[1,2-*b*]quinolin-11-yl)phenyl)-2-((*S*)-3-methyl-2-(6-(4-(2-(methylsulfonyl)pyrimidin-5-yl)-1*H*-1,2,3-triazol-1-yl)hexanamido)butanamido)hexanamide (DL-051, 8.1 mg) and (*S*)-6-(dimethylamino)-*N*-(4-((*S*)-4-ethyl-8-fluoro-4-hydroxy-9-methyl-3,14-dioxo-3,4,12,14-tetrahydro-1*H*-pyrano[3',4':6,7]indolizino[1,2-*b*]quinolin-11-yl)phenyl)-2-((*S*)-3-methyl-2-(6-(4-(2-(methylsulfonyl)pyrimidin-5-yl)-1*H*-1,2,3-triazol-1-yl)hexanamido)butanamido)hexanamide (DL-052, 8.8 mg), both of which were white solids.

DL-051:
LCMS (ESI) [M+H]⁺ = 1048.2, t_{R} = 1.258 min;
¹H NMR (400 MHz, DMSO) δ 10.12 (s, 1H), 9.40 (s, 2H), 8.84 (s, 1H), 8.55 (d, *J* = 7.4 Hz, 1H), 8.26 (s, 1H), 8.05 (d, *J =* 7.8 Hz, 1H), 7.99 - 7.89 (m, 3H), 7.73 (d, *J =* 8.4 Hz, 1H), 7.57 (d, *J* = 8.7 Hz, 2H), 7.36 (s, 1H), 5.41 (s, 2H), 5.10 (s, 2H), 4.40 (m, 3H), 4.22 - 4.10 (m, 1H), 3.44 (s, 3H), 2.40 (s, 3H), 2.30 - 2.25 (m, 2H), 2.24 - 2.20 (m, 2H), 2.19 - 2.15 (m, 6H), 2.03 - 1.93 (m, 2H), 1.92 - 1.78 (m, 6H), 1.72 - 1.66 (m, 1H), 1.61 - 1.54 (m, 2H), 1.48 - 1.42 (m, 2H), 1.30 - 1.26 (m, 2H), 0.91 - 0.86 (m, 9H).

DL-052:
LCMS (ESI) [M+H]⁺ = 1048.2, t_{R} = 1.283 min;
¹H NMR (400 MHz, DMSO) δ 10.34 (s, 1H), 9.45 (s, 2H), 8.94 (s, 1H), 8.20 (s, 1H), 7.99 - 7.85 (m, 4H), 7.75 (d, *J =* 7.4 Hz, 1H), 7.59 (m, 2H), 7.36 (s, 1H), 6.55 (s, 1H), 5.42 (s, 2H), 5.10 (s, 2H), 4.52 - 4.42 (m, 3H), 4.25 - 4.17 (m, 1H), 3.44 (s, 3H), 2.41 (s, 3H), 2.30 - 2.13 (m, 8H), 2.05 - 1.97 (m, 2H), 1.95 - 1.81 (m, 7H), 1.60 - 1.55 (m, 2H), 1.50 - 1.44 (m, 2H), 1.33 - 1.28 (m, 4H), 0.95 - 0.83 (m, 9H).

### III. Antibody Preparation and Identification

### Example 3. Preparation of Anti-NaPi2b Antibodies

### 3.1 Preparation of antigens

1) Antigen preparation: The amino acid sequence of human NaPi2b (h.NaPi2b) was referenced to O95436 in the UniProt protein database, with the second extracellular loop ranging from 234 aa to 362 aa; the amino acid sequence of human Napi2a was referenced to Q06495 in the UniProt protein database; and the amino acid sequence of human Napi2c was referenced to Q8N130 in the UniProt protein database. The amino acid sequence of cynomolgus monkey NaPi2b (c.NaPi2b) was referenced to A0A2K5UHY1 in the UniProt protein database, with the second extracellular loop ranging from 234 aa to 362 aa. The amino acid sequence of rat NaPi2b (r.NaPi2b) was referenced to Q9JJ09 in the UniProt protein database, with the second extracellular loop ranging from 235 aa to 363 aa. The above amino acid sequences were subjected to codon optimization, the full-length sequences were synthesized onto the lentiviral vector pLVX or the pCDNA3.4 vector, and the second extracellular loop sequences were synthesized onto pTT5-hFc (hFc: SEQ ID NO: 85) and pTT5-mFc (mFc: SEQ ID NO: 86).

HEK293-EBNA cells were transiently transfected with the pTT5-hFc or pTT5-mFc plasmid containing the second extracellular loop using PEImax (Polysciences, 24765-1). After 7 days of expression, human (h.) NaPi2b-mFc/NaPi2b-hFc, cynomolgus monkey (c.) NaPi2b-mFc/NaPi2b-hFc, and rat (r.) NaPi2b-mFc/NaPi2b-hFc antigens were obtained by purification using ProA packing (GE, Mabselect XL).

2) Cell line construction: Viruses were prepared using a lentiviral packaging system, and the obtained viruses were used to infect CHO cells, HT29 cells, or HEK293T cells. Puromycin screening was then performed to obtain stable cell lines overexpressing human Napi2a/b/c, namely CHO-h.NaPi2b cells, HT29-h.NaPi2b cells, HEK293T-h.Napi2a cells, and HEK293T-h.Napi2c cells.

### 3.2 Mouse immunization and immune titer assay

Four female mice aged about 6 weeks were selected from each of the CD1 (Vital River), KM (Vital River), and Balb/c (GemPharmatech) strains for group entry. One initial immunization and two booster immunizations were performed using the CHO-h.NaPi2b cells and pCDNA3.4-h.NaPi2b plasmid, and after the immunizations were completed, blood was collected from the mice via the orbital vein.

After the two booster immunizations, the serum titer was measured by ELISA. The assay method was as follows: The night before the experiment, the human NaPi2b-hFc antigen was coated with a CBS coating solution at a coating concentration of 1 µg/mL and a volume of 100 µL, and incubated overnight at 4 °C. The next day, the coating solution was removed, and each well was washed once with 300 µL of PBS. Then, 100 µL of 2% BSA (Biotopped, A6020) (in PBS) was added to each well, and the mixture was blocked at 37 °C for 2 h. Serum was diluted with 2% BSA (in PBS), starting at 500-fold, with a 3-fold dilution across 11 concentration points. After dilution, 100 µL was added to the plate, and the plate was incubated at 37 °C for 2 h. The supernatant was then removed, and each well was washed three times with 300 µL of PBST. Subsequently, 100 µL of anti-mouse HRP secondary antibody (Jackson, 115-035-003) (in 2% BSA) was added to each well, and the plate was incubated at 37 °C for 1 h. The secondary antibody was then removed, and each well was washed five times with 300 µL of PBST. Then, 100 µL of TMB substrate (Huzhou InnoReagents, TMB-S-004) was added to each well for color development for 5-20 min. 50 µL of 2 N HCl was added to each well to stop the reaction, and then the OD_{450 nm} readings were taken using a microplate reader (manufacturer: MD, model: SpectraMax). The data were imported into GraphPad software for fitting, and based on the assay results, one mouse each from CD1, KM, and Balb/c was selected for hybridoma fusion.

### 3.3 Hybridoma fusion screening

Hybridoma fusion: The spleens and lymph nodes of the mice were collected and prepared into cell suspensions. The suspensions were mixed with SP2/0 mouse myeloma cells in a 1:1 ratio. The cells were resuspended in a cell electrofusion buffer and subjected to electrofusion using the BTX-ECM2001 electrofusion system. The fusion cells were resuspended in a complete fusion culture medium (RPMI160 + 15% FBS + 1× HAT), transferred into 96-well cell culture plates at 2-2.5 × 10⁴ cells/well, and incubated at 37 °C in 5% CO₂ for 7 days before screening. Two runs of fusion were performed with 85 plates in the first run and 40 plates in the second run.

Parent clone screening: A preliminary ELISA screening experiment was performed with human NaPi2b-hFc protein using the method described above, and positive hybridoma clones were selected after the preliminary screening experiment. An ELISA rescreening experiment was performed on microplates coated with human, cynomolgus monkey or rat NaPi2b-hFc, and a cell-based ELISA rescreening experiment was performed using NaPi2b-positive OVCAR3 tumor cells. Finally, positive clones were selected for subcloning. By the above method, 22 parent clones were selected for subcloning from the first run of fusion, and 16 parent clones were selected for subcloning from the second run of fusion.

Subclone screening: An ELISA screening experiment on microplates coated with human, cynomolgus monkey or rat NaPi2b-hFc and a cell-based ELISA screening experiment were sequentially performed using the method described above. One to three positive clones from each plate, totaling 43 positive clones, were selected and subjected to expansion culture in a serum-free culture medium.

### 3.4 Murine antibody evaluation and sequence acquisition

Hybridoma cells were cultured in a serum-free culture medium, and the collected monoclonal supernatants were purified using ProA packing to obtain murine antibodies. ELISA and cell-based ELISA evaluation experiments on microplates coated with human NaPi2b-hFc, cynomolgus monkey NaPi2b-hFc, rat NaPi2b-hFc and OVCAR3 were sequentially performed using the method described above. The murine antibodies were diluted, starting at 10 µg/mL, with a 3-fold dilution across 11 concentration points. A total of 39 murine antibodies were evaluated. The results of the ELISA and cell affinity assay performed on some high-affinity monoclonal murine antibodies are shown in Table 1.

**Table 1. Affinity assay on some murine antibodies**

| Clone No. | h.NaPi2b-hFc EC50 (ng/mL) | c.NaPi2b-hFc EC50 (ng/mL) | r.NaPi2b-hFc EC50 (ng/mL) | OVCAR3 cell-based ELISA EC50 (ng/mL) |
|---|---|---|---|---|
| 16G5B3 | 11.69 | 5.14 | 8.63 | 13.46 |
| 60A12B3 | 5.89 | 3.12 | 5.44 | 14.23 |
| 22H9C4 | 4.28 | 4.09 | 7.72 | 10.48 |
| 66C12D12 | 3.01 | 1.70 | 2.71 | 6.80 |
| 34F2A10 | 7.11 | 6.35 | 7.02 | 15.80 |

Sequence acquisition was performed on the selected high-affinity monoclonals as follows: About 1 × 10⁵ candidate hybridoma cells were collected. RNA was extracted using Trizol, and then cDNA was obtained by reverse transcription through PolyA using a PrimeScript RT reagent kit. Forward primers were designed upstream of the heavy chain and the light chain, and reverse primers were designed in the heavy chain CH1 region and the light chain CL region. The products were amplified by PCR, and the fragments were recovered using an agarose gel extraction kit and sent to Beijing Tsingke Biotechnology Co., Ltd. for sequencing. Among them, 5 clone numbers were 16G5B3, 60A12B3, 22H9C4, 66C12D12, and 34F2A10. Their corresponding murine antibodies 16G5B3, 60A12B3, 22H9C4, 66C12D12, and 34F2A10 were each sequenced. The specific sequences of the variable regions and CDRs of the murine antibodies are shown in the sequence information table.

### 3.5 Antibody humanization

The method of CDR grafting was employed. Firstly, a human germline sequence with the highest homology to the original murine sequence was found by a conventional BLAST method to serve as a template. Subsequently, CDRs of the murine antibody were grafted to the human template to construct a chimera. Then, FR amino acids capable of retaining the original conformation of the murine antibody were analyzed according to the structure, and the corresponding amino acids in the chimera were back-mutated to murine amino acids to keep the original affinity. Finally, the constructed humanized antibody was subjected to calculation and immunogenicity analysis to find a high-immunogenicity fragment, which was then replaced by a low-immunogenicity fragment. The murine antibody 16G5B3 was humanized to obtain humanized variable regions 16G5B3-hz1 and 16G5B3-hz2. The murine antibodies 60A12B3, 22H9C4, 66C12D12, and 34F2A10 were humanized to obtain humanized variable regions 60A12B3-hz1, 22H9C4-hz1, 66C12D12-hz1, and 34F2A10-hz1, respectively. The specific sequences of the variable regions and CDRs of each humanized antibody are shown in the sequence information table, where the CDRs are provided according to IMGT and Kabat definitions.

### 3.6 Anti-NaPi2b humanized antibody expression

The heavy chain variable region of each humanized antibody was linked to a heavy chain IgG1 constant region (SEQ NO: 70), and the light chain variable region of each humanized antibody was linked to a Kappa constant region (SEQ NO: 80). The heavy and light chain variable regions of antibody C1 (abbreviated as C1, with heavy and light chain variable region sequences sourced from IMGT, ID: 478) were linked to the IgG1 constant region (SEQ NO: 70) and the Kappa constant region (SEQ NO: 80), respectively. The amino acid sequences obtained from these linkages were submitted to General Biol for gene synthesis, subjected to codon optimization, and then constructed onto a PTT5 vector. Once the plasmids were synthesized, HEK293E cells were transfected with them using PEImax. After expression for about 7 days, the supernatants were collected by centrifugation. The supernatants were purified using ProA packing. The purified antibodies were all exchanged into PBS buffer by ultrafiltration, determined for concentration, and stored at -20 °C.

The antibodies obtained by expression after linking the humanized variable regions 16G5B3-hz1, 16G5B3-hz2, 60A12B3-hz1, 22H9C4-hz1, 66C12D12-hz1, and 34F2A10-hz1 were named 16G5B3-hz1 antibody, 16G5B3-hz2 antibody, 60A12B3-hz1 antibody, 22H9C4-hz1 antibody, 66C12D12-hz1 antibody, and 34F2A10-hz1 antibody, respectively. The sequences of the constant regions and heavy and light chains of each humanized antibody are shown in the sequence information table.

### Example 4. Identification of Anti-NaPi2b Antibodies

### 4.1 Evaluation of affinity of humanized antibodies by ELISA

1) ELISA method: The antigen proteins h.NaPi2b-mFc, r.NaPi2b-mFc, and c.NaPi2b-mFc were coated with CBS at 1 µg/mL and then blocked with 2% BSA (in PBS) at 37 °C for 2 h. Serially diluted antibodies (starting at 10 µg/mL with a 3-fold dilution across 11 concentration points) were each added, and the mixture was incubated at 37 °C for 2 h. Then, an HRP-labeled anti-human secondary antibody (Jackson, 109-035-088) was added, and the mixture was incubated at 37 °C for 1 h. A TMB substrate was added for color development, and then the reaction was stopped by 2 M HCl before absorbance readings at 450 nm were taken using a microplate reader.
2) Cell-based ELISA method: OVCAR3 cells were digested with trypsin, collected by centrifugation, resuspended in a culture medium, and added to a 96-well plate at 5 ×10⁴ cells/well for incubation overnight. The cells were then fixed with 4% paraformaldehyde and blocked with 2% BSA (in PBS) at 37 °C for 2 h. Serially diluted antibodies (starting at 10 µg/mL, with a 3-fold dilution across 11 concentration points) were each added, and the plate was incubated at 37 °C for 2 h. Then, an HRP-labeled anti-human secondary antibody (Jackson, Cat. No. 109-035-088) was added, and the plate was incubated at 37 °C for 1 h. A TMB substrate was added for color development, and then the reaction was stopped by 2 M HCl before absorbance readings at 450 nm were taken using a microplate reader.

The experimental results are shown in Table 2, indicating that the 16G5B3-hz2 antibody, the 22H9C4-hz1 antibody, and the 66C12D12-hz1 antibody all exhibited relatively high affinity for human, rat and cynomolgus monkey NaPi2b proteins, as well as for OVCAR3 cells endogenously expressing NaPi2b.

**Table 2. Evaluation of affinity of humanized antibodies by ELISA**

| Antibody | h. NaPi2b EC50 (ng/mL) | c.NaPi2b EC50 (ng/mL) | r.NaPi2b EC50 (ng/mL) | OVCAR3 Cell-based ELISA EC50 (ng/mL) |
|---|---|---|---|---|
| 60A12B3-hz1 | Weak binding | / | / | / |
| 16G5B3-hz1 | Weak binding | 2748 | 3685 | Weak binding |
| 16G5B3-hz2 | 53.99 | 57.63 | 27.07 | 49.89 |
| 22H9C4-hz1 | 44.76 | 40.96 | 22.74 | 30.86 |
| 34F2A10-hz1 | 90.24 | Weak binding | 1575 | 65.56 |
| 66C12D12-hz1 | 64.7 | 109.5 | 47.3 | 20.83 |
| C1 | 54.23 | 291.8 | Weak binding | 240.7 |

| | | | | |
|---|---|---|---|---|
| **: "*/*"* indicates not detected | | | | |

### 4.2 Evaluation of affinity of humanized antibodies by flow cytometry

OVCAR3 cells were digested with trypsin, collected by centrifugation, washed three times with pre-cooled PBS, resuspended in 1% BSA (in PBS), and added to a 96-well pointed bottom plate at 1 × 10⁵ cells/well. Serially diluted antibodies (starting at 20 µg/mL, with a 4-fold dilution across 8 concentration points) were each added, and the plate was incubated at 4 °C for 1 h. The cells were then washed three times with pre-cooled PBS. An anti-human APC fluorescent secondary antibody (BioLegend, Cat. No. 410712) was added at 1 µL/well, and the plate was incubated at 4 °C for 0.5 h. The cells were further washed three times with pre-cooled PBS, then resuspended, and assayed using a flow cytometer (Sony, LE-SA3800GA).

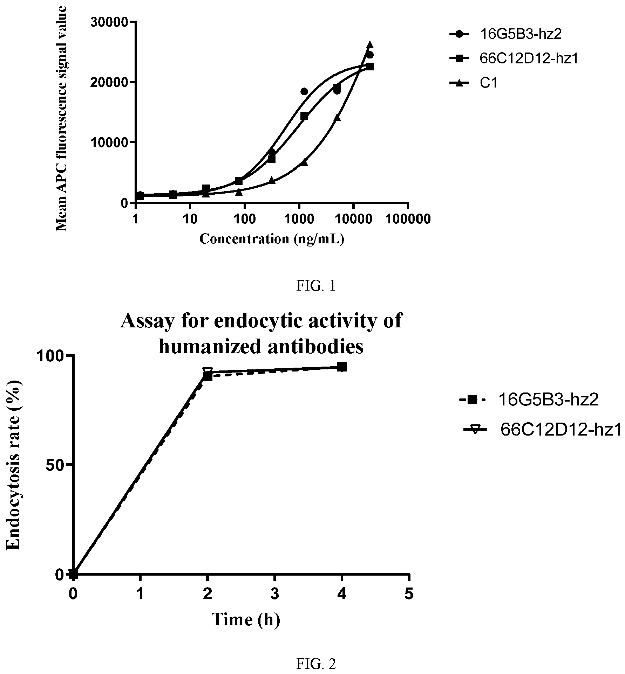

The experimental results are shown in FIG. 1, indicating that the 16G5B3-hz2 antibody and the 66C12D12-hz1 antibody both exhibited relatively high affinity for OVCAR3 cells, with EC50 values of 560.9 ng/mL and 957.9 ng/mL, respectively, and both were superior to the C1 antibody.

### 4.3 Evaluation of dynamic affinity of humanized antibodies

The dynamic affinity of the humanized antibodies was determined by ForteBio. The experimental procedures were as follows: 1. Sensor preparation: a ProA sensor was taken and pre-wetted with a PBST diluent (pH 7.4) for 10 min. 2. Sample dilution: the antibodies to be immobilized were labeled with biotin and then diluted to 5 µg/ mL, the antigens h.NaPi2b-mFc and c. NaPi2b-mFc were diluted starting from 500 nM, with a 2-fold dilution across 5 concentration points, and 0 concentration point was set. 3. The program was set, the sensor plate and the sample plate were placed, and then the program was started; the sensor was regenerated with a 20 mM glycine solution (pH 1.7). 4. The data were analyzed using an Octet analysis software. The experimental results are shown in Table 3, indicating that the 16G5B3-hz2 antibody and the 66C12D12-hz1 antibody both exhibited relatively high affinity for human and monkey NaPi2b.

**Table 3. Assay for dynamic affinity of humanized antibodies**

| Antibody | h.NaPi2b-mFc | | | c. NaPi2b-mFc | | |
|---|---|---|---|---|---|---|
| | KD (M) | ka (1/Ms) | kdis (1/s) | KD (M) | ka (1/Ms) | kdis (1/s) |
| 16G5B3-hz2 | 1.44E-11 | 2.46E+04 | 3.56E-07 | 4.11E-09 | 5.35E+04 | 2.20E-04 |
| 66C12D12-hz1 | 8.54E-10 | 2.66E+04 | 2.27E-05 | 1.59E-08 | 3.09E+04 | 4.91E-04 |
| C1 | 8.54E-10 | 2.66E+04 | 2.27E-05 | 6.55E-08 | 1.99E+04 | 1.30E-03 |

### 4.4 Evaluation of non-specificity of humanized antibodies

HEK293T-h.Napi2a and HEK293T-h.Napi2c cells were each digested with trypsin, collected by centrifugation, washed three times with pre-cooled PBS, resuspended in 1% BSA (in PBS), and added to a 96-well pointed bottom plate at 3 × 10⁵ cells/well. The test antibodies at 50 µg/mL were each added, and the plate was incubated at 4 °C for 1 h. The cells were then washed three times with pre-cooled PBS. An anti-human APC fluorescent secondary antibody was added at 1 µL/well, and the plate was incubated at 4 °C for 0.5 h. The cells were further washed three times with pre-cooled PBS, then resuspended, and assayed using a flow cytometer.

The experimental results are shown in Table 4, indicating that the 16G5B3-hz2 antibody, the 22H9C4-hz1 antibody, and the 66C12D12-hz1 antibody did not non-specifically recognize human Napi2a or Napi2c.

**Table 4. Assay for non-specific binding of humanized antibodies**

| Antibody | HEK293T-h.Napi2a | | | HEK293T-h.Napi2c | | |
|---|---|---|---|---|---|---|
| | Positive rate | APC-Mean | APC-Median | Positive rate | APC-Mean | APC-Median |
| Blank control | 0.10% | 955.9 | 765.7 | 0.39% | 812.3 | 715.1 |
| 16G5B3-hz2 | 0.19% | 1144.6 | 916.2 | 0.54% | 927.9 | 822.2 |
| 22H9C4-hz1 | 0.19% | 1634.1 | 1382.6 | 0.46% | 1045.9 | 951.3 |
| 66C12D12-hz1 | 0.30% | 1405.1 | 1186.4 | 1.17% | 1262.5 | 1148.2 |
| C1 | 0.59% | 1443.3 | 1117.5 | 0.14% | 1071.6 | 972.1 |

### 4.5 Evaluation of endocytic activity of humanized antibodies

HCC4006 cells (Nanjing Cobioer) were digested with trypsin, collected by centrifugation, incubated with the test antibody at a concentration of 10 µg/mL at 4 °C for 1 h, then washed 3 times with PBS, and resuspended in RPMI 1640 + 10% FBS. Then, the cells were divided into 3 portions, which were then incubated at 37 °C for 0 h, 2 h, and 4 h, respectively. The cells were washed three times with PBS, then 1 µL of anti-human APC fluorescent secondary antibody was added, and the mixture was incubated at 4 °C for 0.5 h. The cells were further washed three times with PBS, then resuspended, and assayed on a machine. The endocytosis rate was calculated according to the following formula: endocytosis rate (%) = [1 - (average fluorescence value of test sample at this time point - average fluorescence value of negative control sample at this time point)/(average fluorescence value of test sample at 0 h - average fluorescence value of negative control sample at 0 h)] × 100.

The experimental results are shown in Table 5 and FIG. 2, indicating that the 16G5B3-hz2 antibody and the 66C12D12-hz1 antibody exhibited strong endocytic activity, and their endocytosis rates were increased over time.

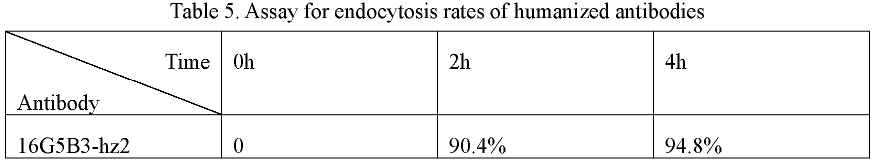

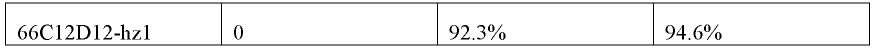

### 4.6 Evaluation of serum stability of humanized antibodies

1) Sample treatment: The humanized candidate antibodies 16G5B3-hz2 and 66C12D12-hz1 were diluted to 20 µg/mL with human serum and sterilized by filtration using a 0.22 µm filter. For each candidate, 3 time points were set: day 1, day 8, and day 15. Each tube contained 500 µL. The tubes were sealed with a sealing film and then placed in an incubator at 37 °C. Samples were collected on day 1, day 8, and day 15, and then stored in a freezer at -80 °C.
2) ELISA method: The antigen protein h.NaPi2b-mFc was coated with CBS at 1 µg/mL and then blocked with 2% BSA (in PBS) at 37 °C for 2 h. Serially diluted antibodies were each added, and the mixture was incubated at 37 °C for 2 h. Then, an HRP-labeled anti-human secondary antibody was added, and the mixture was incubated at 37 °C for 1 h. A TMB substrate was added for color development, and then the reaction was stopped by 2 M HCl before absorbance readings at 450 nm were taken using a microplate reader.

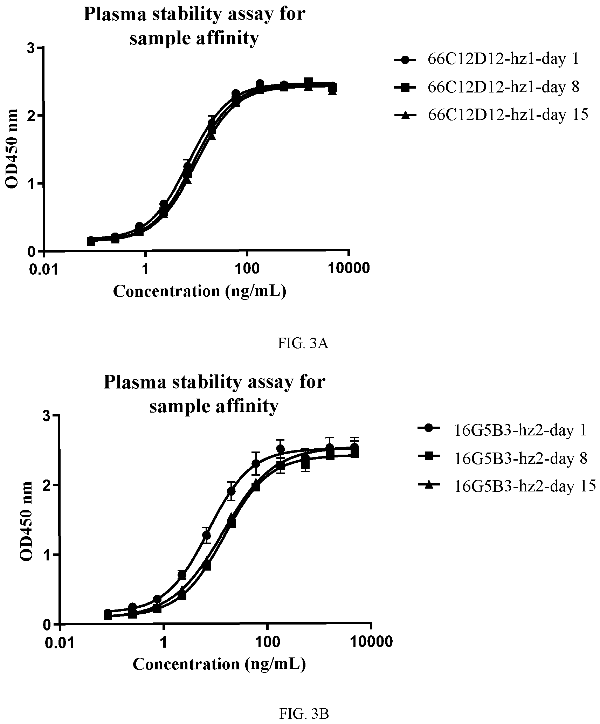

The experimental results are shown in FIGs. 3A and 3B, indicating that the 16G5B3-hz2 antibody and the 66C12D12-hz1 antibody showed no significant change in affinity in plasma on day 1, day 8, and day 15, exhibiting good serum stability.

### IV. Conjugation of Compounds Containing Cellular Bioactive Molecules and Linkers with Antibodies

### Example 5: Preparation of NaPi2b-ADCs

### Example 5.1 Preparation of NaPi2b-ADC-07

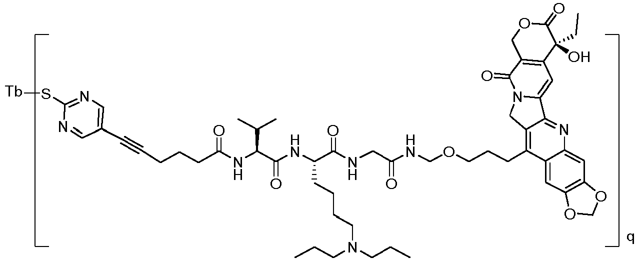

Tb is a NaPi2b antibody (including the following 66C12D2-hz1, 16G5B3-hz2, C1, etc.), and q is 1, 2, 3, 4, 5, 6, 7, 8, 9, or 10.

### 5.1.1. Preparation of NaPi2b-ADC-07 (DAR8) sample

30 mg of the anti-NaPi2b antibody 66C12D2-hz1 was diluted with a diluent (20 mM PB, pH 7.5), a sodium edetate solution at a final concentration of 1 mM was added, and the mixture was mixed well. A TCEP solution that was 5.75 times the equivalent of the antibody was added, and the mixture was mixed well and left to stand at room temperature for 90 min. DL-037 dissolved in dimethyl sulfoxide that was 12 times the equivalent of the antibody was added to the above solution system, and the mixture was mixed well and left to stand at room temperature for 2 h to obtain a conjugate sample. After the reaction was completed, the sample was exchanged into a 20 mM histidine buffer at pH 6.0 using a 30 KDa ultrafiltration tube to remove low-molecular weight substances, and finally the sample was concentrated to obtain a solution of 66C12D2-hz1-ADC07 (DAR8) containing the anti-NaPi2b antibody 66C12D2-hz1 ADC composition. The DAR value was determined by the mass spectrometry method,
with the following chromatographic conditions: chromatographic column: PLRP-S, 2.1 × 50 mm, *5* µm; mobile phase A: 0.1% FA/H₂O, mobile phase B: 0.1% FA/ACN; column temperature: 30 °C; sample chamber temperature: 8 °C; flow rate: 0.6 mL/min; injection volume: 2 µL.

**Table 6. Mobile phase gradient**

| Time (min) | 1 | 5 | 5.1 | 7 | 10 |
|---|---|---|---|---|---|
| Mobile phase A | 90 | 40 | 10 | 90 | 90 |
| Mobile phase B | 10 | 60 | 90 | 10 | 10 |

Sample treatment: 50 µg of the sample was taken, 2 µL of 1 M DTT was added, and ultrapure water was added to bring the volume to 50 µL, so that the solution was diluted to a concentration of about 1.0 mg/mL. The solution was mixed well and reduced at room temperature for 30 min.

LC/MS model: Agilent 1290-6545XT Q-TOF.

Conditions for mass spectrometry: gas temp: 320 °C, drying gas: nebulizer: 35 psi; sheath gas temp: 350 °C; sheath gas flow: 11 L/min; m/z: 500-3000.

The results are as follows:

**Table 7. Theoretical and measured molecular weights of 66C12D2-hz1-ADC07 (DAR8)**

| Peptide chain | | mAb | DAR1 | DAR2 | DAR3 |
|---|---|---|---|---|---|
| LC | Theoretical value | 23379.91 | 24397.31 | NA | NA |
| | Measured value | Undetectable | 24394 | Undetectable | Undetectable |
| HC | Theoretical value | 50280.5 | 51297.9 | 52315.3 | 53332.7 |
| | Measured value | Undetectable | 51297 | 52312 | 35328 |

In the table, mAb represents the unconjugated monoclonal antibody; LC represents the light chain of the antibody; HC represents the heavy chain of the antibody; DAR1 represents a conjugate comprising a light chain or a heavy chain conjugated to 1 toxin molecule; DAR2 represents a conjugate comprising a light chain or a heavy chain conjugated to 2 toxin molecules; DAR3 represents a conjugate comprising a light chain or a heavy chain conjugated to 3 toxin molecules; wherein the theoretical molecular weight of the monoclonal antibody was calculated based on the G0F glycoform. In the following text, mAb, LC, HC, DAR1, DAR2, and DAR3 are as described above.

In the 66C12D2-hz1-ADC07 (DAR8) sample, the light chain was conjugated to 0-1 toxin molecule (the proportions of LC and DAR1 were 0% and 100%, respectively), and the heavy chain was conjugated to 0-3 toxin molecules (the proportions of mAb, DAR1, DAR2, and DAR3 were 0%, 1%, 1%, and 98%, respectively). Therefore, the drug-to-antibody ratio (DAR value) of the 66C12D2-hz1-ADC07 (DAR8) sample was calculated to be 8.0. Based on the different numbers of toxin molecules conjugated to the heavy and light chains, it can be inferred that q can be 4, 5, 6, 7, or 8.

Following the same procedures as described above, but replacing 66C12D2-hz1 with 16G5B3-hz2, a solution of 16G5B3-hz2-ADC07 (DAR8) containing an anti-NaPi2b antibody 16G5B3-hz2 ADC composition was obtained.

### 5.1.2 Preparation of NaPi2b-ADC-07 (DAR6) sample

30 mg of the anti-NaPi2b antibody 16G5B3-hz2 was diluted with a diluent (20 mM PB, pH 7.5), a sodium edetate solution at a final concentration of 1 mM was added, and the mixture was mixed well. A TCEP solution that was 5.75 times the equivalent of the antibody was added, and the mixture was mixed well and left to stand at room temperature for 90 min. DL-037 dissolved in dimethyl sulfoxide that was 7.0 times the equivalent of the antibody was added to the above solution system, and the mixture was mixed well and left to stand at room temperature for 2 h to obtain a conjugate sample. After the reaction was completed, the sample was exchanged into a 20 mM histidine buffer at pH 6.0 using a 30 KDa ultrafiltration tube to remove low-molecular weight substances, and finally the sample was concentrated to obtain a solution of 16G5B3-hz2-ADC07 (DAR6) containing the anti-NaPi2b antibody 16G5B3-hz2 ADC composition. The results of the determination using the same mass spectrometry method as in 7.1.1 are as follows:

**Table 8. Theoretical and measured molecular weights of 16G5B3-hz2-ADC07 (DAR6)**

| Peptide chain | | mAb | DAR1 | DAR2 | DAR3 |
|---|---|---|---|---|---|
| LC | Theoretical value | 23449.1 | 24466.5 | NA | NA |
| | Measured value | 23445 | 24463 | Undetectable | Undetectable |
| HC | Theoretical value | 50207.4 | 51224.8 | 52242.2 | 53259.6 |
| | Measured value | Undetectable | 51218 | 52237 | 53255 |

In the 16G5B3-hz2-ADC07 (DAR6) sample, the light chain was conjugated to 0-1 toxin molecule (the proportions of LC and DAR1 were 18% and 82%, respectively), and the heavy chain was conjugated to 0-3 toxin molecules (the proportions of mAb, DAR1, DAR2, and DAR3 were 0%, 11%, 28%, and 60%, respectively). Therefore, the DAR value of the 16G5B3-hz2-ADC07 (DAR6) sample was calculated to be 6.6. Based on the different numbers of toxin molecules conjugated to the heavy and light chains, it can be inferred that q can be 2, 3, 4, 5, 6, 7, or 8.

Following the same procedures as described above, but replacing 16G5B3-hz2 with 66C12D2-hz1, a solution of 66C12D2-hz1-ADC07 (DAR6) containing an anti-NaPi2b antibody 66C12D2-hz1 ADC composition was obtained.

### 5.1.3 Preparation of NaPi2b-ADC-07 (DAR1) sample

30 mg of the anti-NaPi2b antibody 16G5B3-hz2 was diluted with a diluent (20 mM PB, pH 7.5), a sodium edetate solution at a final concentration of 1 mM was added, and the mixture was mixed well. A TCEP solution that was 5.75 times the equivalent of the antibody was added, and the mixture was mixed well and left to stand at room temperature for 90 min. DL-037 dissolved in dimethyl sulfoxide that was 1.5 times the equivalent of the antibody was added to the above solution system, and the mixture was mixed well and left to stand at room temperature for 2 h to obtain a conjugate sample. After the reaction was completed, the sample was exchanged into a 20 mM histidine buffer at pH 6.0 using a 30 KDa ultrafiltration tube to remove low-molecular weight substances, and finally the sample was concentrated to obtain a solution of 16G5B3-hz2-ADC07 (DAR1) containing the anti-NaPi2b antibody 16G5B3-hz2 ADC composition. The results of the determination using the same mass spectrometry method as in 7.1.1 are as follows:

**Table 9. Theoretical and measured molecular weights of 16G5B3-hz2-ADC07 (DAR1)**

| Peptide chain | | mAb | DAR1 | DAR2 | DAR3 |
|---|---|---|---|---|---|
| LC | Theoretical value | 23449.1 | 24466.5 | NA | NA |
| | Measured value | 23445 | Undetectable | Undetectable | Undetectable |
| HC | Theoretical value | 50207.4 | 51224.8 | 52242.2 | 53259.6 |
| | Measured value | 50200 | 51218 | 52237 | Undetectable |

In the 16G5B3-hz2-ADC07 (DAR1) sample, the light chain was conjugated to 0-1 toxin molecule (the proportions of LC and DAR1 were 0% and 100%, respectively), and the heavy chain was conjugated to 0-3 toxin molecules (the proportions of mAb, DAR1, DAR2, and DAR3 were 56%, 32%, 12%, and 0%, respectively). Therefore, the drug-to-antibody ratio (DAR value) of the 16G5B3-hz2-ADC07 (DAR1) sample was calculated to be 1.1. Based on the different numbers of toxin molecules conjugated to the heavy and light chains, it can be inferred that q can be 2, 3, 4, 5, 6, 7, or 8.

Following the same procedures as described above, but replacing 16G5B3-hz2 with 66C12D2-hz1, a solution of 66C12D2-hz1-ADC07 (DAR1) containing an anti-NaPi2b antibody 66C12D2-hz1 ADC composition was obtained.

### Example 5.2: Preparation of NaPi2b-ADC-04

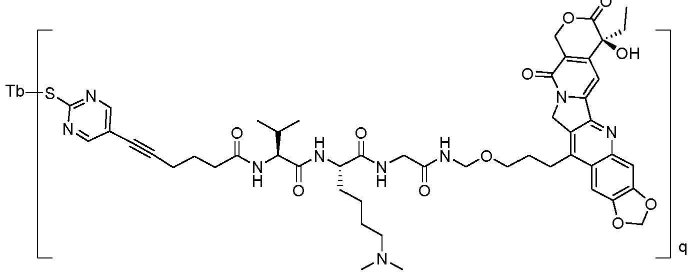

Tb is a NaPi2b antibody, and q is 1, 2, 3, 4, 5, 6, 7, 8, 9, or 10.

Following the same procedures as in 5.1.1, but replacing DL-037 with DL-019, a solution containing an anti-NaPi2b antibody-ADC composition was prepared.

### Example 5.3: Preparation of NaPi2b-ADC-06

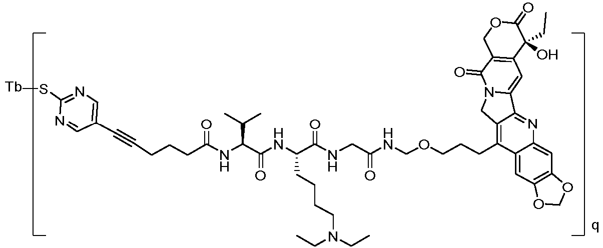

Tb is a NaPi2b antibody, and q is 1, 2, 3, 4, 5, 6, 7, 8, 9, or 10.

Following the same procedures as in 5.1.1, but replacing DL-037 with DL-036, a solution containing an anti-NaPi2b antibody-ADC composition was prepared.

### V. Assay for In Vitro Bioactivity of Bioactive Molecules

### Example 6.1: Assay 1 for Inhibitory Activity of Bioactive Molecules on In Vitro Cell Activity

Firstly, tumor cells were cultured. The bioactive molecules of the present disclosure were co-cultured with the tumor cells, and then a CCK8 reagent (Dojindo China Co., Ltd., Cat: CK04, Lot: JJ744) was added. Readings were taken using a microplate reader (manufacturer: Molecular Devices, model: SpectraMax M2) (detection wavelength: 450 nm) to determine the activity of dehydrogenase in the mitochondria, so as to evaluate the inhibitory effects of the bioactive molecules on cell proliferation. The sources of tumor cells are shown in Table 10.

**Table 10.**

| Cell name | Tumor type | Source |
|---|---|---|
| NCl-H358 | Human non-small cell lung cancer | ATCC, CRL-5807 |
| HT29 | Human colon cancer cell | ATCC, HTB-38 |
| LS174T | Human colon adenocarcinoma cell | ATCC, CL-187 |
| NCl-H322M | Human non-small cell lung cancer | ATCC |
| PANC-1 | Human pancreatic cancer tumor cell | ATCC, CRL-1469 |
| HCC1806 | Breast cancer | Nanjing Cobioer Biosciences |

The *in vitro* cell activity was assayed as follows: The bioactive molecules were diluted (12 concentration gradients) with the corresponding assay medium (containing 2% FBS). Tumor cells were digested by a conventional method using trypsin, collected in a tube, counted, and resuspended in the corresponding assay medium (containing 2% FBS). The diluted bioactive molecules were added to a 96-well plate, and then the cells were added. Subsequently, 20 µL of CCK8 reagent was added to each well, and the mixture was allowed to react for 4 h. Readings were then taken using a microplate reader (detection wavelength: 450 nm). The results are shown in Tables 11.1, 11.2, 11.3, 11.4, 11.5, and 11.6.

DXD was prepared by referring to CN104755494B, with the specific structure as follows:

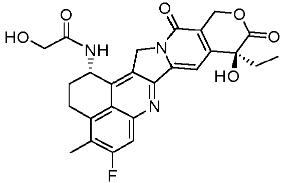

**Table 11.1. Results of killing of NC1-H358 cells by bioactive molecules**

| Name | Cell name | EC₅₀(nM) |
|---|---|---|
| A1.10 | NCl-H358 | 8.11 |
| DXD | | 39.02 |
| | | |

| Name | Cell name | EC₅₀(nM) |
|---|---|---|
| A1.8D | NCl-H358 | 7.919 |
| A1.15a | | 3.882 |
| DXD | | 21.43 |
| | | |

| Name | Cell name | EC₅₀(nM) |
|---|---|---|
| A1.4 | NCl-H358 | 29.91 |
| A1.8 | | 296.4 |
| A1.9 | | 60.79 |
| A1.11 | | 202.9 |
| A1.12 | | 167.1 |
| A1.13 | | 49.19 |
| A1.14 | | 122.6 |
| DXD | | 148.45±10.87 (n=4) |

**Table 11.2. Results of killing of HT29 cells by bioactive molecules**

| Name | Cell name | EC₅₀(nM) |
|---|---|---|
| A1.10 | HT29 | 5.965 |
| DXD | | 49.47 |
| A1.15a | HT29 | 3.574 |
| A1.15b | | 47.52 |
| DXD | | 64.84 |
| A1.4 | HT29 | 29.45 |
| A1.8 | | 367.1 |
| A1.9 | | 57.5 |
| A1.11 | | 284.5 |
| A1.12 | | 211.8 |
| A1.13 | | 96.85 |
| A1.14 | | 155.9 |
| DXD | | 301.875±69.00 (n=4) |

**Table 11.3. Results of killing of LS174T cells by bioactive molecules**

| Name | Cell name | EC₅₀(nM) |
|---|---|---|
| A1.15a | LS174T | 19.49 |
| A1.15b | | 44.62 |
| DXD | | 82.05 |

**Table 11.4. Results of killing of NCl-H322M cells by bioactive molecules**

| Name | Cell name | EC₅₀(nM) |
|---|---|---|
| A1.9 | NCl-H322M | 30.45 |
| DXD | | 76.89 |

**Table 11.5. Results of killing of PANC-1 cells by bioactive molecules**

| Name | Cell name | EC₅₀(nM) |
|---|---|---|
| A1.9 | PANC-1 | 94.54 |
| DXD | | 966 |

**Table 11.6. Results of killing of HCC1806 cells by bioactive molecules**

| Name | Cell name | EC₅₀(nM) |
|---|---|---|
| A1.9 | HCC1806 (7500 cells/well, 3 days) | 1.9 |
| Dxd | | 37 |

The assay results indicate that the bioactive molecules of the present disclosure had tumor cell-killing effects.

### Example 6.2: Assay 2 for Inhibitory Activity of Bioactive Molecules on In Vitro Cell Activity

Firstly, tumor cells NUCG-4, PC-9, HT29, NCI-H358, KYSE520, A431, and A549 were cultured. The bioactive molecules or fragments of the present disclosure were co-cultured with the tumor cells, and then a CCK8 reagent (Dojindo China Co., Ltd., Cat: CK04, Lot: JJ744) was added. Readings were taken using a microplate reader (manufacturer: Molecular Devices, model: SpectraMax M2) (detection wavelength: 450 nm) to determine the activity of dehydrogenase in the mitochondria, so as to evaluate the inhibitory effects of the bioactive molecules on cell proliferation. The sources of tumor cells are shown in Table 12.

**Table 12. Sources of tumor cells**

| Cell name | Tumor type | Source |
|---|---|---|
| NUCG-4 | Gastric cancer | Nanjing Cobioer |
| PC-9 | Lung adenocarcinoma | ATCC |
| HT29 | Colon cancer | ATCC, HTB-38 |
| NCI-H358 | Non-small cell lung cancer | ATCC, CRL-5807 |
| KYSE520 | Esophageal cancer | ATCC |
| A431 | Human skin squamous cell carcinoma (human epidermal carcinoma) cell | ATCC |
| A549 | Non-small cell lung cancer | ATCC |

The *in vitro* cell activity was assayed as follows: Tumor cells were digested by a conventional method using trypsin, collected in a tube, counted, resuspended in the corresponding assay medium (containing 2% FBS), and added to a 96-well plate at 5000-10000 cells/well. Bioactive molecules diluted with DMSO (starting at a concentration of 10 µM, with a 3-fold dilution (12 concentration gradients)) were added to the 96-well plate at 100 µL/well, and the plate was incubated at 37 °C with 5% CO₂ for 3-4 days. Subsequently, 20 µL of CCK8 reagent was added to each well, and the mixture was allowed to react for 2-6 h. Readings were then taken using a microplate reader (detection wavelength: 450 nm). The bioactive molecules and their assay results are shown in Table 13.

**Table 13. Assay for activity of A1.9 and B1.14**

| Cell | A1.9 EC₅₀ nM | B1.14EC₅₀ nM | DXD EC₅₀ nM |
|---|---|---|---|
| NUCG-4 | 8.73 | 184.8 | 43.32 |
| PC-9 | 2.06 | 34.32 | 16.69 |
| HT29 | 20.32 | 367.9 | 210.5 |
| NCI-H358 | 46.2 | 581.2 | 261.6 |
| A431 | 5.71 | 67.97 | 25.08 |
| A549 | 65.86 | 252.3 | 262.2 |

The assay results indicate that the bioactive molecules A1.9 and B1.14 had tumor cell-killing effects, with A1.9 exhibiting a stronger tumor-killing effect than Dxd in multiple tumor cell lines. In the assay for the inhibitory activity on *in vitro* cell activity of KYSE520, A1.9 had an EC50 value of 49.62 nM, indicating its ability to kill KYSE520 cells.

### Example 7: Test of In Vivo Activity of Anti-NaPi2b Antibody-Drug Conjugates

### 7.1 Test of efficacy of NaPi2b antibody-drug conjugates on HT29 human colon cancer xenograft tumor

To verify the *in vivo* efficacy of anti-human NaPi2b ADC drugs, and to evaluate the anti-tumor effects of the test drugs in a female Balb/c Nude mouse model with subcutaneous xenograft, female Balb/c Nude mice aged

5-6 weeks were purchased (Vital River). HT29-h.NaPi2b cells grown to the logarithmic growth phase were digested with EDTA and resuspended in PBS. Each mouse was subcutaneously inoculated with 5 × 10⁶ cells. When the tumor grew to a size of 100-200 mm³, intravenous administration was performed once a week at 1 mg/kg or 3 mg/kg. The specific regimen is shown in the table below. The main observation indexes of the experiment were as follows: 1) TGI (%), calculated as: TGI (%) = (1 - T/C) × 100% (T and C are relative tumor volumes at a specific time point for treatment and control groups, respectively); 2) photographs showing the tumor volume size, and tumor weight at the end of the experiment.

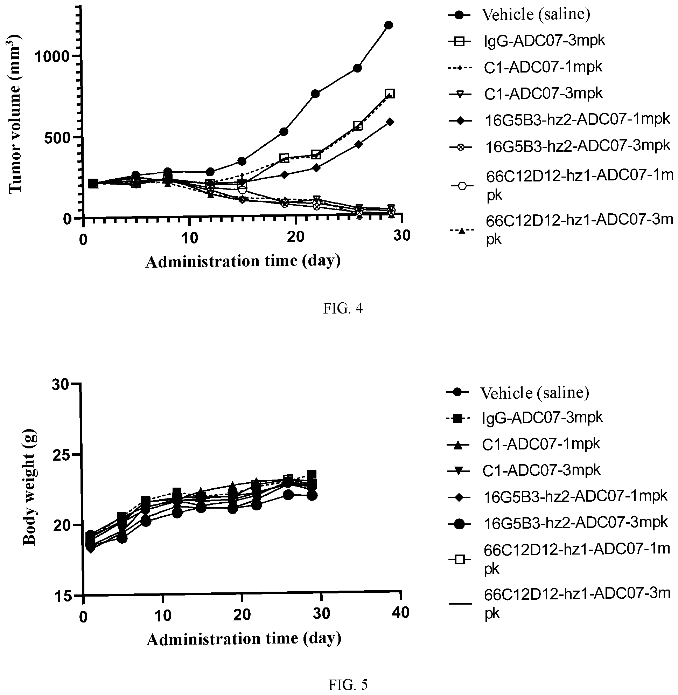

The experimental results are shown in Table 14 and FIG. 4, indicating that 16G5B3-hz2-ADC07 (DAR8) and 66C12-hz1-ADC07 (DAR8) both had good anti-tumor effects in mice; at a dose of 3 mg/kg, their TGI was comparable to that of C1-ADC07 (DAR8), and at a dose of 1 mg/kg, both were superior to C1-ADC07 (DAR8); among them, 66C12-hz1-ADC07 (DAR8) achieved a TGI of 97.83% at 1 mg/kg, which was significantly superior to the other two. The body weight changes of the mice are shown in FIG. 5, indicating that the two ADCs had no impact on the body weight of the mice throughout the administration.

**Table 14. In vivo efficacy of anti-human NaPi2b ADCs in the HT29-h.NaPi2b CDX model**

| Group | Number of mice | Sample | Dose (mg/kg) | Route of administration | Frequency of administration | TGI (%) | P value |
|---|---|---|---|---|---|---|---|
| 1 | 5 | Normal saline | / | i.v. | QW*3 | / | / |
| 2 | 5 | IgG-ADC07(DAR8) | 3 | i.v. | QW*3 | 21.71 | 0.735 |
| 3 | 5 | C1-ADC07(DAR8) | 1 | i.v. | QW*3 | 37.46 | 0.226 |
| 4 | 5 | C1-ADC07(DAR8) | 3 | i.v. | QW*3 | 96.91 | <0.001 |
| 5 | 5 | 16G5B3-hz2-ADC07(DAR8) | 1 | i.v. | QW*3 | 51.12 | 0.049 |
| 6 | 5 | 16G5B3-hz2-ADC07(DAR8) | 3 | i.v. | QW*3 | 99.34 | <0.001 |
| 7 | 5 | 66C12D12-hz1-ADC07(DAR8) | 1 | i.v. | QW*3 | 97.83 | <0.001 |
| 8 | 5 | 66C12D12-hz1-ADC07(DAR8) | 3 | i.v. | QW*3 | 100.00 | <0.001 |

### 7.2 Test of efficacy of NaPi2b antibody-drug conjugates on IGR-OV1 human ovarian cancer xenograft tumor

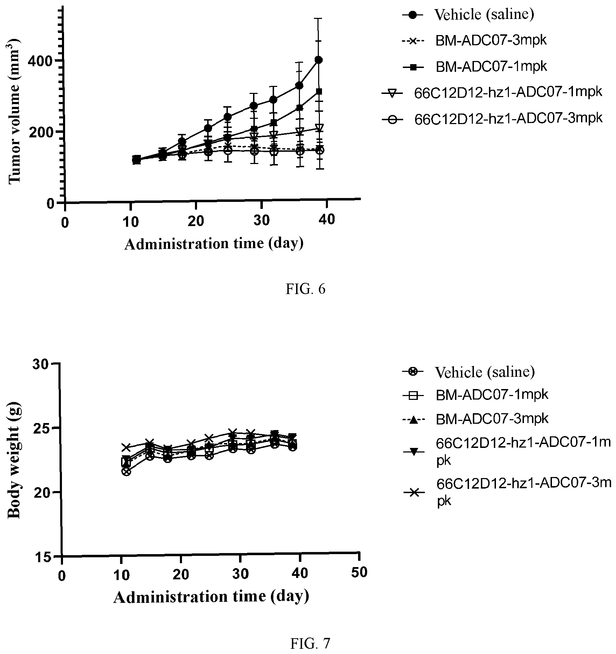

Using a similar method, the anti-tumor effect of the test drug 66C12D2-hz1-ADC07 (DAR8) in a female BALB/c nude mouse animal model with subcutaneous xenograft of the human ovarian cancer IGR-OV1 cell line was evaluated. The experimental results are shown in Table 15 and FIG. 6, indicating that 66C12D2-hz1-ADC07 (DAR8) had good anti-tumor effect in mice; at a dose of 3 mg/kg, its TGI was equivalent to that of C1-ADC07 (DAR8), and at a dose of 1 mg/kg, it was superior to C1-ADC07 (DAR8). The body weight changes of the mice are shown in FIG. 7, indicating that 66C12D2-hz1-ADC07 (DAR8) had no impact on the body weight of the mice throughout the administration.

**Table 15. In vivo efficacy of anti-human NaPi2b ADCs in the IGR-OV1 CDX model**

| Group | Number of mice | Sample | Dose (mg/kg) | Route of administration | Frequency of administration | TGI (%) | P value |
|---|---|---|---|---|---|---|---|
| 1 | 5 | Normal saline | / | i.v. | QW*3 | / | / |
| 2 | 5 | C1- ADC07(DAR8) | 1 | i.v. | QW*3 | 22.73 | 0.646 |
| 3 | 5 | C1- ADC07(DAR8) | 3 | i.v. | QW*3 | 63.76 | <0.001 |
| 4 | 5 | 66C12D2-hz1-ADC07(DAR8) | 1 | i.v. | QW*3 | 48.88 | 0.079 |
| 5 | 5 | 66C12D12-hz1-ADC07(DAR8) | 3 | i.v. | QW*3 | 64.59 | <0.001 |

Thus, the ADCs of the present disclosure have wider therapeutic indexes and improved therapeutic effects. Although specific embodiments of the present disclosure have been described in detail, those skilled in the art will understand that various modifications and variations can be made to the details based on all the teachings that have been published, and these changes are all within the protection scope of the present disclosure. The protection scope of the present disclosure is given by the appended claims and any equivalents thereof.

## Claims

1. An antibody-drug conjugate of the formula XV, 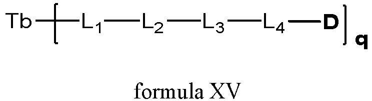
or a stereoisomer of the antibody-drug conjugate, a prodrug thereof, a pharmaceutically acceptable salt thereof, or a pharmaceutically acceptable solvate thereof,
wherein:
Tb is an anti-NaPi2b antibody or an antigen-binding fragment thereof;
q is a drug-to-antibody coupling ratio;
D is a bioactive molecular fragment;
L₁ is an extension unit;
L₂ is absent or is a connector unit;
L₃ is selected from Val-AA'-Gly, Val-AA¹, Ala-AA', Gly-AA', AA¹-Gly, Ala-AA'-Gly, Gly-AA'-Gly, AA¹-Ala-Asn, and AA¹; the structure of the amino acid residue represented by AA¹ is shown as follows: 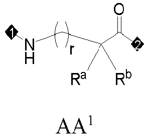
wherein: R^{a} and R^{b} are each independently selected from H, 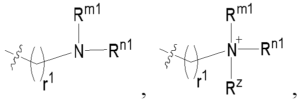 and 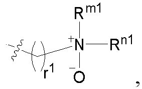 and R^{a} and R^{b} are not both H; or
R^{a} and R^{b}, together with the carbon atom to which they are both attached, form a 4-10 membered heterocyclic ring, the 4-10 membered heterocyclic ring being optionally substituted with one or more R⁰;
r and r¹ are each independently selected from any integer from 0 to 20;
R^{m1} and Rⁿ¹ are each independently selected from H, C1-6 alkyl, C3-6 cycloalkyl, and -COOR^{x1};
R^{x1} is selected from C1-6 alkyl; or
R^{m1} and Rⁿ¹, together with the nitrogen atom to which they are both attached, form a 4-10 membered heterocyclic ring, the 4-10 membered heterocyclic ring being optionally substituted with one or more R^{0'};
R^{z} is selected from C1-6 alkyl;
R⁰ and R⁰' are each independently selected from C1-6 alkyl, C3-6 cycloalkyl, -NR^{m2}Rⁿ², and 4-10 membered heterocyclyl optionally substituted with C1-6 alkyl;
R^{m2} and Rⁿ² are each independently selected from H and C1-6 alkyl;
L₄ is absent or present, and when L₄ is present, L₄ is selected from 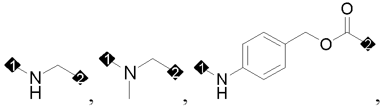 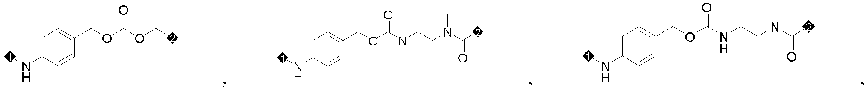 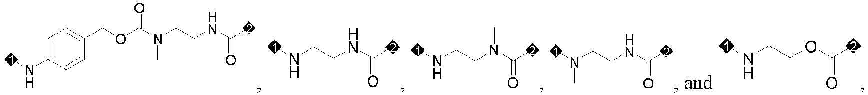 wherein position 1 is attached to L₃, and position 2 is attached to D.

2. The antibody-drug conjugate or the stereoisomer of the antibody-drug conjugate, the prodrug thereof, the pharmaceutically acceptable salt thereof, or the pharmaceutically acceptable solvate thereof according to claim 1, wherein one or more of the following conditions are met:
(1) the antibody or the antigen-binding fragment thereof represented by Tb comprises a Fab, a Fab', a F(ab')2, an Fd, an Fv, a dAb, a complementarity determining region fragment, a non-human antibody, a humanized antibody, a chimeric antibody, a fully human antibody, a probody, a monoclonal antibody, a bispecific antibody, or a multispecific antibody;
(2) q is selected from any numerical value between 0.1 and 16.0;
(3) D is a molecular fragment having anti-tumor bioactivity.

3. The antibody-drug conjugate or the stereoisomer of the antibody-drug conjugate, the prodrug thereof, the pharmaceutically acceptable salt thereof, or the pharmaceutically acceptable solvate thereof according to claim 2, wherein one or more of the following conditions are met:
(1) q is selected from 0.1, 1, 2, 3, 4, 5, 6, 7, 8, 9, 10, 11, 12, and any numerical value therebetween;
(2) the bioactive molecule in the bioactive molecular fragment is a DNA topoisomerase inhibitor or a tubulin inhibitor;
(3) L₁ is selected from 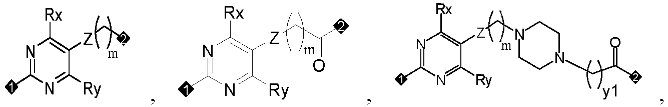 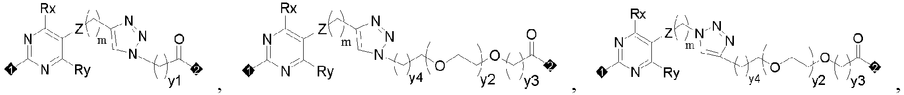 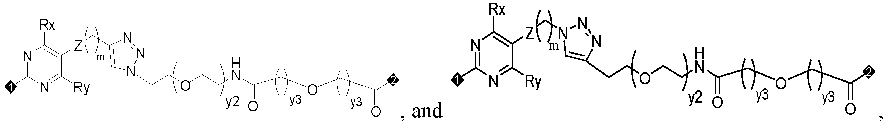 wherein each Z is independently selected from a direct bond, a carbon-carbon triple bond, a carbon-carbon double bond, C6-10 aryl, 5-10 membered heteroaryl, and acylamino; Rx and Ry are each independently selected from H and C1-4 alkyl; each m is independently selected from 0, 1, 2, 3, 4, 5, and 6; y1 is selected from any integer between 1 and 6; each y2 is independently selected from any integer between 0 and 15; each y3 is independently selected from 1, 2, and 3; each y4 is independently selected from 0 and 1; position 1 is attached to Tb via a sulfur (S) atom, and position 2 is attached to L₂ or L₃;
(4) L₂ is absent or present, and when L₂ is present, L₂ is selected from 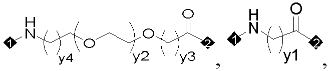 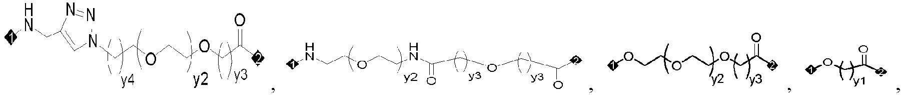 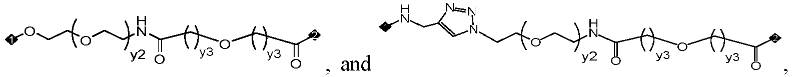 wherein y1 is selected from any integer between 1 and 6, each y2 is independently selected from any integer between 0 and 10, each y3 is independently selected from 1 and 2, each y4 is independently selected from 0 and 1, position 1 is attached to L₁, and position 2 is attached to L₃;
(5) in the amino acid residue of AA¹, either R^{a} or R^{b} is H, and the other is selected from 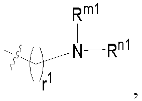 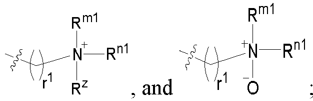 or
in the amino acid residue of AA¹, R^{a} and R^{b}, together with the carbon atom to which they are both attached, form a 5-6 membered heterocyclic ring substituted with R⁰;
(6) in the amino acid residue of AA¹, r and r¹ are each independently selected from 0, 1, 2, 3, 4, and 5;
(7) in the amino acid residue of AA¹, R^{m1} and Rⁿ¹ are each independently selected from H, methyl, ethyl, n-propyl, n-butyl, -COOCH3, -COOCH2CH3, -COOCH2CH2CH3, -COOCH(CH3)2, -COOC(CH3)3, and - COOCH2CH2CH2CH3; or R^{m1} and Rⁿ¹, together with the nitrogen atom to which they are both attached, form a 5-6 membered heterocyclic ring substituted with R^{0'};
(8) in the amino acid residue of AA¹, R^{z} is methyl;
(9) in the amino acid residue of AA¹, R⁰ and R^{0'} are each independently selected from C1-6 alkyl, -NR^{m2}Rⁿ², and 5-6 membered heterocyclyl optionally substituted with C1-6 alkyl.

4. The antibody-drug conjugate or the stereoisomer of the antibody-drug conjugate, the prodrug thereof, the pharmaceutically acceptable salt thereof, or the pharmaceutically acceptable solvate thereof according to claim 3, wherein one or more of the following conditions are met:
(1) q is selected from 0.1, 1, 2, 3, 4, 5, 6, 7, 8, and any numerical value therebetween;
(2) when the bioactive molecule in the bioactive molecular fragment is a DNA topoisomerase inhibitor, the DNA topoisomerase inhibitor is a camptothecin bioactive molecule;
(3) when the bioactive molecule in the bioactive molecular fragment is a tubulin inhibitor, the tubulin inhibitor is an MMAF tubulin inhibitor or an MMAE tubulin inhibitor;
(4) in L₁, each Z is independently selected from a direct bond, a carbon-carbon triple bond, and a carbon-carbon double bond;
(5) in L₁, y1 is 4, 5, or 6;
(6) in L₁, each y2 is independently selected from any integer between 6 and 10;
(7) L₂ is absent or present, and when L₂ is present, L₂ is selected from 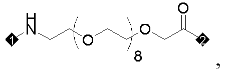 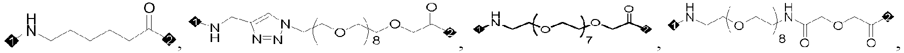 and 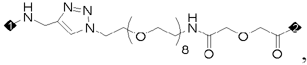 wherein position 1 is attached to L₁, and position 2 is attached to L₃;
(8) in the amino acid residue represented by AA¹, either R^{a} or R^{b} is H, and the other is selected from 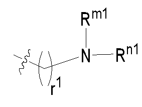 and 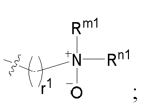 or R^{a} and R^{b}, together with the carbon atom to which they are both attached, form a 5-6 membered heterocyclic ring substituted with R⁰, the 5-6 membered heterocyclic ring substituted with R⁰ being a piperidine ring or piperazine ring substituted with R⁰;
(9) in the amino acid residue of AA¹, r and r¹ are each independently selected from 0 and 4;
(10) in the amino acid residue of AA¹, R^{m1} and Rⁿ¹ are each independently selected from H, C1-6 alkyl, C3-6 cycloalkyl, and *tert*-butoxycarbonyl; or R^{m1} and Rⁿ¹, together with the nitrogen atom to which they are both attached, form a piperidine ring or piperazine ring substituted with R^{0'};
(11) in the amino acid residue of AA¹, R⁰ is selected from C1-6 alkyl and 5-6 membered heterocyclyl substituted with C1-6 alkyl, the 5-6 membered heterocyclyl being selected from piperidinyl and piperazinyl;
(12) in the amino acid residue of AA¹, R^{0'} is selected from C1-6 alkyl and -NR^{m2}Rⁿ²;
(13) in the amino acid residue of AA¹, R^{m2} and Rⁿ² are methyl;
(14) L₄ is absent or present, and when L₄ is present, L₄ is 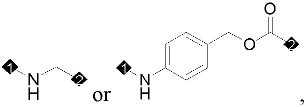 wherein position 1 is attached to L₃, and position 2 is attached to D.

5. The antibody-drug conjugate or the stereoisomer of the antibody-drug conjugate, the prodrug thereof, the pharmaceutically acceptable salt thereof, or the pharmaceutically acceptable solvate thereof according to any one of claims 1-4, wherein one or more of the following conditions are met:
(1) q is selected from 2, 3, 4, 5, 6, 7, 8, and any numerical value therebetween;
(2) L₁ is selected from 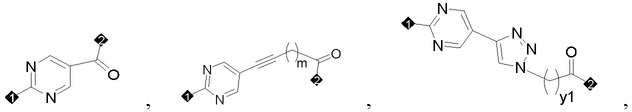 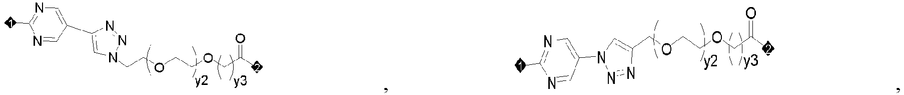 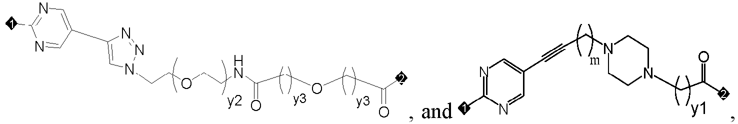 wherein m is selected from 2, 3, and 4, y1 is selected from any integer between 1 and 6, each y2 is independently selected from any integer between 0 and 10, each y3 is independently selected from 1 and 2, position 1 is attached to Tb via a sulfur (S) atom, and position 2 is attached to L₂ or L₃;
(3) L₃ is selected from AA¹, AA'-Gly, Val-AA¹-Gly, and AA'-Ala-Asn;
(4) in the amino acid residue represented by AA¹, R^{a} and R^{b}, together with the carbon atom to which they are both attached, form a 5-6 membered heterocyclic ring substituted with R⁰, the 5-6 membered heterocyclic ring substituted with R⁰ being a piperidine ring substituted with R⁰;
(5) in the amino acid residue of AA¹, either r or r¹ is 0, and the other is 4;
(6) in the amino acid residue of AA¹, R^{m1} and Rⁿ¹ are each independently selected from H and C1-6 alkyl; or R^{m1} and Rⁿ¹, together with the nitrogen atom to which they are both attached, form a piperidine ring substituted with R^{0'}
(7) in the amino acid residue of AA¹, R⁰ is selected from methyl, ethyl, and 5-6 membered heterocyclyl substituted with methyl, the 5-6 membered heterocyclyl being piperidinyl; preferably, R⁰ is selected from methyl, ethyl, and 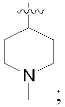
(8) in the amino acid residue of AA¹, R^{0'} is selected from methyl and -NR^{m2}Rⁿ²;
(9) when the bioactive molecule in the bioactive molecular fragment is a DNA topoisomerase inhibitor, and the DNA topoisomerase inhibitor is a camptothecin bioactive molecule, the camptothecin bioactive molecule is camptothecin, DXD, camptothecin with a modified substituent, or DXD with a modified substituent.

6. The antibody-drug conjugate or the stereoisomer of the antibody-drug conjugate, the prodrug thereof, the pharmaceutically acceptable salt thereof, or the pharmaceutically acceptable solvate thereof according to any one of claims 1-5, wherein one or more of the following conditions are met:
(1) q is selected from 3, 4, 5, 6, 7, 8, and any numerical value therebetween; preferably, q is selected from 4, 5, 6, 7, 8, and any numerical value therebetween; preferably, q is selected from 6, 7, 8, and any numerical value therebetween;
(2) L₁ is selected from 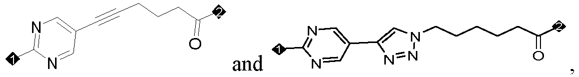 wherein position 1 is attached to Tb via a sulfur (S) atom, and position 2 is attached to L₂ or L₃;
(3) L₂ is absent or is 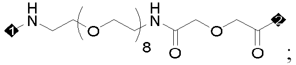
(4) L₃ is selected from AA¹ and Val-AA¹-Gly; preferably, L₃ is selected from Val-AA¹-Gly;
(5) in the amino acid residue represented by AA¹, R^{a} and R^{b}, together with the carbon atom to which they are both attached, form 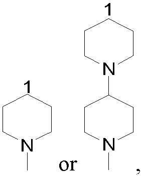 wherein the carbon atom at position 1 is the carbon atom to which R^{a} and R^{b} are both attached;
(6) in r and r', when r is 4, and r¹ is 0, R^{m1} and Rⁿ¹ are each independently selected from H and C1-6 alkyl; when r is 0, and r¹ is 4, R^{m1} and Rⁿ¹ are each independently selected from C1-6 alkyl, or R^{m1} and Rⁿ¹, together with the nitrogen atom to which they are both attached, form 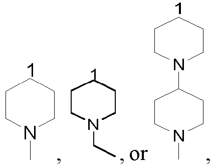 wherein the carbon atom at position 1 is the carbon atom to which R^{a} and R^{b} are both attached.

7. The antibody-drug conjugate or the stereoisomer of the antibody-drug conjugate, the prodrug thereof, the pharmaceutically acceptable salt thereof, or the pharmaceutically acceptable solvate thereof according to any one of claims 1-6, wherein the amino acid residue represented by AA¹ is selected from 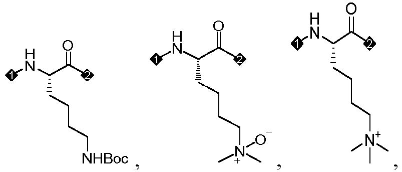 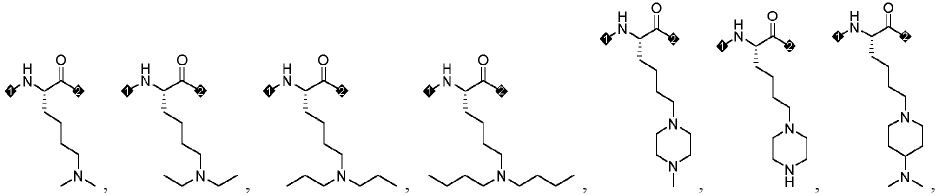 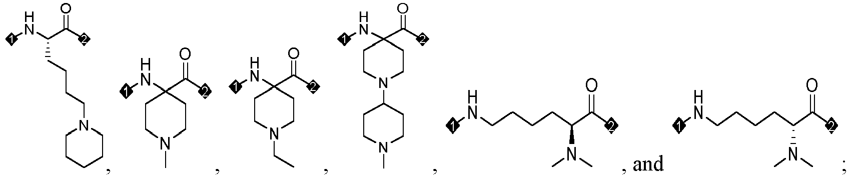 most preferably, the amino acid residue represented by AA¹ is selected from

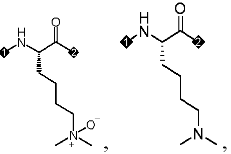

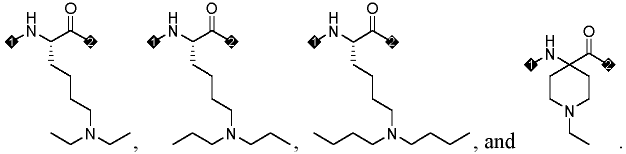

8. The antibody-drug conjugate or the stereoisomer of the antibody-drug conjugate, the prodrug thereof, the pharmaceutically acceptable salt thereof, or the pharmaceutically acceptable solvate thereof according to any one of claims 1-7, wherein
L₃ is selected from 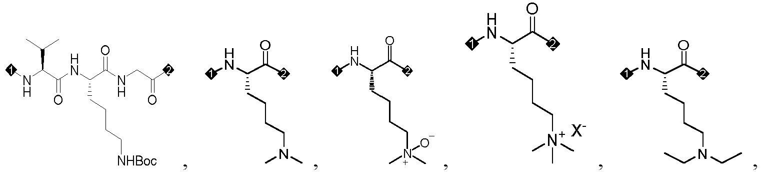 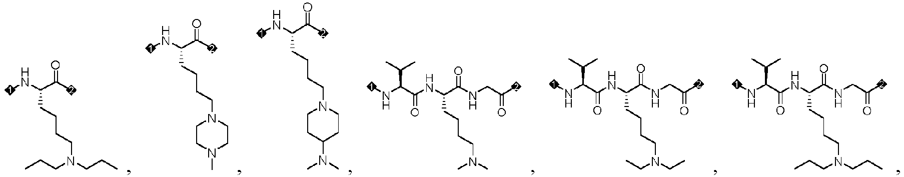 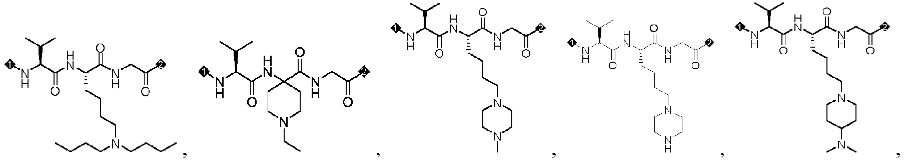 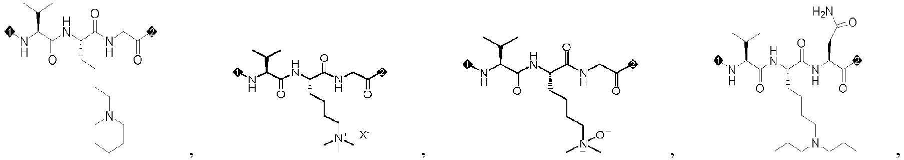 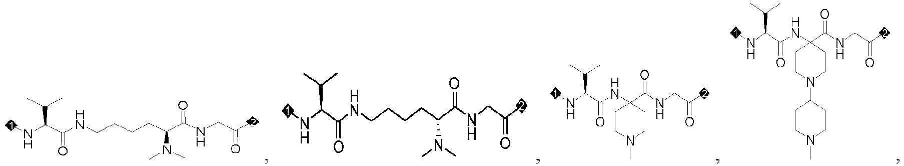 and 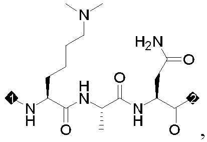 wherein X⁻ is selected from a halide ion, a carboxylate ion, a sulfate ion, a hydrogen sulfate ion, and OH⁻, position 1 is attached to L₁ or L₂, and position 2 is attached to L₄ or D;
most preferably, L₃ is selected from 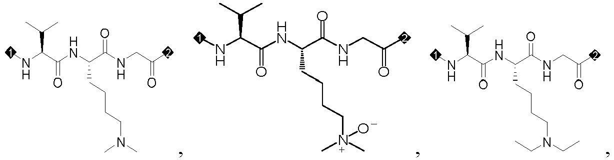 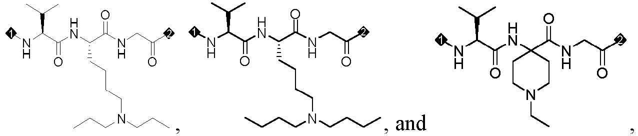 wherein position 1 is attached to L₁ or L₂, and position 2 is attached to L₄ or D.

9. The antibody-drug conjugate or the stereoisomer of the antibody-drug conjugate, the prodrug thereof, the pharmaceutically acceptable salt thereof, or the pharmaceutically acceptable solvate thereof according to any one of claims 1-8, wherein
the structure of 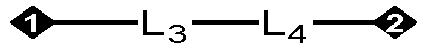 is selected from the following structural fragments: 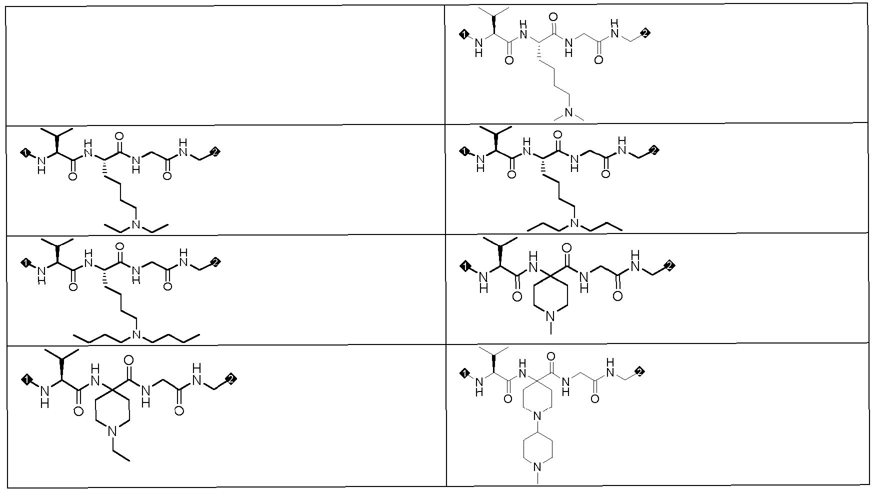
preferably, the structure of 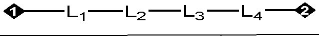
is selected from the following: 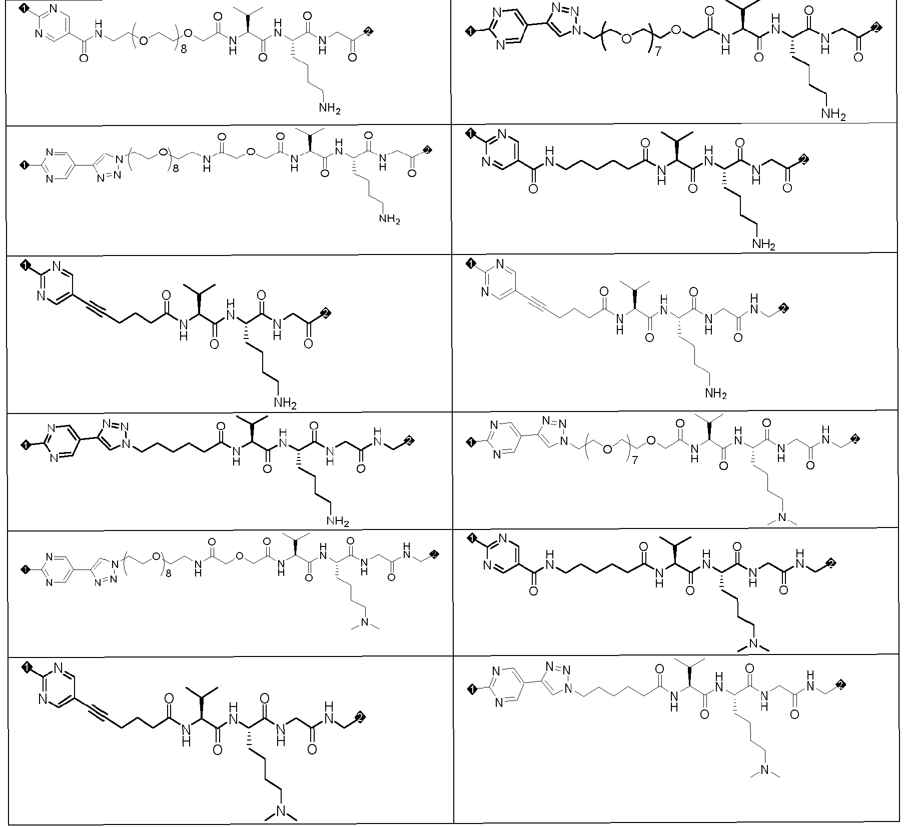 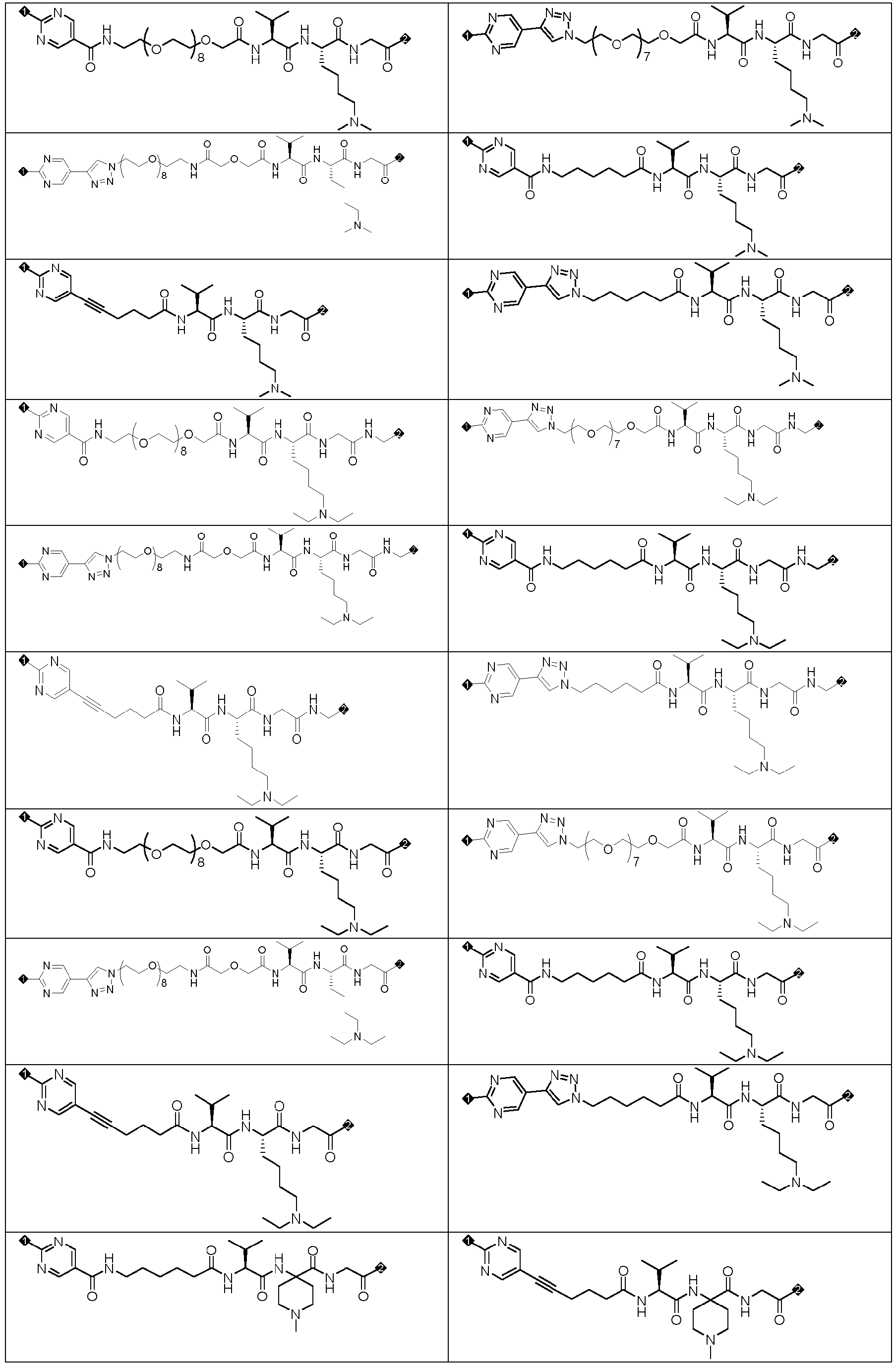 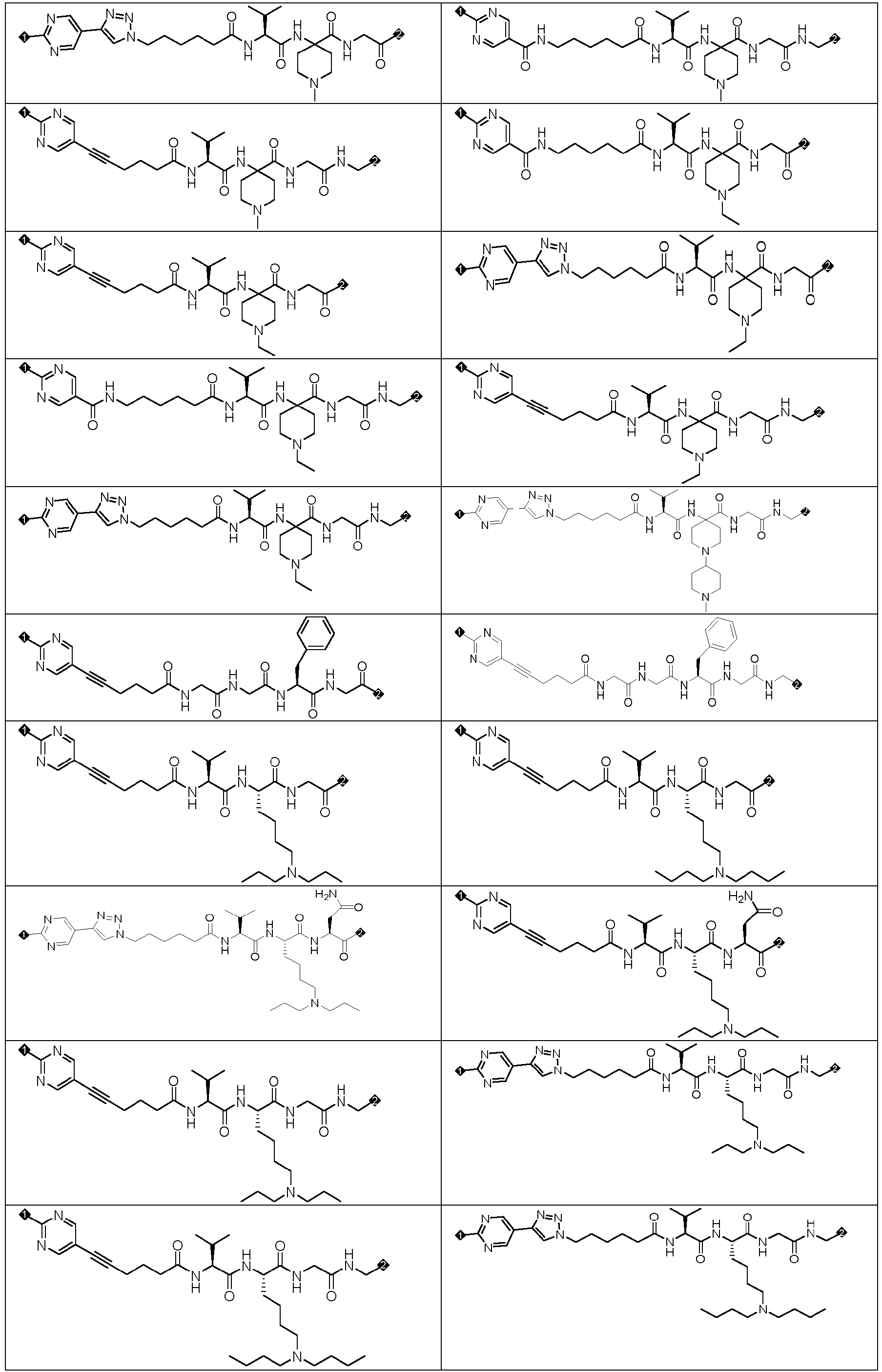
wherein position 1 is attached to Tb, and position 2 is attached to D.

10. The antibody-drug conjugate or the stereoisomer of the antibody-drug conjugate, the prodrug thereof, the pharmaceutically acceptable salt thereof, or the pharmaceutically acceptable solvate thereof according to any one of claims 1-9, wherein
the antibody-drug conjugate has a structure of formula I: 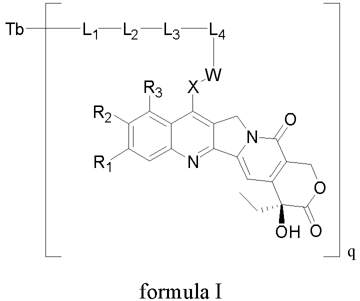
wherein: Tb, L1, L2, L3, L4, and q are as defined in any one of claims 1-9;
R₁ and R₂ are each independently selected from H, halogen, -OH, optionally substituted C1-6 alkyl, and optionally substituted C1-6 alkoxy, or
R₁ and R₂, together with the carbon atoms to which they are attached, form a 5-7 membered carbocyclic ring, or a 5-7 membered heterocyclic ring comprising one or more O, S, N, carbonyl, sulfoxide group or sulfone group or any combination thereof;
R₃ is selected from H, halogen, -OH, -NH₂, optionally substituted C1-6 alkyl, and optionally substituted C1-6 alkoxy, or
R₃ and X, together with the carbon atoms to which they are attached, form a 5-7 membered carbocyclic ring, or a 5-7 membered heterocyclic ring comprising one or more O, S, N, carbonyl, sulfoxide group or sulfone group or any combination thereof, or
R₃ and R₂, together with the carbon atoms to which they are attached, form a 5-7 membered carbocyclic ring, or a 5-7 membered heterocyclic ring comprising one or more O, S, N, carbonyl, sulfoxide group or sulfone group or any combination thereof;
W is absent or present, and when W is present, W is selected from -O-, -S-, -NR₄-, 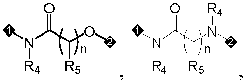 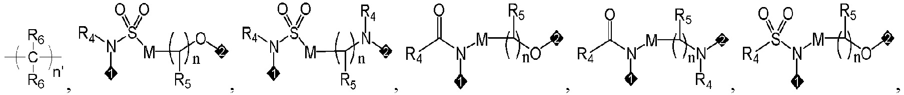
and 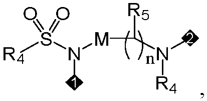 wherein position 1 is attached to X, and position 2 is attached to L₄ or L₃;
X is selected from a direct bond, optionally substituted -O-(CH₂)ₙ₃-, -NR₄-(CH₂)ₙ₃-, -S-(CH₂)ₙ₃-, carbonyl-(CH₂)ₙ₃-, -SO₂-(CH₂)ₙ₃-, -(CH₂)ₙ₁-, 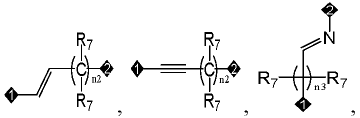 C3-6 cycloalkyl, C6-10 aryl, 5-10 membered heteroaryl, and 4-10 membered heterocyclyl, wherein position 1 is attached to the parent ring, and position 2 is attached to W or L₄; the substituent is selected from one or more C1-4 alkyl, C3-6 cycloalkyl, and C3-6 cycloalkyl formed by multiple C1-4 alkyl together with the carbon atom to which they are simultaneously attached;
each M is independently selected from a direct bond and -CR^{5a}R^{5b}-;
R₄, R₅, R^{5a}, R^{5b}, R₆, and R₇ are each independently selected from H, optionally substituted C1-4 alkyl, optionally substituted C1-4 alkoxy, and optionally substituted C3-6 cycloalkyl;
n, n', n1, n2, and n3 are each independently selected from any integer between 0 and 6;
position 1 is attached to the camptothecin parent nucleus, and position 2 is attached to W or L₄.

11. The antibody-drug conjugate or the stereoisomer of the antibody-drug conjugate, the prodrug thereof, the pharmaceutically acceptable salt thereof, or the pharmaceutically acceptable solvate thereof according to claim 10, wherein one or more of the following conditions are met:
(1) R₁ and R₂ are each independently selected from H, halogen, and C1-4 alkyl; or R₁ and R₂, together with the carbon atoms to which they are attached, form a 5-6 membered heterocyclic ring comprising 1, 2, or 3 O, S, or N, or any combination thereof;
(2) R₃ is selected from H and C1-4 alkyl; or R₃ and X, together with the carbon atoms to which they are attached, form a 5-6 membered carbocyclic ring;
(3) W is absent or present, and when W is present, W is selected from -O-, -S-, -NR₄-, 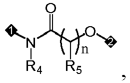 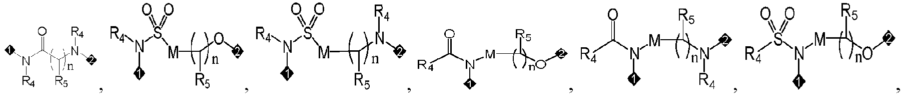 and 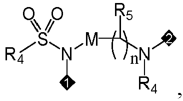 wherein position 1 is attached to X, and position 2 is attached to L₄ or L₃;
(4) X is selected from optionally substituted -(CH₂)ₙ₁-, 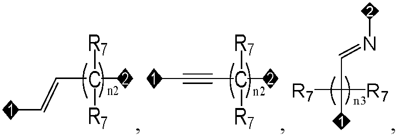 C6-10 aryl, 5-10 membered heteroaryl, and 4-10 membered heterocyclyl, wherein position 1 is attached to the parent ring, and position 2 is attached to W or L₄; the substituent is selected from 1 or 2 C1-4 alkyl, and C3-6 cycloalkyl formed by 2 C1-4 alkyl together with the carbon atom to which they are both attached;
(5) R₄ and R₅ are each independently selected from H, C1-4 alkyl, and C3-6 cycloalkyl;
(6) R^{5a} and R^{5b} are each independently selected from H and C1-4 alkyl;
(7) each R₇ is independently selected from H and C1-4 alkyl;
(8) n is selected from 1, 2, and 3;
(9) n1 is selected from 1, 2, 3, and 4;
(10) n2 is 1;
(11) n3 is 0.

12. The antibody-drug conjugate or the stereoisomer of the antibody-drug conjugate, the prodrug thereof, the pharmaceutically acceptable salt thereof, or the pharmaceutically acceptable solvate thereof according to claim 10 or 11, wherein one or more of the following conditions are met:
(1) R₁ is selected from H and halogen, and R₂ is selected from H and C1-4 alkyl; or R₁ and R₂, together with the carbon atoms to which they are attached, form 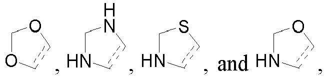 wherein the dotted line indicates the position at which the heterocyclic ring is fused to the phenyl ring;
(2) R₃ is H; or R₃ and X, together with the carbon atoms to which they are attached, form 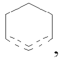 wherein the dotted line indicates the position at which the carbocyclic ring is fused to the phenyl ring and the pyridine ring;
(3) W is absent or present, and when W is present, W is selected from -O-, -S-, -NR₄-, 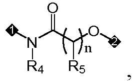 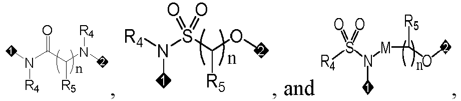 wherein position 1 is attached to X, and position 2 is attached to L₄ or L₃;
(4) each R₄ is independently selected from H, C1-4 alkyl, and C3-6 cycloalkyl, and R₅ is H;
(5) R^{5a} and R^{5b} are each independently selected from H and methyl;
(6) R₇ is H;
(7) n is 1.

13. The antibody-drug conjugate or the stereoisomer of the antibody-drug conjugate, the prodrug thereof, the pharmaceutically acceptable salt thereof, or the pharmaceutically acceptable solvate thereof according to any one of claims 10-12, wherein one or more of the following conditions are met:
(1) R₁ is H or F, and R₂ is H or methyl; or R₁ and R₂, together with the carbon atoms to which they are attached, form 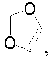 wherein the dotted line indicates the position at which the heterocyclic ring is fused to the phenyl ring;
(2) W is selected from -O-, -NR₄-, and 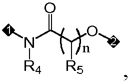 wherein position 1 is attached to X, and position 2 is attached to L₄ or L₃;
(3) each R₄ is independently selected from H, methyl, ethyl, n-propyl, isopropyl, *tert-butyl,* and cyclopropyl, and R₅ is H;
(4) X is selected from optionally substituted 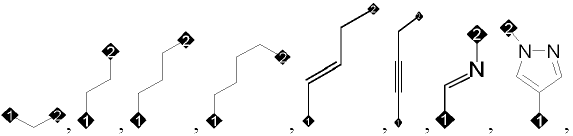 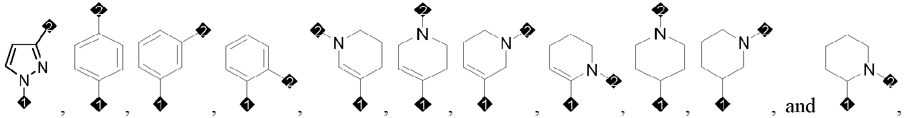 wherein position 1 is attached to the parent ring, and position 2 is attached to W or L₄; the substituent is selected from 1 or 2 C1-4 alkyl, and C3-6 cycloalkyl formed by 2 C1-4 alkyl together with the carbon atom to which they are both attached; the C1-4 alkyl is, for example, methyl; the C3-6 cycloalkyl is, for example, cyclopropyl.

14. The antibody-drug conjugate or the stereoisomer of the antibody-drug conjugate, the prodrug thereof, the pharmaceutically acceptable salt thereof, or the pharmaceutically acceptable solvate thereof according to any one of claims 10-13, wherein
(1) W is selected from -O- and -NR₄-;
(2) X is selected from 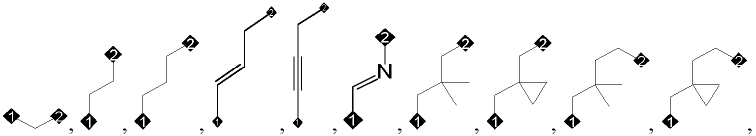 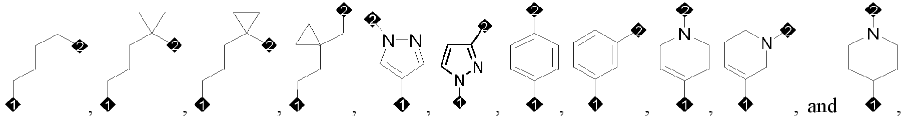 wherein position 1 is attached to the parent ring, and position 2 is attached to W or L₄.

15. The antibody-drug conjugate or the stereoisomer of the antibody-drug conjugate, the prodrug thereof, the pharmaceutically acceptable salt thereof, or the pharmaceutically acceptable solvate thereof according to any one of claims 10-14, wherein
when W is absent, X is selected from 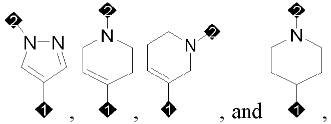 wherein position 1 is attached to the parent ring, and position 2 is attached to L₄; when W is present, X is selected from 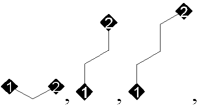 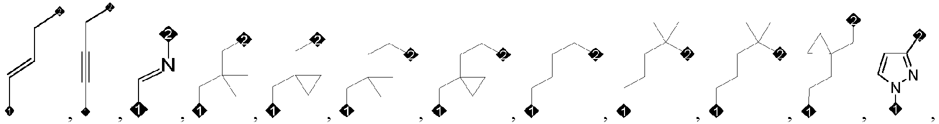 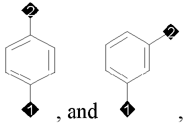 wherein position 1 is attached to the parent ring, and position 2 is attached to W.

16. The antibody-drug conjugate or the stereoisomer of the antibody-drug conjugate, the prodrug thereof, the pharmaceutically acceptable salt thereof, or the pharmaceutically acceptable solvate thereof according to any one of claims 10-15, wherein
the structure of 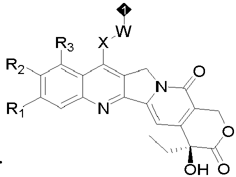
is selected from the following: 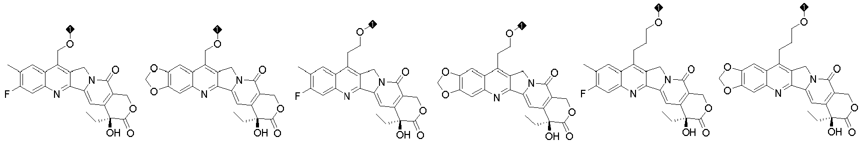 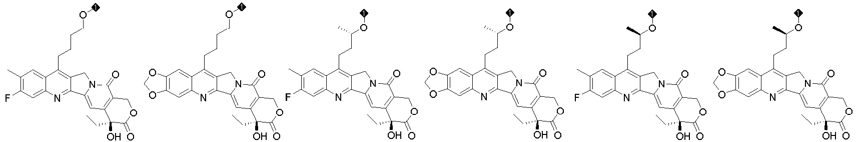 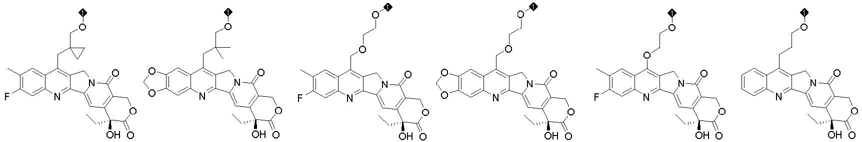 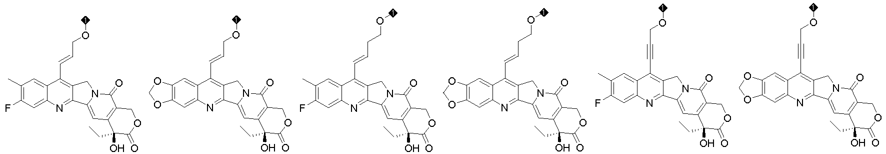 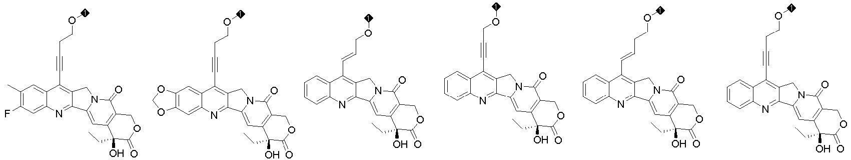 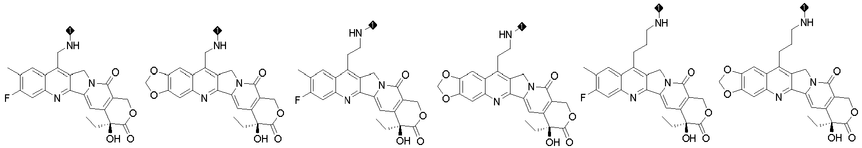 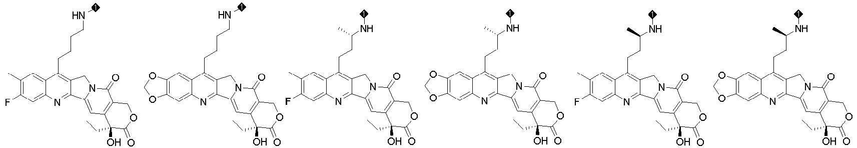 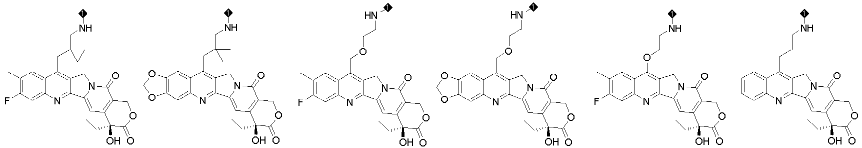 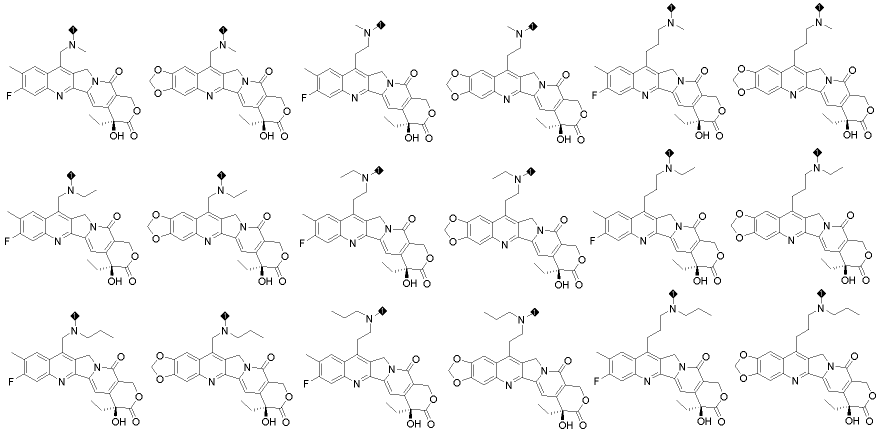 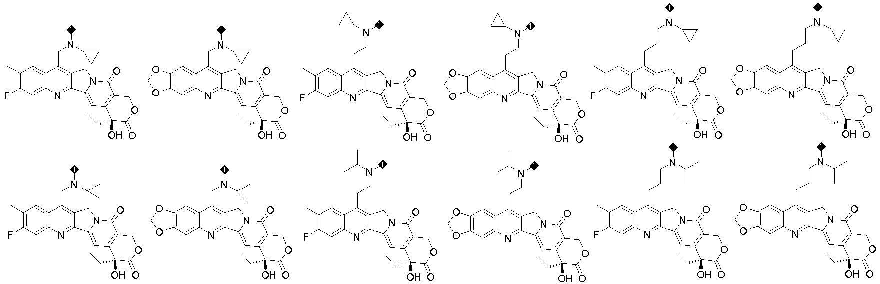 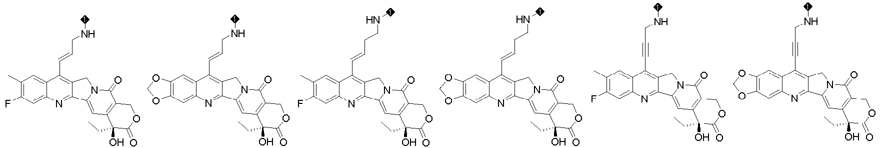 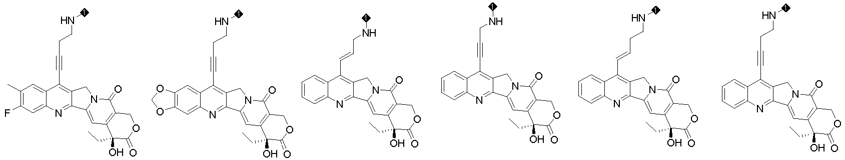 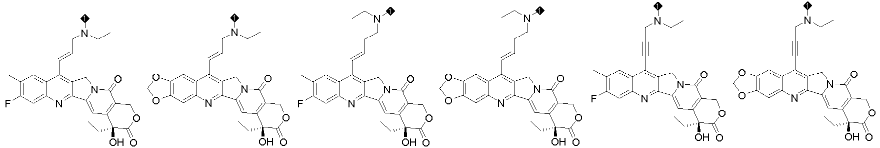 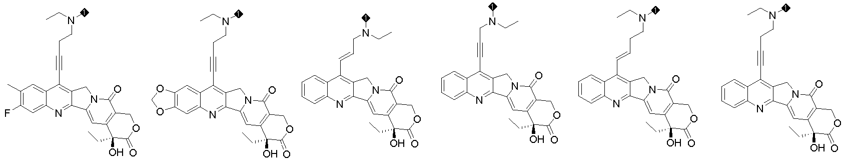 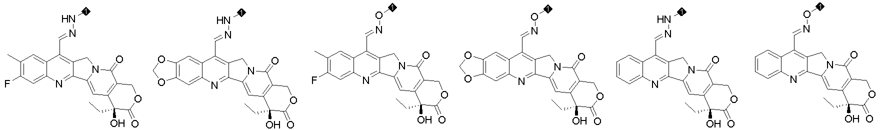 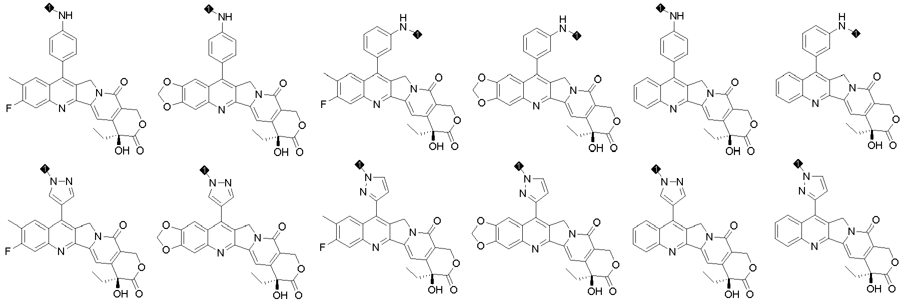 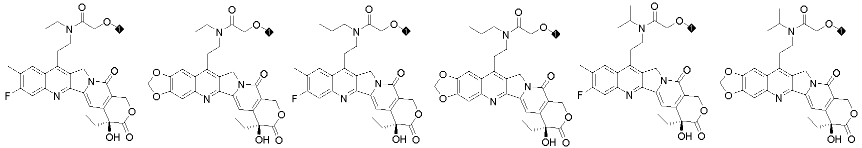 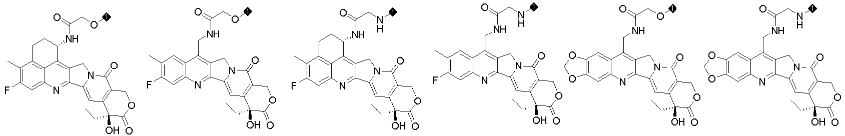 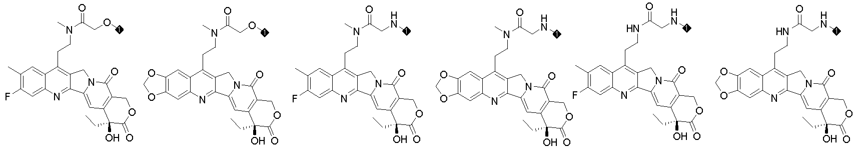
wherein position 1 is attached to L₄; when L₄ is absent, position 1 is attached to L₃.

17. The antibody-drug conjugate or the stereoisomer of the antibody-drug conjugate, the prodrug thereof, the pharmaceutically acceptable salt thereof, or the pharmaceutically acceptable solvate thereof according to any one of claims 10-16, wherein the antibody-drug conjugate has a structure of formula I-1, formula I-2, or formula I-3: 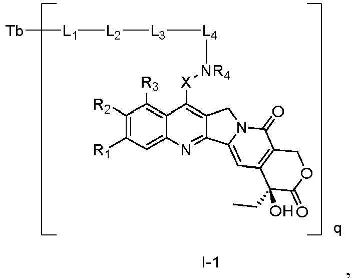
wherein Tb, L₁, L₂, L₃, L₄, X, R₁, R₂, R₃, R₄, and q are as defined in any one of claims 1-16; 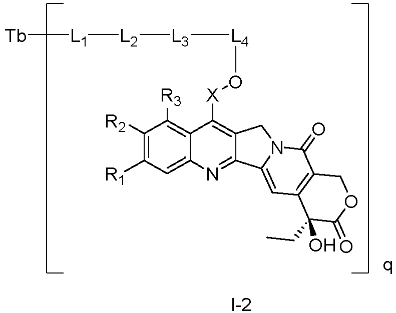
wherein Tb, L₁, L₂, L₃, L₄, X, R₁, R₂, R₃, and q are as defined in any one of claims 1-16; 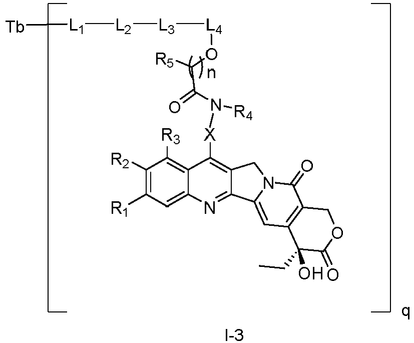
wherein Tb, L₁, L₂, L₃, L₄, X, R₁, R₂, R₃, R₄, R₅, n, and q are as defined in any one of claims 1-16.

18. The antibody-drug conjugate or the stereoisomer of the antibody-drug conjugate, the prodrug thereof, the pharmaceutically acceptable salt thereof, or the pharmaceutically acceptable solvate thereof according to any one of claims 10-17, wherein the antibody-drug conjugate has a structure of formula I-1A, formula I-1B, formula I-2A, formula I-2B, formula I-3A, or formula I-3B: 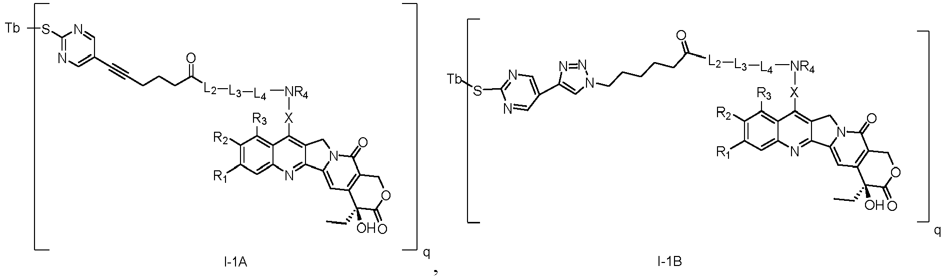
wherein Tb, L₂, L₃, L₄, X, R₁, R₂, R₃, R₄, and q are as defined in any one of claims 1-16; 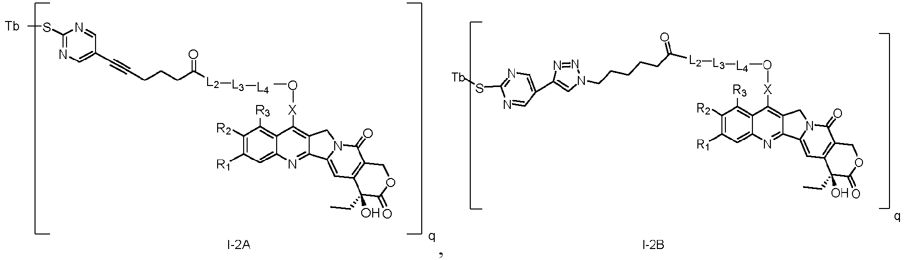
wherein Tb, L₂, L₃, L₄, X, R₁, R₂, R₃, and q are as defined in any one of claims 1-16; 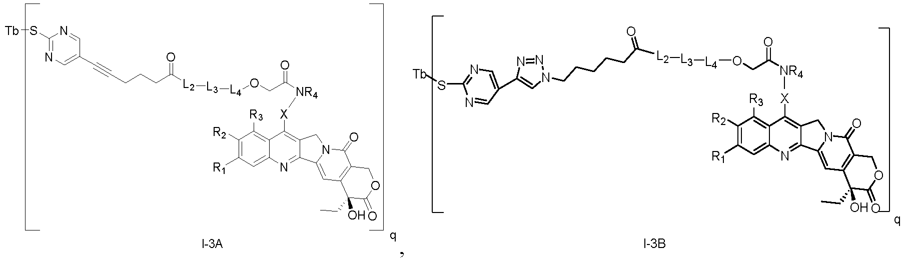
wherein Tb, L₂, L₃, L₄, X, R₁, R₂, R₃, R₄, and q are as defined in any one of claims 1-16.

19. The antibody-drug conjugate or the stereoisomer of the antibody-drug conjugate, the prodrug thereof, the pharmaceutically acceptable salt thereof, or the pharmaceutically acceptable solvate thereof according to any one of claims 10-18, wherein the antibody-drug conjugate has a structure of formula I-A or formula I-B: 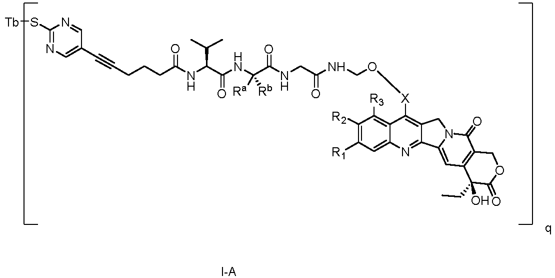
wherein Tb, X, R₁, R₂, R₃, R^{a}, R^{b}, and q are as defined in any one of claims 1-16; 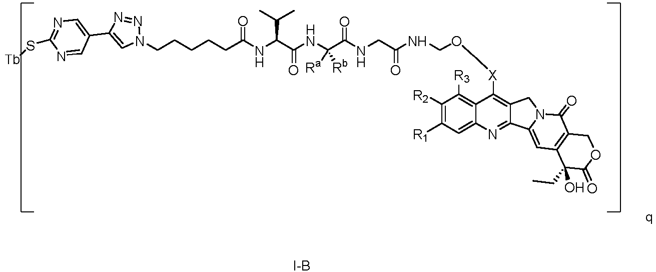
wherein Tb, X, R₁, R₂, R₃, R^{a}, R^{b}, and q are as defined in any one of claims 1-16.

20. The antibody-drug conjugate or the stereoisomer of the antibody-drug conjugate, the prodrug thereof, the pharmaceutically acceptable salt thereof, or the pharmaceutically acceptable solvate thereof according to any one of claims 1-19, wherein the antibody-drug conjugate is selected from the following:

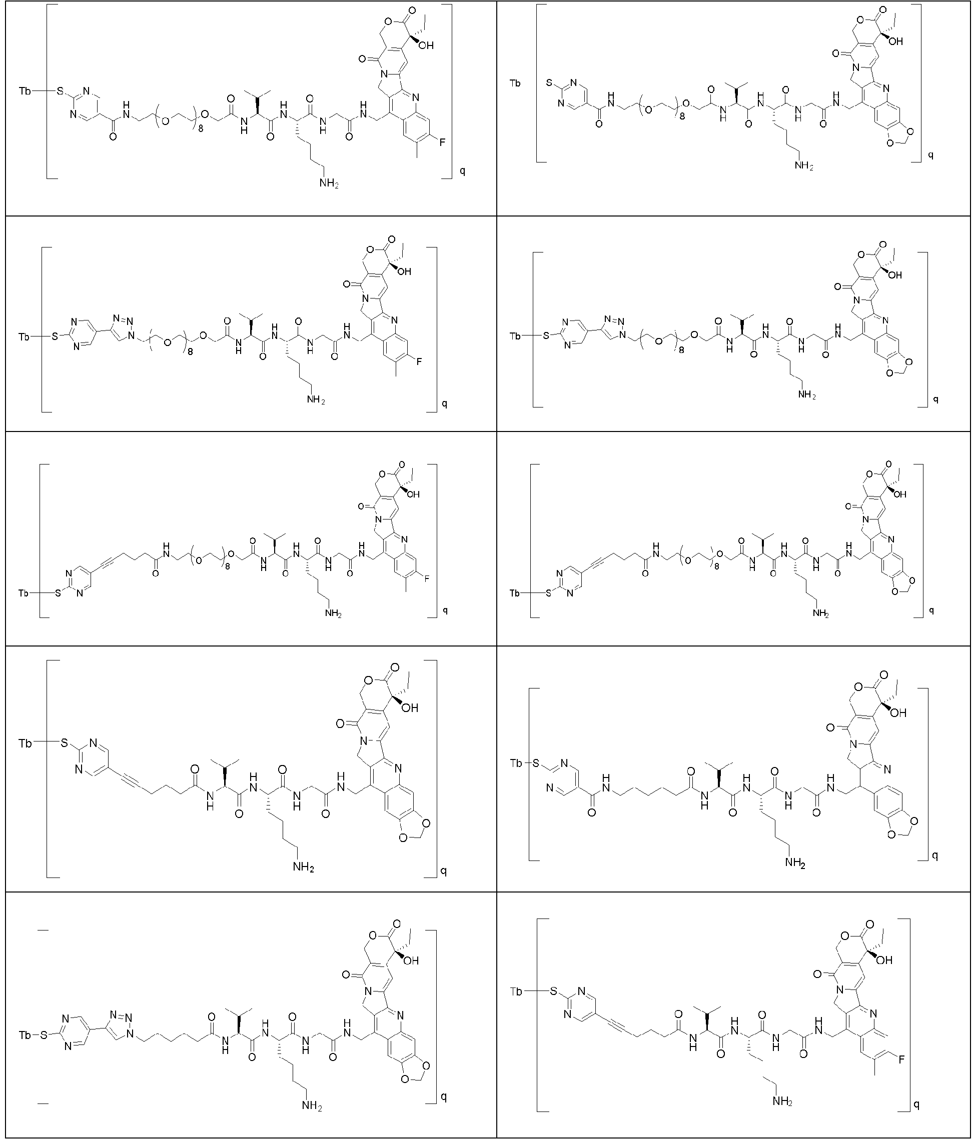

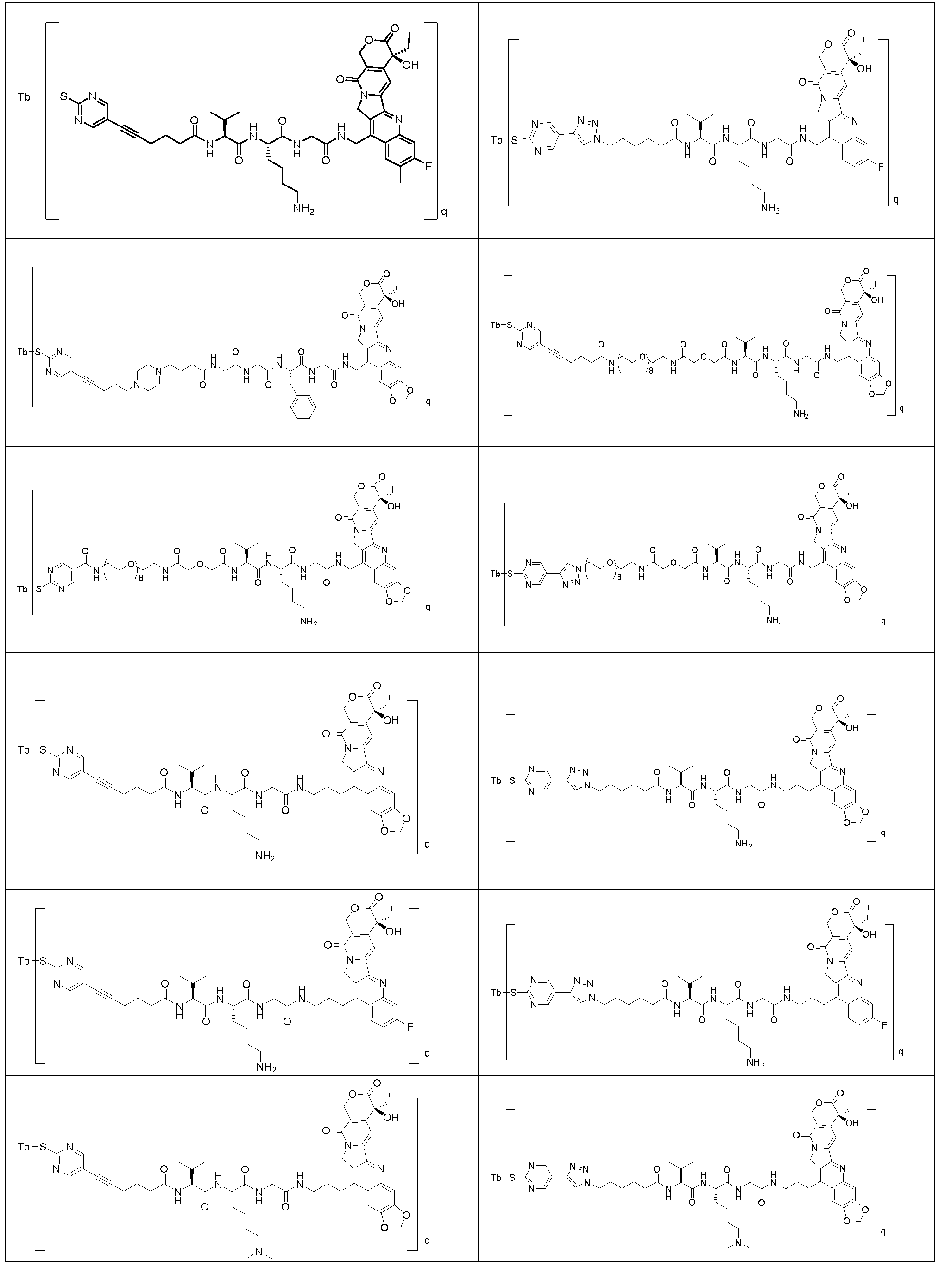

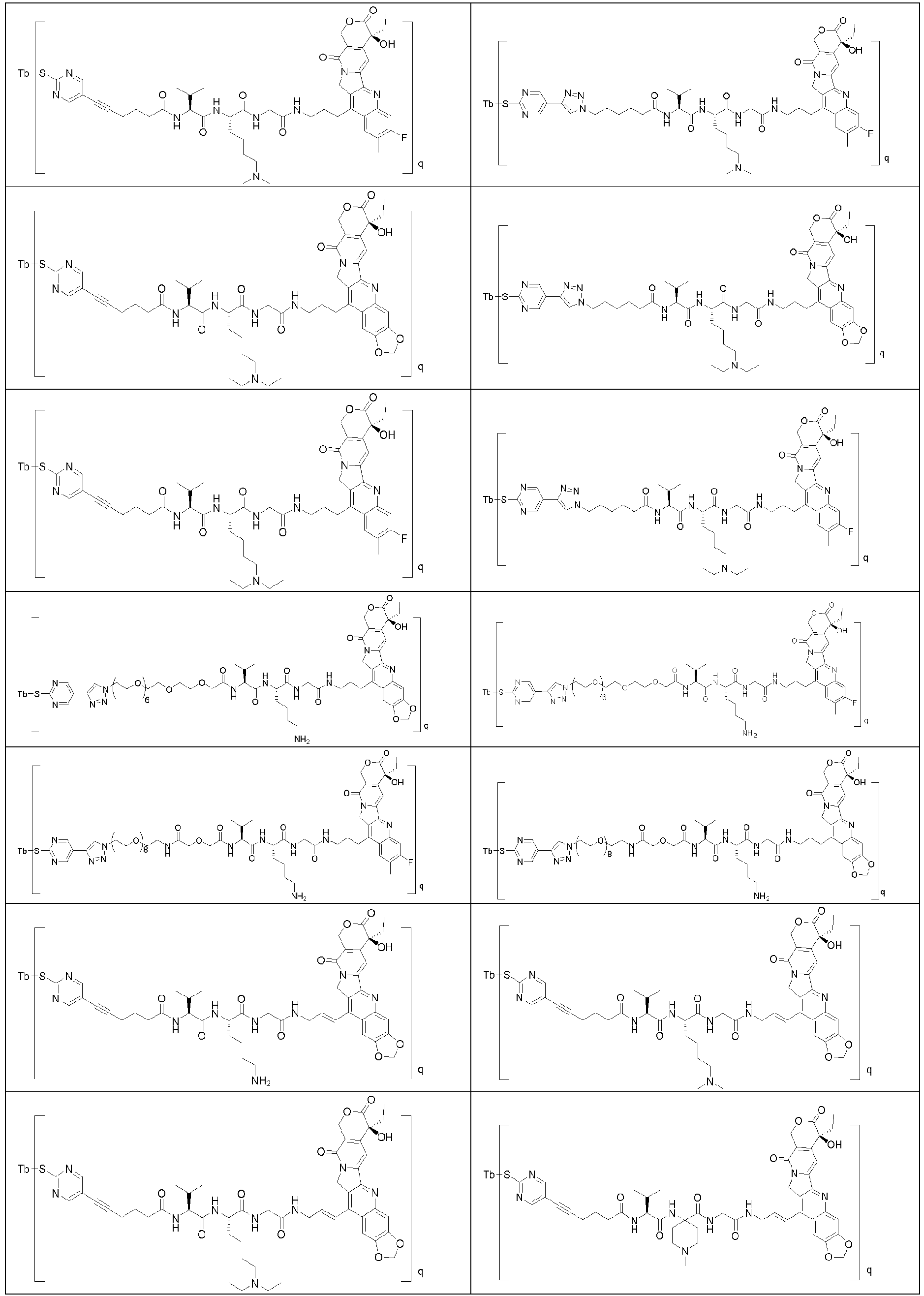

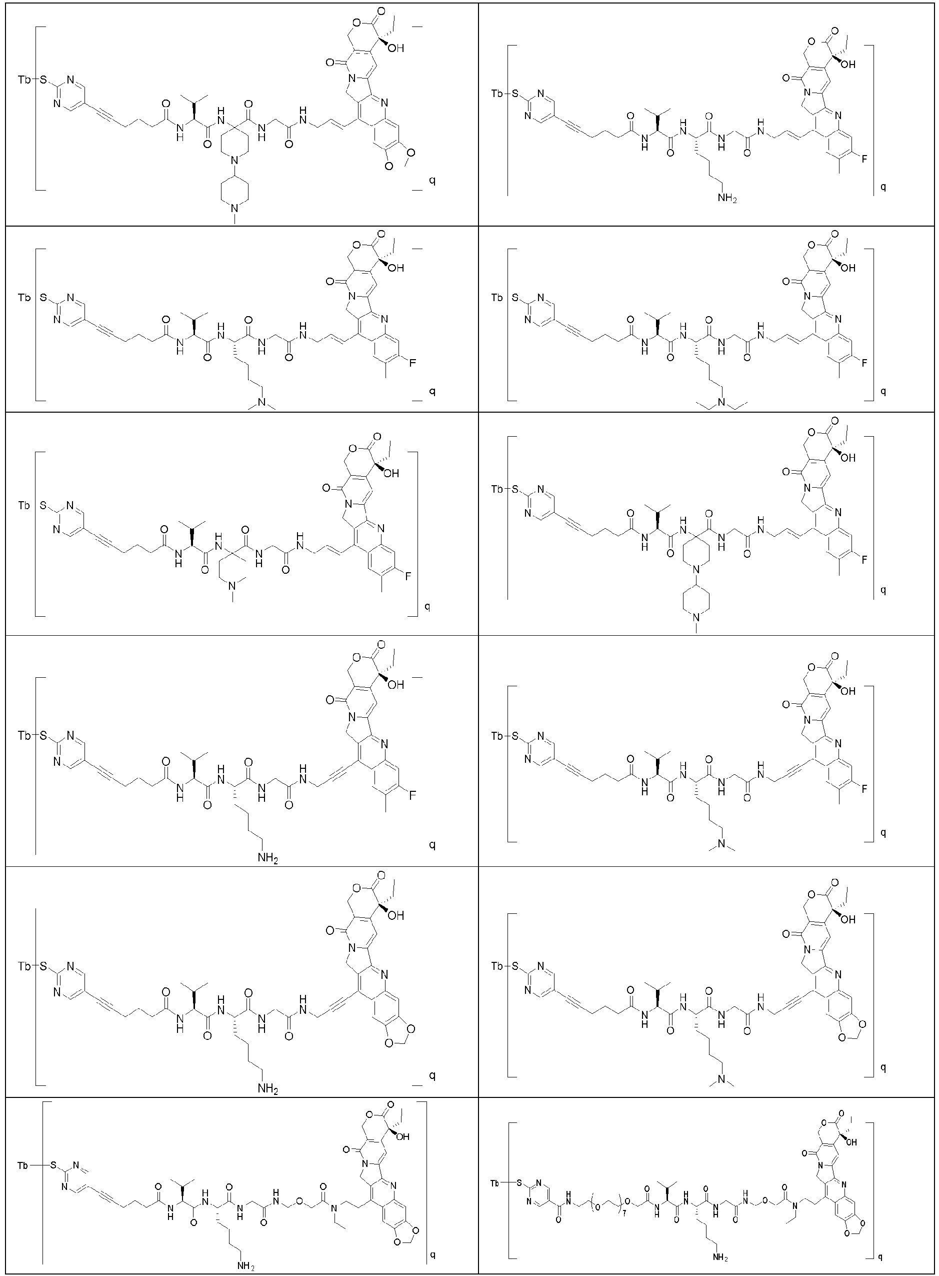

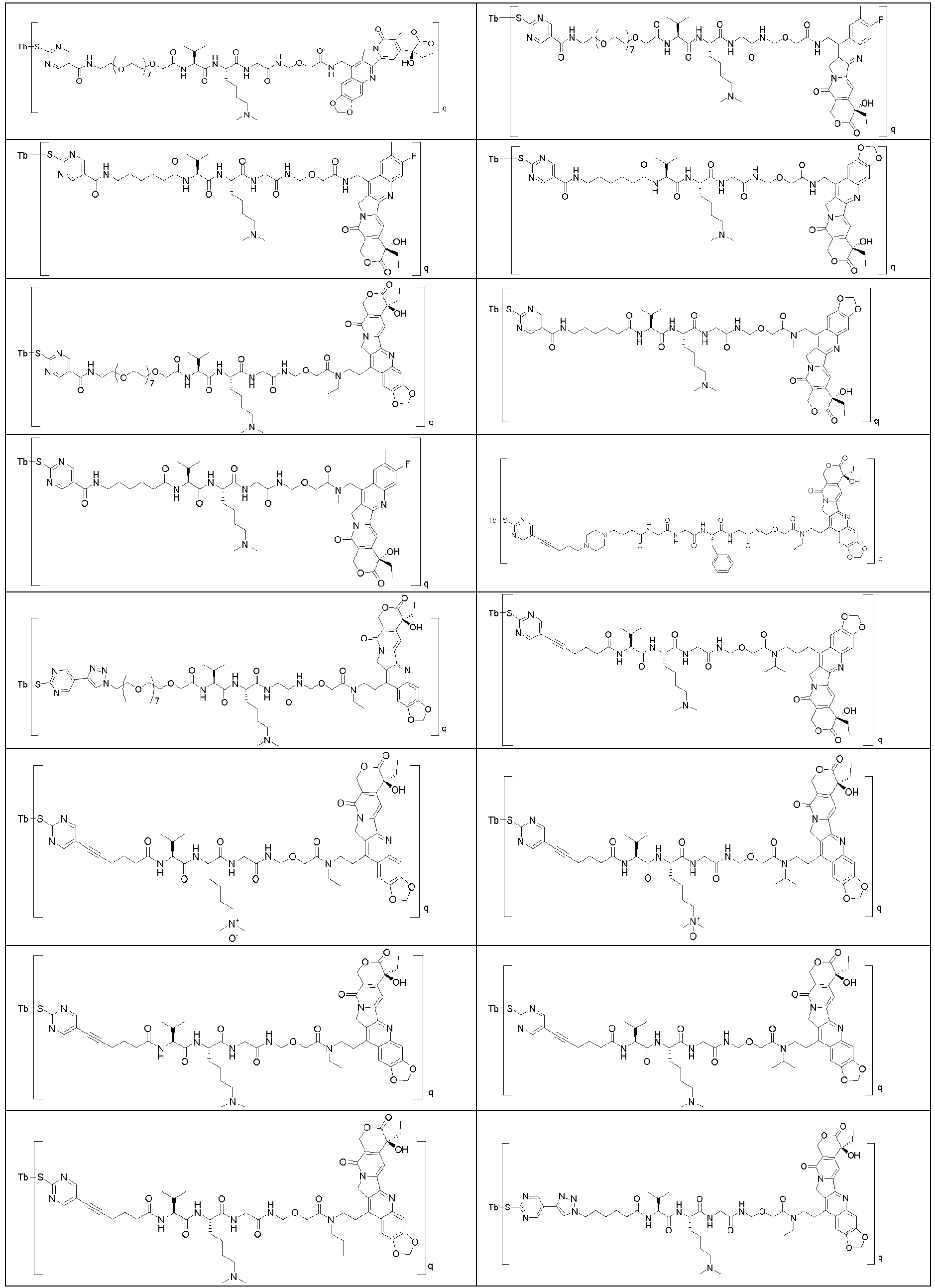

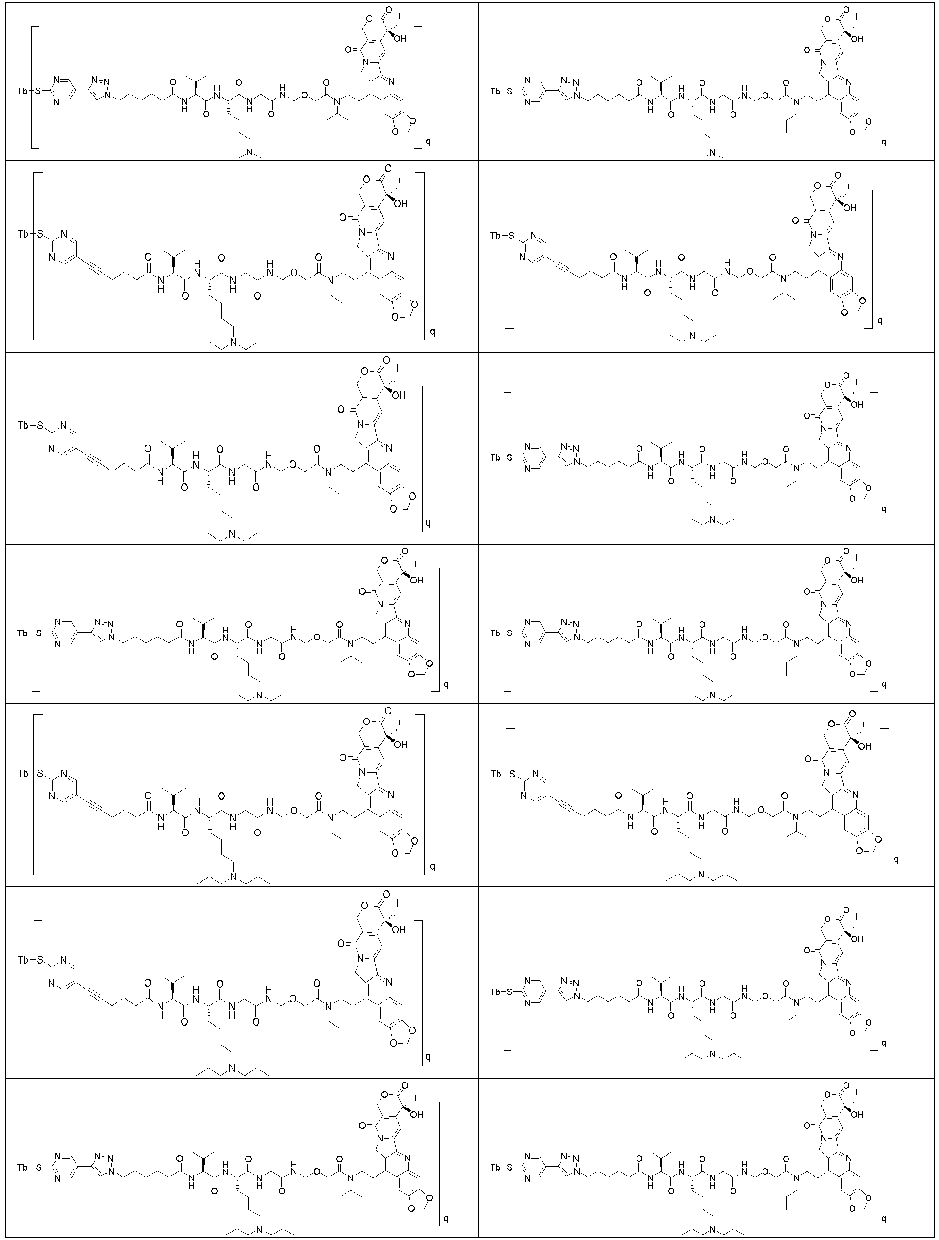

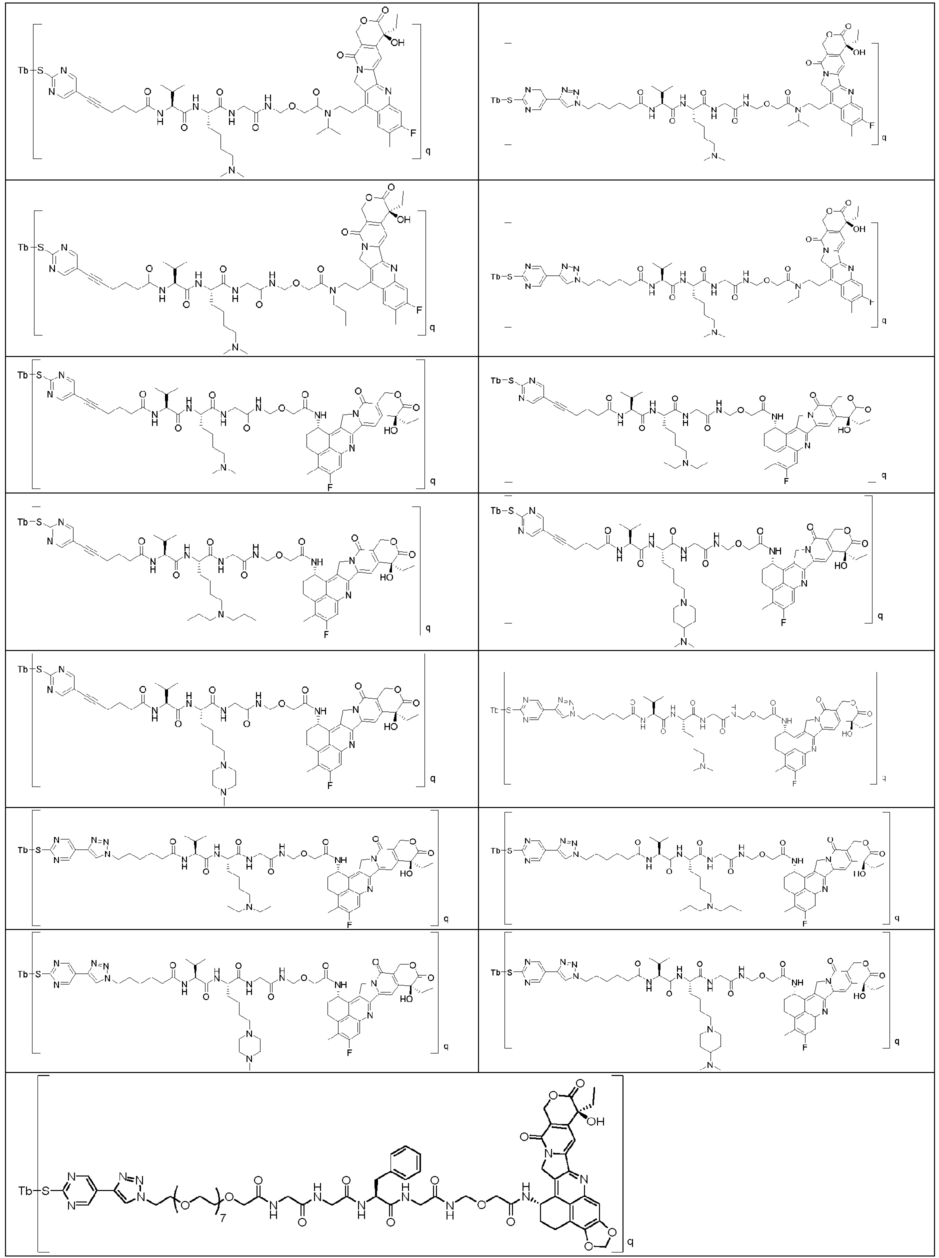

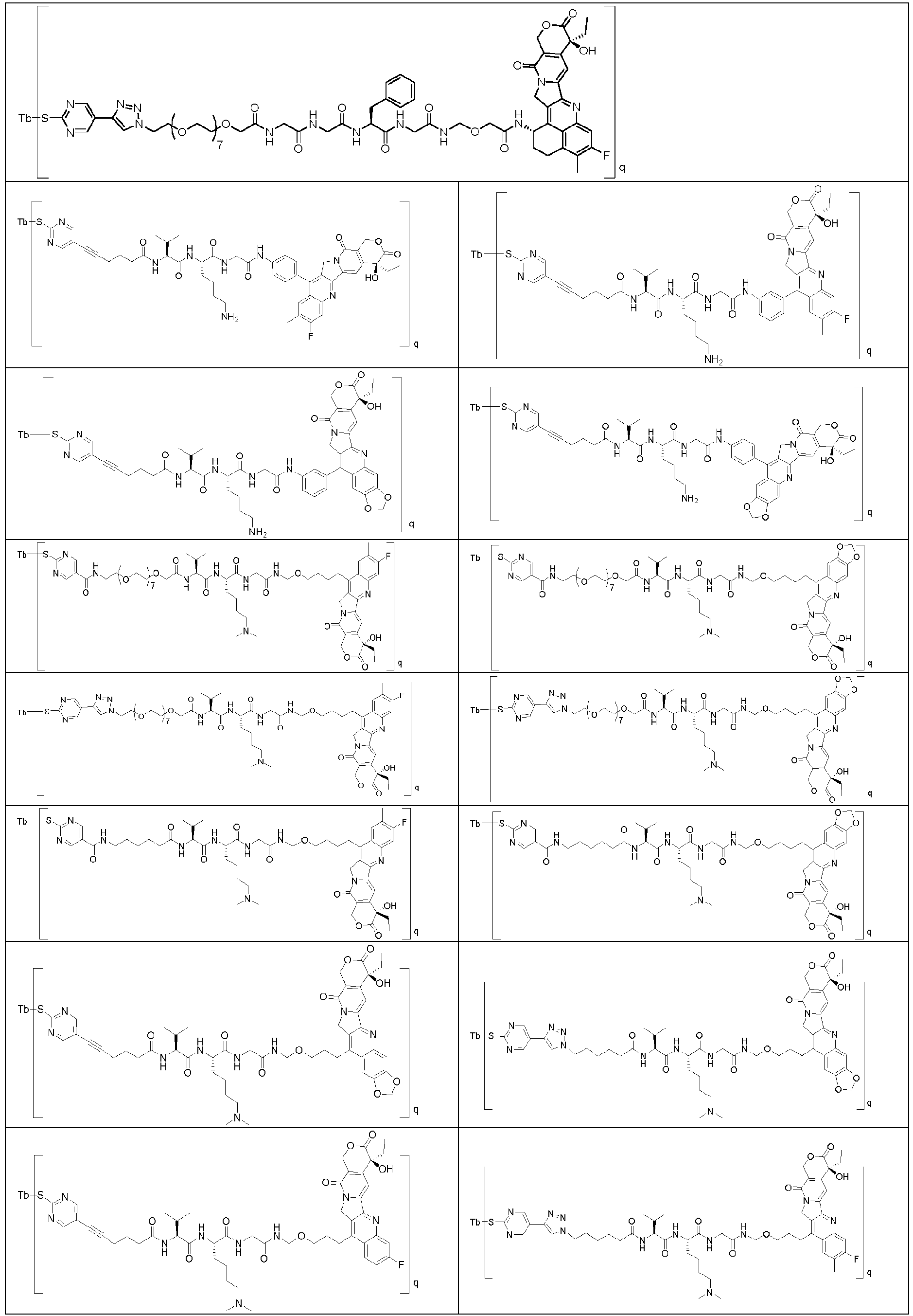

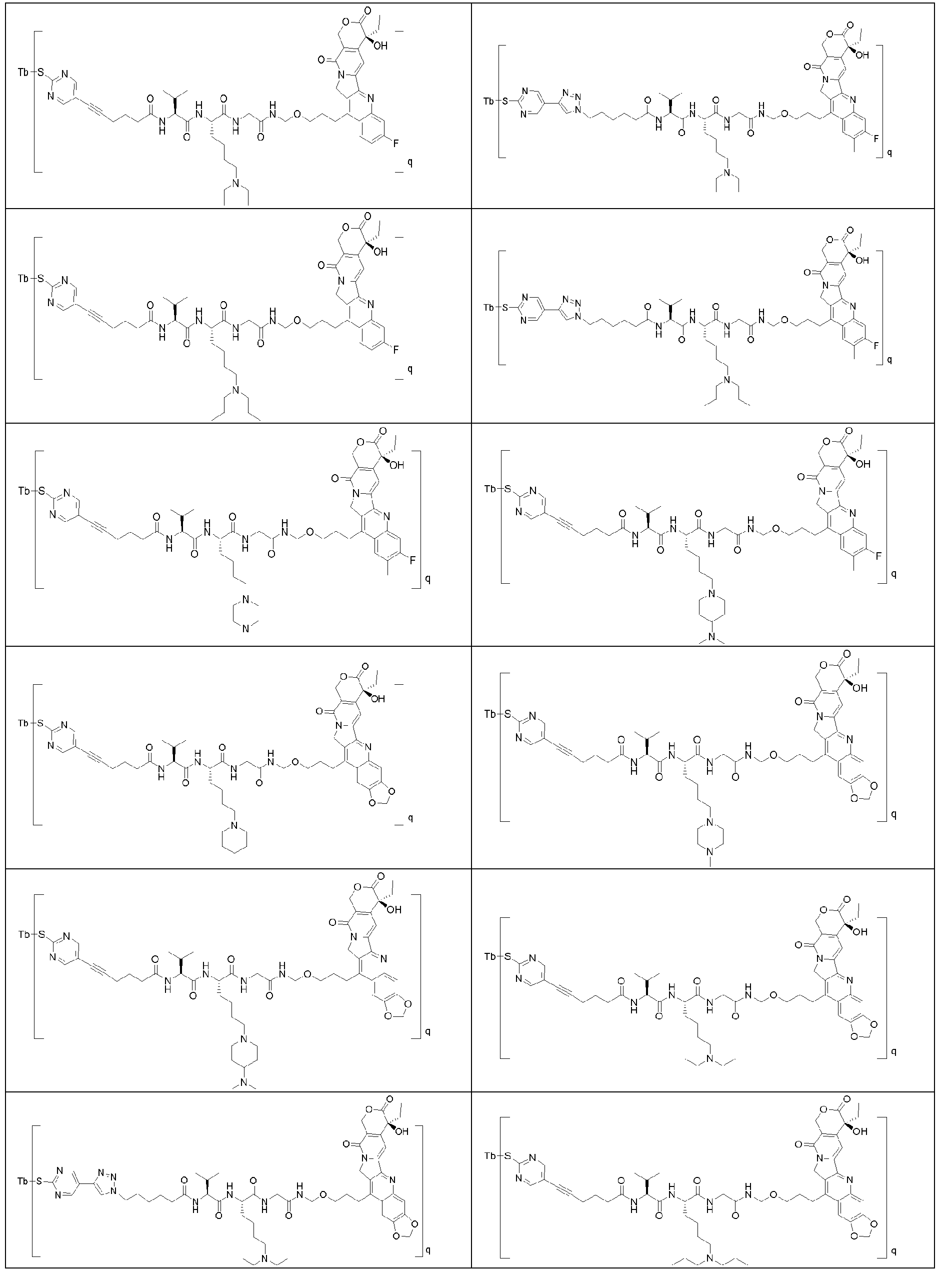

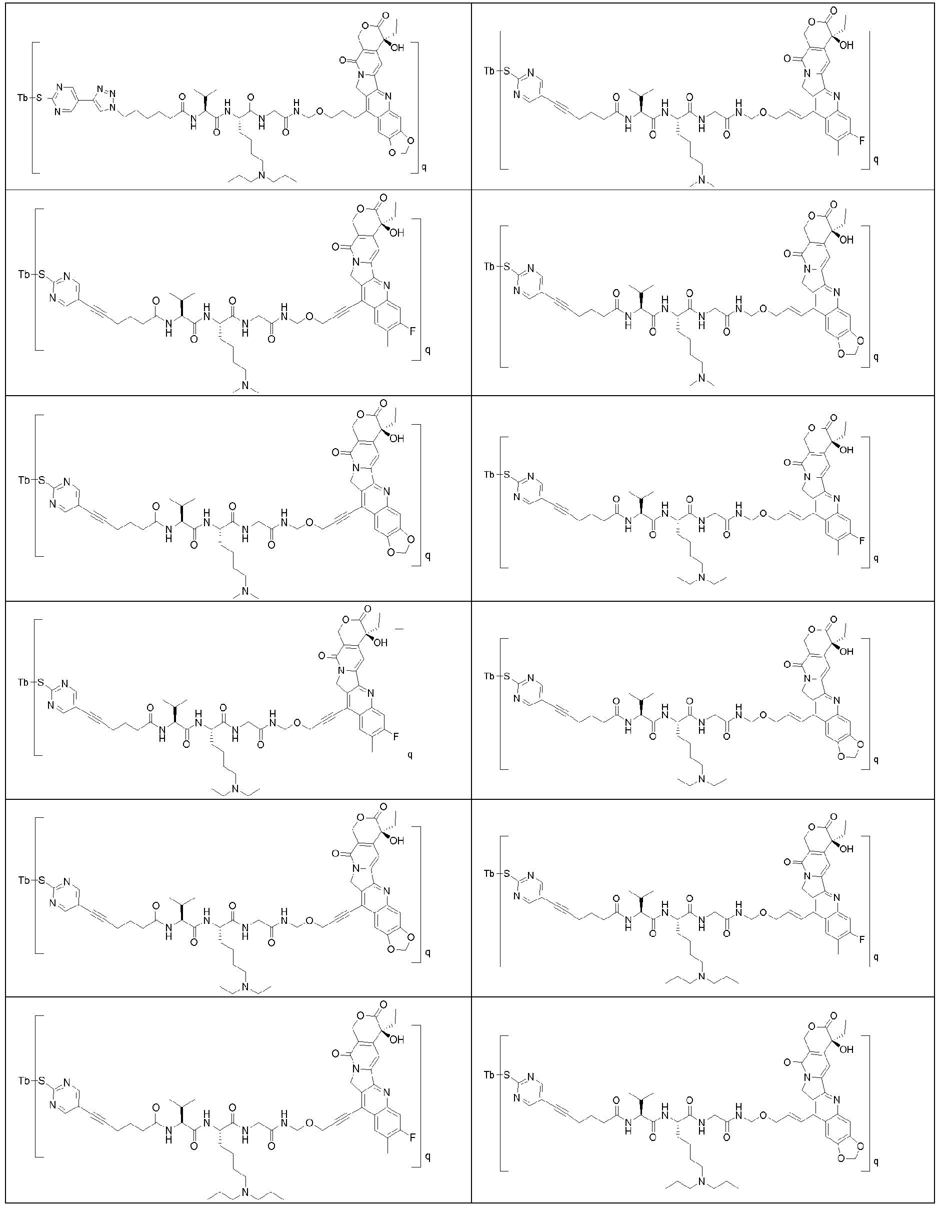

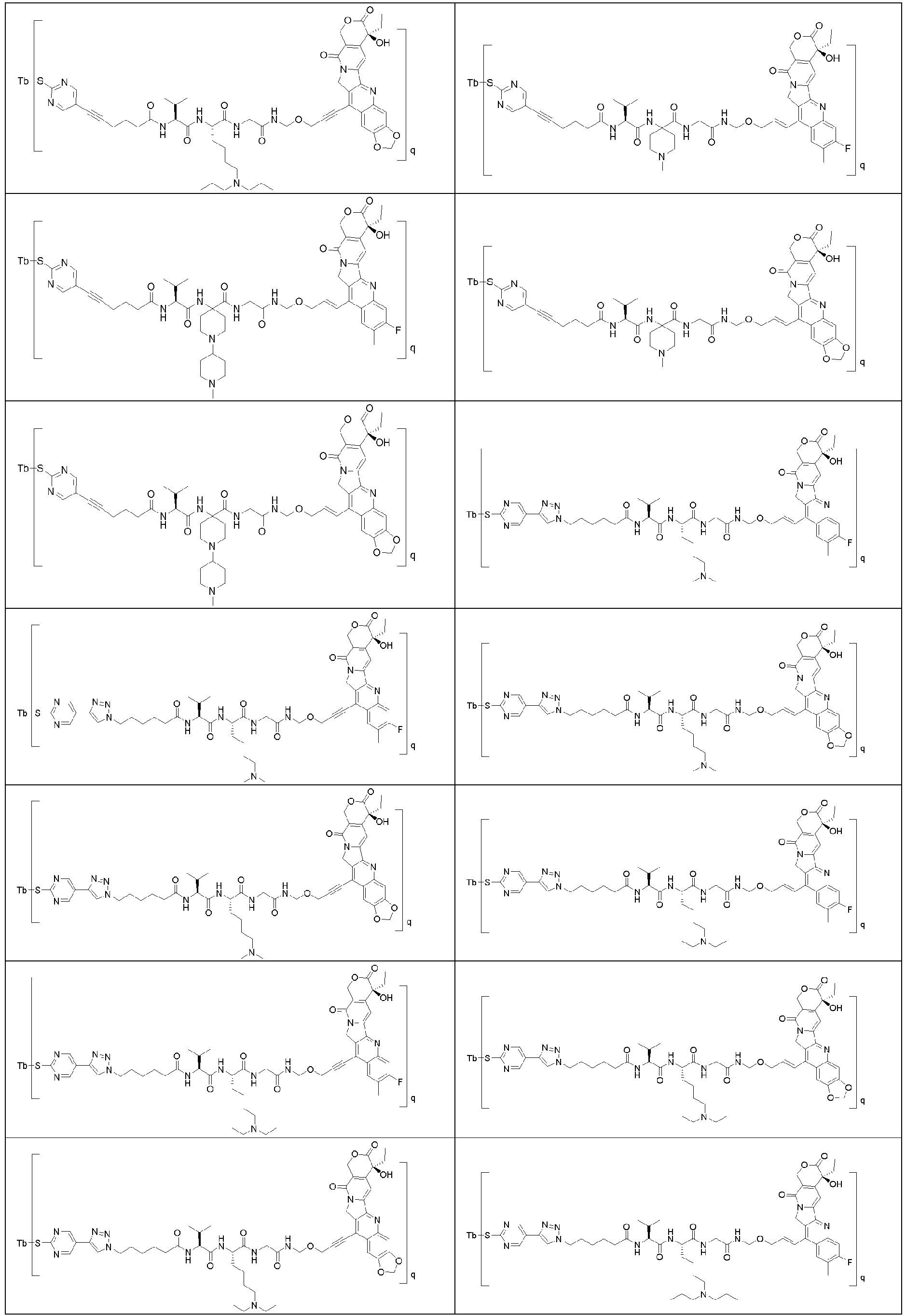

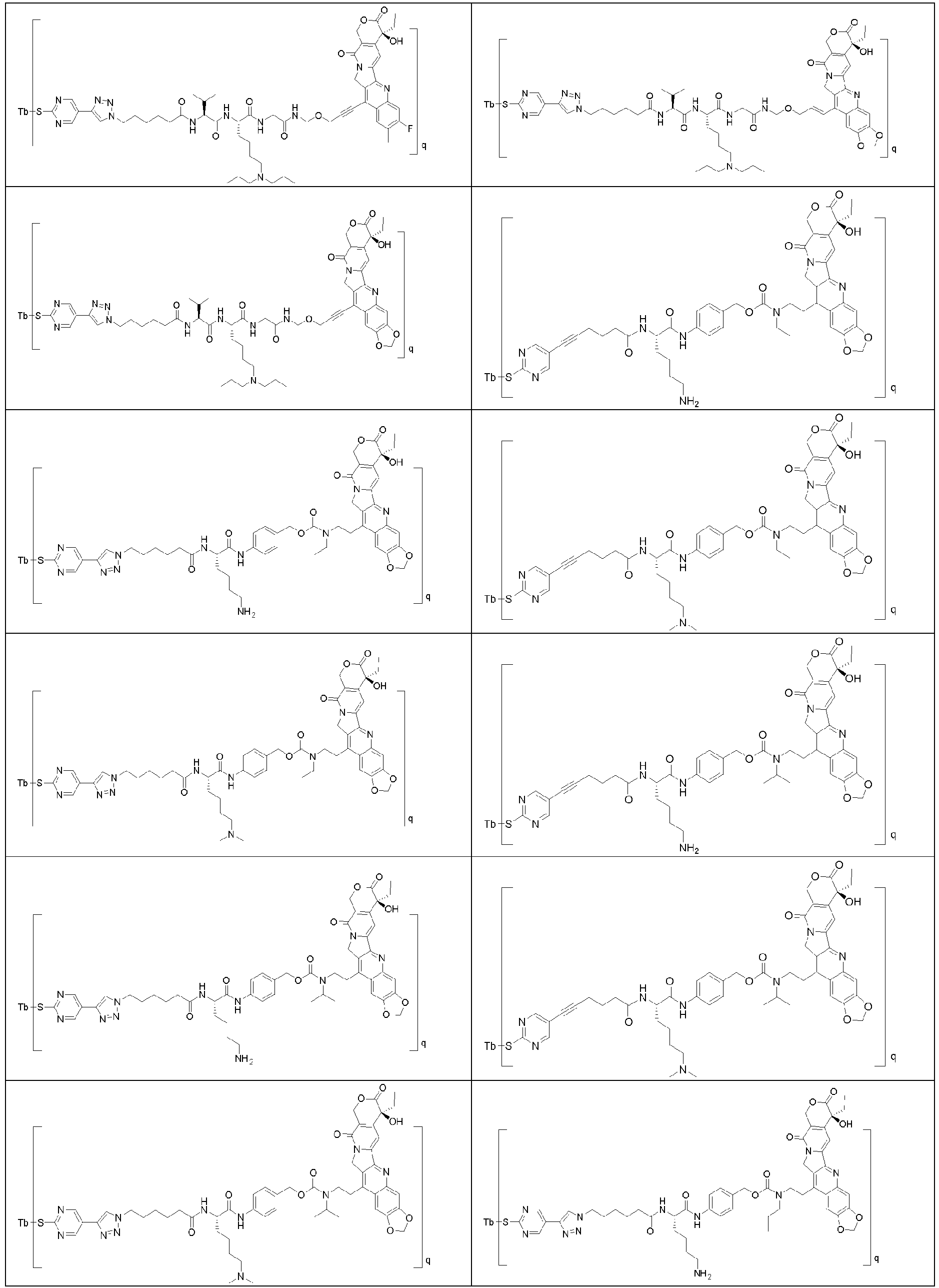

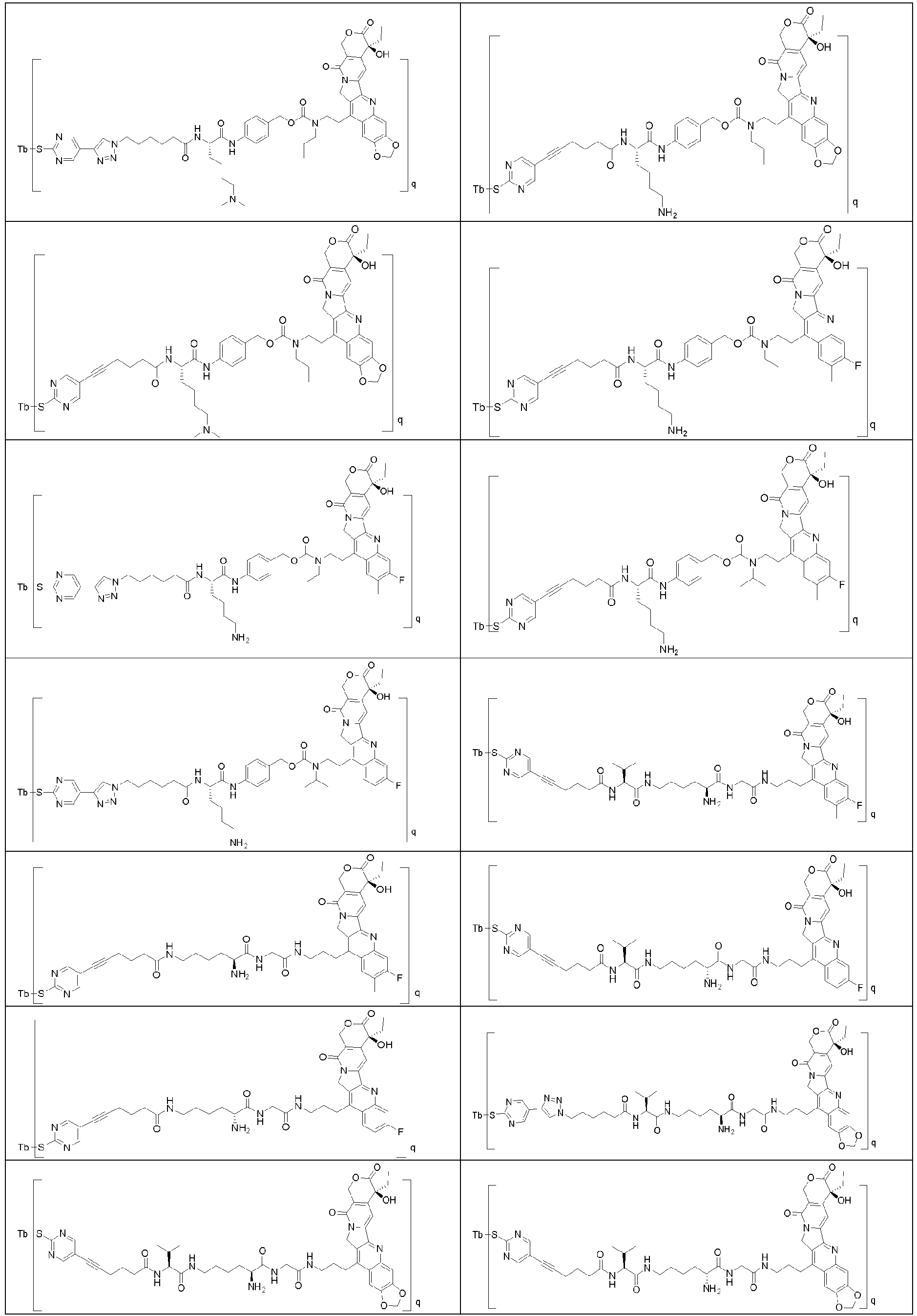

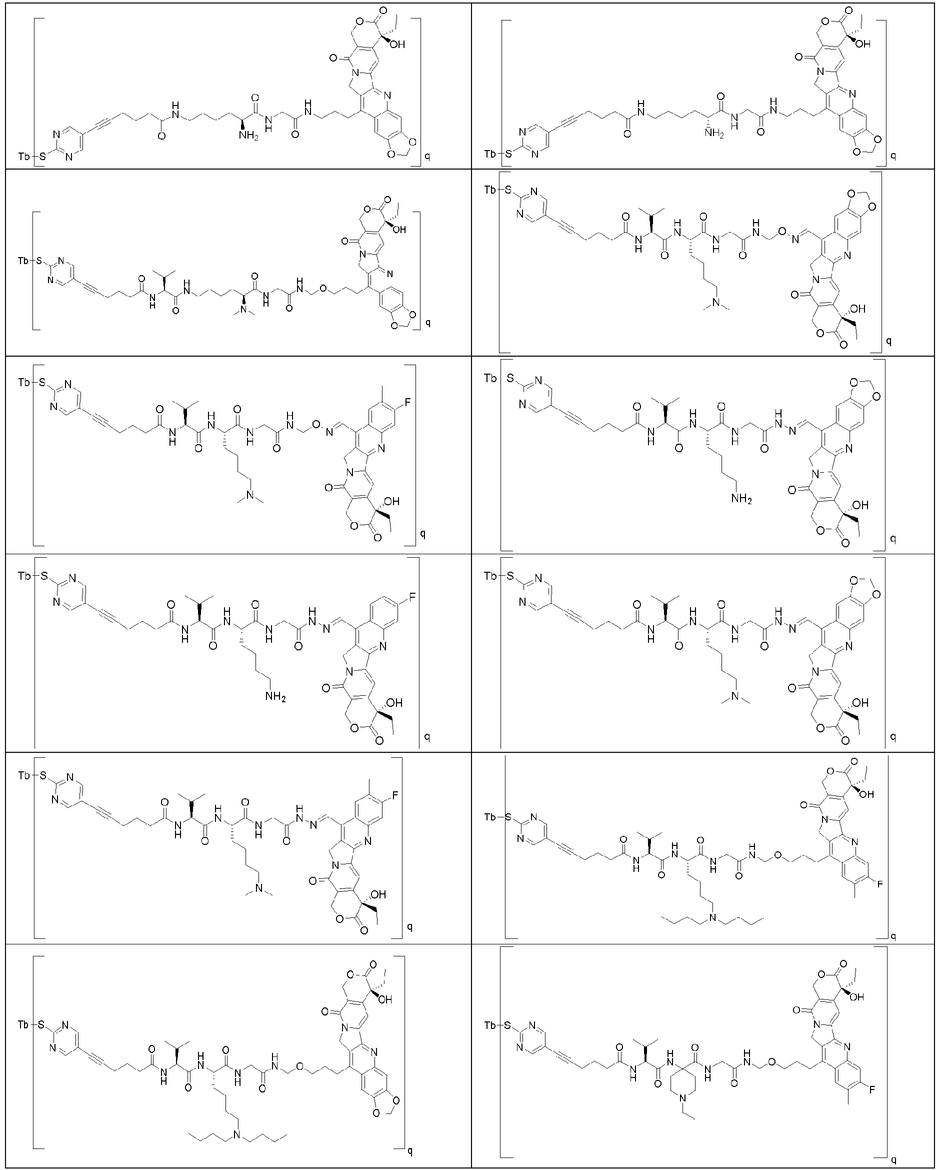

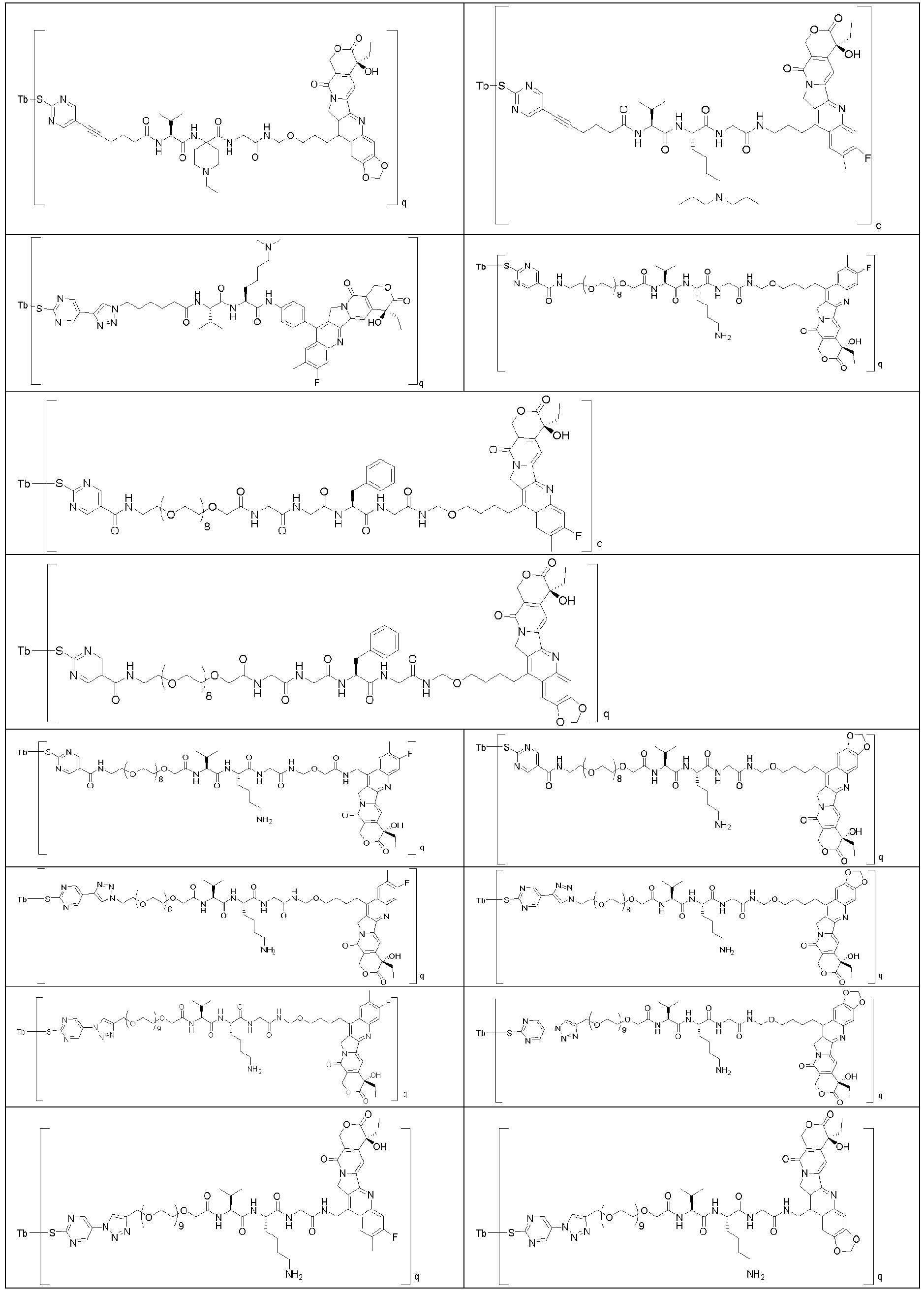

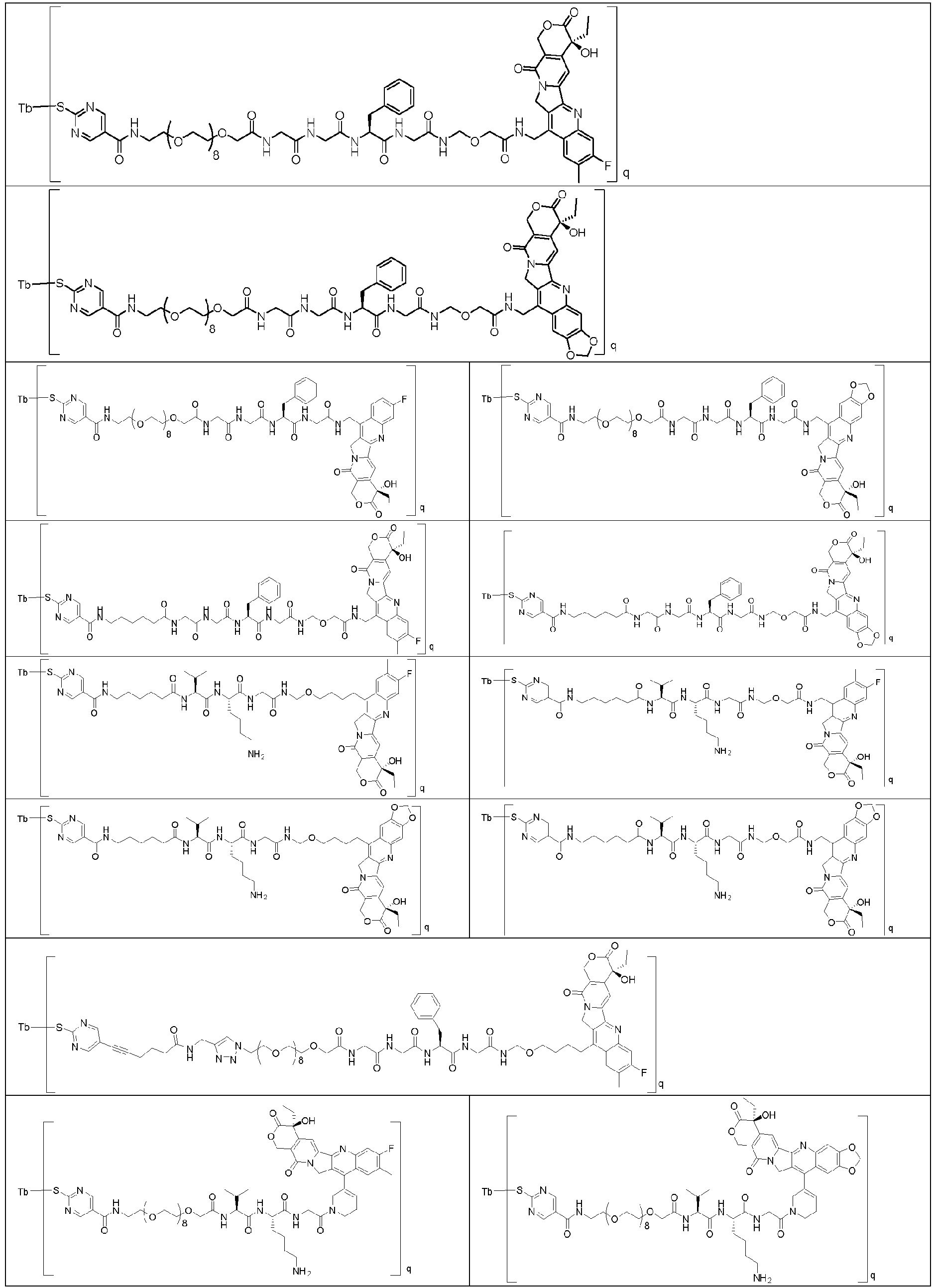

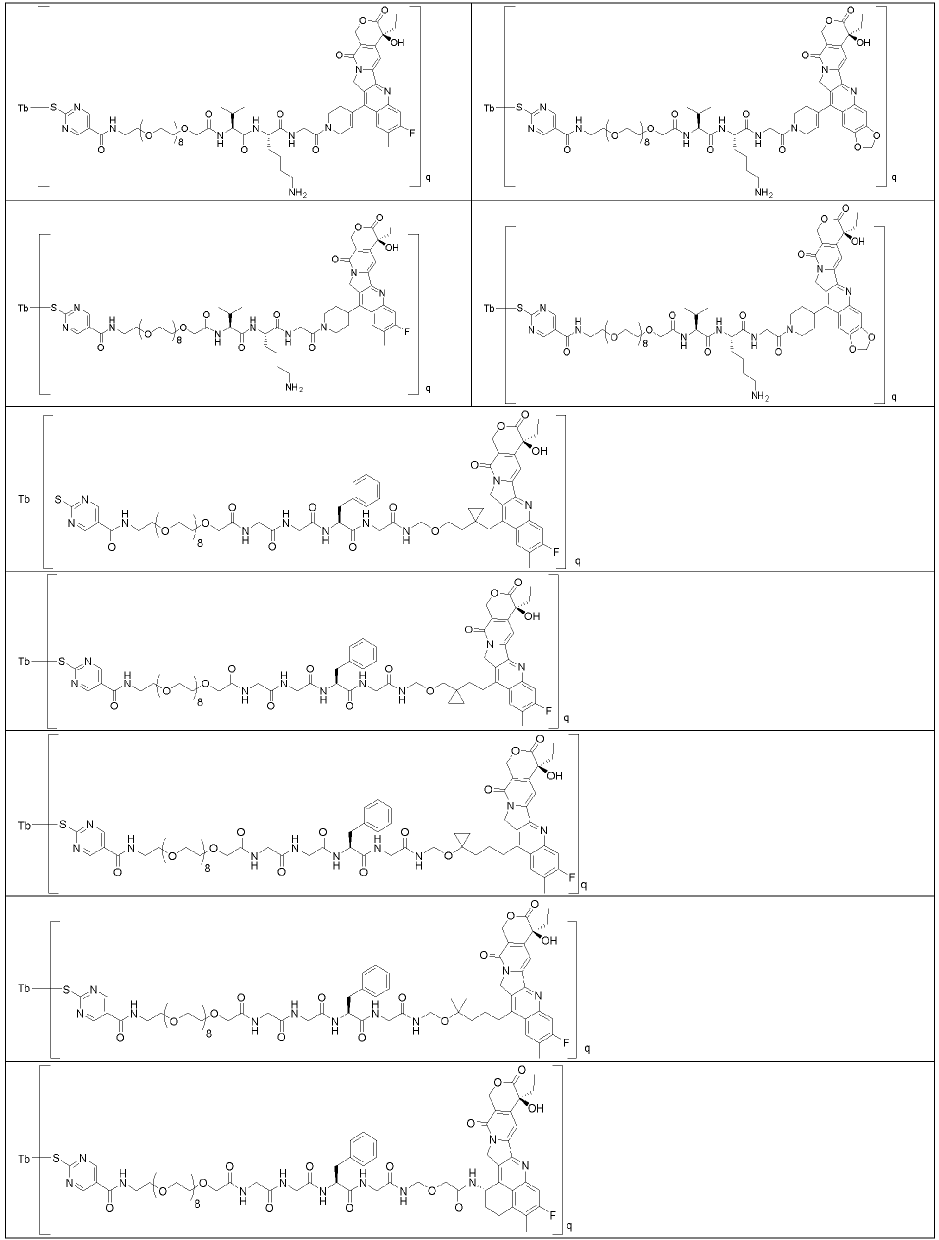

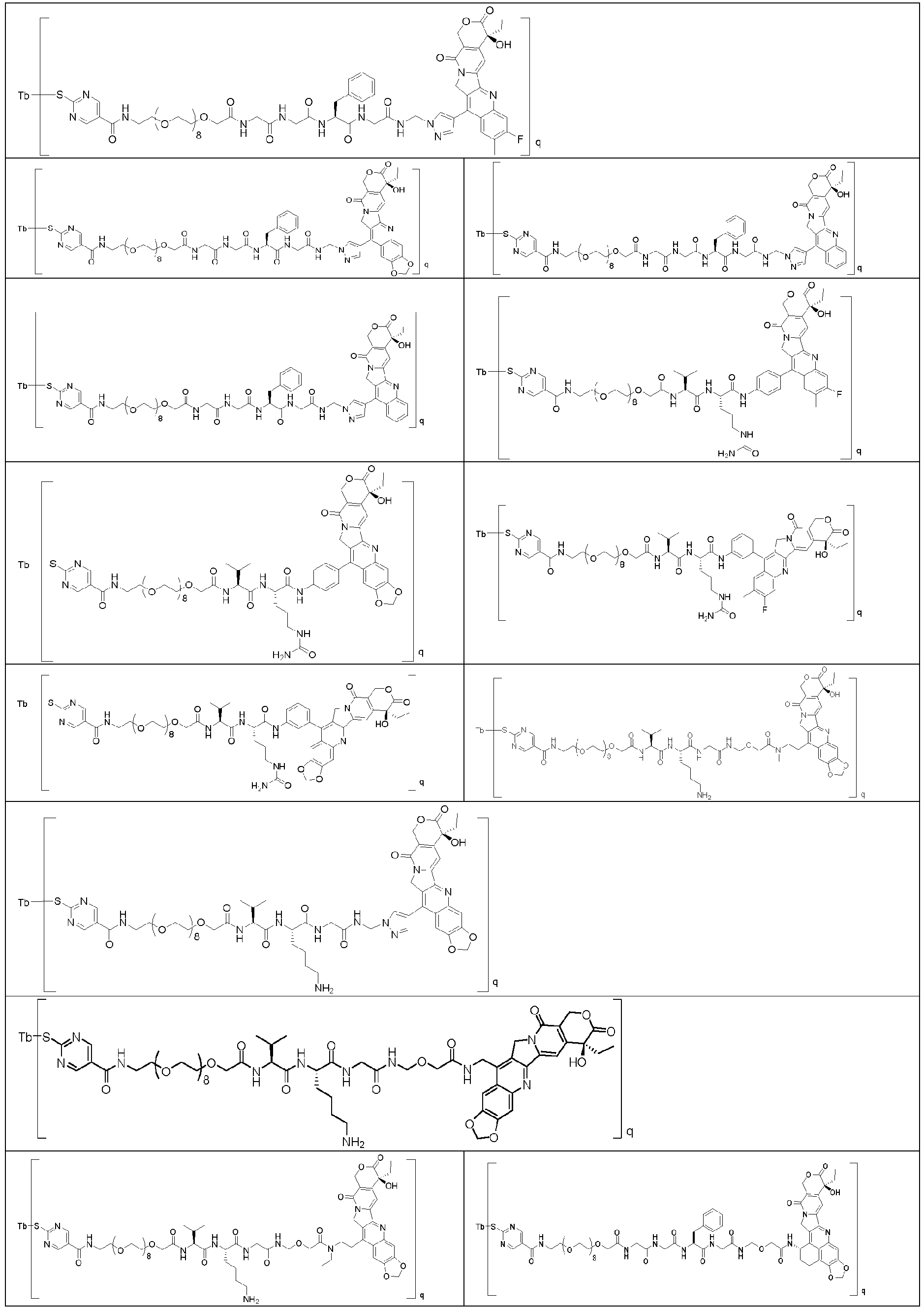

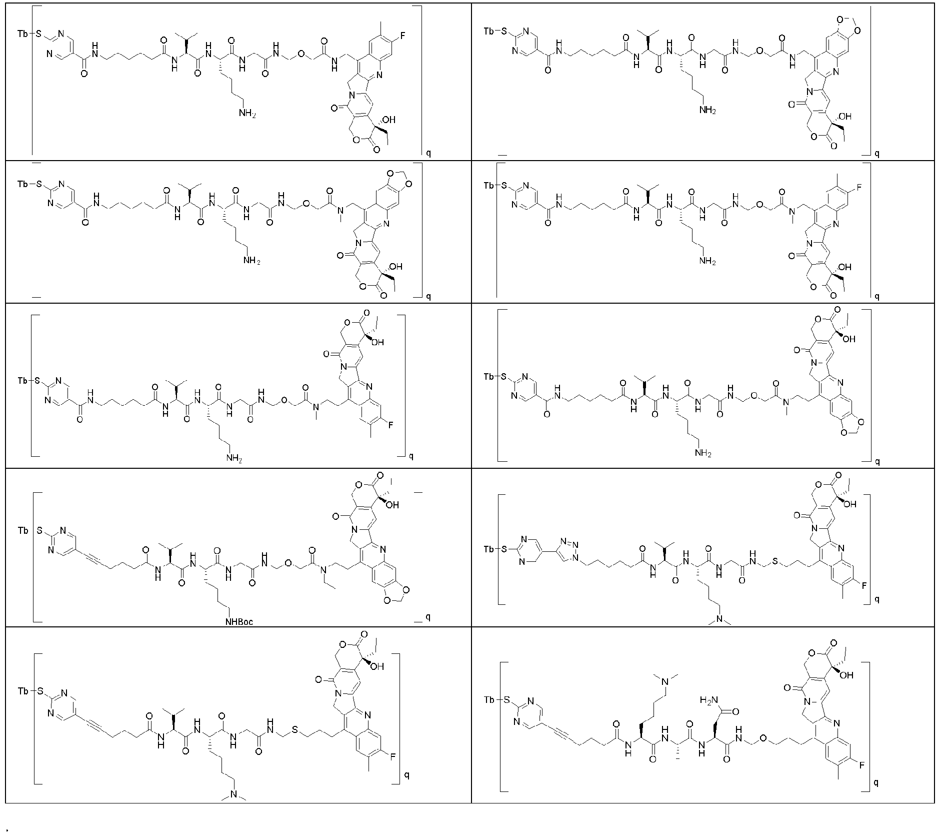

21. The antibody-drug conjugate or the stereoisomer of the antibody-drug conjugate, the prodrug thereof, the pharmaceutically acceptable salt thereof, or the pharmaceutically acceptable solvate thereof according to any one of claims 1-20, wherein
Tb is an anti-NaPi2b antibody or an antigen-binding fragment thereof, preferably, the anti-NaPi2b antibody is selected from antibodies lifastuzumab, upifitamab, 60A12B3-hz1, 22H9C4-hz1, 66C12D12-hz1, 34F2A10-hz1, C1, 16G5B3-hz2, and 16G5B3-hz1, or antigen-binding fragments thereof.

22. An anti-NaPi2b antibody or an antigen-binding fragment thereof, comprising the following complementarity determining regions (CDRs):
(a) HCDR1, HCDR2, and HCDR3 comprised in a heavy chain variable region VH set forth in SEQ ID NO: 1, 17, 19, 34, 36, 52, 54, 71, 73, 74, or 33, and/or
(b) LCDR1, LCDR2, and LCDR3 comprised in a light chain variable region VL set forth in SEQ ID NO: 2, 18, 20, 35, 37, 53, 55, 72, 75, or 51.

23. The antibody or the antigen-binding fragment thereof according to claim 22, comprising:
(1) a VH and/or a VL, wherein as defined according to the IMGT numbering system:
(a) the VH comprises HCDR1 with the sequence of SEQ ID NO: 11, HCDR2 with the sequence of SEQ ID NO: 12, and HCDR3 with the sequence of SEQ ID NO: 14; and/or
the VL comprises LCDR1 with the sequence of SEQ ID NO: 15, LCDR2 with the sequence that is WAS, and LCDR3 with the sequence of SEQ ID NO: 10;
(b) the VH comprises HCDR1 with the sequence of SEQ ID NO: 11, HCDR2 with the sequence of SEQ ID NO: 13, and HCDR3 with the sequence of SEQ ID NO: 14; and/or
the VL comprises LCDR1 with the sequence of SEQ ID NO: 15, LCDR2 with the sequence that is WAS, and LCDR3 with the sequence of SEQ ID NO: 10;
(c) the VH comprises HCDR1 with the sequence of SEQ ID NO: 28, HCDR2 with the sequence of SEQ ID NO: 29, and HCDR3 with the sequence of SEQ ID NO: 31; and/or
the VL comprises LCDR1 with the sequence of SEQ ID NO: 32, LCDR2 with the sequence that is YTS, and LCDR3 with the sequence of SEQ ID NO: 27;
(d) the VH comprises HCDR1 with the sequence of SEQ ID NO: 28, HCDR2 with the sequence of SEQ ID NO: 30, and HCDR3 with the sequence of SEQ ID NO: 31; and/or
the VL comprises LCDR1 with the sequence of SEQ ID NO: 32, LCDR2 with the sequence that is YTS, and LCDR3 with the sequence of SEQ ID NO: 27;
(e) the VH comprises HCDR1 with the sequence of SEQ ID NO: 46, HCDR2 with the sequence of SEQ ID NO: 47, and HCDR3 with the sequence of SEQ ID NO: 49; and/or
the VL comprises LCDR1 with the sequence of SEQ ID NO: 50, LCDR2 with the sequence that is SAS, and LCDR3 with the sequence of SEQ ID NO: 45;
(f) the VH comprises HCDR1 with the sequence of SEQ ID NO: 46, HCDR2 with the sequence of SEQ ID NO: 48, and HCDR3 with the sequence of SEQ ID NO: 49; and/or
the VL comprises LCDR1 with the sequence of SEQ ID NO: 50, LCDR2 with the sequence that is SAS, and LCDR3 with the sequence of SEQ ID NO: 45;
(g) the VH comprises HCDR1 with the sequence of SEQ ID NO: 65, HCDR2 with the sequence of SEQ ID NO: 66, and HCDR3 with the sequence of SEQ ID NO: 68; and/or
the VL comprises LCDR1 with the sequence of SEQ ID NO: 69, LCDR2 with the sequence that is WAS, and LCDR3 with the sequence of SEQ ID NO: 64;
(h) the VH comprises HCDR1 with the sequence of SEQ ID NO: 65, HCDR2 with the sequence of SEQ ID NO: 67, and HCDR3 with the sequence of SEQ ID NO: 68; and/or
the VL comprises LCDR1 with the sequence of SEQ ID NO: 69, LCDR2 with the sequence that is WAS, and LCDR3 with the sequence of SEQ ID NO: 64;
or
(i) the VH comprises HCDR1 with the sequence of SEQ ID NO: 76, HCDR2 with the sequence of SEQ ID NO: 77, and HCDR3 with the sequence of SEQ ID NO: 78; and/or
the VL comprises LCDR1 with the sequence of SEQ ID NO: 79, LCDR2 with the sequence that is HTS, and LCDR3 with the sequence of SEQ ID NO: 16; or
(2) a VH and/or a VL, wherein as defined according to the Kabat numbering system:
(a) the VH comprises HCDR1 with the sequence of SEQ ID NO: 3, HCDR2 with the sequence of SEQ ID NO: 4, and HCDR3 with the sequence of SEQ ID NO: 6; and/or
the VL comprises LCDR1 with the sequence of SEQ ID NO: 7, LCDR2 with the sequence of SEQ ID NO: 9, and LCDR3 with the sequence of SEQ ID NO: 10;
(b) the VH comprises HCDR1 with the sequence of SEQ ID NO: 3, HCDR2 with the sequence of SEQ ID NO: 5, and HCDR3 with the sequence of SEQ ID NO: 6; and/or
the VL comprises LCDR1 with the sequence of SEQ ID NO: 8, LCDR2 with the sequence of SEQ ID NO: 9, and LCDR3 with the sequence of SEQ ID NO: 10;
(c) the VH comprises HCDR1 with the sequence of SEQ ID NO: 21, HCDR2 with the sequence of SEQ ID NO: 22, and HCDR3 with the sequence of SEQ ID NO: 24; and/or
the VL comprises LCDR1 with the sequence of SEQ ID NO: 25, LCDR2 with the sequence of SEQ ID NO: 26, and LCDR3 with the sequence of SEQ ID NO: 27;
(d) the VH comprises HCDR1 with the sequence of SEQ ID NO: 21, HCDR2 with the sequence of SEQ ID NO: 23, and HCDR3 with the sequence of SEQ ID NO: 24; and/or
the VL comprises LCDR1 with the sequence of SEQ ID NO: 25, LCDR2 with the sequence of SEQ ID NO: 26, and LCDR3 with the sequence of SEQ ID NO: 27;
(e) the VH comprises HCDR1 with the sequence of SEQ ID NO: 38, HCDR2 with the sequence of SEQ ID NO: 39, and HCDR3 with the sequence of SEQ ID NO: 41; and/or
the VL comprises LCDR1 with the sequence of SEQ ID NO: 42, LCDR2 with the sequence of SEQ ID NO: 44, and LCDR3 with the sequence of SEQ ID NO: 45;
(f) the VH comprises HCDR1 with the sequence of SEQ ID NO: 38, HCDR2 with the sequence of SEQ ID NO: 40, and HCDR3 with the sequence of SEQ ID NO: 41; and/or
the VL comprises LCDR1 with the sequence of SEQ ID NO: 43, LCDR2 with the sequence of SEQ ID NO: 44, and LCDR3 with the sequence of SEQ ID NO: 45;
(g) the VH comprises HCDR1 with the sequence of SEQ ID NO: 56, HCDR2 with the sequence of SEQ ID NO: 57, and HCDR3 with the sequence of SEQ ID NO: 60; and/or
the VL comprises LCDR1 with the sequence of SEQ ID NO: 61, LCDR2 with the sequence of SEQ ID NO: 63, and LCDR3 with the sequence of SEQ ID NO: 64;
(h) the VH comprises HCDR1 with the sequence of SEQ ID NO: 56, HCDR2 with the sequence of SEQ ID NO: 58, and HCDR3 with the sequence of SEQ ID NO: 60; and/or
the VL comprises LCDR1 with the sequence of SEQ ID NO: 62, LCDR2 with the sequence of SEQ ID NO: 63, and LCDR3 with the sequence of SEQ ID NO: 64; or
(i) the VH comprises HCDR1 with the sequence of SEQ ID NO: 56, HCDR2 with the sequence of SEQ ID NO: 59, and HCDR3 with the sequence of SEQ ID NO: 60; and/or
the VL comprises LCDR1 with the sequence of SEQ ID NO: 62, LCDR2 with the sequence of SEQ ID NO: 63, and LCDR3 with the sequence of SEQ ID NO: 64;
preferably, the anti-NaPi2b antibody or the antigen-binding fragment thereof binds to human NaPi2b, monkey NaPi2b, and/or rat NaPi2b.

24. The anti-NaPi2b antibody or the antigen-binding fragment thereof according to claim 22 or 23, comprising:
(a) the VH set forth in SEQ ID NO: 1 or 17, and/or the VL set forth in SEQ ID NO: 2 or 18;
(b) the VH set forth in SEQ ID NO: 19 or 34, and/or the VL set forth in SEQ ID NO: 20 or 35;
(c) the VH set forth in SEQ ID NO: 36 or 52, and/or the VL set forth in SEQ ID NO: 37 or 53;
(d) the VH set forth in SEQ ID NO: 54, 71, or 73, and/or the VL set forth in SEQ ID NO: 55 or 72;
(e) the VH set forth in SEQ ID NO: 74 or 33, and/or the VL set forth in SEQ ID NO: 75 or 51;
(f) a VH having at least 70%, at least 80%, at least 85%, at least 90%, at least 91%, at least 92%, at least 93%, at least 94%, at least 95%, at least 96%, at least 97%, at least 98%, or at least 99% sequence identity compared with any one of the VHs in (a)-(e); and/or a VL having at least 70%, at least 80%, at least 85%, at least 90%, at least 91%, at least 92%, at least 93%, at least 94%, at least 95%, at least 96%, at least 97%, at least 98%, or at least 99% sequence identity compared with any one of the VLs in (a)-(e); or
(g) a VH having one or several amino acid substitutions, deletions, or additions, or any combination thereof, such as 1, 2, 3, 4, 5, 6, 7, 8, 9, or 10 amino acid substitutions, deletions, or additions, or any combination thereof, compared with any one of the VHs in (a)-(e); and/or a VL having one or several amino acid substitutions, deletions, or additions, or any combination thereof, such as 1, 2, 3, 4, 5, 6, 7, 8, 9, or 10 amino acid substitutions, deletions, or additions, or any combination thereof, compared with any one of the VLs in (a)-(e); preferably, the substitution is a conservative substitution.

25. The anti-NaPi2b antibody or the antigen-binding fragment thereof according to any one of claims 22-24, comprising:
(a) the VH with the sequence set forth in SEQ ID NO: 1 and the VL with the sequence set forth in SEQ ID NO: 2;
(b) the VH with the sequence set forth in SEQ ID NO: 17 and the VL with the sequence set forth in SEQ ID NO: 18;
(c) the VH with the sequence set forth in SEQ ID NO: 19 and the VL with the sequence set forth in SEQ ID NO: 20;
(d) the VH with the sequence set forth in SEQ ID NO: 34 and the VL with the sequence set forth in SEQ ID NO: 35;
(e) the VH with the sequence set forth in SEQ ID NO: 36 and the VL with the sequence set forth in SEQ ID NO: 37;
(f) the VH with the sequence set forth in SEQ ID NO: 52 and the VL with the sequence set forth in SEQ ID NO: 53;
(g) the VH with the sequence set forth in SEQ ID NO: 54 and the VL with the sequence set forth in SEQ ID NO: 55;
(h) the VH with the sequence set forth in SEQ ID NO: 71 and the VL with the sequence set forth in SEQ ID NO: 72;
(i) the VH with the sequence set forth in SEQ ID NO: 73 and the VL with the sequence set forth in SEQ ID NO: 72;
(j) the VH with the sequence set forth in SEQ ID NO: 74 and the VL with the sequence set forth in SEQ ID NO: 75;
(k) the VH with the sequence set forth in SEQ ID NO: 33 and the VL with the sequence set forth in SEQ ID NO: 51;
(l) a VH and a VL, wherein the VH has at least 70%, at least 80%, at least 85%, at least 90%, at least 91%, at least 92%, at least 93%, at least 94%, at least 95%, at least 96%, at least 97%, at least 98%, or at least 99% sequence identity, and/or the VL has at least 70%, at least 80%, at least 85%, at least 90%, at least 91%, at least 92%, at least 93%, at least 94%, at least 95%, at least 96%, at least 97%, at least 98%, or at least 99% sequence identity, compared with the VH and the VL in any one of groups (a) to (k), respectively; or
(m) a VH and a VL, wherein the VH has one or several amino acid substitutions, deletions, or additions, or any combination thereof, such as 1, 2, 3, 4, 5, 6, 7, 8, 9, or 10 amino acid substitutions, deletions, or additions, or any combination thereof, and/or the VL has one or several amino acid substitutions, deletions, or additions, or any combination thereof, such as 1, 2, 3, 4, 5, 6, 7, 8, 9, or 10 amino acid substitutions, deletions, or additions, or any combination thereof, compared with the VH and the VL in any one of groups (a) to (k), respectively; preferably, the substitution is a conservative substitution.

26. The anti-NaPi2b antibody or the antigen-binding fragment thereof according to any one of claims 22-25, wherein the antibody or the antigen-binding fragment thereof is a chimeric antibody, a humanized antibody, or a fully human antibody;
optionally, the antibody or the antigen-binding fragment thereof is selected from scFv, Fab, Fab', (Fab')₂, Fv fragment, disulfide-linked Fv (dsFv), diabody, and multispecific antibody;
for example, the multispecific antibody is a bispecific antibody or a trispecific antibody or a tetraspecific antibody.

27. The anti-NaPi2b antibody or the antigen-binding fragment thereof according to any one of claims 22-26, further comprising:
(a) a heavy chain constant region CH of a human immunoglobulin or a variant thereof; and/or
(b) a light chain constant region CL of a human immunoglobulin or a variant thereof,
wherein the variant has at least 70%, at least 80%, at least 85%, at least 90%, at least 91%, at least 92%, at least 93%, at least 94%, at least 95%, at least 96%, at least 97%, at least 98%, or at least 99% sequence identity compared with the wild-type sequence from which it is derived; or the variant has one or more amino acid substitutions, deletions, or additions, or any combination thereof, such as at most 50, at most 45, at most 40, at most 35, at most 30, at most 25, at most 20, at most 15, at most 10, or at most 5 amino acid substitutions, deletions, or additions, or any combination thereof, e.g., 1, 2, 3, 4, 5, 6, 7, 8, 9, or 10 amino acid substitutions, deletions, or additions, or any combination thereof, compared with the wild-type sequence from which it is derived; preferably, the substitution is a conservative substitution;
preferably, the heavy chain constant region is an IgG heavy chain constant region, such as an IgG1, IgG2, IgG3 or IgG4 heavy chain constant region; and/or the light chain constant region is a κ or λ light chain constant region;
more preferably, the antibody or the antigen-binding fragment thereof comprises a human IgG1 heavy chain constant region; and/or the antibody or the antigen-binding fragment thereof comprises a human κ light chain constant region.

28. The anti-NaPi2b antibody or the antigen-binding fragment thereof according to claim 27, wherein
the heavy chain constant region comprises a CH set forth in SEQ ID NO: 70 or a variant thereof, wherein the variant has at most 20 conservative amino acid substitutions, such as at most 20, at most 15, at most 10, or at most 5 conservative amino acid substitutions, e.g., 1, 2, 3, 4, 5, 6, 7, 8, 9, or 10 conservative amino acid substitutions, compared with SEQ ID NO: 70, or has at least 70%, at least 80%, at least 85%, at least 90%, at least 91%, at least 92%, at least 93%, at least 94%, at least 95%, at least 96%, at least 97%, at least 98%, or at least 99% sequence identity compared with SEQ ID NO: 70; and/or
the light chain constant region comprises a CL set forth in SEQ ID NO: 80 or a variant thereof, wherein the variant has at most 20 conservative amino acid substitutions, such as at most 20, at most 15, at most 10, or at most 5 conservative amino acid substitutions, e.g., 1, 2, 3, 4, 5, 6, 7, 8, 9, or 10 conservative amino acid substitutions, compared with SEQ ID NO: 80, or has at least 70%, at least 80%, at least 85%, at least 90%, at least 91%, at least 92%, at least 93%, at least 94%, at least 95%, at least 96%, at least 97%, at least 98%, or at least 99% sequence identity compared with SEQ ID NO: 80;
preferably, the antibody or the antigen-binding fragment thereof comprises the heavy chain constant region CH set forth in SEQ ID NO. 70 and the light chain constant region CL set forth in SEQ ID NO. 80.

29. The anti-NaPi2b antibody or the antigen-binding fragment thereof according to any one of claims 22-28, wherein the antibody comprises:
(a) a heavy chain comprising the VH set forth in SEQ ID NO: 71 and the CH set forth in SEQ ID NO: 70, and a light chain comprising the VL set forth in SEQ ID NO: 72 and the CL set forth in SEQ ID NO: 80;
(b) a heavy chain comprising the VH set forth in SEQ ID NO: 73 and the CH set forth in SEQ ID NO: 70, and a light chain comprising the VL set forth in SEQ ID NO: 72 and the CL set forth in SEQ ID NO: 80, preferably a heavy chain set forth in SEQ ID NO: 81 and a light chain set forth in SEQ ID NO: 82; or
(c) a heavy chain comprising the VH set forth in SEQ ID NO: 17 and the CH set forth in SEQ ID NO: 70, and a light chain comprising the VL set forth in SEQ ID NO: 18 and the CL set forth in SEQ ID NO: 80, preferably a heavy chain set forth in SEQ ID NO: 83 and a light chain set forth in SEQ ID NO: 84.

30. The antibody-drug conjugate or the stereoisomer of the antibody-drug conjugate, the prodrug thereof, the pharmaceutically acceptable salt thereof, or the pharmaceutically acceptable solvate thereof according to any one of claims 1-21, wherein Tb is selected from the anti-NaPi2b antibody or the antigen-binding fragment thereof according to any one of claims 22-29.

31. The antibody-drug conjugate or the stereoisomer of the antibody-drug conjugate, the prodrug thereof, the pharmaceutically acceptable salt thereof, or the pharmaceutically acceptable solvate thereof according to any one of claims 1-21 and 30, wherein the antibody-drug conjugate is selected from: 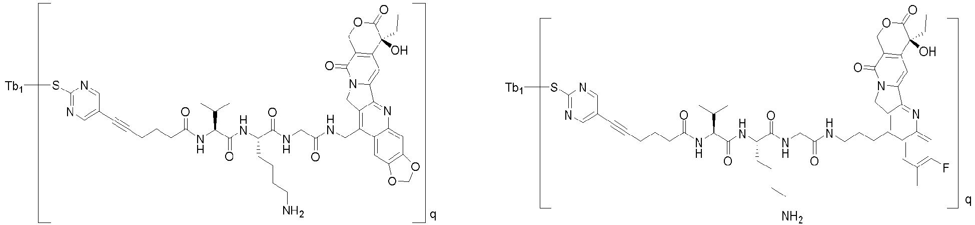 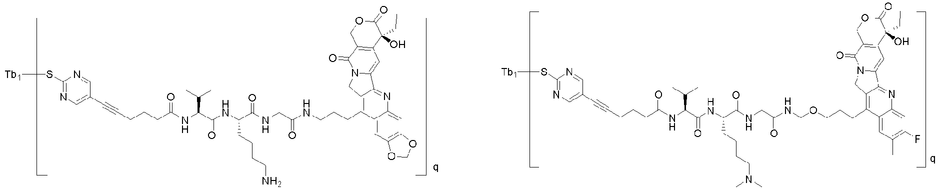 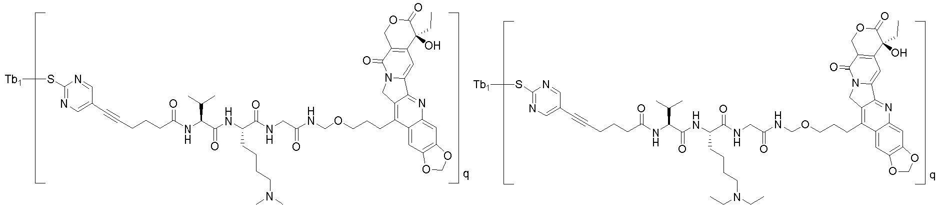 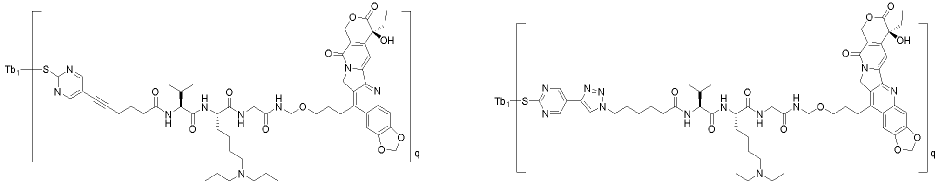 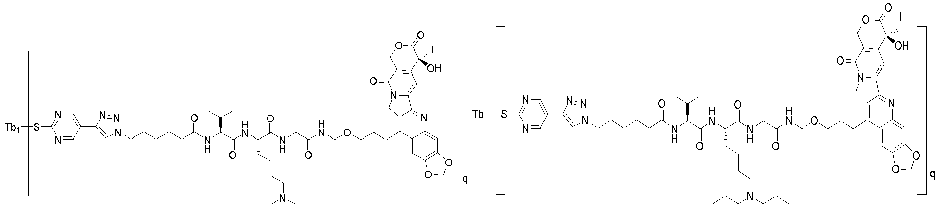 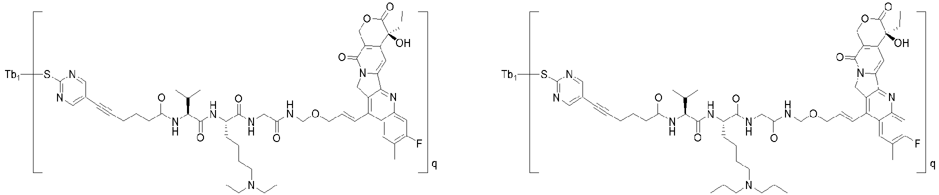 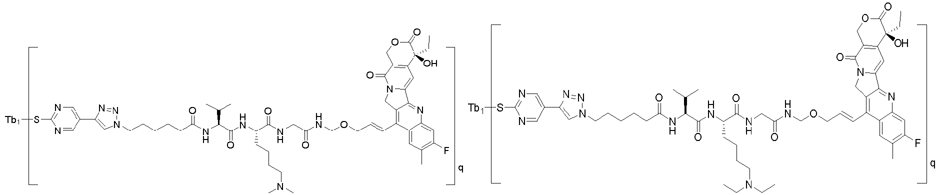 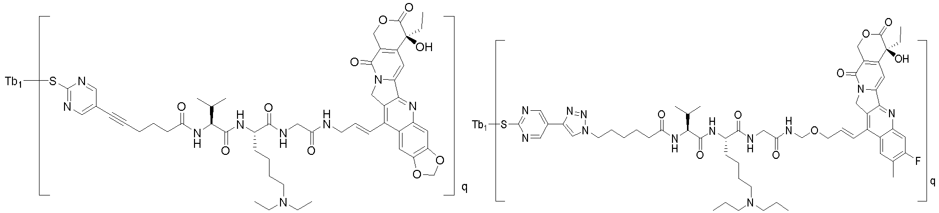 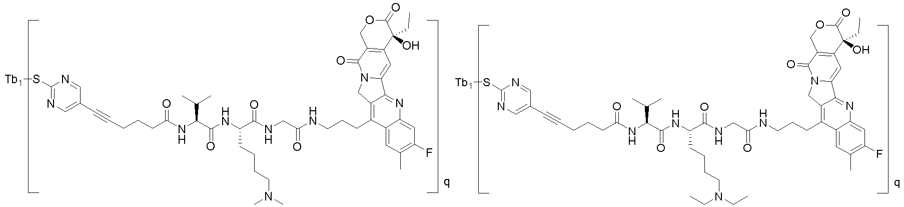 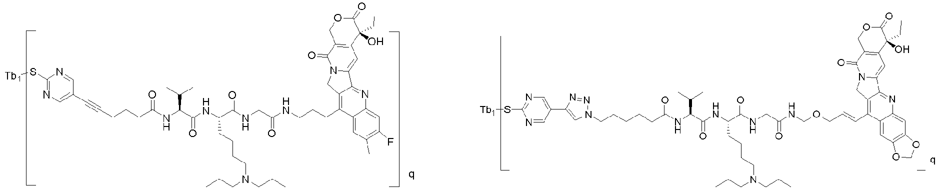 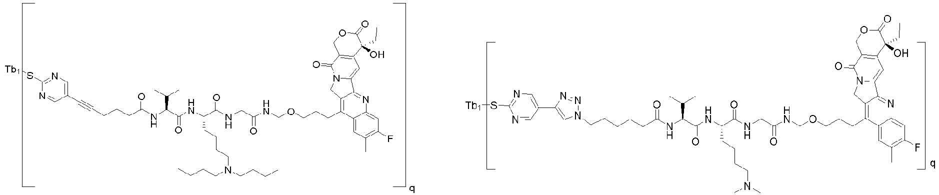 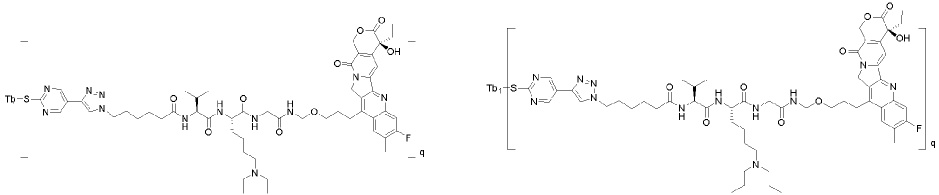 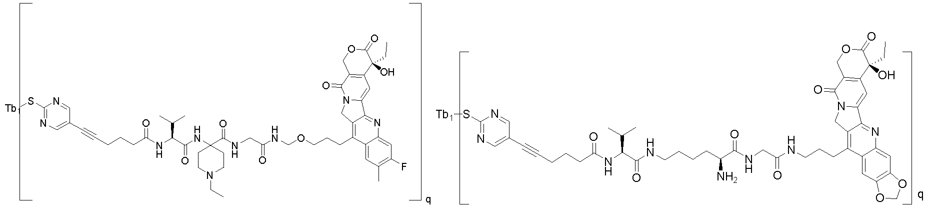 wherein Tb₁ is lifastuzumab, upifitamab, 60A12B3-hz1, 22H9C4-hz1, 66C12D12-hz1, 34F2A10-hz1, C1, 16G5B3-hz2, and 16G5B3-hz1 antibody; q is selected from any numerical value between 0.1 and 16.0, preferably any numerical value between 2 and 8, and more preferably q is 2, 4, 6, or 8.

32. A method for preparing the antibody-drug conjugate according to any one of claims 1-21, 30, and 31, comprising:
subjecting Tb and a drug-linker conjugate of formula III 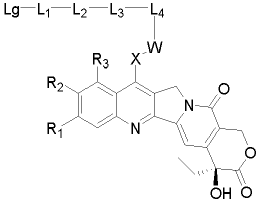
to a conjugation reaction in a suitable solvent and under suitable conditions,
wherein:
L₁, L₂, L₃, L₄, and Tb are as defined in any one of claims 1-21;
R₁, R₂, R₃, X, and W are as defined in any one of claims 1-21;
Lg is a leaving group, and Lg is selected from halogen, a sulfone group, a tertiary amine salt group (Me3N+ or Et3N+), a diazonium salt group, -OMs, MeSO2-, and CF3SO3-.

33. The method according to claim 32, comprising a step of subjecting Tb and the drug-linker conjugate of formula III 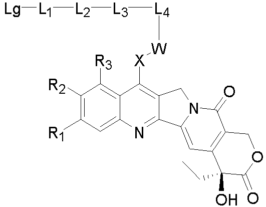 to the conjugation reaction in the suitable solvent and under the suitable conditions to form a C-S bond,
wherein preferably, Tb and the drug-linker conjugate are in a molar ratio of 1:(1-20);
preferably, the conjugation reaction is carried out in water and/or an organic solvent; preferably, the organic solvent is selected from N,N-dimethylformamide, dimethyl sulfoxide, N-methylpyrrolidone, nitriles (e.g., acetonitrile), alcohols (e.g., methanol and ethanol), and any combination thereof; the nitrile can be acetonitrile, and the alcohol can be methanol or ethanol;
preferably, the method further comprises a step of purifying the conjugate product; preferably, the conjugate product is purified by chromatography; preferably, the chromatography comprises one or more of ion exchange chromatography, hydrophobic chromatography, reversed-phase chromatography, or affinity chromatography.

34. An isolated nucleic acid molecule, encoding the anti-NaPi2b antibody or the antigen-binding fragment thereof according to any one of claims 22-29.

35. A vector, comprising the isolated nucleic acid molecule according to claim 34, wherein preferably, the vector is a cloning vector or an expression vector.

36. A host cell, comprising the isolated nucleic acid molecule according to claim 34 or the vector according to claim 35.

37. A method for preparing the anti-NaPi2b antibody or the antigen-binding fragment thereof according to any one of claims 22-29, comprising culturing the host cell according to claim 36 under conditions allowing expression of the antibody or the antigen-binding fragment thereof, and isolating the antibody or the antigen-binding fragment thereof from the cultured host cell culture.

38. An antibody-drug conjugate, wherein the antibody is the anti-NaPi2b antibody or the antigen-binding fragment thereof according to any one of claims 22-29, attached via a linker to a conjugate moiety; the conjugate moiety is selected from: a detectable label, a radioisotope, a fluorescent substance, a luminescent substance, a colored substance, an enzyme, polyethylene glycol, a nuclide, a nucleic acid, a small molecule toxin, a polypeptide having binding activity, a protein, a receptor, a ligand, and other active substances that inhibit tumor cell growth and promote tumor cell apoptosis or necrosis.

39. A population of antibody-drug conjugates, comprising the antibody-drug conjugate according to any one of claims 1-21, 30, 31, and 38, wherein the antibody-drug conjugates have one, two, or more q values.

40. A pharmaceutical composition, comprising the antibody-drug conjugate according to any one of claims 1-21, 30, 31, and 38, or the stereoisomer of the antibody-drug conjugate, the prodrug thereof, the pharmaceutically acceptable salt thereof, or the pharmaceutically acceptable solvate thereof, or the anti-NaPi2b antibody or the antigen-binding fragment thereof according to any one of claims 22-29, or the nucleic acid molecule according to claim 34, or the vector according to claim 35, or the host cell according to claim 36, or the population of antibody-drug conjugates according to claim 39, wherein preferably, the pharmaceutical composition further comprises a pharmaceutically acceptable carrier and/or excipient.

41. The population according to claim 39 or the pharmaceutical composition according to claim 40, wherein the drug-to-antibody ratio (DAR) is selected from integers and decimals between 1 to 10;
preferably, the drug-to-antibody ratio (DAR) of the population is selected from 1.5-2.5, 3.5-4.5, 5.5-6.5, and 7.5-8.5;
preferably, the drug-to-antibody ratio (DAR) of the population is selected from about 2.0, 4.0, 6.0, and 8.0;
preferably, the drug-to-antibody ratio (DAR) of the population is selected from 2, 2.5, 3, 3.5, 4, 4.5, 5, 5.5, 6, 6.5, 7, 7.2, 7.4, 7.5, 7.6, 7.7, 7.8, 7.9, 8.0, 8.1, 8.2, 8.3, 8.4, 8.5, 8.7, 8.9, and 9.

42. Use of the antibody-drug conjugate according to any one of claims 1-21, 30, 31, and 38, or the stereoisomer of the antibody-drug conjugate, the prodrug thereof, the pharmaceutically acceptable salt thereof, or the pharmaceutically acceptable solvate thereof, or the anti-NaPi2b antibody or the antigen-binding fragment thereof according to any one of claims 22-29, or the nucleic acid molecule according to claim 34, or the vector according to claim 35, or the host cell according to claim 36, or the population of antibody-drug conjugates according to claim 39, or the pharmaceutical composition according to claim 40 in the manufacture of a medicament for treating and/or preventing a disease associated with abnormal cell activity,
wherein optionally, the disease associated with abnormal cell activity can be a cancer disease;
preferably, the cancer disease is selected from esophageal cancer, brain tumor, lung cancer, squamous cell carcinoma, bladder cancer, gastric cancer, ovarian cancer, peritoneal cancer, pancreatic cancer, breast cancer, head and neck cancer, cervical cancer, endometrial cancer, colon cancer, rectal cancer, colorectal cancer, liver cancer, kidney cancer, urothelial cancer, epidermal cancer, non-Hodgkin's lymphoma, central nervous system tumor, prostate cancer, and thyroid cancer; the esophageal cancer is, for example, esophageal adenocarcinoma or esophageal squamous cell carcinoma; the lung cancer is, for example, small cell lung cancer, non-small cell lung cancer, or lung adenocarcinoma; the central nervous system tumor is, for example, neuroglioma, glioblastoma multiforme, glioma, or sarcoma; the colon cancer is, for example, human colon adenocarcinoma;
preferably, the cancer disease is selected from colon cancer, colorectal cancer, colon adenocarcinoma, small cell lung cancer, and ovarian cancer;
more preferably, the cancer disease is a cancer disease associated with NaPi2b.
